(19) Europäisches Patentamt
European Patent Office
Office européen des brevets

(11) EP 4 215 525 A1

(12) **EUROPEAN PATENT APPLICATION**
published in accordance with Art. 153(4) EPC

(43) Date of publication:
**26.07.2023 Bulletin 2023/30**

(21) Application number: **21868671.5**

(22) Date of filing: **16.09.2021**

(51) International Patent Classification (IPC):
***C07D 403/10*** *(2006.01)* ***A61K 31/4425*** *(2006.01)*
***A61P 7/02*** *(2006.01)*

(52) Cooperative Patent Classification (CPC):
**A61K 31/4425; A61P 7/02; C07D 403/10**

(86) International application number:
**PCT/CN2021/118671**

(87) International publication number:
**WO 2022/057849 (24.03.2022 Gazette 2022/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.09.2020 CN 202010978095**
**28.05.2021 CN 202110597053**

(71) Applicants:
- **Zhejiang Hisun Pharmaceutical Co., Ltd.**
  **Taizhou, Zhejiang 318000 (CN)**
- **Shanghai Aryl Pharmtech Co., Ltd.**
  **Songjiang, Shanghai 201612 (CN)**

(72) Inventors:
- **QIU, Haibo**
  **Shanghai 201612 (CN)**
- **MA, Yutao**
  **Shanghai 201612 (CN)**
- **CHEN, Ahuan**
  **Shanghai 201612 (CN)**
- **ZHU, Yabo**
  **Zhejiang 318000 (CN)**
- **ZHANG, Binhao**
  **Zhejiang 318000 (CN)**
- **LU, Yongping**
  **Zhejiang 318000 (CN)**
- **YE, Cheng**
  **Shanghai 201612 (CN)**
- **HU, Taishan**
  **Shanghai 201612 (CN)**
- **QIAN, Wenjian**
  **Zhejiang 318000 (CN)**
- **CHEN, Lei**
  **Zhejiang 318000 (CN)**

(74) Representative: **Dehns**
**St. Bride's House**
**10 Salisbury Square**
**London EC4Y 8JD (GB)**

(54) **PIPERAZINE DERIVATIVE, PREPARATION METHOD THEREFOR AND USE THEREOF**

(57) The present invention relates to a piperazine derivative, a preparation method therefor, and use thereof in medicine. Specifically, the present invention relates to a piperazine derivative represented by general formula (I), a preparation method therefor, and a pharmaceutically acceptable salt or prodrug thereof, and use thereof as a therapeutic agent, in particular, as an inhibitor of coagulation factor XIa (FXIa), wherein the definition of each substituent in general formula (I) is the same as that in the description.

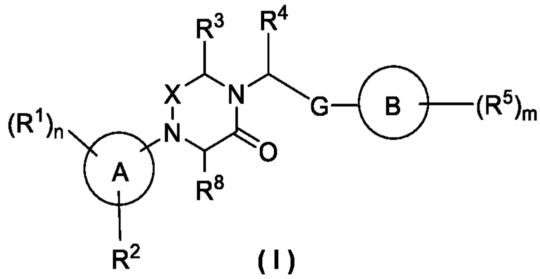
(I)

EP 4 215 525 A1

## Description

## TECHNICAL FIELD

The present invention relates to a piperazine derivative, a preparation method therefor, a pharmaceutical composition containing the derivative and use thereof as a therapeutic agent, in particular as an inhibitor of coagulation factor XIa (FXIa).

## BACKGROUND

Common cardiovascular diseases such as stroke, thrombus and myocardial infarction have become the main causes of death in the world. In the world death statistics in 2017, the death caused by the cardiovascular diseases accounted for about 32% of the total number of deaths in the world. The pathological process of these diseases is often closely related to thrombosis or the attack of stroke or heart disease caused by slow blood flow. Therefore, anticoagulants have been widely used in the treatment of such diseases. At present, traditional anticoagulants comprise a vitamin K antagonist, a fibrinolytic agent, heparin, anti-platelet aggregation and other drugs. However, due to the limitations of poor compliance, large variation coefficient among individuals, narrow therapeutic window and other factors of such drugs, oral anticoagulants of non-vitamin K antagonists have been continuously developed and marketed in recent years, and mainly comprise an antagonist directly inhibiting thrombin and an antagonist acting on coagulation factors. At present, the safety and effectiveness of the oral anticoagulants of non-vitamin K antagonists on the market have been significantly improved, but there is an adverse reaction of hemorrhage. In order to overcome this problem, it is of great significance to develop drugs with low hemorrhage risk. Studies at home and abroad have proved that FXIa inhibitors are potential drugs for treating cardiovascular and cerebrovascular diseases, especially thromboembolic diseases, and also provide a new direction for overcoming the adverse reaction of hemorrhage. The thromboembolic diseases comprise arterial cardiovascular thromboembolism, venous cardiovascular thromboembolism, arterial cerebrovascular thromboembolism, venous cerebrovascular thromboembolism, and ventricular or peripheral circulation thromboembolism. The thromboembolic diseases further comprise unstable angina pectoris, acute coronary syndrome, atrial fibrillation, myocardial infarction, sudden ischemic death, transient ischemic attack, stroke, atherosclerosis, peripheral occlusive arterial diseases, venous thrombosis, thrombophlebitis, arterial embolism, coronary artery thrombosis, cerebral arterial thrombosis, cerebral embolism, renal embolism, pulmonary embolism, and thrombosis caused by a medical implant, device or operation, wherein blood is contacted with an artificial surface capable of promoting thrombosis. Meanwhile, the venous thrombosis further comprises deep venous thrombosis.

FXI is a serine protease with a molecular weight of about 80 kD, and is composed of two subunits connected by a disulfide bond, and there are 607 amino acids on each polypeptide chain. Each subunit comprises a heavy chain region and a light chain region. The heavy chain has four AP structural domains A1 to A4, wherein A1 is bound to thrombin; A2 is bound to high-molecular-weight kininogen; A3 is bound to a coagulation factor IX and heparin; and A4 is bound to an activated coagulation factor XII. Therefore, the four AP domains are sites where FXI interacts with other protein kinases or factors. FXIa is an active state of FXI, and plays an active role in intrinsic coagulation. At the beginning of coagulation, the coagulation factor XII is activated to form XIIa, and XIIa activates the activity of FXI to form FXIa, so that a series of cascade reactions are caused to promote coagulation. Therefore, the inhibition of FXIa is an effective way to prevent thrombosis or slow blood flow.

Plasma prekallikrein is a liver-derived precursor of trypsin-like serine protease plasma kallikrein, and circulates in plasma bound to the high-molecular-weight kininogen. The plasma prekallikrein is activated by the activated coagulation factor XII or prolyl carboxypeptidase to form plasma kallikrein. The plasma kallikrein regulates the activities of several proteolytic cascades in a cardiovascular system, such as an intrinsic pathway of coagulation, a kallikrein-kinin system, a fibrinolysis system, a reninangiotensin system and a complement pathway. The plasma kallikrein plays a core role in the pathogenesis of thrombosis, inflammation and blood pressure regulation.

At present, there is fierce competition for the clinical development of the FXIa inhibitors at home and abroad, wherein BMS-986177, which is a FXIa inhibitor developed by the BMS Company, has entered phase II clinical trial, and is used for preventing and treating major thrombosis and other diseases. Early clinical studies have shown that the FXIa inhibitors can slow down the thrombosis, and can significantly reduce the risk of hemorrhage at the same time. At present, a series of patent applications for the FXIa inhibitors have been published, comprising WO2017151746A1, WO2017151018A1, WO2018039094A1, etc., and the studies and applications of the FXIa inhibitors have made some progress, but there is still a huge room for improvement. Therefore, it is still necessary to continue to study and develop new FXIa inhibitors.

## SUMMRAY

The present invention aims at providing a piperazine derivative of general formula (I) or a stereoisomer, a tautomer, a pharmaceutically acceptable salt or a prodrug thereof:

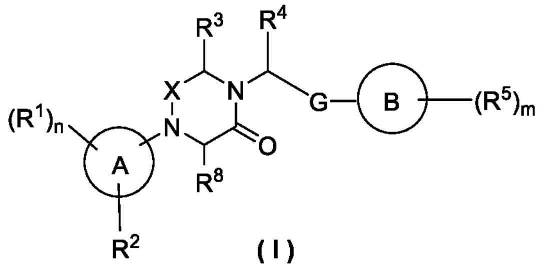

(I)

wherein:

X is selected from C=O or $CR^7$; and preferably, X is selected from C=O;
G is selected from bond or -C(O)-NH-; and preferably, G is selected from -C(O)-NH-;
ring A is selected from aryl or heteroaryl;
ring B is selected from cycloalkyl, heterocyclyl, aryl, heteroaryl or fused ring; wherein, the heteroaryl is preferably a 5- to 10- membered heteroaryl;
$R^1$ are the same or different, and are each independently selected from hydrogen atom, deuterium atom, halogen, alkyl, haloalkyl, nitro, cyano, hydroxyalkyl, cycloalkyl, heterocyclyl, aryl, heteroaryl, $-OR^9$ or $-NR^{10}R^{11}$;
$R^2$ is selected from halogen, cyano, alkoxy, heteroaryl or $-C(O)R^9$; wherein, the alkoxy or heteroaryl is optionally further substituted by one or more substituents selected from alkyl, haloalkyl, nitro, cyano, halogen, cycloalkyl, heterocyclyl, aryl, heteroaryl, $-OR^9$, $-C(O)R^9$, $-C(O)OR^9$, - $NHC(O)R^9$, $-NHC(O)OR^9$, $-NR^{10}R^{11}$ or $-C(O)NR^{10}R^{11}$,
$R^3$ is selected from hydrogen atom, halogen or alkyl; wherein, the alkyl is optionally further substituted by one or more substituents selected from halogen, hydroxyl or alkoxy; and $R^3$ is preferably hydrogen atom;
$R^4$ is selected from hydrogen atom, deuterium atom, alkyl, cycloalkyl, heterocyclyl, aryl, heteroaryl or $-OR^9$, wherein the alkyl, cycloalkyl, heterocyclyl, aryl or heteroaryl is optionally further substituted by one or more substituents selected from halogen, hydroxyl or $R^6$;
$R^5$ are the same or different and are each independently selected from hydrogen atom, alkyl, halogen, nitro, cyano, cycloalkyl, heterocyclyl, aryl, heteroaryl, $-OR^9$, $-C(O)R^9$, $-C(O)OR^9$, - $NHC(O)R^9$, $-NHC(O)OR^9$, $-NR^{10}R^{11}$, $-C(O)NR^{10}R^{11}$, $-CH_2NHC(O)OR^9$, $-CH_2NR^{10}R^{11}$, $-S(O)_rR^9$ or - $S(O)_rNR^{10}R^{11}$, wherein the alkyl, cycloalkyl, heterocyclyl, aryl or heteroaryl is optionally further substituted by one or more substituents selected from deuterium atom, halogen, nitro, cyano, hydroxyl, =O, $-OR^9$, $-C(O)R^9$, $-C(O)OR^9$, $-NHC(O)R^9$, $-NHC(O)OR^9$, $-NR^{10}R^{11}$, $-C(O)NR^{10}R^{11}$, - $CH_2NHC(O)OR^9$, $-CH_2NR^{10}R^{11}$ or $-S(O)_rR^9$;
alternatively, two $R^5$ together with the same carbon atom bound therewith form C=O;
$R^6$ is selected from deuterium atom, halogen, alkyl, alkoxy, amino, nitro, cyano, hydroxyl, cycloalkyl, heterocyclyl, aryl or heteroaryl, wherein the alkyl, alkoxy, cycloalkyl, heterocyclyl, aryl or heteroaryl is optionally further substituted by one or more $R^A$ substituents;
$R^A$ is selected from deuterium atom, halogen, alkyl, hydroxyl, alkoxy, amino, nitro, cyano, cycloalkyl, heterocyclyl, aryl, heteroaryl or $-NR^{13}C(O)R^{14}$, wherein the alkyl, alkoxy, cycloalkyl, heterocyclyl, aryl or heteroaryl is optionally further substituted by one or more substituents selected from deuterium atom, halogen, hydroxyl, alkyl, haloalkyl, alkoxy, haloalkoxy or cyano;
$R^7$ is selected from hydrogen atom, halogen or $-OR^B$, and is preferably hydrogen atom;
$R^B$ is selected from alkyl, cycloalkyl, heterocyclyl, aryl or heteroaryl, wherein the alkyl, cycloalkyl, heterocyclyl, aryl or heteroaryl is optionally further substituted by one or more substituents selected from deuterium atom, halogen, alkyl, haloalkyl, hydroxyl, alkoxy or haloalkoxy;
$R^8$ is selected from hydrogen atom, halogen or alkyl; wherein the alkyl is optionally further substituted by one or more substituents selected from halogen, hydroxyl or alkoxy; and $R^8$ is preferably hydrogen atom;
$R^9$ is selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxyl, halogen, nitro, cyano, alkyl, alkoxy, haloalkyl, haloalkoxy, cycloalkyl, heterocyclyl, aryl, heteroaryl, =O, $-C(O)R^{12}$, -C(O)OR12, $-OC(O)R^{12}$, $-OC(O)OR^{12}$, - $NR^{13}R^{14}$, $-C(O)NR^{13}R^{14}$, $-SO_2NR^{13}R^{14}$ or $-NR^{13}C(O)R^{14}$;
$R^{10}$ and $R^{11}$ are each independently selected from hydrogen atom, hydroxyl, halogen, alkyl, alkoxy, cycloalkyl, heterocyclyl, aryl or heteroaryl, wherein the alkyl, alkoxy, cycloalkyl, heterocyclyl, aryl or heteroaryl is optionally

further substituted by one or more substituents selected from hydroxyl, halogen, nitro, cyano, alkyl, alkoxy, cycloalkyl, heterocyclyl, aryl, heteroaryl, =O, -C(O)$R^{12}$, - C(O)O$R^{12}$, -OC(O)$R^{12}$, -N$R^{13}R^{14}$, -C(O)N$R^{13}R^{14}$, -$SO_2$N$R^{13}R^{14}$ or -N$R^{13}$C(O)$R^{14}$;

alternatively, $R^{10}$ and $R^{11}$ together with the connected N atom form one 4- to 8- membered heterocyclyl, wherein the 4- to 8- membered heterocycle internally contains one or more N, O or $S(O)_n$, and the 4- to 8- membered heterocycle is optionally further substituted by one or more substituents selected from hydroxyl, halogen, nitro, cyano, alkyl, alkoxy, cycloalkyl, heterocyclyl, aryl, heteroaryl, =O, -C(O)$R^{12}$, -C(O)O$R^{12}$, -OC(O)$R^{12}$, -N$R^{13}R^{14}$, -C(O)N$R^{13}R^{14}$, -$SO_2$N$R^{13}R^{14}$ or -N$R^{13}$C(O)$R^{14}$;

$R^{12}$, $R^{13}$ and $R^{14}$ are each independently selected from hydrogen atom, alkyl, amino, cycloalkyl, heterocyclyl, aryl or heteroaryl, wherein the alkyl, cycloalkyl, heterocyclyl, aryl or heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or carboxylate;

m is 0, 1, 2, 3, or 4;

n is 0, 1, 2, 3, or 4; and

r is 0, 1 or 2.

The present invention provides a compound represented by general formula (I) or a stereoisomer, a tautomer, a pharmaceutically acceptable salt or a prodrug thereof, which is a compound represented by general formula (II) or a stereoisomer, a tautomer, a pharmaceutically acceptable salt or a prodrug thereof,

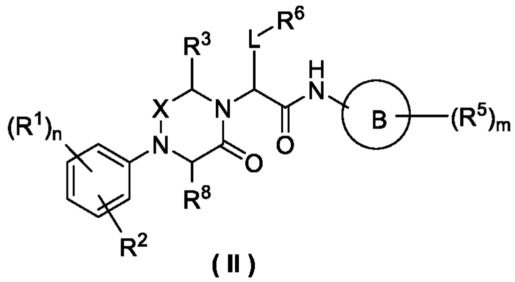

( II )

wherein:

L is selected from bond or alkylene, wherein the alkylene is optionally further substituted by one or more substituents selected from deuterium atom, halogen or hydroxyl; and

ring B, X, $R^1$ to $R^3$, $R^5$, $R^6$, $R^8$, m and n are as defined in general formula (I).

The present invention provides a compound represented by general formula (I) or a stereoisomer, a tautomer, a pharmaceutically acceptable salt or a prodrug thereof, which is a compound represented by general formula (III) or a stereoisomer, a tautomer, a pharmaceutically acceptable salt or a prodrug thereof,

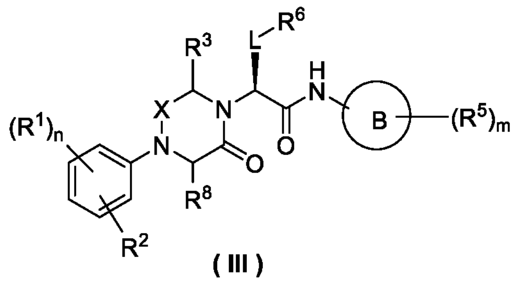

( III )

wherein: ring B, X, L, $R^1$ to $R^3$, $R^5$, $R^6$, $R^8$, m and n are as defined in general formula (II).

The present invention provides a compound represented by general formula (I) or a stereoisomer, a tautomer, a pharmaceutically acceptable salt or a prodrug thereof, which is a compound represented by general formula (IV) or a stereoisomer, a tautomer, a pharmaceutically acceptable salt or a prodrug thereof,

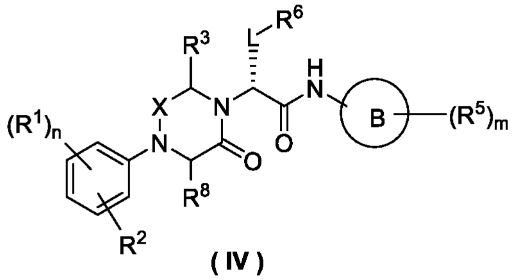

**( IV )**

wherein: ring B, X, L, $R^1$ to $R^3$, $R^5$, $R^6$, $R^8$, m and n are as defined in general formula (II).

In a preferred solution of the present invention, in the compound represented by general formula (I), (II), (III) or (IV) or the stereoisomer, the tautomer, the pharmaceutically acceptable salt or the prodrug thereof, ring B is selected from 3- to 6- membered cycloalkyl, 4- to 8- membered heterocyclyl, phenyl, 5- to 10- membered heteroaryl or 8- to 10-membered fused ring; wherein, the 3- to 6-membered cycloalkyl is preferably cyclohexyl; and the 4- to 8- membered heterocyclyl is preferably tetrahydropyranyl or piperidinyl.

In a preferred embodiment of the present invention, in the compound represented by general formula (I), (II), (III) or (IV) or the stereoisomer, the tautomer, the pharmaceutically acceptable salt or the prodrug thereof,

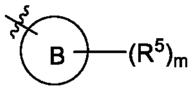

is selected from:

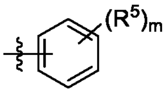 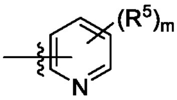 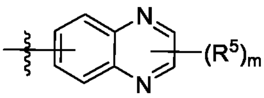 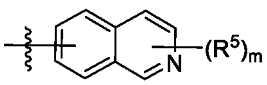 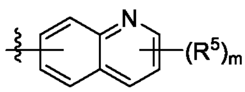

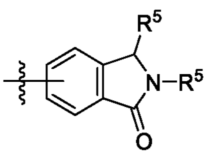 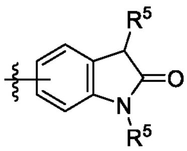 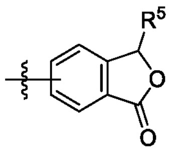 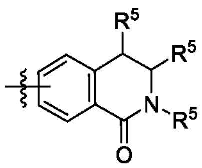 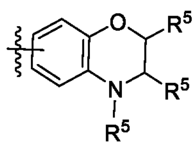

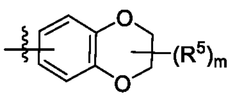 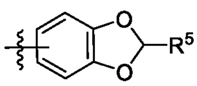 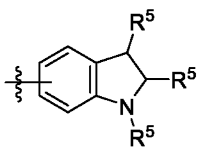 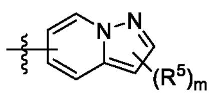 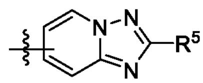

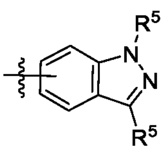 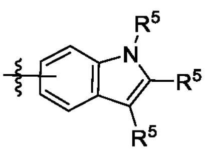 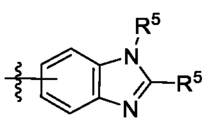 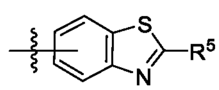 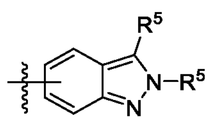

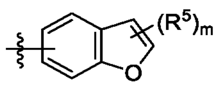 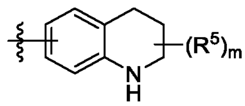 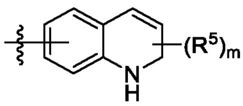 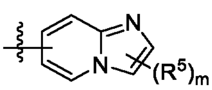 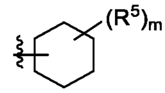

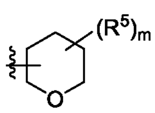 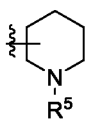 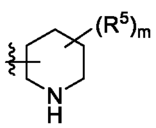 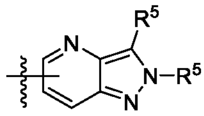 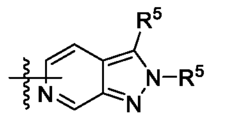 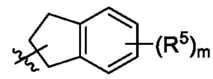

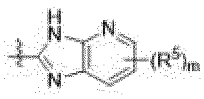 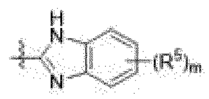 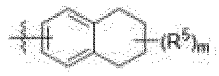 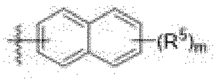 or 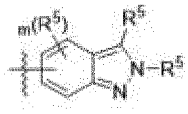

wherein: $R^5$ and m are as defined in general formula (I).

In a preferred embodiment of the present invention, in the compound represented by general formula (I), (II), (III) or (IV) or the stereoisomer, the tautomer, the pharmaceutically acceptable salt or the prodrug thereof,

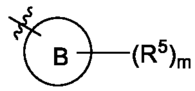

is selected from:

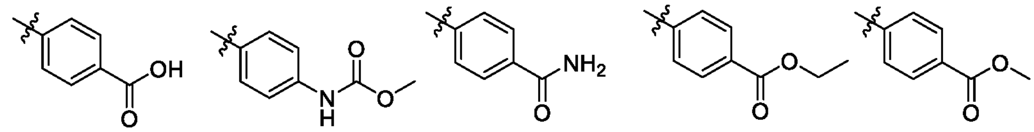

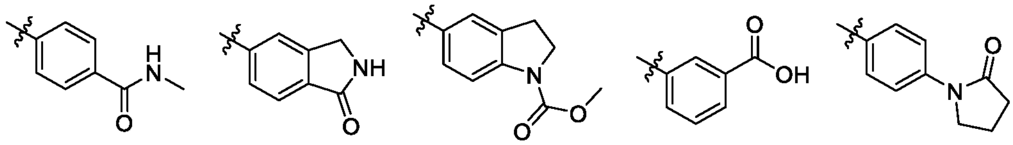

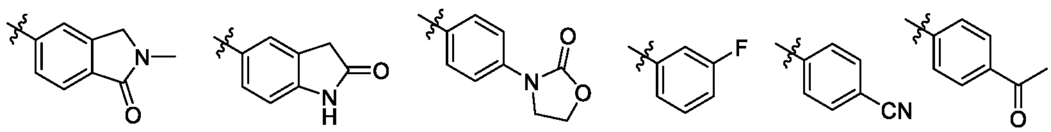

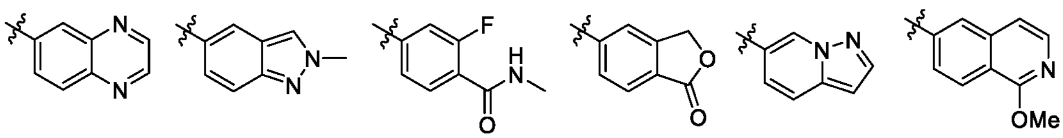

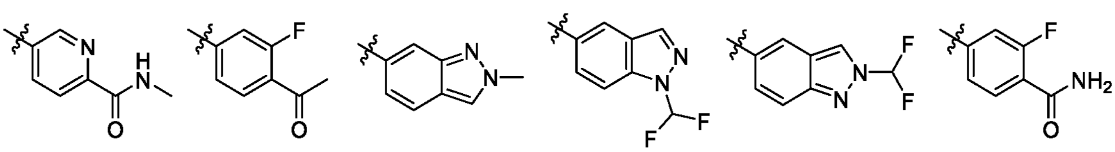

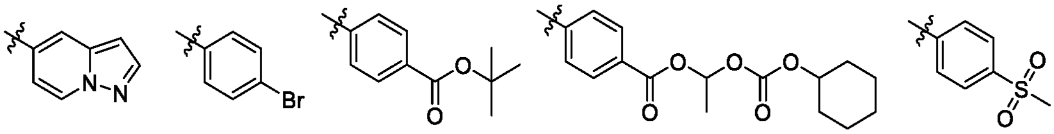

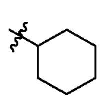 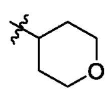 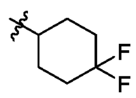 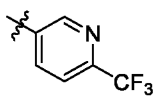 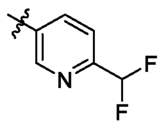 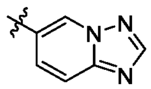 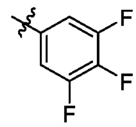

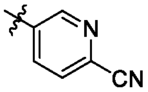 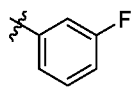 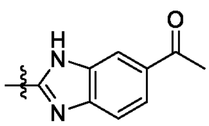 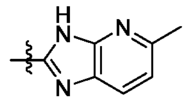 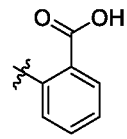 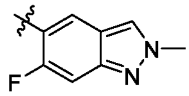

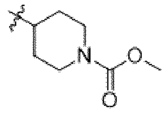 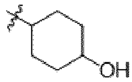 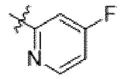 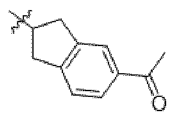 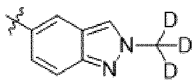 or 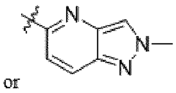 .

In a preferred embodiment of the present invention, the compound represented by general formula (II) or the stereoisomer, the tautomer, the pharmaceutically acceptable salt or the prodrug thereof is a compound represented by general formula (V) or a stereoisomer, a tautomer, a pharmaceutically acceptable salt or a prodrug thereof,

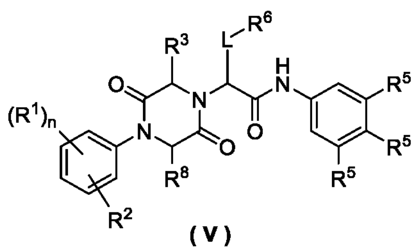

**( V )**

wherein: L, $R^1$ to $R^3$, $R^5$, $R^6$, $R^8$ and n are as defined in general formula (II).

In a preferred embodiment of the present invention, the compound represented by general formula (V) or the stereoisomer, the tautomer, the pharmaceutically acceptable salt or the prodrug thereof is a compound represented by general formula (V-A) and (V-B) or a stereoisomer, a tautomer, a pharmaceutically acceptable salt or a prodrug thereof,

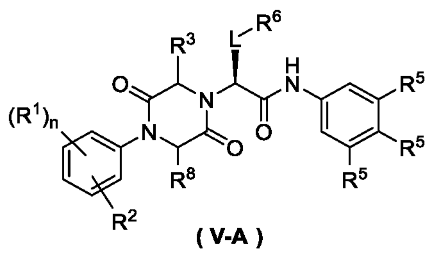

**( V-A )**

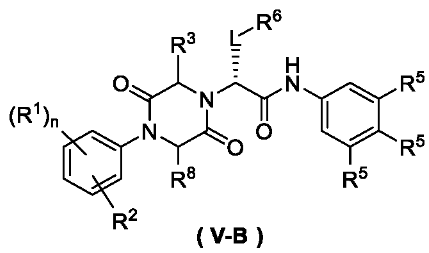

**( V-B )**

wherein: L, $R^1$ to $R^3$, $R^5$, $R^6$, $R^8$ and n are as defined in general formula (V).

In a preferred embodiment of the present invention, the compound represented by general formula (II) or the stereoisomer, the tautomer, the pharmaceutically acceptable salt or the prodrug thereof is a compound represented by general formula (VI) or a stereoisomer, a tautomer, a pharmaceutically acceptable salt or a prodrug thereof,

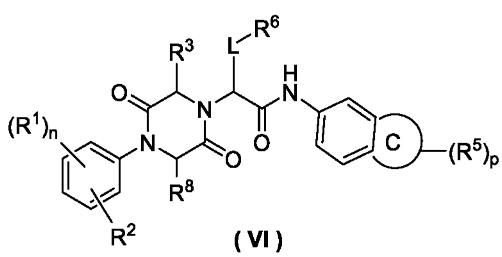

**( VI )**

wherein:

ring C is selected from 5- to 6- membered heteroaryl, 5- to 6- membered aryl, 4- to 8- membered heterocyclyl or 4- to 8- membered cycloalkyl;
p is 0, 1 or 2; and
L, $R^1$ to $R^3$, $R^5$, $R^6$, $R^8$ and n are as defined in general formula (II).

In a preferred embodiment of the present invention, the compound represented by general formula (VI) or the stereoisomer, the tautomer, the pharmaceutically acceptable salt or the prodrug thereof is a compound represented by general formula (VI-A) and (VI-B) or a stereoisomer, a tautomer, a pharmaceutically acceptable salt or a prodrug thereof,

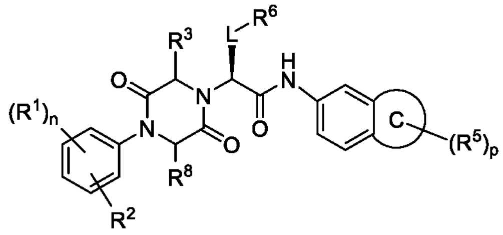
( VI-A )

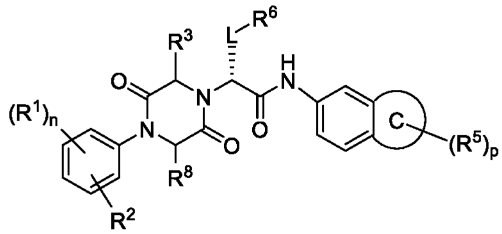
( VI-B )

wherein: ring C, L, $R^1$ to $R^3$, $R^5$, $R^6$, $R^8$, p and n are as defined in general formula (VI).

In a preferred embodiment of the present invention, the compound represented by general formula (I), (II), (III), (IV), (V), (V-A), (V-B), (VI), (VI-A) or (VI-B) or the stereoisomer, the tautomer, the pharmaceutically acceptable salt or the prodrug thereof, $R^2$ is selected from 5- membered heteroaryl; wherein the 5- membered heteroaryl is optionally further substituted by one or more substituents selected from alkyl, haloalkyl, cyano or halogen; preferably, $R^2$ is selected from triazolyl or tetrazolyl; wherein the triazolyl is optionally further substituted by halogen; wherein the halogen is preferably Cl.

In a preferred embodiment of the present invention, in the compound represented by general formula (I), (II), (III), (IV), (V), (V-A), (V-B), (VI), (VI-A) or (VI-B) or the stereoisomer, the tautomer, the pharmaceutically acceptable salt or the prodrug thereof,

L is $-(CR^aR^b)s-$, wherein s is 1, 2, 3 or 4;
$R^a$ and $R^b$ are each independently selected from hydrogen atom, deuterium atom or alkyl;
$R^6$ is selected from alkyl, alkoxy, 3- to 8- membered cycloalkyl, 4- to 8- membered heterocyclyl, phenyl or 5- to 10- membered heteroaryl, wherein the alkyl, alkoxy, 3-8 membered cycloalkyl, 4- to 8- membered heterocyclyl, phenyl or 5- to 10- membered heteroaryl is optionally further substituted by one or more $R^A$ substituents; and
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano.

In a preferred embodiment of the present invention, in the compound represented by general formula (I), (II), (III), (IV), (V), (V-A), (V-B), (VI), (VI-A) or (VI-B) or the stereoisomer, the tautomer, the pharmaceutically acceptable salt or the prodrug thereof:

L is selected from $-CH_2-$, $-CD_2-$ or $-CH_2CH_2-$;
$R^6$ is selected from alkyl, alkoxy, 3- to 8- membered cycloalkyl, 4- to 8- membered heterocyclyl, phenyl or 5- to 10- membered heteroaryl, wherein the alkyl, alkoxy, 3- to 8- membered cycloalkyl, 4-to 8- membered heterocyclyl, phenyl or 5- to 10- membered heteroaryl is optionally further substituted by one or more $R^A$ substituents; and
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano.

In a preferred embodiment of the present invention, in the compound represented by general formula (I), (II), (III), (IV), (V), (V-A), (V-B), (VI), (VI-A) or (VI-B) or the stereoisomer, the tautomer, the pharmaceutically acceptable salt or the prodrug thereof:

$R^6$ is selected from alkyl, alkoxy, phenyl, pyridinyl, pyrazolyl, imidazolyl, 3- to 6- membered cycloalkyl or 4- to 6-

membered heterocyclyl, wherein the alkyl, alkoxy, phenyl, pyridinyl, pyrazolyl, imidazolyl, 3- to 6- membered cycloalkyl or 4- to 6- membered heterocyclyl is optionally further substituted by one or more $R^A$ substituents; and
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano.

In a preferred embodiment of the present invention, in the compound represented by general formula (I), (II), (III), (IV), (V), (V-A), (V-B), (VI), (VI-A) or (VI-B) or the stereoisomer, the tautomer, the pharmaceutically acceptable salt or the prodrug thereof:

$R^5$ are the same or different, and are each independently selected from hydrogen atom, alkyl, haloalkyl, halogen, cyano, heterocyclyl, $-OR^9$, $-C(O)R^9$, $-C(O)OR^9$, $-NHC(O)R^9$, $-NHC(O)OR^9$, - $NR^{10}R^{11}$, $-C(O)NR^{10}R^{11}$, $-CH_2NHC(O)OR^9$, $-CH_2NR^{10}R^{11}$, $-S(O)_rR^9$ or $-S(O)_rNR^{10}R^{11}$;
alternatively, two $R^5$ together with the same carbon atom bound therewith form C=O;
$R^9$ is selected from hydrogen atom or $C_1$-$C_4$ alkyl, wherein the $C_1$-$C_4$ alkyl is optionally further substituted by one or more substituents selected from hydroxyl, halogen, nitro, cyano or $-OC(O)OR^{12}$; and
$R^{10}$ and $R^{11}$ are each independently selected from hydrogen atom, $C_1$-$C_4$ alkyl or 5- to 6- membered heterocyclyl, wherein the $C_1$-$C_4$ alkyl or 5- to 6- membered heterocyclyl is optionally further substituted by one or more substituents selected from halogen, hydroxyl, $C_1$-$C_4$ alkoxy, nitro, cyano or =O.

In a preferred embodiment of the present invention, the compound represented by formula (I), or the stereoisomer, the tautomer, the pharmaceutically acceptable salt or the prodrug thereof is a compound represented by general formula (VII) or a stereoisomer, a tautomer, a pharmaceutically acceptable salt or a prodrug thereof,

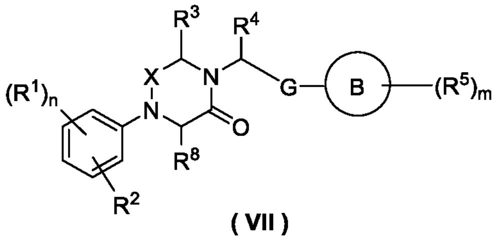

( VII )

wherein:

X is selected from C=O or $CR^7$;
G is selected from bond or -C(O)-NH-;
ring B is selected from 3- to 6- membered cycloalkyl, 4- to 8- membered heterocyclyl, phenyl, 5-to 10- membered heteroaryl or 8- to 10- membered fused ring;
$R^1$ are the same or different, and are each independently selected from hydrogen atom, deuterium atom, halogen, alkyl or haloalkyl;
$R^2$ is selected from halogen, cyano, alkoxy, heteroaryl or $-C(O)R^9$; wherein the alkoxy or heteroaryl is optionally further substituted by one or more substituents selected from haloalkyl or halogen;
$R^3$ is selected from hydrogen atom;
$R^4$ is selected from hydrogen atom, deuterium atom or alkyl, wherein the alkyl is optionally further substituted by one or more substituents selected from halogen, hydroxyl or $R^6$;
$R^5$ are the same or different, and are each independently selected from hydrogen atom, alkyl, halogen, nitro, cyano, cycloalkyl, heterocyclyl, haloalkyl, aryl, heteroaryl, $-OR^9$, $-C(O)R^9$, $-C(O)OR^9$, $-NHC(O)R^9$, $-NHC(O)OR^9$, $-NR^{10}R^{11}$, $-C(O)NR^{10}R^{11}$, $-CH_2NHC(O)OR^9$, $-CH_2NR^{10}R^{11}$, $-S(O)_2R^9$ or $-S(O)_2NR^{10}R^{11}$, wherein the alkyl, cycloalkyl, heterocyclyl, aryl or heteroaryl is optionally further substituted by one or more substituents selected from deuterium atom, halogen or =O;
alternatively, two $R^5$ together with the same carbon atom bound therewith form C=O;
$R^6$ is selected from alkyl, alkoxy, phenyl, pyridinyl, pyrazolyl, imidazolyl, 3- to 6- membered cycloalkyl or 4- to 6- membered heterocyclyl, wherein the alkyl, alkoxy, phenyl, pyridinyl, pyrazolyl, imidazolyl, 3- to 6- membered cycloalkyl or 4- to 6- membered heterocyclyl is optionally further substituted by one or more $R^A$ substituents;
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano;

$R^7$ is selected from hydrogen atom;
$R^8$ is selected from hydrogen atom or $C_1$-$C_4$ alkyl;
$R^9$ is selected from hydrogen atom or $C_1$-$C_4$ alkyl, wherein the $C_1$-$C_4$ alkyl is optionally further substituted by one or more substituents selected from hydroxyl, halogen, nitro, cyano or -OC(O)OR$^{12}$;
$R^{10}$ and $R^{11}$ are each independently selected from hydrogen atom, $C_1$-$C_4$ alkyl or 5- to 6- membered heterocyclyl, wherein the $C_1$-$C_4$ alkyl or 5- to 6- membered heterocyclyl is optionally further substituted by one or more substituents selected from halogen, hydroxyl, $C_1$-$C_4$ alkoxy, nitro, cyano or =O;
$R^{12}$, $R^{13}$ and $R^{14}$ are each independently selected from hydrogen atom, alkyl or cycloalkyl;
m is 0, 1 or 2; and
n is 0, 1 or 2.

Typical compounds of the present invention comprise, but are not limited to:

| No. of compound | Structure | Name |
|---|---|---|
| 1 | 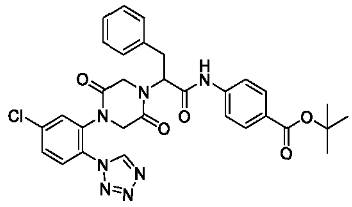 | Tert-butyl 4-(2-(4-(5-chloro-2-(1*H*-tetrazol-1-yl)phenyl)-2,5-dioxopiperazin-1-yl)-3-phenylpropanamido) benzoate |
| 2 | 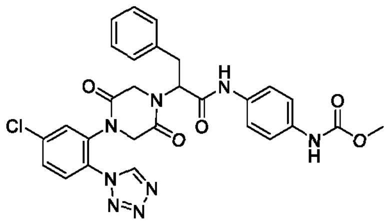 | Methyl (4-(2-(4-(5-chloro-2-(1*H*-tetrazol-1-yl)phenyl)-2,5-dioxopiperazin-1-yl)-3-phenylpropanamido)phenyl) carbamate |
| 3 | 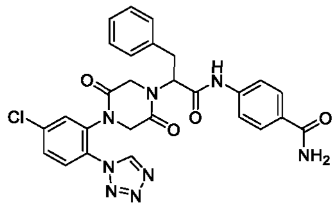 | 4-(2-(4-(5-chloro-2-(1*H*-tetrazol-1-yl)phenyl)-2,5-dioxopiperazin-1-yl)-3-phenylpropanami do)benzamide |
| 4 | 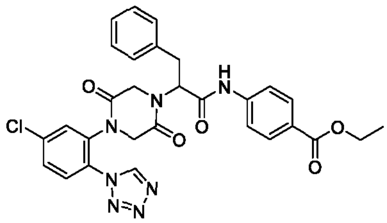 | Ethyl 4-(2-(4-(5-chloro-2-(1*H*-tetrazol-1-yl)phenyl)-2,5-dioxopiperazin-1-yl)-3-phenylpropanamido)benzoate |
| 5 | 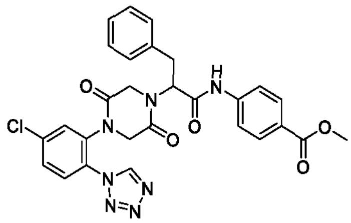 | Methyl 4-(2-(4-(5-chloro-2-(1*H*-tetrazol-1-yl)phenyl)-2,5-dioxopiperazin-1-yl)-3-phenylpropanamido)benzoate |

(continued)

| No. of compound | Structure | Name |
|---|---|---|
| 6 | 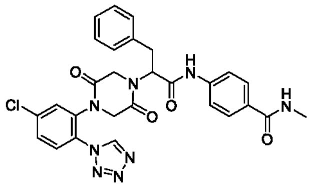 | 4-(2-(4-(5-chloro-2-(1*H*-tetrazol-1-yl)phenyl)-2,5-dioxopiperazin-1-yl)-3-phenylpropanami do)-N-methylbenzamide |
| 7 | 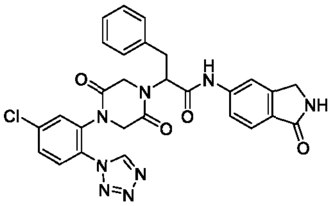 | 2-(4-(5-chloro-2-(1*H*-tetrazol-1-yl)phenyl)-2,5-dioxopiperazin-1-yl)-*N*-(1-oxoisoindolin-5-yl)-3-phenylpropanamide |
| 8 | 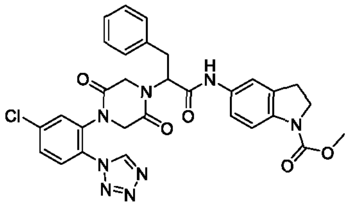 | Methyl 5-(2-(4-(5-chloro-2-(1*H*-tetrazol-1-yl)phenyl)-2,5-dioxopiperazin-1-yl)-3-phenylpropanamido)indoline-1-carboxylate |
| 9 | 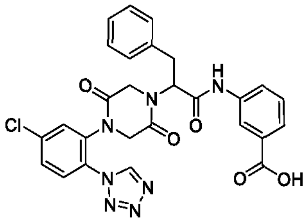 | 3-(2-(4-(5-chloro-2-(1*H*-tetrazol-1-yl)phenyl)-2,5-dioxopiperazin-1-yl)-3-phenylpropanamido)benzoic acid |
| 10 | 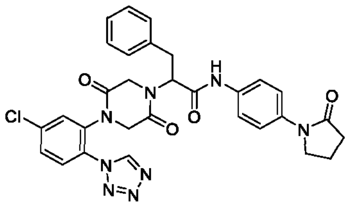 | 2-(4-(5-chloro-2-(1*H*-tetrazol-1-yl)phenyl)-2,5-dioxopiperazin-1-yl)-*N*-(4-(2-oxopyrrolidin-1 -yl)phenyl)-3 - phenylpropanamide |
| 11 | 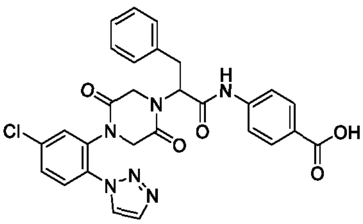 | 4-(2-(4-(5-chloro-2-(1*H*-1,2,3-triazol-1-yl)phenyl)-2,5-dioxopiperazin-1-yl)-3-phenylpropanamido)benzoic acid |
| 12 | 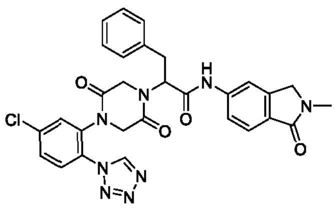 | 2-(4-(5-chloro-2-(1*H*-tetrazol-1-yl)phenyl)-2,5-dioxopiperazin-1-yl)-*N*-(2-methyl-1-oxoisoindolin-5-yl)-3-phenylpropanamide |

(continued)

| No. of compound | Structure | Name |
|---|---|---|
| 13 | 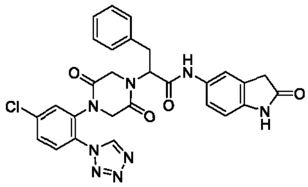 | 2-(4-(5-chloro-2-(1*H*-tetrazol-1-yl)phenyl)-2,5-dioxopiperazin-1-yl)-*N*-(2-oxoindolin-5-yl)-3-phenylpropanamide |
| 14 | 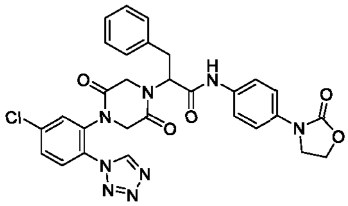 | 2-(4-(5-chloro-2-(1*H*-tetrazol-1-yl)phenyl)-2,5-dioxopiperazin-1-yl)-*N*-(4-(2-oxooxazolidin-3-yl) phenyl)-3-phenylpropanamide |
| 15 | 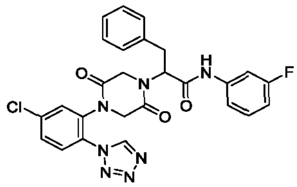 | 2-(4-(5-chloro-2-(1*H*-tetrazol-1-yl)phenyl)-2,5-dioxopiperazin-1-yl)-*N*-(3-fluorophenyl)-3 -phenylpropanamide |
| 16 | 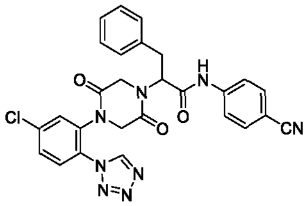 | 2-(4-(5-chloro-2-(1*H*-tetrazol-1-yl)phenyl)-2,5-dioxopiperazin-1-yl)-*N*-(4-cyanophenyl)-3 -phenylpropanamide |
| 17 | 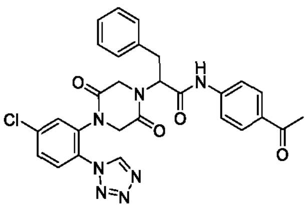 | *N*-(4-acetylphenyl)-2-(4-(5-chloro-2-(1*H*-tetrazol-1-yl) phenyl)-2,5-dioxopiperazin-1-yl)-3-phenylpropanamide |
| 18 | 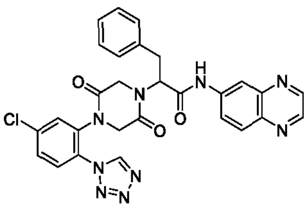 | 2-(4-(5-chloro-2-(1*H*-tetrazol-1-yl)phenyl)-2,5-dioxopiperazin-1-yl)-3-phenyl-*N*-(quinoxalin-6-yl) propanamide |
| 19 | 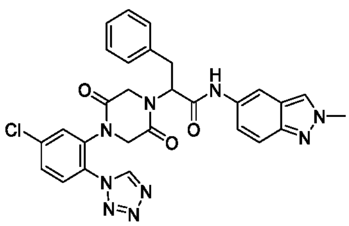 | 2-(4-(5-chloro-2-(1*H*-tetrazol-1-yl)phenyl)-2,5-dioxopiperazin-1-yl)-*N*-(2-methyl-2H indazol-5-yl)-3-phenylpropanamide |

(continued)

| No. of compound | Structure | Name |
|---|---|---|
| 20 | 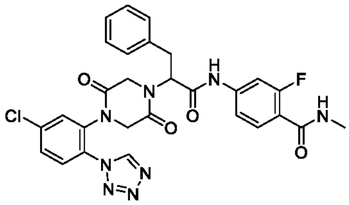 | 4-(2-(4-(5-chloro-2-(1*H*-tetrazol-1-yl)phenyl)-2,5-dioxopiperazin-1-yl)-3 - phenylpropanamido)-2-fluoro-*N*-methylbenzamide |
| 21 | 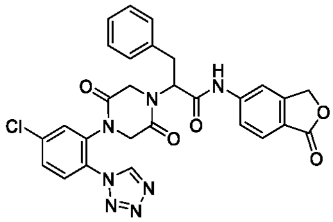 | 2-(4-(5-chloro-2-(1*H*-tetrazol-1-yl)phenyl)-2,5-dioxopiperazin-1-yl)-*N*-(1-oxo-1,3-dihydroisobenzofuran-5-yl)-3-phenylpropanamide |
| 22 | 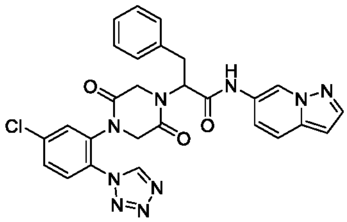 | 2-(4-(5-chloro-2-(1*H*-tetrazol-1-yl)phenyl)-2,5-dioxopiperazin-1-yl)-3-phenyl-*N*-(pyrazolo[1,5-a] pyridin-6-yl)propanamide |
| 23 | 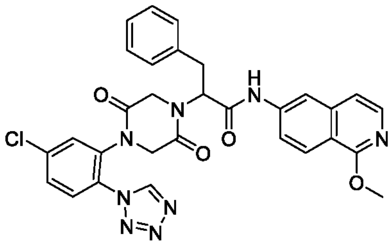 | 2-(4-(5-chloro-2-(1*H*-tetrazol-1-yl)phenyl)-2,5-dioxopiperazin-1-yl)-*N*-(1-methoxyisoquinolin-6-yl)-3-phenylpropanamide |
| 24 | 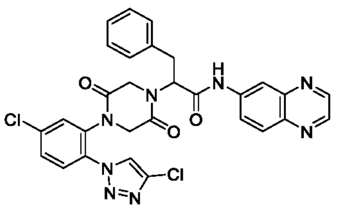 | 2-(4-(5-chloro-2-(4-chloro-1*H*-1,2,3-triazol-1-yl)phenyl)-2,5-dioxopiperazin-1-yl)-3-phenyl-*N*-(quinoxalin-6-yl) propanamide |
| 25 | 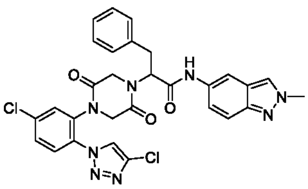 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-phenylpropanamide |
| 26 | 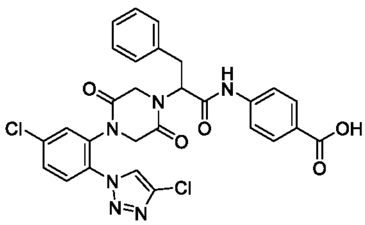 | 4-(2-(4-(5-chloro-2-(4-chloro-1*H*-1,2,3-triazol-1-yl) phenyl)-2,5-dioxopiperazin - 1-yl)-3-phenylpropanamido)benzoic acid |

(continued)

| No. of compound | Structure | Name |
|---|---|---|
| 27 | 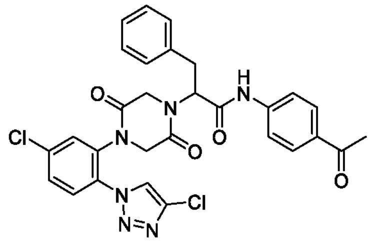 | *N*-(4-acetylphenyl)-2-(4-(5-chloro-2-(4-chloro-1*H*-1,2,3-triazol-1-yl)phenyl)-2,5-dioxopiperazin-1-yl)-3-phenylpropanamide |
| 28 | 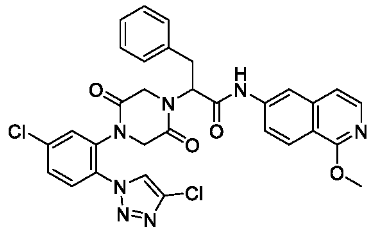 | 2-(4-(5-chloro-2-(4-chloro-1*H*-1,2,3-triazol-1-yl)phenyl)-2,5-dioxopiperazin-1-yl)-*N*-(1-methoxyisoquinolin-6-yl)-3 - phenylpropanamide |
| 29 | 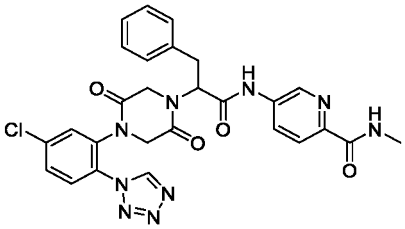 | 5-(2-(4-(5-chloro-2-(1*H*-tetrazol-1-yl)phenyl)-2,5-dioxopiperazin-1-yl)-3-phenylpropanamido)-*N*-methylpicolinamide |
| 30 | 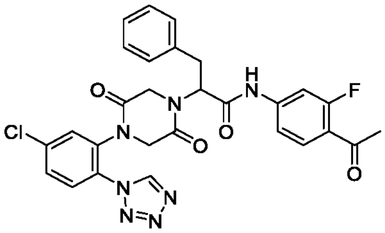 | *N*-(4-acetyl-3-fluorophenyl)-2-(4-(5-chloro-2-(1*H*-tetrazol-1-yl)phenyl)-2,5-dioxopiperazin-1-yl)-3-phenylpropanamide |
| 31 | 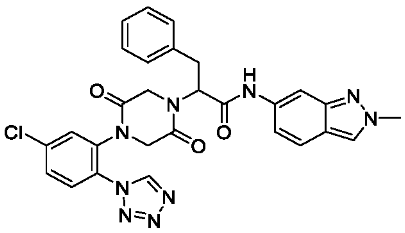 | 2-(4-(5-chloro-2-(1*H*-tetrazol-1-yl)phenyl)-2,5-dioxopiperazin-1-yl)-*N*-(2-methyl-2*H*-indazol-6-yl)-3-phenylpropanamide |
| 32 | 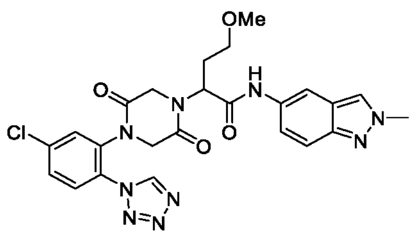 | 2-(4-(5-chloro-2-(1*H*-tetrazol-1-yl)phenyl)-2,5-dioxopiperazin-1-yl)-4-methoxy-*N*-(2-methyl-2*H*-indazol-5-yl)butanamide |

(continued)

| No. of compound | Structure | Name |
|---|---|---|
| 33 | 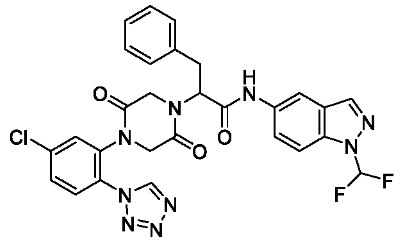 | 2-(4-(5-chloro-2-(1*H*-tetrazol-1-yl)phenyl)-2,5-dioxopiperazin-1-yl)-*N*-(1-(difluoromethyl)-1*H*-indazol-5-yl)-3-phenylpropanamide |
| 34 | 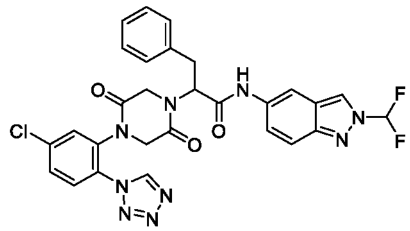 | 2-(4-(5-chloro-2-(1*H*-tetrazol-1-yl)phenyl)-2,5-dioxopiperazin-1-yl)-*N*-(2-(difluoromethyl)-2*H*-indazol-5-yl)-3-phenylpropanamide |
| 35 | 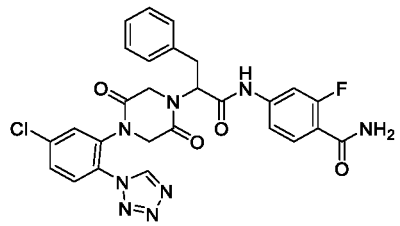 | 4-(2-(4-(5-chloro-2-(1*H*-tetrazol-1-yl)phenyl)-2,5-dioxopiperazin-1-yl)-3-phenylpropanamido)-2-fluorobenzamide |
| 36 | 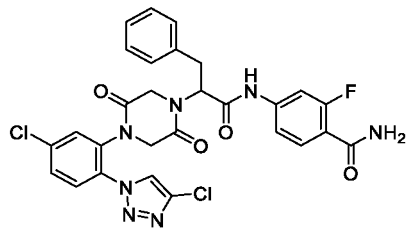 | 4-(2-(4-(5-chloro-2-(4-chloro-1*H*-1,2,3-triazol-1-yl) phenyl)-2,5-dioxopiperazin - 1-yl)-3-phenylpropanamido)-2-fluorobenzamide |
| 37 | 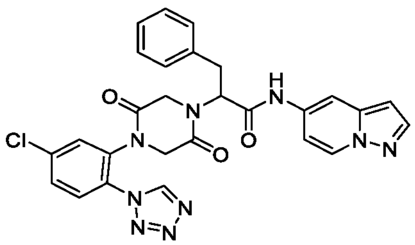 | 2-(4-(5-chloro-2-(1*H*-tetrazol-1-yl)phenyl)-2,5-dioxopiperazin-1-yl)-3-phenyl-*N*-(pyrazolo[1,5-a] pyridin-5-yl)propanamide |
| 38 | 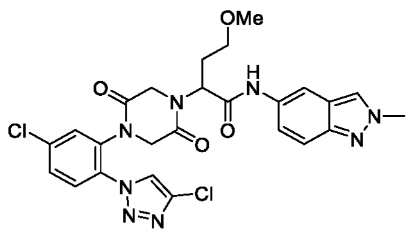 | 2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide |

(continued)

| No. of compound | Structure | Name |
|---|---|---|
| 39 | 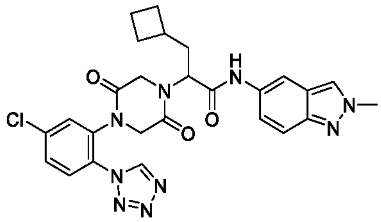 | 2-(4-(5-chloro-2-(1*H*-tetrazol-1-yl)phenyl)-2,5-dioxopiperazin-1-yl)-3-cyclobutyl-*N*-(2-methyl-2*H*-indazol-5-yl)propanamidee |
| 40 | 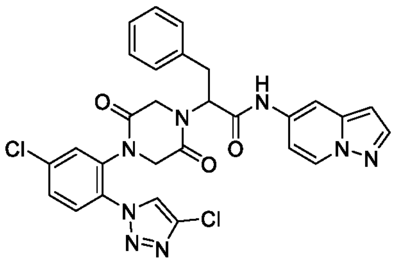 | 2-(4-(5-chloro-2-(4-chloro-1*H*-1,2,3-triazol-1-yl)phenyl)-2,5-dioxopiperazin-1-yl)-3-phenyl-*N*-(pyrazolo[1,5-a] pyridin-5-yl)propanamide |
| 41 | 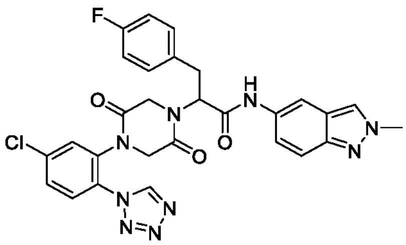 | 2-(4-(5-chloro-2-(1*H*-tetrazol-1-yl)phenyl)-2,5-dioxopiperazin-1-yl)-3-(4-fluorophenyl)-*N*-(2-methyl-2*H*-indazol-5-yl)propanamide |
| 42 | 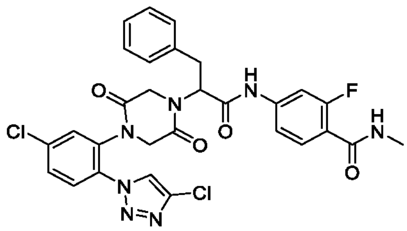 | 4-(2-(4-(5-chloro-2-(4-chloro-1*H*-1,2,3-triazol-1-yl) phenyl)-2,5-dioxopiperazin-1 -yl)-3 -phenylpropanamido)-2-fluoro-*N*-methylbenzamide |
| 43 | 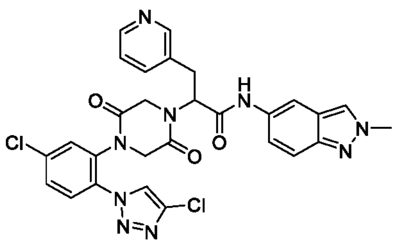 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-3-yl)propanamide |
| 44 | 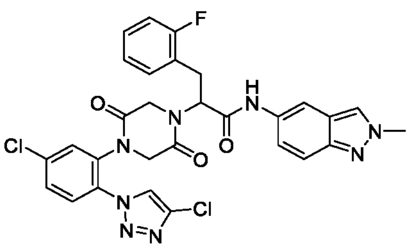 | 2-(4-(5-chloro-2-(4-chloro-1*H*-1,2,3-triazol-1-yl)phenyl)-2,5-dioxopiperazin-1-yl)-3-(2-fluorophenyl)-*N*-(2-methyl-2*H*-indazol-5-yl)propanamide |

(continued)

| No. of compound | Structure | Name |
|---|---|---|
| 45 | 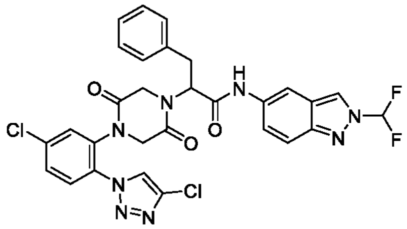 | 2-(4-(5-chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-phenylpropanamide |
| 46 | 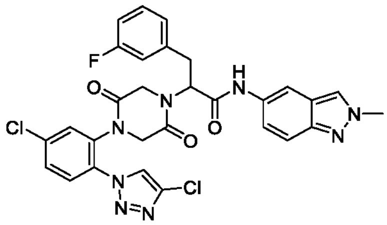 | 2-(4-(5-chloro-2-(4-chloro-1*H*-1,2,3-triazol-1-yl)phenyl)-2,5-dioxopiperazin-1-yl)-3-(3-fluorophenyl)-*N*-(2-methyl-2*H*-indazol-5-yl)propanamide |
| 47 | 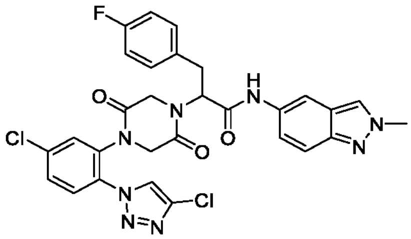 | 2-(4-(5-chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide |
| 48 | 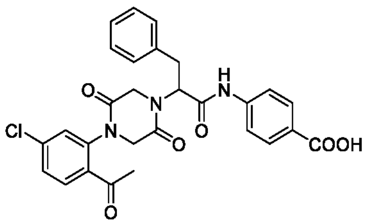 | 4-(2-(4-(2-acetyl-5-chlorophenyl)-2,5-dioxopiperazin-1-yl)-3-phenylpropanamido)benzoic acid |
| 49 | 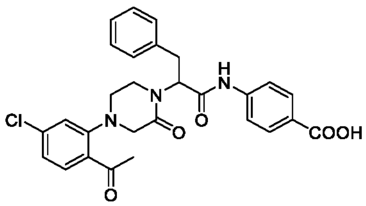 | 4-(2-(4-(2-acetyl-5-chlorophenyl)-2-oxopiperazin-1-yl)-3-phenylpropanamido)benzoic acid |
| 50 | 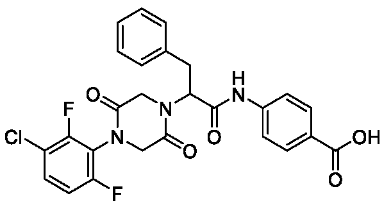 | 4-(2-(4-(3-chloro-2,6-difluorophenyl)-2,5-dioxopiperazin-1-yl)-3-phenylpropanamido)benzoic acid |

(continued)

| No. of compound | Structure | Name |
|---|---|---|
| 51 | 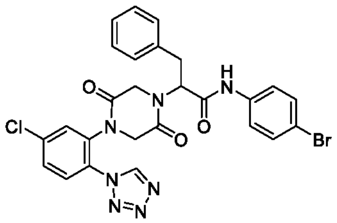 | *N*-(4-bromophenyl)-2-(4-(5-chloro-2-(1*H*-tetrazol-1-yl) phenyl)-2,5-dioxopiperazin-1-yl)-3-phenylpropanamide |
| 52 | 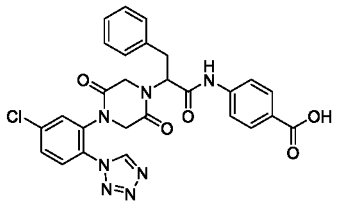 | 4-(2-(4-(5-chloro-2-(1*H*-tetrazol-1-yl)phenyl)-2,5-dioxopiperazin-1-yl)-3-phenylpropanamido)benzoic acid |
| 53 | 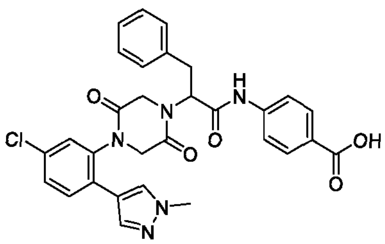 | 4-(2-(4-(5-chloro-2-(1-methyl-1*H*-pyrazol-4-yl)phenyl)-2,5-dioxopiperazin-1-yl)-3-phenylpropanamido)benzoic acid |
| 54 | 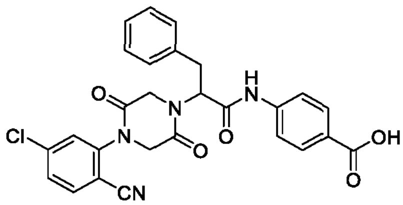 | 4-(2-(4-(5-chloro-2-cyanophenyl)-2,5-dioxopiperazin-1-yl)-3-phenylpropanamido)benzoic acid |
| 55 | 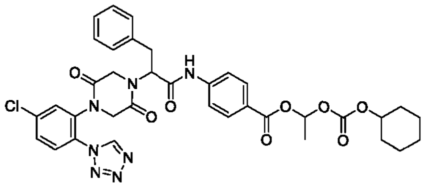 | 1-(((cyclohexyloxy)carbonyl)oxy)ethyl 4-(2-(4-(5-chloro-2-(1*H*-tetrazol-1-yl)phenyl)-2,5-dioxopiperazin-1-yl)-3-phenylpropanamido)benzoate |
| 56 | 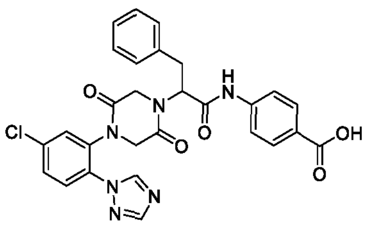 | 4-(2-(4-(5-chloro-2-(1*H*-1,2,4-triazol-1-yl)phenyl)-2,5-dioxopiperazin-1-yl)-3-phenylpropanamido)benzoic acid |
| 57 | 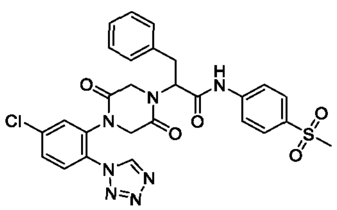 | 2-(4-(5-chloro-2-(1*H*-tetrazol-1-yl)phenyl)-2,5-dioxopiperazin-1-yl)-*N*-(4-(methylsulfonyl)phenyl)-3-phenylpropanamide |

(continued)

| No. of compound | Structure | Name |
|---|---|---|
| 58 | 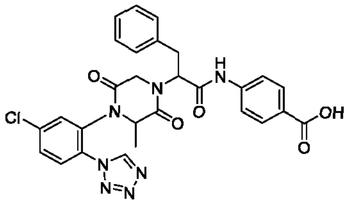 | 4-(2-(4-(5-chloro-2-(1*H*-tetrazol-1-yl)phenyl)-3-methyl-2,5-dioxopiperazin-1-yl)-3-phenylpropanamido)benzoic acid |
| 59 | 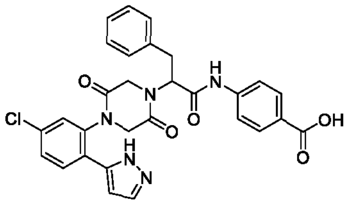 | 4-(2-(4-(5-chloro-2-(1*H*-pyrazol-5-yl)phenyl)-2,5-dioxopiperazin-1-yl)-3-phenylpropanamido)benzoic acid |
| 60 | 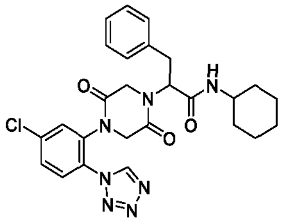 | 2-(4-(5-chloro-2-(1*H*-tetrazol-1-yl)phenyl)-2,5-dioxopiperazin-1-yl)-*N*-cyclohexyl-3-phenylpropanamide |
| 61 | 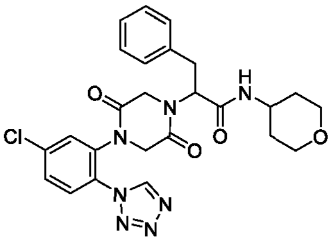 | 2-(4-(5-chloro-2-(1*H*-tetrazol-1-yl)phenyl)-2,5-dioxopiperazin-1-yl)-3-phenyl-*N*-(tetrahydro-2*H*-pyran-4-yl)propanamide |
| 62 | 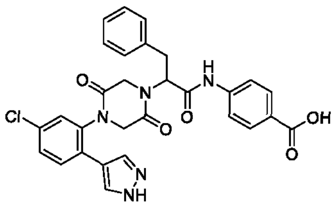 | 4-(2-(4-(5-chloro-2-(1*H*-pyrazol-4-yl)phenyl)-2,5-dioxopiperazin-1-yl)-3-phenylpropanamido)benzoic acid |
| 63 | 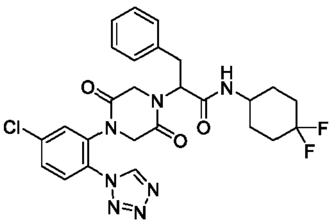 | 2-(4-(5-chloro-2-(1*H*-tetrazol-1-yl)phenyl)-2,5-dioxopiperazin-1-yl)-*N*-(4,4-difluorocyclohexyl)-3-phenylpropanamide |

(continued)

| No. of compound | Structure | Name |
|---|---|---|
| 64 | 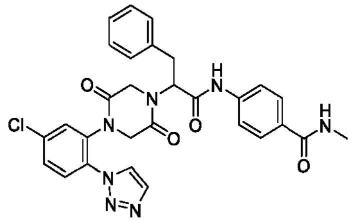 | 4-(2-(4-(5-chloro-2-(1*H*-1,2,3-triazol-1-yl)phenyl)-2,5-dioxopiperazin-1-yl)-3-phenylpropanamido)-*N*-methylbenzamide |
| 65 | 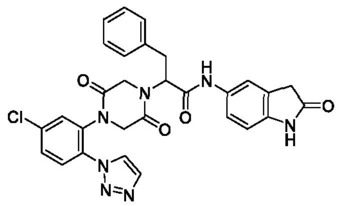 | 2-(4-(5-chloro-2-(1*H*-1,2,3-triazol-1-yl)phenyl)-2,5-dioxopiperazin-1-yl)-*N*-(2-oxoindolin-5-yl)-3-phenylpropanamide |
| 66 | 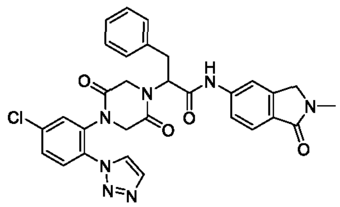 | 2-(4-(5-chloro-2-(1*H*-1,2,3-triazol-1-yl)phenyl)-2,5-dioxopiperazin-1-yl)-*N*-(2-methyl-1-oxoisoindolin-5-yl)-3-phenylpropanamide |
| 67 | 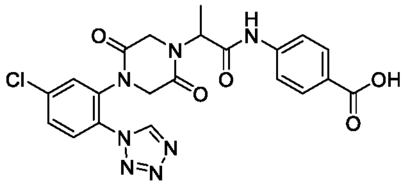 | 4-(2-(4-(5-chloro-2-(1*H*-tetrazol-1-yl)phenyl)-2,5-dioxopiperazin-1-yl)propanamido)benzoic acid |
| 68 | 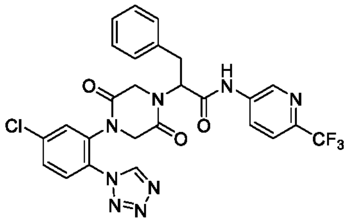 | 2-(4-(5-chloro-2-(1*H*-tetrazol-1-yl)phenyl)-2,5-dioxopiperazin-1-yl)-3-phenyl-*N*-(6-(trifluoromethyl) pyridin-3 - yl)propanamide |
| 69 | 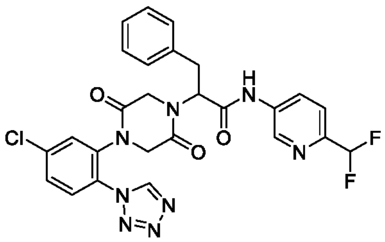 | 2-(4-(5-chloro-2-(1*H*-tetrazol-1-yl)phenyl)-2,5-dioxopiperazin-1-yl)-*N*-(6-(difluoromethyl)pyridin-3-yl)-3-phenylpropanamide |
| 70 | 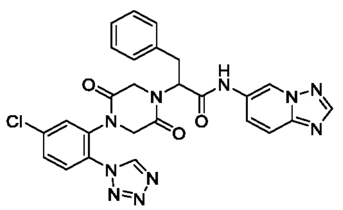 | *N*-([1,2,4]triazolo[1,5-a]pyridin-6-yl)-2-(4-(5-chloro-2-(1*H*-tetrazol-1-yl)phenyl)-2,5-dioxopiperazin-1-yl)-3-phenylpropanamide |

(continued)

| No. of compound | Structure | Name |
|---|---|---|
| 71 | 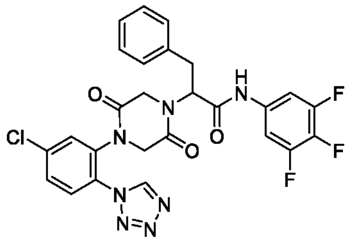 | 2-(4-(5-chloro-2-(1*H*-tetrazol-1-yl)phenyl)-2,5-dioxopiperazin-1-yl)-3-phenyl-*N*-(3,4,5-trifluorophenyl) propanamide |
| 72 | 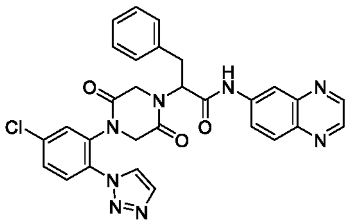 | 2-(4-(5-chloro-2-(1*H*-1,2,3-triazol-1 - yl)phenyl)-2, 5-dioxopiperazin-1-yl)-3 - phenyl-*N*-(quinoxalin-6-yl) propanamide |
| 73 | 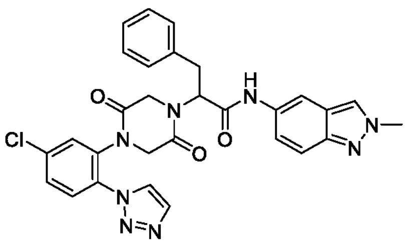 | 2-(4-(5-chloro-2-(1*H*-1,2,3-triazol-1 - yl)phenyl)-2,5-dioxopiperazin-1-y1)-*N*-(2-methyl-2*H*-indazol-5-yl)-3-phenylpropanamide |
| 74 | 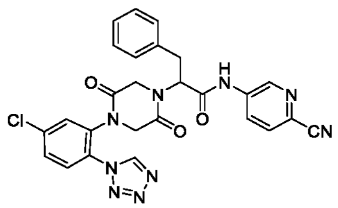 | 2-(4-(5-chloro-2-(1*H*-tetrazol-1-yl)phenyl)-2,5-dioxopiperazin-1-yl)-*N*-(6-cyanopyridin-3-yl)-3-phenylpropanamide |
| 75 | 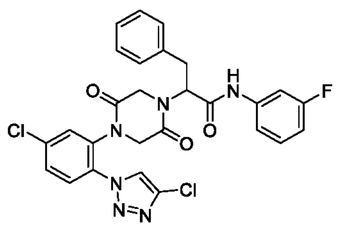 | 2-(4-(5-chloro-2-(4-chloro-1*H*-1,2,3-triazol-1-yl)phenyl)-2,5-dioxopiperazin-1-yl)-*N*-(3-fluorophenyl)-3-phenylpropanamide |
| 76 | 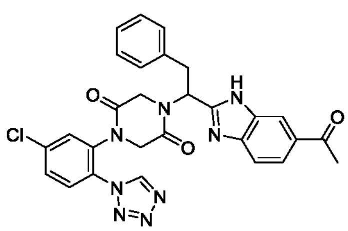 | 1-(1-(6-acetyl-1*H*-benzo[*d*]imidazol-2-yl)-2-phenylethyl)-4-(5-chloro-2-(1*H*-tetrazol-1-yl)phenyl) piperazine-2,5-dione |

(continued)

| No. of compound | Structure | Name |
|---|---|---|
| 77 | 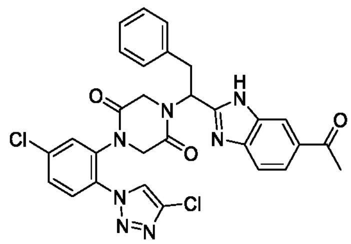 | 1-(1-(6-acetyl-1*H*-benzo[*d*]imidazol-2-yl)-2-phenylethyl)-4-(5-chloro-2-(4-chloro-1*H*-1,2,3-triazol-1-yl)phenyl)piperazine-2,5-dione |
| 78 | 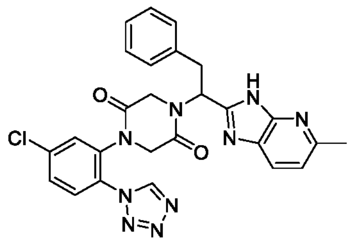 | 1-(5-chloro-2-(1*H*-tetrazol-1-yl)phenyl)-4-(1-(5-methyl-3*H*-imidazo[4,5-b]pyridin-2-yl)-2-phenylethyl) piperazine-2,5-dione |
| 79 | 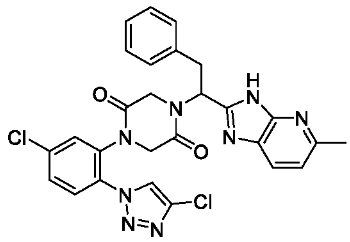 | 1-(5-chloro-2-(4-chloro-1*H*-1,2,3-triazol-1-yl)phenyl)-4-(1-(5-methyl-3*H*-imidazo[4,5-b]pyridin-2-yl)-2-phenylethyl)piperazine-2,5-dione |
| 80 | 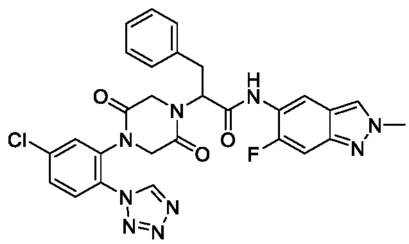 | 2-(4-(5-chloro-2-(1*H*-tetrazol-1-yl)phenyl)-2,5-dioxopiperazin-1-yl)-*N*-(6-fluoro-2-methyl-2*H*-indazol-5-yl)-3-phenylpropanamide |
| 81 | 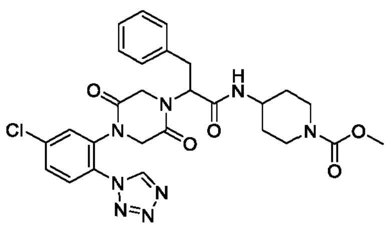 | Methyl 4-(2-(4-(5-chloro-2-(1*H*-tetrazol-1-yl)phenyl)-2,5-dioxopiperazin-1-yl)-3-phenylpropanamido)piperidine-1 - carboxylate |
| 82 | 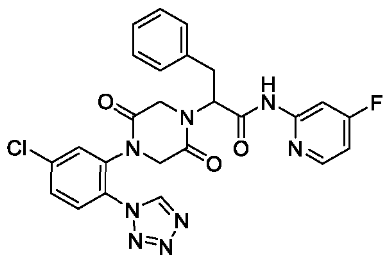 | 2-(4-(5-chloro-2-(1*H*-tetrazol-1-yl)phenyl)-2,5-dioxopiperazin-1-yl)-*N*-(4-fluoropyridin-2-yl)-3-phenylpropanamide |

(continued)

| No. of compound | Structure | Name |
|---|---|---|
| 83 | 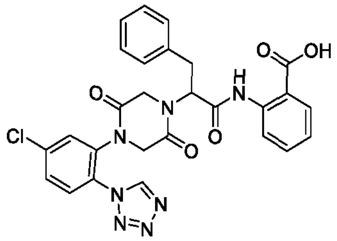 | 2-(2-(4-(5-chloro-2-(1*H*-tetrazol-1 - yl)phenyl)-2,5-dioxopiperazin-1-yl)-3-phenylpropanamido)benzoic acid |
| 84 | 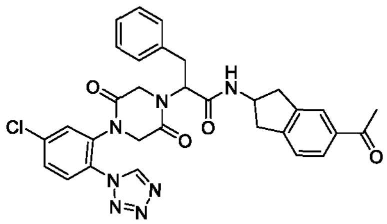 | *N*-(5-acetyl-2,3-dihydro-1*H*-inden-2-yl)-2-(4-(5-chloro-2-(1*H*-tetrazol-1-yl)phenyl)-2,5-dioxopiperazin-1-yl)-3-phenylpropanamide |
| 85 | 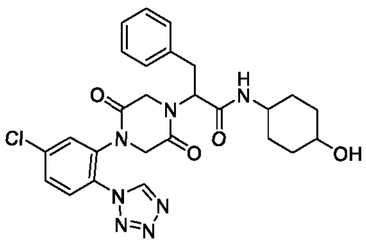 | 2-(4-(5-chloro-2-(1*H*-tetrazol-1-yl)phenyl)-2,5-dioxopiperazin-1-yl)-*N*-(4-hydroxycyclohexyl)-3-phenylpropanamide |
| 86 | 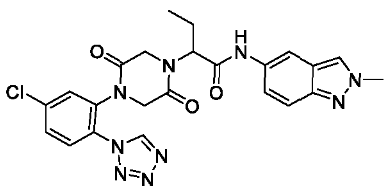 | 2-(4-(5-chloro-2-(1*H*-tetrazol-1-yl)phenyl)-2,5-dioxopiperazin-1-yl)-*N*-(2-methyl-2*H*-indazol-5-yl) butanamide |
| 87 | 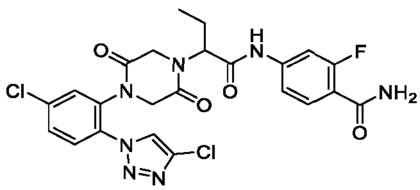 | 4-(2-(4-(5-chloro-2-(4-chloro-1*H*-1,2,3-triazol-1-yl) phenyl)-2,5-dioxopiperazin-1-yl)butanamido)-2-fluorobenzamide |
| 88 | 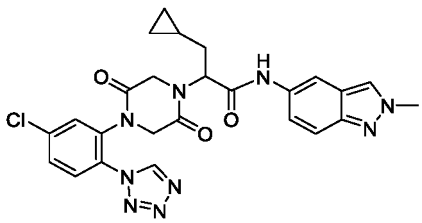 | 2-(4-(5-chloro-2-(1*H*-tetrazol-1-yl)phenyl)-2,5-dioxopiperazin-1-yl)-3-cyclopropyl-*N*-(2-methyl-2*H*-indazol-5-yl)propanamide |

(continued)

| No. of compound | Structure | Name |
|---|---|---|
| 89 | 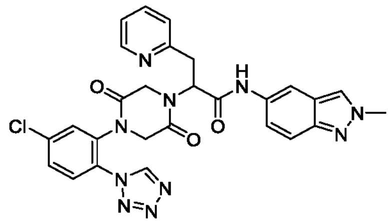 | 2-(4-(5-chloro-2-(1*H*-tetrazol-1-yl)phenyl)-2,5-dioxopiperazin-1-yl)-*N*-(2-methyl-2*H*-indazol-5-yl)-3-(pyridin-2-yl)propanamide |
| 90 | 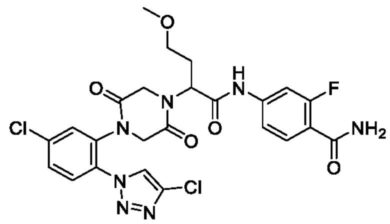 | 4-(2-(4-(5-chloro-2-(4-chloro-1*H*-1,2,3-triazol-1-yl)phenyl)-2,5-dioxopiperazin-1-yl)-4-methoxybutanamido)-2-fluorobenzamide |
| 91 | 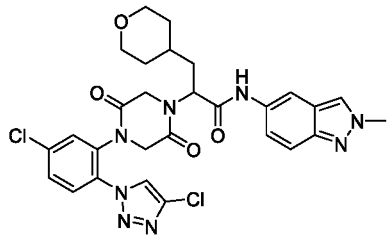 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(tetrahydro-2*H*-pyran-4-yl)propanamide |
| 92 | 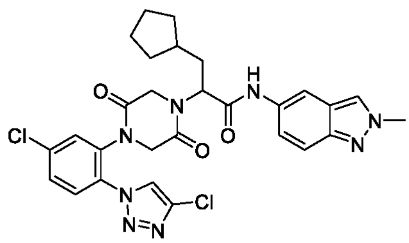 | 2-(4-(5-chloro-2-(4-chloro-1*H*-1,2,3-triazol-1-yl)phenyl)-2,5-dioxopiperazin-1-yl)-3-cyclopentyl-*N*-(2-methyl-2*H*-indazol-5-yl)propanamide |
| 93 | 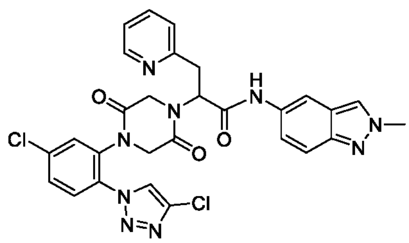 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-2-yl)propanamide |
| 94 | 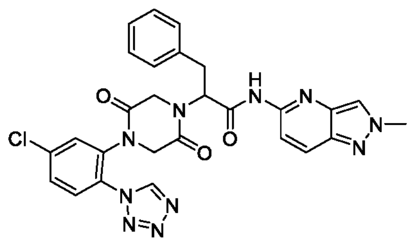 | 2-(4-(5-chloro-2-(1*H*-tetrazol-1-yl)phenyl)-2,5-dioxopiperazin-1-yl)-*N*-(2-methyl-2*H*-pyrazolo[4,3-b]pyridin-5-yl)-3-phenylpropanamide |

(continued)

| No. of compound | Structure | Name |
|---|---|---|
| 95 | 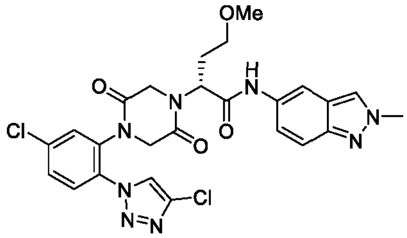 | (R)-2-(4-(5-chloro-2-(4-chloro-1*H*-1,2,3-triazol-1-yl) phenyl)-2,5-dioxopiperazin-1-yl)-4-methoxy-*N*-(2-methyl-2*H*-indazol-5-yl)butanamide |
| 96 | 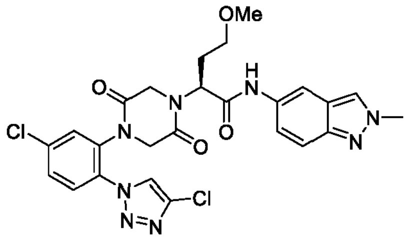 | (S)-2-(4-(5-chloro-2-(4-chloro-1*H*-1,2,3-triazol-1-yl) phenyl)-2,5-dioxopiperazin-1-yl)-4-methoxy-*N*-(2-methyl-2*H*-indazol-5-yl)butanamide |
| 97 | 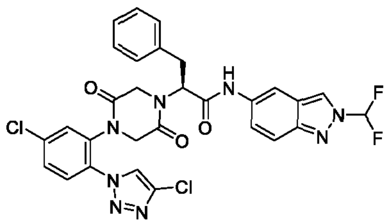 | (S)-2-(4-(5-chloro-2-(4-chloro-1*H*-1,2,3-triazol-1-yl) phenyl)-2,5-dioxopiperazin-1-yl)-*N*-(2-(difluoromethyl)-2*H*-indazol-5-yl)-3-phenylpropanamide |
| 98 | 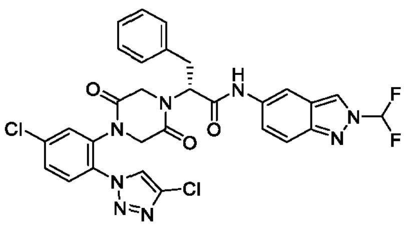 | (R)-2-(4-(5-chloro-2-(4-chloro-1*H*-1,2,3-triazol-1-yl) phenyl)-2,5-dioxopiperazin-1-yl)-*N*-(2-(difluoromethyl)-2*H*-indazol-5-yl)-3-phenylpropanamide |
| 99 | 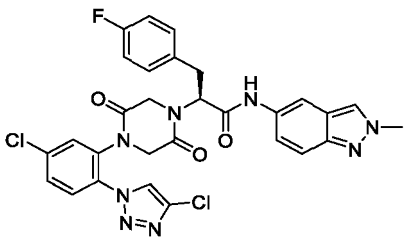 | (R)-2-(4-(5-chloro-2-(4-chloro-1*H*-1,2,3-triazol-1-yl) phenyl)-2,5-dioxopiperazin-1-yl)-3 -(4-fluorophenyl)-*N*-(2-methyl-2*H*-indazol-5-yl) propanamide |
| 100 | 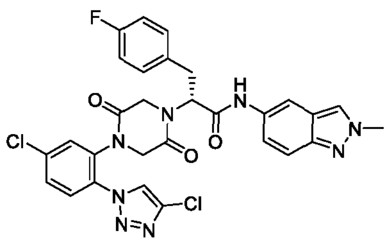 | (S)-2-(4-(5-chloro-2-(4-chloro-1*H*-1,2,3-triazol-1-yl) phenyl)-2,5-dioxopiperazin-1-yl)-3-(4-fluorophenyl)-*N*-(2-methyl-2*H*-indazol-5-yl) propanamide |

(continued)

| No. of compound | Structure | Name |
|---|---|---|
| 101 | 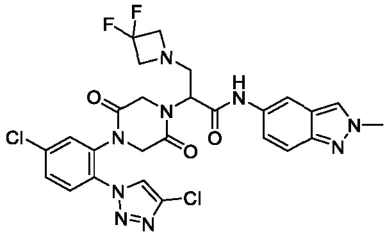 | 2-(4-(5-chloro-2-(4-chloro-1*H*-1,2,3-triazol-1-yl)phenyl)-2,5-dioxopiperazin-1-yl)-3-(3,3-difluoroazetidin-1-yl)-*N*-(2-methyl-2*H*-indazol-5-yl)propanamide |
| 102 | 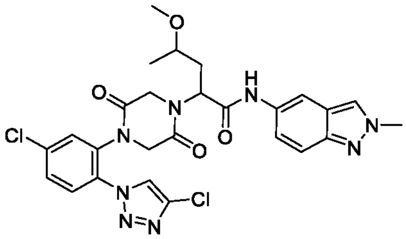 | 2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)pentanamide |
| 103 | 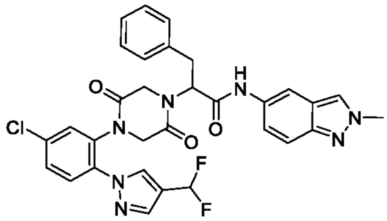 | 2-(4-(5-chloro-2-(4-(difluoromethyl)-1*H*-pyrazol-1-yl) phenyl)-2,5-dioxopiperazin-1-yl)-*N*-(2-methyl-2*H*-indazol-5-yl)-3-phenylpropanamide |
| 104 | 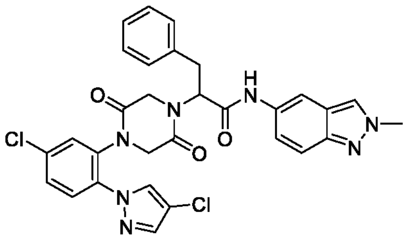 | 2-(4-(5-chloro-2-(4-chloro-1*H*-pyrazol-1-yl)phenyl)-2,5-dioxopiperazin-1-yl)-*N*-(2-methyl-2*H*-indazol-5-yl)-3-phenylpropanamide |
| 105 | 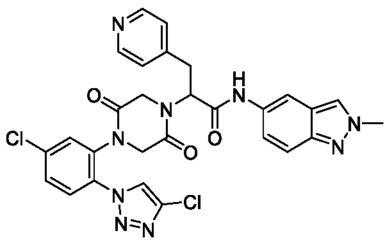 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-4-yl)propanamide |
| 106 | 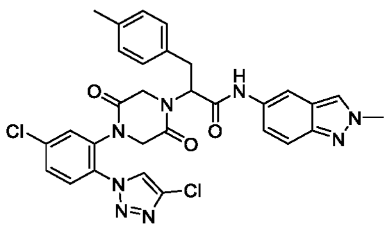 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(p-tolyl)propanamide |

(continued)

| No. of compound | Structure | Name |
|---|---|---|
| 107 | 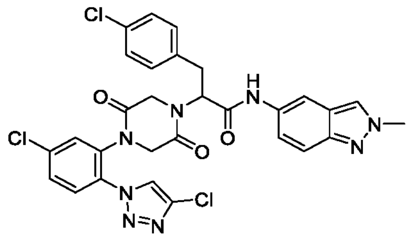 | 2-(4-(5-chloro-2-(4-chloro-1*H*-1,2,3-triazol-1-yl)phenyl)-2,5-dioxopiperazin-1-yl)-3-(4-chlorophenyl)-*N*-(2-methyl-2*H*-indazol-5-yl)propanamide |
| 108 | 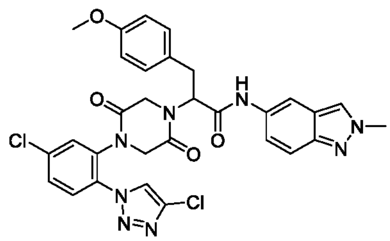 | 2-(4-(5-chloro-2-(4-chloro-1*H*-1,2,3-triazol-1-yl)phenyl)-2,5-dioxopiperazin-1-yl)-3-(4-methoxyphenyl)-*N*-(2-methyl-2*H*-indazol-5-yl)propanamide |
| 109 | 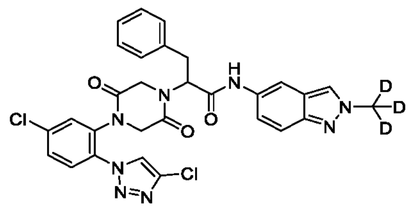 | 2-(4-(5-chloro-2-(4-chloro-1*H*-1,2,3-triazol-1-yl)phenyl)-2,5-dioxopiperazin-1-yl)-N-(2-(methyl-$d_3$)-2*H*-indazol-5-yl)-3 - phenylpropanamide |
| 110 | 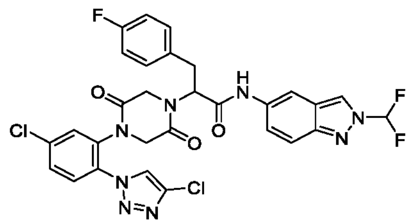 | 2-(4-(5-chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-(4-fluorophenyl)propanamide |
| 111 | 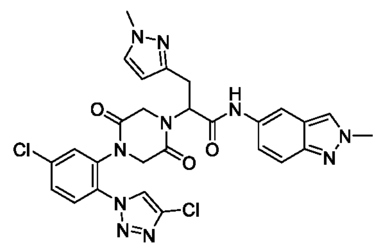 | 2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)-*N*-(2-methyl-2*H*-indazol-5-yl)propanamide |
| 112 | 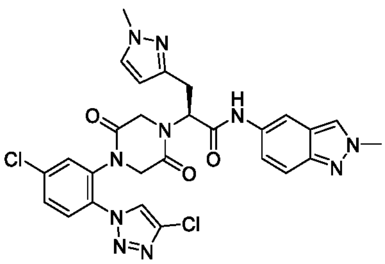 | (S)-2-(4-(5-chloro-2-(4-chloro-1*H*-1,2,3-triazol-1-yl) phenyl)-2,5-dioxopiperazin-1-yl)-3-(1-methyl-1*H*-pyrazol-3-yl)-*N*-(2-methyl-2*H*-indazol-5-yl) propanamide |

(continued)

| No. of compound | Structure | Name |
|---|---|---|
| 113 | 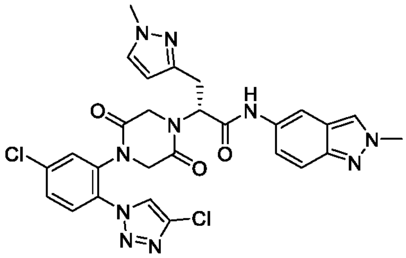 | (R)-2-(4-(5-chloro-2-(4-chloro-1*H*-1,2,3-triazol-1-yl) phenyl)-2,5-dioxopiperazin-1-yl)-3-(1-methyl-1*H*-pyrazol-3-yl)-*N*-(2-methyl-2*H*-indazol-5-yl) propanamide |
| 114 | 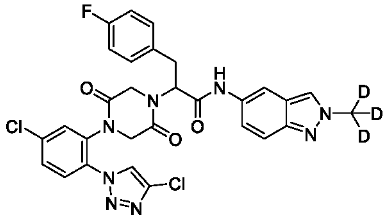 | 2-(4-(5-chloro-2-(4-chloro-1*H*-1,2,3-triazol-1-yl)phenyl)-2,5-dioxopiperazin-1-yl)-3-(4-fluorophenyl)-*N*-(2-(methyl-$d_3$)-2*H*-indazol-5-yl) propanamide |
| 115 | 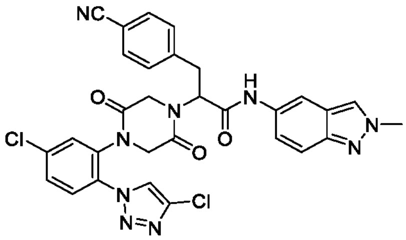 | 2-(4-(5-chloro-2-(4-chloro-1*H*-1,2,3-triazol-1-yl)phenyl)-2,5-dioxopiperazin-1-yl)-3-(4-cyanophenyl)-*N*-(2-methyl-2*H*-indazol-5-yl)propanamide |
| 116 | 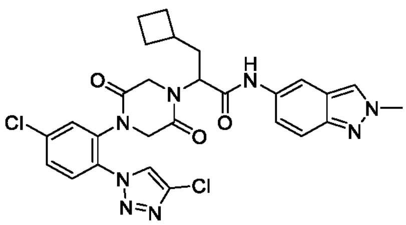 | 2-(4-(5-chloro-2-(4-chloro-1*H*-1,2,3-triazol-1-yl)phenyl)-2,5-dioxopiperazin-1-yl)-3-cyclobutyl-*N*-(2-methyl-2*H*-indazol-5-yl)propanamide |
| 117 | 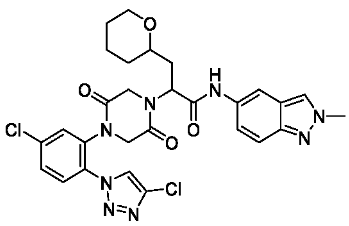 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(tetrahydro-2*H*-pyran-2-yl)propanamide |
| 118 | 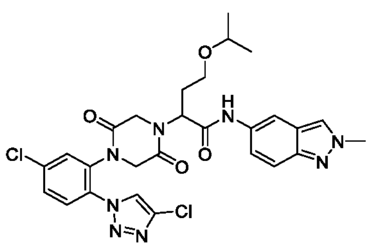 | 2-(4-(5-chloro-2-(4-chloro-1*H*-1,2,3-triazol-1-yl)phenyl)-2,5-dioxopiperazin-1-yl)-4-isopropoxy-*N*-(2-methyl-2*H*-indazol-5-yl)butanamide |

(continued)

| No. of compound | Structure | Name |
|---|---|---|
| 119 | 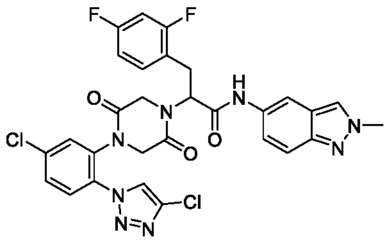 | 2-(4-(5-chloro-2-(4-chloro-1*H*-1,2,3-triazol-1-yl)phenyl)-2,5-dioxopiperazin-1-yl)-3-(2,4-difluorophenyl)-*N*-(2-methyl-2*H*-indazol-5-yl)propanamide |
| 120 | 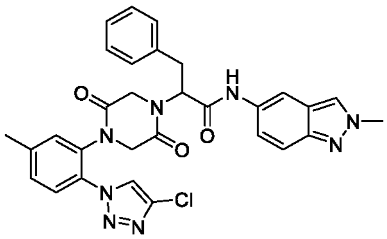 | 2-(4-(2-(4-chloro-1*H*-1,2,3-triazol-1-yl)-5-methylphenyl)-2,5-dioxopiperazin-1-yl)-*N*-(2-methyl-2*H*-indazol-5-yl)-3 - phenylpropanamide |
| 121 | 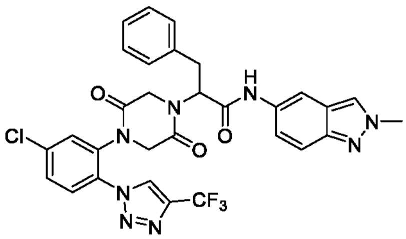 | 2-(4-(5-chloro-2-(4-(trifluoromethyl)-1*H*-1,2,3-triazol-1-yl)phenyl)-2,5-dioxopiperazin-1-yl)-*N*-(2-methyl-2*H*-indazol-5-yl)-3-phenylpropanamide |
| 122 | 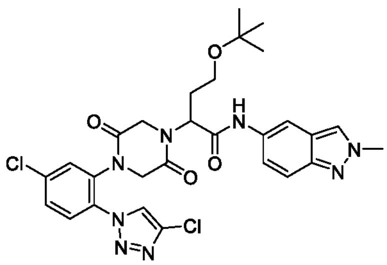 | 4-(tert-butoxy)-2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)butanamide |
| 123 | 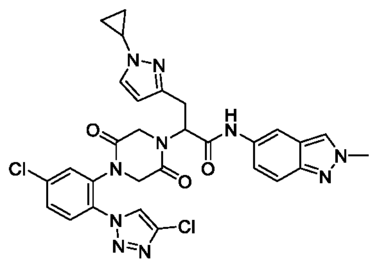 | 2-(4-(5-chloro-2-(4-chloro-1*H*-1,2,3-triazol-1-yl)phenyl)-2,5-dioxopiperazin-1-yl)-3-(1-cyclopropyl-1*H*-pyrazol-3-yl)-*N*-(2-methyl-2H-indazol-5-yl)propanamide |
| 124 | 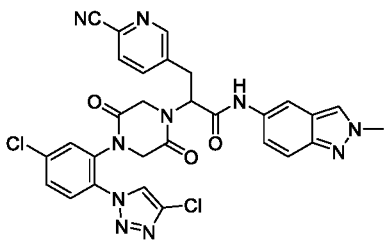 | 2-(4-(5-chloro-2-(4-chloro-1*H*-1,2,3-triazol-1-yl)phenyl)-2,5-dioxopiperazin-1-yl)-3-(6-cyanopyridin-3-yl)-*N*-(2-methyl-2*H*-indazol-5-yl)propanamide |

(continued)

| No. of compound | Structure | Name |
|---|---|---|
| 125 | 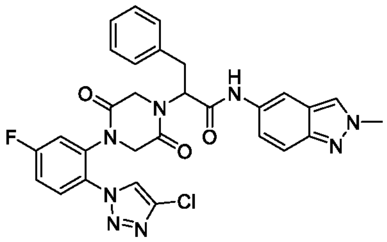 | 2-(4-(2-(4-chloro-1*H*-1,2,3-triazol-1-yl)-5-fluorophenyl)-2,5-dioxopiperazin-1-yl)-*N*-(2-methyl-2*H*-indazol-5-yl)-3-phenylpropanamide |
| 126 | 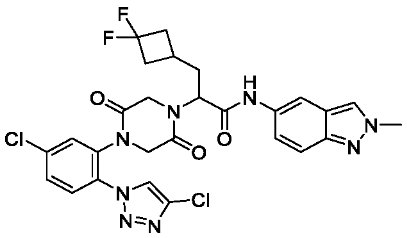 | 2-(4-(5-chloro-2-(4-chloro-1*H*-1,2,3-triazol-1-yl)phenyl)-2,5-dioxopiperazin-1-yl)-3-(3,3-difluorocyclobutyl)-*N*-(2-methyl-2*H*-indazol-5-yl)propanamide |
| 131 | 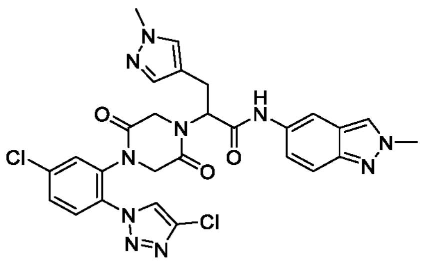 | 2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)-*N*-(2-methyl-2*H*-indazol-5-yl)propanamide |
| 132 | 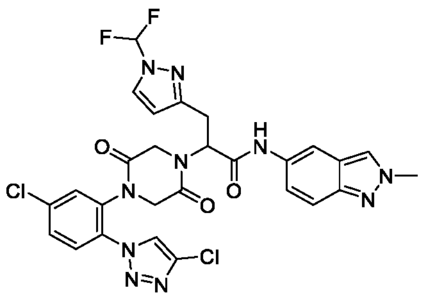 | 2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl)-*N*-(2-methyl-2*H*-indazol-5-yl) propanamide |
| 133 | 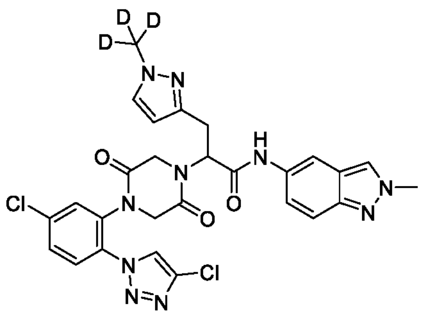 | 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(1-(methyl-$d_3$)-1*H*-pyrazol-3 - yl)propanamide |

(continued)

| No. of compound | Structure | Name |
|---|---|---|
| 134 | 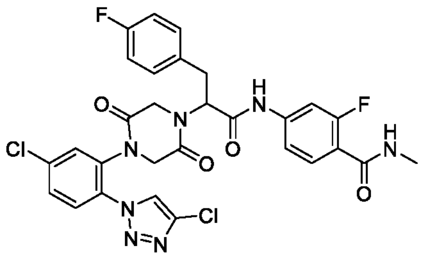 | 4-(2-(4-(5-chloro-2-(4-chloro-1*H*-1,2,3-triazol-1-yl) phenyl)-2,5-dioxopiperazin-1-yl)-3-(4-fluorophenyl) propanamido)-2-fluoro-*N*-methylbenzamide |
| 135 | 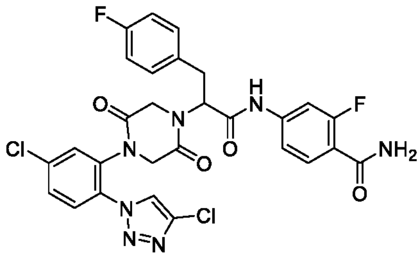 | 4-(2-(4-(5-chloro-2-(4-chloro-1*H*-1,2,3-triazol-1-yl) phenyl)-2,5-dioxopiperazin-1-yl)-3-(4-fluorophenyl) propanamido)-2-fluorobenzamide |
| 136 | 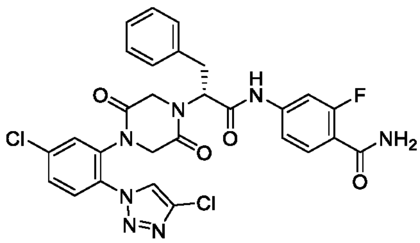 | (R)-4-(2-(4-(5-chloro-2-(4-chloro-1*H*-1,2,3-triazol-1-yl) phenyl)-2,5-dioxopiperazin-1-yl)-3-phenylpropanamido)-2-fluorobenzamide |
| 137 | 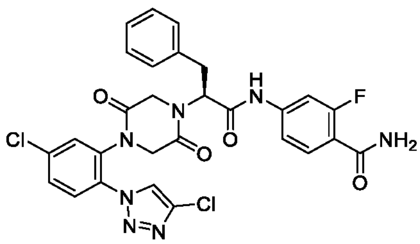 | (S)-4-(2-(4-(5-chloro-2-(4-chloro-1*H*-1,2,3-triazol-1-yl) phenyl)-2,5-dioxopiperazin-1 -yl)-3 -phenylpropanamido)-2-fluorobenzamide |
| 138 | 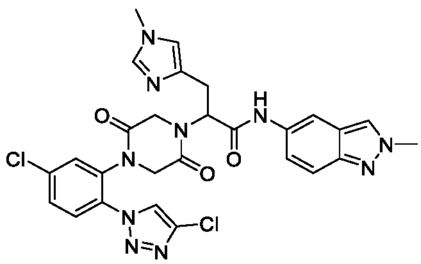 | 2-(4-(5-chloro-2-(4-chloro-1*H*-1,2,3-triazol-1-yl)phenyl)-2,5-dioxopiperazin-1-yl)-3-(1-methyl-1*H*-imidazol-4-yl)-*N*-(2-methyl-2*H*-indazol-5-yl)propanamide |

or a stereoisomer thereof, a tautomer thereof, a pharmaceutically acceptable salt thereof or a prodrug thereof.

The present invention provides a preparation method for a compound represented by general formula (II), or a stereoisomer thereof, a tautomer thereof, a pharmaceutically acceptable salt thereof or a prodrug thereof, which comprises the following step of:

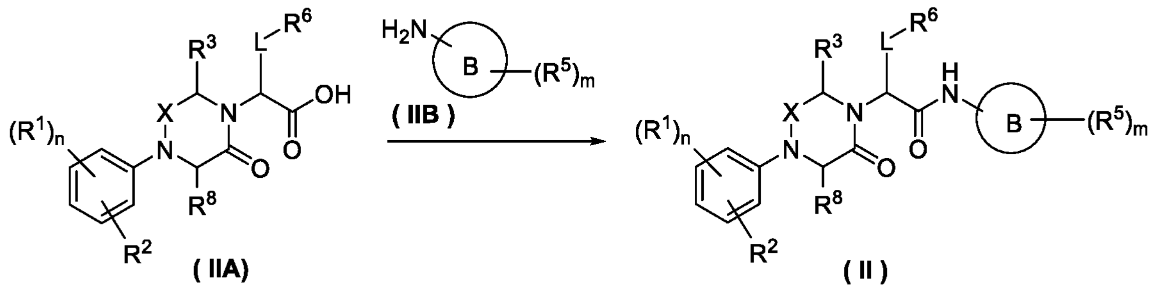

subjecting the compound represented by general formula (IIA) and the compound represented by general formula (IIB) to a condensation reaction, and optionally further hydrolyzing the mixture under acidic conditions to obtain the compound represented by general formula (II);

wherein, ring B, X, L, $R^1$ to $R^3$, $R^5$, $R^6$, $R^8$, m and n are as defined in general formula (II).

The present invention provides a compound represented by general formula (IIA), or a stereoisomer thereof, a tautomer thereof or a pharmaceutically acceptable salt thereof, wherein:

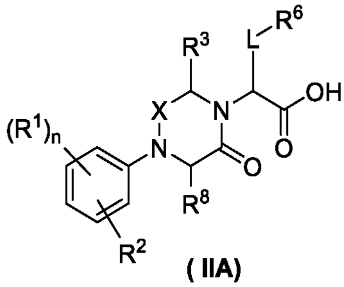

( IIA)

wherein, X, L, $R^1$ to $R^3$, $R^6$, $R^8$ and n are as defined in general formula (II).

Typical compounds of formula (IIA) comprise, but are not limited to:

| No. of compound | Structure | Name |
|---|---|---|
| 1i | 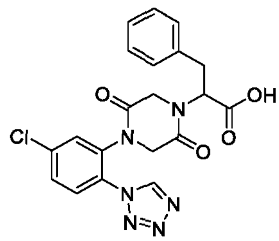 | 2-(4-(5-chloro-2-(1*H*-tetrazol-1-yl)phenyl)-2,5-dioxopiperazin-1-yl)-3-phenylpropanoic acid |
| 11f | 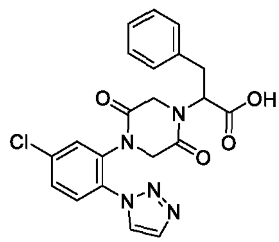 | 2-(4-(5-chloro-2-(1*H*-1,2,3-triazol-1-yl)phenyl)-2,5-dioxopiperazin-l-yl)-3-phenylpropanoic acid |

(continued)

| No. of compound | Structure | Name |
|---|---|---|
| 24h | 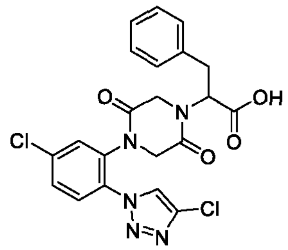 | 2-(4-(5-chloro-2-(4-chloro-1*H*-1,2,3-triazol-1-yl)phenyl)-2,5-dioxopiperazin-1-yl)-3-phenylpropanoic acid |
| 32g | 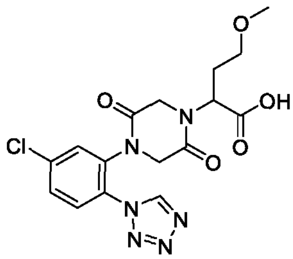 | 2-(4-(5-chloro-2-(1*H*-tetrazol-1-yl)phenyl)-2,5-dioxopiperazin-1-yl)-4-methoxybutanoic acid |
| 38d | 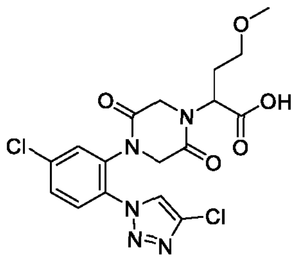 | 2-(4-(5-chloro-2-(4-chloro-1*H*-1,2,3-triazol-1-yl)phenyl)-2,5-dioxopiperazin-1-yl)-4-methoxybutanoic acid |
| 39h | 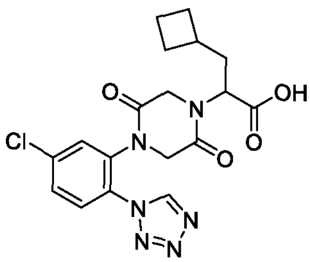 | 2-(4-(5-chloro-2-(1*H*-tetrazol-1-yl)phenyl)-2,5-dioxopiperazin-1-yl)-3-cyclobutylpropanoic acid |
| 41e | 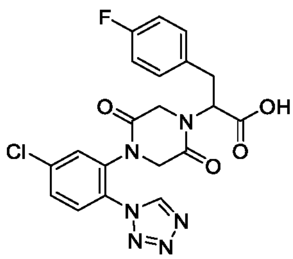 | 2-(4-(5-chloro-2-(1*H*-tetrazol-1-yl)phenyl)-2,5-dioxopiperazin-1-yl)-3-(4-fluorophenyl)propanoic acid |
| 43g | 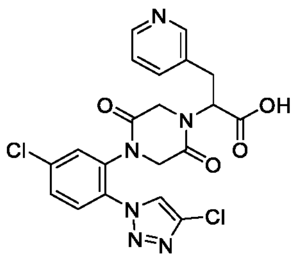 | 2-(4-(5-chloro-2-(4-chloro-1*H*-1,2,3-triazol-1-yl)phenyl)-2,5-dioxopiperazin-1-yl)-3-(pyridin-3-yl)propanoic acid |

(continued)

| No. of compound | Structure | Name |
|---|---|---|
| 44g | 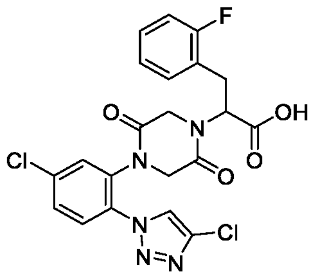 | 2-(4-(5-chloro-2-(4-chloro-1*H*-1,2,3-triazol-1-yl)phenyl)-2,5-dioxopiperazin-1-yl)-3-(2-fluorophenyl)propanoic acid |
| 46g | 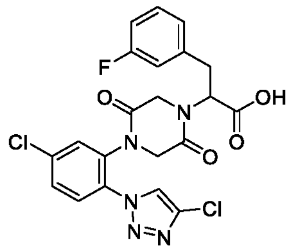 | 2-(4-(5-chloro-2-(4-chloro-1*H*-1,2,3-triazol-1-yl)phenyl)-2,5-dioxopiperazin-1-yl)-3-(3-fluorophenyl)propanoic acid |
| 47d | 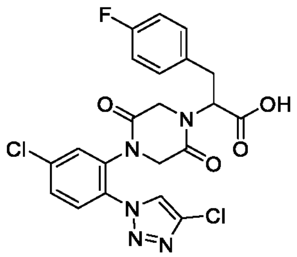 | 2-(4-(5-chloro-2-(4-chloro-1*H*-1,2,3-triazol-1-yl)phenyl)-2,5-dioxopiperazin-1-yl)-3-(4-fluorophenyl)propanoic acid |
| 49j | 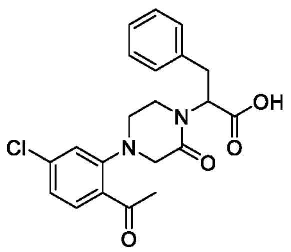 | 2-(4-(2-acetyl-5-chlorophenyl)-2-oxopiperazin-1-yl)-3-phenylpropanoic acid |
| 105g | 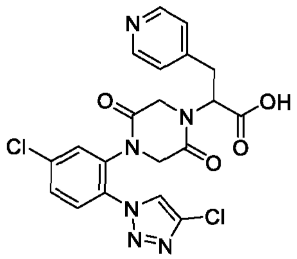 | 2-(4-(5-chloro-2-(4-chloro-1*H*-1,2,3 - triazol-1-yl)phenyl)-2,5-dioxopiperazin-1-yl)-3-(pyridin-4-yl)propanoic acid |
| 106e | 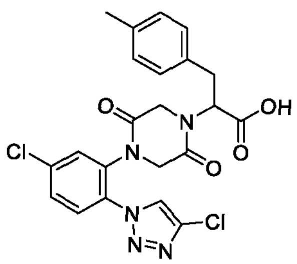 | 2-(4-(5-chloro-2-(4-chloro-1*H*-1,2,3-triazol-1-yl)phenyl)-2,5-dioxopiperazin-1-yl)-3-(*p*-tolyl)propanoic acid |

(continued)

| No. of compound | Structure | Name |
|---|---|---|
| 107g | 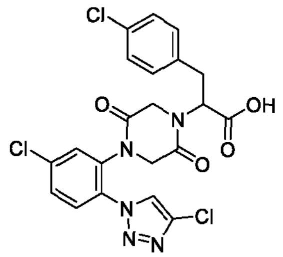 | 2-(4-(5-chloro-2-(4-chloro-1*H*-1,2,3-triazol-1-yl)phenyl)-2,5-dioxopiperazin-1-yl)-3-(4-chlorphenyl)propanoic acid |
| 108g | 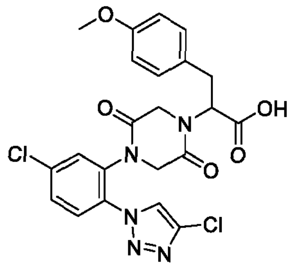 | 2-(4-(5-chloro-2-(4-chloro-1*H*-1,2,3-triazol-1-yl)phenyl)-2,5-dioxopiperazin-1-yl)-3-(4-methoxyphenyl)propanoic acid |
| 111i | 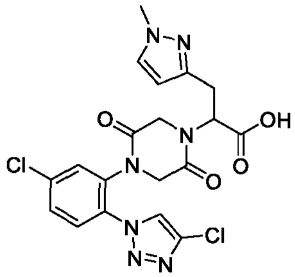 | 2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)propanoic acid |
| 115g | 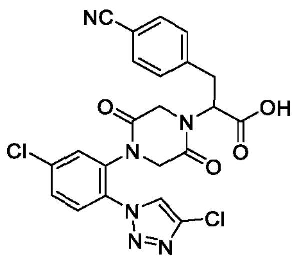 | 2-(4-(5-chloro-2-(4-chloro-1*H*-1,2,3-triazol-1-yl)phenyl)-2,5-dioxopiperazin-1-yl)-3-(4-cyanophenyl)propanoic acid |
| 116d | 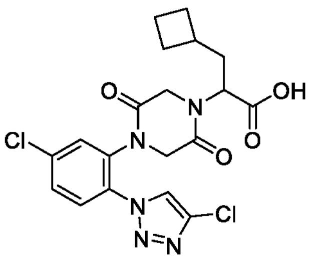 | 2-(4-(5-chloro-2-(4-chloro-1*H*-1,2,3-triazol-1-yl)phenyl)-2,5-dioxopiperazin-1-yl)-3-cyclobutylpropanoic acid |

(continued)

| No. of compound | Structure | Name |
|---|---|---|
| 117h | 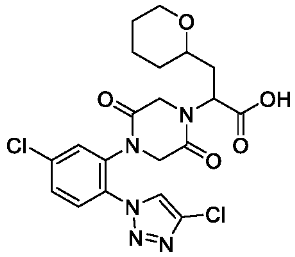 | 2-(4-(5-chloro-2-(4-chloro-1*H*-1,2,3-triazol-1-yl)phenyl)-2,5-dioxopiperazin-1-yl)-3-(tetrahydro-2*H*-pyran-2-yl)propanoic acid |
| 118h | 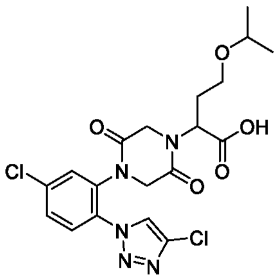 | 2-(4-(5-chloro-2-(4-chloro-1*H*-1,2,3-triazol-1-yl)phenyl)-2,5-dioxopiperazin-1-yl)-4-isopropoxybutanoic acid |
| 119g | 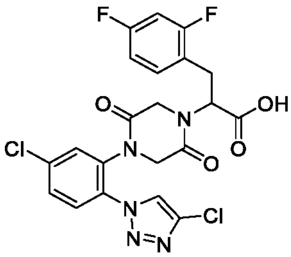 | 2-(4-(5-chloro-2-(4-chloro-1*H*-1,2,3-triazol-1-yl)phenyl)-2,5-dioxopiperazin-1-yl)-3-(2,4-difluorophenyl)propanoic acid |
| 120i | 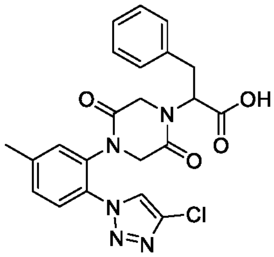 | 2-(4-(2-(4-chloro-1*H*-1,2,3-triazol-1-yl)-5-methylphenyl)-2,5-dioxopiperazin-1-yl)-3-phenylpropanoic acid |
| 121h | 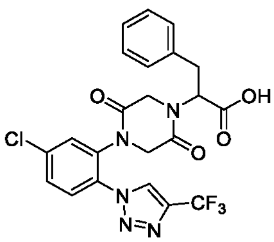 | 2-(4-(5-chloro-2-(4-(trifluoromethyl)-1*H*-1,2,3-triazol-1-yl)phenyl)-2,5-dioxopiperazin-1 -yl)-3 -phenylpropanoic acid |

(continued)

| No. of compound | Structure | Name |
|---|---|---|
| 122i | 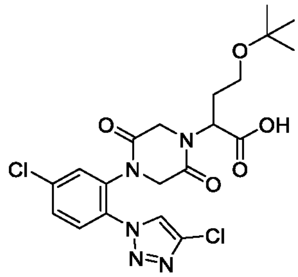 | 4-(tert-butoxy)-2-(4-(5-chloro-2-(4-chloro-1*H*-1,2,3-triazol-1-yl) phenyl)-2,5-dioxopiperazin-1-yl)butanoic acid |
| 123j | 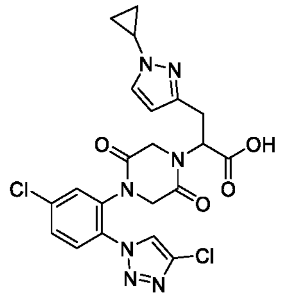 | 2-(4-(5-chloro-2-(4-chloro-1*H*-1,2,3-triazol-1-yl)phenyl)-2,5-dioxopiperazin-1-y1)-3-(1-cyclopropyl-1*H*-pyrazol-3-yl)propanoic acid |
| 131k | 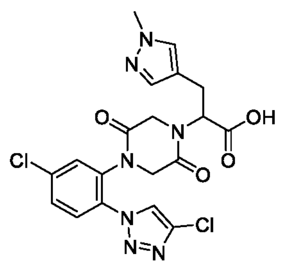 | 2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)propanoic acid |
| 132k | 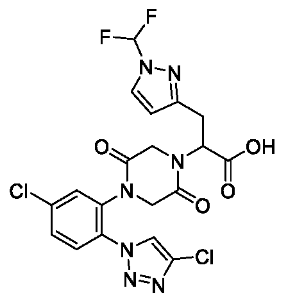 | 2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl) propanoic acid |
| 133j | 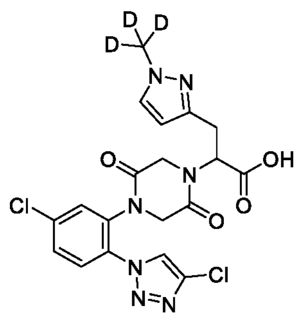 | 2-(4-(5-chloro-2-(4-chloro-1*H*-1,2,3-triazol-1-yl)phenyl)-2,5-dioxopiperazin-1-yl)-3-(1-(methyl-$d_3$)-1*H*-pyrazol-3-yl)propanoic acid |

or a stereoisomer thereof, a tautomer thereof or a pharmaceutically acceptable salt thereof. Note: if there is a difference between the drawn structure and the given name of the structure, the drawn structure will be given greater weight.

In another aspect, the present invention provides a pharmaceutical composition, and the pharmaceutical composition contains an effective dose of a compound represented by general formula (I), (II), (III), (IV), (V), (V-A), (V-B), (VI), (VI-A), (VI-B) or (VII), or a stereoisomer thereof, a tautomer thereof, a pharmaceutically acceptable salt thereof or a prodrug thereof, and a pharmaceutically acceptable carrier, an excipient or a combination thereof.

In another aspect, the present invention provides a method for inhibiting a protease of a coagulation factor XIa, wherein the method comprises administering to a patient a pharmaceutical composition, and the pharmaceutical composition contains an effective dose of a compound represented by general formula (I), (II), (III), (IV), (V), (V-A), (V-B), (VI), (VI-A), (VI-B) or (VII), or a stereoisomer thereof, a tautomer thereof, a pharmaceutically acceptable salt thereof or a prodrug thereof, and a pharmaceutically acceptable carrier, an excipient or a combination thereof.

In another aspect, the present invention provides a compound represented by general formula (I), (II), (III), (IV), (V), (V-A), (V-B), (VI), (VI-A), (VI-B) or (VII), or a stereoisomer thereof, a tautomer thereof, a pharmaceutically acceptable salt thereof or a prodrug thereof, or use of a pharmaceutical composition thereof (comprising the pharmaceutical composition provided by the present invention, the same below) in preparing an inhibitor of coagulation factor XIa or a dual inhibitor of coagulation factor XIa and plasma kallikrein.

Another aspect of the present invention relates to a method for preventing and/or treating a disease mediated by a coagulation factor XIa, which comprises administering to a patient a therapeutically effective dose of a compound represented by general formulas (I), (II), (III), (IV), (V), (V-A), (V-B), (VI), (VI-A), (VI-B) or (VII), or a stereoisomer thereof, a tautomer thereof, a mesomer thereof, a racemate thereof, an enantiomer thereof, a diastereomer thereof or a mixture thereof, a pharmaceutically acceptable salt thereof or a prodrug thereof, or a pharmaceutical composition containing the compound.

Wherein, the disease mediated by the coagulation factor XIa is preferably a cardiovascular and cerebrovascular disease.

The cardiovascular and cerebrovascular disease is preferably selected from a coagulation disease or a thromboembolic disease.

The thromboembolic disease is preferably selected from arterial cardiovascular thromboembolism, venous cardiovascular thromboembolism, arterial cerebrovascular thromboembolism, venous cerebrovascular thromboembolism, and ventricular or peripheral circulation thromboembolism; and the thromboembolic disease is more preferably selected from unstable angina pectoris, acute coronary syndrome, atrial fibrillation, myocardial infarction, sudden ischemic death, transient ischemic attack, stroke, atherosclerosis, peripheral occlusive arterial diseases, venous thrombosis, thrombophlebitis, arterial embolism, coronary artery thrombosis, cerebral arterial thrombosis, cerebral embolism, renal embolism, pulmonary embolism, and thrombosis caused by a medical implant, device or surgery, wherein blood is contacted with an artificial surface capable of promoting thrombosis. The venous thrombosis is preferably deep venous thrombosis.

Another aspect of the present invention relates to a method for preventing and/or treating a cardiovascular and cerebrovascular disease, which comprises administering to a patient a therapeutically effective dose of a compound represented by general formulas (I), (II), (III), (IV), (V), (V-A), (V-B), (VI), (VI-A), (VI-B) or (VII), or a stereoisomer thereof, a tautomer thereof, a mesomer thereof, a racemate thereof, an enantiomer thereof, a diastereomer thereof or a mixture thereof, a pharmaceutically acceptable salt thereof or a prodrug thereof, or a pharmaceutical composition containing the compound.

Another aspect of the present invention relates to an anti-coagulation method, which comprises administering to a patient a therapeutically effective dose of a compound represented by general formulas (I), (II), (III), (IV), (V), (V-A), (V-B), (VI), (VI-A), (VI-B) or (VII), or a stereoisomer thereof, a tautomer thereof, a mesomer thereof, a racemate thereof, an enantiomer thereof, a diastereomer thereof or a mixture thereof, a pharmaceutically acceptable salt thereof or a prodrug thereof, or a pharmaceutical composition containing the compound.

Another aspect of the present invention relates to a method for preventing and/or treating a thromboembolic disease, which comprises administering to a patient a therapeutically effective dose of a compound represented by general formulas (I), (II), (III), (IV), (V), (V-A), (V-B), (VI), (VI-A), (VI-B) or (VII), or a stereoisomer thereof, a tautomer thereof, a mesomer thereof, a racemate thereof, an enantiomer thereof, a diastereomer thereof or a mixture thereof, a pharmaceutically acceptable salt thereof or a prodrug thereof, or a pharmaceutical composition containing the compound.

The thromboembolic disease is preferably selected from arterial cardiovascular thromboembolism, venous cardiovascular thromboembolism, arterial cerebrovascular thromboembolism, venous cerebrovascular thromboembolism, and ventricular or peripheral circulation thromboembolism; and the thromboembolic disease is more preferably selected from unstable angina pectoris, acute coronary syndrome, atrial fibrillation, myocardial infarction, sudden ischemic death, transient ischemic attack, stroke, atherosclerosis, peripheral occlusive arterial diseases, venous thrombosis, thrombophlebitis, arterial embolism, coronary artery thrombosis, cerebral arterial thrombosis, cerebral embolism, renal embolism, pulmonary embolism, and thrombosis caused by a medical implant, device or surgery, wherein blood is contacted with an artificial surface capable of promoting thrombosis. The venous thrombosis is preferably deep venous thrombosis.

Another aspect of the present invention relates to a compound represented by general formula (I), (II), (III), (IV), (V), (V-A), (V-B), (VI), (VI-A), (VI-B) or (VII), or a stereoisomer thereof, a tautomer thereof, a pharmaceutically acceptable salt thereof or a prodrug thereof, or use of a pharmaceutical composition thereof in preparing a medicament for treating or preventing a disease mediated by a coagulation factor XIa. The disease mediated by the coagulation factor XIa is preferably a cardiovascular and cerebrovascular disease.

Wherein, the disease mediated by the coagulation factor XIa is preferably a cardiovascular and cerebrovascular disease.

The cardiovascular and cerebrovascular disease is preferably selected from a coagulation disease or a thromboembolic disease.

The thromboembolic disease is preferably selected from arterial cardiovascular thromboembolism, venous cardiovascular thromboembolism, arterial cerebrovascular thromboembolism, venous cerebrovascular thromboembolism, and ventricular or peripheral circulation thromboembolism; and the thromboembolic disease is more preferably selected from unstable angina pectoris, acute coronary syndrome, atrial fibrillation, myocardial infarction, sudden ischemic death, transient ischemic attack, stroke, atherosclerosis, peripheral occlusive arterial diseases, venous thrombosis, thrombophlebitis, arterial embolism, coronary artery thrombosis, cerebral arterial thrombosis, cerebral embolism, renal embolism, pulmonary embolism, and thrombosis caused by a medical implant, device or surgery, wherein blood is contacted with an artificial surface capable of promoting thrombosis. The venous thrombosis is preferably deep venous thrombosis.

Another aspect of the present invention relates to a medicament for inhibiting a coagulation factor XIa, which comprises a compound represented by general formula (I), (II), (III), (IV), (V), (V-A), (V-B), (VI), (VI-A), (VI-B) or (VII), or a stereoisomer thereof, a tautomer thereof, a mesomer thereof, a racemate thereof, an enantiomer thereof, a diastereomer thereof or a mixture thereof, a pharmaceutically acceptable salt thereof or a prodrug thereof, or a pharmaceutical composition containing the compound.

In another aspect, the present invention provides use of a compound represented by general formula (I), (II), (III), (IV), (V), (V-A), (V-B), (VI), (VI-A), (VI-B) or (VII), or a stereoisomer thereof, a tautomer thereof, a pharmaceutically acceptable salt thereof or a prodrug thereof, or a pharmaceutical composition thereof in preparing an anti-coagulation medicament.

In another aspect, the present invention provides use of a compound represented by general formula (I), (II), (III), (IV), (V), (V-A), (V-B), (VI), (VI-A), (VI-B) or (VII), or a stereoisomer thereof, a tautomer thereof, a pharmaceutically acceptable salt thereof or a prodrug thereof, or a pharmaceutical composition thereof in preparing a medicament for treating or preventing a thromboembolic disease.

The thromboembolic disease is selected from arterial cardiovascular thromboembolism, venous cardiovascular thromboembolism, arterial cerebrovascular thromboembolism, venous cerebrovascular thromboembolism, and ventricular or peripheral circulation thromboembolism; and the thromboembolic disease is preferably selected from unstable angina pectoris, acute coronary syndrome, atrial fibrillation, myocardial infarction, sudden ischemic death, transient ischemic attack, stroke, atherosclerosis, peripheral occlusive arterial diseases, venous thrombosis, thrombophlebitis, arterial embolism, coronary artery thrombosis, cerebral arterial thrombosis, cerebral embolism, renal embolism, pulmonary embolism, and thrombosis caused by a medical implant, device or surgery, wherein blood is contacted with an artificial surface capable of promoting thrombosis. The venous thrombosis is preferably deep venous thrombosis.

The pharmaceutical preparation of the present invention may be administered topically, orally, percutaneously, rectally, vaginally, parenterally, intranasally, intrapulmonarily, intraocularly, intravenously, intramuscularly, intraarterially, intrathecally, intracapsularly, intradermally, intraperitoneally, subcutaneously, subcuticularly or inhalationally. The pharmaceutical composition containing active ingredients may be in a form suitable for oral administration, such as a tablet, a troche, a lozenge, a water or oil suspension, a dispersible powder or granule, an emulsion, a hard or soft capsule, or a syrup or elixir. The tablet contains active ingredients and non-toxic pharmaceutically acceptable excipients used for mixing and suitable for preparing the tablet.

The preparation of the present invention is suitable to exist in a form of unit measurement, and the preparation may be prepared by any method well known in pharmaceutical technology. An amount of active ingredients capable of being combined with a carrier substance to produce a single dosage form may vary depending on a host to be treated and a specific administration mode. The amount of active ingredients capable of being combined with the carrier substance to produce the single dosage form generally refers to an amount of compounds capable of exerting a therapeutic effect.

Dosage forms for topical or transdermal administration of the compound of the present invention may comprise a powder, a spray, an ointment, a paste, a cream, a lotion, a gel, a solution, a patch and an inhalant. The active compounds may be mixed with the pharmaceutically acceptable carrier under an aseptic condition, and may be mixed with any preservative, buffer or propellant needed possibly.

When the compound of the present invention is administered to humans and animals in a form of medicament, the compound may be provided separately or in a form of pharmaceutical composition, and the pharmaceutical composition contains the active ingredients combined with the pharmaceutically acceptable carrier, such as 0.1% to 99.5%

(more preferably, 0.5% to 90%) of the active ingredients.

Examples of pharmaceutically acceptable carriers comprise, but are not limited to: (1) sugars, such as lactose, glucose and sucrose; (2) starch, such as corn starch and potato starch; (3) cellulose and derivatives thereof, such as sodium carboxymethyl cellulose, ethyl cellulose and cellulose acetate; (4) powdered tragacanth gum; (5) malt; (6) gelatin; (7) talc; (8) excipients, such as cocoa butter and suppository wax; (9) oils, such as peanut oil, cottonseed oil, safflower oil, sesame oil, olive oil, corn oil and soybean oil; (10) diol, such as propylene glycol; (11) polyol, such as glycerol, sorbitol, mannitol and polyethylene glycol; (12) ester, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) phosphate buffer solution; (21) cyclodextrin, such as a targeting ligand bound to nano-particles, such as AccurinsTM; and (22) other non-toxic compatible substances used in pharmaceutical preparations, such as a polymer-based composition.

Examples of pharmaceutically acceptable antioxidants comprise, but are not limited to: (1) watersoluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, $\alpha$-tocopherol and the like; and (3) metal chelating agents, such as citric acid, ethylenediamine tetraacetic acid (EDTA), sorbitol, tartaric acid, phosphoric acid and the like. Solid dosage forms (such as a capsule, a lozenge and a pill, a troche, a powder, a granule and the like) may comprise one or more pharmaceutically acceptable carriers, such as sodium citrate or dicalcium phosphate, and/or any one of the followings: (1) a filler or an extender, such as starch, lactose, sucrose, glucose, mannitol and/or silicic acid; (2) a binder, such as carboxymethyl cellulose, alginate, gelatin, polyvinylpyrrolidone, sucrose and/or Arabic gum; (3) a humectant, such as glycerol; (4) a disintegrant, such as agar, calcium carbonate, potato or cassava starch, alginic acid, certain silicate and sodium carbonate; (5) a dissolution retardant, such as paraffin wax; (6) an absorption accelerator, such as a quaternary ammonium compound; (7) a wetting agent, such as cetyl alcohol and glycerol monostearate; (8) an absorbent, such as kaolin and bentonite; (9) a lubricant, such as talc, calcium stearate, magnesium stearate, solid polyethylene glycol, sodium lauryl sulfate, and a mixture thereof; and (10) a colorant. Liquid dosage forms may comprise pharmaceutically acceptable emulsion, microemulsion, solution, suspension, syrup and elixir. In addition to active ingredients, the liquid dosage forms may contain inert diluents commonly used in the technical field, such as water or other solvents; and solubilizers and emulsifiers, such as ethanol, isopropanol, ethyl carbonate, ethyl acetate, benzyl alcohol, benzyl benzoate, propylene glycol, 1,3-butanediol, oil (especially cottonseed oil, peanut oil, corn oil, germ oil, olive oil, castor oil and sesame oil), glycerol, tetrahydrofuran methanol, polyethylene glycol and fatty acid ester of sorbitan, and a mixture thereof.

In addition to active compounds, the suspension may also contain a suspending agent, such as ethoxylated isostearyl alcohol, polyoxyethylene sorbitol and dehydrated sorbitol ester, microcrystalline cellulose, aluminum hydroxide oxide, bentonite, agar and tragacanth, and a mixture thereof.

In addition to active compounds, an ointment, a paste, a cream and a gel may also contain an excipient, such as animal fat and vegetable fat, oil, wax, paraffin, starch, tragacanth, a cellulose derivative, polyethylene glycol, polysiloxane, bentonite, silicic acid, talc and zinc oxide, or a mixture thereof.

In addition to active compounds, a powder and a spray may also contain an excipient, such as lactose, talc, silicic acid, aluminum hydroxide, calcium silicate and polyamide powder, or a mixture of the above substances. The spray may contain other commonly used propellants, such as chlorofluorocarbon, and volatile unsubstituted hydrocarbon, such as butane and propane.

## Detailed description of the invention

Unless stated to the contrary, some terms used in the specification and the claims of the present invention are defined as follows.

"Bond" refers to that a labeled substituent does not exist, and two end parts of the substituent are directly connected to form the bond.

"Alkyl" refers to an aliphatic hydrocarbon group comprising a $C_1$-$C_{20}$ straight chain or having a branched chain when taken as one group or a part of one group. Preferably, the alkyl is $C_1$-$C_{10}$ alkyl, and more preferably, the alkyl is $C_1$-$C_6$ alkyl. Embodiments of an alkyl group comprise, but are not limited to, methyl, ethyl, n-propyl, isopropyl, n-butyl, isobutyl, tert-butyl, sec-butyl, n-pentyl, 1,1-dimethyl propyl, 1,2-dimethyl propyl, 2,2-dimethyl propyl, 1-ethyl propyl, 2-methyl butyl, 3-methyl butyl, n-hexyl, 1-ethyl-2-methyl propyl, 1,1,2-trimethyl propyl, 1,1-dimethyl butyl, 1,2-dimethyl butyl, 2,2-dimethyl butyl, 1,3-dimethyl butyl, 2-ethyl butyl, 2-methyl pentyl, 3-methyl pentyl, 4-methyl pentyl, 2,3-dimethyl butyl, and the like. The alkyl may be substituted or unsubstituted.

"Alkenyl" refers to the alkyl as defined above consisting of at least two carbon atoms and at least one carbon-carbon double bond, and representative examples comprise, but are not limited to, vinyl, 1-propenyl, 2-propenyl, 1-,2- or 3-butenyl, and the like. The alkenyl may be optionally substituted or unsubstituted.

"Alkynyl" refers to an aliphatic hydrocarbon group containing one carbon-carbon triple bond, and may be a

straight chain or have a branched chain. Preferably, the alkynyl is $C_2$-$C_{10}$ alkynyl, more preferably, the alkynyl is $C_2$-$C_6$ alkynyl, and most preferably, the alkynyl is $C_2$-$C_4$ alkynyl. Embodiments of an alkynyl group comprise, but are not limited to, ethynyl, 1-propinyl, 2-propinyl, 1-, 2- or 3-butynyl, and the like. The alkynyl may be substituted or unsubstituted.

"Alkylene" is divalent alkyl. Preferably, the alkylene is $C_1$-$C_{10}$ alkylene, more preferably, the alkylene is $C_1$-$C_6$ alkylene, and particularly preferably, the alkylene is $C_1$-$C_4$ alkylene. Embodiments of an alkylene group comprise, but are not limited to, methylene, ethylene, $-CH(CH_3)_2-$, n-propylidene, and the like. The alkylene may be substituted or unsubstituted.

"Cycloalkyl" refers to a saturated or partially saturated carboatomic ring of a monocyclic ring, a fused ring, a bridged ring and a spirocyclic ring. Preferably, the cycloalkyl is $C_3$-$C_{12}$ cycloalkyl, more preferably, the cycloalkyl is $C_3$-$C_8$ cycloalkyl, and most preferably, the cycloalkyl is $C_3$-$C_6$ cycloalkyl. Embodiments of the cycloalkyl of the monocyclic ring comprise, but are not limited to, cyclopropyl, cyclobutyl, cyclopentyl, cyclopentenyl, cyclohexyl, cyclohexenyl, cyclohexadienyl, cycloheptyl, cyclohepttrienyl, cyclooctyl and the like, and are preferably cyclopropyl and cyclohexenyl. The cycloalkyl may be optionally substituted or unsubstituted.

"Spiroalkyl" refers to a 5- to 18- membered polycyclic group with two or more cyclic structures, and single rings share one carbon atom (called a spiro atom) with each other, and the ring contains one or more double bonds, but no ring has a fully conjugated $\pi$ electron aromatic system. Preferably, the spiroalkyl is 6- to 14- membered, and more preferably, the spiroalkyl is 7- to 10- membered.. The spiroalkyl is classified into mono-, di- or multi-spiroalkyl according to a number of shared carbon atoms between rings, is preferably the mono- and di-spiroalkyl, and preferably is 4 membered/5 membered, 4 membered/6 membered, 5 membered/5 membered or 5 membered/6 membered. Non-limiting embodiments of the "spiroalkyl" comprise, but are not limited to, spiro [4.5] decyl, spiro [4.4] nonyl, spiro [3.5] nonyl and spiro [2.4] heptyl.

"Fused cycloalkyl" refers to a 5- to 18- membered full-carbon polycyclic group with two or more cyclic structures sharing one pair of carbon atoms, and one or more rings may contain one or more double bonds, but no ring has a fully conjugated $\pi$ electron aromatic system. Preferably, the fused cycloalkyl is 6- to 12- membered, and more preferably, the fused cycloalkyl is 7- to 10- membered. The fused cycloalkyl may be classified into bicyclic, tricyclic, tetracyclic or polycyclic fused cycloalkyl according to a number of constituent rings, is preferably bicyclic or tricyclic fused cycloalkyl, and is more preferably bicycloalkyl as 5 membered/5 membered or 5 membered/6 membered. Non-limiting embodiments of the "fused cycloalkyl" comprise, but are not limited to, bicyclo [3.1.0] hexyl, bicyclo [3.2.0] heptyl-1-alkenyl, bicyclo [3.2.0] heptyl, decahydronaphthyl or tetradecahydrophenanthryl.

"Bridged cycloalkyl" refers to a 5- to 18- membered full-carbon polycyclic group with two or more cyclic structures sharing two carbon atoms not directly connected with each other, and one or more rings may contain one or more double bonds, but no ring has a fully conjugated $\pi$ electron aromatic system. Preferably, the bridged cycloalkyl is 6- to 12- membered, and more preferably, the bridged cycloalkyl is 7- to 10- membered. Preferably, the bridged cycloalkyl is 6- to 14- membered, and more preferably, the bridged cycloalkyl is 7- to 10- membered . The bridged cycloalkyl may be classified into bicyclic, tricyclic, tetracyclic or polycyclic bridged cycloalkyl according to a number of constituent rings, is preferably the bicyclic, tricyclic or tetracyclic bridged cycloalkyl, and is more preferably the bicyclic or tricyclic bridged cycloalkyl. Non-limiting embodiments of the "bridged cycloalkyl" comprise, but are not limited to, (1s,4s)-bicyclo [2.2.1] heptyl, bicyclo [3.2.1] octyl, (1s,5s)-bicyclo [3.3.1] nonyl, bicyclo [2.2.2] octyl, and (1r,5r)-bicyclo [3.3.2] decyl.

"Heterocyclyl", "heterocycle" or "heterocyclic" may be used interchangeably in the present application, and all refers to non-aromatic heterocyclyl, wherein one or more ring-forming atoms are heteroatoms, such as oxygen, nitrogen and sulfur atoms, comprising a monocyclic ring, a fused ring, a bridged ring and a spirocyclic ring. Preferably, the heterocyclyl is a 5- to 7- membered monocyclic ring or a 7- to 10- membered bicyclic or tricyclic ring, which may contain 1, 2 or 3 atoms selected from nitrogen, oxygen and/or sulfur. Examples of the "heterocyclyl" comprise, but are not limited to, morpholinyl, oxetanyl, thiomorpholinyl, tetrahydropyranyl, 1,1-dioxothiomorpholinyl, piperidinyl, 2-oxopiperidinyl, pyrrolidinyl, 2-oxopyrrolidinyl, piperazine-2-one, 8-oxa-3-aza-bicyclo [3.2.1] octyl and piperazinyl. The heterocyclyl may be substituted or unsubstituted.

"Spiro-heterocyclyl" refers to a 5- to 18- membered polycyclic group with two or more cyclic structures, and single rings share one atom with each other, and the ring contains one or more double bonds, but no ring has a fully conjugated $\pi$ electron aromatic system, wherein one or more ring atoms are selected from heteroatoms of nitrogen, oxygen or $S(O)_r$ (wherein r is selected from 0, 1 or 2), and the remaining ring atoms are carbon. Preferably, the spiro-heterocyclyl is 6- to 14- membered, and more preferably, the spiro-heterocyclyl is 7- to 10- membered. The spiro-heterocyclyl is divided into mono-, di- or multi-spiro-heterocyclyl according to a number of shared spiro atoms between rings, is preferably the mono- and di-spiro-heterocyclyl, and is more preferably a 4 membered/4 membered, 4 membered/5 membered, 4 membered/6 membered, 5 membered/5 membered or 5 membered/6 membered mono-spiro-heterocyclyl. Non-limiting embodiments of the "spiro-heterocyclyl" comprise, but are not limited to, 1,7-dioxane [4.5] decyl, 2-oxa-7-azaspiro [4.4] nonyl, 7-oxaspiro [3.5] nonyl and 5-oxaspiro [2.4] heptyl.

"Fused heterocyclyl" refers to a full carbon polycyclic group with two or more cyclic structures sharing one pair of atoms with each other, and one or more rings may contain one or more double bonds, but no ring has a fully conjugated

$\pi$ electron aromatic system, wherein one or more ring atoms are selected from heteroatoms of nitrogen, oxygen or $S(O)_r$ (wherein r is selected from 0, 1 or 2), and the remaining ring atoms are carbon. Preferably, the fused heterocyclyl is 6- to 14- membered, and more preferably, the fused heterocyclyl is 7- to 10- membered. The fused heterocyclyl may be divided into bicyclic, tricyclic, tetracyclic or polycyclic fused heterocyclyl according to a number of constituent rings, is preferably the bicyclic or tricyclic fused heterocyclyl, and is more preferably a 5 membered /5 membered or 5 membered/6 membereds bicyclic fused heterocyclyl. Non-limiting embodiments of the "fused heterocyclyl" comprise, but are not limited to, octahydropyrrolo [3,4-c] pyrrolyl, octahydro-1H-isoindolyl, 3-azabicyclo [3.1.0] hexyl, and octahydrobenzo [b][1,4] dioxine.

"Bridged heterocyclyl" refers to a 5- to 14- membered or 5- to 18- membered polycyclic group with two or more cyclic structures sharing two atoms not directly connected with each other, and one or more rings may contain one or more double bonds, but no ring has a fully conjugated $\pi$ electron aromatic system, wherein one or more ring atoms are selected from heteroatoms of nitrogen, oxygen or $S(O)_r$ (wherein r is selected from 0, 1 or 2), and the remaining ring atoms are carbon. Preferably, the bridged heterocyclyl is 6- to 14- membered, and more preferably, the bridged heterocyclyl is 7- to 10-membered. The bridged heterocyclyl may be divided into bicyclic, tricyclic, tetracyclic or polycyclic bridged heterocyclyl according to a number of constituent rings, is preferably a bicyclic, tricyclic or tetracyclic bridged heterocyclyl, and is more preferably a bicyclic or tricyclic bridged heterocyclyl. Non-limiting embodiments of the "bridged heterocyclyl" comprise, but are not limited to, 2-azabicyclo [2.2.1] heptyl, 2-azabicyclo [2.2.2] octyl and 2-azabicyclo [3.3.2] decyl.

"Aryl" refers to a carbocyclic aromatic system containing one or two rings, wherein the rings may be connected together in a fused manner. The term "aryl" comprises monocyclic or bicyclic aryl, such as aromatic groups of phenyl, naphthyl and tetrahydronaphthyl. Preferably, the aryl is $C_6$-$C_{10}$ aryl, more preferably, the aryl is the phenyl and the naphthyl, and most preferably, the aryl is the naphthyl. The aryl may be substituted or unsubstituted.

"Heteroaryl" refers to a 5- to 6- membered aromatic monocyclic ring or a 8- to 10- membered aromatic bicyclic ring, which may contain 1 to 4 atoms selected from nitrogen, oxygen and/or sulfur. Preferably, the heteroaryl is bicyclic heteroaryl. Embodiments of the "heteroaryl" comprise, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothiophenyl, benzimidazolyl, indolyl, isoindolyl, 1,3-dioxo-isoindolyl, quinolyl, indazolyl, benzisothiazolyl, benzoxazolyl, benzisoxazolyl,

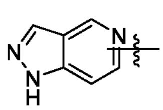 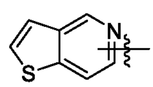 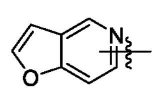 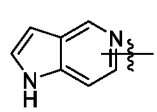 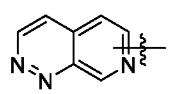 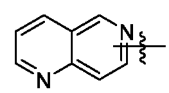

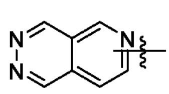 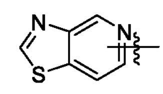 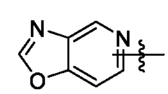 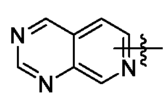 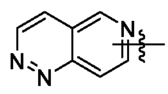 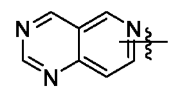

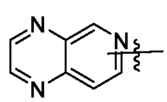 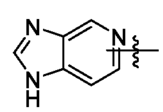 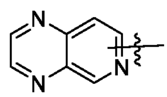 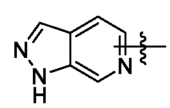 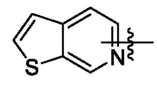 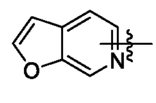

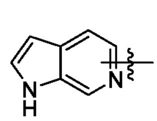 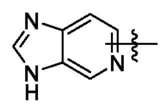 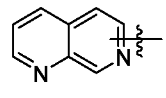 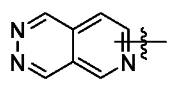 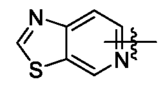 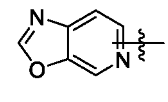

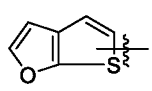 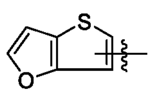 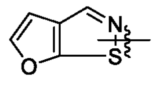 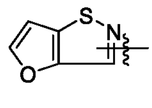 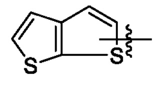 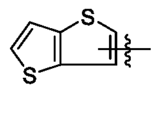

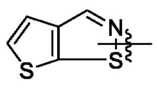 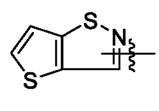 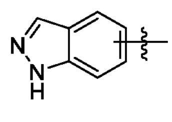 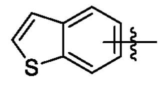 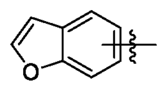 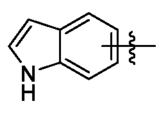

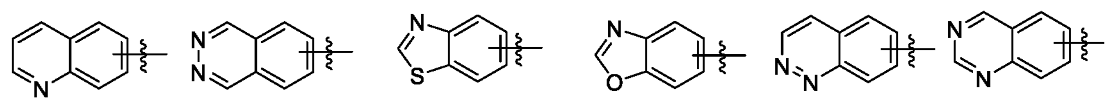

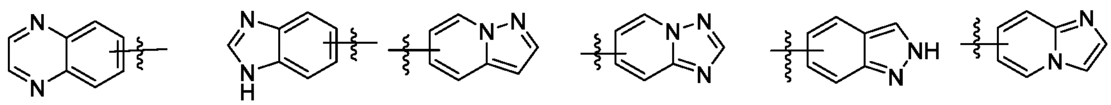

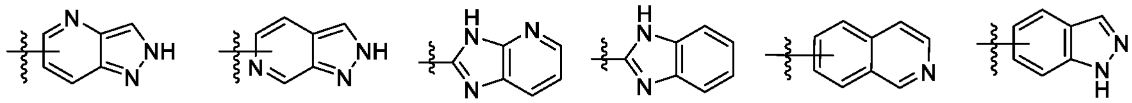

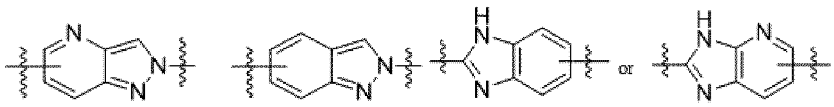

or

The heteroaryl may be substituted or unsubstituted.

"Fused ring" refers to a polycyclic group with two or more cyclic structures sharing one pair of atoms with each other, and one or more rings may contain one or more double bonds, but at least one ring does not have a fully conjugated $\pi$ electron aromatic system. Meanwhile, at least one ring has the fully conjugated $\pi$ electron aromatic system, wherein zero, one or more ring atoms are selected from heteroatoms of nitrogen, oxygen or $S(O)_r$ (wherein r is selected from 0, 1 or 2), and the remaining ring atoms are carbon. The fused ring preferably comprises a bicyclic or tricyclic fused ring, wherein the bicyclic fused ring is preferably a fused ring of aryl or heteroaryl and monocyclic heterocyclyl or monocyclic heterocyclyl. Preferably, the fused ring is 7- to 14- membered, and more preferably, the fused ring is 8- to 10- membered . Embodiments of the "fused ring" comprise, but are not limited to,

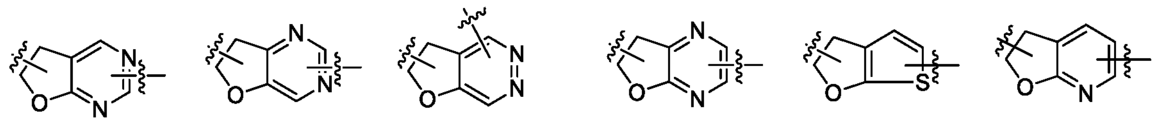

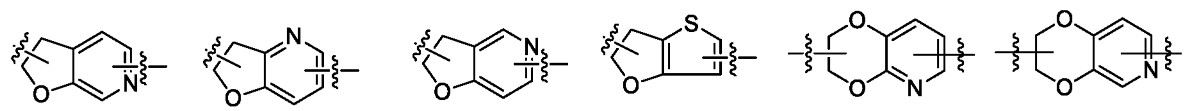

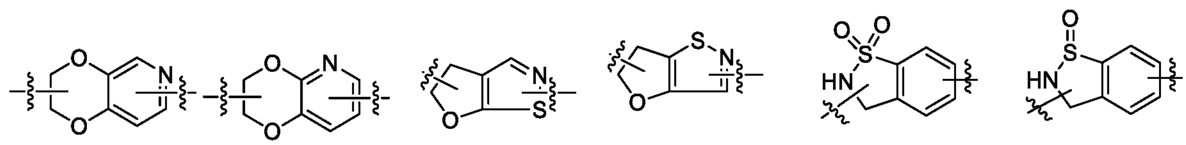

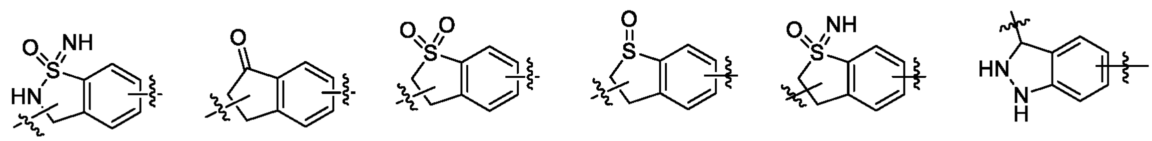

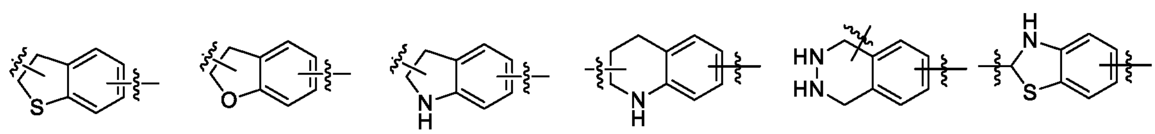

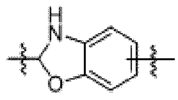 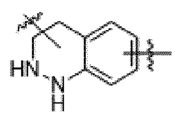 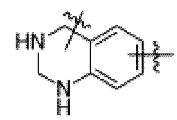 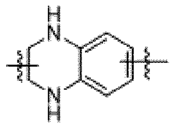 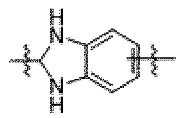 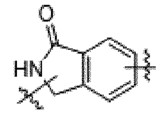

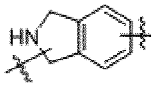 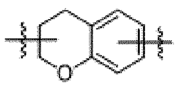 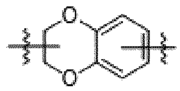 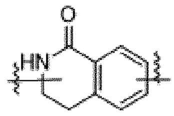 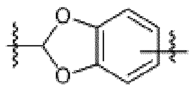 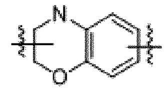

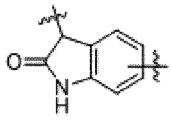 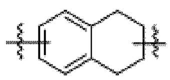 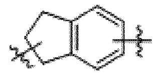 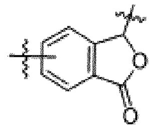 or 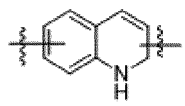 .

The fused ring may be substituted or unsubstituted.

"Alkoxy" refers to a group of (alkyl-O-). The alkyl is defined herein. $C_1$-$C_6$ alkoxy is preferably selected. Examples of the alkoxy comprise, but are not limited to, methoxy, ethoxy, n-propoxy, isopropoxy, n-butoxy, isobutoxy, tert-butoxy, and the like.

"Haloalkyl" refers to a group in which alkyl is optionally further substituted by one or more halogens, wherein the alkyl is defined herein.

"Hydroxyalkyl" refers to a group in which alkyl is optionally further substituted by one or more hydroxyls, wherein the alkyl is defined herein.

"Haloalkoxy" refers to a group in which alkyl of (alkyl-O-) is optionally further substituted by one or more halogens, wherein the alkoxy is defined herein.

"Hydroxyl" refers to a -OH group.

"Halogen" refers to fluorine, chlorine, bromine and iodine.

"Amino" refers to $-NH_2$.

"Cyano" refers to -CN.

"Nitro" refers to $-NO_2$.

"Benzyl" refers to $-CH_2$-phenyl.

"Carboxyl" refers to -C(O)OH.

"Carboxylate group" refers to -C(O)O-alkyl or -C(O)O-cycloalkyl, wherein the alkyl and the cycloalkyl are defined as above.

"Me" refers to methyl.

"DMSO" refers to dimethyl sulfoxide.

"Boc" refers to tert-butoxycarbonyl.

"T3P" refers to propylphosphonic anhydride.

"HATU" refers to 2-(7-azabenzotriazole)-N,N,N',N'-tetramethylurea hexafluorophosphate.

"Substituted" refers to that one or more hydrogen atoms, preferably at most 5 hydrogen atoms, and more preferably 1 to 3 hydrogen atoms, in a group, are independently substituted by a corresponding number of substituents. It goes without saying that the substituents are only in their possible chemical positions, and those skilled in the art can determine (by experiment or theory) possible or impossible substitutions without excessive efforts. For example, amino or hydroxyl with free hydrogen may be unstable when combined with carbon atoms with unsaturated (such as olefinic) bonds.

"Substitution" or "substituted" in the specification, unless otherwise specified, refers to that the group may be substituted by one or more groups selected from the following substituents: alkyl, alkenyl, alkynyl, alkoxy, alkyl sulphanyl, alkyl amino, halogen, sulfhydryl, hydroxyl, nitro, cyano, cycloalkyl, heterocyclyl, aryl, heteroaryl, cycloalkoxy, heterocycloalkoxy, cycloalkthiol, heterocycloalkthiol, amino, haloalkyl, hydroxyalkyl, carboxyl, carboxylate group, =O, $-C(O)R^9$, $-C(O)OR^9$, $-NHC(O)R^9$, $-NHC(O)OR^9$, $-NR^{10}R^{11}$, $-C(O)NR^{10}R^{11}$, $-CH_2NHC(O)OR^9$, $-CH_2NR^{10}R^{11}$ or $-S(O)_rR^9$.

$R^9$ is selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxyl, halogen, nitro, cyano, alkyl, alkoxy, haloalkyl, haloalkoxy, cycloalkyl, heterocyclyl, aryl, heteroaryl, =O, $-C(O)R^{12}$, $-C(O)OR^{12}$, $-OC(O)R^{12}$, $-NR^{13}R^{14}$, $-C(O)NR^{13}R^{14}$, $-SO_2NR^{13}R^{14}$ or $-NR^{13}C(O)R^{14}$.

$R^{10}$ and $R^{11}$ are independently selected from hydrogen atom, hydroxyl, halogen, alkyl, alkoxy, cycloalkyl, heterocyclyl, aryl or heteroaryl, wherein the alkyl, alkoxy, cycloalkyl, heterocyclyl, aryl or heteroaryl is optionally further

substituted by one or more substituents selected from hydroxyl, halogen, nitro, cyano, alkyl, alkoxy, cycloalkyl, heterocyclyl, aryl, heteroaryl, =O, -C(O)$R^{12}$, -C(O)O$R^{12}$, - OC(O)$R^{12}$, -N$R^{13}R^{14}$, -C(O)N$R^{13}R^{14}$, -$SO_2$N$R^{13}R^{14}$ or -N$R^{13}$C(O)$R^{14}$.

Alternatively, $R^{10}$ and $R^{11}$ form a 4- to 8- membered heterocyclyl together with atoms to which $R^{10}$ and $R^{11}$ are connected, wherein the 4- to 8- membered heterocyclyl contains one or more N, O or $S(O)_r$, and the 4- to 8- membered heterocyclyl is optionally further substituted by one or more substituents selected from hydroxyl, halogen, nitro, cyano, alkyl, alkoxy, cycloalkyl, heterocyclyl, aryl, heteroaryl, =O, -C(O)$R^{12}$, -C(O)O$R^{12}$, -OC(O)$R^{12}$, -N$R^{13}R^{14}$, -C(O)N$R^{13}R^{14}$, -$SO_2$N$R^{13}R^{14}$ or - N$R^{13}$C(O)$R^{14}$.

$R^{12}$, $R^{13}$ and $R^{14}$ are independently selected from hydrogen atom, alkyl, amino, cycloalkyl, heterocyclyl, aryl or heteroaryl, wherein the alkyl, cycloalkyl, heterocyclyl, aryl or heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or carboxylate.

r is 0, 1 or 2.

The compound of the present invention may contain an asymmetric center or a chiral center, thus existing in different stereoisomeric forms. It is expected that all stereoisomeric forms of the compound of the present invention comprise, but are not limited to, a diastereomer, an enantiomer, and an atropisomer and a geometric (conformational) isomer, and a mixture thereof, such as a racemic mixture, which are all within the scope of the present invention.

Unless otherwise specified, the structure described in the present invention further comprises all isomers of this structure (such as forms of a diastereomer, an enantiomer, and an atropisomer and a geometric (conformational) isomer; such as Rand S configurations of asymmetric centers, (Z) and (E) double-bond isomers, and (Z) and (E) conformational isomers). Therefore, a single stereoisomer, and an enantiomeric mixture, a diastereomeric mixture and a geometric (conformational) isomer mixture of the compound of the present invention are all within the scope of the present invention.

"Pharmaceutically acceptable salt" refers to some salts of the above compound capable of maintaining original biological activity and suitable for medical use. The pharmaceutically acceptable salt of the compound represented by Formula (I) may be a metal salt or an amine salt formed with a suitable acid.

"Pharmaceutical composition" refers to a mixture containing one or more compounds described herein or their physiologically acceptable salts or prodrugs and other chemical components, and other components such as physiologically acceptable carriers and excipients. The purpose of the pharmaceutical composition is to promote the administration to organisms, which is beneficial for the absorption of active ingredients, thus exerting biological activity.

Method for synthesizing the compounds of the present invention

In order to achieve the objects of the present invention, the following technical solutions are adopted by the present invention.

A preparation method for a compound represented by general formula (II) or a stereoisomer, a tautomer, a pharmaceutically acceptable salt or a prodrug thereof according to the present invention, comprises the following steps of:

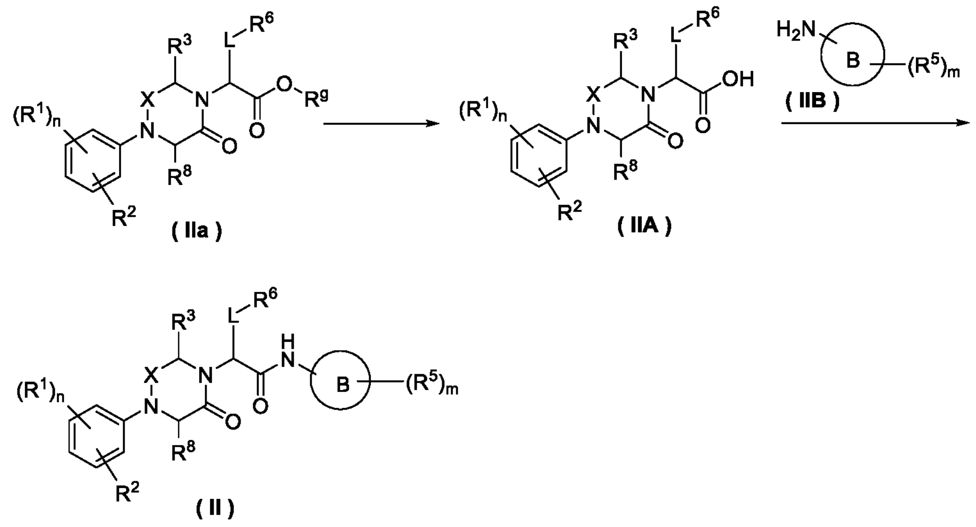
(IIa) (IIA) (IIB) (II)

subj ecting the compound of general formula (IIa) to hydrolysis to obtain the compound of general formula (IIA); and subjecting the compound of general formula (IIA) and the compound of general formula (IIB) to a condensation reaction,

and optionally further hydrolyzing the mixture under acidic conditions to obtain the compound of general formula (II); wherein:

$R^8$ is selected from alkyl; and
ring B, X, L, $R^1$ to $R^3$, $R^5$, $R^6$, $R^8$, m and n are as defined in general formula (II).

## DETAILED DESCRIPTION

The following examples are used to further describe the present invention, but these examples do not limit the scope of the present invention.

## Examples

The examples show the preparation of representative compounds represented by formula (I) and related structural identification data. It should be noted that the following examples are only used to illustrate the present invention, but not to limit the present invention. $^1H$ NMR spectrum was measured by Bruker instrument (400 MHz), and chemical shift was expressed in ppm, employing tetramethylsilane internal referebce (0.00 ppm). $^1H$ NMR was expressed as follows: s = singlet, d = doublet, t = triplet, m = multiplet, br = broadened, dd = doublet of doublets, and dt = doublet of triplets. If a coupling constant was provided, it was in the unit of Hz.
A mass spectrum was determined by LC/MS, and an ionization method may be ESI or APCI.
For preparative liquid separation, Gilson preparative high performance liquid chromatograph with a model number of GX-281, was used.
Yantai Huanghai HSGF254 or Qingdao GF254 silica gel plate was used as silica gel plates for thin layer chromatography. The silica gel plates used for thin layer chromatography (TLC) had a specification of 0.15 mm to 0.2 mm, and products separated and purified by TLC had a specification of 0.4 mm to 0.5 mm.
Yantai Huanghai silica gel with 200-300 meshes was used as a carrier for column chromatography.
In the following examples, unless otherwise specified, all temperatures were in Celsius. Unless otherwise specified, various starting materials and reagents were commercially available or synthesized according to known methods, and the commercially available materials and reagents were directly used without further purification. Unless otherwise specified, the commercially available manufacturers included but were not limited to Shanghai Accela ChemBio Inc., Shanghai Hao Hong Biomedical Technology Co., Ltd., Shanghai Bide Pharmatech Ltd., Sa'en Chemical Technology Co., Ltd., and Shanghai LinkChem Co., Ltd.
$CD_3OD$: Deuterated methanol.
$CDCl_3$: Deuterated chloroform.
DMSO-$d_6$: Deuterated dimethyl sulfoxide.
Unless otherwise specified in the examples, a solution in the reaction referred to an aqueous solution.
The compounds were purified by an eluent system of column chromatography and thin layer chromatography, wherein the system was selected from: A: petroleum ether and ethyl acetate system; B: dichloromethane and methanol system; C: dichloromethane and ethyl acetate system; and D: dichloromethane and ethanol system. The volume ratios of the solvents varied according to the polarity of the compounds, and may also be adjusted by adding a small amount of acidic or basic reagents, such as acetic acid or triethylamine, or the like.
Room temperature: 20°C to 30°C.

Example 1

*Tert*-butyl 4-(2-(4-(5-chloro-2-(1*H*-tetrazole-1-yl)phenyl)-2,5-dioxopiperazin-1-yl)-3-phenylpropanamido) benzoate

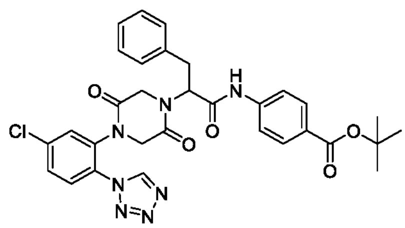

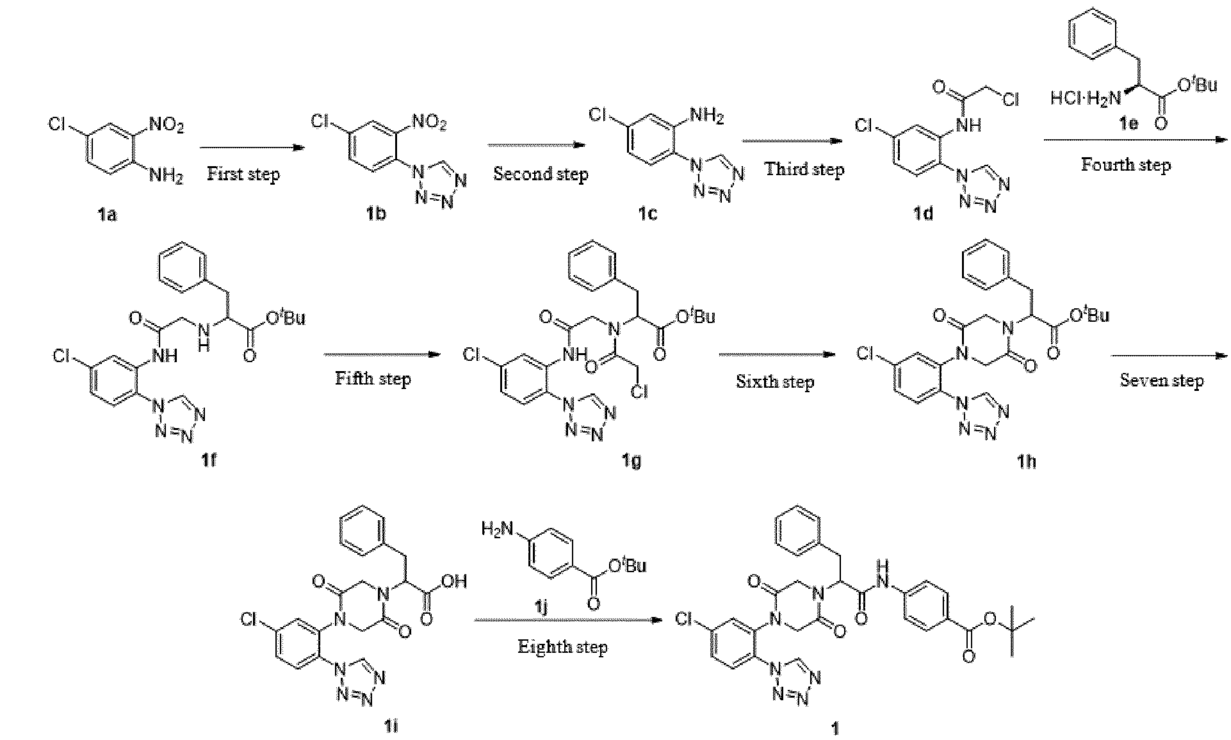


Step 1

1-(4-Chloro-2-nitrophenyl)-1*H*-tetrazole

4-Chloro-2-nitroaniline 1a (10 g, 57.95 mmol), azidotrimethylsilane (29.1 mL, 173.84 mmol) and trimethyl orthoformate (8.62 mL, 173.84 mmol) were added into acetic acid (300 mL) in turn, and were reacted under stirring at 60°C for 28 hours. After the reaction was completed, saturated sodium nitrite solution (200 mL) was slowly added dropwise at 0°C to quench the reaction, and the reaction solution was extracted with ethyl acetate (200 mL×3). Organic phases were combined, washed with water (100 mL) and saturated sodium chloride solution (100 mL) in turn, dried with anhydrous sodium sulfate, filtered and concentrated under reduced pressure, and the obtained residue was purified by silica gel column chromatography (eluent: system A) to obtain 1-(4-chloro-2-nitrophenyl)-1*H*-tetrazole 1b (8 g) with ayield of 36%.
MS m/z (ESI): 226.0 [M+H]

Step 2

5-Chloro-2-(1*H*-tetrazol-1-yl)aniline

1- (4-Chloro-2-nitrophenyl)- l/7-tetrazole 1b (0.5 g, 2.22 mmol), ammonium chloride (124.12 mg, 2.22 mmol) and reduced iron powder (1.24 g, 22.16 mmol) were dissolved in absolute ethanol (10 mL) in turn, and then added with water (2 mL), and stirred for reaction at 80°C for 2 hours. After the reaction was completed, the system was filtered, the filtrate was concentrated under reduced pressure, and the obtained residue was further separated and purified by silicagel column chromatography (eluent: system A) to obtain 5-chloro-2-(1*H*-tetrazol-1-yl)aniline 1c (0.4 g) with a yield of 92%.
MS m/z (ESI): 196.1 [M+H]

Step 3

2-Chloro-*N*-(5-chloro-2-(1*H*-tetrazole-1-yl)phenyl)acetamide

5-Chloro-2-(1*H*-tetrazol-1-yl)aniline 1c (450 mg, 2.30 mmol) and potassium carbonate (317.47 mg, 2.30 mmol) were dissolved in acetonitrile (15 mL) in turn, slowly added dropwise with acetonitrile solution (15 mL) containing chloroacetyl chloride (0.18 mL, 2.30 mmol) at 0°C, and continuously stirred for 2 hours at 0°C. After quenching with water, the reaction solution was extracted with ethyl acetate (50 mL×3). Organic phases were combined, washed with water

(20 mL) and saturated sodium chloride solution (20 mL) in turn, dried with anhydrous sodium sulfate, filtered and concentrated under reduced pressure, and the obtained residue was further separated and purified by silicagel column chromatography (eluent: system A) to obtain 2-chloro-*N*-(5-chloro-2-(1*H*-tetrazol-1-yl phenyl)acetamide 1d (0.57 g) with ayield of 91%.
MS m/z (ESI): 272.0 [M+H]

Step 4

Tert-butyl (2-((5-chloro-2-(1*H*-tetrazol-1-yl)phenyl)amino)-2-oxoethyl)phenylalaninate

2-Chloro-*N*-(5-chloro-2-(1*H*-tetrazol-1-yl phenyl)acetamide 1d (0.9 g, 3.31 mmol) and tert-butyl 3-phenyl-L-alaninate hydrochloride 1e (1.10 g, 4.96 mmol) were dissolved in N,N-dimethylformamide (100 mL), added with N,N-diisopropylethylamine (547 μL, 3.31 mmol), and reacted at 70°C for 18 hours. After the reaction was completed, the reaction solution was concentrated under reduced pressure. After most solvents were removed, water (150mL) was added for dilution, the reaction solution was extracted with ethyl acetate (100 mL×3). Organic phases were combined, washed with water (100 mL×2) and saturated sodium chloride solution (50 mL), dried with anhydrous sodium sulfate, filtered and concentrated under reduced pressure, and the obtained residue was purified by silica gel column chromatography (eluent: system A) to obtain *tert*-butyl (2-((5-chloro-2-(1*H* tetrazole-1-yl)phenyl)amino)-2-oxoethyl)phenylalaninate 1f (1.2 g) with a yield of 79%.
MS m/z (ESI): 457.1 [M+H]

Step 5

*Tert*-butyl *N*-(2-((5-chloro-2-(1*H*-tetrazol-1-yl)phenyl)amino)-2-oxoethyl)-*N*-(2-chloroacetyl)phenylalaninate

*Tert*-butyl (2-((5-chloro-2-(1*H*-tetrazol-1-yl)phenyl)amino)-2-oxoethyl)phenylalaninate 1f (1.2 g, 2.63 mmol) was dissolved in acetonitrile (20 mL), added with potassium carbonate (544.46 mg, 3.94 mmol), slowly dropwise added with chloroacetyl chloride (0.25 mL, 3.15 mmol) at 0°C, and reacted for 3 hours at 0°C. Water (20 mL) was added to quench the reaction, and then the reaction solution was stirred for 10 minutes for liquid separation. An aqueous phase was extracted with ethyl acetate (30 mL×2). Organic phases were combined, dried with anhydrous sodium sulfate, filtered, and concentrated under reduced pressure, and the obtained residue was further separated and purified by silicagel column chromatography (eluent: system A) to obtain *tert*-butyl *N*-(2-((5-chloro-2-(1*H*-tetrazol-1-yl)phenyl)amino)-2-oxoethyl)-*N*-(2-chloroacetyl)phenylalaninate 1g (1 g) with a yield of 72%.
MS m/z (ESI): 477.0 [M+H-56]

Step 6

*Tert*-butyl 2-(4-(5-chloro-2-(1*H*-tetrazol-1-yl)phenyl)-2,5-dioxopiperazin-1-yl)-3-phenylpropanoate

*Tert-butyl* N-(2-((5-chloro-2-(1*H*-tetrazol-1-yl)phenyl)amino)-2-oxoethyl)-N-(2-chloroacetyl) phenylalaninate 1g (0.4 g, 0.75 mmol) was dissolved in methanol (20 mL), added with sodium methylate (0.14 mL, 5.4 M methanol solution, 0.75 mmol) at 0°C, and reacted for 2 hours at 0°C. After the reaction was completed, 2N hydrochloric acid aqueous solution was added to adjust the pH to neutrality, then the reaction solution was concentrated under reduced pressure, and the obtained residue was further separated and purified by silica gel column chromatography (eluent: system A) to obtain *tert*-butyl 2-(4-(5-chloro-2-(1*H*-tetrazol-1-yl)phenyl)-2,5-dioxopiperazin-1-yl- 3-phenylpropanoate 1h (0.2 g) with a yield of 54%.
MS m/z (ESI): 497.1 [M+H]

Step 7

2-(4-(5-Chloro-2-(1*H*-tetrazol-1-yl)phenyl)-2,5-dioxopiperazin-1-yl)-3-phenylpropanoic acid

*Tert-butyl* 2-(4-(5-chloro-2-(1*H*-tetrazol-1-yl)phenyl)-2,5-dioxopiperazin-1-yl)-3-phenylpropanoate 1h (200 mg, 402.46 μmol) was dissolved in dichloromethane (5 mL), added with trifluoroacetic acid (0.1 mL), and reacted at room temperature for 4 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-3-phenylpropanoic acid 1i (144 mg) with a yield of 81%.

MS m/z (ESI): 441.1 [M+H]

Step 8

*Tert*-butyl 4-(2-(4-(5-chloro-2-(1*H*-tetrazol-1-yl)phenyl)-2,5-dioxopiperazin-1-yl)-3-phenylpropanamido)benzoate

2-(4-(5-Chloro-2-(1*H*-tetrazol-1-yl)phenyl)-2,5-dioxopiperazin-1-yl)-3-phenylpropionic acid 1i (140 mg, 317.58 μmol) and tert-butyl 4-aminobenzoate 1j (92.05 mg, 476.36 μmol) were dissolved in ethyl acetate (10 mL), added with N,N-diisopropylethylamine (0.26 mL, 1.59 mmol) and propylphosphonic anhydride (404 mg,635.15 μmol, 50% ethyl acetate solution), and reacted at 60°C for 12 hours. After the reaction was completed, the reaction solution was concentrated under reduced pressure, and the obtained residue was further separated and purified by silicagel column chromatography (eluent: system A) to obtain *tert*-butyl 4-(2-(4-(5-chloro-2-(1*H*-tetrazol-1-yl)phenyl)-2,5-dioxopiperazin-1-yl)-3-phenylpropanamido) benzoate 1 (190 mg) with a yield of 97%.

MS m/z (ESI): 615.9 [M+H]
$^1$H NMR (400 MHz, DMSO-$d_6$) δ 10.48 (s, 1H), 9.71 (s, 1H), 7.97-7.60 (m, 7H), 7.40-7.16 (m, 5H), 5.41-5.27 (m, 1H), 4.76-3.87 (m, 4H), 3.33-3.17 (m, 1H), 3.16-3.04 (m, 1H), 1.54 (s, 9H).

Example 2

Methyl (4-(2-(4-(5-chloro-2-(1*H*-tetrazol-1-yl)phenyl)-2,5-dioxopiperazin-1-yl)-3-phenylpropanamido)phenyl)carbamate

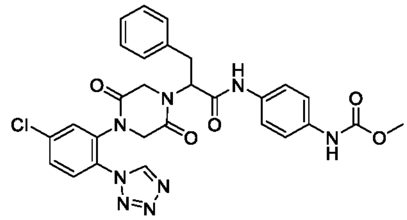

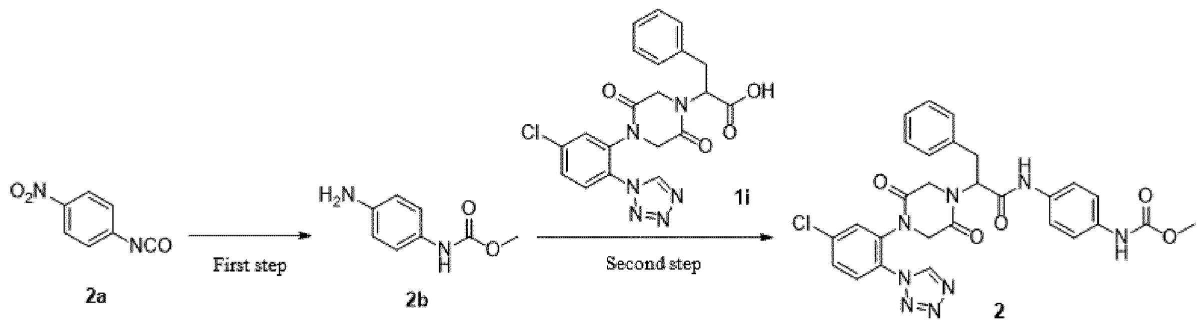


Step 1

Methyl (4-aminophenyl) carbamate

In hydrogen atmosphere, 1-isocyanato-4-nitrobenzene 2a (3 g, 18.28 mmol) was dissolved in dichloromethane (50 mL), added with raney nickel (0.5 g, 18.28 mmol), and reacted at room temperature for 4 hours. After the reaction was completed, the system was filtered, the filtrate was concentrated under reduced pressure, and the obtained residue was further separated and purified by silicagel column chromatography (eluent: system A) to obtain methyl (4-aminophenyl)carbamate 2b (3 g) with a yield of 99%.
MS m/z (ESI): 167.0 [M+H]

Step 2

Methyl (4-(2-(4-(5-chloro-2-(1*H*-tetrazol-1-yl)phenyl)-2,5-dioxopiperazin-1-yl)-3-phenylpropanamido)phenyl) carbamate

2-(4-(5-Chloro-2-(1*H*-tetrazol-1-yl)phenyl)-2,5-dioxopiperazine-1-yl)-3-phenylpropanoic acid 1i (100 mg, 226.84 μmol) and methyl (4-aminophenyl)carbamate 2b (56.54 mg, 340.26 μmol) were dissolved in ethyl acetate (20 mL), added with N,N-diisopropylethylamine (187 μL, 1.13 mmol) and propylphosphonic anhydride (433 mg, 680.52 μmol, 50% ethyl acetate solution), and reacted at room temperature for 5 hours. After the reaction was completed, the reaction solution was concentrated under reduced pressure, and the obtained residue was further separated and purified by silicagel column chromatography (eluent: system B) to obtain methyl (4-(2-(4-(5-chloro-2-(1*H*-tetrazol-1-yl)phenyl)-2,5-dioxopiperazin-1-yl)-3-phenylpropanamido)phenyl) carbamate 2 (81 mg) with a yield of 60%.

MS m/z (ESI): 589.1[M+H]

$^1$H NMR (400 MHz, DMSO-$d_6$) δ 10.10 (s, 1H), 9.71 (s, 1H), 9.59 (s, 1H), 7.86-7.64 (m, 3H), 7.48 (d, J = 8.6 Hz, 2H), 7.39 (d, J = 8.5 Hz, 2H), 7.36-7.16 (m, 5H), 5.37-5.23 (m, 1H), 4.64-3.88 (m, 4H), 3.65 (s, 3H), 3.28-3.13 (m, 1H), 3.13-2.96 (m, 1H).

Example 3

4-(2-(4-(5-Chloro-2-(1*H*-tetrazol-1-yl)phenyl)-2,5-dioxopiperazin-1-yl)-3-phenylpropanami do)benzamide

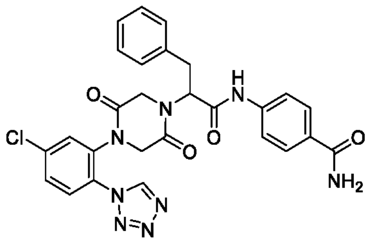

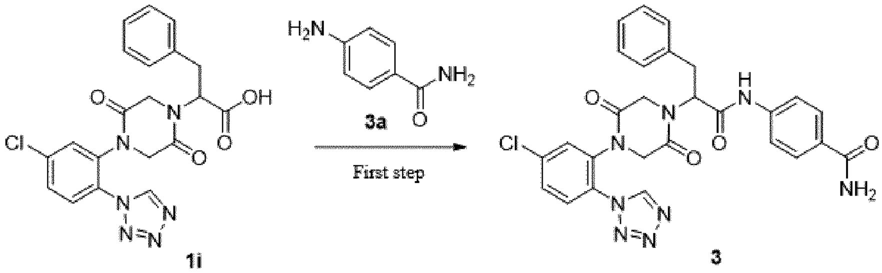


1i 3

2-(4-(5-Chloro-2-(1*H*-tetrazol-1-yl)phenyl)-2,5-dioxopiperazin-1-yl)-3-phenylpropanoic acid 1i (100 mg, 226.84 μmol) and p-aminobenzamide 3a (46.33 mg, 340.26 μmol) were dissolved in ethyl acetate (10 mL), added with N,N-diisopropylethylamine (187 μL, 1.13 mmol) and propylphosphonic anhydride (433 mg, 680.52 μmol, 50% ethyl acetate solution), and reacted at room temperature for 50 hours. After the reaction was completed, the reaction solution was concentrated under reduced pressure, and the obtained residue was further separated and purified by silicagel column chromatography (eluent: system B) to obtain 4-(2-(4-(5-chloro-2-(1*H*-tetrazol-1-yl)phenyl)-2,5-dioxopiperazin-1-yl)-3-phenylpropanamido)benzamide 3 (69 mg) with a yield of 53%.

MS m/z (ESI): 559.1 [M+H]

$^1$H NMR (400 MHz, DMSO-$d_6$) δ 10.38 (s, 1H), 9.71 (s, 1H), 7.96-7.71 (m, 5H), 7.65 (d, J = 8.4 Hz, 2H), 7.40-7.12 (m, 5H), 5.41-5.26 (m, 1H), 4.78-3.89 (m, 4H), 3.28-3.18 (m, 1H), 3.15-3.02 (m, 1H).

Example 4

Ethyl 4-(2-(4-(5-chloro-2-(1*H*-tetrazol-1-yl)phenyl)-2,5-dioxopiperazin-1-yl)-3-phenylpropanamido) benzoate

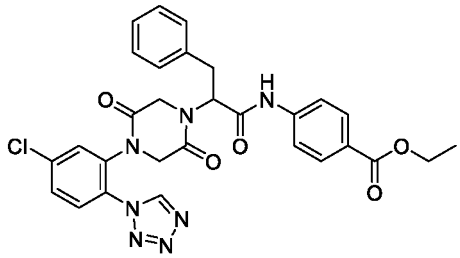

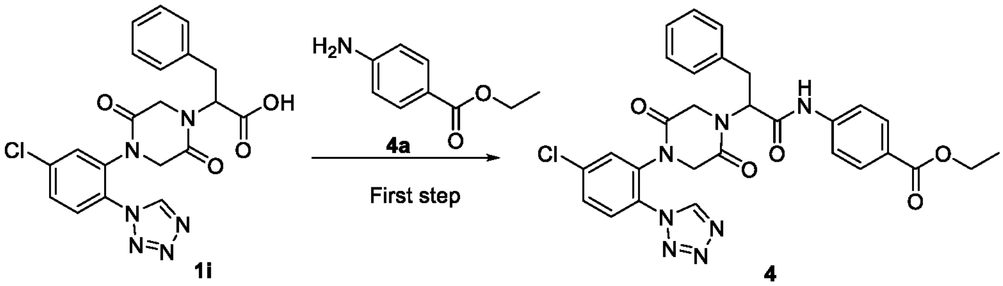


1i 4

2-(4-(5-Chloro-2-(1*H*-tetrazol-1-yl)phenyl)-2,5-dioxopiperazin-1-yl)-3-phenylpropanoic acid 1i (50 mg, 113.42 μmol) and ethyl p-ainobenzoate 4a (28.10 mg, 170.13 μmol) were dissolved in ethyl acetate (10 mL), added with N,N-diisopropylethylamine (94 μL, 567.10 μmol) and propylphosphonic anhydride (217 mg, 340.26 μmol, 50% ethyl acetate solution), and reacted at 60°C for 2 hours. After the reaction was completed, the reaction solution was concentrated under reduced pressure, and the obtained residue was further separated and purified by silicagel column chromatography (eluent: system A) to obtain ethyl 4-(2-(4-(5-chloro-2-(1*H*-tetrazol-1-yl)phenyl-2,5-dioxopiperazin-1-yl)-3-phenylpropanamido) benzoate 4 (52 mg) with a yield of 73%.

MS m/z (ESI): 587.9 [M+H]

$^1$H NMR (400 MHz, DMSO-$d_6$) δ 10.51 (s, 1H), 9.71 (s, 1H), 7.94 (d, J = 8.4 Hz, 2H), 7.87-7.64 (m, 5H), 7.41-7.15 (m, 5H), 5.40-5.27 (m, 1H), 4.68-3.89 (m, 4H), 4.29 (q, J = 7.1 Hz, 2H), 3.28-3.18 (m, 1H), 3.18-3.03 (m, 1H), 1.32 (t, J = 7.1 Hz, 3H).

Example 5

Methyl 4-(2-(4-(5-Chloro-2-(1*H*-tetrazol-1-yl)phenyl)-2,5-dioxopiperazin-1-yl)-3-phenylpropanamido)benzoate

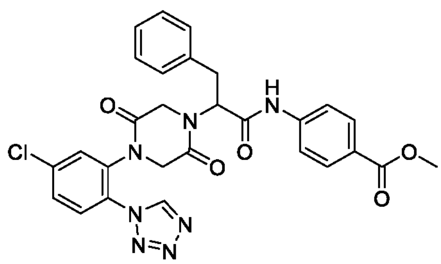

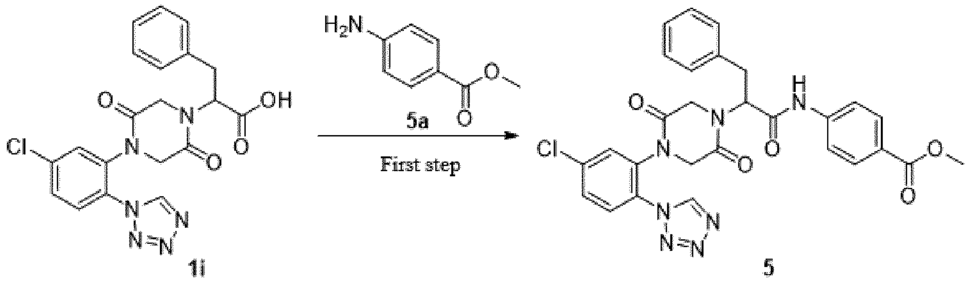


1i 5

2-(4-(5-Chloro-2-(1*H*-tetrazol-1-yl)phenyl)-2,5-dioxopiperazin-1-yl)-3-phenylpropanoic acid 1i (50 mg, 113.42 μmol) and methyl p-aminobenzoate 5a (25.72 mg, 170.13 μmol) were dissolved in ethyl acetate (6 mL), added with N,N-diisopropylethylamine (94 μL, 567.10 μmol) and propylphosphonic anhydride (217 mg, 340.26 μmol, 50% ethyl acetate solution), and reacted at 60°C for 2 hours. After the reaction was completed, the reaction solution was concentrated under reduced pressure, and the obtained residue was further separated and purified by silicagel column chromatography (eluent: system A) to obtain methyl 4-(2-(4-(5-chloro-2-(1*H*-tetrazol-1-yl)phenyl)-2,5-dioxopiperazin-1-yl)-3-phenylpropanamido) benzoate 5 (50 mg) with a yield of 76%.

MS m/z (ESI): 574.2 [M+H]

$^1$H NMR (400 MHz, DMSO-$d_6$) δ 10.51 (s, 1H), 9.71 (s, 1H), 7.94 (d, J = 8.4 Hz, 2H), 7.87-7.62 (m, 5H), 7.44-7.13 (m, 5H), 5.41-5.26 (m, 1H), 4.62-3.90 (m, 4H), 3.83 (s, 3H), 3.28-3.20 (m, 1H), 3.16-3.03 (m, 1H).

Example 6

4-(2-(4-(5-Chloro-2-(1*H*-tetrazol-1-yl)phenyl)-2,5-dioxopiperazin-1-yl)-3-phenylpropanamido)-*N*-methylbenzamide

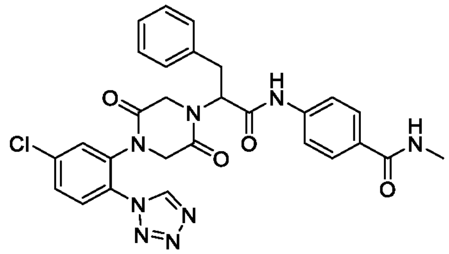

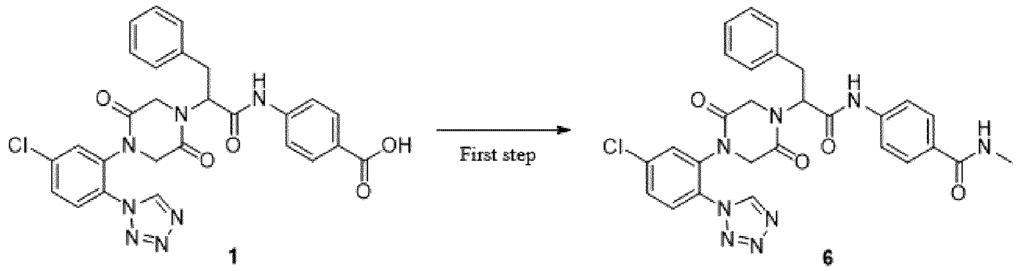


1 6

4-(2-(4-(5-Chloro-2-(1*H*-tetrazol-1-yl)phenyl)-2,5-dioxopiperazin-1-yl)-3-phenylpropanamido)benzoic acid 1 (60 mg, 107.15 μmol) and methylamine hydrochloride (14.5 mg, 214.30 μmol) were dissolved in ethyl acetate (6 mL), added with N,N-diisopropylethylamine (89 μL, 535.75 μmol) and propylphosphonic anhydride (205 mg, 321.45 μmol, 50% ethyl acetate solution), and reacted at 60°C for 2 hours. After the reaction was completed, the reaction solution was concentrated under reduced pressure, and the obtained residue was further separated and purified by silicagel column chromatography (eluent: system B) to obtain 4-(2-(4-(5-chloro-2-(1*H*-tetrazol-1-yl)phenyl)-2,5-dioxopiperazin-1-yl)-3-phenylpropanamido)-N-methylbenzamide 6 (14 mg) with a yield of 22%.

MS m/z (ESI): 573.2 [M+H]

$^1$H NMR (400 MHz, DMSO-$d_6$) δ 10.40 (s, 1H), 9.70 (s, 1H), 8.46-8.26 (m, 1H), 7.99-7.52 (m, 7H), 7.44-7.11 (m,

5H), 5.45-5.19 (m, 1H), 4.64-3.87 (m, 4H), 3.15-2.97 (m, 2H), 2.76 (d, J= 4.4 Hz, 3H).

Example 7

2-(4-(5-Chloro-2-(1*H*-tetrazol-1-yl)phenyl)-2,5-dioxopiperazine-1-yl)-*N*-(1-oxoisoindolin-5-yl)-3-phenylpropanamide

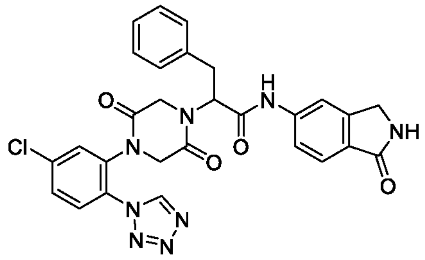

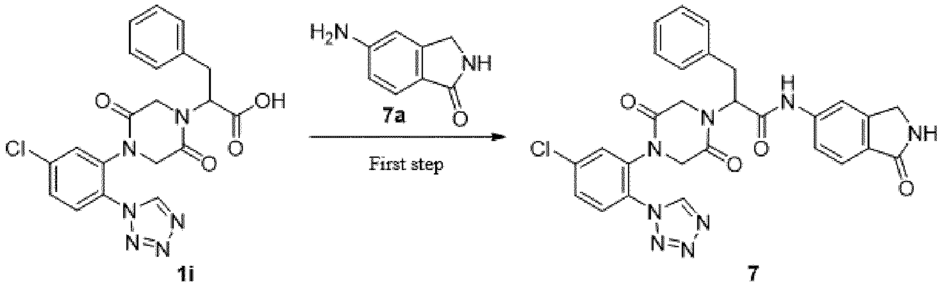


1i

7

2-(4-(5-Chloro-2-(1*H*-tetrazol-1-yl)phenyl)-2,5-dioxopiperazin-1-yl)-3-phenylpropanoic acid 1i (50 mg, 113.42 μmol) and 5-aminoisoindolin-1-one 7a (25.21 mg, 170.13 μmol) were dissolved in ethyl acetate (6 mL), added with N,N-diisopropylethylamine (112 μL, 680.52 μmol) and propylphosphonic anhydride (217 mg, 340.26 μmol, 50% ethyl acetate solution), and reacted at 70°C for 4 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-N-(1-oxoisoindolin-5-yl)-3-phenylpropanamide 7 (21 mg) with a yield of 32%. MS m/z (ESI): 570.9 [M+H]
$^1$H NMR (400 MHz, DMSO-$d_6$) δ 10.48 (s, 1H), 9.73 (s, 1H), 8.43 (s, 1H), 7.94 (s, 1H), 7.87-7.72 (m, 3H), 7.67-7.53 (m, 2H), 7.38-7.19 (m, 5H), 5.41-5.29 (m, 1H), 4.60-3.90 (m, 4H), 4.35 (s, 2H), 3.31-3.02 (m, 2H).

Example 8

Methyl 5-(2-(4-(5-chloro-2-(1*H*-tetrazol-1-yl)phenyl)-2,5-dioxopiperazin-1-yl)-3-phenylpropanamido)indoline-1 -carboxylate

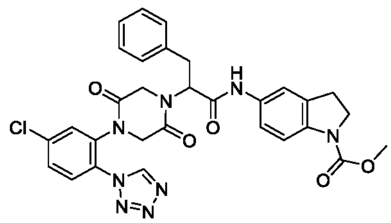

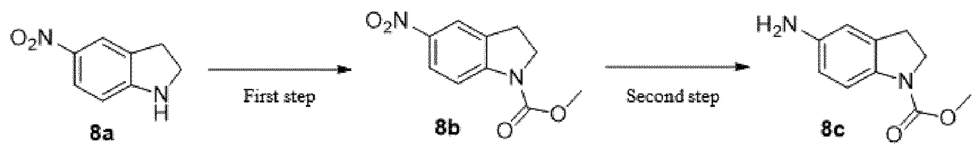

8a 8b 8c

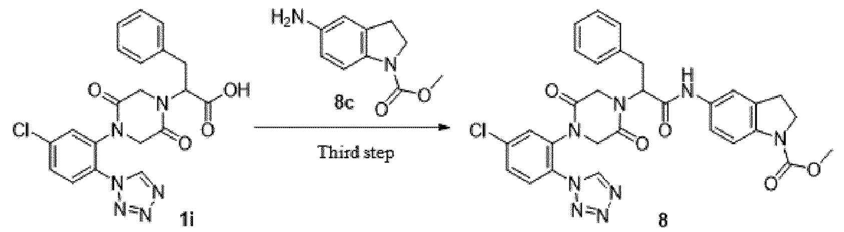

1i 8c 8

Step 1

Methyl 5-nitroindoline-1-carboxylate

5-Nitroindoline 8a (1 g, 6.09 mmol) was dissolved in dichloromethane (12.5 mL), respectively added with toluene solution (12.5 mL) containing methyl chloroformate (863.45 45 mg, 9.14 mmol) and dichloromethane solution (12.50 mL) containing pyridine (493 μL, 6.09 mmol), and stirred at 80°C for reaction for 3 hours. After the reaction was completed, the reaction solution was concentrated under reduced pressure, and the obtained residue was further separated and purified by silicagel column chromatography (eluent: system A) to obtain methyl 5-nitroindoline-1-carboxylate 8b (1.2 g) with a yield of 89%.
MS m/z (ESI): 223.0 [M+H]

Step 2

Methyl 5-aminoindoline-1-carboxylate

Methyl 5-nitroindoline-1-carboxylate 8b (0.67 g, 3.02 mmol) was dissolved in ethanol (10 mL) and water (1 mL), sequentially added with reduced iron powder (1.68 g, 30.15 mmol) and ammonium chloride (159.81 mg, 3.02 mmol), and reacted under stirring at 80°C for 1 hour. After the reaction was completed, the reaction solution was filtered, the filtrate was concentrated under reduced pressure, and the obtained residue was further separated and purified by silicagel column chromatography (eluent: system B) to obtain methyl 5-aminoindoline-1-carboxylate 8c (0.42 g) with a yield of 72%.
MS m/z (ESI): 193.1 [M+H]

Step 3

Methyl 5-(2-(4-(5-chloro-2-(1*H*-tetrazol-1-yl)phenyl)-2,5-dioxopiperazine-1-yl)-3-phenylpropanamido) indoline-1-carboxylate

2-(4-(5-Chloro-2-(1*H*-tetrazol-1-yl)phenyl)-2,5-dioxopiperazin-1-yl)-3-phenylpropnoic acid 1i (50 mg, 113.42 μmol) and methyl 5-aminoindoline-1-carboxylate 8c (32.70 mg, 170.13 μmol) were dissolved in ethyl acetate (10 mL), added with N,N-diisopropylethylamine (94 μL, 567.10 μmol) and propylphosphonic anhydride (217 mg, 340.26 μmol, 50% ethyl acetate solution), and reacted at room temperature for 24 hours. After the reaction was completed, the reaction solution was concentrated under reduced pressure, and the obtained residue was further separated and purified by silicagel column chromatography (eluent: system B) to obtain methyl 5-(2-(4-(5-chloro-2-(1*H*-tetrazol-1-yl)phenyl)-2,5-dioxopiperazin-1-yl)-3-phenylpropanamido) indoline-1-carboxylate 8 (35 mg) with a yield of 49%.

MS m/z (ESI): 614.8 [M+H]
$^1$H NMR (400 MHz, DMSO-$d_6$) δ 10.09 (s, 1H), 9.72 (s, 1H), 7.86-7.47 (m, 5H), 7.38-7.12 (m, 6H), 5.34-5.25 (m, 1H), 4.52-3.86 (m, 4H), 3.95 (t, J = 9.4 Hz, 2H), 3.73 (s, 3H), 3.28-3.15 (m, 2H), 3.09 (t, J = 9.4 Hz, 2H).

Example 9

3-(2-(4-(5-Chloro-2-(1*H*-tetrazol-1-yl)phenyl)-2,5-dioxopiperazin-1-yl)-3-phenylpropanamido)benzoic acid

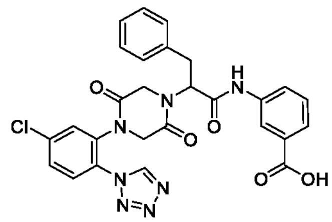

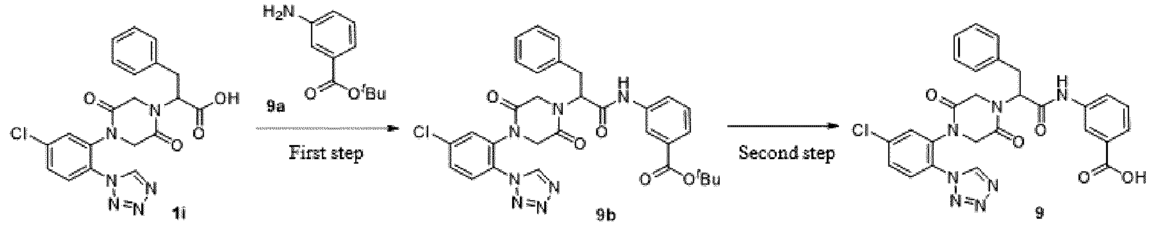


Step 1

*Tert*-butyl 3-(2-(4-(5-chloro-2-(1*H*-tetrazol-1-yl)phenyl)-2,5-dioxopiperazin-1-yl)-3-phenylpropanamido) benzoate

2-(4-(5-Chloro-2-(1*H*-tetrazol-1-yl)phenyl)-2,5-dioxopiperazin-1-yl)-3-phenylpropanoic acid 1i (100 mg, 226.84 μmol) and *tert*-butyl 3-aminobenzoate 9a (65.75 mg, 340.26 μmol) were dissolved in ethyl acetate (15 mL), added with N,N-diisopropylethylamine (187 μL, 1.13 μmol) and propylphosphonic anhydride (433 mg, 680.52 μmol, 50% ethyl acetate solution), and reacted at 60°C for 2 hours. After the reaction was completed, the reaction solution was concentrated under reduced pressure, and the obtained residue was further separated and purified by silicagel column chromatography (eluent: system A) to obtain *tert*-butyl 3-(2-(4-(5-chloro-2-(1*H*-tetrazol-1-yl)phenyl)-2,5-dioxopiperazin-1-yl)-3-phenylpropanamido) benzoate 9b (0.11 g) with a yield of 79%.
MS m/z (ESI): 616.2[M+H]

Step 2

3-(2-(4-(5-Chloro-2-(1*H*-tetrazol-1-yl)phenyl)-2,5-dioxopiperazin-1-yl)-3-phenylpropanamido)benzoic acid

*Tert-butyl* 3-(2-(4-(5-chloro-2-(1*H*-tetrazol-1-yl)phenyl)-2,5-dioxopiperazin-1-yl)-3-phenylpropanamido) benzoate 9b (0.11 g, 178.55 μmol) was dissolved in dichloromethane (15 mL), added with trifluoroacetic acid (1.5 mL), and reacted at room temperature for 3 hours. After the reaction was completed, the reaction solution was concentrated under reduced pressure, and the obtained residue was further separated and purified by silicagel column chromatography (eluent: system B) to obtain 3-(2-(4-(5-chloro-2-(1*H*-tetrazol-1-yl)phenyl)-2,5-dioxopiperazin-1-yl)-3-phenylpropanamido)benzoic acid 9 (52 mg) with a yield of 52%.

MS m/z (ESI): 560.1 [M+H]
$^1$H NMR (400 MHz, DMSO-$d_6$) δ 10.32 (s, 1H), 9.72 (s, 1H), 8.16 (s, 1H), 7.92-7.57 (m, 5H), 7.46-7.16 (m, 6H), 5.31 (dd, J = 10.1, 5.8 Hz, 1H), 4.58-3.86 (m, 4H), 3.18-3.00 (m, 1H), 2.93-2.84 (m, 1H).

Example 10

2-(4-(5-Chloro-2-(1*H*-tetrazol-1-yl)phenyl)-2,5-dioxopiperazin-1-yl)-N-(4-(2-oxopyrrolidin-1-yl)phenyl)-3-phenylpropanamide

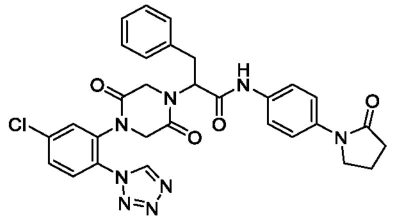

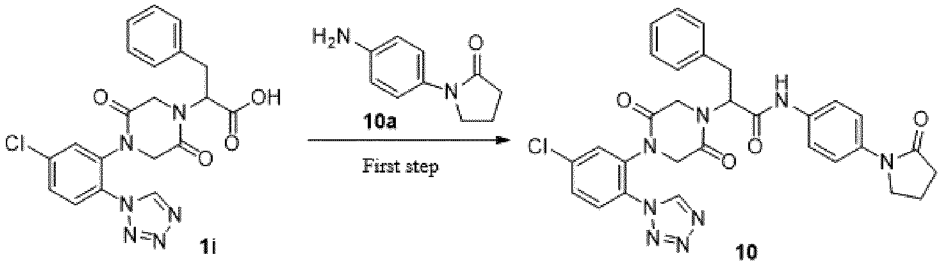


1i

10

2-(4-(5-Chloro-2-(1*H*-tetrazol-1-yl)phenyl)-2,5-dioxopiperazin-1-yl)-3-phenylpropanoic acid 1i (50 mg, 113.42 μmol) and 1-(4-aminophenyl)pyrrolidin-2-one 10a (29.98 mg, 170.13 μmol) were dissolved in ethyl acetate (5 mL), added with N,N-diisopropylethylamine (87.95 mg, 680.52 μmol) and propylphosphonic anhydride (433 mg, 680.52 μmol, 50% ethyl acetate solution), and reacted at 70°C for 4 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-N-(4-(2-oxopyrrolidin-1-yl)phenyl)-3-phenylpropanamide 10 (34 mg) with a yield of 50%.

MS m/z (ESI): 599.2 [M+H]
$^1$H NMR (400 MHz, DMSO-$d_6$) δ 10.17 (s, 1H), 9.71 (s, 1H), 7.85-7.70 (m, 3H), 7.59 (q, J = 9.0 Hz, 4H), 7.37-7.17 (m, 5H), 5.37-5.27 (m, 1H), 4.56-3.90 (m, 4H), 3.81 (t, J = 7.0 Hz, 2H), 3.29-3.18 (m, 1H), 3.13-3.02 (m, 1H), 2.48-2.45 (m, 2H), 2.11-2.00 (m, 2H).

Example 11

4-(2-(4-(5-Chloro-2-(1*H*-1,2,3-triazol-1-yl)phenyl)-2,5-dioxopiperazin-1-yl)-3 phenylpropanamido)benzoic acid

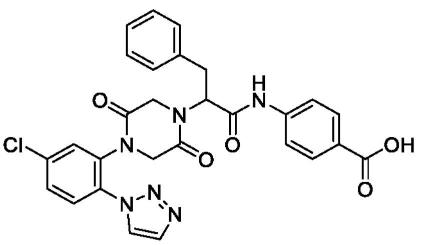

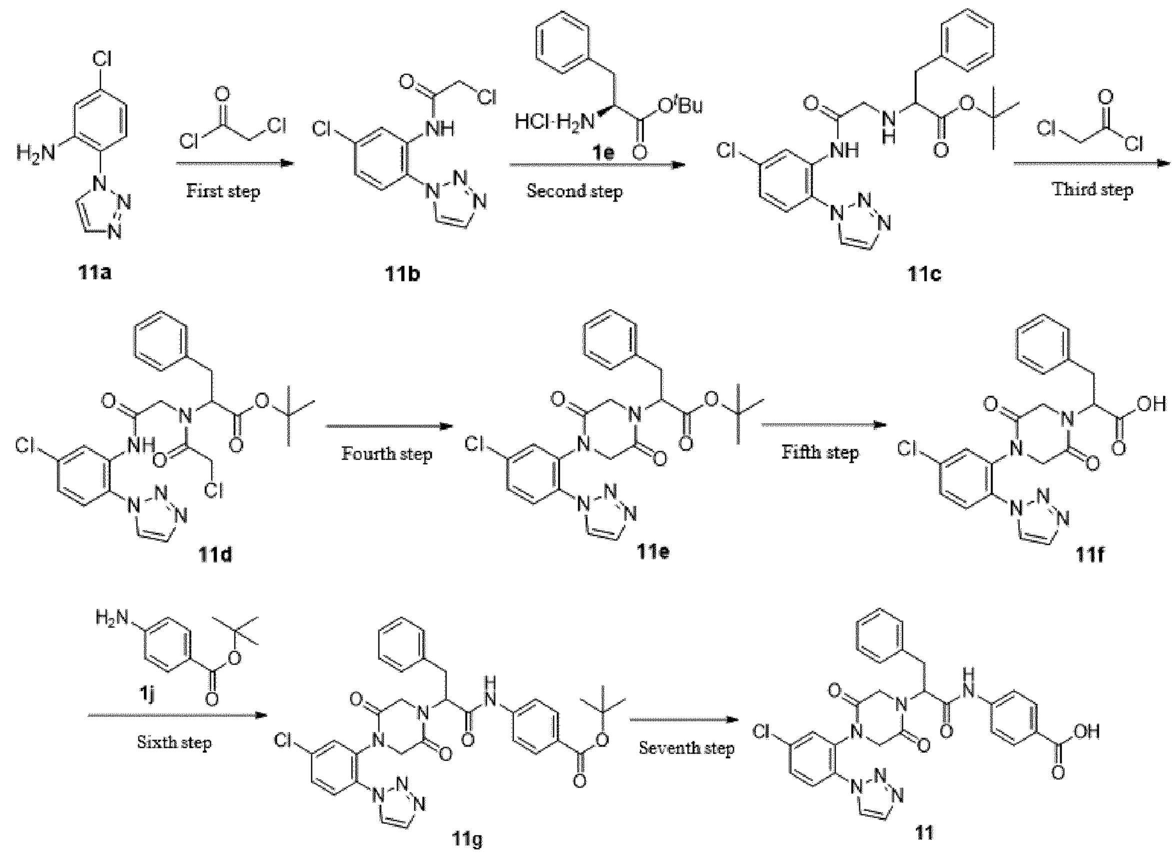


Step 1

2-Chloro-*N*-(5-chloro-2-(1*H*-1,2,3-triazol-1-yl)phenyl)acetamide

5-Chloro-2-(triazol-1-yl)aniline 11a (2.4 g, 12.33 mmol, prepared according to the published patent CN 108863962, page 4 of the specification, paragraphs [0013]-[0015], step A-1) was dissolved in dichloromethane (40 mL), added with triethylamine (3.74 g, 37.00 mmol), added with 2-chloroacetyl chloride (2.78 g, 24.66 mmol, 1.96 mL) at 0°C, and reacted for 1 hour. After quenching with 30 mL of water, the reaction solution was subjected to liquid separation, and then an aqueous phase was extracted with dichloromethane (50 mL×3). Organic phases were combined, washed with saturated sodium chloride solution (50 mL), dried with anhydrous sodium sulfate, filtered, and concentrated under reduced pressure, and the obtained residue was further separated and purified by silicagel column chromatography (eluent: system B) to obtain 2-chloro-*N*-(5-chloro-2-(1*H*-1,2,3-triazol-1-yl)phenyl)acetamide 11b (2.4 g) with a yield of 72%.
MS m/z (ESI): 271.0 [M+H]

Step 2

*Tert*-butyl (2-((5-chloro-2-(1*H*-1,2,3-triazol-1-yl)phenyl)amino)-2-oxoethyl)phenylalaninate

2-Chloro-*N*-(5-chloro-2-(1*H*-1,2,3-triazol-1-yl)phenyl)acetamide 11b (2.4 g, 8.85 mmol) and tert-butyl 3-phenyl-L-alaninate hydrochloride 1e (3.42 g, 13.28 mmol) were dissolved in N,N-dimethylformamide (50 mL), added with N,N-diisopropylethylamine (5.72 g, 44.26 mmol, 7.32 mL), and reacted overnight at 60°C. After the reaction was completed, the reaction solution was extracted with ethyl acetate (100 mL×3). Organic phases were combined, washed with water (200 mL×3) and saturated sodium chloride solution (200 mL), dried with anhydrous sodium sulfate, filtered and concentrated under reduced pressure, and the obtained residue was further separated and purified by silicagel column chromatography (eluent: system B) to obtain *tert*-butyl (2-((5-chloro-2-(1*H*-1,2,3-triazol-1-yl)phenyl)amino)-2-oxoethyl)phenylalaninate 11c (3.3 g) with a yield of 82%.
MS m/z (ESI): 456.7 [M+H]

Step 3

*Tert*-butyl *N*-(2-((5-Chloro-2-(1*H*-1,2,3-triazol-1-yl)phenyl)amino)-2-oxoethyl)-*N*-(2-chloroacetyl)phenylalaninate

*Tert*-butyl (2-((5-chloro-2-(1*H*-1,2,3-triazol-1-yl)phenyl)amino)-2-oxoethyl)phenylalaninate 11c (2 g, 4.39 mmol) was dissolved in dichloromethane (50 mL), placed in ice bath, added with triethylamine (2.22 g, 21.93 mmol, 3.05 mL), then slowly dropwise added with 2-chloroacetyl chloride (594.52 mg, 5.26 mmol, 418.68 μL) and stirred in ice bath for reaction for 1 hour. 2-chloroacetyl chloride (594.52 mg, 5.26 mmol, 418.68 μL) was added, and the reaction was continuously stirred under ice bath for reaction for 1 hour. LC-MS test showed that the reaction was completed. After quenching with 30 mL of water, the reaction solution was subjected to liquid separation, and then an aqueous phase was extracted with dichloromethane (50 mL×3). Organic phases were combined, washed with saturated sodium chloride solution (50 mL), dried with anhydrous sodium sulfate, filtered, and concentrated under reduced pressure, and the obtained residue was further separated and purified by silica gel column chromatography (eluent: system B) to obtain *tert*-butyl *N*-(2-((5-chloro-2-(1*H*-1,2,3-triazol-1-yl)phenyl)amino)-2-oxoethyl)-*N*-(2-chloroacetyl)phenylalaninate11d (2g) with a yield of 86%.
MS m/z (ESI): 476.0 [M+H-56]

Step 4

Tert-butyl 2-(4-(5-Chloro-2-(1*H*-1,2,3-triazol-1-yl)phenyl)-2,5-dioxopiperazin-1-yl)-3-phenylpropanoate

*Tert-butyl* *N*-(2-((5-chloro-2-(1*H*-1,2,3-triazol-1-yl)phenyl)amino)-2-oxoethyl)-*N*-(2-chloroacetyl)phenylalaninate 11d (2 g, 3.76 mmol) was dissolved in methanol (30 mL), slowly dropwise added with sodium methylate solution (5.4M, 765.20 μL) in ice bath, and reacted in ice bath for 30 minutes. LC-MS test showed that the reaction was completed. 2M dilute hydrochloric acid was added to adjust the pH to about 6, then the reaction solution was concentrated under reduced pressure, and the obtained residue was further separated and purified by silica gel column chromatography (eluent: system B) to obtain tert-butyl 2-(4-(5-chloro-2-(1*H*-1,2,3-triazol-1-yl)phenyl)-2,5-dioxopiperazin-1-yl)-3-phenylpropanoate 11e (1.5 g) with ayield of 81%.
MS m/z (ESI): 440.0 [M+H-56]

Step 5

2-(4-(5-Chloro-2-(1*H*-1,2,3-triazol-1-yl)phenyl)-2,5-dioxopiperazin-1-yl)-3-phenylpropanoic acid

Tert-butyl 2-(4-(5-chloro-2-(1*H*-1,2,3-triazole-1-yl)phenyl)-2,5-dioxopiperazin-1-yl)-3-phenylpropanoate 1 1e (1.5 g, 3.02 mmol) was dissolved in dichloromethane (20 mL), slowly dropwise added with trifluoroacetic acid (344.85 mg, 3.02 mmol, 5 mL), and reacted at room temperature for 1 hour. After the reaction was completed, the reaction solution was concentrated under reduced pressure, and the obtained residue was further separated and purified by silicagel column chromatography (eluent: system B) to obtain 2-(4-(5-chloro-2-(1*H*-1,2,3-triazol-1-yl)phenyl)-2,5-dioxopiperazin-1-yl)-3-phenylpropanoic acid 11f (1.33 g) with a yield of 100%.
MS m/z (ESI): 440.1 [M+H]

Step 6

*Tert*-butyl 4-(2-(4-(5-Chloro-2-(1*H*-1,2,3-triazol-1-yl)phenyl)-2,5-dioxopiperazin-1-yl)-3-phenylpropanamido) benzoate

2-(4-(5-Chloro-2-(1*H*-1,2,3-triazol-1-yl)phenyl)-2,5-dioxopiperazin-1-yl)-3-phenylpropanoic acid 11f (240 mg, 545.64 μmol) and tert-butyl 4-aminobenzoate 1j (126.53 mg, 654.77 μmol) were dissolved in ethyl acetate (10 mL), added with N,N-diisopropylethylamine (423.11 mg, 3.27 mmol) and propylphosphonic anhydride (1.04 g, 1.64 mmol, 50% ethyl acetate solution), and reacted at 65°C for 3 hours. After the reaction was completed, the reaction solution was concentrated under reduced pressure, and the obtained residue was further separated and purified by silicagel column chromatography (eluent: system B) to obtain *tert*-butyl 4-(2-(4-(5-chloro-2-(1*H*-1,2,3-triazol-1-yl)phenyl)-2,5-dioxopiperazin-1-yl)-3-phenylpropanamido) benzoate 11g (200 mg) with a yield of 60%.
MS m/z (ESI): 615.2 [M+H]

Step 7

4-(2-(4-(5-Chloro-2-(1H-1,2,3-triazole-1-yl)phenyl)-2,5-dioxiperazine-1-yl)-3-phenylpropanamido)benzoic acid

*Tert-butyl* 4-(2-(4-(5-chloro-2-(1*H*-1,2,3-triazol-1-yl)phenyl)-2,5-dioxopiperazin-1-yl)-3-phenylpropanamido) benzoate 11g (200 mg, 325.16 μmol) was dissolved in dichloromethane (12 mL), slowly dropwise added with trifluoroacetic acid (37.08 mg, 325.16 μmol, 4 mL), and reacted at room temperature for 1 hour. After the reaction was completed, the reaction solution was concentrated under reduced pressure, and the obtained residue was further separated and purified by silicagel column chromatography (eluent: system B) to obtain 4-(2-(4-(5-chloro-2-(1*H*-1,2,3-triazol-1-yl)phenyl)-2,5-dioxopiperazin-1-yl)-3-phenylpropanamido)benzoic acid 11 (110 mg) with a yield of 59%.

MS m/z (ESI): 558.8 [M+H]

$^1$H NMR (400 MHz, DMSO-$d_6$) δ 12.75 (s, 1H), 10.49 (s, 1H), 8.19 (s, 1H), 7.92 (d, J = 8.5 Hz, 2H), 7.81-7.58 (m, 6H), 7.37-7.23( m, 5H), 5.36 (dd, J = 10.3, 5.8 Hz, 1H), 4.45-3.90 (m, 4H), 3.31-3.21 (m, 1H), 3.19-3.06 (m, 1H).

Example 12

2-(4-(5-Chloro-2-(1*H*-tetrazol-1-yl)phenyl)-2,5-dioxopiperazin-1-yl)-N-(2-methyl-1-oxoisoindolin-5-yl)-3-phenylproanamide

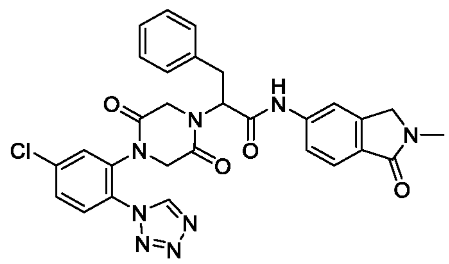

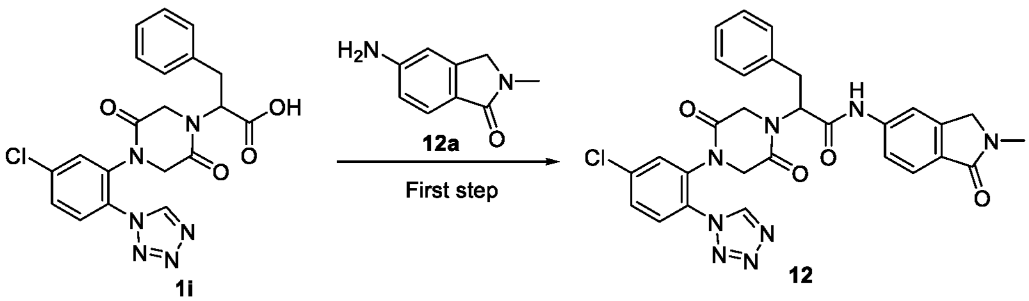


1i

12

2-(4-(5-Chloro-2-(1*H*-tetrazol-1-yl)phenyl)-2,5-dioxopiperazin-1-yl)-3-phenylpropanoic acid 1i (50 mg, 113.42 μmol) and 5-amino-2-methylisoindolin-1-one 12a (27.59 mg, 170.13 μmol) were dissolved in ethyl acetate (5 mL), added with N,N-diisopropylethylamine (87.95 mg, 680.52 μmol) and propylphosphonic anhydride (216.53 mg, 340.26 μmol, 50% ethyl acetate solution), and reacted at 70°C for 4 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-*N*-(2-methyl-1-oxoisoindolin-5-yl)-3-phenylpropanamide 12 (35 mg) with a yield of 52%.

MS m/z (ESI): 585.0 [M+H]

$^1$H NMR (400 MHz, DMSO-$d_6$) δ 10.48 (s, 1H), 9.72 (s, 1H), 7.95 (s, 1H), 7.81 (d, J = 8.6 Hz, 1H), 7.75 (dd, J= 8.7, 2.2 Hz, 1H), 7.69-7.48 (m, 2H), 7.36-7.21 (m, 5H), 5.41-5.29 (m, 1H), 4.60-3.90 (m, 4H), 4.44 (s, 2H), 3.30-3.20 (m, 1H), 3.15-3.08 (m, 1H), 3.05 (s, 3H).

Example 13

2-(4-(5-Chloro-2-(1*H*-tetrazol-1-yl)phenyl)-2,5-dioxopiperazin-1-yl)-N-(2-oxoindolin-5-yl)-3-phenylpropanamide

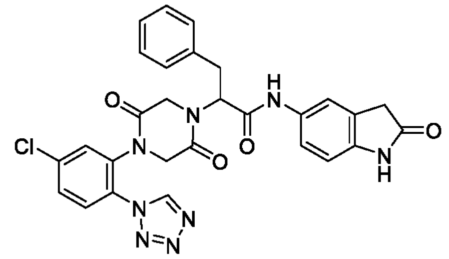

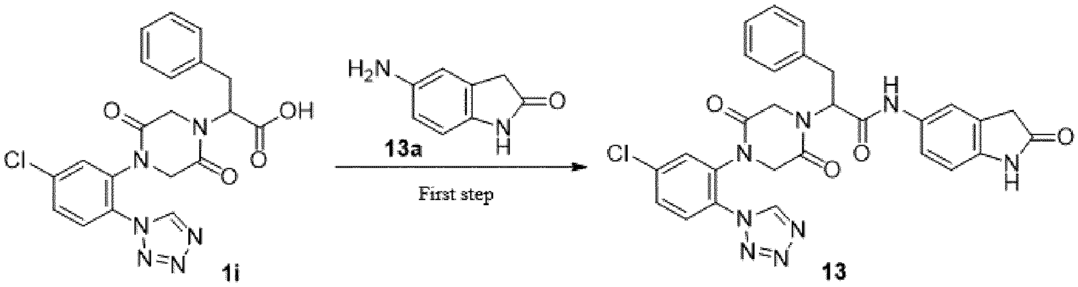


1i

13

2-(4-(5-Chloro-2-(1*H*-tetrazol-1-yl)phenyl)-2,5-dioxopiperazin-1-yl)-3-phenylpropanoic acid 1i (50 mg, 113.42 μmol) and 5-aminoindolin-2-one 13a (25.21 mg, 170.13 μmol) were dissolved in ethyl acetate (5 mL), added with N,N-diisopropylethylamine (87.95 mg, 680.52 μmol) and propylphosphonic anhydride (216.53 mg, 340.26 μmol, 50% ethyl acetate solution), and reacted at 70°C for 4 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-l-yl)-N-(2-oxoindolin-5-yl)-3-phenylpropanamide 13 (33 mg) with a yield of 57%. MS m/z (ESI): 571.0 [M+H]
$^1$H NMR (400 MHz, DMSO-$d_6$) δ 10.32 (s, 1H), 10.02 (s, 1H), 9.72 (s, 1H), 7.86-7.72 (m, 2H), 7.47 (s, 1H), 7.37-7.15 (m, 7H), 6.75 (d, J = 8.4 Hz, 1H), 5.36- 5.26 (m, 1H), 4.57-3.88 (m, 4H), 3.47 (s, 2H), 3.26-3.15 (m, 1H), 3.11-2.98 (m, 1H).

Example 14

2-(4-(5-Chloro-2-(1*H*-tetrazol-1-yl)phenyl)-2,5-dioxopiperazin-1-yl)-*N*-(4-(2-oxooxazolidin-3-yl)phenyl)-3-phenylpropanamide

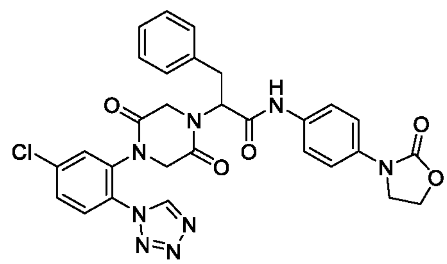

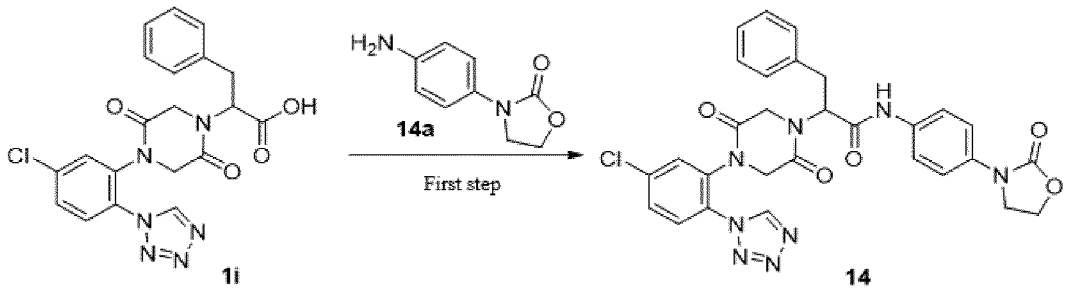


1i 14

2-(4-(5-Chloro-2-(1*H*-tetrazol-1-yl)phenyl)-2,5-dioxopiperazin-1-yl)-3-phenylpropanoic acid 1i (50 mg, 113.42 μmol) and 3-(4-aminophenyl)oxazolidin-2-one 14a (30.32 mg, 170.13 μmol, prepared according to the published patent CN 105384739, pages 40-41 of the specification, paragraphs [0171]-[0178], method 1) were dissolved in ethyl acetate (5 mL), added with propylphosphonic anhydride (216.53 mg, 340.26 μmol, 50% ethyl acetate solution) and N,N-diisopropylethylamine (87.95 mg, 680.52 μmol, 112.47 μL), and reacted at room temperature overnight. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-*N*-(4-(2-oxooxazolidin-3-yl)phenyl)-3-phenylpropanamide 14 (22 mg) with a yield of 99%.

MS m/z (ESI): 600.8[M+H]

$^1$H NMR (400 MHz, DMSO-$d_6$) δ 10.19 (s, 1H), 9.72 (s, 1H), 7.81 (d, J = 8.6 Hz, 1H), 7.75 (dd, J = 8.6, 2.2 Hz, 1H), 7.60 (d, J = 8.7 Hz, 2H), 7.52 (d, J = 8.8 Hz, 2H), 7.37 -7.17 (m, 5H), 5.33 (dd, J = 10.0, 5.7 Hz, 1H), 4.65-3.91 (m, 4H), 4.43 (t, J = 8.0 Hz, 2H), 4.04 (t, J = 8.0 Hz, 2H), 3.29-3.18 (m, 1H), 3.15-3.03 (m, 1H).

Example 15

2-(4-(5-Chloro-2-(1*H*-tetrazol-1-yl)phenyl)-2,5-dioxopiperazine-1-yl)-*N*-(3-fluorophenyl)-3-phenylpropanamide

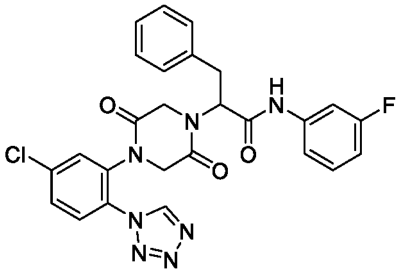

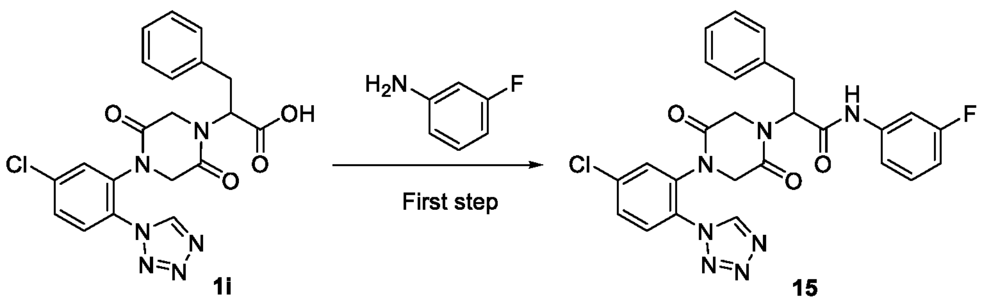


1i 15

2-(4-(5-Chloro-2-(1*H*-tetrazol-1-yl)phenyl)-2,5-dioxopiperazin-1-yl)-3-phenylpropanoic acid 1i (50 mg, 113.42 μmol) and 3-fluoroaniline (18.90 mg, 170.13 μmol) were dissolved in ethyl acetate (5 mL), added with N,N-diisopropylethylamine (87.95 mg, 680.52 μmol) and propylphosphonic anhydride (216.53 mg, 340.26 μmol, 50% ethyl acetate solution), and reacted at 70°C for 4 hours. After the reaction was completed, the reaction solution was concentrated under reduced pressure, and the obtained residue was further separated and purified by silicagel column chromatography

(eluent: system B) to obtain 2-(4-(5-chloro-2-(1*H*-tetrazole-1-yl)phenyl)-2,5-dioxopiperazin-1-yl)-*N*-(3-fluorophenyl)-3-phenylpropanamide 15 (17 mg) with a yield of 28%.

MS m/z (ESI): 534.1[M+H]

$^1$H NMR (400 MHz, DMSO-$d_6$) $\delta$ 10.38 (s, 1H), 9.71 (s, 1H), 7.93-7.65 (m, 3H), 7.58 (d, J= 11.5 Hz, 1H), 7.43-7.18 (m, 7H), 6.92 (t, J = 8.5 Hz, 1H), 5.37-5.24 (m, 1H), 4.69-3.88 (m, 4H), 3.28-3.15 (m, 1H), 3.15-3.01 (m, 1H).

Example 16

2-(4-(5-Chloro-2-(1*H*-tetrazol-1-yl)phenyl)-2,5-dioxopiperazin-1-yl)-*N*-(4-cyanophenyl)-3-phenylpropanamide

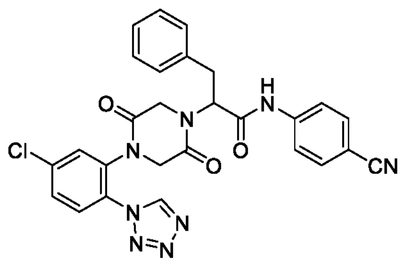

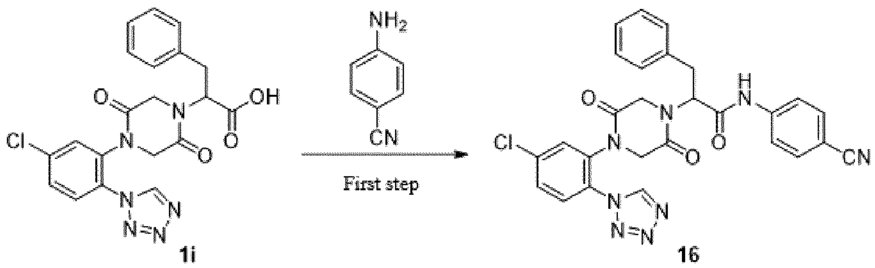


2-(4-(5-Chloro-2-(1*H*-tetrazol-1-yl)phenyl)-2,5-dioxopiperazin-1-yl)-3-phenylpropanoic acid 1i (50 mg, 113.42 μmol), 4-aminobenzonitrile (13.40 mg, 113.42 μmol), N,N-diisopropylethylamine (73.29 mg, 567.10 μmol) and propylphosphonic anhydride (216.53 mg, 340.26 μmol, 50% ethyl acetate solution) were dissolved in ethyl acetate (7 mL) in turn, and continuously reacted under stirring at room temperature for 16 hours. After the reaction was completed, the reaction solution was concentrated under reduced pressure, and the obtained residue was subjected to liquid phase purification (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 2-(4-(5-chloro-2-(177-tetrazol-1-yl)phenyl)-2,5-dioxopiperazin-1-yl)-N-(4-cyanophenyl)-3-phenylpropanamide 16 (23 mg) with a yield of 37%.

MS m/z (ESI): 5 4 1. 1 [M+H]

$^1$H NMR (400 MHz, DMSO-$d_6$) $\delta$ 10.61 (s, 1H), 9.70 (s, 1H), 7.94-7.60 (m, 7H), 7.41-7.13 (m, 5H), 5.31 (dd, J= 10.2, 5.8 Hz, 1H), 4.78-3.88 (m, 4H), 3.30-3.17 (m, 1H), 3.15-3.00 (m, 1H).

Example 17

*N*-(4-acetylphenyl)-2-(4-(5-chloro-2-(1*H*-tetrazol-1-yl)phenyl)-2,5-dioxopiperazin-1-yl)-3-phenylpropanamide

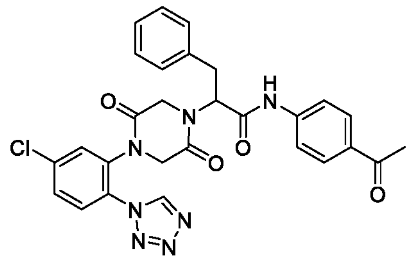

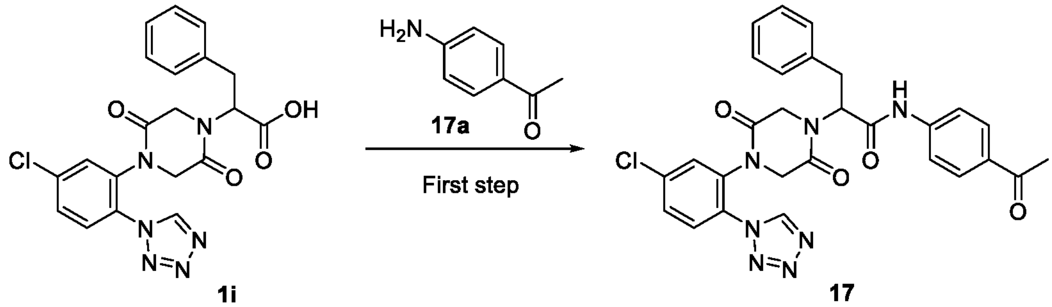


1i 17

2-(4-(5-Chloro-2-(1*H*-tetrazol-1-yl)phenyl)-2,5-dioxopiperazin-1-yl)-3-phenylpropanoic acid 1i (50 mg, 113.42 μmol) and 1-(4-aminophenyl)ethan-1-one 17a (23.00 mg, 170.13 μmol) were dissolved in ethyl acetate (5 mL), added with N,N-diisopropylethylamine (87.95 mg, 680.52 μmol) and propylphosphonic anhydride (216.53 mg, 340.26 μmol, 50% ethyl acetate solution), and reacted at 60°C for 4 hours. After the reaction was completed, the reaction solution was concentrated under reduced pressure, and the obtained residue was further separated and purified by silicagel column chromatography (eluent: system B) to obtain *N*-(4-acetylphenyl)-2-(4-(5-chloro-2-(1*H*-tetrazol-1-yl)phenyl)-2,5-dioxopiperazine-1-yl)-3-phenylpropanamide 17 (35 mg) with a yield of 51%.

MS m/z (ESI): 558.2[M+H]

$^1$H NMR (400 MHz, DMSO-$d_6$) δ 10.51 (s, 1H), 9.72 (s, 1H), 7.95 (d, J = 8.3 Hz, 2H), 7.86-7.65 (m, 5H), 7.41-7.15 (m, 5H), 5.35 (dd, J = 10.3, 6.0 Hz, 1H), 4.71-3.91 (m, 4H), 3.30-3.19 (m, 1H), 3.18-3.03 (m, 1H), 2.54 (s, 3H).

Example 18

2-(4-(5-Chloro-2-(1*H*-tetrazol-1-yl)phenyl)-2,5-dioxopiperazin-1-yl)-3-phenyl-*N*-(quinoxalin-6-yl)propanamide

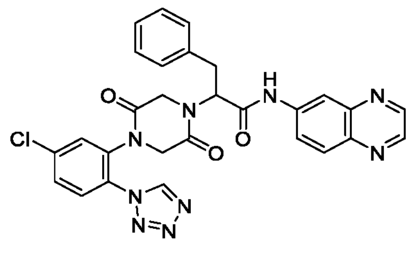

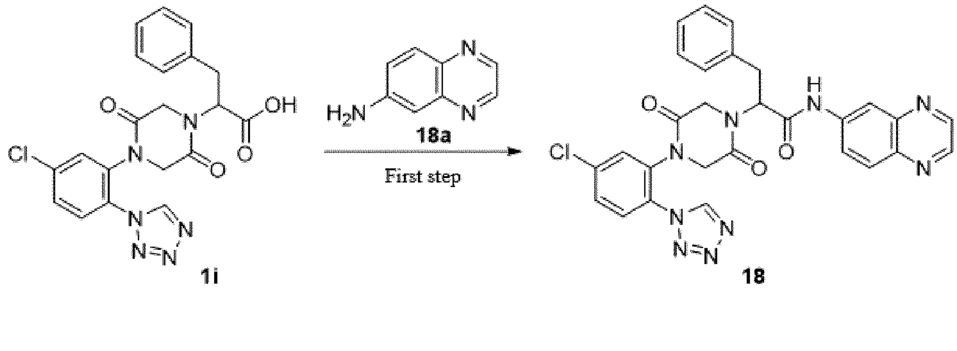


1i 18

2-(4-(5-Chloro-2-(1H-tetrazol-1-yl)phenyl)-2,5-dioxopiperazin-1-yl)-3-phenylpropanoic acid 1i (30 mg, 68.05 μmol), quinoxalin-6-amine 18a (14.82 mg, 102.08 μmol), N,N-diisopropylethylamine (43.98 mg, 340.26 μmol) and propylphosphonic anhydride (129.92 mg, 204.16 μmol, 50% ethyl acetate solution) were dissolved in ethyl acetate (5 mL) in turn, and stirred at room temperature for reaction for 16 hours. After the reaction was completed, the reaction solution was concentrated under reduced pressure, and the obtained residue was subjected to preparative liquid phase purification (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 2-(4-(5-chloro-2-(1*H*-tetrazol-1-yl)phenyl)-2,5-dioxopiperazin-1-yl)-3-phenyl-N-(quinoxalin-6-yl)propanamide 18 (26 mg) with a yield of 67%.

MS m/z (ESI): 567.9[M+H]

$^1$H NMR (400 MHz, DMSO-$d_6$) δ 10.69 (s, 1H), 9.72 (s, 1H), 8.90 (s, 1H), 8.84 (s, 1H), 8.50 (s, 1H), 8.07 (d, J = 9.1 Hz, 1H), 7.95 (d, J = 9.2 Hz, 1H), 7.89-7.62 (m, 3H), 7.41-7.18 (m, 5H), 5.45-5.33 (m, 1H), 4.65-3.94 (m, 4H), 3.23-3.06 (m, 2H).

Example 19

2-(4-(5-Chloro-2-(1*H*-tetrazol-1-yl)phenyl)-2,5-dioxopiperazin-1-yl)-*N*-(2-methyl-2*H*-indazol-5-yl)-3-phenylpropanamide

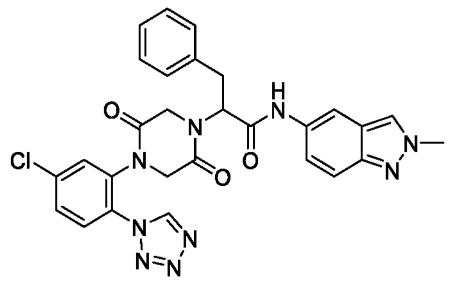

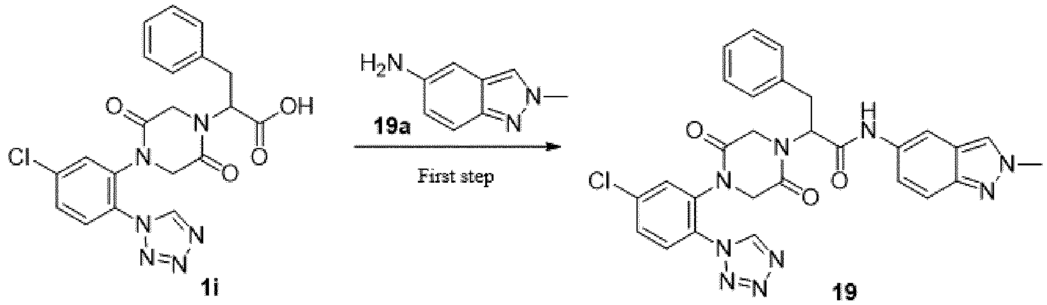


2-(4-(5-Chloro-2-(1*H*-tetrazol-1-yl)phenyl)-2,5-dioxopiperazin-1-yl)-3-phenylpropanoic acid 1i (30 mg, 68.05 μmol), 2-methyl-2*H*-indazol-5-amine 19a (15.02 mg, 102.08 μmoll), N,N-diisopropylethylamine (43.98 mg, 340.26 μmol) and propylphosphonic anhydride (129.92 mg, 204.16 μmol, 50% ethyl acetate solution) were dissolved in ethyl acetate (4 mL) in turn, and reacted at room temperature for 16 hours. After the reaction was completed, the reaction solution was concentrated under reduced pressure, and the obtained residue was subjected to preparative liquid phase purification (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 2-(4-(5-chloro-2-(1*H*-tetrazol-1-yl)phenyl)-2,5-dioxopiperazin-1-yl)-*N*-(2-methyl-2*H*-indazol-5-yl)-3-phenylpropanamide 19 (23 mg) with a yield of 59%.

MS m/z (ESI): 570.2[M+H]

$^1$H NMR (400 MHz, DMSO-$d_6$) δ 10.13 (s, 1H), 9.73 (s, 1H), 8.27 (s, 1H), 8.08 (s, 1H), 7.93-7.65 (m, 3H), 7.54 (d, J = 9.2 Hz, 1H), 7.43-7.16 (m, 6H), 5.44-5.28 (m, 1H), 4.57-3.90 (m, 4H), 4.13 (s, 3H), 3.31-3.20 (m, 1H), 3.15-3.02 (m, 1H).

Example 20

4-(2-(4-(5-Chloro-2-(1*H*-tetrazol-1-yl)phenyl)-2,5-dioxopiperazin-1-yl)-3-phenylpropanamido)-2-fluoro-*N*-methylbenzamide

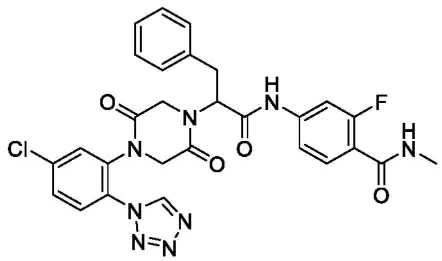

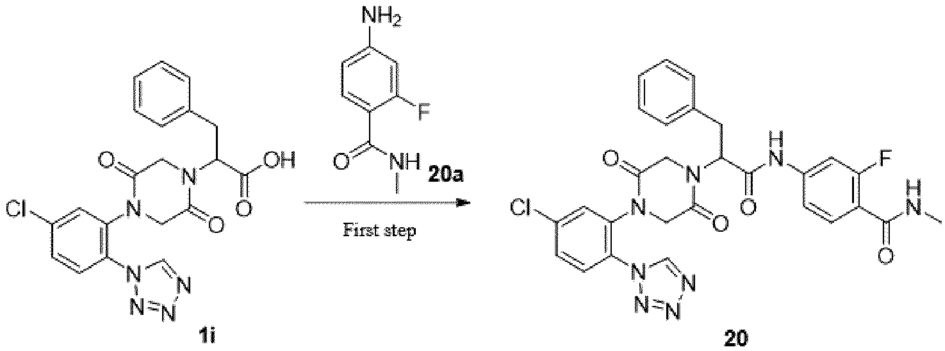


1i

20

2-(4-(5-Chloro-2-(1*H*-tetrazol-1-yl)phenyl)-2,5-dioxopiperazin-1-yl)-3-phenylpropanoic acid 1i (30 mg, 68.05 $\mu$mol), 4-amino-2-fluoro-N-methylbenzamide 20a (17.17 mg, 102.08 $\mu$mol), N,N-diisopropylethylamine (43.98 mg, 340.26 $\mu$mol) and propylphosphonic anhydride (129.92 mg, 204.16 $\mu$mol, 50% ethyl acetate solution) were dissolved in ethyl acetate (5 mL) in turn, and reacted under stirring at room temperature for 16 hours. After the reaction was completed, the reaction solution was concentrated under reduced pressure, and the obtained residue was subjected to preparative liquid phase purification (separation column AKZONOBEL Kromasil; 250$\times$21.2 mm I.D.; 5 $\mu$___m, 20 mL/min; mobile phase A: 0.05% TFA+$H_2O$, mobile phase B: $CH_3CN$) to obtain 4-(2-(4-(5-chloro-2-(1*H*-tetrazol-1-yl)phenyl)-2,5-dioxopiperazin-1-yl)-3-phenylpropanamido)-2-fluoro-*N*-methylbenzamide 20 (18 mg) with a yield of 40%.

MS m/z (ESI): 591.1 [M+H]

$^1$H NMR (400 MHz, DMSO-$d_6$) $\delta$ 10.54 (s, 1H), 9.71 (s, 1H), 8.10 (s, 1H), 7.89-7.72 (m, 3H), 7.71-7.59 (m, 2H), 7.43-7.17 (m, 6H), 5.38-5.23 (m, 1H), 4.73-3.89 (m, 4H), 3.30-3.19 (m, 1H), 3.15-3.03 (m, 1H), 2.77 (d, J = 4.5 Hz, 3H).

Example 21

2-(4-(5-Chloro-2-(1*H*-tetrazol-1-yl)phenyl)-2,5-dioxopiperazin-1-yl)-N-(1-oxo-1,3-dihydroisobenzofuran-5-yl)-3-phenylpropanamide

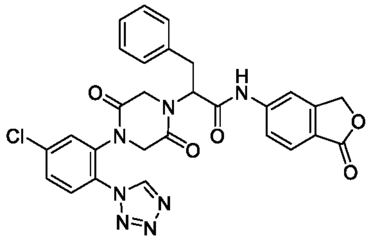

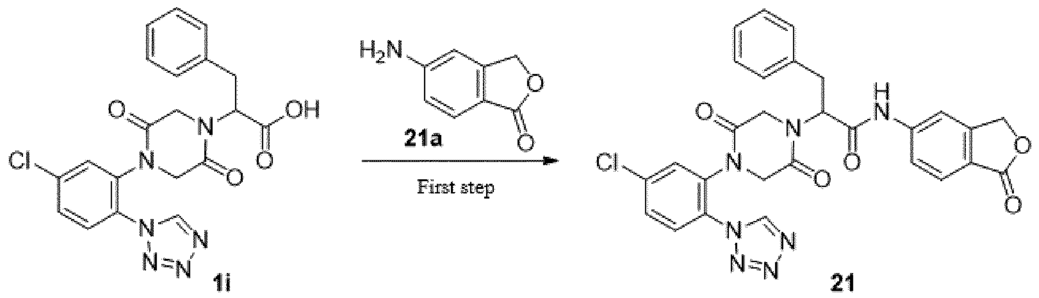


1i

21

2-(4-(5-Chloro-2-(1*H*-tetrazol-1-yl)phenyl)-2,5-dioxopiperazine-1-yl)-3-phenylpropanoic acid 1i (50 mg, 113.42 μmol) and 5-aminoisobenzofuran-1(3*H*)-one 21a (25.37 mg, 170.13 μmol) were dissolved in ethyl acetate (5 mL), added with N,N-diisopropylethylamine (87.95 mg, 680.52 μmol) and propylphosphonic anhydride (216.53 mg, 340.26 μmol, 50% ethyl acetate solution), and reacted at 60°C for 4 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-*N*-(1-oxo-1,3-dihydroisobenzofuran-5-yl)-3-phenylpropanamide 21 (24 mg) with a yield of 35%.

MS m/z (ESI): 571.9 [M+H]
$^{1}$H NMR (400 MHz, DMSO-$d_6$) δ 10.82 (s, 1H), 9.73 (s, 1H), 8.05 (s, 1H), 7.93-7.54 (m, 5H), 7.47-7.04 (m, 5H), 5.52-5.22 (m, 1H), 4.68-3.82 (m, 4H), 4.00 (s, 2H), 3.19-2.97 (m, 2H).

Example 22

2-(4-(5-Chloro-2-(1*H*-tetrazol-1-yl)phenyl)-2,5-dioxopiperazin-1-yl)-3-phenyl-*N*-(pyrazolo[1,5-a]pyridin-6-yl)propanamide

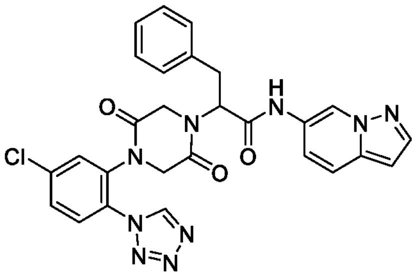

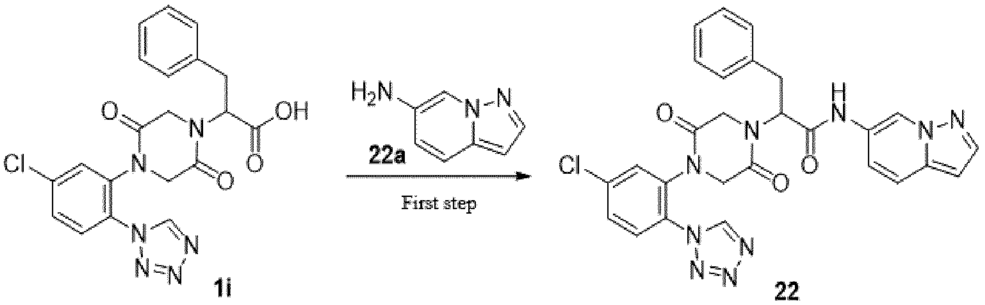


1i

22

2-(4-(5-Chloro-2-(1*H*-tetrazol-1-yl)phenyl)-2,5-dioxopiperazin-1-yl)-3-phenylpropanoic acid 1i (50 mg, 113.42 μmol) and pyrazolo[1,5-a]pyridin-6-amine 22a (22.65 mg, 170.13 μmol, prepared according to the published patent WO 2019099311, page 96 of the specification, paragraph [000344]) were dissolved in ethyl acetate (5 mL), added with N,N-diisopropylethylamine (87.95 mg, 680.52 μmol) and propylphosphonic anhydride (216.53 mg, 340.26 μmol, 50% ethyl acetate solution), and reacted at 60°C for 4 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-3-phenyl-

*N*-(pyrazolo[1,5-a]pyridin-6-yl)propanamide 22 (18 mg) with a yield of 32%.

MS m/z (ESI): 555.9 [M+H]
$^1$H NMR (400 MHz, DMSO-$d_6$) δ 10.36 (s, 1H), 9.74 (s, 1H), 9.23 (s, 1H), 7.95 (s, 1H), 7.87-7.59 (m, 4H), 7.40-7.12 (m, 6H), 6.59 (s, 1H), 5.40-5.26 (m, 1H), 4.66-3.91 (m, 4H), 3.33-3.19 (m, 1H), 3.16-3.03 (m, 1H).

Example 23

2-(4-(5-Chloro-2-(1*H*-tetrazol-1-yl)phenyl)-2,5-dioxopiperazin-1-yl)-*N*-(1-methoxyisoquinolin-6-yl)-3-phenylpropanamide

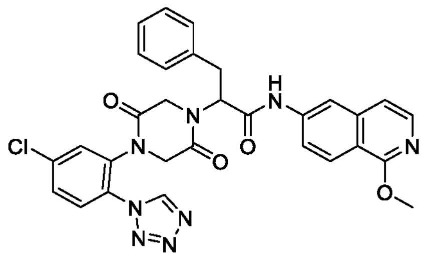

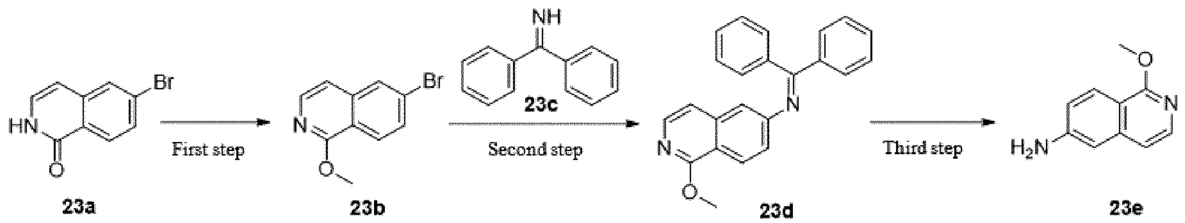


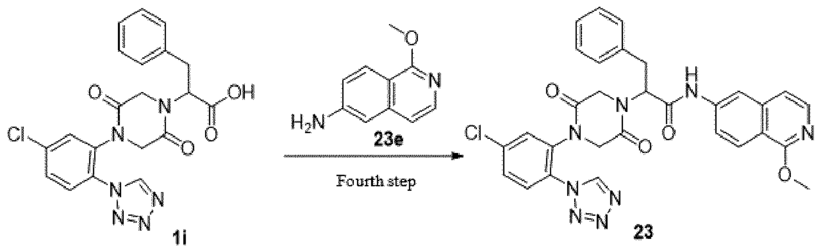


Step 1

6-Bromo-1-methoxyisoquinoline

6-Bromoisoquinolin-1(2*H*)-one 23a (500 mg, 2.23 mmol), methyl iodide (1.58 g, 11.16 mmol) and silver carbonate (1.24 g, 4.46 mmol) were dissolved in toluene (5 mL) in turn, and stirred at 40°C for reaction for 16 hours. After the reaction was completed, the reaction solution was concentrated under reduced pressure, and the obtained residue was further separated and purified by silicagel column chromatography (eluent: system B) to obtain 6-bromo-1-methoxyisoquinoline 23b (160 mg) with a yield of 30%.
MS m/z (ESI): 237.9 [M+H]

Step 2

*N*-(1-methoxyisoquinolin-6-yl)-1,1-diphenylmethanimine

Under the protection of argon, 6-bromo-1-methoxyisoquinoline 23b (160 mg, 672.04 μmol), diphenylmethylamine 23c (243.59 mg, 1.34 mmol), tris(dibenzylideneacetone)dipalladium (61.54 mg, 67.20 μmol),1,1'-binaphthyl-2,2'-diphemyl phosphine (41.85 mg, 67.20 μmol) and sodium tert-butoxide (129.17 mg, 1.34 mmol) were dissolved in toluene (10 mL) in turn, subjected to argon replacement for three times, and reacted at 85°C for 20 hours. After the reaction was completed, the reaction solution was cooled to room temperature and concentrated under reduced pressure, and the obtained residue was further separated and purified by silicagel column chromatography (eluent: system A) to obtain *N*-(1-methoxyisoquinolin-6-yl)-1,1-diphenylmethanimine 23d (200 mg) with a yield of 88%.
MS m/z (ESI): 339.1 [M+H]

Step 3

1-Methoxyisoquinolin-6-amine

*N*-(1-methoxyisoquinolin-6-yl)-1,1-diphenylmethanimine 23d (197.42 mg, 583.40 μmol) and 2M dilute hydrochloric acid (21.27 mg, 583.40 μmol) were dissolved in trifluoroacetic acid (6 mL) in turn, and reacted at room temperature for 18 hours. After the reaction was completed, the reaction solution was concentrated under reduced pressure to move most trifluoroacetic acid, and the obtained residue was further separated and purified by silicagel column chromatography (eluent: system A) to obtain 1-methoxyisoquinolin-6-amine 23e (92 mg) with a yield of 91%.
MS m/z (ESI): 175.1 [M+H]

Step 4

2-(4-(5-Chloro-2-(1*H*-tetrazol-1-yl)phenyl)-2,5-dioxopiperazin-1-yl)-*N*-(1-methoxyisoquinolin-6-yl)-3-phenylpropanamide

2-(4-(5-Chloro-2-(1*H*-tetrazol-1-yl)phenyl)-2,5-dioxopiperazin-1-yl)-3-phenylpropanoic acid 1i (50 mg, 113.42 μmol), 1-methoxyisoquinolin-6-amine 23e (29.64 mg, 170.13 μmol), N,N-diisopropylethylamine (219.88 mg, 1.70 μmol) and propylphosphonic anhydride (216.53 mg, 340.26 μmol, 50% ethyl acetate solution) were dissolved in ethyl acetate (10 mL) in turn, and reacted at room temperature for 18 hours. After the reaction was completed, the reaction solution was concentrated under reduced pressure, and the obtained residue was subjected to preparative liquid phase purification (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 2-(4-(5-chloro-2-(1*H*-tetrazol-1-yl)phenyl)-2,5-dioxopiperazin-1-yl)-*N*-(1-methoxyisoquinolin-6-yl)-3-phenylpropanamide 23 (7 mg) with a yield of 10%.

MS m/z (ESI): 597.2 [M+H]
$^1$H NMR (400 MHz, DMSO-$d_6$) δ 10.56 (s, 1H), 9.73 (s, 1H), 8.28-8.24 (m, 1H), 8.13 (d, J = 8.9 Hz, 1H), 7.96 (d, J= 5.9 Hz, 1H), 7.84-7.73 (m, 3H), 7.71-7.65 (m, 1H), 7.37-7.23 (m, 6H), 5.42-5.34 (m, 1H), 4.40-3.92 (m, 4H), 4.04 (s, 3H), 3.18-3.07 (m, 2H).

Example 24

2-(4-(5-Chloro-2-(4-chloro-1*H*-1,2,3-triazol-1-yl)phenyl)-2,5-dioxopiperazin-1-yl)-3-phenyl-*N*-(quinoxalin-6-yl)propanamide

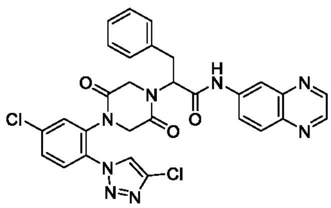

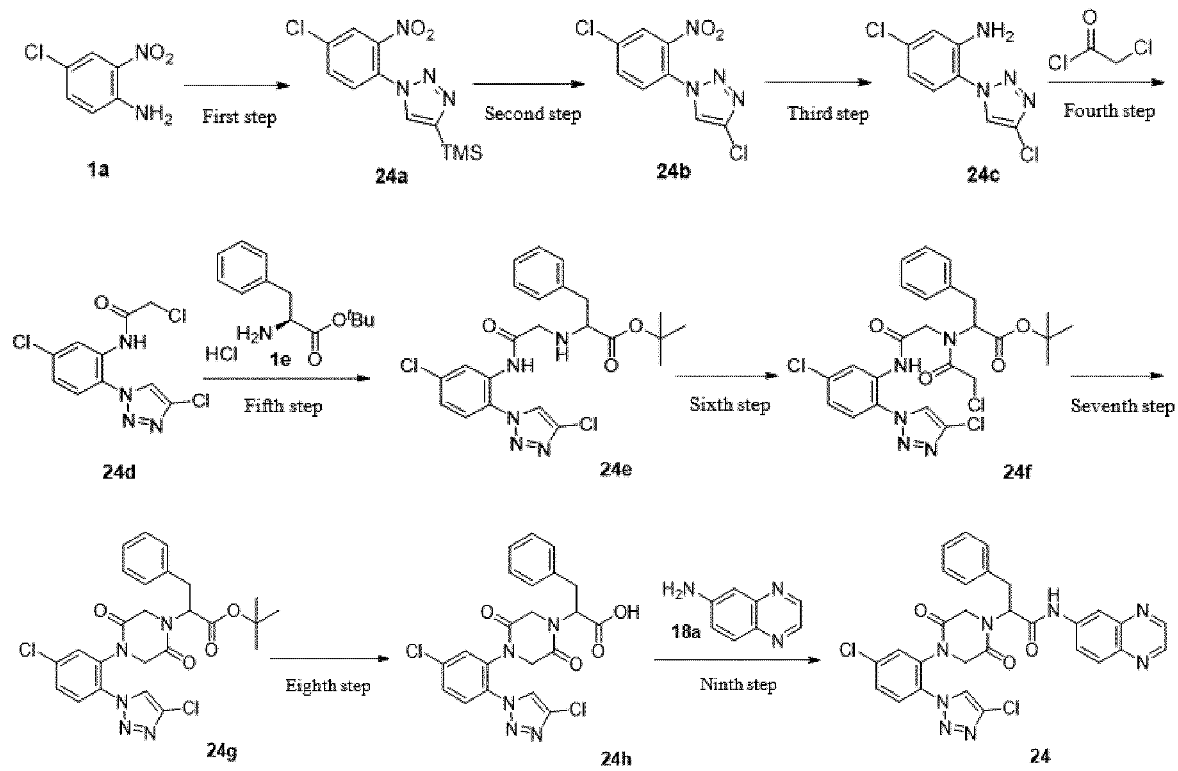


1a 24a 24b 24c 24d 1e 24e 24f 24g 24h 18a 24

Step 1

1-(4-Chloro-2-nitrophenyl)-4-(trimethylsilyl)-1*H*-1,2,3-triazole

4-Chloro-2-nitroaniline 1a (5 g, 28.97 mmol) was dissolved in acetonitrile (300 mL), stirred in ice bath for 20 minutes, added with isoamyl nitrite (5.09 g, 43.46 mmol, 5.84 mL) and azidotrimethylsilane (5.01 g, 43.46 mmol, 5.72 mL) in turn, sitrred in ice bath for 20 minutes, then the ice bath was removed, and the reaction solution was reacted at room temperature for 2 hours, then added with cuprous oxide (4.15 g, 28.97 mmol) and trimethylsilylacetylene (8.54 g, 86.92 mmol, 12.28 mL) in turn, and continuously stirred at room temperature for reaction for 4 hours. After the reaction was completed, the reaction solution was filtered and concentrated under reduced pressure, and the obtained residue was further separated and purified by silicagel column chromatography (eluent: system B) to obtain 1-(4-chloro-2-nitrophenyl)-4-(trimethylsilyl)-1*H*-1,2,3-triazole 24a (5 g) with a yield of 58%.
MS m/z (ESI): 297.0 [M+H]

Step 2

4-Chloro-1-(4-chloro-2-nitrophenyl)-1*H*-1,2,3 -triazole

1-(4-chloro-2-nitrophenyl)-4-(trimethylsilyl)-1*H*-1,2,3-triazole 24a (5 g, 16.85 mmol) was dissolved in acetonitrile (93 mL), added with silica gel (20.87 g, 16.85 mmol), stirred at room temperature for reaction for 10 minutes, and then added with N-chlorosuccinimide (11.25 g, 84.24 mmol), and reacted at 80°C for 1 hour. After the reaction was completed, the reaction solution was filtered and concentrated under reduced pressure, and the obtained residue was further separated and purified by silicagel column chromatography (eluent: system B) to obtain 4-chloro-1-(4-chloro-2-nitrophenyl)-1*H*-1,2,3-triazole 24b (1.65 g) with a yield of 38%.
MS m/z (ESI): 259.0 [M+H]

Step 3

5-Chloro-2-(4-chloro-1*H*-1,2,3-triazol-1-yl)aniline

4-Chloro-1-(4-chloro-2-nitrophenyl)-1*H*-1,2,3-triazole 24b (1.75 g, 6.76 mmol) was dissolved in a mixed solvent of ethanol (20 mL) and water (5 mL), added with ammonium chloride (722.72 mg, 13.51 mmol) and iron powder (3.77

g, 67.55 mL), and reacted overnight at 80°C. After the reaction was completed, the reaction solution was filtered, the filtrate was concentrated under reduced pressure, and the obtained residue was further separated and purified by silicagel column chromatography (eluent: system B) to obtain 5-chloro-2-(4-chloro-1*H*-1,2,3-triazol-1-yl)aniline 24c (1.3 g) with a yield of 84%.
MS m/z (ESI): 228.9 [M+H]

Step 4

2-Chloro-N-(5-chloro-2-(4-chloro-1*H*-1,2,3-triazol-1-yl)phenyl)acetamide

5-Chloro-2-(4-chloro-1*H*-1,2,3-triazol-1-yl)aniline 24c (1.3 g, 5.68 mmol) was dissolved in dichloromethane (20 mL), added with triethylamine (3.45 g, 34.05 mmol, 4.73 mL), slowly dropwise added with 2-chloroacetyl chloride (769.17 mg, 6.81 mmol, 541.67 μL) at 0°C, reacted at 0°C for 1 hour, added with 30 mL of water to quench the reaction, then the reaction solution was subjected to liquid separation. An aqueous phase was extracted with dichloromethane (50 mL×3). Organic phases were combined and washed with saturated sodium chloride solution (50 mL), dried with anhydrous sodium sulfate, filtered, and concentrated under reduced pressure. The obtained residue was further separated and purified by silicagel column chromatography (eluent: system B) to obtain 2-chloro-*N*-(5-chloro-2-(4-chloro-1*H*-1,2,3-triazol-1-yl)phenyl)acetamide 24d (1 g) with a yield of 58%.
MS m/z (ESI): 304.8 [M+H]

Step 5

*Tert*-butyl (2-((5-chloro-2-(4-chloro-1*H*-1,2,3-triazol-1-yl)phenyl)amino)-2-oxoethyl)phenylalaninate

2-chloro-*N*-(5-chloro-2-(4-chloro-1*H*-1,2,3-triazol-1-yl)phenyl)acetamide 24d (1 g, 3.27 mmol) and *tert*-butyl L-phenylalaninate hydrochloride 1e (1.09 g, 4.91 mmol) were dissolved in N,N-dimethylformamide (20 mL), added with N,N-diisopropylethylamine (2.11 g, 16.36 mmol, 2.70 mL),and reacted at 80°C for 1 hour. After the reaction was completed, the reaction solution was added with 50 mL of water and extracted with ethyl acetate (50 mL×3). Organic phases were combined, washed with saturated sodium chloride solution (50 mL), dried with anhydrous sodium sulfate, filtered, and concentrated under reduced pressure. The obtained residue was further separated and purified by silicagel column chromatography (eluent: system B) to obtain *tert*-butyl (2-((5-chloro-2-(4-chloro-1*H*-1,2,3-triazol-1-yl)phenyl)amino)-2-oxoethyl)phenylalaninate 24e (1.3 g) with a yield of 81%.
MS m/z (ESI): 489.9 [M+H]

Step 6

*Tert*-butyl N-(2-((5-chloro-2-(4-chloro-1*H*-1,2,3-triazol-1-yl)phenyl)amino)-2-oxoethyl)-N-(2-chloroacetyl)phenylalaninate

*Tert-butyl* (2-((5-chloro-2-(4-chloro-1*H*-1,2,3-triazol-1-yl)phenyl)amino)-2-oxoethyl)phenylalaninate 24e (1.3 g, 2.65 mmol) was dissolved in dichloromethane (25 mL), added with triethylamine (1.61 g, 15.91 mmol, 2.21 mL), then slowly dropwise added with 2-chloroacetyl chloride (359.29 mg, 3.18 mmol, 253.02 μL) at 0°C and stirred at 0°C for reaction for 1 hour. After quenching with 30 mL of water, the reaction solution was subjected to liquid separation, and then an aqueous phase was extracted with dichloromethane (50 mL×3). Organic phases were combined, washed with saturated sodium chloride solution (50 mL), dried with anhydrous sodium sulfate, filtered, and concentrated under reduced pressure, and the obtained residue was further separated and purified by silicagel column chromatography (eluent: system B) to obtain *tert*-butyl *N*-(2-((5-chloro-2-(4-chloro-1*H*-1,2,3-triazol-1-yl)phenyl)amino)-2-oxoethyl)-*N*-(2-chloroacetyl)phenylalaninate 24f (1.1 g) with a yield of 73%.
MS m/z (ESI): 509.8[M+H-56]

Step 7

*Tert*-butyl 2-(4-(5-chloro-2-(4-chloro-1*H*-1,2,3-triazol-1-yl)phenyl)-2,5-dioxopiperazin-1-yl)-3-phenylpropanoate

*Tert*-butyl *N*-(2-((5-chloro-2-(4-chloro-1*H*-1,2,3-triazol-1-yl)phenyl)amino-yl)phenyl)amino)-2-oxoethyl)-*N*-(2-chloroacetyl)phenylalaninate 24f (1.1 g, 1.94 mmol) was dissolved in methanol (15 mL), slowly dropwise added with sodium methylate (395.29 μL, 2.134 mmol) at 0°C, and reacted at 0°C for 1 hour. After the reaction was completed, 2M dilute hydrochloric acid was added to adjust the pH to about 6, then the reaction solution was concentrated under reduced

pressure, and the obtained residue was further separated and purified by silica gel column chromatography (eluent: system B) to obtain *tert*-butyl 2-(4-(5-chloro-2-(4-chloro-1*H*-1,2,3-triazol-1-yl)phenyl)-2,5-dioxopiperazin-1-yl)-3-phenylpropanoate 24g (916 mg) with a yield of 89%.
MS m/z (ESI): 529.8 [M+H]

Step 8

2-(4-(5-chloro-2-(4-chloro-1*H*-1,2,3-triazol-1-yl)phenyl)-2,5-dioxopiperazin-1-yl)-3-phenylpropanoic acid

*Tert*-butyl 2-(4-(5-chloro-2-(4-chloro-1*H*-1,2,3-triazol-1-yl)phenyl)-2,5-dioxopiperazin-1-yl)-3-phenylpropanoate 24g (916 mg, 1.73 mmol) was dissolved in dichloromethane (10 mL), slowly dropwise added with trifluoroacetic acid (196.91 mg, 1.73 mmol, 3 mL), and reacted at room temperature for 1 hour. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-phenylpropanoic acid 24h (764 mg) with a yield of 93%.
MS m/z (ESI): 473.8 [M+H]

Step 9

2-(4-(5-chloro-2-(4-chloro-1*H*-1,2,3-triazol-1-yl)phenyl)-2,5-dioxopiperazin-1-yl)-3-phenyl-*N-(quinoxalin-6-yl)propanamide*

2-(4-(5-chloro-2-(4-chloro-1*H*-1,2,3-triazol-1-yl)phenyl)-2,5-dioxopiperazin-1-yl)-3-phenylpropanoic acid 24h (50 mg, 105.42 μmol) and quinoxalin-6-amine 18a (22.95 mg, 158.13 μmol) were dissolved in ethyl acetate (5 mL), added with N,N-diisopropylethylamine (81.75 mg, 632.52 μmol) and 1-propylphosphonic anhydride (201.25 mg, 316.26 μmol, 50% ethyl acetate solution), and reacted at 65°C for 3 hours. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-phenyl-*N*-(quinoxalin-6-yl)propanamide 24 (28 mg) with a yield of 44%.

MS m/z (ESI): 600.8 [M+H]
$^1$H NMR (400 MHz, DMSO-$d_6$) δ 10.69 (s, 1H), 8.90 (s, 1H), 8.84 (d, J = 1.9 Hz, 1H), 8.64 (s, 1H), 8.54 (s, 1H), 8.07 (d, J = 9.0 Hz, 1H), 7.94 (d, J = 9.1 Hz, 1H), 7.82-7.65 (m, 3H), 7.40-7.16 (m, 5H), 5.49-5.37 (m, 1H), 4.45-3.97 (m, 4H), 3.38-3.29 (m, 1H), 3.23-3.08 (m, 1H).

Example 25

2-(4-(5-Chloro-2-(4-chloro-1*H*-1,2,3-triazol-1-yl)phenyl)-2,5-dioxopiperazin-1-yl)-*N*-(2-methyl-2*H*-indazol-5-yl)-3-phenylpropanamide

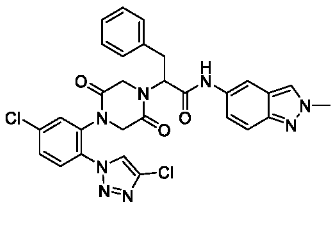

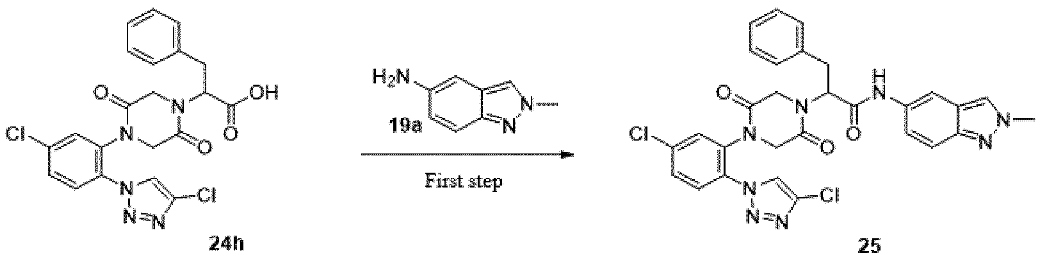


24h 25

2-(4-(5-Chloro-2-(4-chloro-1*H*-1,2,3-triazol-1-yl)phenyl)-2,5-dioxopiperazin-1-yl)-3-phenylpropanoic acid 24h (50 mg, 105.42 μmol) and 2-methyl-277-indazol-5-amine 19a (23.27 mg, 158.13 μmol) were dissolved in ethyl acetate (5 mL), added with N,N-diisopropylethylamine (81.75 mg, 632.52 μmol) and propylphosphonic anhydride (201.25 mg, 316.26 μmol, 50% ethyl acetate solution), and reacted at 65°C for 3 hours. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-phenylpropanamide 25 (28 mg) with a yield of 44%.

MS m/z (ESI): 602.8 [M+H]

$^1$H NMR (400 MHz, DMSO-$d_6$) δ 10.13 (s, 1H), 8.65 (s, 1H), 8.26 (s, 1H), 8.12 (s, 1H), 7.81-7.62 (m, 3H), 7.54 (d, J = 9.2 Hz, 1H), 7.40-7.15 (m, 6H), 5.47-5.31 (m, 1H), 4.42-3.92 (m, 4H), 4.13 (s, 3H), 3.36-3.21 (m, 1H), 3.16-3.03 (m, 1H).

Example 26

4-(2-(4-(5-Chloro-2-(4-chloro-1*H*-1,2,3-triazol-1-yl)phenyl)-2,5-dioxopiperazin-1-yl)-3-phenylpropanamido)benzoic acid

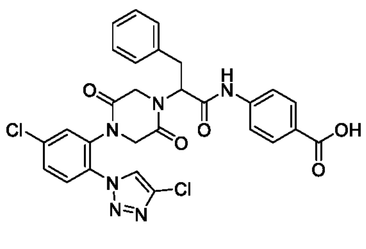

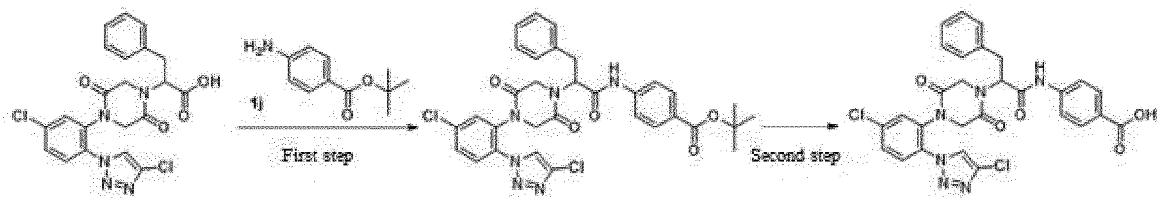


24h 26a 26

Step 1

*Tert*-butyl 4-(2-(4-(5-chloro-2-(4-chloro-1*H*-1,2,3-triazol-1-yl)phenyl)-2,5-dioxopiperazin-1-yl)-3-phenylpropanamido) benzoate

2-(4-(5-Chloro-2-(4-chloro-1*H*-1,2,3-triazol-1-yl)phenyl)-2,5-dioxopiperazin-1-yl)-3-phenylpropanoic acid 24h (50 mg, 105.42 μmol) and *tert*-butyl 4-aminobenzoate 1j (30.56 mg, 158.13 μmol) were dissolved in ethyl acetate (5 mL), added with N,N-diisopropylethylamine (68.12 mg, 527.10 μmol) and propylphosphonic anhydride (201.25 mg, 316.26 μmol, 50% ethyl acetate solution), and reacted at 60°C for 3 hours. After the reaction was completed, the reaction solution was concentrated under reduced pressure, and the obtained residue was further separated and purified by silicagel column chromatography (eluent: system B) to obtain tert-butyl 4-(2-(4-(5-chloro-2-(4-chloro-1*H*-1,2,3-triazol-1-

yl)phenyl)-2,5-dioxopiperazin-1-yl)-3-phenylpropanamido) benzoate 26a (34 mg) with a yield of 50%.
MS m/z (ESI): 648.8 [M+H]

Step 2

4-(2-(4-(5-chloro-2-(4-chloro-1*H*-1,2,3-triazol-1-yl)phenyl)-2,5-dioxopiperazin-1-yl)-3-phenylpropanamido)benzoic acid

*Tert-butyl* 4-(2-(4-(5 -chloro-2-(4-chloro-1*H*-1,2,3-triazol-1-yl)phenyl)-2,5-dioxopiperazin-1-yl)-3-phenylpropanamido) benzoate 26a (34 mg, 52.35 μmol) was dissolved in dichloromethane (10 mL), slowly dropwise added with trifluoroacetic acid (9.00 g, 78.93 mmol, 3 mL), and reacted at room temperature for 2 hours. After the reaction was completed, the reaction solution was concentrated under reduced pressure, and the obtained residue was further separated and purified by silicagel column chromatography (eluent: system B) to obtain 4-(2-(4-(5-chloro-2-(4-chloro-1*H*-1,2,3-triazol-1-yl)phenyl)-2,5-dioxiperazin-1-yl)-3-phenylpropanamido)benzoic acid 26 (8 mg) with a yield of 26%. MS m/z (ESI): 592.8 [M+H]
$^1$H NMR (400 MHz, DMSO-$d_6$) δ 12.75 (s, 1H), 10.49 (s, 1H), 8.64 (s, 1H), 7.91 (d, J = 8.4 Hz, 2H), 7.81-7.60 (m, 5H), 7.34-7.15 (m, 5H), 5.42-5.34 (m, 1H), 4.44-3.93 (m, 4H), 3.29-3.20 (m, 1H), 3.15-3.06 (m, 1H).

Example 27

*N*-(4-acetylphenyl)-2-(4-(5-chloro-2-(4-chloro-1*H*-1,2,3-triazol-1-yl)phenyl)-2,5-dioxopiperazin-1-yl)-3-phenylpropanamide

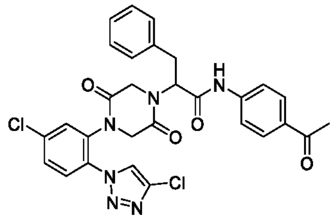

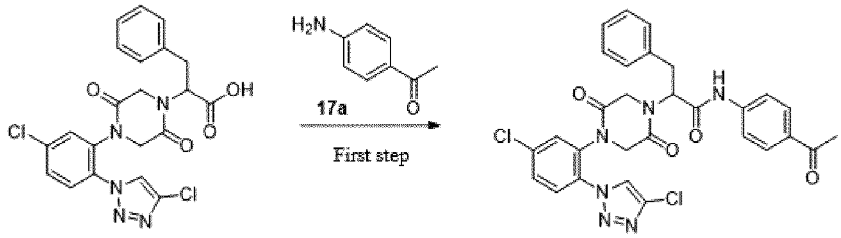


24h

27

2-(4-(5-Chloro-2-(4-chloro-1*H*-1,2,3-triazol-1-yl)phenyl)-2,5-dioxopiperazin-1-yl)-3-phenylpropanoic acid 24h (50 mg, 105.42 μmol) and 1-(4-aminophenyl)ethan-1-one 17a (21.37 mg, 158.13 μmol) were dissolved in ethyl acetate (5 mL), added with N,N-diisopropylethylamine (81.75 mg, 632.52 μmol) and propylphosphonic anhydride (201.25 mg, 316.26 μmol, 50% ethyl acetate solution), and reacted at 65°C for 1 hour. After the reaction was completed, the reaction solution was concentrated under reduced pressure, and the obtained residue was further separated and purified by silicagel column chromatography (eluent: system B) to obtain N-(4-acetylphenyl)-2-(4-(5-chloro-2-(4-chloro-1*H*-1,2,3-triazol-1-yl)phenyl)-2,5-dioxopiperazin-1-yl)-3-phenylpropanamide 27 (27 mg) with a yield of 41%.

MS m/z (ESI): 590.8 [M+H]
$^1$H NMR (400 MHz, DMSO-$d_6$) δ 10.51 (s, 1H), 8.64 (s, 1H), 7.95 (d, J = 8.8 Hz, 2H), 7.86-7.58 (m, 5H), 7.39-7.16 (m, 5H), 5.43-5.34 (m, 1H), 4.34-3.91 (m, 4H), 3.32-3.22 (m, 1H), 3.20-3.07 (m, 1H), 2.54 (s, 3H).

Example 28

2-(4-(5-Chloro-2-(4-chloro-1*H*-1,2,3-triazol-1-yl)phenyl)-2,5-dioxopiperazin-1-yl)-N-(1-methoxyisoquinolin-6-yl)-3-phenylpropanamide

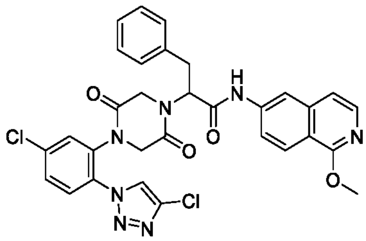

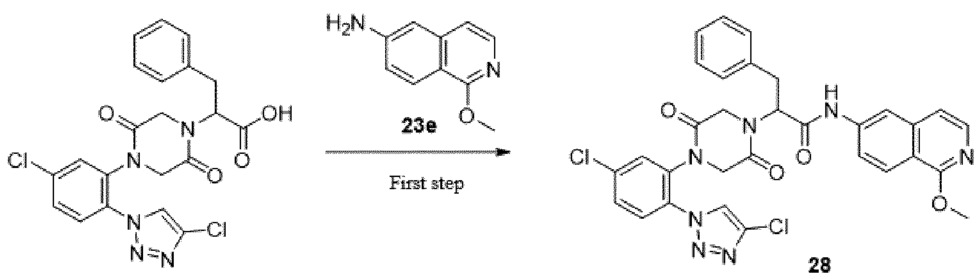


24h

28

2-(4-(5-Chloro-2-(4-chloro-1*H*-1,2,3-triazol-1-yl)phenyl)-2,5-dioxopiperazin-1-yl)-3-phenylpropanoic acid 24h (50 mg, 105.42 μmol), 1-methoxyisoquinolin-6-amine 23e (27.55 mg, 158.13 μmol), N,N-diisopropylethylamine (68.12 mg, 527.10 μmol, 87.11 μL) and propylphosphonic anhydride (50% ethyl acetate solution,201.26 mg, 316.26 μmol, 93.99 μL) were dissolved in ethyl acetate (5 mL) in turn, and continuously reacted under stirring at room temperature for 16 hours. After the reaction was completed, the reaction solution was concentrated under reduced pressure, and the obtained residue was subjected to preparative liquid phase purification (separation column AKZONOBEL Kromasil; 250×21.2 mm I.D.; 5 μm, 20 mL/min; mobile phase A: 0.05% TFA+$H_2O$, mobile phase B: $CH_3CN$) to obtain 2-(4-(5-chloro-2-(4-chloro-1*H*-1,2,3-triazol-1-yl)phenyl)-2,5-dioxopiperazin-1-yl)-*N*-(1-methoxyisoquinolin-6-yl)-3-phenylpropanamide 28 (23 mg) with ayield of 33%.

MS m/z (ESI): 630.2 [M+H]

$^1$H NMR (400 MHz, DMSO-$d_6$) δ 10.59 (s, 1H), 8.64 (s, 1H), 8.29 (s, 1H), 8.13 (d, J = 9.0 Hz, 1H), 7.96 (d, J= 5.9 Hz, 1H), 7.81-7.58 (m, 5H), 7.42-7.14 (m, 5H), 5.49 - 5.35 (s, 1H), 4.53-3.90 (m, 4H), 4.03 (s, 3H), 3.21-3.08 (m, 2H).

Example 29

5-(2-(4-(5-Chloro-2-(1*H*-tetrazol-1-yl)phenyl)-2,5-dioxopiperazin-1-yl)-3-phenylpropanamido)-*N*-methylpicolinamide

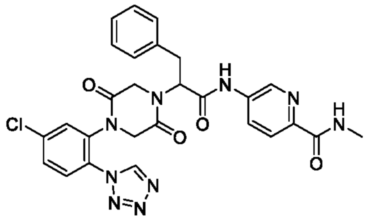

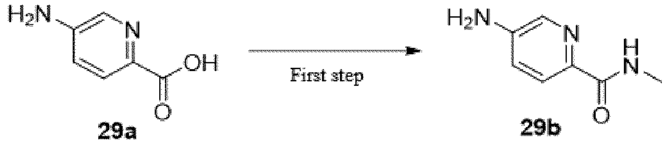


29a 29b

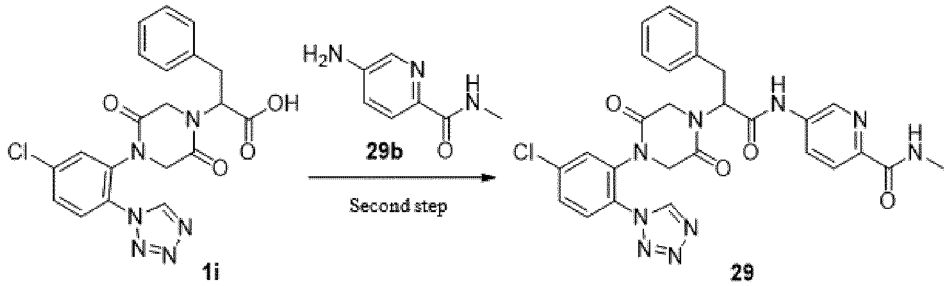


1i 29

Step 1

5-amino-*N*-methylpicolinamide

5-aminopicolinic acid 29a (500 mg, 3.62 mmol) and methylamine hydrochloride (367 mg, 5.43 mmol) were dissolved in N,N-dimethylformamide (20 mL), added with 2-(7-azobenzotriazole)-N,N,N',N'-tetramethylurea hexafluorophosphate (668 mg, 1.81 mmol) and triethylamine (2.51 mL, 18.1 mmol) in turn, and stirred at room temperature for reaction for 16 hours. After the reaction was completed, the reaction solution was added with water (50 mL) and extracted with ethyl acetate (50 mL×3). Organic phases were combined, washed with water (50 mL×2) and saturated sodium chloride solution (30 mL), dried with anhydrous sodium sulfate, filtered, and concentrated under reduced pressure. The obtained residue was further separated and purified by silicagel column chromatography (eluent: system B) to obtain 5-amino-*N*-methylpicolinamide 29b (260 mg) with a yield of 48%.
MS m/z (ESI): 152.1 [M+H]

Step 2

5-(2-(4-(5-Chloro-2-(1*H*-tetrazol-1-yl)phenyl)-2,5-dioxopiperazin-1-yl)-3-phenylpropanamido)-*N*-methylpicolinamide

2-(4-(5-Chloro-2-(1*H*-tetrazol-1-yl)phenyl)-2,5-dioxopiperazin-1-yl)-3-phenylpropanoic acid 1i (50 mg, 113.42 μmol), 5-amino-*N*-methylpicolinamide 29b (25.72 mg, 170.13 μmol), N,N-diisopropylethylamine (73.29 mg, 567.10 μmol, 93.72 μL) and propylphosphonic anhydride (50% ethyl acetate solution, 216.53 mg, 340.26 μmol, 101.22 μL) were dissolved in ethyl acetate (5 mL) in turn, and continuously stirred at room temperature for 16 hours. After the reaction was completed, the reaction solution was concentrated under reduced pressure, and the obtained residue was further separated and purified by silicagel column chromatography (eluent: system B) to obtain 5-(2-(4-(5-chloro-2-(1*H*-tetrazol-1-yl)phenyl)-2,5-dioxopiperazin-1-yl)-3-phenylpropanamido)-*N*-methylpicolinamide 29 (15 mg) with a yield of 22%.

MS m/z (ESI): 574.2 [M+H]
$^1$H NMR (400 MHz, DMSO-$d_6$) $\delta$ 10.61 (s, 1H), 9.72 (d, J = 10.0 Hz, 1H), 8.84 (s, 1H), 8.73-8.64 (m, 1H), 8.16 (dd, J = 8.6, 2.5 Hz, 1H), 8.01 (d, J = 8.5 Hz, 1H), 7.85-7.71 (m, 3H), 7.37-7.16 (m, 5H), 5.34-5.27 (m, 1H), 4.60-3.91 (m, 4H), 3.30-3.22 (m, 1H), 3.17-3.06 (m, 1H), 2.81 (d, J= 4.8 Hz, 3H).

Example 30

*N*-(4-acetyl-3-fluorophenyl)-2-(4-(5-chloro-2-(1*H*-tetrazol-1-yl)phenyl)-2,5-dioxopiperazin-1-yl)-3-phenylpropanamide

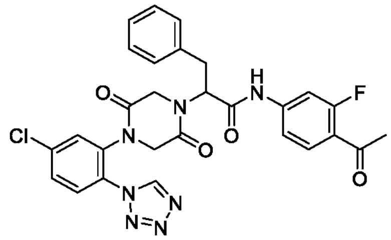

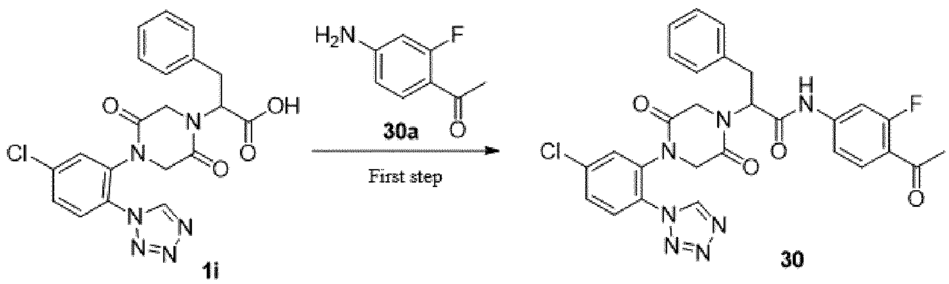


1i 30

2-(4-(5-Chloro-2-(1*H*-tetrazol-1-yl)phenyl)-2,5-dioxopiperazin-1-yl)-3-phenylpropanoic acid 1i (70 mg, 158.79 μmol) and 1-(4-amino-2-fluorophenyl)ethan-1-one 30a (36.48 mg, 238.18 μmol, prepared according to literature Synthesis (2018), 50(3), 555-564, page 557, Table 2) were dissolved in ethyl acetate (10 mL), added with N,N-diisopropylethylamine (123.13 mg, 952.73 μmol) and propylphosphonic anhydride (50% ethyl acetate solution, 303.14 mg, 476.36 μmol), and reacted at 60°C for 1 hour. After the reaction was completed, the reaction solution was concentrated under reduced pressure, and the obtained residue was further separated and purified by silicagel column chromatography (eluent: system B) to obtain *N*-(4-acetyl-3-fluorophenyl)-2-(4-(5-chloro-2-(1*H*-tetrazol-1-yl)phenyl)-2,5-dioxopiperazin-1-yl)-3-phenylpropanamide 30 (15 mg) with a yield of 16%. MS m/z (ESI): 575.8 [M+H]

$^1$H NMR (400 MHz, DMSO-$d_6$) δ 10.67 (s, 1H), 9.71 (s, 1H), 7.89-7.67 (m, 5H), 7.40 7.41 (d, J = 8.7 Hz, 1H), 7.37-7.20 (m, 5H), 5.35-5.24 (m, 1H), 4.63-3.89 (m, 4H), 3.31-3.18 (m, 1H), 3.17-3.03 (m, 1H), 2.54 (d, J = 4.2 Hz, 3H).

Example 31

2-(4-(5-Chloro-2-(1*H*-tetrazol-1-yl)phenyl)-2,5-dioxopiperazin-1-yl)-N-(2-methyl-2H-indazol-6-yl)-3 -phenylpropanamide

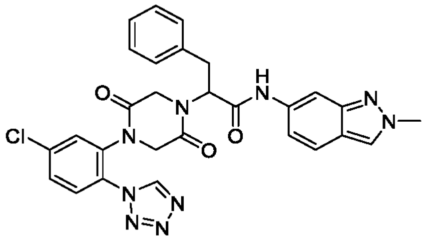

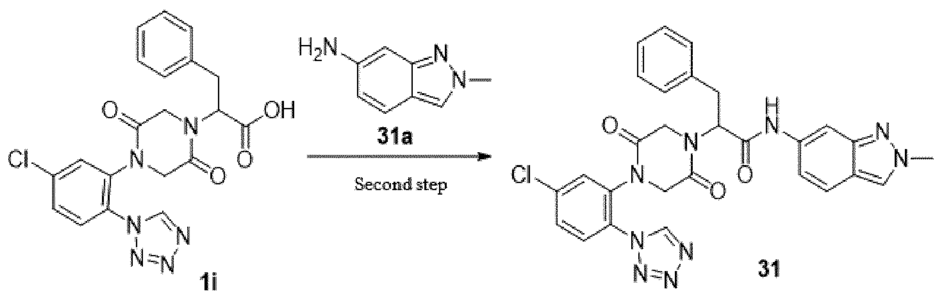


1i 31

2-(4-(5-Chloro-2-(1*H*-tetrazol-1-yl)phenyl)-2,5-dioxopiperazin-1-yl)-3-phenylpropanoic acid 1i (50 mg, 113.42

μmol), 2-methyl-2*H*-indazol-6-amine 31a (25.04 mg, 170.13 μmol), N,N-diisopropylethylamine (73.29 mg, 567.10 μmol, 93.72 μL) and propylphosphonic anhydride (50% ethyl acetate solution, 216.41 mg, 340.26 μmol, 202.44 μL) were dissolved in ethyl acetate (5 mL) in turn, and continuously reacted under stirring at room temperature for 16 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-N-(2-methyl-2*H*-indazol-6-yl)-3-phenylpropanamide 31 (21.7 mg) with a yield of 33%.

MS m/z (ESI): 569.9 [M+H]
$^1$H NMR (400 MHz, DMSO-$d_6$) δ 10.19 (s, 1H), 9.72 (s, 1H), 8.26 (s, 1H), 8.01 (s, 1H), 7.82 (d, J = 8.5 Hz, 1H), 7.75 (dd, J = 8.6, 2.1 Hz, 1H), 7.63 (d, J = 8.9 Hz, 1H), 7.39-7.17 (m, 5H), 7.08 (d, J = 9.1 Hz, 1H), 5.38 (dd, J = 10.1, 5.8 Hz, 1H), 4.54-4.07 (m, 4H), 4.13 (s, 3H), 3.99 (d, J = 17.2, 1H), 3.33-3.19 (m, 1H), 3.18-3.04 (m, 1H).

Example 32

2-(4-(5-Chloro-2-(1*H*-tetrazol-1-yl)phenyl)-2,5-dioxopiperazin-1-yl)-4-methoxy-*N*-(2-methyl-2*H*-indazol-5-yl)butanamide

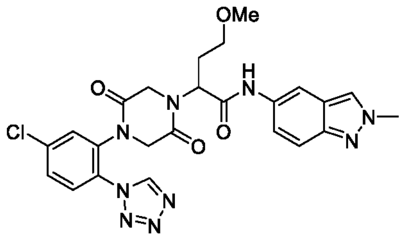

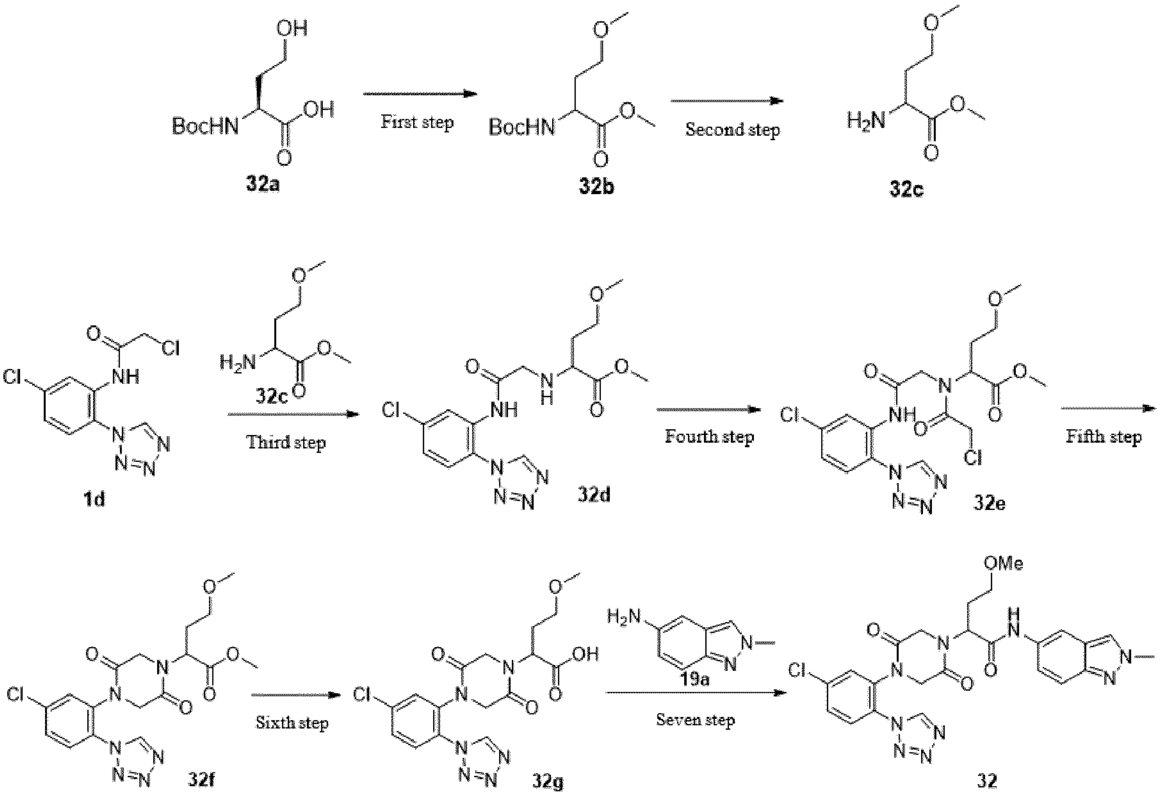

Step 1

Methyl *N*-(*tert*-butoxycarbonyl) O-methylhomoserinate

N-Boc-L-homoserine 32a (1 g, 4.56 mmol) was dissolved in N,N-dimethylformamide (20 mL), added with sodium hydride (383.15 mg, 9.58 mmol, 60%) in ice bath, reacted for 20 minutes, then slowly dropwise added with methyl iodide (2.59 g, 18.25 mmol, 1.14 mL), and reacted at room temperature overnight. The reaction solution was added with water for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system A) to obtain methyl *N*-(*tert*-butoxycarbonyl) O-methylhomoserinate 32b (370 mg) with a yield of 32%.
MS m/z (ESI): 147.1 [M-100]

Step 2

Methyl O-methylhomoserinate

Methyl *N*-(*tert*-butoxycarbonyl) O-methylhomoserinate 32b (370 mg, 1.50 mmol) was dissolved in dichloromethane (8 mL), slowly dropwise added with trifluoroacetic acid (2 mL), and reacted for 2 hours at room temperature. After the reaction was completed, the reaction solution was concentrated under reduced pressure to obtain methyl O-methylhomoserinate 32c (3 84 mg), which was directly used for next step.
MS m/z (ESI):148.1 [M+H]

Step 3

Methyl *N*-(2-((5-chloro-2-(1*H*-tetrazol-1-yl)phenyl)amino)-2-oxoethyl)- O-methylhomoserinate

2-Chloro-*N*-(5-chloro-2-(1*H* tetrazol-1-yl)phenyl)acetamide 1d (369.76 mg, 1.36 mmol) and methyl O-methylhomoserinate 32c (390 mg, 1.49 mmol) were dissolved in N,N-dimethylformamide (10 mL), added with N,N-diisopropylethylamine (1.05 g, 8.15 mmol, 1.35 mL), and reacted at 80°C overnight. The reaction solution was added with water for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system B) to obtain methyl *N*-(2-((5-chloro-2-(1*H*-tetrazol-1-yl)phenyl)amino)-2-oxoethyl)-O-methylhomoserinate 32d (400 mg) with a yield of 77%.
MS m/z (ESI): 382.9 [M+H]

Step 4

Methyl *N*-(2-((5-chloro-2-(1*H*-tetrazol-1-yl)phenyl)amino)-2-oxoethyl)-*N*-(2-chloroacetyl)-O-methylhomoserinate

Methyl *N*-(2-((5-chloro-2-(1*H*-tetrazol-1-yl)phenyl)amino)-2-oxoethyl)-O-methylhomoserinate 32d (400 mg, 1.04 mmol) was dissolved in dichloromethane (10 mL), added with triethylamine (528.68 mg, 5.22 mmol, 726.21 μL), then slowly dropwise added with chloroacetyl chloride (177.03 mg, 1.57 mmol, 124.67 μL) in ice bath, and reacted in ice bath for 1 hour. The reaction solution was added with water for quenching the reaction, and extracted with dichloromethane (30 mL×3). Organic phases were combined, dried with anhydrous sodium sulfate, filtered and concentrated under reduced pressure, and the obtained residue was further separated and purified by silicagel column chromatography (eluent: system B) to obtain methyl *N*-(2-((5-chloro-2-(1*H*-tetrazol-1-yl)phenyl)amino)-2-oxoethyl)-*N*-(2-chloroacetyl)-O-methylhomoserinate 32e (194 mg) with a yield of 41%.
MS m/z (ESI): 458.9 [M+H]

Step 5

Methyl 2-(4-(5-chloro-2-(1*H*-tetrazol-1-yl)phenyl)-2,5-dioxopiperazin-1-yl)- 4-methoxybutanoate

Methyl N-(2-((5-chloro-2-(1*H*-tetrazol-1-yl)phenyl)amino)-2-oxoethyl)-*N*-(2-chloroacetyl)-O-methylhomoserinate 32e (194 mg, 422.40 μmol) was dissolved in methanol (5 mL), slowly dropwise added with sodium methylate (5.4M

methanol solution, 86.04 μL, 2.28 mmol) in ice bath, and reacted in ice bath for 30 minutes. After the reaction was completed, 2M dilute hydrochloric acid was dropwise added to adjust the pH to 6, then the reaction solution was concentrated under reduced pressure, and the obtained residue was further separated and purified by silica gel column chromatography (eluent: system B) to obtain methyl 2-(4-(5-chloro-2-(1*H*-tetrazol-1-yl)phenyl)-2,5-dioxopiperazin-1-yl)-4-methoxybutanoate 32f (100 mg) with a yield of 56%.
MS m/z (ESI): 422.9 [M+H]

Step 6

2-(4-(5-Chloro-2-(1*H*-tetrazol-1-yl)phenyl)-2,5-dioxopiperazin-1-yl)-4-methoxybutanoic acid

Methyl 2-(4-(5-chloro-2-(1*H*-tetrazol-1-yl)phenyl)-2,5-dioxopiperazin-1-yl)- 4-methoxybutanoate 32f (100 mg, 236.51 μmol) was dissolved in mixed solution of tetrahydrofuran, methanol and water (3 mL+1 mL+1 mL), added with lithium hydroxide monohydrate (19.85 mg, 473.01 μmol) in ice bath, and reacted in ice bath for 1 hour. After the reaction was completed, 2M dilute hydrochloric acid was dropwise added to adjust the pH to 6, then the reaction solution was concentrated under reduced pressure, and the obtained residue was further separated and purified by silica gel column chromatography (eluent: system B) to obtain 2-(4-(5-chloro-2-(1*H*-tetrazol-1-yl)phenyl)-2,5-dioxopiperazin-1-yl)-4-methoxybutanoic acid 32g (65 mg) with ayield of 65%.
MS m/z (ESI): 408.9 [M+H]

Step 7

2-(4-(5-Chloro-2-(1*H*-tetrazol-1-yl)phenyl)-2,5-dioxopiperazin-1-yl)-4-methoxy-*N*-(2-methyl-2*H*-indazol-5-yl)butanamide

2-(4-(5-Chloro-2-(1*H*-tetrazol-1-yl)phenyl)-2,5-dioxpiperazin-1-yl)-4-methoxybutanoic acid 32g (65 mg, 159.00 μmol) and 2-methyl-2*H*-indazol-5-amine 19a (35.10 mg, 238.51 μmol) were dissolved in ethyl acetate (5 mL), added with propylphosphonic anhydride (50% ethyl acetate solution, 303.55 mg, 477.01 μmol) and N,N-diisopropylethylamine (123.30 mg, 954.02 μmol, 157.67 μL), and reacted at 60°C for 2 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-4-methoxy-*N*-(2-methyl-2*H*-indazol-5-yl)butanamide 32 (16 mg) with a yield of 17%.

MS m/z (ESI): 537.9 [M+H]
$^1$H NMR (400 MHz, DMSO-$d_6$) δ 10.07 (s, 1H), 9.81 (s, 1H), 8.25 (s, 1H), 8.08 (s, 1H), 7.91 (s, 1H), 7.88-7.73 (m, 2H), 7.53 (d, J = 9.1 Hz, 1H), 7.27 (d, J = 9.3 Hz, 1H), 5.23-5.01 (s, 1H), 4.80-4.00 (m, 4H), 4.13 (s, 3H),3.97-3.69(m, 2H) 3.28 (s, 3H), 2.18-1.91 (m, 2H).

Example 33

2-(4-(5-chloro-2-(1*H*-tetrazol-1-yl)phenyl)-2,5-dioxopiperazin-1-yl)-*N*-(1-(difluoromethyl)-1*H*-indazol-5-yl)-3-phenylpropanamide

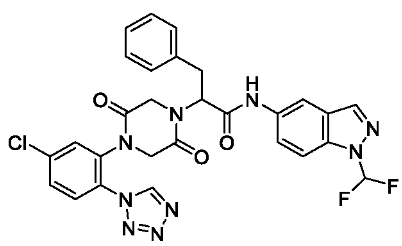

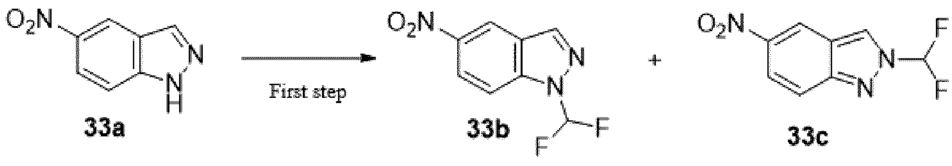


33a 33b 33c

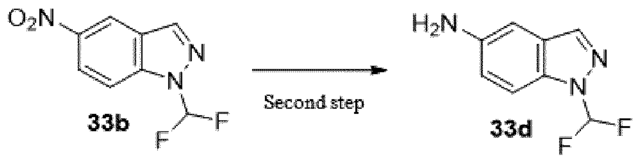


33b 33d

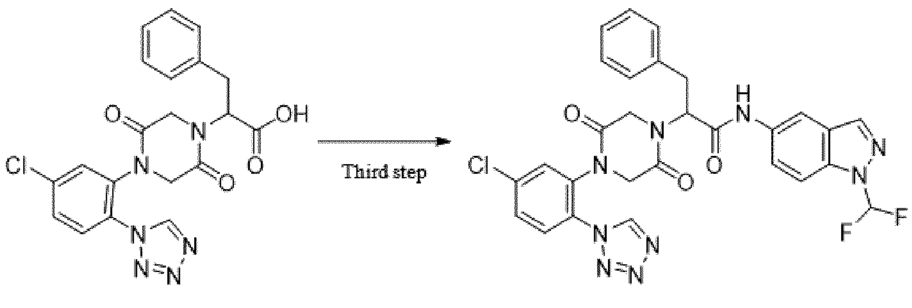


1i 33

Step 1

1-Difluoromethyl-5-nitroindazole 33b

2-Difluoromethyl-5-nitroindazole 33c

5-nitroindazole 33a (3 g, 18.39 mmol) was dissolved in N,N-dimethylformamide (80 mL), added with sodium hydride (1.47 g, 36.78 mmol, 60%) in ice bath, stirred for 10 minutes in ice bath, then added with sodium difluorochloroacetate (5.61 g, 36.78 mmol), reacted at room temperature for 30 minutes after the addition, and then heated to 80°C and reacted for 2 hours. The reaction solution was added with water for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system A) to obtain 1-difluoromethyl-5-nitroindazole 33b (1.3 g) with a yield of 33%; and 2-difluoromethyl-5-nitroindazole 33c (1.7 g) with a yield of 43%.

**33b** MS m/z (ESI): 213.9 [M+H]
**33c** MS m/z (ESI): 213.9 [M+H]

Step 2

1-(Difluoromethyl)-1*H*-indazol-5-amine

1-difluoromethyl-5-nitroindazole 33b (300 mg, 1.41 mmol) was dissolved in a mixed solvent of ethanol (24 mL) and water (6 mL), added with iron powder (786.10 mg, 14.08 mmol) and ammonium chloride (150.58 mg, 2.82 mmol), and reacted at 80°C for 2 hours. After the reaction was completed, the reaction solution was filtered and concentrated under reduced pressure, and the obtained residue was further separated and purified by silicagel column chromatography (eluent: system A) to obtain 1-(difluoromethyl)-1*H*-indazol-5-amine 33d (214 mg) with a yield of 83%.
MS m/z (ESI): 184.0 [M+H]

Step 3

2-(4-(5-Chloro-2-(1*H*-tetrazol-1-yl)phenyl)-2,5-dioxopiperazin-1-yl)-*N*-(1-(difluoromethyl)-1*H*-indazol-5-yl)-3-phenylpropanamide

2-(4-(5-Chloro-2-(1*H*-tetrazol-1-yl)phenyl)-2,5-dioxopiperazin-1-yl)-3-phenylpropanoic acid 1i (75 mg, 170.13 μmol) and 1-(difluoromethyl)-1*H*-indazol-5-amine 33d (47 mg, 255.2 μmol) were dissolved in ethyl acetate (8 mL), added with propylphosphonic anhydride (50% ethyl acetate solution, 324.61 mg, 510.39 μmol) and N,N-diisopropylethylamine (131.93 mg, 1.02 mmol, 168.70 μL), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-*N*(1-(difluoromethyl)-1*H*-indazol-5-yl)-3-phenylpropanamide 33 (12 mg) with a yield of 11%.

MS m/z (ESI): 605.8 [M+H]
$^1$H NMR (400 MHz, DMSO-$d_6$) δ 10.38 (s, 1H), 9.73 (s, 1H), 8.40 (s, 1H), 8.24 (s, 1H), 8.14 (t, J = 58.3 Hz, 1H), 7.96-7.54 (m, 5H), 7.49-7.09 (m, 5H), 5.36 (s, 1H), 4.64-3.88 (m, 4H), 3.35-3.19 (m, 1H), 3.17-3.00 (m, 1H).

Example 34

2-(4-(5-Chloro-2-(1*H*-tetrazol-1-yl)phenyl)-2,5-dioxopiperazin-1-yl)-*N*-(2-(difluoromethyl)-2*H*-indazol-5-yl)-3-phenylpropanamide

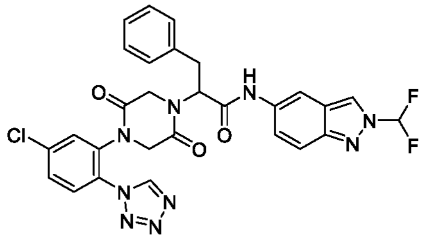

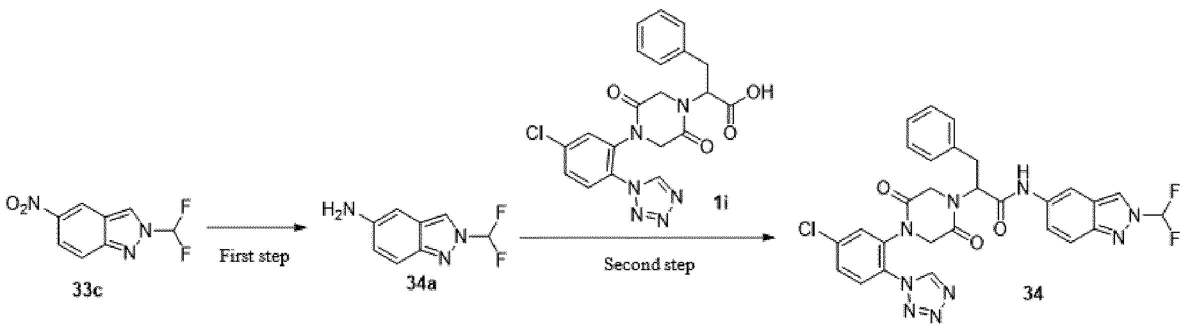


Step 1

2-(Difluoromethyl)-2*H*-indazol-5-amine

2-Difluoromethyl-5-nitroindazole 33c (300 mg, 1.41 mmol) was dissolved in a mixed solvent of ethanol (24 mL) and water (6 mL), added with iron powder (786.10 mg, 14.08 mmol) and ammonium chloride (150.58 mg, 2.82 mmol), and reacted at 80°C for 2 hours. After the reaction was completed, the reaction solution was filtered, the filtrate was concentrated under reduced pressure, and the obtained residue was further separated and purified by silicagel column chromatography (eluent: system B) to obtain 2-(difluoromethyl)-2*H*-indazol-5-amine 34a (200 mg) with a yield of 78%.
MS m/z (ESI): 184.0 [M+H]

Step 2

2-(4-(5-Chloro-2-(1*H*-tetrazol-1-yl)phenyl)-2,5-dioxiperazin-1-yl)-*N*-(2-(difluoromethyl)-2*H*-indazol-5-yl)-3-phenylpropanamide

2-(4-(5-Chloro-2-(1*H*-tetrazole-1-yl)phenyl)-2,5-dioxopiperazin-1-yl)-3-phenylpropanoic acid 1i (75 mg, 170.13 μmol) and 2-(difluoromethyl)-2*H*-indazol-5-ainine 34a (47 mg, 255.2 μmol) were dissolved in ethyl acetate (8 mL), added with propylphosphonic anhydride (50% ethyl acetate solution, 324.61 mg, 510.39 μmol) and N,N-diisopropylethylamine (131.93 mg, 1.02 mmol, 168.70 μL), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-*N*-(2-(difluoromethyl)-2*H*-indazol-5-yl)-3-phenylpropanamide 34 (20 mg) with a yield of 19%.

MS m/z (ESI): 605.8 [M+H]
$^1$H NMR (400 MHz, DMSO-$d_6$) δ 10.25 (s, 1H), 9.72 (d, J = 1.6 Hz, 1H), 8.81 (s, 1H), 8.22 (s, 1H), 8.10 (t, J = 59.0 Hz, 1H), 7.90-7.59 (m, 4H), 7.39 (d, J = 9.4 Hz, 1H), 7.37-7.17 (m, 5H), 5.48-5.26 (m, 1H), 4.73-3.87 (m, 4H), 3.31-3.19 (m, 1H), 3.17-3.02 (m, 1H).

Example 35

4-(2-(4-(5-Chloro-2-(1*H*-tetrazol-1-yl)phenyl)-2,5-dioxopiperazin-1-yl)-3-phenylpropanamido)-2-fluorobenzamide

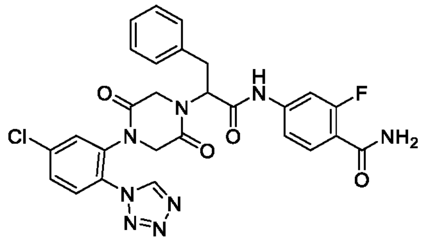

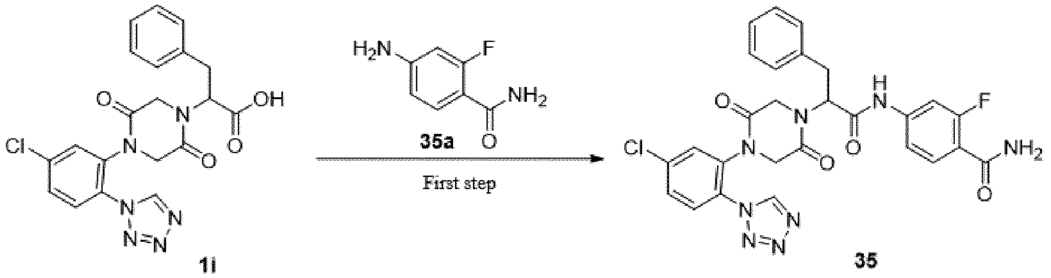


1i

35

2-(4-(5-Chloro-2-(1*H*-tetrazol-1-yl)phenyl)-2,5-dioxopiperazin-1-yl)-3-phenylpropanoic acid 1i (60 mg, 136.10 μmol) and 4-amino-2-fluorobenzamide 35a (31.47 mg, 204.16 μmol, prepared according to the published patent US 20170275282 A1, page 19 of the specification, paragraphs [0503]-[0505], Example 1.1B) were dissolved in ethyl acetate (6 mL), added with propylphosphonic anhydride (50% ethyl acetate solution, 259.83 mg, 408.31 μmol) and *N,N*-diisopropylethylamine (87.95 mg, 680.52 μmol), and reacted at 60°C for 16 hours. After the reaction was completed, the reaction solution was concentrated under reduced pressure, and the obtained residue was further separated and purified by silicagel column chromatography (eluent: system B) to obtain 4-(2-(4-(5-chloro-2-(1*H*-tetrazol-1-yl)phenyl)-2,5-dioxopiperazin-1-yl)-3-phenylpropanamido)-2-fluorobenzamide 35 (21 mg) with a yield of 27%.

MS m/z (ESI): 576.8 [M+H]
$^1$H NMR (400 MHz, DMSO-$d_6$) δ 10.55 (s, 1H), 9.72 (s, 1H), 7.84-7.60 (m, 5H), 7.60-7.50 (m, 2H), 7.41-7.18 (m, 6H), 5.39-5.21 (m, 1H), 4.57-3.86 (m, 4H), 3.29-3.16 (m, 1H), 3.15-3.04 (m, 1H).

Example 36

4-(2-(4-(5-Chloro-2-(4-chloro-1*H*-1,2,3-triazol-1-yl)phenyl)-2,5-dioxopiperazin-1-yl)-3-phenylpropanamido)-2-fluorobenzamide

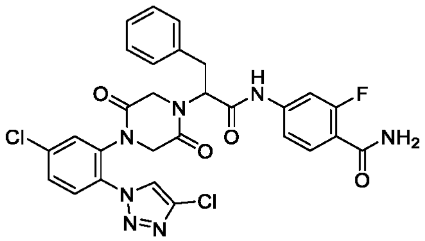

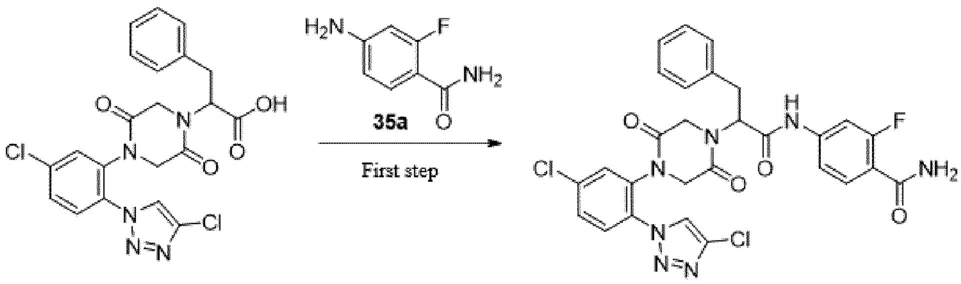


24h

36

2-(4-(5-Chloro-2-(4-chloro-1*H*-1,2,3-triazol-1-yl)phenyl)-2,5-dioxopiperazin-1-yl)-3-phenylpropanoic acid 24h (50 mg, 105.42 μmol) and 4-amino-2-fluorobenzamide 35a (24.37 mg, 158.13 μmol, prepared according to the published patent US 20170275282 A1, page 19 of the specification, paragraphs [0503]-[0505], Example 1.1B) were dissolved in ethyl acetate (5 mL), added with N,N-diisopropylethylamine (81.75 mg, 632.52 μmol) and propylphosphonic anhydride (50% ethyl acetate solution, 201.25 mg, 316.26 μmol), and reacted at 60°C for 3 hours. After the reaction was completed, the reaction solution was concentrated under reduced pressure, and the obtained residue was further separated and purified by silicagel column chromatography (eluent: system B) to obtain 4-(2-(4-(5-chloro-2-(4-chloro-1*H*-1,2,3-triazol-1-yl)phenyl)-2,5-dioxopiperazin-1-yl)-3-phenylpropanamido)-2-fluorobenzamide 36 (17 mg) with a yield of 26%.

MS m/z (ESI): 609.8 [M+H]

$^1$H NMR (400 MHz, DMSO-$d_6$) δ 10.55 (s, 1H), 8.64 (s, 1H), 7.86-7.61 (m, 5H), 7.60-7.46 (m, 2H), 7.44-7.18 (m, 6H), 5.44-5.24 (m, 1H), 4.54-3.91 (m, 4H), 3.29-3.22 (m, 1H), 3.19-3.05 (m, 1H).

Example 37

2-(4-(5-Chloro-2-(1*H*-tetrazol-1-yl)phenyl)-2,5-dioxopiperazin-1-yl)-3-phenyl-*N*-(pyrazolo[1,5-a]pyridin-5-yl)propanamide

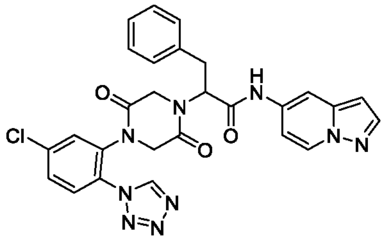

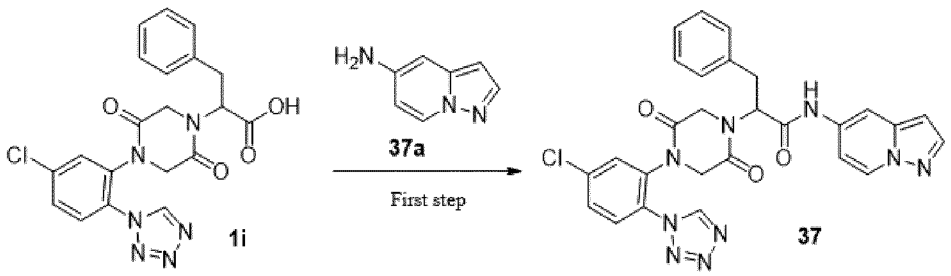

1i
37

2-(4-(5-Chloro-2-(1*H*-tetrazol-1-yl)phenyl)-2,5-dioxopiperazin-1-yl)-3-phenylpropanoic acid 1i (80 mg, 181.47 μmol) and pyrazolo[1,5-a]pyridin-5-amine 37a (36.24 mg, 272.21 μmol) were dissolved in ethyl acetate (5 mL), added with propylphosphonic anhydride (50% ethyl acetate solution,346.45 mg, 544.42 μmol) and N,N-diisopropylethylamine (117.27 mg, 907.36 μmol), and reacted at 60°C for 2 hours. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-3-phenyl-N-(pyrazolo[1,5-a]pyridin-5-yl)propanamide 37 (50 mg) with a yield of 49%.

MS m/z (ESI): 555.7 [M+H]

$^1$H NMR (400 MHz, DMSO-$d_6$) δ 10.44 (s, 1H), 9.72 (s, 1H), 8.61 (d, J = 7.5 Hz, 1H), 8.10 (s, 1H), 7.93 (s, 1H), 7.89-7.65 (m, 2H), 7.44-7.13 (m, 6H), 6.92 (d, J = 7.5 Hz, 1H), 6.52 (s, 1H), 5.41-5.25 (m, 1H), 4.71-3.91 (m, 4H), 3.30-3.17 (m, 1H), 3.15-3.03 (m, 1H).

Example 38

2-(4-(5-Chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide

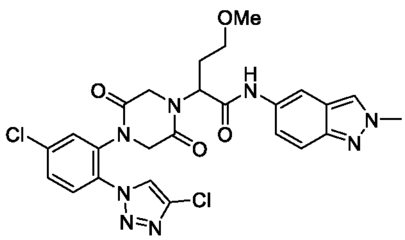

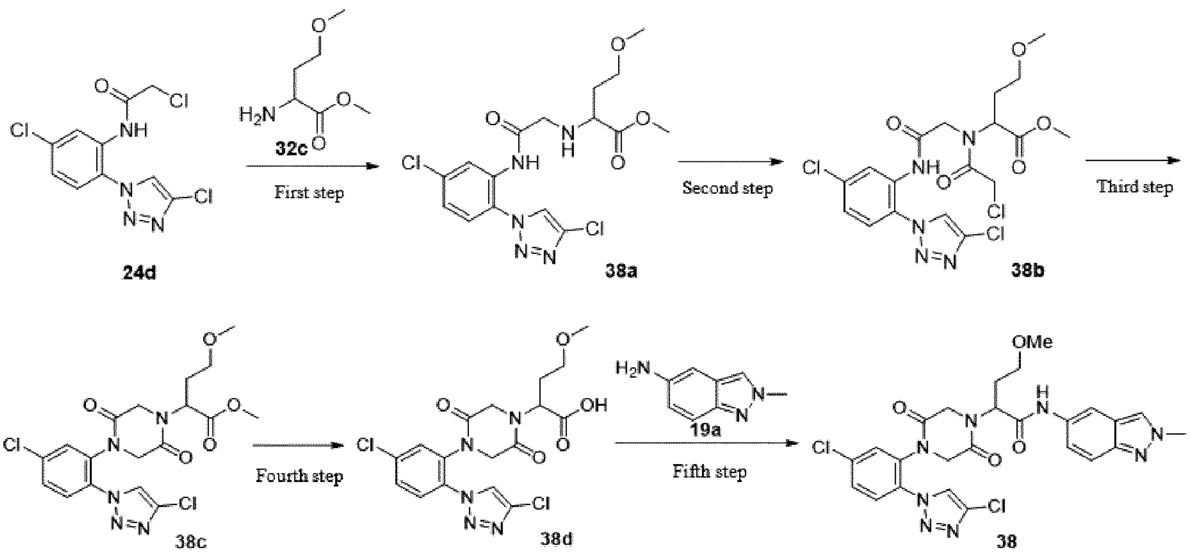

24d
38a
38b
38c
38d
38

Step 1

Methyl *N*-(2-((5-chloro-2-(4-chloro-1*H*-1,2,3-triazol-1-yl)phenyl)amino)-2-oxoethyl)- O-methylhomoserinate

2-Chloro-*N*-(5-chloro-2-(4-chloro-1*H*-1,2,3-triazol-1-yl)phenyl)acetamide 24d (1 g, 3.27 mmol) and methyl O-methylhomoserinate 32c (1.71 g, 6.55 mmol) were dissolved in N,N-dimethylformamide (15 mL), added with N,N-diisopropylethylamine (2.11 g, 16.36 mmol, 2.70 mL), and reacted at 75°C overnight. The reaction solution was added with 30 mL of water for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system A) to obtain methyl *N*-(2-((5-chloro-2-(4-chloro-1*H*-1,2,3-triazol-1-yl)phenyl)amino)-2-oxoethyl)- O-methylhomoserinate 38a (1.1 g) with a yield of 81%.
MS m/z (ESI): 415.9 [M+H]

Step 2

Methyl *N*-(2-((5-chloro-2-(4-chloro-1*H*-1,2,3-triazol-1-yl)phenyl)amino)-2-oxoethyl)-*N*-(2-chloroacetyl)- O-methylhomoserinate

Methyl *N*-(2-((5-chloro-2-(4-chloro-1*H*-1,2,3-triazol-1-yl)phenyl)amino)-2-oxoethyl)- O-methylhomoserinate 38a (400 mg, 1.04 mmol) was dissolved in acetonitrile (8 mL), added with potassium carbonate (1.66 g, 12.01 mmol), slowly dropwise added with chloroacetyl chloride (542.66 mg, 4.80 mmol, 382.15 μL) in ice bath, and reacted for 1 hour in ice bath. The reaction solution was added with 30 mL of water for quenching, and extracted with ethyl acetate (30 mL×3), and then organic phases were combined, dried with anhydrous sodium sulfate, filtered, and concentrated under reduced pressure. The obtained residue was further separated and purified by silicagel column chromatography (eluent: system A) to obtain methyl *N*-(2-((5-chloro-2-(4-chloro-1*H*-1,2,3-triazol-1-yl)phenyl)amino)-2-oxoethyl)-*N*-(2-chloroacetyl)-O-methylhomoserinate 38b (700 mg) with a yield of 59%.
MS m/z (ESI): 491.8 [M+H]

Step 3

Methyl 2-(4-(5-chloro-2-(4-chloro-1*H*-1,2,3-triazol-1-yl)phenyl)-2,5-dioxopiperazin-1-yl)-4-methoxybutanoate

Methyl *N*-(2-((5-chloro-2-(4-chloro-1*H*-1,2,3-triazol-1-yl)phenyl)amino)-2-oxoethyl)-*N*-(2-chloroacetyl)- O-methylhomoserinate 38b (650 mg, 1.32 mmol) was dissolved in methanol (20 mL), slowly dropwise added with sodium methylate (5.4M methanol solution, 268.72 μL, 1.45 mmol) in ice bath, and reacted in ice bath for 30 minutes. 2M dilute hydrochloric acid was dropwise added to adjust the pH to 6, then the reaction solution was concentrated under reduced pressure, and the obtained residue was further separated and purified by silicagel column chromatography (eluent: system A) to obtain methyl 2-(4-(5-chloro-2-(4-chloro-1*H*-1,2,3-triazol-1-yl)phenyl)-2,5-dioxopiperazin-1-yl)-4-methoxybutanoate 38c (505 mg) with a yield of 84%.
MS m/z (ESI): 455.8 [M+H]

Step 4

2-(4-(5-Chloro-2-(4-chloro-1*H*-1,2,3-triazol-1-yl)phenyl)-2,5-dioxopiperazin-1-yl)-4-methoxybutanoic acid

Methyl 2-(4-(5-chloro-2-(4-chloro-1*H*-1,2,3-triazol-1-yl)phenyl)-2,5-dioxopiperazin-1-yl)-4-methoxybutanoate 38c (470 mg, 1.03 mmol) was dissolved in mixed solvent of tetrahydrofuran (9 mL), methanol (3 mL) and water (3 mL), slowly dropwise added with lithium hydroxide monohydrate (86.44 mg, 2.06 mmol) in ice bath, and reacted in ice bath for 2 hours. 2M dilute hydrochloric acid was dropwise added to adjust the pH to 6, then the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-4-methoxybutanoic acid 38d (346 mg) with a yield of 76%.
MS m/z (ESI): 442.1 [M+H]

Step 5

2-(4-(5-Chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide

2-(4-(5-Chloro-2-(4-chloro-1*H*-1,2,3-triazol-1-yl)phenyl)-2,5-dioxopiperazin-1-yl)-4-methoxybutanoic acid 38d (100 mg, 226.12 μmol) and 2-methyl-2*H*-indazol-5-amine 19a (49.92 mg, 339.17 μmol) were dissolved in ethyl acetate (8 mL), added with propylphosphonic anhydride (50% ethyl acetate solution, 431.67 mg, 678.35 μmol) and N,N-diisopropylethylamine (175.34 mg, 1.36 mmol, 224.22 μL), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide 38 (65 mg) with a yield of 50%.

MS m/z (ESI): 570.8 [M+H]

$^1$H NMR (400 MHz, DMSO-$d_6$) δ 10.06 (s, 1H), 8.74 (s, 1H), 8.25 (s, 1H), 8.09 (s, 1H), 7.87 (s, 1H), 7.80-7.68 (m, 2H), 7.53 (d, J = 9.1 Hz, 1H), 7.27 (d, J = 9.4 Hz, 1H), 5.12 (s, 1H), 4.69-3.95 (m, 4H), 4.13 (s, 3H), 3.49-3.40 (m, 1H), 3.39-3.15 (m, 1H), 3.26 (s, 3H), 2.19-1.96 (m, 2H).

Example 39

2-(4-(5-Chloro-2-(1*H*-tetrazol-1-yl)phenyl)-2,5-dioxopiperazin-1-yl)-3-cyclobutyl-*N*-(2-methyl-2*H*-indazol-5-yl)propanamide

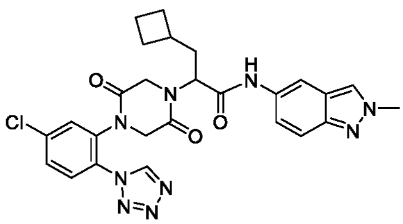

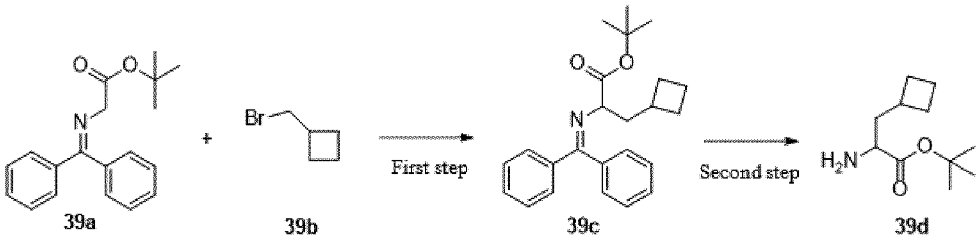


39a 39b 39c 39d

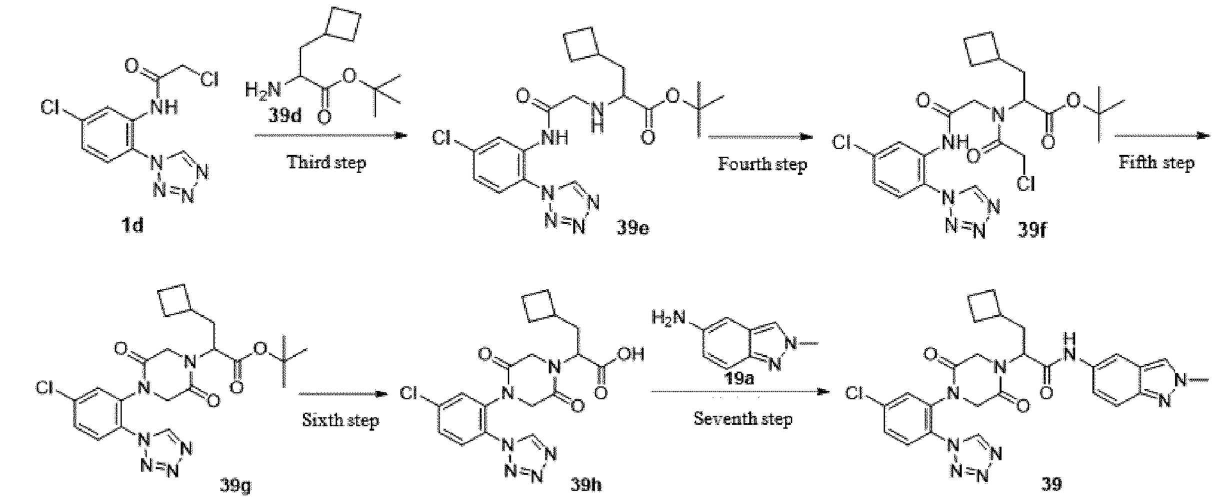


Step 1

*Tert*-butyl 3-cyclobutyl-2-((diphenylmethylene)amino) propanoate

*Tert*-butyl 2-((diphenylmethylene)amino)acetate 39a (3 g, 10.16 mmol) was dissolved in N,N-dimethylformamide (30 mL), added with potassium tert-butoxide (1.25 g, 11.17 mmol) in batches at 0°C, reacted for 15 minutes at 0°C, then added with (bromomethyl)cyclobutane 39b (1.66 g, 11.17 mmol, 1.25 mL), reacted to room temperature and then reacted for 3 hours. The reaction solution was added with 30 mL of water for quenching the reaction and extracted with ethyl acetate (30 mL×3), then organic phases were combined, washed with water (50 mL×3) and saturated sodium chloride solution (50 mL), dried with anhydrous sodium sulfate, filtered, and concentrated under reduced pressure to obtain crude product of *tert*-butyl 3-cyclobutyl-2-((diphenylmethylene)amino) propanoate 39c (2.9 g), which was directly used for next reaction.
MS m/z (ESI): 364.2 [M+H]

Step 2

Ethyl 2-amino-3-cyclobutylpropanoate

The crude product of *tert*-butyl 3-cyclobutyl-2-((diphenylmethylene)amino) propanoate 39c obtained in the last step was dissolved in tetrahydrofuran (20 mL), added with 2M dilute hydrochloric acid (20 mL), and reacted at room temperature overnight. The reaction solution was dropwise added with saturated sodium bicarbonate solution to adjust the pH to 8, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system B) to obtain tert-butyl 2-amino-3-cyclobutylpropanoate 39d (1.06 g) with a yield of 66%.
MS m/z (ESI): 144.1 [M+H-56]

Step 3

*Tert*-butyl 2-((2-((5-chloro-2-(1*H*-tetrazol-1-yl)phenyl)amino)-2-oxoethyl)amino)-3-cyclobutylpropanoate

2-chloro-*N*-(5-chloro-2-(1H-tetrazol-1-yl)phenyl)acetamide 1d (350 mg, 1.29 mmol) and *tert*-butyl 2-amino-3-cyclobutylpropanoate 39d (563.98 mg, 2.83 mmol) were dissolved in N,N-dimethylformamide (10 mL), added with N,N-diisopropylethylamine (831.23 mg, 6.43 mmol, 1.06 mL), and reacted at 70°C for 15 hours. The reaction solution was added with 30 mL of water for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system A) to obtain *tert*-butyl 2-((2-((5-chloro-2-(1*H*-tetrazol-1-yl)phenyl)amino)-2-oxoethyl)amino)-3-cyclobutylpropanoate 39e (390 mg) with a yield of 70%.
MS m/z (ESI): 435.0 [M+H]

Step 4

*Tert*-butyl 2-(2-chloro-*N*-(2-((5-chloro-2-(1*H*-tetrazol-1-yl)phenyl)amino)-2-oxoethyl)acetamido)-3-cyclobutylpropanoate

*Tert*-butyl 2-((2-((5-chloro-2-(1*H*-tetrazol-1-yl)phenyl)amino)-2-oxoethyl)amino)-3-cyclobutylpropanoate 39e (240 mg, 551.83 μmol) was dissolved in acetonitrile (10 mL), added with potassium carbonate (381.34 mg, 2.76 mmol), slowly dropwise added with chloroacetyl chloride (124.65 mg, 1.10 mmol, 87.78 μL) in ice bath, and reacted for 1 hour in ice bath. The reaction solution was added with 30 mL of water for quenching the reaction and extracted with ethyl acetate (30 mL×3), then organic phases were combined, washed with water (50 mL×3) and saturated sodium chloride solution (50 mL) in turn, dried with anhydrous sodium sulfate, filtered, and concentrated under reduced pressure to obtain crude product of *tert*-butyl 2-(2-chloro-*N*-(2-((5-chloro-2-(1*H*-tetrazol-1-yl)phenyl)amino)-2-oxoethyl)acetamido)-3-cyclobutylpropanoate 39f (282 mg), which was directly used for next reaction.
MS m/z (ESI): 510.9 [M+H]

Step 5

*Tert*-butyl 2-(4-(5-chloro-2-(1*H*-tetrazol-1-yl)phenyl)-2,5-dioxopiperazin-1-yl)-3-cyclobutylpropanoate

The crude product of *tert*-butyl 2-(2-chloro-*N*-(2-((5-chloro-2-(1*H*-tetrazol-1-yl)phenyl)amino)-2-oxoethyl)acetamido)-3-cyclobutylpropanoate 39f (282 mg, 551.43 μmol) obtained in the last step was dissolved in methanol (9.88 mL), slowly dropwise added with sodium methylate (5.4M methanol solution, 122.54 μL, 661.72 μmol) at 0°C, and continuously reacted at 0°C for 1 hour. 2M dilute hydrochloric acid was dropwise added to adjust the pH to 6, then the reaction solution was concentrated under reduced pressure, and the obtained residue was further separated and purified by silicagel column chromatography (eluent: system A) to obtain *tert*-butyl 2-(4-(5-chloro-2-(1*H*-tetrazol-1-yl)phenyl)-2,5-dioxopiperazin-1-yl)-3-cyclobutylpropanoate 39g (96 mg) with a yield of 37%.
MS m/z (ESI): 475.1 [M+H]

Step 6

2-(4-(5-Chloro-2-(1*H*-tetrazol-1-yl)phenyl)-2,5-dioxopiperazin-1-yl)-3-cyclobutylpropanoic acid

*Tert*-butyl 2-(4-(5-chloro-2-(1*H*-tetrazol-1-yl)phenyl)-2,5-dioxopiperazin-1-yl)-3-cyclobutylpropanoate 39g (90 mg, 189.50 μmol) was dissolved in dichloromethane (8 mL), slowly dropwise added with trifluoroacetic acid (2 mL), and reacted at room temperature for 1 hour. After the reaction was completed, the reaction solution was concentrated under reduced pressure to obtain crude product of 2-(4-(5-chloro-2-(1*H*-tetrazol-1-yl)phenyl)-2,5-dioxopiperazin-1-yl)-3-cyclobutylpropanoic acid 39h (79 mg), which was directly used for next reaction.
MS m/z (ESI): 418.9 [M+H]

Step 7

2-(4-(5-Chloro-2-(1*H*-tetrazol-1-yl)phenyl)-2,5-dioxopiperazin-1-yl)-3-cyclobutyl-*N*-(2-methyl-2*H*-indazol-5-yl)propanamide

The crude product of 2-(4-(5-chloro-2-(1*H*-tetrazol-1-yl)phenyl)-2,5-dioxopiperazin-1-yl)-3-cyclobutylpropanoic acid 39h (79 mg, 188.62 μmol) obtained in the last step and 2-methyl-2*H*-indazol-5-amine 19a (41.64 mg, 282.93 μmol) were dissolved in ethyl acetate (6 mL), added with propylphosphonic anhydride (50% ethyl acetate solution, 360.09 mg, 565.86 μmol) and N,N-diisopropylethylamine (146.26 mg, 1.13 mmol), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-3-cyclobutyl-*N*-(2-methyl-2*H*-indazol-5-yl)propionamide 39 (40 mg) with a yield of 38%.

MS m/z (ESI): 548.2 [M+H]
$^1$H NMR (400 MHz, DMSO-$d_6$) δ 10.11 (s, 1H), 9.79 (d, J = 5.7 Hz, 1H), 8.24 (d, J = 5.7 Hz, 1H), 8.08 (d, J = 5.7 Hz, 1H), 7.90 (d, J = 5.6 Hz, 1H), 7.87-7.71 (m, 2H), 7.62-7.45 (m, 1H), 7.34-7.18 (m, 1H), 5.08-4.91 (m, 1H), 4.81-4.00 (m, 4H), 4.12 (d, J = 5.7 Hz, 3H), 2.18-1.49 (m, 9H).

Example 40

2-(4-(5-Chloro-2-(4-chloro-1*H*-1,2,3-triazol-1-yl)phenyl)-2,5-dioxopiperazin-1-yl)-3-phenyl-*N*-(pyrazolo[1,5-a]pyridin-5-yl)propanamide

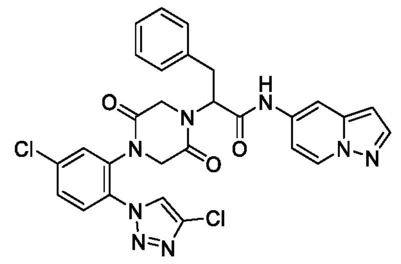

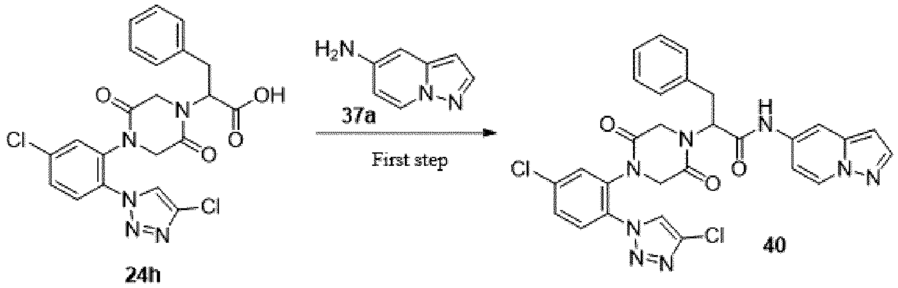


24h

40

2-(4-(5-Chloro-2-(4-chloro-1*H*-1,2,3-triazol-1-yl)phenyl)-2,5-dioxopiperazin-1-yl)-3-phenylpropanoic acid 24h (50 mg, 150.42 μmol) and pyrazolo[1,5-a]pyridin-5-amine 37a (21.05 mg, 158.13 μmol) were dissolved in ethyl acetate (5 mL), added with propylphosphonic anhydride (50% ethyl acetate solution, 201.14 mg, 316.26 μmol, 188.16 μL) and N,N-diisopropylethylamine (68.12 mg, 527.10 μmol, 87.11 μL), and reacted at 60°C for 15 hours. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-phenyl-*N*-(pyrazolo[1,5-a]pyridin-5-yl)propanamide 40 (46.9 mg) with a yield of 75%.

MS m/z (ESI): 588.8 [M+H]

$^1$H NMR (400 MHz, DMSO-$d_6$) δ 10.43 (s, 1H), 8.65 (s, 1H), 8.61 (d, J = 7.5 Hz, 1H), 8.12 (s, 1H), 7.92 (d, J= 2.2 Hz, 1H), 7.81-7.62 (m, 3H), 7.37-7.15 (m, 5H), 6.92 (dd, J= 7.4, 2.3 Hz, 1H), 6.50 (d, J = 2.2 Hz, 1H), 5.47-5.22 (m, 1H), 4.48-3.91 (m, 4H), 3.34-3.20 (m, 1H), 3.18-3.03 (m, 1H).

Example 41

2-(4-(5-Chloro-2-(1*H*-tetrazol-1-yl)phenyl)-2,5-dioxopiperazin-1-yl)-3-(4-fluorophenyl)-*N*-(2-methyl-2*H*-indazol-5-yl)propanamide

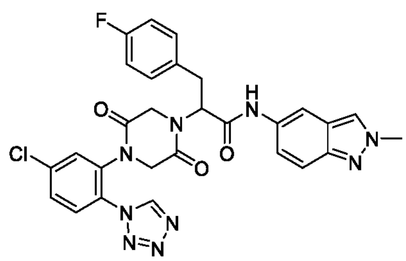

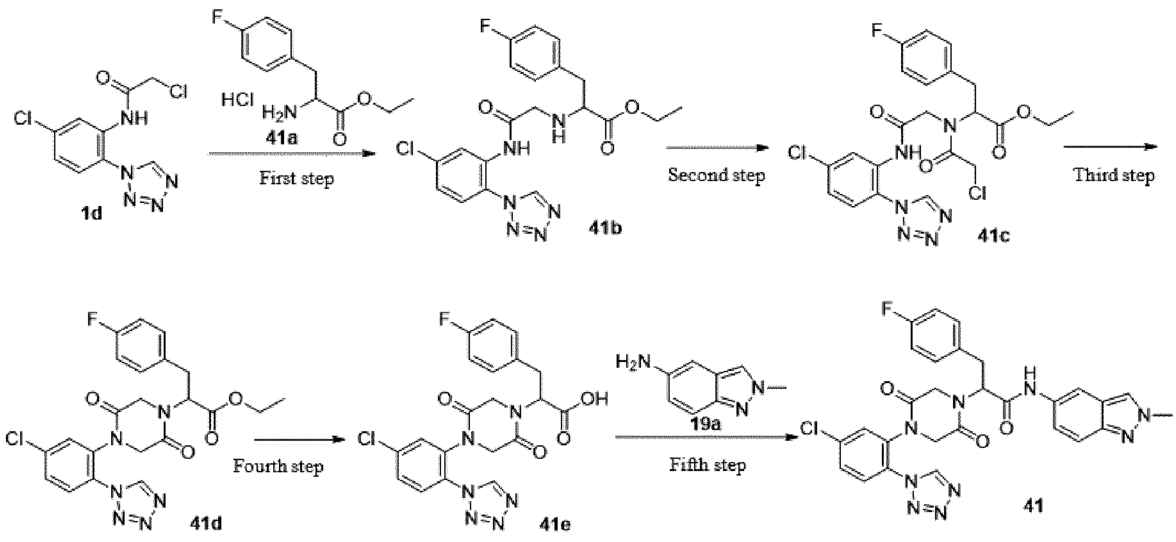


Step 1

Ethyl 2-((2-((5-chloro-2-(1*H*-tetrazol-1-yl)phenyl)amino)-2-oxoethyl)amino)-3-(4-fluorophenyl)propanoate

2-Chloro-*N*-(5-chloro-2-(1*H*-tetrazol-1-yl)phenyl)acetamide 1d (0.3 g, 1.10 mmol) and ethyl 2-amino-3-(4-fluorophenyl)propanoate 41a (1.71 g, 6.55 mmol) were dissolved in N,N-dimethylformamide (10 mL), added with N,N-diisopropylethylamine (712.48 mg, 5.51 mmol), and stirred at 60°C for reaction for 16 hours. The reaction solution was added with 30 mL of water for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system A) to obtain ethyl 2-((2-((5-chloro-2-(1*H*-tetrazole-1-yl)phenyl)amino)-2-oxoethyl)amino)-3-(4-fluorophenyl) propanoate 41b (348 mg) with a yield of 71%.
MS m/z (ESI): 446.9 [M+H]

Step 2

Ethyl 2-(2-chloro-*N*-(2-((5-chloro-2-(1*H*-tetrazol-1-yl)phenyl)amino)-2-oxoethyl)acetamido)-3-(4-fluorophenyl)propanoate

Ethyl 2-((2-((5-chloro-2-(1*H*-tetrazol-1-yl)phenyl)amino)-2-oxoethyl)amino)-3-(4-fluorophenyl)propanoate 41b (348 mg, 778.76 μmol) was dissolved in acetonitrile (10 mL), added with potassium carbonate (214.94 mg, 1.56 mmol), slowly dropwise added with chloroacetyl chloride (131.93 mg, 1.17 mmol, 93.57 μL) in ice bath, and reacted for 2 hours in ice bath. The reaction solution was added with 30 mL of water for quenching the reaction, and then extracted with ethyl acetate (30 mL×3). Organic phases were combined, dried with anhydrous sodium sulfate, filtered and concentrated under reduced pressure, and the obtained residue was further separated and purified by silicagel column chromatography (eluent: system A) to obtain ethyl 2-(2-chloro-*N*-(2-((5-chloro-2-(1*H*-tetrazol-1-yl)phenyl)amino)-2-oxoethyl)acetamido)-3-(4-fluorophenyl)propanoate 41c (250 mg) with a yield of 61%.
MS m/z (ESI): 523.2 [M+H]

Step 3

Ethyl 2-(4-(5-chloro-2-(1H-tetrazole-1-yl)phenyl)-2,5-dioxiperazine-1-yl)-3-(4-fluorophenyl)ethyl propionate

Ethyl 2-(2-chloro-*N*-(2-((5-chloro-2-(1*H*-tetrazol-1-yl)phenyl)amino)-2-oxoethyl)acetamido)-3-(4-fluorophenyl)propanoate 41c (260 mg, 496.81 μmol) was dissolved in methanol (5 mL), slowly dropwise added with sodium methylate (5.4M methanol solution, 110.40 μL, 596.16 mmol) in ice bath, and reacted in ice bath for 2 hours. 2M dilute hydrochloric acid was dropwise added to adjust the pH to 6, then the reaction solution was concentrated under reduced pressure, and the obtained residue was further separated and purified by silicagel column chromatography (eluent: system A) to obtain ethyl 2-(4-(5-chloro-2-(1*H*-tetrazol-1-yl)phenyl)-2,5-dioxopiperazin-1-yl)-3-(4-fluorophenyl)propanoate 41d (100 mg) with a yield of 41%.

MS m/z (ESI): 487.1 [M+H]

Step 4

2-(4-(5-Chloro-2-(1*H*-tetrazol-1-yl)phenyl)-2,5-dioxopiperazin-1-yl)-3-(4-fluorophenyl)propanoic acid

Ethyl 2-(4-(5-chloro-2-(1*H*-tetrazol-1-yl)phenyl)-2,5-dioxopiperazin-1-yl)-3-(4-fluorophenyl)propanoate 41d (100 mg, 205.39 μmmol) was dissolved in mixed solvent of tetrahydrofuran (3 mL), methanol (1 mL) and water (1 mL), added with lithium hydroxide monohydrate (12.94 mg, 308.08 μmol) in ice bath, and reacted in ice bath for 4 hours. 2M dilute hydrochloric acid was dropwise added to adjust the pH to 6, then the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-3-(4-fluorophenyl)propanoic acid 41e (64 mg) with a yield of 68%.
MS m/z (ESI): 459.0 [M+H]

Step 5

2-(4-(5-chloro-2-(1*H*-tetrazol-1-yl)phenyl)-2,5-dioxopiperazin-1-yl)-3-(4-fluorophenyl)-*N*-(2-methyl-2*H*-indazol-5-yl)propanamide

2-(4-(5-chloro-2-(1*H*-tetrazol-1-yl)phenyl)-2,5-dioxopiperazin-1-yl)-3-(4-fluorophenyl)propanoic acid 41e (64 mg, 139.49 μmol) and 2-methyl-2*H*-indazol-5-amine 19a (30.79 mg, 209.23 μmol) were dissolved in ethyl acetate (8 mL), added with propylphosphonic anhydride (50% ethyl acetate solution, 266.29 mg, 418.46 μmol) and N,N-diisopropylethylamine (175.34 mg, 1.36 mmol, 224.22 μL), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(1*H*-tetrazol-1-yl)phenyl)-2,5-dioxopiperazin-1-yl)-3-(4-fluorophenyl)-*N*-(2-methyl-2*H*-indazol-5-yl)propanamide 41 (30 mg) with a yield of 36%.

MS m/z (ESI): 588.2 [M+H]
$^1H$ NMR (400 MHz, DMSO-$d_6$) δ 10.11 (s, 1H), 9.73 (s, 1H), 8.27 (s, 1H), 8.07 (s, 1H), 7.90-7.68 (m, 3H), 7.55 (d, J = 9.1 Hz, 1H), 7.35-7.26 (m, 2H), 7.27-7.19 (m, 1H), 7.19-7.09 (m, 2H), 5.39-5.29 (m, 1H), 4.60-3.92 (m, 4H), 4.13 (s, 3H), 3.27-3.16 (m, 1H), 3.15-3.04 (m, 1H).

Example 42

4-(2-(4-(5-Chloro-2-(4-chloro-1*H*-1,2,3-triazol-1-yl)phenyl)-2,5-dioxopiperazin-1-yl)-3-phenylpropanamido)-2-fluoro-*N*-methylbenzamide

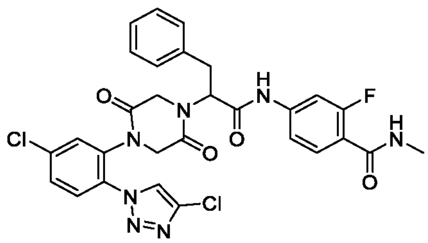

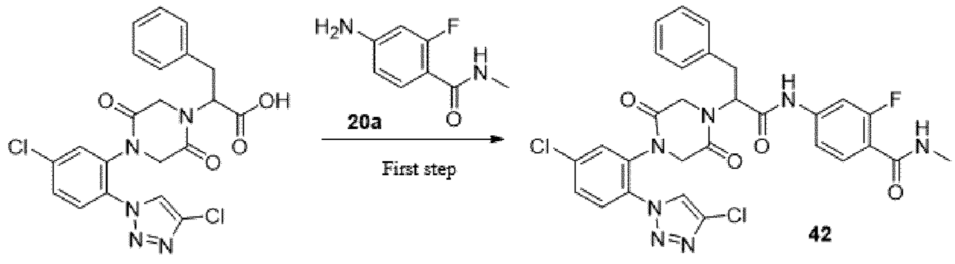


24h 42

2-(4-(5-Chloro-2-(4-chloro-1*H*-1,2,3-triazol-1-yl)phenyl)-2,5-dioxopiperazin-1-yl)-3-phenylpropanoic acid 24h (50 mg, 150.42 μmol) and 4-amino-2-fluoro-*N*-methylbenzamide 20a (226.59 mg, 158.13 μmol) were dissolved in ethyl acetate (10 mL), added with N,N-diisopropylethylamine (68.12 mg, 527.10 μmol) and propylphosphonic anhydride (50% ethyl acetate solution, 201.26 mg, 316.26 μmol), and reacted at 60°C for 2 hours. After the reaction was completed, the reaction solution was concentrated under reduced pressure, and the obtained residue was further separated and purified by silicagel column chromatography (eluent: system B) to obtain 4-(2-(4-(5-chloro-2-(4-chloro-1*H*-1,2,3-triazol-1-yl)phenyl)-2,5-dioxopiperazin-1-yl)-3-phenylpropanamido)-2-fluoro-*N*-methylbenzamide 42 (57 mg) with a yield of 86%.

MS m/z (ESI): 623.8 [M+H]

$^1$H NMR (400 MHz, DMSO-$d_6$) δ 10.56 (s, 1H), 8.63 (s, 1H), 8.14-8.04 (m, 1H), 7.84-7.56 (m, 5H), 7.40-7.17 (m, 6H), 5.40-5.24 (m, 1H), 4.48-3.90 (m, 4H), 3.32-3.20 (m, 1H), 3.17-3.04 (m, 1H), 2.76 (d, J = 4.5 Hz, 3H).

Example 43

2-(4-(5-Chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-3-yl)propanamide

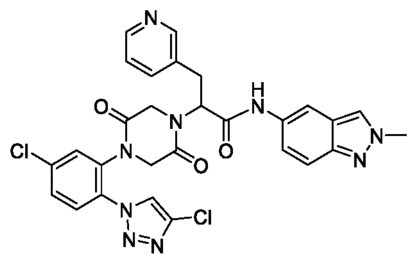

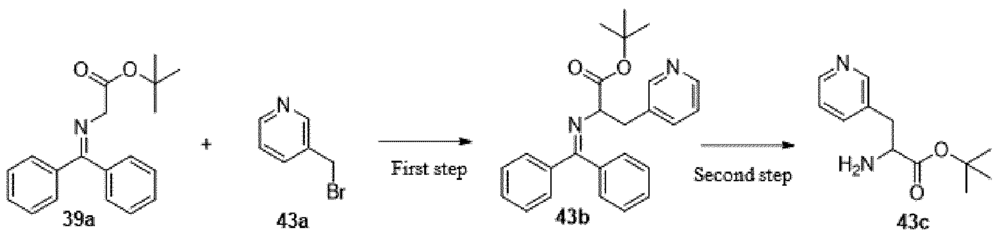


39a 43a 43b 43c

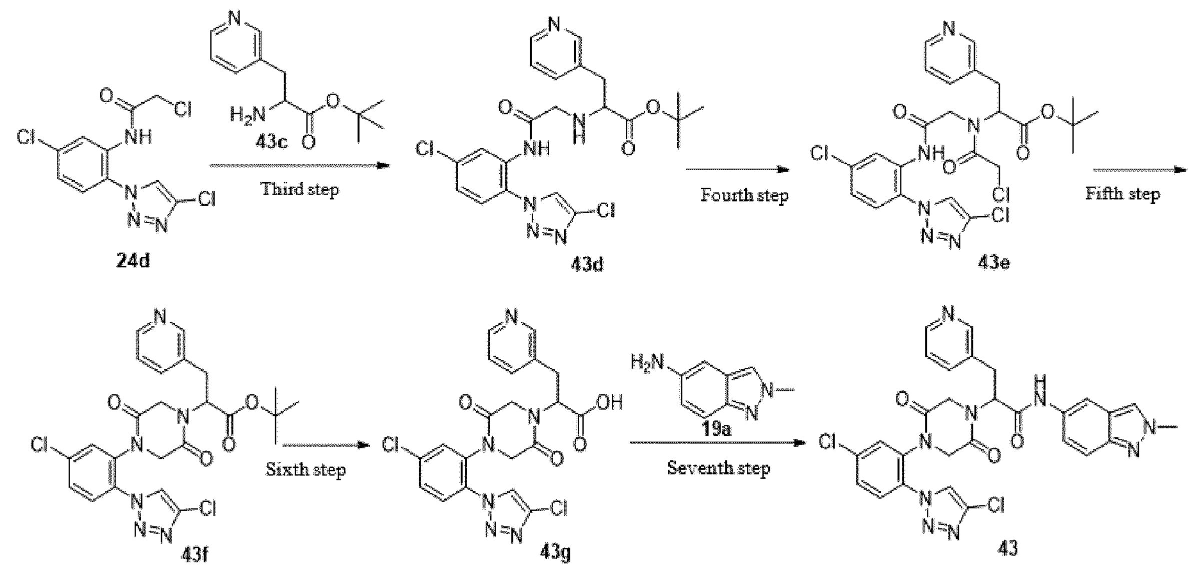


Step 1

*Tert*-butyl 2-((diphenylmethylene)amino)-3-(pyridin-3-yl)propanoate

*Tert*-butyl 2-((diphenylmethylene)amino)acetate 39a (2.95 g, 10 mmol) was dissolved in N,N-dimethylformamide (30 mL), added with potassium tert-butoxide (2.47 g, 22.00 mmol) in batches at 0°C, reacted for 15 minutes at 0°C, then added with 3-(bromomethyl)pyridine hydrobromide 43a (2.77 g, 11.00 mmol), reacted and when the temperature was increased to room temperature and then reacted for 3 hours. The reaction solution was added with 30 mL of water for quenching the reaction and extracted with ethyl acetate (30 mL×3), then organic phases were combined, washed with water (50 mL×3) and saturated sodium chloride solution (50 mL)in turn, dried with anhydrous sodium sulfate, filtered, and concentrated under reduced pressure to obtain crude product of *tert*-butyl 2-((diphenylmethylene)amino)-3-(pyridin-3-yl)propanoate 43b (3.86 g), which was directly used for the next reaction.
MS m/z (ESI): 387.0 [M+H]

Step 2

*Tert*-butyl 2-amino-3-(pyridin-3-yl) propanoate

The crude product of *tert*-butyl 2-((diphenylmethylene)amino)-3-(pyridin-3-yl)propanoate 43b (3.86 g, 10 mmol) obtained in last step was dissolved in tetrahydrofuran (20 mL), added with 2M dilute hydrochloric acid (20 mL), and reacted at room temperature overnight. The reaction solution was dropwise added with saturated sodium bicarbonate solution to adjust the pH to 8, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system B) to obtain tert-butyl 2-amino-3-(pyridin-3-yl)propanoate 43c (1.06 g) with a yield of 39%.
MS m/z (ESI): 222.9 [M+H]

Step 3

*Tert*-butyl 2-((2-((5-chloro-2-(4-chloro-1*H*-1,2,3-triazol-1-yl)phenyl)amino)-2-oxoethyl)amino)-3-(pyridin-3-yl)propanoate

2-Chloro-*N*-(5-chloro-2-(4-chloro-1*H*-1,2,3-triazol-1-yl)phenyl)acetamide 24d (200 mg, 654.56 μmol) and *tert*-butyl 2-amino-3-(pyridin-3-yl)propanoate 43c (291.2 mg, 1.31 mmol) were dissolved in N,N-dimethylformamide (6 mL), added with N,N-diisopropylethylamine (422.98 mg, 3.27 mmol, 540.89 μL), and reacted at 75°C for 6 hours. The reaction solution was added with 40 mL of water for quenching the reaction, and then extracted with ethyl acetate (40 mL×3). Organic phases were combined, washed with water (40 mL) and saturated sodium chloride solution (40 mL) in turn, dried with anhydrous sodium sulfate, filtered and concentrated under reduced pressure, and the obtained residue

was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(pyridin-3-yl)propanoate 43d (299 mg) with a yield of 93%.
MS m/z (ESI): 490.9 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3 -(pyridin-3-yl)propanoate

*Tert*-butyl 2-((2-((5-chloro-2-(4-chloro-1*H*-1,2,3-triazol-1-yl)phenyl)amino)-2-oxoethyl)amino)-3-(pyridin-3-yl)propanoate 43d (299 mg, 608.50 μmol) was dissolved in dichloromethane (10 mL), added with triethylamine (307.87 mg, 3.04 mmol, 422.90 μL), slowly dropwise added with chloroacetyl chloride (103.09 mg, 912.76 μmol, 72.60 μL) in ice bath, and reacted for 2 hours in ice bath. The reaction solution was added with 30 mL of water for quenching the reaction and extracted with ethyl acetate (30 mL×3), then organic phases were combined, washed with saturated sodium chloride solution (50 mL), dried with anhydrous sodium sulfate, filtered, and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(pyridin-3-yl)propanoate 43e (345 mg), which was directly used for next reaction.
MS m/z (ESI): 566.8 [M+H]

Step 5

*Tert*-butyl 2-(4-(5-chloro-2-(4-chloro-1*H*-1,2,3-triazol-1-yl)phenyl)-2,5-dioxopiperazin-1-yl)-3-(pyridin-3 -yl)propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(pyridin-3-yl)propanoate 43e (345 mg, 607.55 μmol) obtained in the last step was dissolved in methanol (10 mL), slowly dropwise added with sodium methylate (5.4M methanol solution, 135.01 μL, 729.05 μmol) at 0°C, and continuously reacted at 0°C for 1 hour. 2M dilute hydrochloric acid was dropwise added to adjust the pH to 7, then the reaction solution was concentrated under reduced pressure, and the obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(pyridin-3-yl)propanoate 43f (220 mg) with a yield of 68%.
MS m/z (ESI): 531.1 [M+H]

Step 6

2-(4-(5-chloro-2-(4-chloro-1*H*-1,2,3-triazol-1-yl)phenyl)-2,5-dioxopiperazin-1-yl)-3-(pyridin-3-yl)propanoic acid

*Tert*-butyl 2-(4-(5-chloro-2-(4-chloro-1*H*-1,2,3-triazol-1-yl)phenyl)-2,5-dioxopiperazin-1-yl)-3-(pyridin-3-yl)propanoate 43f(220 mg, 414.01 μmol) was dissolved in dichloromethane (6 mL), slowly dropwise added with trifluoroacetic acid (2 mL), and reacted at room temperature for 2 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(pyridin-3-yl)propanoic acid 43g (196 mg), which was directly used for the next reaction.
MS m/z (ESI): 474.8 [M+H]

Step 7

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-3-yl)propanamide

The crude product of 2-(4-(5-chloro-2-(4-chloro-1*H*-1,2,3-triazol-1-yl)phenyl)-2,5-dioxopiperazin-1-yl)-3-(pyridin-3-yl)propanoic acid 43g (196 mg, 412.38 μmol) obtained in the last step and 2-methyl-2*H*-indazol-5-amine 19a (91.04 mg, 618.58 μmol) were dissolved in ethyl acetate (10 mL), added with propylphosphonic anhydride (50% ethyl acetate solution, 524.85 mg, 824.77 μmol) and N,N-diisopropylethylamine (266.48 mg, 2.06 mmol, 340.77 μL), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-3-yl)propanamide 43 (14 mg) with a yield of 5%.

MS m/z (ESI): 603.8 [M+H]

$^1$H NMR (400 MHz, DMSO-$d_6$) δ 10.20 (s, 1H), 8.67 (s, 1H), 8.53 (s, 1H), 8.45 (s, 1H), 8.27 (s, 1H), 8.10 (s, 1H), 7.86-7.62 (m, 4H), 7.55 (d, J= 9.1 Hz, 1H), 7.39-7.30 (m, 1H), 7.28-7.18 (m, 1H), 5.42 (s, 1H), 4.43-4.01 (m, 4H), 4.14 (s, 3H), 3.19-3.07 (m, 2H).

Example 44

2-(4-(5-Chloro-2-(4-chloro-1*H*-1,2,3-triazol-1-yl)phenyl)-2,5-dioxopiperazin-1-yl)-3-(2-fluorophenyl)-*N*-(2-methyl-2*H*-indazol-5-yl)propanamide

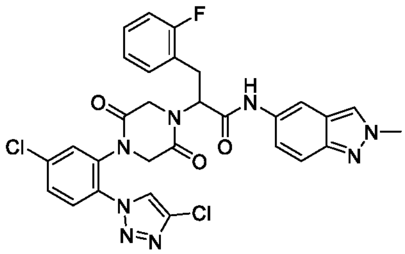

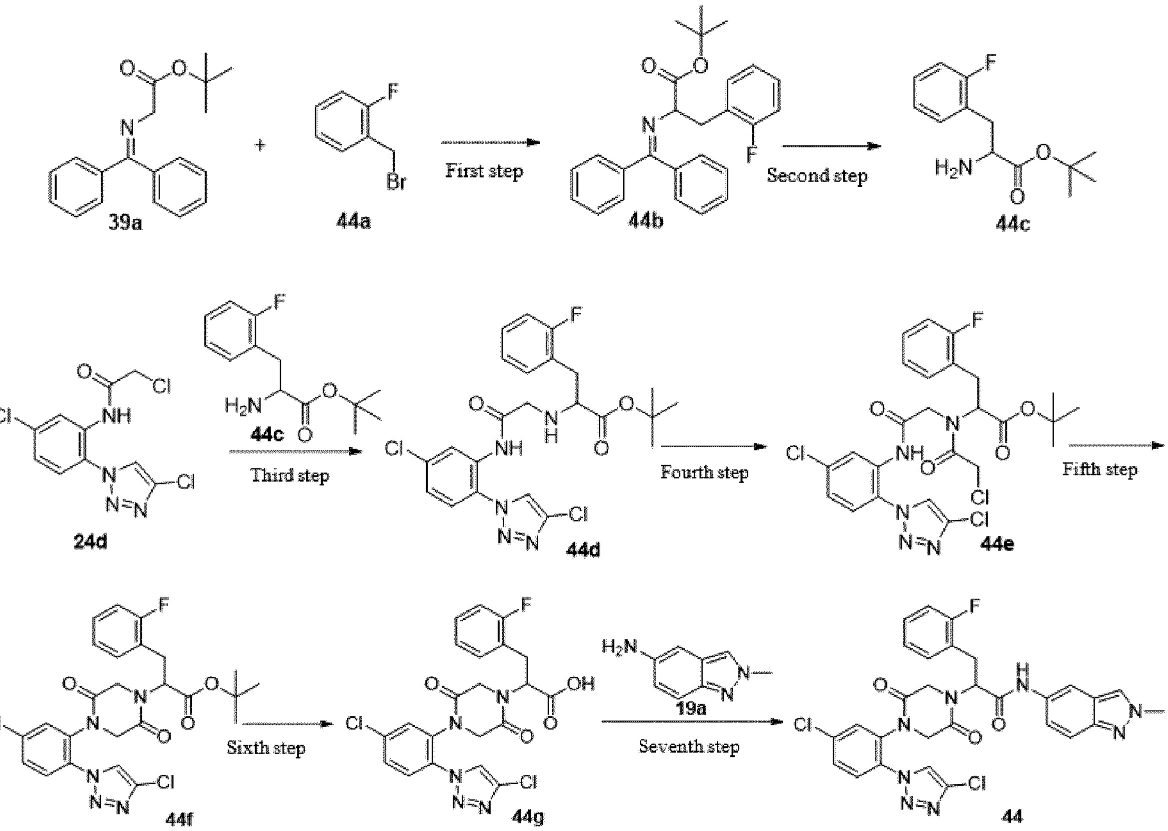

39a 44a 44b 44c 24d 44c 44d 44e 44f 44g 19a 44

Step 1

*Tert*-butyl 2-((diphenylmethylene)amino)-3-(2-fluorophenyl)propanoate

*Tert*-butyl 2-((diphenylmethylene)amino)acetate 39a (2.95 g, 10 mmol) was dissolved in N,N-dimethylformamide (30 mL), added with potassium tert-butoxide (1.23 g, 11.00 mmol) in batches at 0°C, reacted for 15 minutes at 0°C, then added with 1-(bromomethyl)-2-florobenzene 44a (2.08 g, 11.00 mmol), reacted to room temperature and then reacted for 3 hours. The reaction solution was added with 40 mL of water for quenching the reaction and extracted with

ethyl acetate (40 mL×3), then organic phases were combined, washed with water (40 mL) and saturated sodium chloride solution (40 mL) in turn, dried with anhydrous sodium sulfate, filtered, and concentrated under reduced pressure to obtain crude product of *tert*-butyl 2-((diphenylmethylene)amino)-3-(2-fluorophenyl)propanoate 44b (4 g), which was directly used for the next reaction.
MS m/z (ESI): 404.0 [M+H]

Step 2

*Tert*-butyl 2-amino-3 -(2-fluorophenyl)propanoate

The crude product of *tert*-butyl 2-((diphenylmethylene)amino)-3-(2-fluorophenyl)propanoate 44b (4 g, 10 mmol) obtained in the last step was dissolved in tetrahydrofuran (20 mL), added with 2M dilute hydrochloric acid (20 mL), and reacted at room temperature overnight. The reaction solution was dropwise added with saturated sodium bicarbonate solution to adjust the pH to 8, and then extracted with ethyl acetate (30 mL×3), washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system B) to obtain *tert*-butyl 2-amino-3-(2-fluorophenyl)propanoate 44c (1.41 g) with a yield of 59%.
MS m/z (ESI): 184.1 [M+H-56]

Step 3

*Tert*-butyl 2-((2-((5-chloro-2-(4-chloro-1*H*-1,2,3-triazol-1-yl)phenyl)amino)-2-oxoethyl)amino)-3-(2-fluorophenyl)propanoate

2-chloro-*N*-(5-chloro-2-(4-chloro-1*H*-1,2,3-triazol-1-yl)phenyl)acetamide 24d (200 mg, 654.56 μmol) and *tert*-butyl 2-amino-3-(2-fluorophenyl)propanoate 44c (313.25 mg, 1.31 mmol) were dissolved in N,N-dimethylformamide (6 mL), added with N,N-diisopropylethylamine (422.98 mg, 3.27 mmol, 540.89 μL), and reacted at 75°C for 6 hours. The reaction solution was added with 80 mL of water for quenching the reaction, and then extracted with ethyl acetate (80 mL×3). Organic phases were combined, washed with water (80 mL) and saturated sodium chloride solution (80 mL) in turn, dried with anhydrous sodium sulfate, filtered and concentrated under reduced pressure, and the obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(2-fluorophenyl)propanoate 44d (286 mg) with a yield of 86%.
MS m/z (ESI): 507.9 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(2-fluorophenyl)propanoate

*Tert*-butyl 2-((2-((5-chloro-2-(4-chloro-1*H*-1,2,3-triazol-1-yl)phenyl)amino)-2-oxoethyl)amino)-3-(2-fluorophenyl)propanoate 44d (286 mg, 562.58 μmol) was dissolved in dichloromethane (10 mL), added with triethylamine (284.64 mg, 2.81 mmol, 390.99 μL), slowly dropwise added with chloroacetyl chloride (95.31 mg, 843.87 μmol, 67.12 μL) in ice bath, and reacted for 2 hours in ice bath. The reaction solution was added with 30 mL of water for quenching the reaction and extracted with ethyl acetate (30 mL×3), then organic phases were combined, washed with saturated sodium chloride solution (50 mL), dried with anhydrous sodium sulfate, filtered, and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(2-fluorophenyl)propanoate 44e (329 mg), which was directly used for next reaction.
MS m/z (ESI): 584.4 [M+H]

Step 5

*Tert*-butyl 2-(4-(5-chloro-2-(4-chloro-1*H*-1,2,3-triazol-1-yl)phenyl)-2,5-dioxopiperazin-1-yl)-3-(2-fluorophenyl)propanoate

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(2-fluorophenyl)propanoate 44e (329 mg, 562.53 μmol) obtained in the last step was dissolved in methanol (10 mL), slowly dropwise added with sodium methylate (5.4M methanol solution, 114.59 μL, 618.79 μmol) at 0°C, and continuously

reacted at 0°C for 1 hour. 2M dilute hydrochloric acid was dropwise added to adjust the pH to 6, then the reaction solution was concentrated under reduced pressure, and the obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(2-fluorophenyl)propanoate 44f (230 mg) with a yield of 75%.
MS m/z (ESI): 547.8 [M+H]

Step 6

2-(4-(5-Chloro-2-(4-chloro-1*H*-1,2,3-triazol-1-yl)phenyl)-2,5-dioxopiperazin-1-yl)-3-(2-fluorophenyl)propionic acid

*Tert*-butyl 2-(4-(5-chloro-2-(4-chloro-1*H*-1,2,3-triazol-1-yl)phenyl)-2,5-dioxopiperazin-1-yl)-3-(2-fluorophenyl)propanoate 44f (230 mg, 419.41 μmol) was dissolved in dichloromethane (6 mL), slowly dropwise added with trifluoroacetic acid (2 mL), and reacted at room temperature for 2 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(2-fluorophenyl)propanoic acid 44g (206 mg), which was directly used for the next reaction. MS m/z (ESI): 491.8 [M+H]

Step 7

2-(4-(5-Chloro-2-(4-chloro-1*H*-1,2,3-triazol-1-yl)phenyl)-2,5-dioxopiperazin-1-yl)-3-(2-fluorophenyl)-*N*-(2-methyl-2*H*-indazol-5-yl)propanamide

The crude product of 2-(4-(5-chloro-2-(4-chloro-1*H*-1,2,3-triazol-1-yl)phenyl)-2,5-dioxopiperazin-1-yl)-3-(2-fluorophenyl)propanoic acid 44g (206 mg, 418.46 μmol) obtained in the last step and 2-methyl-2*H*-indazol-5-amine 19a (92.38 mg, 627.68 μmol) were dissolved in ethyl acetate (10 mL), added with propylphosphonic anhydride (50% ethyl acetate solution, 532.58 mg, 836.91 μmol) and N,N-diisopropylethylamine (270.41 mg, 2.09 mmol, 345.79 μL), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(2-fluorophenyl)-N-(2-methyl-2H indazol-5-yl)propanamide 44 (136 mg) with a yield of 52%.

MS m/z (ESI): 620.8 [M+H]
$^1$H NMR (400 MHz, DMSO-$d_6$) δ 10.08 (s, 1H), 8.69 (s, 1H), 8.27 (s, 1H), 8.07 (s, 1H), 7.82-7.65 (m, 3H), 7.53 (d, J= 9.1 Hz, 1H), 7.38-7.26 (m, 2H), 7.26-7.10 (m, 3H), 5.39-5.30 (m, 1H), 4.50-4.00 (m, 4H), 4.13 (s, 3H), 3.33-3.20 (m, 1H), 3.19-3.06 (m, 1H).

Example 45

2-(4-(5-Chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-phenylpropanamide

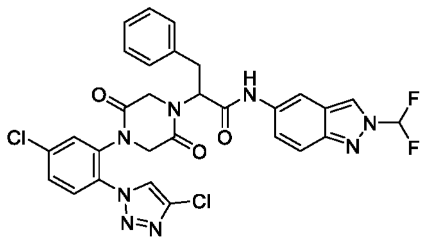

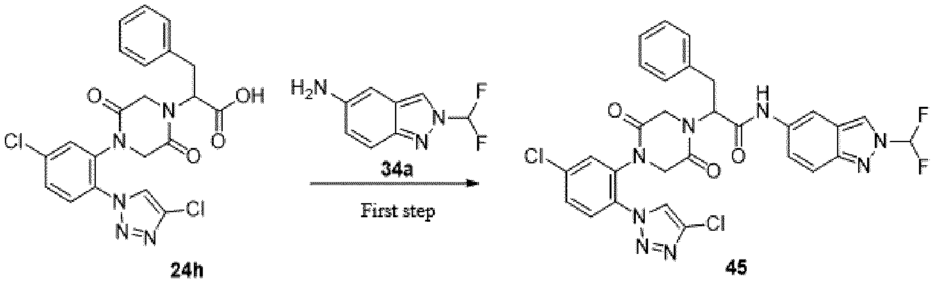


24h 45

2-(4-(5-chloro-2-(4-chloro-1*H*-1,2,3-triazol-1-yl)phenyl)-2,5-dioxopiperazin-1-yl)-3-phenylpropanoic acid 24h (67 mg, 141.26 μmol) and 2-(difluoromethyl)-2*H*-indazol-5-amine 34a (38.8 mg, 211.89 μmol) were dissolved in ethyl acetate (5 mL), added with propylphosphonic anhydride (50% ethyl acetate solution, 179.79 mg, 282.52 μmol) and N,N-diisopropylethylamine (73.03 mg, 565.05 μmol, 93.38 μL), and reacted at 60°C for 1 hour. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-phenylpropanamide 45 (20 mg) with a yield of 21%.

MS m/z (ESI): 638.7 [M+H]
$^1$H NMR (400 MHz, DMSO-$d_6$) δ 10.28 (s, 1H), 8.82 (s, 1H), 8.65 (s, 1H), 8.25 (s, 1H), 8.10 (t, J = 59.1 Hz, 1H), 7.84-7.58 (m, 4H), 7.44-7.16 (m, 6H), 5.47-5.31 (m, 1H), 4.56-3.88 (m, 4H), 3.36-3.22 (m, 1H), 3.20-3.03 (m, 1H).

Example 46

2-(4-(5-Chloro-2-(4-chloro-1*H*-1,2,3-triazol-1-yl)phenyl)-2,5-dioxopiperazin-1-yl)-3-(3-fluorophenyl)-*N*-(2-methyl-2*H*-indazol-5-yl)propanamide

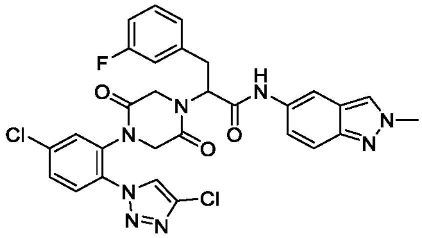

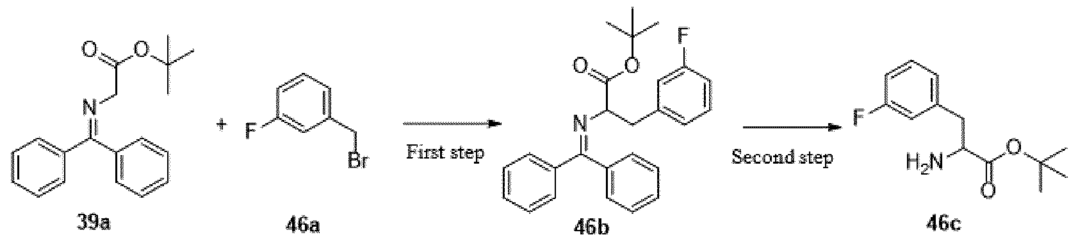


39a 46a 46b 46c

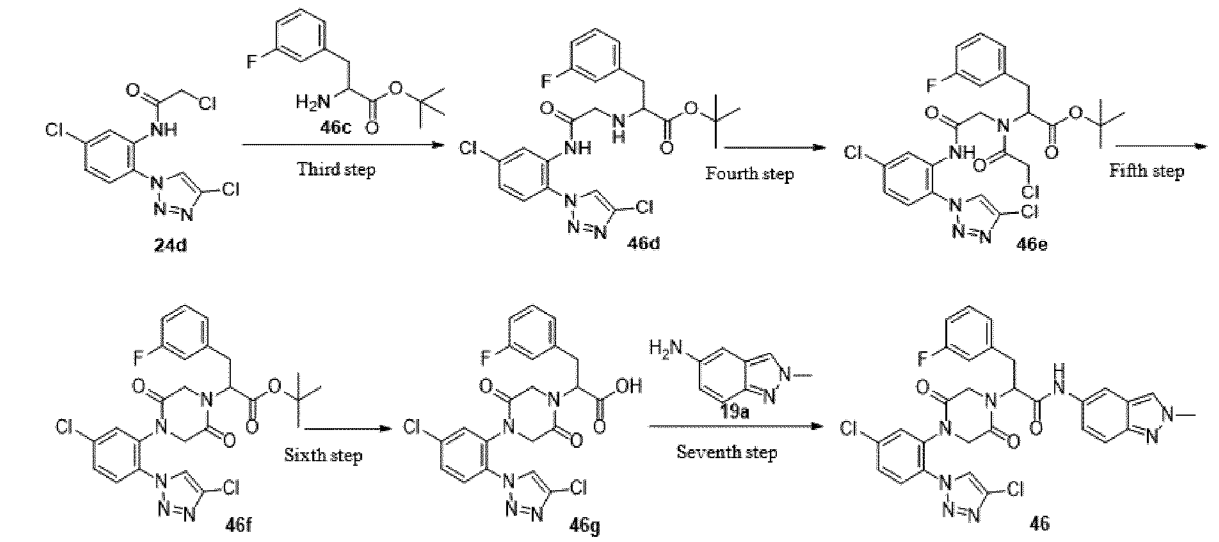


Step 1

*Tert*-butyl 2-((diphenylmethylene)amino)-3-(3-fluorophenyl)propanoate

*Tert*-butyl 2-((diphenylmethylene)amino)acetate 39a (3.75 g, 12.70 mmol) was dissolved in N,N-dimethylformamide (30 mL), added with potassium tert-butoxide (1.31 g, 11.64 mmol) in batches at 0°C, reacted for 15 minutes at 0°C, then added with 1-(bromomethyl)-3-florobenzene 46a (2 g, 10.58 mmol), reacted and the temperature was increased to room temperature and then reacted for 3 hours. The reaction solution was added with 40 mL of water for quenching the reaction and extracted with ethyl acetate (40 mL×3), then organic phases were combined, washed with water (40 mL) and saturated sodium chloride solution (40 mL) in turn, dried with anhydrous sodium sulfate, filtered, and concentrated under reduced pressure to obtain crude product of *tert*-butyl 2-((diphenylmethylene)amino)-3-(3-fluorophenyl)propanoate 46b (4.269 g), which was directly used for next reaction.
MS m/z (ESI): 404.2 [M+H]

Step 2

*Tert*-butyl 2-amino-3 -(3 -fluorophenyl)propanoate

The crude product of *tert*-butyl 2-((diphenylmethylene)amino)-3-(3-fluorophenyl) propanoate 46b (4.269 g, 10.58 mmol) obtained in the last step was dissolved in tetrahydrofuran (10 mL), added with 2M dilute hydrochloric acid (10 mL), and reacted at room temperature overnight. The reaction solution was dropwise added with saturated sodium bicarbonate solution to adjust the pH to 8, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure, and the obtained residue was further separated and purified by silicagel column chromatography (eluent: system B) to obtain *tert*-butyl 2-amino-3-(3-fluorophenyl)propanoate 46c (1.98 g) with a yield of 78%.
MS m/z (ESI): 184.1 [M+H-56]

Step 3

*Tert*-butyl 2-((2-((5-chloro-2-(4-chloro-1*H*-1,2,3-triazol-1-yl)phenyl)amino)-2-oxoethyl)amino)-3-(3 -fluorophenyl)propanoatee

2-chloro-*N*-(5-chloro-2-(4-chloro-1*H*-1,2,3-triazol-1-yl)phenyl)acetamide 24d (250 mg, 818.20 μmol) and *tert*-butyl 2-amino-3-(3-fluorophenyl)propanoate 46c (293.68 mg, 1.23 mmol) were dissolved in N,N-dimethylformamide (10 mL), added with N,N-diisopropylethylamine (528.72 mg, 4.09 mmol, 676.11 μL), and reacted at 80°C for 5 hours. The reaction solution was added with 80 mL of water for quenching the reaction, and then extracted with ethyl acetate (80 mL×3). Organic phases were combined, washed with water (80 mL) and saturated sodium chloride solution (80 mL) in turn, dried with anhydrous sodium sulfate, filtered and concentrated under reduced pressure, and the obtained

residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(3-fluorophenyl)propanoate 46d (220 mg) with a yield of 53%.
MS m/z (ESI): 508.1 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(3 -fluorophenyl)propanoate

*Tert*-butyl 2-((2-((5-chloro-2-(4-chloro-1*H*-1,2,3-triazol-1-yl)phenyl)amino)-2-oxoethyl)amino)-3-(3-fluorophenyl)propanoate 46d (220 mg, 432.75 μmol) was dissolved in dichloromethane (10 mL), added with triethylamine (43.71 mg, 432.75 μmol, 60.04 μL), slowly dropwise added with chloroacetyl chloride (73.31 mg, 649.13 μmol, 51.66 μL) in ice bath, and reacted for 2 hours in ice bath. The reaction solution was added with 30 mL of water for quenching the reaction and extracted with ethyl acetate (30 mL×3), then organic phases were combined, washed with saturated sodium chloride solution (50 mL), dried with anhydrous sodium sulfate, filtered, and concentrated under reduced pressure to obtain crude product of *tert*-butyl 2-(2-chloro-*N*-(2-((5-chloro-2-(4-chloro-1*H*-1,2,3 -triazol -1-yl)phenyl)amino)-2-oxoethyl)acetamido)-3 -(3 -fluorophenyl)propanoate 46e (232 mg), which was directly used for next reaction.
MS m/z (ESI): 527.7 [M+H-56]

Step 5

*Tert*-butyl 2-(4-(5-chloro-2-(4-chloro-1*H*-1,2,3-triazol-1-yl)phenyl)-2,5-dioxopiperazin-1-yl)-3-(3-fluorophenyl)propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(3-fluorophenyl)propanoate 46e (230 mg, 393.26 μmol) obtained in last step was dissolved in methanol (6 mL), slowly dropwise added with sodium methylate (5.4M methanol solution, 72.83 μL, 393.28 μmol) at 0°C, and continuously reacted at 0°C for 2 hours. 2M dilute hydrochloric acid was dropwise added to adjust the pH to 6, then the reaction solution was concentrated under reduced pressure, and the obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(3-fluorophenyl)propanoate 46f (154 mg) with a yield of 71%.
MS m/z (ESI): 491.8 [M+H-56]

Step 6

2-(4-(5-Chloro-2-(4-chloro-1*H*-1,2,3-triazol-1-yl)phenyl)-2,5-dioxopiperazin-1-yl)-3-(3-fluorophenyl)propanoic acid

*Tert*-butyl 2-(4-(5-chloro-2-(4-chloro-1*H*-1,2,3-triazol-1-yl)phenyl)-2,5-dioxopiperazin-1-yl)-3-(3-fluorophenyl)propanoate 46f (154 mg, 280.82 μmol) was dissolved in dichloromethane (10 mL), slowly dropwise added with trifluoroacetic acid (2 mL), and reacted at room temperature for 2 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(3-fluorophenyl)propanoic acid 46g (100 mg), which was directly used for next reaction.
MS m/z (ESI): 491.8 [M+H]

Step 7

2-(4-(5-chloro-2-(4-chloro-1*H*-1,2,3-triazol-1-yl)phenyl)-2,5-dioxopiperazin-1-yl)-3-(3-fluorophenyl)-*N*-(2-methyl-2H-indazol-5-yl)propanamide

The crude product of 2-(4-(5-chloro-2-(4-chloro-1*H*-1,2,3-triazol-1-yl)phenyl)-2,5-dioxopiperazin-1-yl)-3-(3-fluorophenyl)propanoic acid 46g (100 mg, 203.13 μmol) obtained in the last step and 2-methyl-2*H*-indazol-5-amine 19a (44.84 mg, 304.70 μmol) were dissolved in ethyl acetate (10 mL), added with propylphosphonic anhydride (50% ethyl acetate solution, 387.80 mg, 609.40 μmol) and N,N-diisopropylethylamine (131.26 mg, 1.02 mmol, 167.85 μL), and reacted at 60°C for 15 hours. The reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(3-fluorophenyl)-*N*-(2-methyl-2H-indazol-5-yl)propanamide 46 (49 mg) with a yield of 39%.

MS m/z (ESI): 620.8 [M+H]
$^1$H NMR (400 MHz, DMSO-$d_6$) $\delta$ 10.12 (s, 1H), 8.65 (s, 1H), 8.26 (s, 1H), 8.10 (s, 1H), 7.81-7.63 (m, 3H), 7.55 (d, J = 9.2 Hz, 1H), 7.36 (q, J = 7.4 Hz, 1H), 7.28-7.02 (m, 4H), 5.49-5.34 (m, 1H), 4.48-3.90 (m, 4H), 4.14 (s, 3H), 3.34-3.22 (m, 1H), 3.20-3.07 (m, 1H).

Example 47

2-(4-(5-Chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide

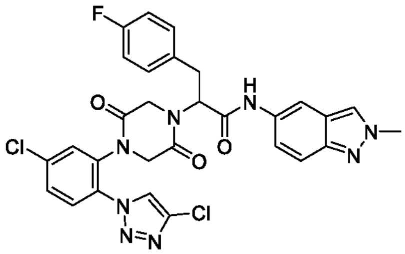

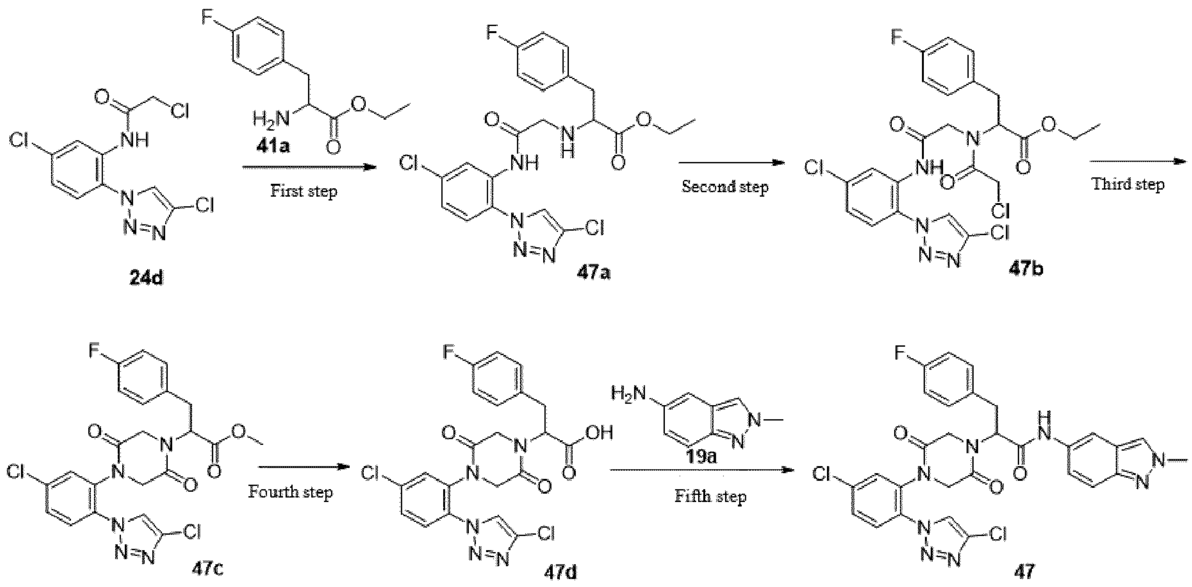


Step 1

Ethyl 2-((2-((5-chloro-2-(4-chloro-1*H*-1,2,3-triazol-1-yl)phenyl)amino)-2-oxoethyl)amino)-3-(4-fluorophenyl)propanoate

2-chloro-N-(5-chloro-2-(4-chloro-1*H*-1,2,3-triazol-1-yl)phenyl)acetamide 24d (220 mg, 720.02 $\mu$mol) and ethyl 2-amino-3-(4-fluorophenyl)propanoate 41a (228.14 mg, 921.05 $\mu$mol) were dissolved in N,N-dimethylformamide (10 mL), added with N,N-diisopropylethylamine (465.28 mg, 3.60 mmol), and stirred at 60°C for reaction for 5 hours. The reaction solution was added with 30 mL of water for quenching the reaction, and then extracted with ethyl acetate (30 mL$\times$3). Organic phases were combined, washed with water (50 mL$\times$3) and saturated sodium chloride solution (50 mL) in turn, dried with anhydrous sodium sulfate, filtered and concentrated under reduced pressure, and the obtained residue was further separated and purified by silicagel column chromatography (eluent: system A) to obtain ethyl 2-((2-((5-chloro-2-(4-chloro-1*H*-1,2,3-triazol-1-yl)phenyl)amino)-2-oxoethyl)amino)-3-(4-fluorophenyl)propanoate 47a (130 mg) with a yield of 38%.
MS m/z (ESI): 479.8 [M+H]

Step 2

Ethyl 2-(2-chloro-*N*-(2-((5-chloro-2-(4-chloro-1*H*-1,2,3-triazol-1-yl)phenyl)amino)-2-oxoethyl)acetamido)-3-(4-fluorophenyl)propanoate

Ethyl 2-((2-((5-chloro-2-(4-chloro-1*H*-1,2,3-triazol-1-yl)phenyl)amino)-2-oxoethyl)amino)-3-(4-fluorophenyl)propanoate 47a (130 mg, 270.65 μmol) was dissolved in dichloromethane (10 mL), added with triethylamine (109.55 mg, 1.08 mmol), slowly dropwise added with chloroacetyl chloride (45.85 mg, 405.98 μmol, 32.33 μL) in ice bath, and reacted for 2 hours in ice bath. The reaction solution was added with 30 mL of water for quenching the reaction, and then extracted with ethyl acetate (30 mL×3). Organic phases were combined, dried with anhydrous sodium sulfate, filtered and concentrated under reduced pressure, and the obtained residue was further separated and purified by silicagel column chromatography (eluent: system A) to obtain ethyl 2-(2-chloro-*N*-(2-((5-chloro-2-(4-chloro-1*H*-1,2,3-triazol-1-yl)phenyl)amino)-2-oxoethyl)acetamido)-3-(4-fluorophenyl)propanoate 47b (150 mg) with a yield of 99.5%.
MS m/z (ESI): 555.7 [M+H]

Step 3

Methyl 2-(4-(5-chloro-2-(4-chloro-1*H*-1,2,3-triazol-1-yl)phenyl)-2,5-dioxopiperazin-1-yl)-3-(4-fluorophenyl)propanoate

Ethyl 2-(2-chloro-*N*-(2-((5-chloro-2-(4-chloro-1*H*-1,2,3-triazol-1-yl)phenyl)amino)-2-oxoethyl)acetamido)-3-(4-fluorophenyl)propanoate 47b (150 mg, 269.40 μmol) was dissolved in methanol (8 mL), slowly dropwise added with sodium methylate (5.4M methanol solution, 49.89 μL, 269.41 mmol) in ice bath, and continuously reacted in ice bath for 2 hours. 2M dilute hydrochloric acid was dropwise added to adjust the pH to 6, then the reaction solution was concentrated under reduced pressure, and the obtained residue was further separated and purified by silicagel column chromatography (eluent: system A) to obtain methyl 2-(4-(5-chloro-2-(4-chloro-1*H*-1,2,3-triazol-1-yl)phenyl)-2,5-dioxopiperazin-1-yl)-3-(4-fluorophenyl)propanoate 47c (87 mg) with a yield of 64%. MS m/z (ESI): 506.0 [M+H]

Step 4

2-(4-(5-Chloro-2-(4-chloro-1*H*-1,2,3-triazol-1-yl)phenyl)-2,5-dioxopiperazin-1-yl)-3-(4-fluorophenyl)propanoic acid

Methyl 2-(4-(5-chloro-2-(4-chloro-1*H*-1,2,3-triazol-1-yl)phenyl)-2,5-dioxopiperazin-1-yl)-3-(4-fluorophenyl)propanoate 47c (87 mg, 171.82 μmol) was dissolved in mixed solvent of tetrahydrofuran (6 mL), methanol (2 mL) and water (2 mL), slowly dropwise added with lithium hydroxide monohydrate (7.22 mg, 171.82 μmol) in ice bath, and reacted in ice bath for 3 hours. 2M dilute hydrochloric acid was dropwise added to adjust the pH to 6, then the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(4-fluorophenyl)propanoic acid 47d (67 mg) with a yield of 79%.
MS m/z (ESI): 491.8 [M+H]

Step 5

2-(4-(5-Chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide

2-(4-(5-Chloro-2-(4-chloro-1*H*-1,2,3-triazol-1-yl)phenyl)-2,5-dioxopiperazin-1-yl)-3-(4-fluorophenyl)propanoic acid 47d (67 mg, 136.10 μmol) and 2-methyl-2*H*-indazol-5-amine 19a (30.05 mg, 204.15 μmol) were dissolved in ethyl acetate (10 mL), added with propylphosphonic anhydride (50% ethyl acetate solution, 259.82 mg, 408.3 μmol) and N,N-diisopropylethylamine (87.9 mg, 680.5 μmol, 112.47 μL), and reacted at 60°C for 3 hours. After the reaction was completed, the reaction solution was concentrated under reduced pressure, and the obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide 47 (41 mg) with a yield of 47%.

MS m/z (ESI): 620.8 [M+H]
$^1$H NMR (400 MHz, DMSO-$d_6$) δ 10.12 (s, 1H), 8.66 (s, 1H), 8.26 (s, 1H), 8.10 (s, 1H), 7.87-7.63 (m, 3H), 7.54 (d, J = 9.2 Hz, 1H), 7.42-6.96 (m, 5H), 5.46-5.28 (m, 1H), 4.60-3.91 (m, 4H), 4.13 (s, 3H), 3.29-3.18 (m, 1H), 3.16-3.04 (m, 1H).

Example 48 was synthesized according to the methods for synthesizing of Examples 1-47 of the present invention. The spectrum parameters of Example 48 were shown in the following table:

| No. and structure of example | MS m/z (ESI): | $^1$H NMR (400 MHz, DMSO-$d_6$) |
|---|---|---|
| 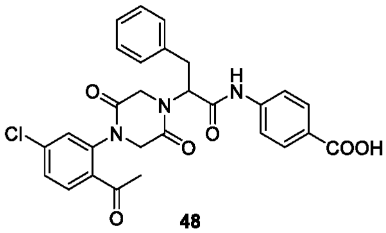 48 | 396.9 [M-136] | δ 12.76 (s, 1H), 10.53 (s, 1H), 7.92 (d, J = 8.3 Hz, 2H), 7.80 (d, J = 8.4 Hz, 1H), 7.73 (d, J = 8.4 Hz, 2H), 7.55 (dd, J = 8.5, 2.1 Hz, 1H), 7.45 (s, 1H), 7.37-7.29 (m, 4H), 7.28-7.21 (m, 1H), 5.45 (dd, J= 10.6, 5.7 Hz, 1H), 4.41-4.08 (m, 4H), 3.32-3.14 (m, 2H), 2.31 (s, 3H). |

Example 49

4-(2-(4-(2-Acetyl-5-chlorophenyl)-2-oxopiperazin-1-yl)-3-phenylpropanamido)benzoic acid

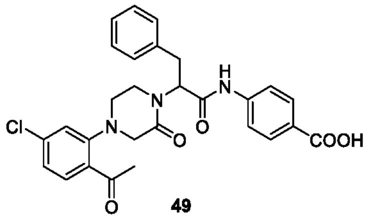

49

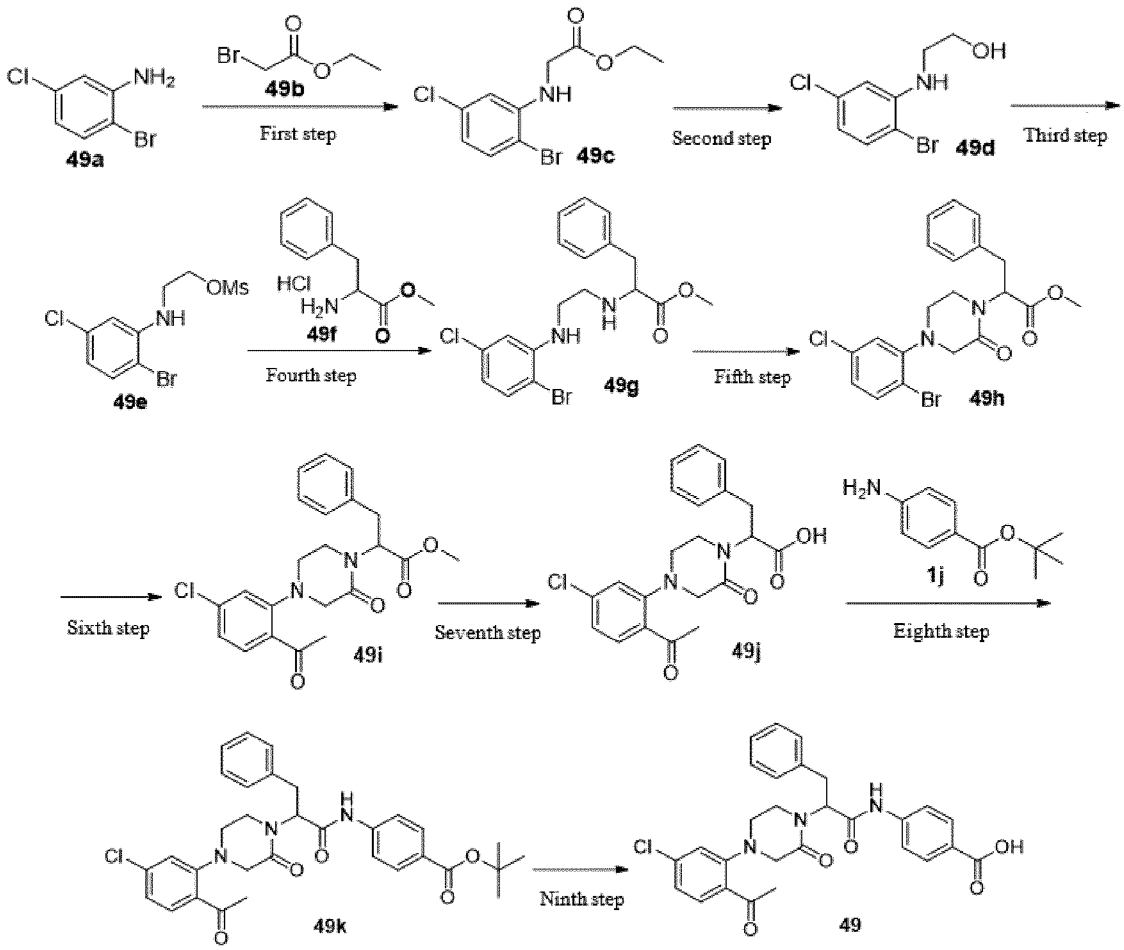


Step 1

Ethyl (2-bromo-5-chlorophenyl)glycinate

2-bromo-5-chloroaniline 49a (5 g, 24.2 mmol) and ethyl bromoacetate 49b (8.08 g, 5.4 mL, 48.4 mmol) were dissolved in N,N-dimethylformamide (60 mL), added with potassium tert-butoxide (5.4 g, 48.4 mmol) in ice bath, stirred for 10 minutes in ice bath, heated to 100°C, and reacted overnight. The reaction solution was added with water (300 mL) for quenching the reaction, and then extracted with ethyl acetate (200 mL×2). Organic phases were combined, washed with water (200 mL×2) and saturated sodium chloride solution (20 mL) in turn, dried with anhydrous sodium sulfate, filtered and concentrated under reduced pressure, and the obtained residue was further separated and purified by silicagel column chromatography (eluent: system A) to obtain ethyl (2-bromo-5-chlorophenyl)glycinate 49c (7.08 g) with a yield of 100%.
MS m/z (ESI): 291.9 [M+H]

Step 2

2-((2-Bromo-5-chlorophenyl)amino)ethane-1-ol

Ethyl (2-bromo-5-chlorophenyl)glycinate 49c (4 g, 13.7 mmol) was dissolved in tetrahydrofuran (40 mL), added with lithium aluminium hydride (779 mg, 20.5 mmol) at 0°C, and then reacted in ice bath for 30 minutes. The reaction solution was added with water (1 mL) for quenching the reaction and then added with 15% sodium hydroxide aqueous solution (1 mL) and water (3 mL), stirred for 10 minutes, added with anhydrous sodium sulfate, dried, filtered, and concentrated under reduced pressure to obtain crude product of 2-((2-bromo-5-chlorophenyl)amino)ethane-1-ol 49d (1.8 g), which was directly used for next reaction.
MS m/z (ESI): 249.9 [M+H]

Step 3

2-((2-bromo-5-chlorophenyl)amino)ethyl methanesulfonate

2-((2-Bromo-5-chlorophenyl)amino)ethane-1-ol 49d (1.8 g, 7.2 mmol) and triethylamine (3 mL, 21.6 mmol) were dissolved in dichloromethane (30 mL), added with methanesulfonyl chloride (0.83 mL, 10.8 mmol) at 0°C, and continuously reacted at 0°C for 1 hour. The reaction solution was added with water (30 mL) for quenching the reaction and extracted with ethyl acetate (50 mL×3), then organic phases were combined, dried with anhydrous sodium sulfate, filtered, and concentrated under reduced pressure to obtain crude product of 2-((2-bromo-5-chlorophenyl)amino)ethyl methanesulfonate 49e (2.37 g), which was directly used for the next reaction.
MS m/z (ESI): 327.9 [M+H]

Step 4

Methyl (2-((2-bromo-5-chlorophenyl)amino)ethyl)phenylalaninate

2-((2-Bromo-5-chlorophenyl)amino)ethyl methanesulfonate 49e (5.1 g, 15.5 mmol) and methyl phenylalanine hydrochloride 49f (5 g, 23.3 mmol) were dissolved in acetonitrile (100 mL), added with potassium carbonate (12.9 g, 93 mmol), and stirred at 80°C for reaction for 48 hours. The reaction solution was filtered and concentrated under reduced pressure, and the obtained residue was further separated and purified by silicagel column chromatography (eluent: system A) to obtain methyl (2-((2-bromo-5-chlorophenyl)amino)ethyl)phenylalaninate 49g (1.35 g) with a yield of 21%.
MS m/z (ESI): 410.9 [M+H]

Step 5

Methyl 2-(4-(2-bromo-5-chlorophenyl)-2-oxopiperazin-1-yl)-3-phenylpropanoate

Methyl (2-((2-bromo-5-chlorophenyl)amino)ethyl)phenylalaninate 49g (300 mg, 0.729 mmol) was dissolved in acetonitrile (20 mL), added with potassium carbonate (302 mg, 2.187 mmol), slowly dropwise added with chloroacetyl chloride (90 mg, 0.802 mmol) at 0°C, continuously stirred at 0°C for 30 minutes, then added with potassium iodide (363 mg, 2.187 mmol), and refluxed for 6 hours. The reaction solution was filtered and concentrated under reduced pressure, and the obtained residue was further separated and purified by silicagel column chromatography (eluent: system A) to obtain methyl 2-(4-(2-bromo-5-chlorophenyl)-2-oxopiperazin-1-yl)-3-phenylpropanoate 49h (240 mg) with a yield of 73%.
MS m/z (ESI): 450.8 [M+H]

Step 6

2-(4-(2-acetyl-5-chlorophenyl)-2-oxopiperazine-1-yl)-3-phenylpropionate

Methyl 2-(4-(2-bromo-5-chlorophenyl)-2-oxopiperazin-1-yl)-3-phenylpropanoate 49h (240 mg, 0.53 mmol), tributyl(1-ethoxyvinyl)tin (959 mg, 2.66 mmol) and bis(triphenylphosphine)palladium(II) chloride (37.2 mg, 0.053 mmol) were dissolved in 1,4-dioxane (10 mL), heated to 100°C under argon atmosphere, and reacted overnight. The reaction solution was concentrated under reduced pressure, and the obtained residue was further separated and purified by silicagel column chromatography (eluent: system A) to obtain methyl 2-(4-(2-acetyl-5-chlorophenyl)-2-oxopiperazin-1-yl)-3-phenylpropanoate 49i (220 mg) with a yield of 90%.
MS m/z (ESI): 414.9 [M+H]

Step 7

2-(4-(2-acetyl-5-chlorophenyl)-2-oxopiperazin-1-yl)-phenylpropanoic acid

Methyl 2-(4-(2-acetyl-5-chlorophenyl)-2-oxopiperazin-1-yl)-3-phenylpropanoate 49i (220 mg, 0.578 mmol) was dissolved in mixed solvent of tetrahydrofuran (6 mL), methanol (2 mL) and water (2 mL), added with lithium hydroxide monohydrate (49 mg, 1.16 mmol) at 0°C, and continuously stirred for reaction for 3 hours. 2M dilute hydrochloric acid was dropwise added to adjust the pH to about 5, then the reaction solution was concentrated under reduced pressure, and the obtained residue was further separated and purified by silicagel column chromatography (eluent: system B) to obtain 2-(4-(2-acetyl-5-chlorophenyl)-2-oxopiperazin-1-yl)-phenylpropanoic acid 49j (200 mg) with a yield of 86%.
MS m/z (ESI): 401.0 [M+H]

Step 8

*Tert*-butyl 4-(2-(4-(2-acetyl-5-chlorophenyl)-2-oxopiperazin-1-yl)-3-phenylpropanamido) benzoate

2-(4-(2-acetyl-5-chlorophenyl)-2-oxopiperazin-1-yl)-phenylpropanoic acid 49j (120 mg, 0.3 mmol) and *tert*-butyl 4-aminobenzoate 1j (58 mg, 0.6 mmol) were dissolved in ethyl acetate (10 mL), added with N,N-diisopropylethylamine (232 mg, 1.8 mmol) and propylphosphonic anhydride (572.72 mg, 900 μmol, ethyl acetate solution), and reacted at 70°C overnight. The reaction solution was concentrated under reduced pressure, and the obtained residue was further separated and purified by silicagel column chromatography (eluent: system B) to obtain *tert*-butyl 4-(2-(4-(2-acetyl-5-chlorophenyl)-2-oxopiperazin-1-yl)-3-phenylpropanamido) benzoate 49k (160 mg) with a yield of 93%.
MS m/z (ESI): 576.2 [M+H]

Step 9

4-(2-(4-(2-acetyl-5-chlorophenyl)-2-oxopiperazin-1-yl)-3-phenylpropanamido)benzoic acid

Tert-butyl 4-(2-(4-(2-acetyl-5-chlorophenyl)-2-oxopiperazin-1-yl)-3-phenylpropanamido) benzoate 49k (160 mg, 0.278mmol) was dissolved in dichloromethane (10 mL), slowly dropwise added with trifluoroacetic acid (1 mL), and reacted at room temperature for 2 hours. After the reaction was completed, the reaction solution was concentrated under reduced pressure, and the obtained residue was further separated and purified by silicagel column chromatography (eluent: system B) to obtain 4-(2-(4-(2-acetyl-5-chlorophenyl)-2-oxopiperazin-1-yl)-3-phenylpropanamido)benzoic acid 49 (33 mg) with a yield of 23%.

MS m/z (ESI): 520.2[M+H]
$^1$H NMR (400 MHz, DMSO-$d_6$) δ 12.75 (s, 1H), 10.48 (s, 1H), 7.90 (d, J = 8.3 Hz, 2H), 7.73 (d, J = 8.3 Hz, 2H), 7.41 (d, J= 8.5 Hz, 1H), 7.34-7.25 (m, 4H), 7.24-7.17 (m, 1H), 7.13-7.06 (m, 2H), 5.53 (dd, J = 10.9, 5.4 Hz, 1H), 3.72-3.59 (m, 2H), 3.57-3.41 (m, 2H), 3.31-3.20 (m, 2H), 3.19-3.08 (m, 2H), 2.34 (s, 3H).

Examples 50-51 were synthesized according to the methods for synthesizing of Examples 1-47 of the present invention. The spectrum parameters of Examples 50-51 were shown in the following table:

| No. and structure of example | MS m/z (ESI): | $^1$H NMR (400 MHz, DMSO-$d_6$) |
|---|---|---|
| 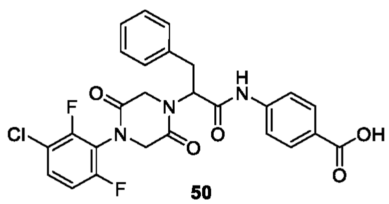 50 | 528.1 [M+H] | δ 12.78 (s, 1H), 10.54 (d, J = 1.6 Hz, 1H), 7.92 (d, J = 8.5 Hz, 2H), 7.73 (d, J = 8.8 Hz, 1H), 7.73-7.65 (m, 1H), 7.36-7.25 (m, 5H), 7.25-7.19 (m, 1H), 5.30 (dd, J = 9.9, 5.9 Hz, 1H), 4.67-3.88 (m, 4H), 3.30-3.15 (m, 1H), 3.13-2.99 (m, 1H). |
| 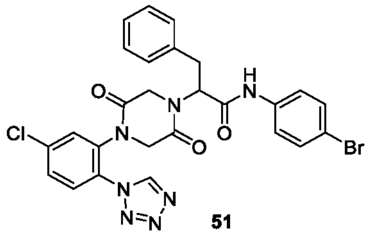 51 | 594.1 [M+H] | δ 10.31 (s, 1H), 9.70 (s, 1H), 7.86-7.69 (m, 3H), 7.62-7.44 (m, 4H), 7.37-7.18 (m, 5H), 5.30 (dd, J = 9.9, 5.9 Hz, 1H), 4.58-3.73 (m, 4H), 3.15-3.00 (m, 2H). |

Example 52

4-(2-(4-(5-chloro-2-(1*H*-tetrazol-1-yl)phenyl)-2,5-dioxopiperazin-1-yl)-3-phenylpropanamido)benzoic acid

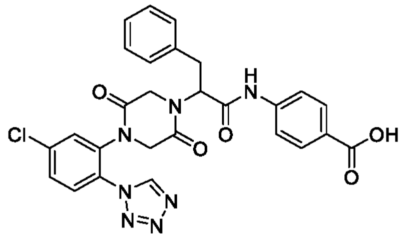

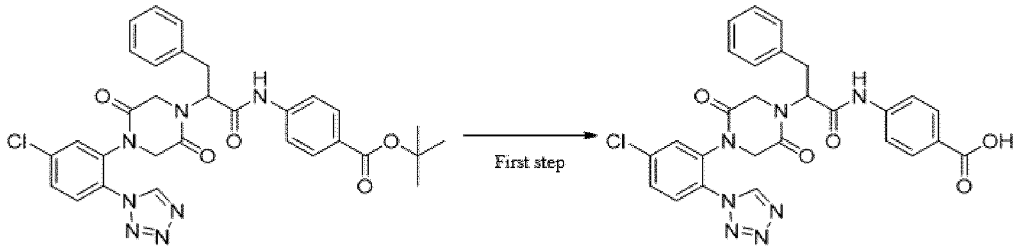


1 52

*Tert*-butyl 4-(2-(4-(5-chloro-2-(1*H*-tetrazol-1-yl)phenyl)-2,5-dioxopiperazin-1-yl)-3-phenylpropanamido) benzoate 1 (90 mg, 146.09 μmol) was dissolved in dichloromethane (15 mL), and then added with trifluoroacetic acid (1.5 mL) and reacted at room temperature for 6 hours. After the reaction was completed, the reaction solution was concentrated under reduced pressure, and the obtained residue was further separated and purified by silicagel column chromatography (eluent: system B) to obtain 4-(2-(4-(5-chloro-2-(1*H*-tetrazol-1-yl)phenyl)-2,5-dioxopiperazin-1-yl)-3-phenylpropanamido)benzoic acid 52 (45 mg) with a yield of 54%.

MS m/z (ESI): 560.1 [M+H]

$^1$H NMR (400 MHz, DMSO-$d_6$) δ 12.77 (s, 1H), 10.50 (s, 1H), 9.72 (s, 1H), 7.91 (d, J = 8.3 Hz, 2H), 7.81 (d, J= 8.6 Hz, 1H), 7.78-7.67 (m, 4H), 7.38-7.19 (m, 5H), 5.39-5.28 (m, 1H), 4.62-3.86 (m, 4H), 3.31-3.18 (m, 1H), 3.18-3.05 (m, 1H).

Examples 53-75 were synthesized according to the methods for synthesizing of Examples 1-47 of the present invention. The spectrum parameters of Examples 53-75 were shown in the following table:

| No. and structure of example | MS m/z (ESI): | $^1$H NMR (400 MHz, DMSO-$d_6$) |
|---|---|---|
| 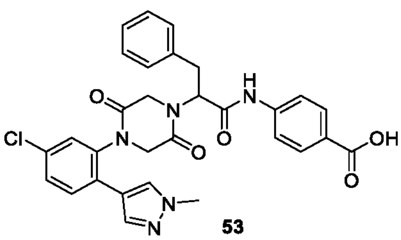 53 | 572.2 [M+H] | δ 12.79 (s, 1H), 10.67 (s, 1H), 8.02-7.86 (m, 2H), 7.81-7.59 (m, 5H), 7.59-7.48 (m, 1H), 7.48-7.19 (m, 6H), 5.67-5.51 (m, 1H), 4.44 (d, J = 16.7 Hz, 1H), 4.39-4.24 (m, 1H), 4.19 (d, J = 16.4 Hz, 1H), 4.08 (d, J = 16.7 Hz, 1H), 3.71 (s, 3H), 3.31-3.10 (m, 2H). |
| 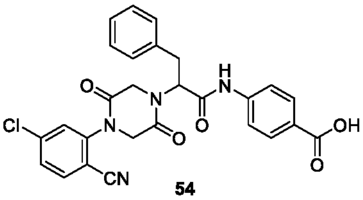 54 | 516.8 [M+H] | δ 12.72 (brs, 1H), 10.44 (s, 1H), 7.99 (d, J = 8.4 Hz, 1H), 7.90 (d, J = 8.5 Hz, 2H), 7.70 (d, J = 8.9 Hz, 2H), 7.70-7.65 (m, 1H), 7.42-7.23 (m, 6H), 4.65 (t, J = 5.4 Hz, 1H), 4.48 (d, J = 16.5 Hz, 1H), 4.02 (d, J = 16.5 Hz, 1H), 3.93 (d, J = 16.5 Hz, 1H), 3.71 (d, J= 16.6 Hz, 1H), 3.30-3.18 (m, 2H). |

(continued)

| No. and structure of example | MS m/z (ESI): | $^1$H NMR (400 MHz, DMSO-$d_6$) |
|---|---|---|
| 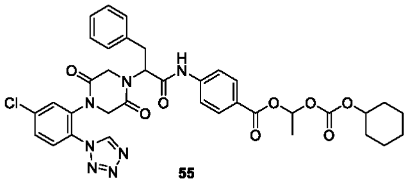 55 | 541.8 [M-187] | δ 10.57 (s, 1H), 9.71 (s, 1H), 7.94 (d, J = 8.3 Hz, 1H), 7.87-7.65 (m, 5H), 7.41-7.14 (m, 6H), 6.90-6.80 (m, 1H), 5.41-5.24 (m, 1H), 4.63-4.49 (m, 1H), 4.65-3.88 (m, 4H), 3.19-3.05 (m, 2H), 1.89-1.76 (m, 2H), 1.69-1.58 (m, 2H), 1.57 (d, J = 5.4 Hz, 3H), 1.52-1.11 (m, 6H). |
| 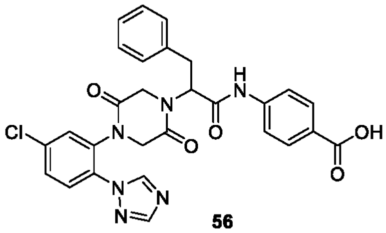 56 | 559.2 [M+H] | δ 10.53 (s, 1H), 8.80 (s, 1H), 8.00-7.83 (m, 3H), 7.78-7.54 (m, 5H), 7.40-7.19 (m, 5H), 5.48-5.34 (t, J = 7.6 Hz, 1H), 4.48-3.89 (m, 4H), 3.18-3.06 (m, 2H). |
| 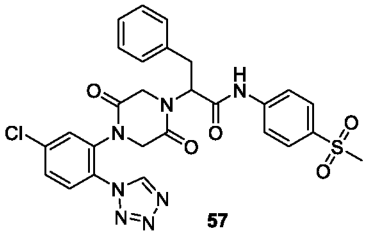 57 | 594.2 [M+H] | δ 10.63 (s, 1H), 9.71 (s, 1H), 8.00-7.67 (m, 7H), 7.40-7.15 (m, 5H), 5.34-5.25 (m, 1H), 4.69-3.91 (m, 4H), 3.31-3.21 (m, 1H), 3.17 (s, 3H), 3.13-3.04 (m, 1H). |
| 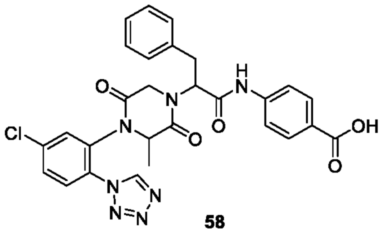 58 | 574.2 [M+H] | δ 10.50-10.35 (m, 1H), 9.70-9.47 (m, 1H), 8.09-7.53 (m, 7H), 7.45-7.08 (m, 5H), 5.52-5.12 (m, 1H), 4.64-3.90 (m, 3H), 3.15-2.99 (m, 1H), 2.93-2.81 (m, 1H), 1.15 (t, J= 7.2 Hz, 3H). |
| 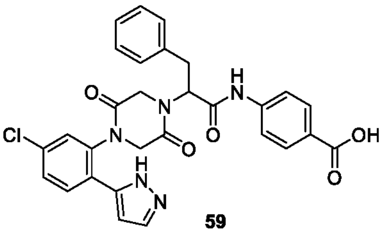 59 | 558.1 [M+H] | δ 12.90 (brs, 2H), 10.58 (s, 1H), 7.93 (d, J = 8.4 Hz, 2H), 7.83 (d, J = 8.6 Hz, 1H), 7.75 (d, J = 8.4 Hz, 2H), 7.59-7.13 (m, 9H), 5.46 (dd, J= 10.6, 5.6 Hz, 1H), 4.49-3.92 (m, 4H), 3.31-3.27 (m, 1H), 3.26-3.16 (m, 1H). |
| 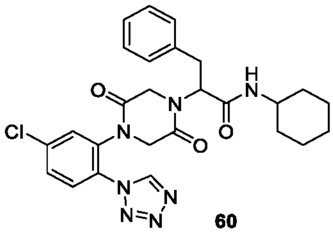 60 | 522.2 [M+H] | δ 9.70 (s, 1H), 7.98 (d, J = 7.9 Hz, 1H), 7.86-7.67 (m, 3H), 7.36-7.10 (m, 5H), 5.21-5.05 (m, 1H), 4.47-3.84 (m, 4H), 3.13-2.99 (m, 1H), 2.95-2.79 (m, 2H), 1.82-1.37 (m, 5H), 1.36-0.98 (m, 5H). |

(continued)

| No. and structure of example | MS m/z (ESI): | $^1$H NMR (400 MHz, DMSO-$d_6$) |
|---|---|---|
| 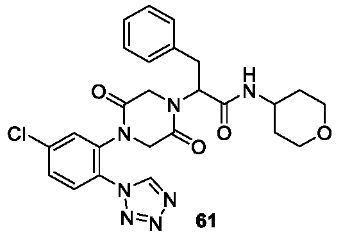 61 | 524.2 [M+H] | δ 9.71 (s, 1H), 8.11 (d, J = 7.6 Hz, 1H), 7.93-7.52 (m, 3H), 7.36-7.14 (m, 5H), 5.20-5.07 (m, 1H), 4.47-3.89 (m, 4H), 3.87-3.67 (m, 3H), 3.40-3.24 (m, 2H), 3.15-3.02 (m, 1H), 2.98-2.85 (m, 1H), 1.75-1.50 (m, 2H), 1.50-1.22 (m, 2H). |
| 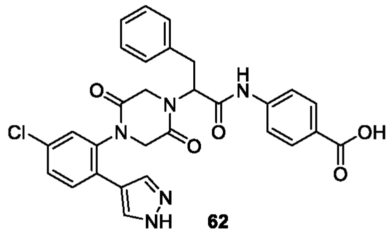 62 | 558.2 [M+H] | δ 12.80 (brs, 1H), 10.57 (s, 1H), 7.98-7.88 (m, 2H), 7.87-7.46 (m, 5H), 7.43 (dd, J= 8.5, 2.3 Hz, 1H), 7.41-7.32 (m, 4H), 7.32-7.18 (m, 2H), 5.56-5.34 (m, 1H), 4.43-4.04 (m, 4H), 3.30-3.13 (m, 2H). |
| 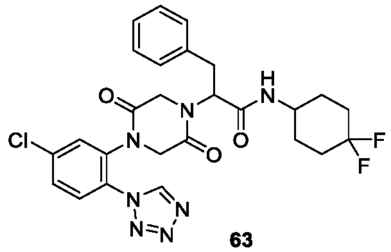 63 | 558.2 [M+H] | δ 9.70 (s, 1H), 8.11 (d, J= 7.7 Hz, 1H), 7.87-7.59 (m, 3H), 7.35-7.13 (m, 5H), 5.21-5.04 (m, 1H), 4.60-3.85 (m, 4H), 3.17-3.04 (m, 1H), 2.99-2.83 (m, 2H), 2.08-1.31 (m, 8H). |
| 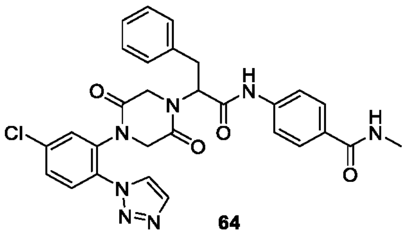 64 | 571.9 [M+H] | δ 10.41 (s, 1H), 8.40-8.29 (m, 1H), 8.19 (brs, 1H), 7.82 (d, J = 8.4 Hz, 2H), 7.76-7.57 (m, 6H), 7.41-7.20 (m, 5H), 5.35 (dd, J= 10.3, 5.7 Hz, 1H), 4.43-3.90 (m, 4H), 3.31-3.22 (m, 1H), 3.17-3.06 (m, 1H), 2.77 (d, J = 4.4 Hz, 3H). |
| 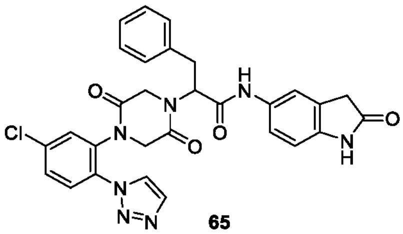 65 | 569.9 [M+H] | δ 10.33 (s, 1H), 10.06 (s, 1H), 8.20 (brs, 1H), 7.79-7.57 (m, 4H), 7.49 (s, 1 H), 7.37-7.20 (m, 6H), 6.76 (d, J = 8.3 Hz, 1H), 5.32 (dd, J = 10.1, 6.0 Hz, 1H), 4.34-3.91 (m, 4H), 3.47 (s, 2H), 3.27-3.18 (m, 1H), 3.13-3.01 (m, 1H). |
| 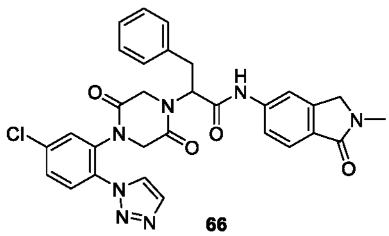 66 | 583.9 [M+H] | δ 10.48 (s, 1H), 8.20 (brs, 1H), 7.95 (s, 1H), 7.79-7.62 (m, 3H), 7.62 (d, J = 8.3 Hz, 1H), 7.56 (d, J = 8.4 Hz, 1H), 7.39-7.21 (m, 5H), 5.36 (dd, J = 10.1, 5.9 Hz, 1H), 4.44 (s, 2H), 4.37-3.93 (m, 4H), 3.32-3.21 (m, 1H), 3.18-3.08 (m, 1H), 3.05 (s, 3H). |

(continued)

| No. and structure of example | MS m/z (ESI): | $^1$H NMR (400 MHz, DMSO-$d_6$) |
|---|---|---|
| 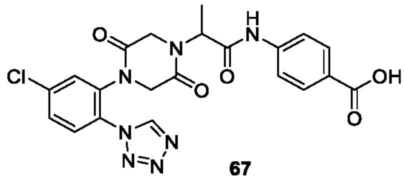 67 | 484.0 [M+H] | δ 10.23 (s, 1H), 9.78 (s, 1H), 7.99-7.44 (m, 5H), 7.61 (d, J = 8.3 Hz, 2H), 5.11-4.94 (m, 1H), 4.53-4.05 (m, 4H), 1.37 (d, J = 7.2 Hz, 3H). |
| 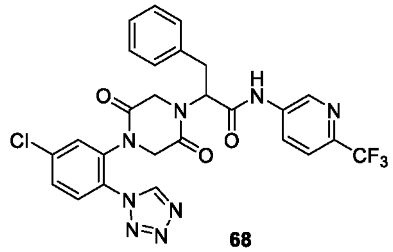 68 | 585.1 [M+H] | δ 10.76 (s, 1H), 9.71 (s, 1H), 8.89 (s, 1H), 8.31 (d, J= 8.5 Hz, 1H), 8.02-7.59 (m, 4H), 7.43-7.14 (m, 5H), 5.31 (s, 1H), 3.99 (m, 4H), 3.34-3.00 (m, 2H). |
| 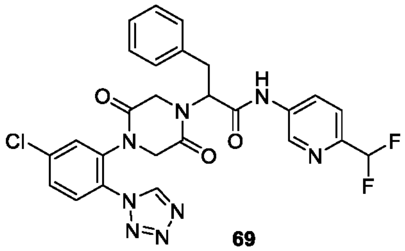 69 | 567.2 [M+H] | δ 10.62 (s, 1H), 9.71 (s, 1H), 8.83 (s, 1H), 8.22 (d, J = 7.9 Hz, 1H), 7.86-7.64 (m, 4H), 7.38-7.19 (m, 5H), 6.92 (t, J= 55.2 Hz, 1H), 5.38-5.24 (m, 1H), 4.69-3.89 (m, 4H), 3.32-3.20 (m, 1H), 3.16-3.04 (m, 1H). |
| 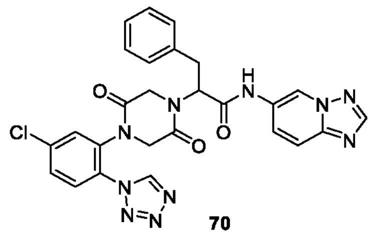 70 | 556.8 [M+H] | δ 10.59 (s, 1H), 9.71 (s, 1H), 9.41 (s, 1H), 8.46 (s, 1H), 7.95-7.51 (m, 5H), 7.48-7.01 (m, 5H), 5.43-5.21 (m, 1H), 4.72-3.88 (m, 4H), 3.31-3.19 (m, 1H), 3.16-3.01 (m, 1H). |
| 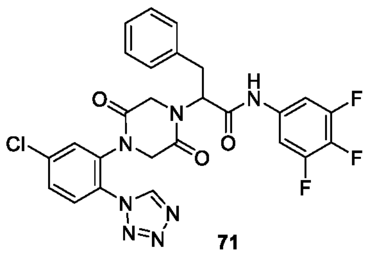 71 | 569.8 [M+H] | δ 10.53 (s, 1H), 9.73 (s, 1H), 7.89-7.68 (m, 2H), 7.67-7.56 (m, 1H), 7.56-7.46 (m, 2H), 7.34-7.16 (m, 5H), 5.24 (s, 1H), 4.66-3.83 (m, 4H), 3.29-3.14 (m, 1H), 3.13-3.01 (m, 1H). |
| 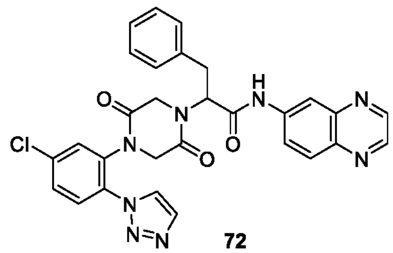 72 | 566.9 [M+H] | δ 10.72 (s, 1H), 8.91 (s, 1H), 8.85 (s, 1H), 8.52 (s, 1H), 8.22 (brs, 1H), 8.09 (d, J = 9.1 Hz, 1H), 7.95 (d, J = 9.2 Hz, 1H), 7.85-7.58 (m, 4H), 7.41-7.18 (m, 5H), 5.41 (dd, J= 10.3, 5.8 Hz, 1H), 4.54-3.87 (m, 4H), 3.41-3.26 (m, 1H), 3.26-3.10 (m, 1H). |

(continued)

| No. and structure of example | MS m/z (ESI): | $^1$H NMR (400 MHz, DMSO-$d_6$) |
|---|---|---|
| 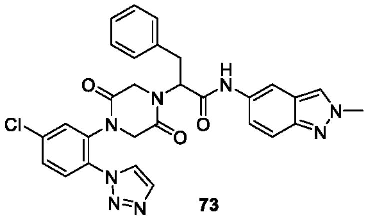 73 | 568.9 [M+H] | δ 10.15 (s, 1H), 8.28 (s, 1H), 8.20 (brs, 1H), 8.10 (s, 1H), 7.85-7.61 (m, 4H), 7.56 (d, J = 9.2 Hz, 1H), 7.41-7.17 (m, 6H), 5.38 (dd, J = 10.2, 6.0 Hz, 1H), 4.53-3.87 (m, 4H), 4.14 (s, 3H), 3.35-3.18 (m, 1H), 3.19-3.02 (m, 1H). |
| 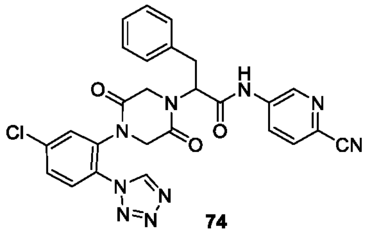 74 | 542.0 [M+H] | δ 10.83 (s, 1H), 9.71 (s, 1H), 8.89 (s, 1H), 8.27 (d, J = 8.6 Hz, 1H), 8.02 (d, J = 8.6 Hz, 1H), 7.88-7.66 (m, 3H), 7.44-7.00 (m, 5H), 5.28 (s, 1H), 4.73-3.79 (m, 4H), 3.28-3.19 (m, 1H), 3.15-3.01 (m, 1H). |
| 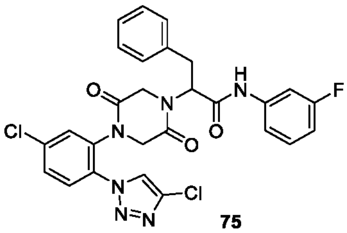 75 | 566.8 [M+H] | δ 10.39 (s, 1H), 8.63 (s, 1H), 7.81-7.66 (m, 3H), 7.62 (d, J = 11.7 Hz, 1H), 7.42-7.18 (m, 7H), 6.97-6.85 (m, 1H), 5.42-5.25 (m, 1H), 4.48-3.90 (m, 4H), 3.32-3.19 (m, 1H), 3.17-3.03 (m, 1H). |

Example 76

1-(1-(6-acetyl-1*H*-benzo[*d*]imidazol-2-yl)-2-phenylethyl)-4-(5-chloro-2-(1*H*-tetrazol-1-yl)phenyl)piperazine-2,5-dione

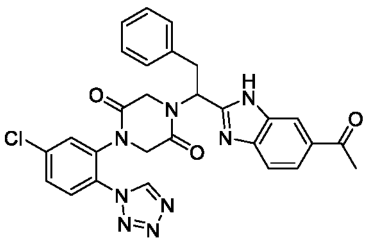

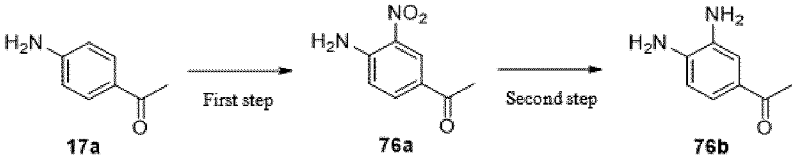

17a 76a 76b

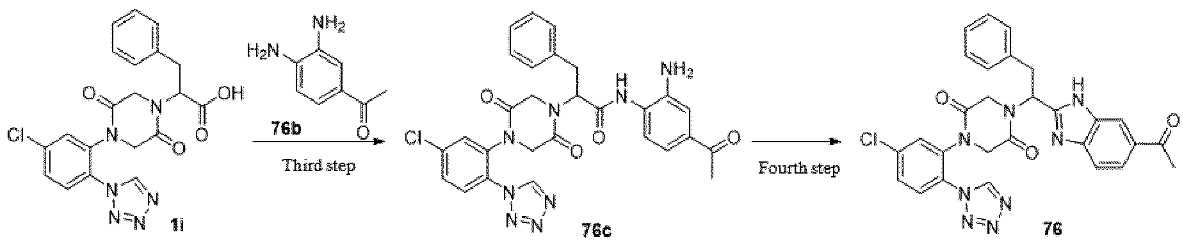


1i

76c

76

Step 1

1 -(4-amino-3 -nitrophenyl)ethan-1 -one

1-(4-aminophenyl)ethan-1-one 17a (5 g, 36.99 mmol) was dissolved in concentrated sulfuric acid (50 mL), slowly added with potassium nitrate (1.87 g, 18.50 mmol) at 0-5°C, and then stirred at 0-5°C for 1 hour. Ethyl acetate (50 mL) was added to dilute the reaction solution, and then the reaction solution was poured into 50 mL of ice water, and subjected to liquid separation. An aqueous phase was extracted with ethyl acetate (50 mL×3), organic phases were combined and washed with saturated sodium chloride solution (50 mL), dried with anhydrous sodium sulfate, filtered, and concentrated under reduced pressure. The obtained residue was further separated and purified by silicagel column chromatography (eluent: system A) to obtain 1-(4-amino-3-nitrophenyl)ethan-1-one 76a (1.8 g) with a yield of 27%.
MS m/z (ESI): 181.0 [M+H]

Step 2

1-(3,4-diaminophenyl)ethan-1-one

1-(4-amino-3-nitrophenyl)ethan-1-one 76a (11 g, 61.06 mmol) was dissolved in mixed solvent of ethanol (16 mL) and water (4 mL), added with iron powder (34.10 g, 610.57 mmol) and ammonium chloride (9.80 g, 183.17 mmol), and reacted at 80°C for 4 hours. The reaction solution was filtered and concentrated under reduced pressure, and the obtained residue was further separated and purified by silicagel column chromatography (eluent: system B) to obtain 1-(3,4-diaminophenyl)ethan-1-one 76b (720 mg) with a yield of 48%.
MS m/z (ESI): 151.1 [M+H]

Step 3

*N*-(4-acetyl-2-aminophenyl)-2-(4-(5-chloro-2-(1*H*-tetrazol-1-yl)phenyl)-2,5-dioxopiperazin-1-yl)-3-phenylpropanamide

2-(4-(5-chloro-2-(1*H*-tetrazol-1-yl)phenyl)-2,5-dioxopiperazin-1-yl)-3-phenylpropanoic acid 1i (100 mg, 226.84 μmol) and 1-(3,4-diaminophenyl)ethan-1-one 76b (40.88 mg, 272.21 μmol) were dissolved in ethyl acetate (10 mL), added with N,N-diisopropylethylamine (175.90 mg, 1.36 mmol) and propylphosphonic anhydride (433.06 mg, 680.52 μmol, 50% ethyl acetate solution), and reacted at 60°C for 3 hours. The reaction solution was concentrated under reduced pressure, and the obtained residue was further separated and purified by silicagel column chromatography (eluent: system B) to obtain *N*-(4-acetyl-2-aminophenyl)-2-(4-(5-chloro-2-(1*H*-tetrazol-1-yl)phenyl)-2,5-dioxopiperazin-1-yl)-3-phenylpropanamide 76c (129 mg) with a yield of 99%.
MS m/z (ESI): 572.8 [M+H]

Step 4

1-(1-(6-acetyl-1*H*-benzo[*d*]imidazol-2-yl)-2-phenethyl)-4-(5-chloro-2-(1*H*-tetrazol-1-yl)phenyl)piperazine-2,5-dione

*N*-(4-acetyl-2-aminophenyl)-2-(4-(5-chloro-2-(1*H*-tetrazol-1-yl)phenyl)-2,5-dioxopiperazin-1-yl)-3-phenylpropanamide 76c (129 mg, 225.13 μmol) was dissolved in glacial acetic acid (5 mL), and reacted at 120°C for 10 hours. LC-MS test showed that the reaction was completed. The reaction solution was concentrated under reduced pressure, and the obtained residue was further separated and purified by silicagel column chromatography (eluent: system B) to obtain 1-(1-(6-acetyl-1*H*-benzo[*d*]imidazol-2-yl)-2-phenylethyl)-4-(5-chloro-2-(1*H*-tetrazol-1-yl)phenyl)piperazine-2,5-dione 76 (27 mg) with a yield of 17%.

MS m/z (ESI): 554.8 [M+H]

1H NMR (400 MHz, DMSO-$d_6$) δ 9.75 (s, 1H), 8.27 (s, 1H), 7.96-7.54 (m, 6H), 7.50-7.13 (m, 5H), 6.02 (s, 1H), 4.51-3.95 (m, 4H), 3.35-3.11 (m, 2H), 2.66 (s, 3H).

Examples 77-79 were synthesized according to the method for synthesizing of Example 76 of the present invention. The spectrum parameters of Examples 77-79 were shown in the following table:

| No. and structure of example | MS m/z (ESI): | $^1$H NMR (400 MHz, DMSO-$d_6$) |
|---|---|---|
| 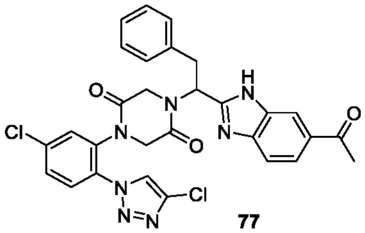 77 | 587.8 [M+H] | δ 8.68 (s, 1H), 8.24 (s, 1H), 7.87 (d, J = 8.5 Hz, 1H), 7.82-7.53 (m, 5H), 7.42-7.13 (m, 5H), 6.07-5.91 (m, 1H), 4.55-3.99 (m, 4H), 3.54-3.18 (m, 2H), 2.64 (s, 3H). |
| 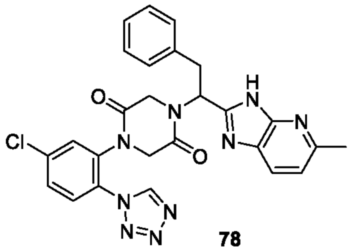 78 | 528.2 [M+H] | δ 9.74 (s, 1H), 8.10 (s, 1H), 7.93-7.50 (m, 3H), 7.41-7.16 (m, 7H), 5.97 (dd, J = 9.2, 6.5 Hz, 1H), 4.67-3.99 (m, 4H), 3.35-3.21 (m, 2H), 2.62 (s, 3H). |
| 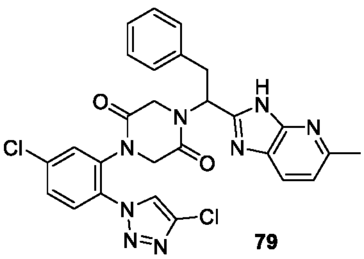 79 | 561.2 [M+H] | δ 8.69 (s, 1H), 8.13 (brs, 1H), 7.87-7.50 (m, 4H), 7.42-7.14 (m, 6H), 6.08-5.91 (m, 1H), 4.70-4.05 (m, 4H), 3.39-3.22 (m, 2H), 2.63 (s, 3H). |

Examples 80-94 were synthesized according to the methods for synthesizing of Examples 1-47 of the present invention. The spectrum parameters of Examples 80-94 were shown in the following table:

| No. and structure of example | MS m/z (ESI): | $^1$H NMR (400 MHz, DMSO-$d_6$) |
|---|---|---|
| 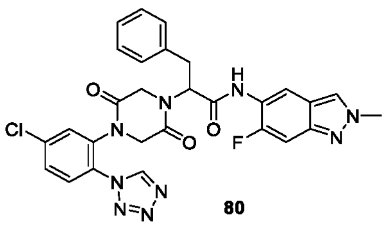 80 | 588.2 [M+H] | δ 9.91 (s, 1H), 9.71 (s, 1H), 8.36 (s, 1H), 8.03 (brs, 1H), 7.86-7.70 (m, 2H), 7.43 (d, J = 11.6 Hz, 1H), 7.38-7.14 (m, 5H), 5.57-5.44 (s, 1H), 4.50-3.86 (m, 4H), 4.14 (s, 3H), 3.33-3.18 (m, 1H), 3.17-3.02 (m, 1H). |
| 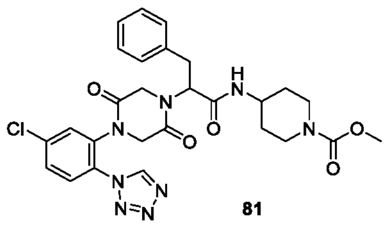 81 | 580.9 [M+H] | δ 9.77 (d, J = 9.6 Hz, 1H), 7.89-7.67 (m, 2H), 7.36-6.94 (m, 6H), 5.64-5.43 (m, 1H), 4.68-3.62 (m, 7H), 3.64-3.46 (m, 2H), 3.52 (s, 3H), 3.10- 2.97 (m, 1H), 2.93-2.75 (m, 2H), 1.85-1.61 (m, 2H), 1.35-1.07 (m, 2H). |

(continued)

| No. and structure of example | MS m/z (ESI): | $^1$H NMR (400 MHz, DMSO-$d_6$) |
|---|---|---|
| 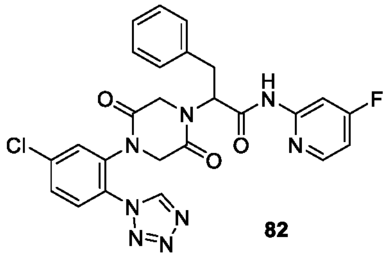 82 | 534.9 [M+H] | δ 11.16 (s, 1H), 9.68 (s, 1H), 8.40 (dd, J = 9.2, 5.7 Hz, 1H), 7.90 (d, J = 11.8 Hz, 1H), 7.83-7.71 (m, 2H), 7.40-7.17 (m, 6H), 7.17-7.04 (m, 1H), 5.42 (dd, J = 11.3, 4.9 Hz, 1H), 4.62-3.87 (m, 4H), 3.18-3.05 (m, 2H). |
| 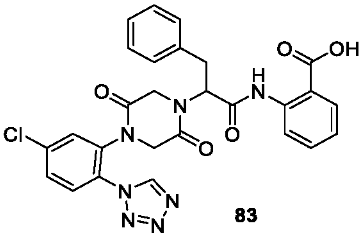 83 | 560.1 [M+H] | δ 9.82 (s, 1H), 8.46 (d, J = 8.3 Hz, 1H), 8.25 (brs, 1H), 7.99 (d, J = 7.8 Hz, 1H), 7.95 (s, 1H), 7.87-7.55 (m, 3H), 7.55-7.41 (m, 1H), 7.38-7.03 (m, 6H), 5.35-5.28 (m, 1H), 4.69-4.08 (m, 4H), 3.29-3.02 (m, 2H). |
| 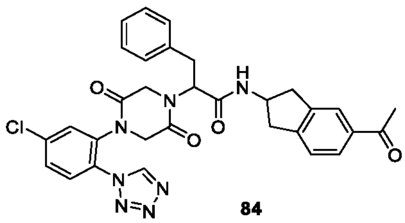 84 | 597.8 [M+H] | δ 9.71 (s, 1H), 8.50 (d, J = 6.9 Hz, 1H), 7.92-7.69 (m, 4H), 7.43-7.12 (m, 6H), 5.32 (t, J = 5.0 Hz, 1H), 5.21-5.07 (m, 1H), 4.54-4.43 (m, 1H), 4.25-3.84 (m, 4H), 3.27-3.17 (m, 2H), 3.14-3.04 (m, 1H), 3.00-2.80 (m, 2H), 2.80-2.68 (m, 1H), 2.55 (s, 3H). |
| 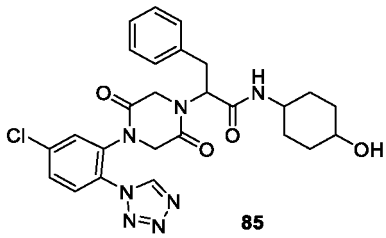 85 | 537.9 [M+H] | δ 9.76-9.64 (m, 1H), 8.09-7.90 (m, 1H), 7.87-7.68 (m, 2H), 7.37-7.10 (m, 6H), 5.28-5.05 (m, 1H), 4.60-3.81 (m, 4H), 3.73-3.61 (m, 1H), 3.63-3.50 (m, 1H), 1.88-1.67 (m, 1H), 1.68-1.05 (m, 10H). |
| 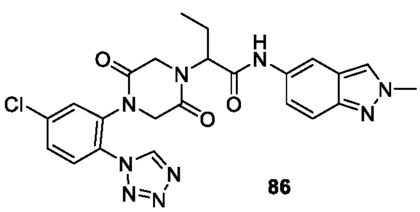 86 | 507.8 [M+H] | δ 10.11 (s, 1H), 9.79 (s, 1H), 8.24 (s, 1H), 8.09 (s, 1H), 7.91 (s, 1H), 7.88-7.71 (m, 2H), 7.54 (d, J = 9.2 Hz, 1H), 7.25 (d, J = 9.2 Hz, 1H), 4.97 (dd, J = 10.4, 5.5 Hz, 1H), 4.85-3.65 (m, 4H), 4.13 (s, 3H), 1.96-1.82 (m, 1H), 1.83-1.68 (m, 1H), 0.98-0.62 (m, 3H). |
| 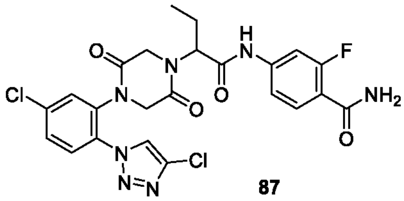 87 | 547.8 [M+H] | δ 10.56 (s, 1H), 8.70 (s, 1H), 7.86 (d, J= 2.0 Hz, 1H), 7.82-7.60 (m, 4H), 7.60-7.43 (m, 2H), 7.35 (dd, J = 8.7, 2.0 Hz, 1H), 4.94 (dd, J= 10.7, 5.3 Hz, 1H), 4.65-3.80 (m, 4H), 1.96-1.71 (m, 2H), 0.99-0.65 (m, 3H). |

(continued)

| No. and structure of example | MS m/z (ESI): | $^1$H NMR (400 MHz, DMSO-$d_6$) |
|---|---|---|
| 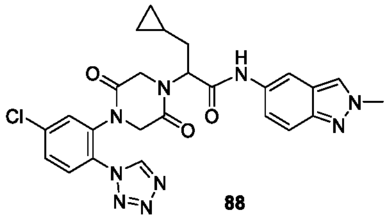 88 | 533.9 [M+H] | δ 10.07 (s, 1H), 9.77 (s, 1H), 8.24 (s, 1H), 8.07 (s, 1H), 7.91 (d, J = 2.2 Hz, 1H), 7.83 (d, J = 8.6 Hz, 1H), 7.77 (dd, J = 8.6, 2.3 Hz, 1H), 7.54 (d, J = 9.1 Hz, 1H), 7.25 (dd, J = 9.2, 1.9 Hz, 1H), 5.19-5.03 (m, 1H), 4.83-4.05 (m, 4H), 4.13 (s, 3H), 2.04-1.89 (m, 1H), 1.58-1.37 (m, 1H), 0.59-0.29 (m, 3H), 0.25-0.06 (m, 2H). |
| 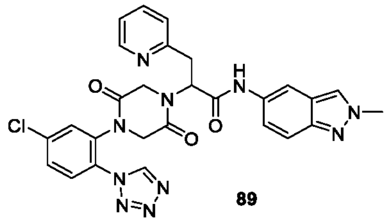 89 | 571.1 [M+H] | δ 10.17 (s, 1H), 9.74 (s, 1H), 8.52 (d, J= 4.9 Hz, 1H), 8.26 (s, 1H), 8.08 (s, 1H), 7.91-7.70 (m, 3H), 7.82 (d, J = 8.6 Hz, 1H), 7.54 (d, J = 9.2 Hz, 1H), 7.36 (d, J = 7.7 Hz, 1H), 7.32-7.23 (m, 2H), 5.49 (dd, J = 9.9, 5.6 Hz, 1H), 4.70-4.02 (m, 4H), 4.13 (s, 3H), 3.29-3.14 (m, 2H). |
| 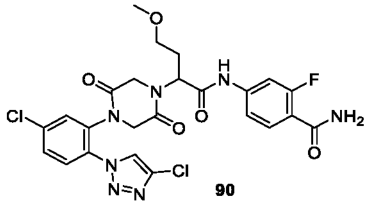 90 | 577.8 [M+H] | δ 10.50 (s, 1H), 8.72 (s, 1H), 7.86 (s, 1H), 7.82-7.60 (m, 4H), 7.60-7.45 (m, 2H), 7.39 (d, J = 8.7 Hz, 1H), 5.07 (s, 1H), 4.66-3.90 (m, 4H), 3.48-3.41 (m, 2H), 3.25 (s, 3H), 2.17-1.95 (m, 2H). |
| 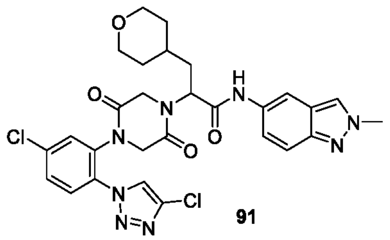 91 | 610.8 [M+H] | δ 10.13 (s, 1H), 8.76 (s, 1H), 8.25 (s, 1H), 8.10 (s, 1H), 7.87 (s, 1H), 7.80-7.68 (m, 2H), 7.54 (d, J = 9.2 Hz, 1H), 7.27 (d, J = 9.2 Hz, 1H), 5.27-5.15 (m, 1H), 4.89-4.04 (m, 4H), 4.13 (s, 3H), 4.03-3.74 (m, 4H), 1.90-1.55 (m, 4H), 1.46-1.10 (m, 3H). |
| 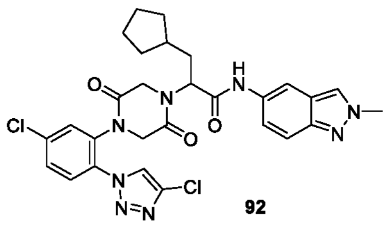 92 | 594.8 [M+H] | δ 10.13 (s, 1H), 8.73 (s, 1H), 8.24 (s, 1H), 8.10 (s, 1H), 7.86 (s, 1H), 7.81-7.68 (m, 2H), 7.54 (d, J = 9.2 Hz, 1H), 7.27 (d, J = 9.2 Hz, 1H), 5.11 (dd, J = 10.5, 5.4 Hz, 1H), 4.82-4.00 (m, 4H), 4.13 (s, 3H), 2.04-1.38 (m, 9H), 1.34-1.04 (m, 2H). |
| 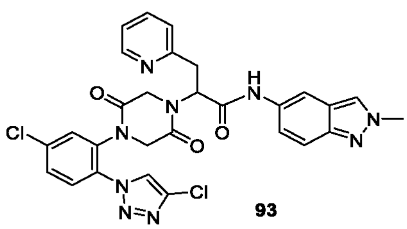 93 | 604.1 [M+H] | δ 10.18 (s, 1H), 8.68 (s, 1H), 8.53 (d, J= 4.9 Hz, 1H), 8.26 (s, 1H), 8.10 (s, 1H), 7.88-7.66 (m, 4H), 7.54 (d, J = 9.1 Hz, 1H), 7.40 (d, J = 7.8 Hz, 1H), 7.35-7.22 (m, 2H), 5.50 (dd, J = 10.0, 5.6 Hz, 1H), 4.57-3.91 (m, 4H), 4.13 (s, 3H), 3.30-3.18 (m, 2H). |

(continued)

| No. and structure of example | MS m/z (ESI): | $^1$H NMR (400 MHz, DMSO-$d_6$) |
|---|---|---|
| 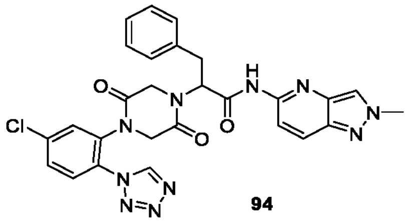 94 | 570.8 [M+H] | NA |

Examples 95 and 96

(*R*)-2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide 95

(*S*)-2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide 96

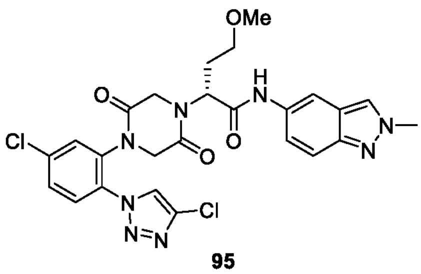

95

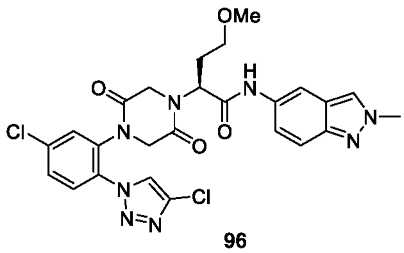

96

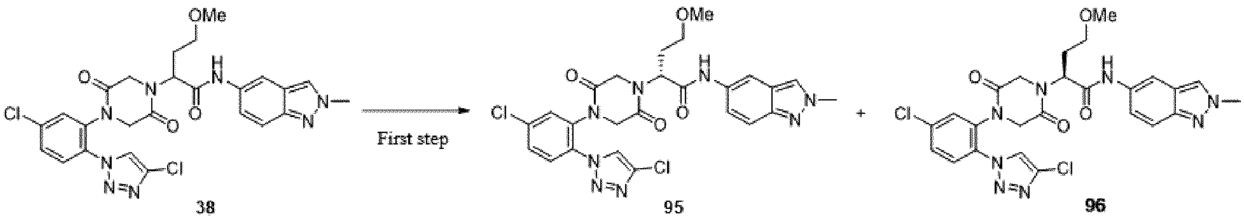


38 95 96

2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide 38 (2.9 g, 5.075 mmol) was subjected to SFC chiral resolution (column model: ChiralCel OJ, 250×30 mm I.D., 5 μm; mobile phase: A for $CO_2$ and B for Ethanol; column pressure: 100 bar; flow rate: 50 mL/min; detection wavelength: 220 nm; column temperature: 38°C) to obtain (*R*)-2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide 95 (retention time ($T_R$): 1.612 min; 1.33 g) and (*S*)-2-(4-(5-chloro-2-(4-chloro-1*H*-1,2,3-triazol-1-yl)phenyl)-2,5-dioxopiperazin-1-yl)-4-methoxy-*N*-(2-methyl-2*H*-indazol-5-yl)butanamide 96 (retention time ($T_R$): 1.944 min; 1.34 g).

**95**

MS m/z (ESI): 571.1 [M+H]

$^1$H NMR (400 MHz, DMSO-$d_6$) δ 10.07 (s, 1H), 8.75 (s, 1H), 8.25 (s, 1H), 8.10 (s, 1H), 7.88 (d, J = 2.0 Hz, 1H), 7.82-7.69 (m, 2H), 7.54 (d, J = 9.1 Hz, 1H), 7.27 (dd, J = 9.2, 1.9 Hz, 1H), 5.13 (s, 1H), 4.71-3.89 (m, 4H), 4.13 (s, 3H), 3.39-3.15 (m, 2H), 3.27 (s, 3H), 2.18-1.97 (m, 2H).

**96**

MS m/z (ESI): 571.1 [M+H]

$^1$H NMR (400 MHz, DMSO-$d_6$) δ 10.07 (s, 1H), 8.75 (s, 1H), 8.25 (s, 1H), 8.10 (s, 1H), 7.88 (d, J = 2.0 Hz, 1H), 7.80-7.69 (m, 2H), 7.54 (d, J = 9.1 Hz, 1H), 7.27 (dd, J = 9.3, 1.9 Hz, 1H), 5.12 (s, 1H), 4.78-3.87 (m, 4H), 4.13 (s, 3H), 3.39-3.15 (m, 2H), 3.27 (s, 3H), 2.17-1.96 (m, 2H).

Examples 97 and 98

(*S*)-2-(4-(5-chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-phenylpropanamide 97

(*R*)-2-(4-(5-chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-phenylpropanamide 98

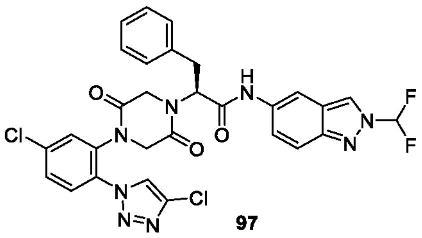
97

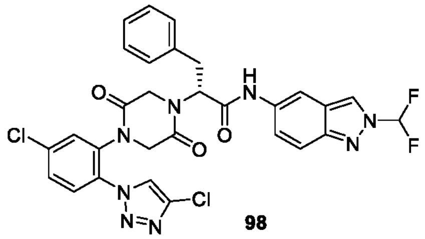
98

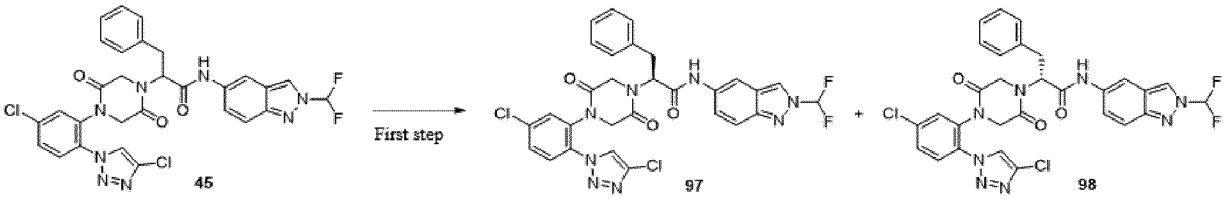
45

97
+
98

2-(4-(5-Chloro-2-(4-chloro-1H 1,2,3-triazol-1-yl)phenyl)-2,5-dioxopiperazin-1-yl)-*N*-(2-(difluoromethyl)-2*H*-indazol-5-yl)-3-phenylpropanamide 45 (900 mg,1.407 mmol) was subjected to SFC chiral resolution (column model: ChiralCel OD, 250×30 mm I.D., 5 μm; mobile phase: A for $CO_2$ and B for Ethanol; column pressure: 100 bar; flow rate: 70 mL/min; detection wavelength: 220 nm; column temperature: 38°C) to obtain (*S*)-2-(4-(5-chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-phenylpropanamide 97 (retention time ($T_R$): 2.863 min; 683 mg) and (*R*)-2-(4-(5-chloro-2-(4-chloro-1*H*-1,2,3-triazol-1-yl)phenyl)-2,5-dioxopiperazin-1-yl)-*N*-(2-(difluoromethyl)-2*H*-indazol-5-yl)-3-phenylpropanamide 98 (retention time ($T_R$): 3.978 min; 152 mg).

**97**

MS m/z (ESI): 639.1 [M+H]

$^1$H NMR (400 MHz, DMSO-$d_6$) δ 10.26 (s, 1H), 8.81 (s, 1H), 8.64 (s, 1H), 8.24 (s, 1H), 8.09 (t, J = 59.1 Hz, 1H), 7.78-7.63 (m, 4H), 7.44-7.18 (m, 6H), 5.45-5.34 (m, 1H), 4.35-3.95 (m, 4H), 3.32-3.24 (m, 1H), 3.16-3.03 (m, 1H).

**98**

MS m/z (ESI): 639.2 [M+H]

$^1$H NMR (400 MHz, DMSO-$d_6$) δ 10.26 (s, 1H), 8.81 (s, 1H), 8.65 (s, 1H), 8.24 (s, 1H), 8.10 (t, J = 59.2 Hz, 1H), 7.86-7.59 (m, 4H), 7.50-7.11 (m, 6H), 5.48-5.34 (m, 1H), 4.51-3.91 (m, 4H), 3.36-3.22 (m, 1H), 3.20-3.03 (m, 1H).

Examples 99 and 100

(*R*)-2-(4-(5-chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide 99

(*S*)-2-(4-(5-chloro-2-(4-chloro-1*H*-1,2,3-triazol-1-yl)phenyl)-2,5-dioxopiperazin-1-yl)-3-(4-fluorophenyl)-*N*-(2-methyl-2H-indazol-5-yl)propanamide 100

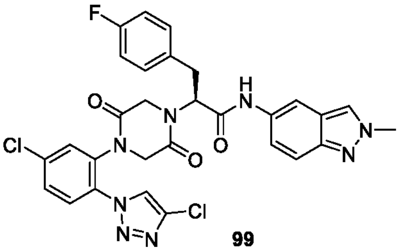

99

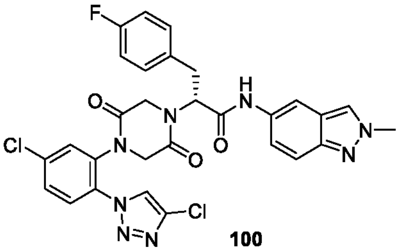

100

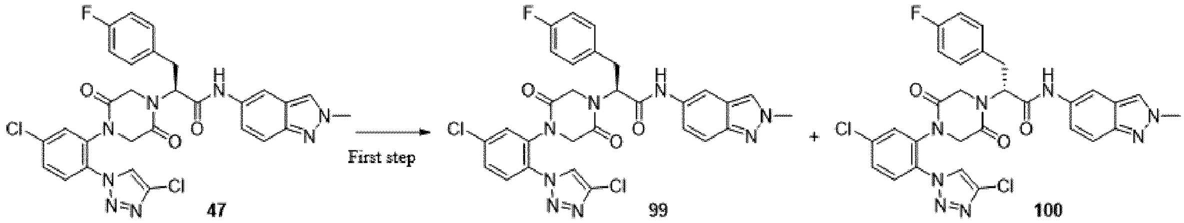


47 99 100

2-(4-(5-chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide 47 (1.05 g, 1.690 mmol) was subjected to SFC chiral resolution (column model: ChiralPak AD, 250×30 mm I.D., 10 μm; mobile phase: A for $CO_2$ and B for Isopropanol; column pressure: 100 bar; flow rate: 80 mL/min; detection wavelength: 220 nm; column temperature: 38°C) to obtain (*R*)-2-(4-(5-chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide 99 (retention time ($T_R$): 3.008 min, 478 mg) and (*S*)-2-(4-(5-chloro-2-(4-chloro-1*H*-1,2,3-triazol-1-yl)phenyl)-2,5-dioxopiperazin-1-yl)-3-(4-fluorophenyl)-*N*-(2-methyl-2*H*-indazol-5-yl)propanamide (retention time 100 ($T_R$): 4.017 min, 462 mg).

**99**

MS m/z (ESI): 621.1 [M+H]
$^1$H NMR (400 MHz, DMSO-$d_6$) δ 10.12 (s, 1H), 8.66 (s, 1H), 8.26 (s, 1H), 8.10 (s, 1H), 7.87-7.63 (m, 3H), 7.54 (d, J = 9.2 Hz, 1H), 7.42-6.96 (m, 5H), 5.48-5.27 (m, 1H), 4.41-3.95 (m, 4H), 4.14 (s, 3H), 3.30-3.20 (m, 1H), 3.17-3.04 (m, 1H).

**100**

MS m/z (ESI): 621.2 [M+H]
$^1$H NMR (400 MHz, DMSO-$d_6$) δ 10.11 (s, 1H), 8.66 (s, 1H), 8.26 (s, 1H), 8.10 (s, 1H), 7.88-7.62 (m, 3H), 7.54 (d, J = 9.1 Hz, 1H), 7.44-7.04 (m, 5H), 5.48-5.28 (m, 1H), 4.55-3.90 (m, 4H), 4.14 (s, 3H), 3.29-3.19 (m, 1H), 3.19-3.01 (m, 1H).

Examples 101-104 were synthesized according to the methods for synthesizing of Examples 1-47 of the present invention. The spectrum parameters of Examples 101-104 were shown in the following table:

| No. and structure of example | MS m/z (ESI): | $^1H$ NMR (400 MHz, DMSO-$d_6$) |
|---|---|---|
| 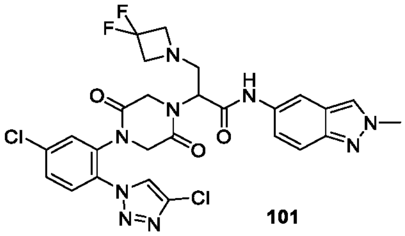 101 | 618.1 [M+H] | δ 10.21 (s, 1H), 8.72 (s, 1H), 8.26 (s, 1H), 8.11 (s, 1H), 7.90 (s, 1H), 7.83-7.63 (m, 2H), 7.55 (d, J = 9.2 Hz, 1H), 7.25 (d, J = 9.3 Hz, 1H), 5.02 (t, J = 7.4 Hz, 1H), 4.75-3.91 (m, 4H), 4.13 (s, 3H), 3.75 (q, J = 11.8 Hz, 2H), 3.63 (q, J = 11.6 Hz, 2H), 3.15-2.90 (m, 2H). |
| 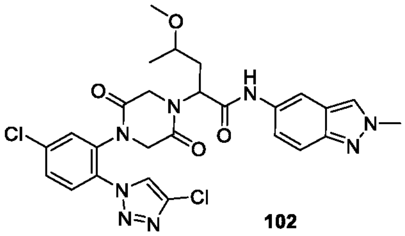 102 | 584.8 [M+H] | δ 10.10 (s, 1H), 8.73 (s, 1H), 8.25 (s, 1H), 8.10 (s, 1H), 7.87 (s, 1H), 7.82-7.68 (m, 2H), 7.60-7.46 (m, 1H), 7.37-7.21 (m, 1H), 5.27-5.11 (m, 1H), 4.68-4.04 (m, 4H), 4.13 (s, 3H), 3.29-3.08 (m, 4H), 2.05-1.82 (m, 2H), 1.15 (d, J = 6.0 Hz, 3H). |
| 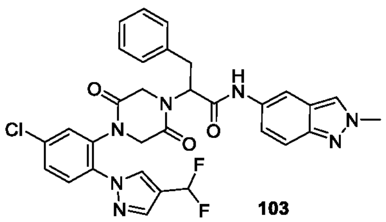 103 | 618.2 [M+H] | δ 10.18 (s, 1H), 8.35 (s, 1H), 8.26 (s, 1H), 8.11 (s, 1H), 7.77-7.51 (m, 5H), 7.37-7.21 (m, 6H), 6.98 (t, J = 52.4 Hz, 1H), 5.46 (dd, J = 10.4, 5.9 Hz, 1H), 4.35-3.90 (m, 4H), 4.14 (s, 3H), 3.35-3.24 (m, 1H), 3.18-3.08 (m, 1H). |
| 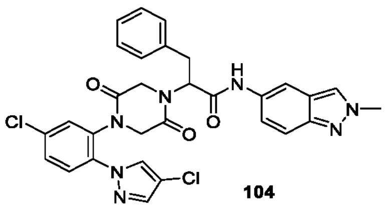 104 | 602.1 [M+H] | δ 10.21 (s, 1H), 8.26 (s, 1H), 8.23 (s, 1H), 8.12 (s, 1H), 7.70-7.42 (m, 5H), 7.42-7.15 (m, 6H), 5.51-5.37 (m, 1H), 4.43-3.91 (m, 4H), 4.13 (s, 3H), 3.17-3.04 (m, 2H). |

Example 105

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-4-yl)propanamide

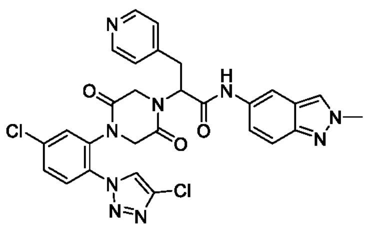

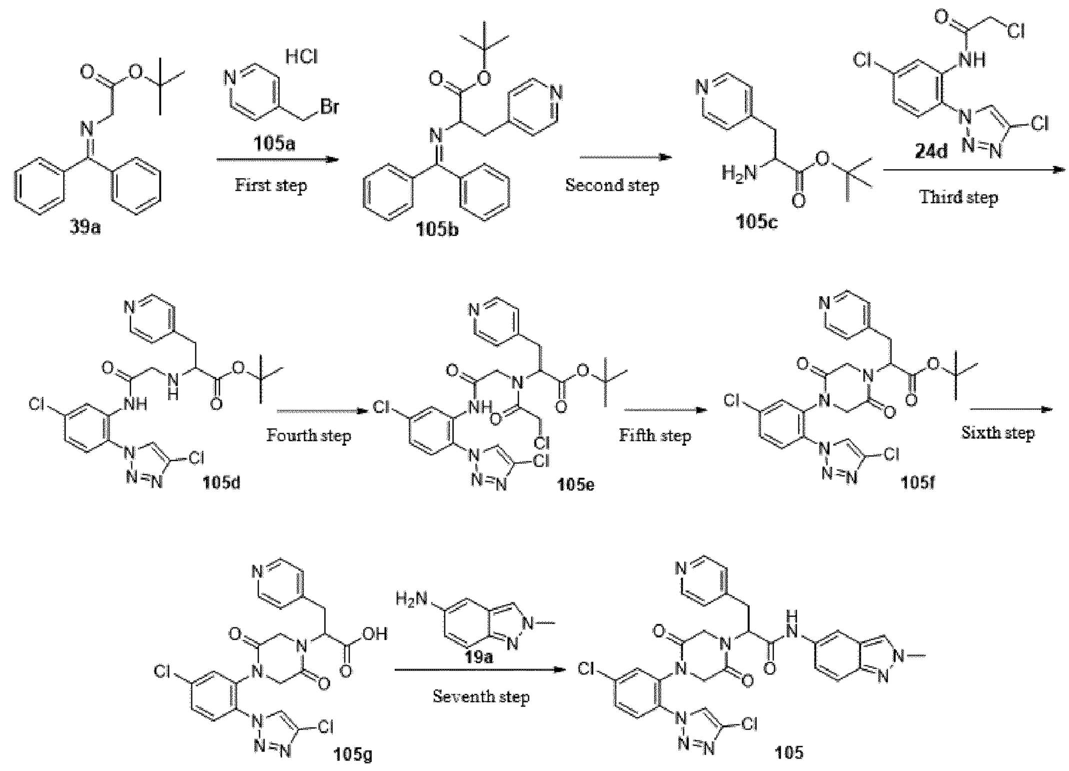


Step 1

*Tert*-butyl 2-((diphenylmethylene)amino)-3-(pyridin-4-yl)propanoate

*Tert*-butyl 2-((diphenylmethylene)amino)acetate 39a (2.81 g, 9.53 mmol) was dissolved in N,N-dimethylformamide (30 mL), added with potassium tert-butoxide (979.90 mg, 8.73 mmol) in batches at 0°C, reacted at 0°C for 15 minutes, then added with 4-(bromomethyl)pyridine hydrogen chloride 105a (2 g, 7.94 mmol), reacted to room temperature, and then reacted for 3 hours. The reaction solution was added with water (30 mL) for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure to obtain crude product of *tert*-butyl 2-((diphenylmethylene)amino)-3-(pyridin-4-yl)propanoate 105b (3.07 g), which was directly used for the next reaction.
MS m/z (ESI): 387.0 [M+H]

Step 2

*Tert*-butyl 2-amino-3-(pyridin-4-yl)propanoate

The crude product of *tert*-butyl 2-((diphenylmethylene)amino)-3-(pyridin-4-yl) propanoate 105b (3.07 g, 7.94 mmol) obtained in the last step was dissolved in tetrahydrofuran (15 mL), added with 2M dilute hydrochloric acid (15 mL), and reacted at room temperature overnight. The reaction solution was dropwise added with saturated sodium bicarbonate solution to adjust the pH to 8, extracted with ethyl acetate (30 mL×3), then organic phases were combined, washed with water (50 mL×3) and saturated sodium chloride solution (50 mL) in turn, dried with anhydrous sodium sulfate, filtered, and concentrated under reduced pressure. The obtained residue was further separated and purified by silicagel column chromatography (eluent: system B) to obtain *tert*-butyl 2-amino-3-(pyridin-4-yl)propanoate105c (190 mg, 1.17 g) with a yield of 66%.
MS m/z (ESI): 223.0 [M+H]

Step 3

*Tert*-butyl 2-((2-((5-chloro-2-(4-chloro-1*H*-1,2,3-triazol-1-yl)phenyl)amino)-2-oxoethyl)amino)-3-(pyridin-4-yl)propanoate

2-Chloro-*N*-(5-chloro-2-(4-chloro-1*H*-1,2,3-triazol-1-yl)phenyl)acetamide 24d (250 mg, 818.20 μmol) and *tert*-butyl 2-amino-3-(pyridin-4-yl)propanoate 105c (272.81 mg, 1.23 mmol) were dissolved in N,N-dimethylformamide (10 mL), added with N,N-diisopropylethylamine (105.74 mg, 818.2 μmol, 135.22 μL), and reacted at 70°C for 5 hours. The reaction solution was added with water (40 mL) for quenching the reaction, extracted with ethyl acetate (40 mL×3), and then washed with water (40 mL) and saturated sodium chloride solution (40 mL) in turn, dried with anhydrous sodium sulfate, filtered, and concentrated under reduced pressure. The obtained residue was further separated and purified by silicagel column chromatography (eluent: system A) to obtain *tert-butyl* 2-((2-((5-chloro-2-(4-chloro-1*H*-1,2,3-triazol-1-yl)phenyl)amino)-2-oxoethyl)amino)-3-(pyridin-4-yl)propanoate 105d (250 mg) with a yield of 62%.
MS m/z (ESI): 490.9 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(pyridin-4-yl)propanoate

*Tert-butyl* 2-((2-((5-chloro-2-(4-chloro-1*H*-1,2,3-triazol-1-yl)phenyl)amino)-2-oxoethyl)amino)-3-(pyridin-4-yl)propanoate 105d (250 mg, 508.78 μmol) was dissolved in dichloromethane (10 mL), added with triethylamine (154.45 mg, 1.53 mmol, 212.16 μL), slowly dropwise added with chloroacetyl chloride (86.20 mg, 763.17 μmol, 60.75 μL) in ice bath, and reacted for 2 hours in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of tert-butyl 2-(2-chloro-*N*-(2-((5-chloro-2-(4-chloro-1*H*-1,2,3-triazol-1-yl)phenyl)amino)-2-oxoethyl)acetamido)-3-(pyridin-4-yl)propanoate 105e (79 mg), which was directly used for the next reaction.
MS m/z (ESI): 567.1 [M+H]

Step 5

*Tert*-butyl 2-(4-(5-chloro-2-(4-chloro-1H-1,2,3-triazole-1-yl)phenyl)-2,5-dioxiperazine-1-yl)-3-(pyridine-4-yl) propionate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(pyridin-4-yl) propanoate 105e (79 mg, 139.12 μmol) obtained in the last step was dissolved in methanol (5 mL), slowly dropwise added with sodium methylate (5.4M methanol solution, 25.76 μL, 139.10 μmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(pyridin-4-yl)propanoate 105f (46 mg) with a yield of 62%.
MS m/z (ESI): 531.2 [M+H]

Step 6

2-(4-(5-Chloro-2-(4-chloro-1*H*-1,2,3-triazol-1-yl)phenyl)-2,5-dioxopiperazin-1-yl)-3-(pyridin-4-yl)propanoic acid

*Tert*-butyl 2-(4-(5-chloro-2-(4-chloro-1*H*-1,2,3-triazol-1-yl)phenyl)-2,5-dioxopiperazin-1-yl)-3-(pyridin-4-yl) propanoate 105f (46 mg, 86.57 μmol) was dissolved in dichloromethane (10 mL), slowly dropwise added with trifluoroacetic acid (1 mL), and reacted at room temperature for 2 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(pyridin-4-yl)propanoic acid 105g (35 mg), which was directly used for the next reaction.
MS m/z (ESI): 475.1 [M+H]

Step 7

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-4-yl)propanamide

The crude product of 2-(4-(5-chloro-2-(4-chloro-1*H*-1,2,3-triazol-1-yl)phenyl)-2,5-dioxopiperazin-1-yl)-3-(pyridin-4-yl)propanoic acid 105g (35 mg, 73.64 μmol) and 2-methyl-2*H*-indazol-5-amine 19a (16.26 mg, 110.46 μmol) were dissolved in ethyl acetate (10 mL), added with propylphosphonic anhydride (50% ethyl acetate solution, 184.5 mg, 294.56 μmol) and N,N-diisopropylethylamine (47.59 mg, 368.20 μmol, 60.86 μL), and reacted at 60°C for 3 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(pyridin-4-yl)propanamide 105 (24 mg) with a yield of 52%.

MS m/z (ESI): 603.8 [M+H]

$^1$H NMR (400 MHz, DMSO-$d_6$) δ 10.15 (s, 1H), 8.66 (s, 1H), 8.50 (d, J = 5.0 Hz, 2H), 8.26 (s, 1H), 8.09 (s, 1H), 7.83-7.67 (m, 3H), 7.55 (d, J= 9.2 Hz, 1H), 7.31 (d, J = 5.0 Hz, 2H), 7.24 (d, J = 9.1 Hz, 1H), 5.52-5.36 (m, 1H), 4.38-3.91 (m, 4H), 4.13 (s, 3H), 3.32-3.24 (m, 1H), 3.21-3.11 (m, 1H).

Example 106

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(*p*-tolyl)propanamide

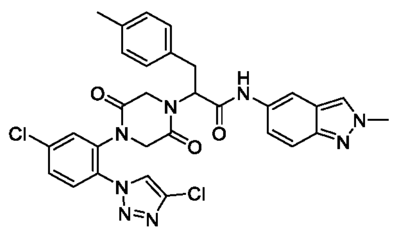

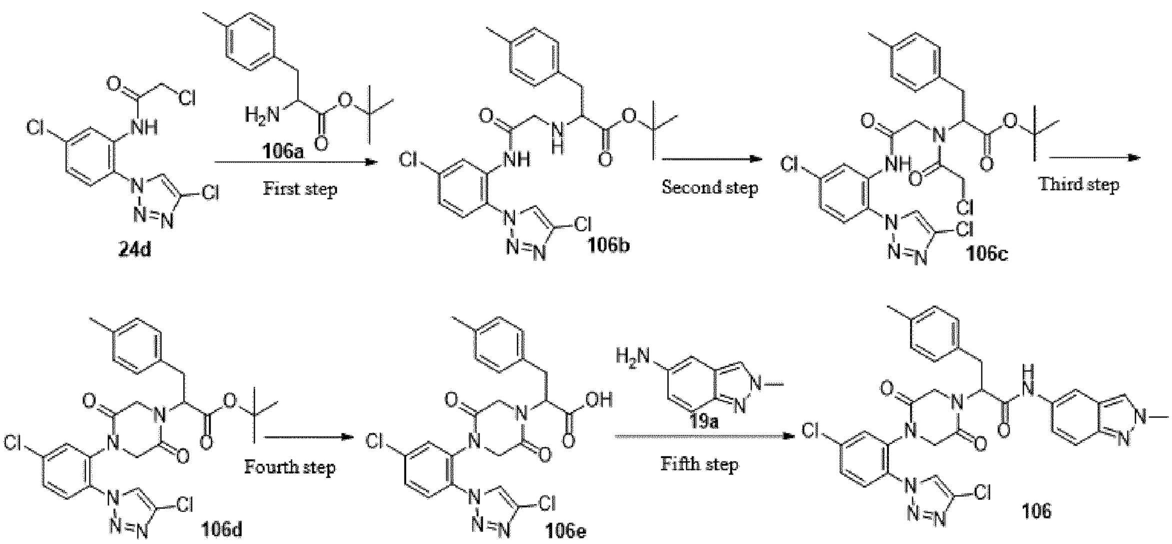

Step 1

*Tert*-butyl 2-((2-((5-chloro-2-(4-chloro-1*H*-1,2,3-triazol-1-yl)phenyl)amino)-2-oxoethyl)amino)-3-(*p*-tolyl) propanoate

2-Chloro-*N*-(5-chloro-2-(4-chloro-1*H*-1,2,3-triazol-1-yl)phenyl)acetamide 24d (300 mg, 981.84 μmol) and *tert*-butyl 2-amino-3-(p-tolyl) propanoate 106a (323.47 mg, 1.37 mmol) were dissolved in N,N-dimethylformamide (10 mL), added with N,N-diisopropylethylamine (126.89 mg, 981.84 μmol), and reacted at 60°C for 7 hours. The reaction solution was added with water (30 mL) for quenching the reaction, and then extracted with ethyl acetate (30 mL×3). Organic phases were combined and then washed with water (50 mL×3) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain tert-butyl 2-((2-((5-chloro-2-(4-chloro-1*H*-1,2,3-triazol-1-yl)phenyl)amino)-2-oxoethyl)amino)-3-(*p*-tolyl)propanoate 106b (200 mg) with a yield of 40%.
MS m/z (ESI): 504.1 [M+H]

Step 2

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(*p*-tolyl)propanoate

*Tert-butyl* 2-((2-((5-chloro-2-(4-chloro-1*H*-1,2,3-triazol-1-yl)phenyl)amino)-2-oxoethyl)amino)-3-(*p*-tolyl)propanoate 106b (200 mg, 396.50 μmol) was dissolved in dichloromethane (5 mL), added with triethylamine (160.49 mg, 1.59 mmol), slowly dropwise added with chloroacetyl chloride (67.17 mg, 594.76 μmol, 47.34 μL) in ice bath, and reacted for 2 hours in ice bath. The reaction solution was added with water (30 mL) for quenching the reaction, and extracted with ethyl acetate (30 mL×3). Organic phases were combined, dried with anhydrous sodium sulfate, filtered, and concentrated under reduced pressure. The obtained residue was further separated and purified by silicagel column chromatography (eluent: system A) to obtain *tert*-butyl 2-(2-chloro-*N*-(2-((5-chloro-2-(4-chloro-1H 1,2,3-triazol-1-yl)phenyl)amino)-2-oxoethyl)acetamido)-3-(*p*-tolyl) propanoate 106c (230 mg) with a yield of 99.9%.
MS m/z (ESI): 523.8 [M+H-56]

Step 3

*Tert*-butyl 2-(4-(5-chloro-2-(4-chloro-1*H*-1,2,3-triazol-1-yl)phenyl)-2,5-dioxopiperazin-1-yl)-3-(*p*-tolyl)propanoate

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(*p*-tolyl) propanoate 106c (230 mg, 395.94 μmol) was dissolved in methanol (10 mL), slowly dropwise added with sodium methylate (5.4M methanol solution, 73.32 μL, 395.93 μmol) in ice bath, and reacted in ice bath for 2 hours. The reaction solution was dropwise added with 2M dilute hydrochloric acid to adjust the pH to 6, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(*p*-tolyl) propanoate 106d (129 mg) with a yield of 60%.
MS m/z (ESI): 487.8 [M+H-56]

Step 4

2-(4-(5-Chloro-2-(4-chloro-1*H*-1,2,3-triazol-1-yl)phenyl)-2,5-dioxopiperazin-1-yl)-3-(*p*-tolyl)propanoic acid

*Tert*-butyl 2-(4-(5-chloro-2-(4-chloro-1*H*-1,2,3-triazol-1-yl)phenyl)-2,5-dioxopiperazin-1-yl)-3-(*p*-tolyl) propanoate 106d (129 mg, 236.95 μmol) was dissolved in dichloromethane (10 mL), added with trifluoroacetic acid (1 mL), and reacted at room temperature for 5 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(*p*-tolyl)propanoic acid 106e (85 mg), which was directly used for the next reaction.
MS m/z (ESI): 487.8 [M+H]

Step 5

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(*p*-tolyl)propanamide

2-(4-(5-chloro-2-(4-chloro-1*H*-1,2,3-triazol-1-yl)phenyl)-2,5-dioxopiperazin-1-yl)-3-(*p*-tolyl)propanoic acid 106e (85 mg, 174.07 μmol) and 2-methyl-2*H*-indazol-5-amine 19a (38.43 mg, 261.10 μmol) were dissolved in ethyl acetate (10 mL), added with propylphosphonic anhydride (50% ethyl acetate solution, 332.31 mg, 522.20 μmol) and N,N-diisopropylethylamine (112.48 mg, 870.33 μmol, 143.84 μL), and reacted at 60°C for 4 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(*p*-tolyl)propanamide 106 (43 mg) with a yield of 40%.

MS m/z (ESI): 616.8 [M+H]

$^1$H NMR (400 MHz, DMSO-$d_6$) δ 10.12 (s, 1H), 8.64 (s, 1H), 8.26 (s, 1H), 8.11 (s, 1H), 7.80-7.68 (m, 2H), 7.54 (d, J = 9.2 Hz, 1H), 7.31-7.04 (m, 6H), 5.44-5.28 (m, 1H), 4.33-3.94 (m, 4H), 4.13 (s, 3H), 3.27-3.15 (m, 1H), 3.11-2.99 (m, 1H), 2.28 (s, 3H).

Example 107

2-(4-(5-Chloro-2-(4-chloro-1*H*-1,2,3-triazol-1-yl)phenyl)-2,5-dioxopiperazin-1-yl)-3-(4-chlorophenyl)-*N*-(2-methyl-2*H*-indazol-5-yl)propanamide

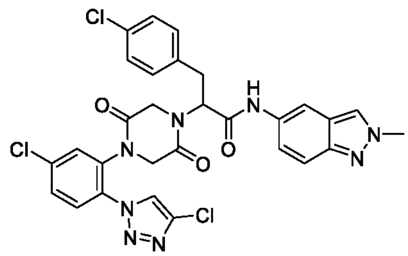

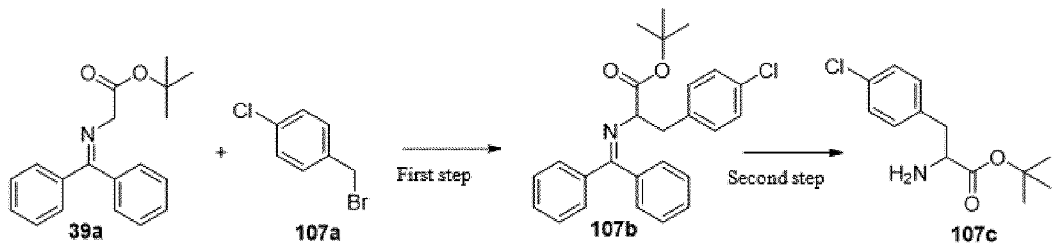


39a 107a 107b 107c

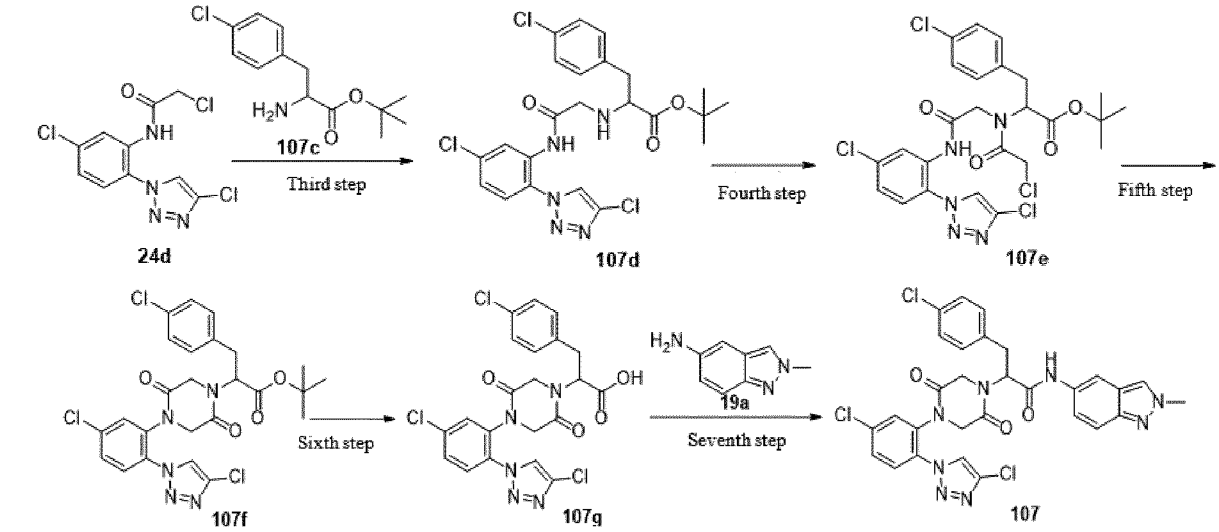


Step 1

*Tert*-butyl 3-(4-chlorophenyl)-2-((diphenylmethylene)amino) propanoate

*Tert*-butyl 2-((diphenylmethylene)amino)acetate 39a (4.31 g, 14.60 mmol) was dissolved in N,N-dimethylformamide (30 mL), added with potassium tert-butoxide (1.64 g, 14.60 mmol) in batches at 0°C, reacted at 0°C for 30 minutes, then added with 1-(bromomethyl)-4-chlorobenzene 107a (2 g, 9.73 mmol), heated to room temperature, and then reacted for 3 hours. The reaction solution was added with water (30 mL) for quenching the reaction, and then extracted with ethyl acetate (30 mL×3). Organic phases were combined, washed with water (50 mL×3) and saturated sodium chloride solution (50 mL) in turn, dried with anhydrous sodium sulfate, filtered and concentrated under reduced pressure to obtain crude product of *tert*-butyl 3-(4-chlorophenyl)-2-((diphenylmethylene)amino)propanoate 107b (4.09 g), which was directly used for the next reaction.
MS m/z (ESI): 420.1 [M+H]

Step 2

*Tert*-butyl 2-amino-3-(4-chlorophenyl) propanoate

The crude product of *tert*-butyl 3-(4-chlorophenyl)-2-((diphenylmethylene)amino) propanoate 107b (4.09, 9.74 mmol) obtained in the last step was dissolved in tetrahydrofuran (10 mL), added with 2M dilute hydrochloric acid (10 mL), and reacted at room temperature overnight. The reaction solution was dropwise added with saturated sodium bicarbonate solution to adjust the pH to 8, extracted with ethyl acetate (30 mL×3), then organic phases were combined, washed with water (50 mL×3) and saturated sodium chloride solution (50 mL) in turn, dried with anhydrous sodium sulfate, filtered, and concentrated under reduced pressure. The obtained residue was further separated and purified by silicagel column chromatography (eluent: system B) to obtain *tert*-butyl 2-amino-3-(4-chlorophenyl)propanoate 107c (2 g) with a yield of 80%.
MS m/z (ESI): 200.1 [M+H-56]

Step 3

*Tert*-butyl 2-((2-((5-chloro-2-(4-chloro-1*H*-1,2,3-triazol-1-yl)phenyl)amino)-2-oxoethyl)amino)-3-(4-chlorophenyl)propanoate

2-chloro-N-(5-chloro-2-(4-chloro-1H-1,2,3-triazole-1-yl)phenyl)acetamide 24d (300 mg, 981.84 μmol) and *tert*-butyl 2-amino-3-(4-chlorophenyl)propanoate 107c (376.64 mg, 1.47 mmol) were dissolved in N,N-dimethylformamide (10 mL), added with N,N-diisopropylethylamine (380.68 mg, 2.95 mmol, 486.8 μL), and reacted at 70°C for 5 hours. The reaction solution was added with water (40 mL) for quenching the reaction, extracted with ethyl acetate (40 mL×3), and then washed with water (40 mL) and saturated sodium chloride solution (40 mL), dried with anhydrous sodium sulfate, filtered, and concentrated under reduced pressure. The obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(4-chlorophenyl) propanoate 107d (420 mg) with a yield of 82%.
MS m/z (ESI): 524.1 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(4-chlorophenyl) propanoate

*Tert*-butyl 2-((2-((5-chloro-2-(4-chloro-1*H*-1,2,3-triazol-1-yl)phenyl)amino)-2-oxoethyl)amino)-3-(4-chlorophenyl) propanoate 107d (420 mg, 800.26 μmol) was dissolved in dichloromethane (10 mL), added with triethylamine (161.96 mg, 1.60 mmol, 222.47 μL), slowly dropwise added with chloroacetyl chloride (135.58 mg, 1.20 mmol, 95.55 μL) in ice bath, and reacted for 2 hours in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(4-chlorophenyl)propanoate 107e (430 mg), which was directly used for the next reaction.
MS m/z (ESI): 543.7 [M+H-56]

Step 5

*Tert*-butyl 2-(4-(5-chloro-2-(4-chloro-1*H*-1,2,3-triazol-1-yl)phenyl)-2,5-dioxopiperazin-1-yl)-3-(4-chlorophenyl) propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(4-chlorophenyl) propanoate 107e (430 mg, 715.11 μmol) obtained in the last step was dissolved in methanol (10 mL), slowly dropwise added with sodium methylate (5.4M methanol solution, 132.43 μL, 715.12 μmol) at 0°C, and continuously reacted at 0°C for 2 hours. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(4-chlorophenyl) propanoate 107f (250 mg) with a yield of 62%.
MS m/z (ESI): 508.7 [M+H-56]

Step 6

2-(4-(5-chloro-2-(4-chloro-1*H*-1,2,3-triazol-1-yl)phenyl)-2,5-dioxopiperazin-1-yl)-3-(4-chlorophenyl)propanoic acid

*Tert*-butyl 2-(4-(5-chloro-2-(4-chloro-1*H*-1,2,3-triazol-1-yl)phenyl)-2,5-dioxopiperazin-1-yl)-3-(4-chlorophenyl) propanoate 107f (250 mg, 442.60 μmol) was dissolved in dichloromethane (10 mL), slowly dropwise added with trifluoroacetic acid (1 mL), and reacted at room temperature for 19 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(4-chlorophenyl)propanoic acid 107g (200 mg), which was directly used for the next reaction. MS m/z (ESI): 507.7 [M+H]

Step 7

2-(4-(5-chloro-2-(4-chloro-1*H*-1,2,3-triazol-1-yl)phenyl)-2,5-dioxopiperazin-1-yl)-3-(4-chlorophenyl)-*N*-(2-methyl-2*H*-indazol-5-yl)propanamide

The crude product of 2-(4-(5-chloro-2-(4-chloro-1*H*-1,2,3-triazol-1-yl)phenyl)-2,5-dioxopiperazin-1-yl)-3-(4-chlorophenyl)propanoic acid 107g (200 mg, 393.13 μmol) obtained in the last step and 2-methyl-2*H*-indazol-5-amine 19a (86.79 mg, 589.69 μmol) were dissolved in ethyl acetate (10 mL), added with propylphosphonic anhydride (50% ethyl acetate solution, 750.9 mg, 1.18 mmol) and N,N-diisopropylethylamine (254.04 mg, 1.97 mmol, 325.58 μL), and reacted at 60°C for 5 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(4-chlorophenyl)-*N*-(2-methyl-2*H*-indazol-5-yl)propanamide 107 (157 mg) with a yield of 61%. MS m/z (ESI): 636.7 [M+H]
$^1$H NMR (400 MHz, DMSO-$d_6$) δ 10.14 (s, 1H), 8.66 (s, 1H), 8.26 (s, 1H), 8.11 (s, 1H), 7.84-7.63 (m, 3H), 7.55 (d, J = 9.2 Hz, 1H), 7.44-7.18 (m, 5H), 5.44-5.30 (m, 1H), 4.39-3.94 (m, 4H), 4.14 (s, 3H), 3.32-3.20 (m, 1H), 3.20-3.03 (m, 1H).

Example 108

2-(4-(5-chloro-2-(4-chloro-1*H*-1,2,3-triazol-1-yl)phenyl)-2,5-dioxopiperazin-1-yl)-3-(4-methoxyphenyl)-*N*-(2-methyl-2*H*-indazol-5-yl)propanamide

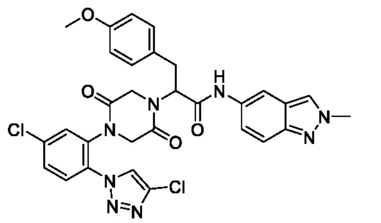

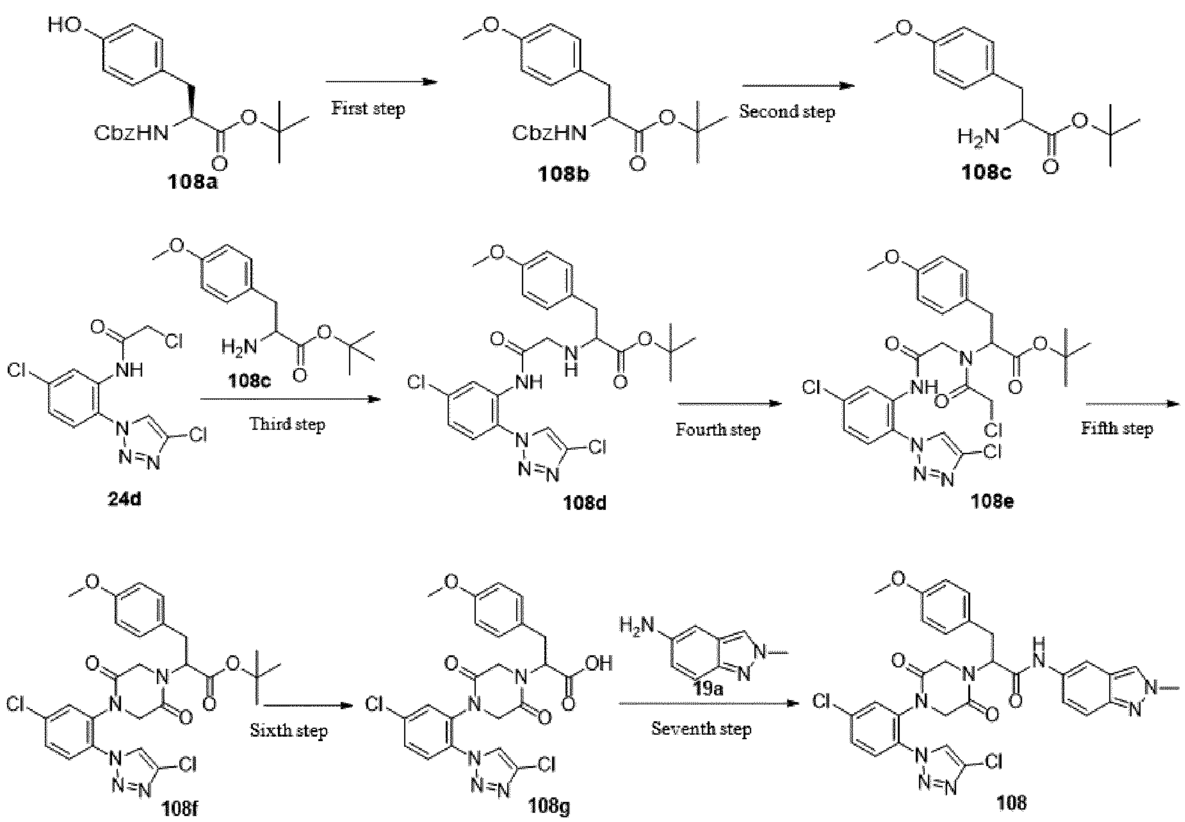


Step 1

*Tert*-butyl 2-(((benzyloxy)carbonyl)amino)-3-(4-methoxyphenyl) propanoate

*Tert*-butyl ((benzyloxy)carbonyl)-L-tyrosinate 108a (2 g, 5.38 mmol) was dissolved in N,N-dimethylformamide (20 mL), added with potassium carbonate (1.49 g, 10.77 mmol), then added with methyl iodide (1.15 g, 8.08 mmol), and reacted at 30°C overnight. The reaction solution was added with water (50 mL), and extracted with ethyl acetate (50 mL×3). Organic phases were combined, washed with water (50 mL) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain tert-butyl 2-(((benzyloxy)carbonyl)amino)-3-(4-methoxyphenyl) propanoate 108b (2 g) with a yield of 96%.
MS m/z (ESI): 330.0 [M+H-56]

Step 2

*Tert*-butyl 2-amino-3-(4-methoxyphenyl) propanoate

Tert-butyl 2-(((benzyloxy)carbonyl)amino)-3-(4-methoxyphenyl)propanoate 108b (2 g, 5.19 mmol) was dissolved in methanol (50 mL), added with 10% palladium carbon (200 mg, 1.88 mmol, containing about 50% of water), and reacted overnight in hydrogen atmosphere. The reaction solution was filtered and concentrated under reduced pressure, and the obtained residue was further separated and purified by silicagel column chromatography (eluent: system B) to obtain *tert*-butyl 2-amino-3-(4-methoxyphenyl)propanoate 108c (1.23 g) with a yield of 94%.
MS m/z (ESI): 252.0 [M+H]

Step 3

*Tert*-butyl 2-((2-((5-chloro-2-(4-chloro-1*H*-1,2,3-triazol-1-yl)phenyl)amino)-2-oxoethyl)amino)-3-(4-methoxyphenyl) propanoate

2-chloro-*N*-(5-chloro-2-(4-chloro-1*H*-1,2,3-triazol-1-yl)phenyl)acetamide 24d (200 mg, 654.56 μmol) and *tert*-butyl 2-amino-3-(4-methoxyphenyl) propanoate 108c (246.76 mg, 981.84 μmol) were dissolved in N,N-dimethylformamide (10 mL), added with N,N-diisopropylethylamine (422.98 mg, 3.27 mmol, 540.89 μL), and reacted at 70°C overnight. The reaction solution was added with water (50 mL) for quenching the reaction, and then extracted with ethyl acetate (50 mL×3). Organic phases were combined and then washed with water (50 mL) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(4-methoxyphenyl) propanoate 108d (310 mg) with a yield of 91%.
MS m/z (ESI): 519.8 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(4-methoxyphenyl)propanoate

*Tert*-butyl 2-((2-((5-chloro-2-(4-chloro-1*H*-1,2,3-triazol-1-yl)phenyl)amino)-2-oxoethyl)amino)-3-(4-methoxyphenyl)propanoate 108d (310 mg, 595.69 μmol) was dissolved in dichloromethane (10 mL), added with triethylamine (301.39 mg, 2.98 mmol, 413.99 μL), slowly dropwise added with chloroacetyl chloride (80.73 mg, 714.82 μmol, 56.86 μL) in ice bath, and reacted for 1 hour in ice bath. The reaction solution was added with water (20 mL) for quenching the reaction, and then extracted with dichloromethane (20 mL×3). Organic phases were combined, washed with saturated sodium chloride solution (50 mL), dried with anhydrous sodium sulfate, filtered and concentrated under reduced pressure to obtain crude product of *tert*-butyl 2-(2-chloro-N-(2-((5-chloro-2-(4-chloro-1*H*-1,2,3-triazol-1-yl)phenyl)amino)-2-oxoethyl)acetamido)-3-(4-methoxyphenyl)propanoate 108e (355 mg), which was directly used for the next reaction.
MS m/z (ESI): 595.7 [M+H]

Step 5

*Tert*-butyl 2-(4-(5-chloro-2-(4-chloro-1*H*-1,2,3-triazol-1-yl)phenyl)-2,5-dioxopiperazin-1-yl)-3-(4-methoxyphenyl) propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(4-methoxyphenyl) propanoate 108e (355 mg, 594.75 μmol) was dissolved in methanol (10 mL), slowly dropwise added with sodium methylate (5.4M methanol solution, 121.15 μL, 654.21 μmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(4-methoxyphenyl) propanoate 108f (230 mg) with a yield of 69%.
MS m/z (ESI): 560.1 [M+H]

Step 6

2-(4-(5-chloro-2-(4-chloro-1*H*-1,2,3-triazol-1-yl)phenyl)-2,5-dioxopiperazin-1-yl)-3-(4-methoxyphenyl)propanoic acid

Tert-butyl 2-(4-(5-chloro-2-(4-chloro-1*H*-1,2,3-triazol-1-yl)phenyl)-2,5-dioxopiperazin-1-yl)-3-(4-methoxyphenyl) propanoate 108f (230 mg, 410.40 μmol) was dissolved in dichloromethane (10 mL), slowly dropwise added with trifluoroacetic acid (1 mL), and reacted at room temperature for 19 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(4-methoxyphenyl)propanoic acid 108g (206 mg), which was directly used for the next reaction.
MS m/z (ESI): 504.1 [M+H]

Step 7

2-(4-(5-chloro-2-(4-chloro-1*H*-1,2,3-triazol-1-yl)phenyl)-2,5-dioxopiperazin-1-yl)-3-(4-methoxyphenyl)-*N*-(2-methyl-2*H*-indazol-5-yl)propanamide

The crude product of 2-(4-(5-chloro-2-(4-chloro-1*H*-1,2,3-triazol-1-yl)phenyl)-2,5-dioxopiperazin-1-yl)-3-(4-methoxyphenyl)propanoic acid 108g (206 mg, 408.47 μmol) obtained in the last step and 2-methyl-2*H*-indazol-5-amine 19a (90.18 mg, 612.70 μmol) were dissolved in ethyl acetate (10 mL), added with propylphosphonic anhydride (50% ethyl acetate solution, 519.87 mg, 816.94 μmol) and N,N-diisopropylethylamine (211.16 mg, 1.63 mmol, 270.03 μL), and reacted at 60°C for 1 hour. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(4-methoxyphenyl)-*N*-(2-methyl-2*H*-indazol-5-yl)propanamide 108 (130 mg) with a yield of 48%.

MS m/z (ESI): 633.2 [M+H]
$^1$H NMR (400 MHz, DMSO-$d_6$) δ 10.13 (s, 1H), 8.65 (s, 1H), 8.26 (s, 1H), 8.12 (s, 1H), 7.80-7.61 (m, 3H), 7.55 (d, J = 9.2 Hz, 1H), 7.31-7.13 (m, 3H), 6.88 (d, J = 8.1 Hz, 2H), 5.43-5.25 (m, 1H), 4.36-3.94 (m, 4H), 4.13 (s, 3H), 3.74 (s, 3H), 3.25-3.15 (m, 1H), 3.09-2.97 (m, 1H).

Example 109

2-(4-(5-Chloro-2-(4-chloro-1*H*-1,2,3-triazol-1-yl)phenyl)-2,5-dioxopiperazin-1-yl)-*N*-(2-(methyl-*d*$_3$)-2*H*-indazol-5-yl)-3-phenylpropanamide

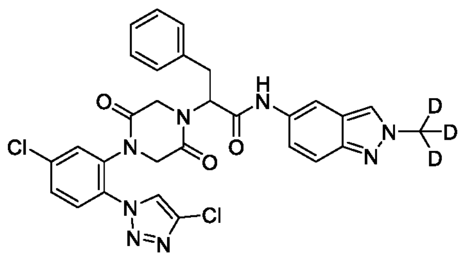

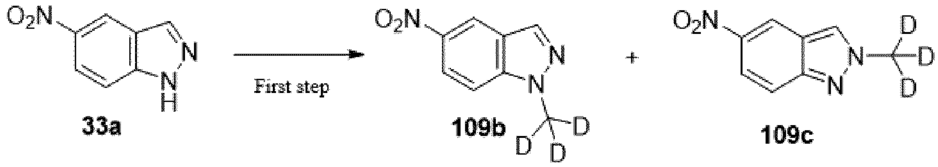


33a 109b 109c

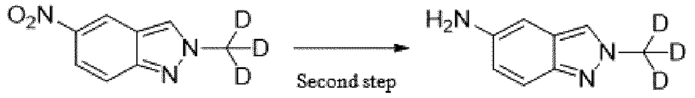


109c 109d

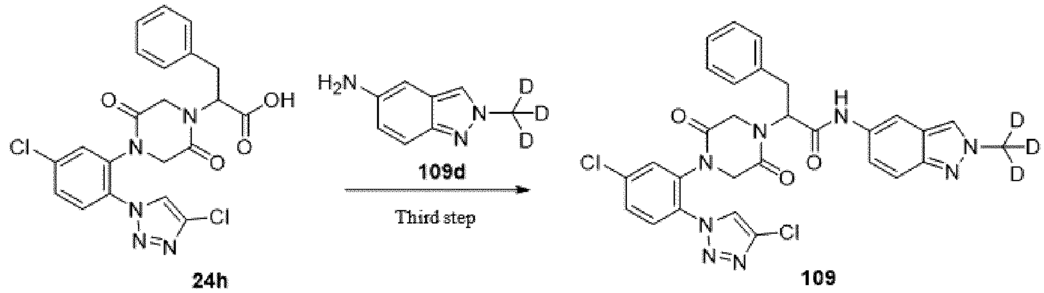


24h 109

Step 1

1-(Methyl-$d_3$)-5-nitro-1*H*-indazole 109b

2-(Methyl-$d_3$)-5-nitro-2*H*-indazole 109c

5-Nitroindazole 33a (1 g, 6.13 mmol) was dissolved in acetonitrile (30 mL), added with potassium carbonate (2.54 g, 18.39 mmol), then slowly dropwise added with iodomethane-$d_3$ (1.33 g, 9.19 mmol, 572.06 μL), and reacted at room temperature overnight. The reaction solution was filtered and concentrated under reduced pressure. The obtained residue was further separated and purified by silicagel column chromatography (eluent: system A) to obtain 1-(methyl-$d_3$)-5-nitro-1*H*-indazole 109b (359 mg) with a yield of 33%; and 2-(methyl-$d_3$)-5-nitro-2*H*-indazole 109c (359 mg) with a yield of 33%.

**109b** MS m/z (ESI): 181.1 [M+H]
**109c** MS m/z (ESI): 181.1 [M+H]

Step 2

2-(Methyl-$d_3$)-2*H*-indazol-5-amine

2-(Methyl-$d_3$)-5-nitro-2*H*-indazole 109c (359 mg, 1.99 mmol) was dissolved in mixed solvent of methanol (8 mL) and water (2 mL), added with iron powder (556.40 mg, 9.96 mmol) and ammonium chloride (213.16 mg, 3.98 mmol), and reacted at 80°C for 2 hours. After the reaction was completed, the reaction solution was filtered and concentrated under reduced pressure. The obtained residue was further separated and purified by silicagel column chromatography (eluent: system A) to obtain 2-(methyl-$d_3$)-2*H*-indazol-5-amine 109d (154 mg) with a yield of 52%.
MS m/z (ESI): 151.1 [M+H]

Step 3

2-(4-(5-Chloro-2-(4-chloro-1*H*-1,2,3-triazol-1-yl)phenyl)-2,5-dioxopiperazin-1-yl)-*N*-(2-(methyl-$d_3$)-2*H*-indazol-5-yl)-3-phenylpropanamide

2-(4-(5-Chloro-2-(4-chloro-1*H*-1,2,3-triazol-1-yl)phenyl)-2,5-dioxopiperazin-1-yl)-3-phenylpropanoic acid 24h (89 mg, 187.65 μmol) and 2-(methyl-$d_3$)-2*H*-indazol-5-amine 109d (42.28 mg, 281.47 μmol) were dissolved in ethyl acetate (5 mL), added with propylphosphonic anhydride (50% ethyl acetate solution, 238.82 mg, 375.29 μmol) and N,N-diisopropylethylamine (97.01 mg, 750.59 μmol, 124.05 μL), and reacted at 60°C for 1 hour. After the reaction was completed, the reaction solution was filtered and concentrated under reduced pressure. The obtained residue was further separated and purified by silicagel column chromatography (eluent: system A) to obtain 2-(4-(5-chloro-2-(4-chloro-1*H*-1,2,3-triazol-1-yl)phenyl)-2,5-dioxopiperazin-1-yl)-*N*-(2-(methyl-$d_3$)-2*H*-indazol-5-yl)-3-phenylpropanamide 109 (50 mg) with a yield of 44%.

MS m/z (ESI): 606.2 [M+H]

$^1$H NMR (400 MHz, DMSO-$d_6$) $\delta$ 10.14 (s, 1H), 8.65 (s, 1H), 8.26 (s, 1H), 8.12 (s, 1H), 7.81-7.58 (m, 3H), 7.54 (d, J = 9.2 Hz, 1H), 7.41-7.13 (m, 6H), 5.45-5.34 (m, 1H), 4.40-3.88 (m, 4H), 3.34-3.19 (m, 1H), 3.16-3.01 (m, 1H).

Example 110

2-(4-(5-chloro-2-(4-chloro-1*H*-1,2,3-triazol-1-yl)phenyl)-2,5-dioxopiperazin-1-yl)-*N*-(2-(difluoromethyl-2*H*-indazol-5-yl)-3-(4-fluorophenyl)propanamide

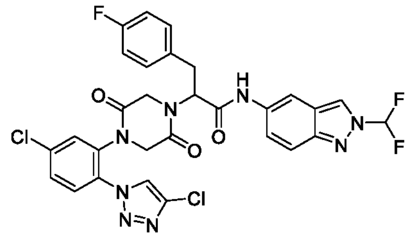

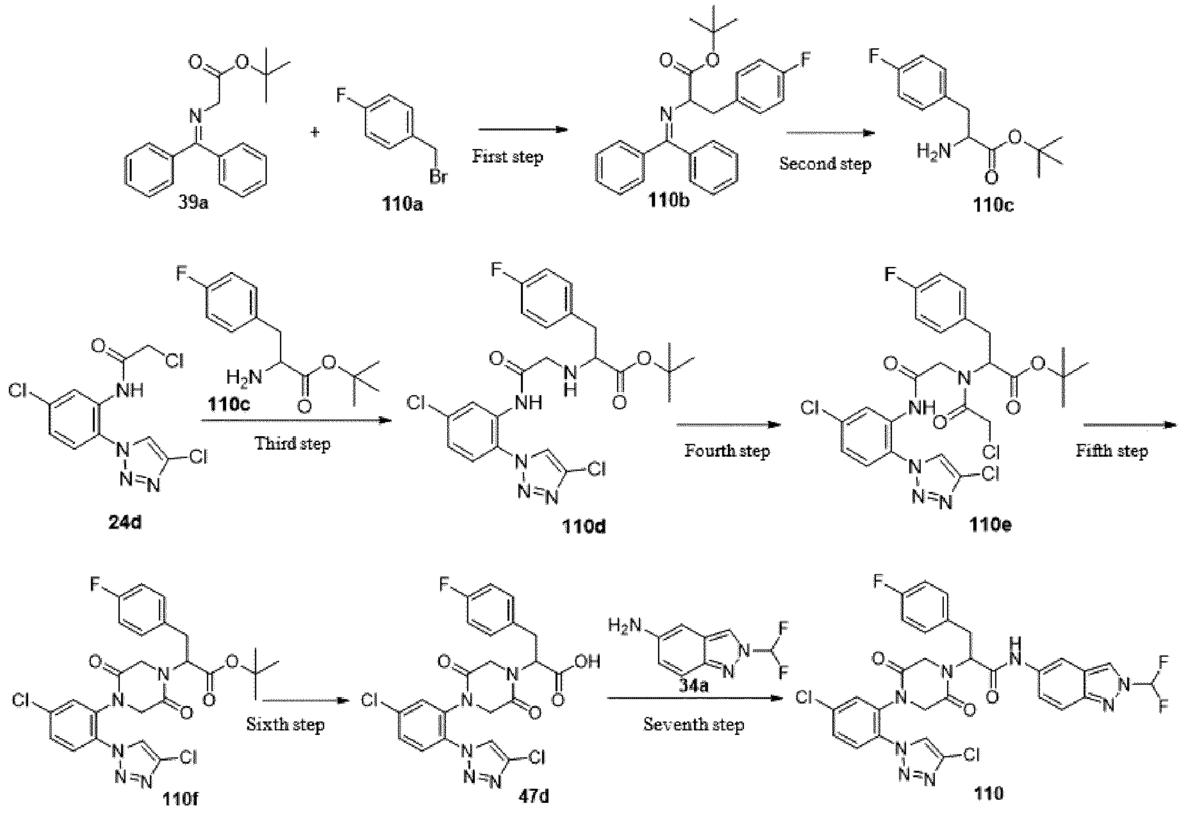


Step 1

*Tert*-butyl 2-((diphenylmethylene)amino)-3-(4-fluorophenyl) propanoate

*Tert*-butyl 2-((diphenylmethylene)amino)acetate 39a (7.50 g, 25.39 mmol) was dissolved in N,N-dimethylformamide (50 mL), added with potassium tert-butoxide (2.85 g, 25.39 mmol) in batches at 0°C, reacted at 0°C for 20 minutes, then added with 1-(bromomethyl)-4-fluorobenzene 110a (3.2 g, 16.93 mmol), reacted and the temperature was increased to room temperature, and then reacted for 3 hours. The reaction solution was added with water (50 mL) for quenching the reaction, and then extracted with ethyl acetate (50 mL×3). Organic phases were combined, washed with water (50 mL×3) and saturated sodium chloride solution (50 mL) in turn, dried with anhydrous sodium sulfate, filtered and concentrated under reduced pressure to obtain crude product of *tert*-butyl 2-((diphenylmethylene)amino)-3-(4-fluorophenyl) propanoate 110b (6.83 g), which was directly used for the next reaction.
MS m/z (ESI): 404.0 [M+H]

Step 2

*Tert*-butyl 2-amino-3-(4-fluorophenyl) propanoate

The crude product of tert-butyl 2-((diphenylmethylene)amino)-3-(4-fluorophenyl) propanoate 110b (6.83 g, 16.93 mmol) obtained in the last step was dissolved in tetrahydrofuran (20 mL), added with 2M dilute hydrochloric acid (20 mL), and reacted at room temperature overnight. The reaction solution was dropwise added with saturated sodium bicarbonate solution to adjust the pH to 8, extracted with ethyl acetate (30 mL×3), then organic phases were combined, washed with water (50 mL×3) and saturated sodium chloride solution (50 mL) in turn, dried with anhydrous sodium sulfate, filtered, and concentrated under reduced pressure. The obtained residue was further separated and purified by silicagel column chromatography (eluent: system B) to obtain *tert*-butyl 2-amino-3-(4-fluorophenyl) propanoate 110c (4 g) with a yield of 99%.
MS m/z (ESI): 184.1 [M+H-56]

Step 3

*Tert*-butyl 2-((2-((5-chloro-2-(4-chloro-1*H*-1,2,3-triazol-1-yl)phenyl)amino)-2-oxoethyl)amino)-3-(4-fluorophenyl)propanoate

2-chloro-*N*-(5-chloro-2-(4-chloro-1*H*-1,2,3-triazol-1-yl)phenyl)acetamide 24d (2.5 g, 8.18 mmol) and tert-butyl 2-amino-3-(4-fluorophenyl)propanoate 110c (2.94 g, 12.27 mmol) were dissolved in N,N-dimethylformamide (40 mL), added with N,N-diisopropylethylamine (3.17 g, 24.55 mmol, 4.05 mL), and reacted at 70°C for 6 hours. The reaction solution was added with water (40 mL) for quenching the reaction, extracted with ethyl acetate (40 mL×3). Organic phases were combined, and then washed with water (40 mL) and saturated sodium chloride solution (40 mL) in turn, dried with anhydrous sodium sulfate, filtered, and concentrated under reduced pressure. The obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(4-fluorophenyl) propanoate 110d (4.02 g) with a yield of 97%.
MS m/z (ESI): 507.9 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(4-fluorophenyl) propanoate

*Tert*-butyl 2-((2-((5-chloro-2-(4-chloro-1*H*-1,2,3-triazol-1-yl)phenyl)amino)-2-oxoethyl)amino)-3-(4-fluorophenyl) propanoate 110d (4.02 g, 7.91 mmol) was dissolved in dichloromethane (20 mL), added with triethylamine (1.60 g, 15.82 mmol, 2.22 mL), slowly dropwise added with chloroacetyl chloride (1.34 g, 11.86 mmol, 950.11 μL) in ice bath, and reacted for 2 hours in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(4-fluorophenyl) propanoate 110e (4.62 g), which was directly used for the next reaction.
MS m/z (ESI): 528.8 [M+H-56]

Step 5

*Tert*-butyl 2-(4-(5-chloro-2-(4-chloro-1*H*-1,2,3-triazol-1-yl)phenyl)-2,5-dioxopiperazin-1-yl)-3-(4-fluorophenyl)propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(4-fluorophenyl)propanoate 110e (4.62 g, 7.90 mmol) obtained in the last step was dissolved in methanol (20 mL), slowly dropwise added with sodium methylate (5.4M methanol solution, 1.46 mL, 7.90 mmol) at 0°C, and continuously reacted at 0°C for 2 hours. The reaction solution was dropwise added with 2M dilute hydrochloric acid to adjust the pH to 7, and concentrated under reduced pressure. The obtained residue was further separated and purified

by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(4-fluorophenyl)propanoate 110f (2.6 g) with a yield of 60%.
MS m/z (ESI): 492.1 [M+H-56]

Step 6

2-(4-(5-chloro-2-(4-chloro-1*H*-1,2,3-triazol-1-yl)phenyl)-2,5-dioxopiperazin-1-yl)-3-(4-fluorophenyl)propanoic acid

*Tert*-butyl 2-(4-(5-chloro-2-(4-chloro-1*H*-1,2,3-triazol-1-yl)phenyl)-2,5-dioxopiperazin-1-yl)-3-(4-fluorophenyl)propanoate 110f (2.6 g, 4.74 mmol) was dissolved in dichloromethane (30 mL), slowly dropwise added with trifluoroacetic acid (3 mL), and reacted at room temperature for 20 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(4-fluorophenyl)propanoic acid 47d (2.25 mg), which was directly used for the next reaction. MS m/z (ESI): 492.1 [M+H]

Step 7

2-(4-(5-chloro-2-(4-chloro-1*H*-1,2,3-triazol-1-yl)phenyl)-2,5-dioxopiperazin-1-yl)-*N*-(2-(difluoromethyl-2*H*-indazol-5-yl)-3-(4-fluorophenyl)propanamide

2-(4-(5-chloro-2-(4-chloro-1*H*-1,2,3-triazol-1-yl)phenyl)-2,5-dioxopiperazin-1-yl)-3-(4-fluorophenyl)propanoic acid 47d (100 mg, 203.13 μmol) and 2-(difluoromethyl)-2*H*-indazol-5-amine 34a (55.81 mg, 304.70 μmol) were dissolved in ethyl acetate (10 mL), added with propylphosphonic anhydride (50% ethyl acetate solution, 517.06 mg, 812.53 μmol) and N,N-diisopropylethylamine (131.26 mg, 1.02 mmol, 167.85 μL), and reacted at 60°C for 2 hours. After the reaction was completed, the reaction solution was filtered and concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-N-(2-(difluoromethyl-2H indazol-5-yl)-3-(4-fluorophenyl)propanamide 110 (58 mg) with a yield of 72%.

MS m/z (ESI): 657.1 [M+H]
$^1$H NMR (400 MHz, DMSO-$d_6$) δ 10.27 (s, 1H), 8.81 (s, 1H), 8.66 (s, 1H), 8.24 (s, 1H), 8.09 (t, J = 59.1 Hz, 1H), 7.83-7.63 (m, 4H), 7.44-7.26 (m, 3H), 7.20-7.07 (m, 2H), 5.44-5.31 (m, 1H), 4.54-3.92 (m, 4H), 3.30-3.21 (m, 1H), 3.18-3.07 (m, 1H).

Example 111

2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)-*N*-(2-methyl-2*H*-indazol-5-yl)propanamide

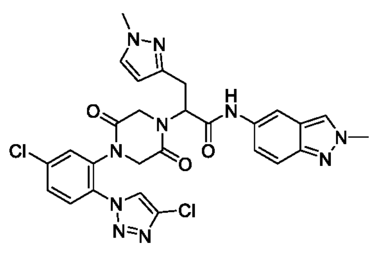

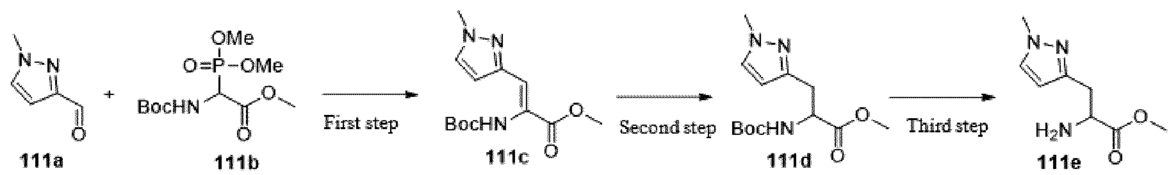


111a 111b 111c 111d 111e

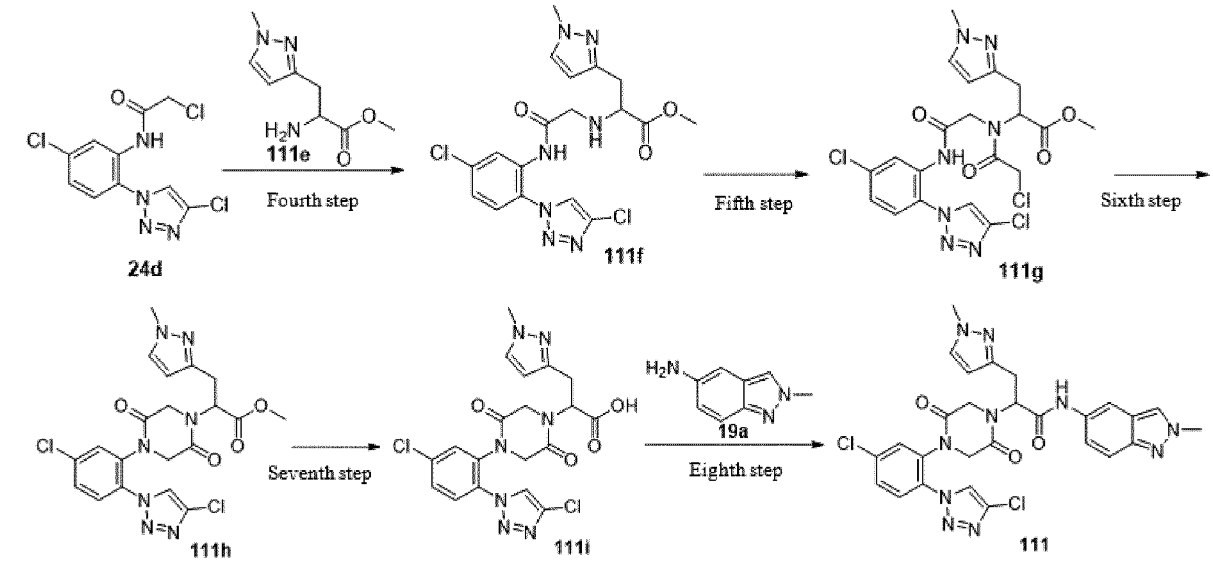


24d 111e 111f 111g 111h 111i 19a 111

Step 1

Methyl (Z)-2-((tert-butoxycarbonyl)amino)-3-(1-methyl-1*H*-pyrazol-3-yl) acrylate

(+/-)-Boc-alpha-phosphonoglycine trimethyles 111b (6.07 g, 20.43 mmol) was dissolved in dichloromethane (10 mL), stirred for 10 minutes, added with 1-methyl-1*H*-pyrazole-3-carbaldehyde 111a (1.5 g, 13.62 mmol) and 1,8-diazabicyclo[5.4.0]undec-7-ene (2.28 g, 14.98 mmol), and stirred at room temperature for reaction overnight. After the reaction was completed, the reaction solution was filtered and concentrated under reduced pressure. The obtained residue was further separated and purified by silicagel column chromatography (eluent: system A) to obtain methyl (*Z*)-2-((tert-butoxycarbonyl)amino)-3-(1-methyl-1*H*-pyrazol-3-yl)acrylate 111c ((3.4 g) with a yield of 89%.
MS m/z (ESI): 182.1 [M+H-100]

Step 2

Methyl 2-((*tert*-butoxycarbonyl)amino)-3-(1-methyl-1*H*-pyrazol-3-yl)propanoate

Methyl (Z)-2-((*tert*-butoxycarbonyl)amino)-3-(1-methyl-1*H*-pyrazol-3-yl) acrylate 111c (2.4 g, 8.53 mmol) was dissolved in methanol (20 mL), added with 10% palladium carbon (907.93 mg, 8.53 mmol, 10%), and stirred for reaction for 25 hours in hydrogen atmosphere. After the reaction was completed, the reaction solution was concentrated under reduced pressure to obtain crude product of methyl 2-((*tert*-butoxycarbonyl)amino)-3-(1-methyl-1*H*-pyrazol-3-yl) propanoate 111d (1.8 g), which was directly used for the next reaction.
MS m/z (ESI): 284.2 [M+H]

Step 3

Methyl 2-amino-3-(1-methyl-1*H*-pyrazol-3-yl)propanoate

Methyl 2-((*tert*-butoxycarbonyl)amino)-3-(1-methyl-1*H*-pyrazol-3-yl)propanoate 111d (1.8 g, 6.35 mmol) was dissolved in dichloromethane (20 mL), slowly dropwise added with trifluoroacetic acid (2 mL), and stirred at room temperature for reaction for 16 hours. After the reaction was completed, the reaction solution was concentrated under reduced pressure to obtain crude product of methyl 2-amino-3-(1-methyl-1*H*-pyrazol-3-yl)propanoate 111e (832 mg).
MS m/z (ESI): 184.2 [M+H]

Step 4

Methyl 2-((2-((5-chloro-2-(4-chloro-1*H*-1,2,3-triazol-1-yl)phenyl)amino)-2-oxoethyl amino)-3-(1-methyl-1*H*-pyrazol-3-yl)propanoate

2-chloro-*N*-(5-chloro-2-(4-chloro-1*H*-1,2,3-triazol-1-yl)phenyl)acetamide 24d (300 mg, 981.84 μmol) and me-

thyl 2-amino-3-(1-methyl-1*H*-pyrazol-3-yl)methyl propanoate 111e (269.82 mg, 1.47 mmol) were dissolved in N,N-dimethylformamide (10 mL), added with N,N-diisopropylethylamine (507.57 mg, 3.93 mmol, 649.07 μL), and reacted at 60°C for 15 hours. The reaction solution was added with water (40 mL) for quenching the reaction, extracted with ethyl acetate (40 mL×3). Organic phases were combined, and then washed with water (40 mL) and saturated sodium chloride solution (40 mL) in turn, dried with anhydrous sodium sulfate, filtered, and concentrated under reduced pressure. The obtained residue was further separated and purified by silicagel column chromatography (eluent: system A) to obtain methyl 2-((2-((5-chloro-2-(4-chloro-1*H*-1,2,3-triazol-1-yl)phenyl)amino)-2-oxoethyl)amino)-3-(1-methyl-1*H*-pyrazol-3-yl)propanoate 111f (400 mg) with a yield of 90%.
MS m/z (ESI): 451.9 [M+H]

Step 5

Methyl 2-(2-chloro-*N*-(2-((5-chloro-2-(4-chloro-1*H*-1,2,3-triazol-1-yl)phenyl)amino)-2-oxoethyl)acetamido)-3-(1-methyl-1*H*-pyrazol-3-yl)methyl propanoate

Methyl 2-((2-((5-chloro-2-(4-chloro-1*H*-1,2,3-triazol-1-yl)phenyl)amino)-2-oxoethyl) amino)-3-(1-methyl-1*H*-pyrazol-3-yl)propanoate 111f (400 mg, 884.38 μmol) was dissolved in dichloromethane (10 mL), added with triethylamine (268.47 mg, 2.65 mmol, 368.78 μL), slowly dropwise added with chloroacetyl chloride (149.83 mg, 1.33 mmol, 202.47 μL) in ice bath, and reacted for 2 hours in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of methyl 2-(2-chloro-*N*-(2-((5-chloro-2-(4-chloro-1*H*-1,2,3-triazol-1-yl)phenyl)amino)-2-oxoethyl)acetamido)-3-(1-methyl-1*H*-pyrazol-3-yl)propanoate 111g (420 mg), which was directly used for the next reaction.
MS m/z (ESI): 528.1 [M+H]

Step 6

Methyl 2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl) propanoate

The crude product of methyl 2-(2-chloro-*N*-(2-((5-chloro-2-(4-chloro-1*H*-1,2,3-triazol-1-yl)phenyl)amino)-2-oxoethyl)acetamido)-3-(1-methyl-1*H*-pyrazol-3-yl)propanoate 111g (420 mg, 794.29 μmol) obtained in last step was dissolved in methanol (10 mL), slowly dropwise added with sodium methylate (5.4M methanol solution, 147.09 μL, 794.29 μmol) at 0°C, and continuously reacted for 2 hours at 0°C. The reaction solution was dropwise added with 2M dilute hydrochloric acid to adjust the pH to 7, and concentrated under reduced pressure. The obtained residue was further separated and purified by silicagel column chromatography (eluent: system A) to obtain methyl 2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)propanoate 111h (130 mg) with a yield of 33%.
MS m/z (ESI): 492.1 [M+H]

Step 7

2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)propanoic acid

Methyl 2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)propanoate 111h (130 mg, 264.06 μmol) was dissolved in a mixed solvent of tetrahydrofuran (10 mL), methanol (3 mL) and water (3 mL), added with lithium hydroxide monohydrate (22.16 mg, 528.12 μmol), and reacted at 0°C for 3 hours. The reaction solution was dropwise added with 2M dilute hydrochloric acid for neutralization, and concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)propanoic acid 111i (76 mg) with a yield of 60%. MS m/z (ESI): 478.0 [M+H]

Step 8

2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)-*N*-(2-methyl-2*H*-indazol-5-yl)propanamide

2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)propanoic acid 111i (76 mg, 158.90 μmol) and 2-methyl-2*H*-indazol-5-amine 19a (35.08 mg, 238.35 μmol) were dissolved in ethyl acetate (10 mL), added with propylphosphonic anhydride (50% ethyl acetate solution, 404.47 mg, 635.60 μmol) and N,N-diisopropylethylamine (102.68 mg, 794.50 μmol, 131.30 μL), and reacted at 60°C for 2 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)-*N*-(2-methyl-2*H*-indazol-5-yl)propanamide 111 (61 mg) with a yield of 59%.

MS m/z (ESI): 606.8 [M+H]

$^1$H NMR (400 MHz, DMSO-$d_6$) δ 10.15 (s, 1H), 8.69 (s, 1H), 8.26 (s, 1H), 8.12 (s, 1H), 7.85 (s, 1H), 7.78-7.69 (m, 2H), 7.60-7.51 (m, 2H), 7.31-7.23 (m, 1H), 6.05 (s, 1H), 5.29 (dd, J = 10.4, 5.6 Hz, 1H), 4.37-4.05 (m, 4H), 4.13 (s, 3H), 3.79 (s, 3H), 3.21-3.12 (m, 1H), 3.11-2.99 (m, 1H).

Examples 112 and 113

(S)-2-(4-(5-chloro-2-(4-chloro-1*H*-1,2,3-triazole-1-yl)phenyl)-2,5-dioxiperazine-1-yl)-3-(1-methyl-1*H*-pyrazole-3-yl)-*N*-(2-methyl-2*H*-indazol-5-yl)propionamide 112

(R)-2-(4-(5-chloro-2-(4-chloro-1*H*-1,2,3-triazole-1-yl)phenyl)-2,5-clioxiperazine-1-yl)-3-(1-methyl-1*H*-pyrazole-3-yl)-*N*-(2-methyl-2*H*-indazol-5-yl)propionamide 113

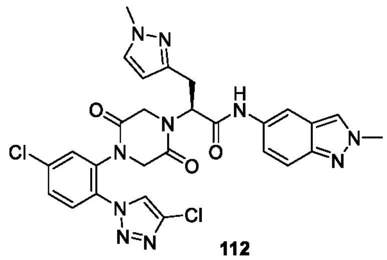

112

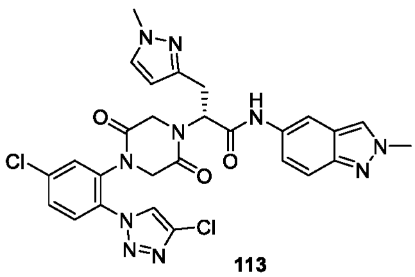

113

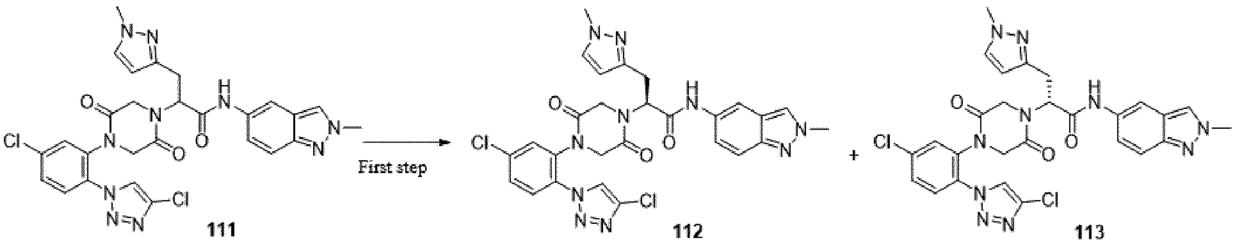


111 112 113

2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)-*N*-(2-methyl-2*H*-indazol-5-yl)propanamide 111 (149 mg, 0.245 mmol) was subjected to SFC chiral resolution (column model: ChiralCel AD, 250×30 mm I.D., 10 μm; mobile phase: A for $CO_2$ and B for Isopropanol; column pressure: 100 bar; flow rate: 80 mL/min; detection wavelength: 220 nm; column temperature: 38°C) to obtain (*S*)-2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)-*N*-(2-methyl-2*H*-indazol-5-yl)propanamide 112 (retention time ($T_R$): 3.527 min; 60 mg) and (*R*)-2-(4-(5-chloro-2-(4-chloro-1*H*-1,2,3-triazol-1-yl)phenyl)-2,5-dioxopiperazin-1-yl)-3-(1-methyl-1*H*-pyrazol-3-yl)-*N*-(2-methyl-2*H*-indazol-5-yl)propanamide 113 (retention time ($T_R$): 4.487 min; 58 mg).

**112**

MS m/z (ESI): 607.1 [M+H]

$^1$H NMR (400 MHz, DMSO-$d_6$) δ 10.12 (s, 1H), 8.69 (s, 1H), 8.25 (s, 1H), 8.12 (s, 1H), 7.85 (s, 1H), 7.78-7.69 (m, 2H), 7.61-7.49 (m, 2H), 7.30-7.23 (m, 1H), 6.05 (s, 1H), 5.35-5.25 (m, 1H), 4.42-4.04 (m, 4H), 4.13 (s, 3H), 3.79 (s, 3H), 3.21-3.12 (m, 1H), 3.10-3.00 (m, 1H).

**113**

MS m/z (ESI): 607.1 [M+H]

$^1$H NMR (400 MHz, DMSO-$d_6$) δ 10.12 (s, 1H), 8.69 (s, 1H), 8.25 (s, 1H), 8.12 (s, 1H), 7.85 (s, 1H), 7.78-7.69 (m, 2H), 7.60-7.51 (m, 2H), 7.31-7.22 (m, 1H), 6.05 (s, 1H), 5.35-5.25 (m, 1H), 4.46-4.01 (m, 4H), 4.13 (s, 3H), 3.79 (s, 3H), 3.21-3.11 (m, 1H), 3.10-2.99 (m, 1H).

Example 114

2-(4-(5-chloro-2-(4-chloro-1*H*-1,2,3-triazol-1-yl)phenyl)-2,5-dioxopiperazin-1-yl)-3-(4-fluorophenyl)-*N*-(2-(methyl-$d_3$)-2*H*-indazol-5-yl)propanamide

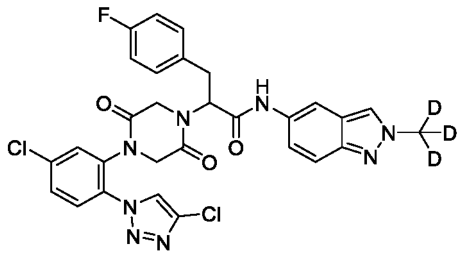

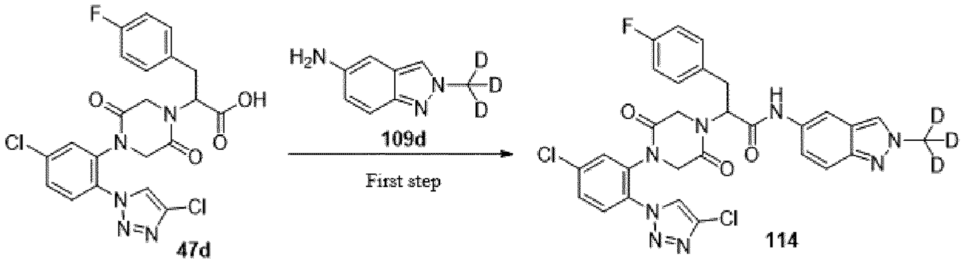


47d

114

2-(4-(5-chloro-2-(4-chloro-1*H*-1,2,3-triazol-1-yl)phenyl)-2,5-dioxopiperazin-1-yl)-3-(4-fluorophenyl)propanoic acid 47d (100 mg, 203.13 μmol) and 2-(methyl-$d_3$)-2*H*-indazol-5-amine 109d (45.76 mg, 304.70 μmol) were dissolved in ethyl acetate (10 mL), added with propylphosphonic anhydride (50% ethyl acetate solution, 517.06 mg, 812.53 μmol) and N,N-diisopropylethylamine (131.26 mg, 1.02 mmol, 167.85 μL), and reacted at 60°C for 2 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(4-fluorophenyl)-*N*-(2-(methyl-*d*3)-2*H*-indazol-5-yl)propanamide 114 (103 mg) with a yield of 80%.

MS m/z (ESI): 624.1 [M+H]

$^1$H NMR (400 MHz, DMSO-$d_6$) δ 10.12 (s, 1H), 8.66 (s, 1H), 8.26 (s, 1H), 8.10 (s, 1H), 7.82-7.65 (m, 3H), 7.54 (d, J = 9.2 Hz, 1H), 7.37-7.28 (m, 2H), 7.28-7.19 (m, 1H), 7.19-7.06 (m, 2H), 5.41-5.32 (m, 1H), 4.47-3.92 (m, 4H), 3.29-3.19 (m, 1H), 3.15-3.06 (m, 1H).

Example 115

2-(4-(5-chloro-2-(4-chloro-1*H*-1,2,3-triazol-1-yl)phenyl)-2,5-dioxopiperazin-1-yl)-3-(4-cyanophenyl)-*N*-(2-methyl-2*H*-indazol-5-yl)propanamide

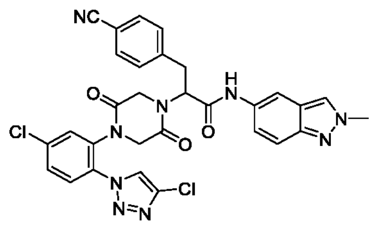

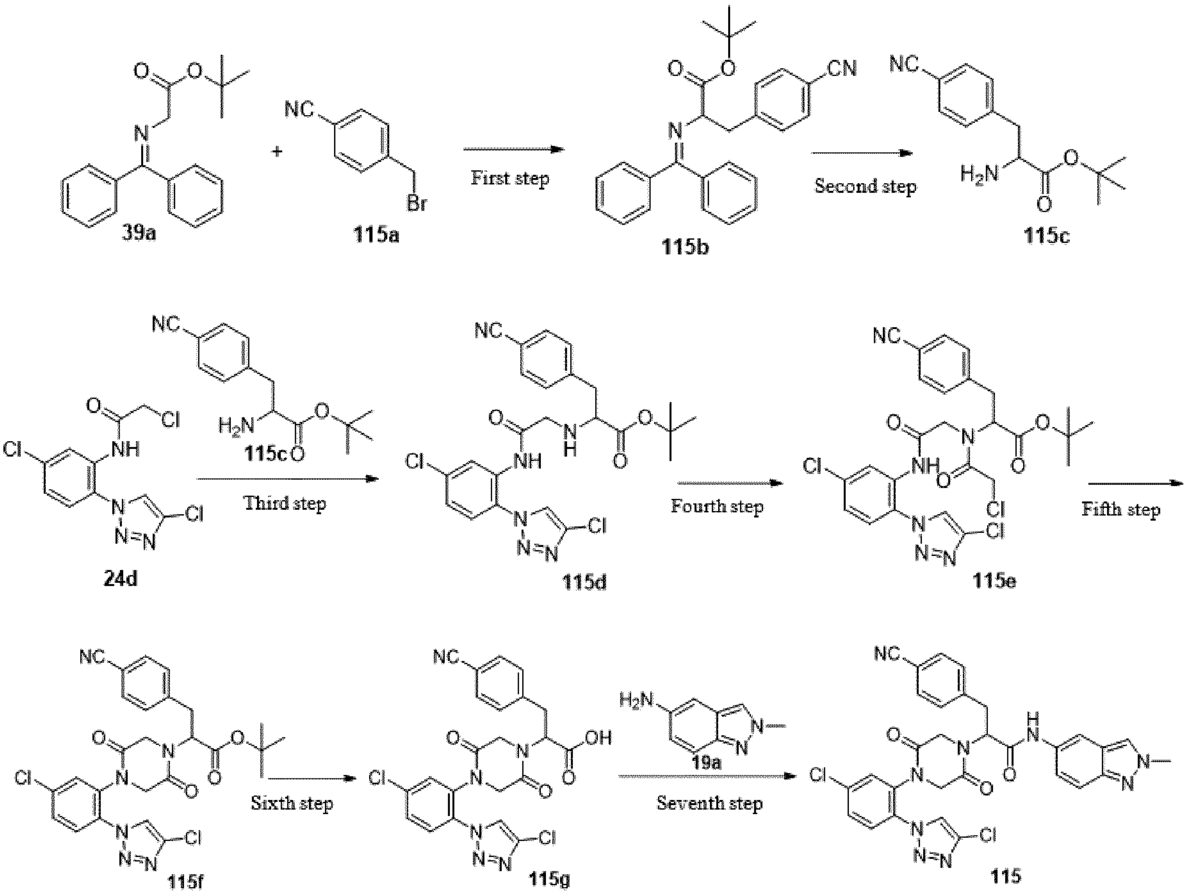


Step 1

*Tert*-butyl 3-(4-cyanophenyl)-2-((diphenylmethylene)amino)propanoate

*Tert*-butyl 2-((diphenylmethylene)amino)acetate 39a (3 g, 10.16 mmol)was dissolved in N,N-dimethylformamide (30 mL), added with potassium tert-butoxide (1.25 g, 11.17 mmol) in batches at 0°C, reacted at 0°C for 15 minutes, then added with 4-(bromomethyl)benzonitrile 115a (2.19 g, 11.17 mmol), heated to room temperature, and then reacted for 3 hours. The reaction solution was added with water (60 mL) for quenching the reaction, and then extracted with ethyl acetate (60 mL×3). Organic phases were combined, washed with water (60 mL×3) and saturated sodium chloride solution (60 mL) in turn, dried with anhydrous sodium sulfate, filtered and concentrated under reduced pressure to obtain crude product of *tert*-butyl 3-(4-cyanophenyl)-2-((diphenylmethylene)amino)propanoate 115b (4.17 g), which was directly used for the next reaction.

MS m/z (ESI): 411.2 [M+H]

Step 2

*Tert*-butyl 2-amino-3-(4-cyanophenyl) propanoate

The crude product of *tert*-butyl 3-(4-cyanophenyl)-2-((diphenylmethylene)amino) propanoate 115b (4.17 g, 10.16 mmol) obtained in the last step was dissolved in tetrahydrofuran (20 mL), added with 2M dilute hydrochloric acid (10 mL), and reacted at room temperature overnight. The reaction solution was dropwise added with saturated sodium bicarbonate solution to adjust the pH to 8, extracted with ethyl acetate (60 mL×3), then organic phases were combined, washed with water (60 mL×3) and saturated sodium chloride solution (60 mL) in turn, dried with anhydrous sodium sulfate, filtered, and concentrated under reduced pressure. The obtained residue was further separated and purified by silicagel column chromatography (eluent: system B) to obtain *tert*-butyl 2-amino-3-(4-cyanophenyl)propanoate 115c (2 g) with a yield of 80%.
MS m/z (ESI): 191.1 [M+H-56]

Step 3

*Tert*-butyl 2-((2-((5-chloro-2-(4-chloro-1H-1,2,3-triazol-1-yl)phenyl)amino)-2-oxoethyl)amino)-3-(4-cyanophenyl)propanoate

2-chloro-*N*-(5-chloro-2-(4-chloro-1*H*-1,2,3-triazol-1-yl)phenyl)acetamide 24d (200 mg, 654.56 μmol) and *tert*-butyl 2-amino-3-(4-cyanophenyl)propanoate 115c (241.83 mg, 981.84 μmol) were dissolved in N,N-dimethylformamide (10 mL), added with N,N-diisopropylethylamine (422.98 mg, 3.27 mmol, 540.89 μL), and reacted at 70°C overnight. The reaction solution was added with water (60 mL) for quenching the reaction, extracted with ethyl acetate (60 mL×3. Organic phases were combined, then washed with water (60 mL) and saturated sodium chloride solution (60 mL), dried with anhydrous sodium sulfate, filtered, and concentrated under reduced pressure. The obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl amino)-3-(4-cyanophenyl) propanoate 115d (267 mg) with a yield of 79%.
MS m/z (ESI): 515.1 [M+H-56]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(4-cyanophenyl)propanoate

*Tert*-butyl 2-((2-((5-chloro-2-(4-chloro-1*H*-1,2,3-triazol-1-yl)phenyl)amino)-2-oxoethyl amino)-3-(4-cyanophenyl)propanoate 115d (267 mg, 518.05 μmol) was dissolved in dichloromethane (10 mL), added with triethylamine (262.11 mg, 2.59 mmol, 360.04 μL), slowly dropwise added with chloroacetyl chloride (87.77 mg, 777.08 μmol, 61.81 μL) in ice bath, and reacted for 1 hour in ice bath. The reaction solution was added with water (50 mL) for quenching the reaction, and then extracted with dichloromethane (50 mL×3). Organic phases were combined, washed with saturated sodium chloride solution (50 mL), dried with anhydrous sodium sulfate, filtered and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(4-cyanophenyl)propanoate 115e (306 mg), which was directly used for the next reaction.
MS m/z (ESI): 535.0 [M+H-56]

Step 5

*Tert*-butyl 2-(4-(5-chloro-2-(4-chloro-1*H*-1,2,3-triazol-1-yl)phenyl)-2,5-dioxopiperazin-1-yl)-3-(4-cyanophenyl) propanoate

The crude product of *tert*-butyl 2-(2-chloro-N-(2-((5-chloro-2-(4-chloro-1*H*-1,2,3-triazol-1-yl)phenyl)amino)-2-oxoethyl)acetamido)-3-(4-cyanophenyl)propanoate 115e (306 mg, 517.00 μmol) obtained in last step was dissolved in methanol (10 mL), slowly dropwise added with sodium methylate (5.4M methanol solution, 105.32 μL, 568.73 μmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain 2-(4-(5-chloro-2-(4-chloro-1*H*-1,2,3-triazol-1-

yl)phenyl)-2,5-dioxopiperazin-1-yl)-3-(4-cyanophenyl)propanoic acid 115f (210 mg) with a yield of 73%.
MS m/z (ESI): 555.0 [M+H]

Step 6

2-(4-(5-chloro-2-(4-chloro-1*H*-1,2,3-triazol-1-yl)phenyl)-2,5-dioxopiperazin-1-yl)-3-(4-cyanophenyl)propanoic acid

*Tert*-butyl 2-(4-(5-chloro-2-(4-chloro-1*H*-1,2,3-triazol-1-yl)phenyl)-2,5-dioxopiperazine-1-yl)-3-(4-cyanophenyl) propanoate 115f (210 mg, 378.10 μmol) was dissolved in dichloromethane (10 mL), slowly dropwise added with trifluoroacetic acid (1 mL), and reacted at room temperature for 2 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(4-cyanophenyl)propanoic acid 115g (188 mg), which was directly used for the next reaction. MS m/z (ESI): 499.0 [M+H]

Step 7

2-(4-(5-chloro-2-(4-chloro-1*H*-1,2,3-triazol-1-yl)phenyl)-2,5-dioxopiperazin-1-yl)-3-(4-cyanophenyl)-*N*-(2-methyl-2*H*-indazol-5-yl)propanamide

The crude product of 2-(4-(5-chloro-2-(4-chloro-1*H*-1,2,3-triazol-1-yl)phenyl)-2,5-dioxopiperazine-1-yl)-3-(4-cyanophenyl)propanoic acid 115g (188 mg, 376.52 μmol) obtained in last step and 2-methyl-2*H*-indazol-5-amine 19a (83.12 mg, 564.78 μmol) were dissolved in ethyl acetate (10 mL), added with propylphosphonic anhydride (50% ethyl acetate solution, 479.21 mg, 753.05 μmol) and N,N-diisopropylethylamine (194.65 mg, 1.51 mmol, 248.91 μL), and reacted at 60°C for 1 hour. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(4-cyanophenyl)-*N*-(2-methyl-2*H*-indazol-5-yl)propanamide 115 (110 mg) with a yield of 45%. MS m/z (ESI): 628.1 [M+H]
$^1$H NMR (400 MHz, DMSO-$d_6$) δ 10.13 (s, 1H), 8.66 (s, 1H), 8.26 (s, 1H), 8.09 (s, 1H), 7.87-7.63 (m, 5H), 7.62-7.44 (m, 3H), 7.23 (d, J = 9.1 Hz, 1H), 5.43 (dd, J = 10.2, 5.8 Hz, 1H), 4.43-3.92 (m, 4H), 4.14 (s, 3H), 3.30-3.14 (m, 2H).

Example 116

2-(4-(5-chloro-2-(4-chloro-1*H*-1,2,3-triazol-1-yl)phenyl)-2,5-dioxopiperazin-1-yl)-3-cyclobutyl-*N*-(2-methyl-2*H*-indazol-5-yl)propanamide

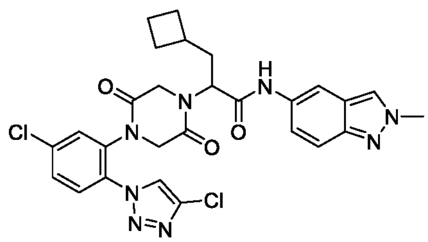

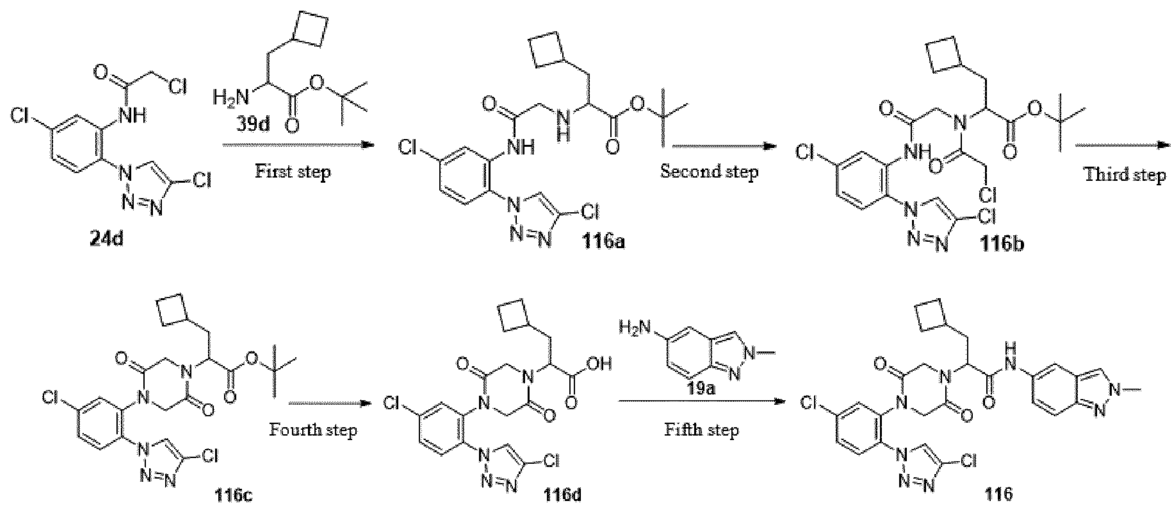


Step 1

*Tert*-butyl 2-((2-((5-chloro-2-(4-chloro-1*H*-1,2,3-triazol-1-yl)phenyl)amino)-2-oxoethyl)amino)-3-cyclobutylpropanoate

2-chloro-*N*-(5-chloro-2-(4-chloro-1*H*-1,2,3-triazol-1-yl)phenyl)acetamide 24d (300 mg, 981.84 μmol) and *tert*-butyl 2-amino-3-cyclobutylpropanoate 39d (293.51 mg, 1.47 mmol) were dissolved in N,N-dimethylformamide (10 mL), added with N,N-diisopropylethylamine (634.47 mg, 4.91 mmol, 811.34 μL), and reacted at 70°C overnight. The reaction solution was added with water (120 mL) for quenching the reaction, and extracted with ethyl acetate (60 mL×3). Organic phases were combined, and then washed with water (60 mL) and saturated sodium chloride solution (60 mL) in turn, dried with anhydrous sodium sulfate, filtered, and concentrated under reduced pressure. The obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-cyclobutylpropanoate 116a (310 mg) with a yield of 67%.
MS m/z (ESI): 468.1 [M+H]

Step 2

*Tert*-butyl 2-(2-chloro-*N*-(2-((5-chloro-2-(4-chloro-1*H*-1,2,3-triazol-1-yl phenyl)amino)-2-oxoethyl)acetamido)-3-cyclobutylpropanoate

*Tert-butyl* 2-((2-((5-chloro-2-(4-chloro-1*H*-1,2,3-triazol-1-yl)phenyl)amino)-2-oxoethyl)amino)-3-cyclobutylpropanoate 116a (310 mg, 661.86 μmol) was dissolved in acetonitrile (10 mL), added with potassium carbonate (457.38 mg, 3.31 mmol), slowly dropwise added with chloroacetyl chloride (89.70 mg, 794.23 μmol, 63.17 μL) in ice bath, and reacted for 1 hour in ice bath. The reaction solution was added with water (50 mL) for quenching the reaction, and then extracted with dichloromethane (50 mL×3). Organic phases were combined, washed with saturated sodium chloride solution (50 mL), dried with anhydrous sodium sulfate, filtered and concentrated under reduced pressure to obtain crude product of *tert*-butyl 2-(2-chloro-*N*-(2-((5-chloro-2-(4-chloro-1*H*-1,2,3-triazol-1-yl phenyl)amino)-2-oxoethyl)acetamido)-3-cyclobutylpropanoate 116b (360 mg), which was directly used for the next reaction.
MS m/z (ESI): 488.0 [M+H-56]

Step 3

*Tert*-butyl 2-(4-(5-chloro-2-(4-chloro-1*H*-1,2,3-triazol-1-yl)phenyl)-2,5-dioxopiperazin-1-yl)-3-yclobutylpropanoate

The crude product of *tert*-butyl 2-(2-chloro-*N*-(2-((5-chloro-2-(4-chloro-1*H*-1,2,3-triazol-1-yl phenyl)amino)-2-oxoethyl)acetylamino)-3-cyclobutylpropanoate 116b (360 mg, 660.72 μmol) obtained in the last step was dissolved in methanol (10 mL), slowly dropwise added with sodium methylate (5.4M methanol solution, 134.59 μL, 726.79 μmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-

triazol-1-yl)phenyl)-2,5-dioxopiperazin-1-yl)-3-cyclobutylpropanoate 116c (300 mg) with a yield of 89%.
MS m/z (ESI): 508.0 [M+H]

Step 4

2-(4-(5-Chloro-2-(4-chloro-1H-1,2,3-triazole-1-yl)phenyl)-2,5-dioxiperazine-1-yl)-3-cyclobutylpropanoic acid

*Tert*-butyl 2-(4-(5-chloro-2-(4-chloro-1*H*-1,2,3-triazol-1-yl)phenyl)-2,5-dioxopiperazin-1-yl)-3-yclobutylpropanoate 116c (100 mg, 196.70 μmol) was dissolved in dichloromethane (5 mL), slowly dropwise added with trifluoroacetic acid (1 mL), and reacted at room temperature overnight. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-cyclobutylpropanoic acid 116d (88 mg), which was directly used for the next reaction.
MS m/z (ESI): 452.1 [M+H]

Step 5

2-(4-(5-Chloro-2-(4-chloro-1*H*-1,2,3-triazol-1-yl)phenyl)-2,5-dioxopiperazin-1-yl)-3-cyclobutyl-*N*-(2-methyl-2*H*-indazol-5-yl)propanamide

The crude product of 2-(4-(5-chloro-2-(4-chloro-1*H*-1,2,3-triazol-1-yl)phenyl)-2,5-dioxopiperazin-1-yl)-3-cyclobutylpropanoic acid 116d (88 mg, 194.57 μmol) obtained in last step and 2-methyl-2*H*-indazol-5-amine 19a (42.95 mg, 291.85 μmol) were dissolved in ethyl acetate (5 mL), added with propylphosphonic anhydride (50% ethyl acetate solution, 247.63 mg, 389.13 μmol) and N,N-diisopropylethylamine (100.58 mg, 778.26 μmol, 128.62 μL), and reacted at 60°C for 1 hour. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-cyclobutyl-*N*-(2-methyl-2*H*-indazol-5-yl)propanamide 116 (59 mg) with a yield of 52%.

MS m/z (ESI): 580.9 [M+H]
$^1$H NMR (400 MHz, DMSO-$d_6$) δ 10.13 (s, 1H), 8.74 (s, 1H), 8.24 (s, 1H), 8.10 (s, 1H), 7.87 (d, J = 2.0 Hz, 1H), 7.81-7.68 (m, 2H), 7.54 (d, J = 9.2 Hz, 1H), 7.28 (dd, J = 9.2, 2.0 Hz, 1H), 5.01 (t, J = 7.9 Hz, 1H), 4.76-4.00 (m, 4H), 4.13 (s, 3H), 2.21-1.55 (m, 9H).

Example 117

2-(4-(5-Chloro-2-(4-chloro-1*H*-1,2,3-triazol-1-yl)phenyl)-2,5-dioxopiperazin-1-yl)-*N*-(2-methyl-2*H*-indazol-5-yl)-3-(tetrahydro-2*H*-pyran-2-yl)propanamide

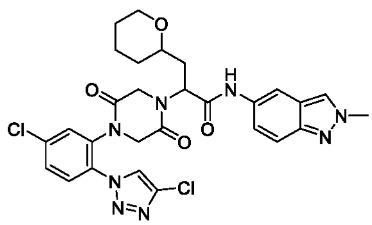

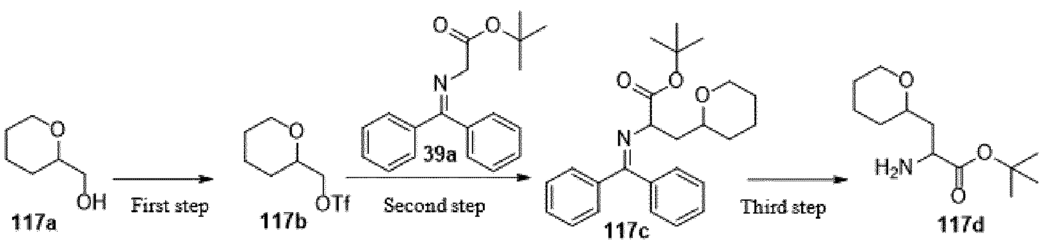

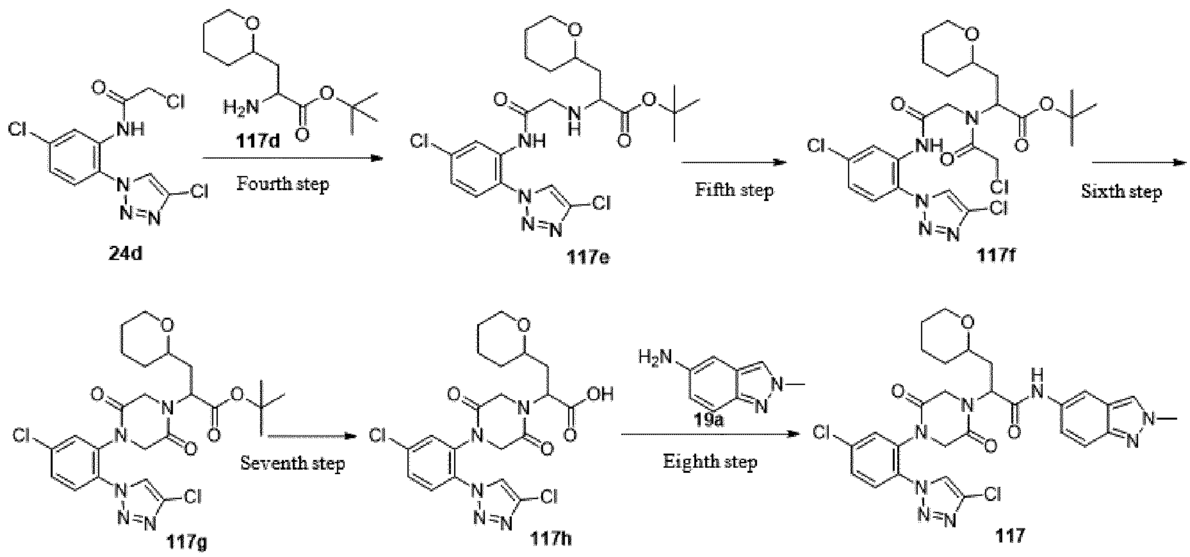


Step 1

(tetrahydro-2*H*-pyran-2-yl)methyl trifluoromethanesulfonate

Tetrahydropyran-2-methanol 117a (3 g, 25.83 mmol, 2.92 mL) was dissolved in dichloromethane (50 mL), added with pyridine (2.45 g, 30.99 mmol, 2.49 mL), cooled to 0°C in nitrogen atmosphere, then slowly dropwise added with trifluoromethanesulfonic anhydride (8.02 g, 28.41 mmol, 4.77 mL), and continuously stirred for 20 minutes in ice bath. The reaction solution was added with ethyl acetate (100 mL) for dilution, washed with 2M dilute hydrochloric acid (50 mL×2), and then washed with saturated sodium chloride solution (50 mL). Organic phases were dried with anhydrous sodium sulfate, filtered, and concentrated under reduced pressure to obtain crude product of (tetrahydro-2*H*-pyran-2-yl)methyl trifluoromethanesulfonate 117b (6.41 g), which was directly used for the next reaction.

Step 2

*Tert*-butyl 2-((diphenylmethylene)amino)-3-(tetrahydro-2*H*-pyran-2-yl) propanoatee

*Tert*-butyl 2-((diphenylmethylene)amino)acetate 39a (5.09 g, 17.22 mmol) was dissolved in tetrahydrofuran (70 mL), cooled to -78°C under the protection of argon, slowly dropwise added with lithium bis(trimethylsilyl)amide (1M tetrahydrofuran solution, 34.43 mL), stirred for 15 minutes, slowly dropwise added with (tetrahydro-2*H*-pyran-2-yl)methyl trifluoromethanesulfonate 117b (6.41 g, 25.82 mmol), continuously stirred for 15 minutes after the addition, slowly heated to room temperature, reacted for 30 minutes, and then added with water (50 mL) for quenching the reaction. The reaction solution was extracted with ethyl acetate (100 mL×3). Organic phases were combined, washed with saturated sodium chloride solution (100 mL), dried with anhydrous sodium sulfate, filtered, and concentrated under reduced pressure to obtain crude product of *tert-butyl* 2-((diphenylmethylene)amino)-3-(tetrahydro-2*H*-pyran-2-yl)propanoate 117c (6.77 g), which was directly used for the next reaction.
MS m/z (ESI): 394.2 [M+H]

Step 3

*Tert*-butyl 2-amino-3-(tetrahydro-2*H*-pyran-2-yl) propanoate

The crude product of *tert*-butyl 2-((diphenylmethylene)amino)-3-(tetrahydro-2*H*-pyran-2-yl) propanoate 117c (6.77 g, 17.20 mmoll) obtained in the last step was dissolved in tetrahydrofuran (20 mL), added with 2M dilute hydrochloric acid (20 mL), and reacted at room temperature overnight. The reaction solution was dropwise added with saturated sodium bicarbonate solution to adjust the pH to 8, extracted with ethyl acetate (100 mL×3), then organic phases were combined, washed with water (100 mL×3) and saturated sodium chloride solution (100 mL) in turn, dried with anhydrous sodium sulfate, filtered, and concentrated under reduced pressure. The obtained residue was further separated and purified by silicagel column chromatography (eluent: system B) to obtain *tert*-butyl 2-amino-3-(tetrahydro-2*H*-pyran-2-yl)propanoate 117d (2 g) with a yield of 51%.

MS m/z (ESI): 174.1 [M+H-56]

Step 4

*tert*-butyl 2-((2-((5-chloro-2-(4-chloro-1*H*-1,2,3-triazol-1-yl)phenyl)amino)-2-oxoethyl)amino)-3-(tetrahydro-2*H*-pyran-2-yl) propanoate

2-chloro-*N*-(5-chloro-2-(4-chloro-1*H*-1,2,3-triazol-1-yl)phenyl)acetamide 24d (300 mg, 981.84 μmol) and *tert*-butyl 2-amino-3-(tetrahydro-2*H*-pyran-2-yl)propanoate 117d (337.73 mg, 1.47 mmol) were dissolved in N,N-dimethylformamide (10 mL), added with N,N-diisopropylethylamine (634.47 mg, 4.91 mmol, 811.34 μL), and reacted at 75°C overnight. The reaction solution was added with water (50 mL) for quenching the reaction, and extracted with ethyl acetate (60 mL×3). Organic phases were combined, and then washed with water (60 mL) and saturated sodium chloride solution (60 mL) in turn, dried with anhydrous sodium sulfate, filtered, and concentrated under reduced pressure. The obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(tetrahydro-2*H*-pyran-2-yl)propanoate 117e (310 mg) with a yield of 63%.
MS m/z (ESI): 497.9 [M+H]

Step 5

Tert-butyl 2-(2-chloro-*N*-(2-((5-chloro-2-(4-chloro-1*H*-1,2,3-triazol-1-yl)phenyl)amino)-2-oxoethyl)acetamido)-3-(tetrahydro-2*H*-pyran-2-yl)propanoate

*Tert-butyl* 2-((2-((5-chloro-2-(4-chloro-1*H*-1,2,3-triazol-1-yl)phenyl)amino)-2-oxoethyl)amino)-3-(tetrahydro-2*H*-pyran-2-yl)propanoate 117e (310 mg, 621.99 μmol) was dissolved in acetonitrile (10 mL), added with potassium carbonate (429.83 mg, 3.11 mmol), slowly dropwise added with chloroacetyl chloride (84.30 mg, 746.39 μmol, 59.37 μL) in ice bath, and reacted for 1 hour in ice bath. The reaction solution was added with water (50 mL) for quenching the reaction, and then extracted with ethyl acetate (60 mL×3). Organic phases were combined, washed with saturated sodium chloride solution (60 mL), dried with anhydrous sodium sulfate, filtered and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(tetrahydro-2*H*-pyran-2-yl) propanoate 117f (357 mg), which was directly used for the next reaction.
MS m/z (ESI): 574.8 [M+H]

Step 6

Tert-butyl 2-(4-(5-chloro-2-(4-chloro-1*H*-1,2,3-triazol-1-yl)phenyl)-2,5-dioxopiperazin-1-yl)-3-(tetrahydro-2*H*-pyran-2-yl)propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(tetrahydro-2*H*-pyran-2-yl) propanoate 117f (357 mg, 621.00 μmol) obtained in the last step was dissolved in methanol (10 mL), slowly dropwise added with sodium methylate (5.4Mmethanol solution, 126.50 μL, 683.10 μmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(tetrahydro-2*H*-pyran-2-yl)propanoate 117g (255 mg) with a yield of 76%.
MS m/z (ESI): 538.1 [M+H]

Step 7

2-(4-(5-Chloro-2-(4-chloro-1*H*-1,2,3-triazol-1-yl)phenyl)-2,5-dioxopiperazin-1-yl)-3-(tetrahydro-2*H*-pyran-2-yl)propanoic acid

Tert-butyl 2-(4-(5-chloro-2-(4-chloro-1*H*-1,2,3-triazol-1-yl)phenyl)-2,5-dioxopiperazin-1-yl)-3-(tetrahydro-2*H*-pyran-2-yl)propanoate 117g (100 mg, 185.73 μmol) was dissolved in dichloromethane (10 mL), slowly dropwise added with trifluoroacetic acid (1 mL), and reacted at room temperature overnight. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(tetrahydro-2*H*-pyran-2-yl)propanoic acid 117h (89 mg), which

was directly used for the next reaction.
MS m/z (ESI): 481.8 [M+H]

Step 8

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(tetrahydro-2*H*-pyran-2-yl)propanamide

The crude product of 2-(4-(5-chloro-2-(4-chloro-1*H*-1,2,3-triazol-1-yl)phenyl)-2,5-dioxopiperazin-1-yl)-3-(tetrahydro-2*H*-pyran-2-yl)propanoic acid 117g (89 mg, 184.53 μmol) obtained in the last step and 2-methyl-2*H*-indazol-5-amine 19a (40.74 mg, 276.79 μmol) were dissolved in ethyl acetate (5 mL), added with propylphosphonic anhydride (50% ethyl acetate solution, 234.85 mg, 369.05 μmol) and N,N-diisopropylethylamine (95.39 mg, 738.10 μmol, 121.99 μL), and reacted at 60°C for 1 hour. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(tetrahydro-2*H*-pyran-2-yl)propanamide 117 (63 mg) with a yield of 55%.

MS m/z (ESI): 611.2 [M+H]
$^1$H NMR (400 MHz, DMSO-$d_6$) δ 10.13-9.93 (m, 1H), 8.85-8.64 (m, 1H), 8.30-8.19 (m, 1H), 8.16-8.04 (m, 1H), 7.88 (s, 1H), 7.82-7.69 (m, 2H), 7.59-7.47 (m, 1H), 7.36-7.23 (m, 1H), 5.35-5.13 (m, 1H), 4.70-3.93 (m, 4H), 4.13 (s, 3H), 3.93-3.78 (m, 1H), 3.35-3.05 (m, 2H), 2.10-1.70 (m, 4H), 1.67-1.37 (m, 4H).

Example 118

2-(4-(5-chloro-2-(4-chloro-1*H*-1,2,3-triazol-1-yl)phenyl)-2,5-dioxopiperazin-1-yl)-4-isopropoxy-*N*-(2-methyl-2*H*-indazol-5-yl)butanamide

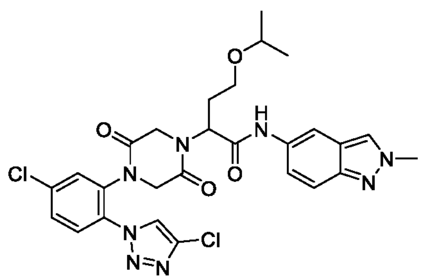

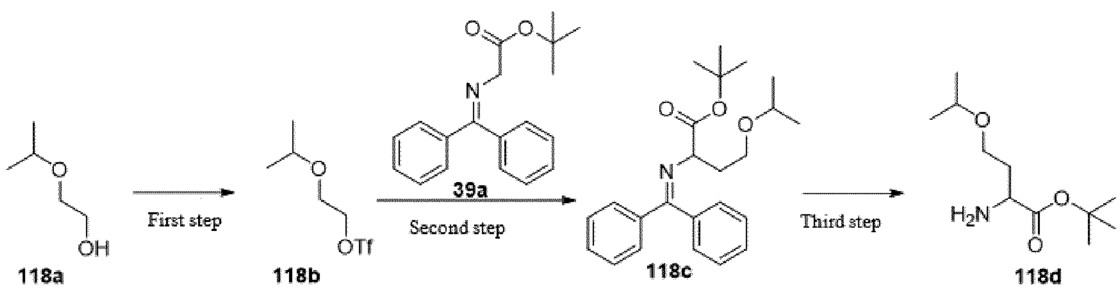

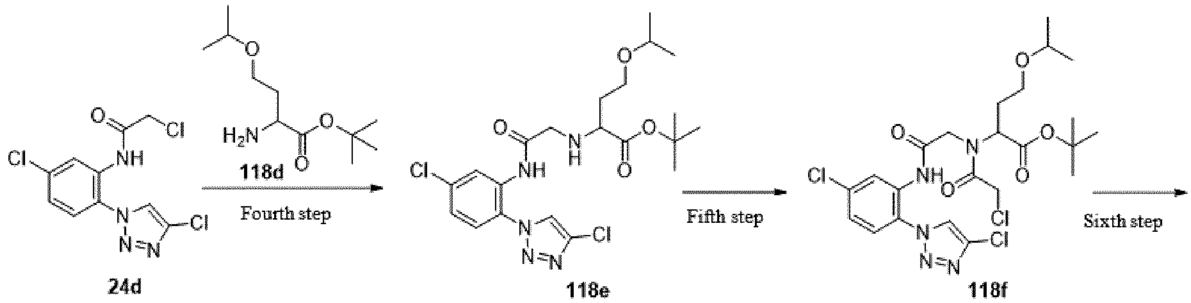


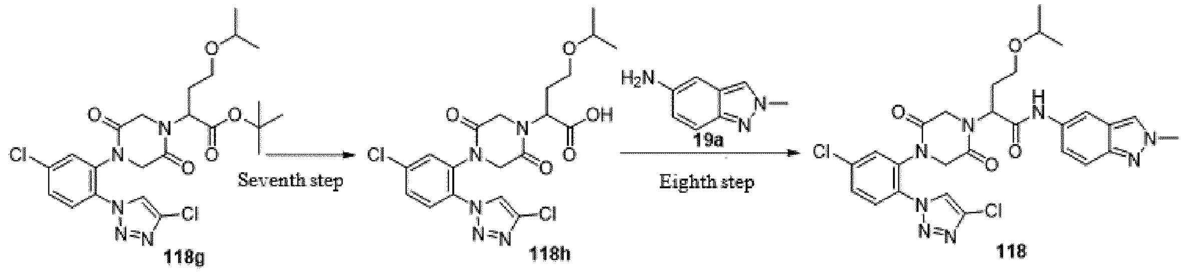


Step 1

2-isopropylethyl trifluoromethanesulfonate

isopropyloxy ethanol 118a (3 g, 28.81 mmol) was dissolved in dichloromethane (50 mL), added with pyridine (2.73 g, 34.57 mmol, 2.78 mL), cooled to 0°C in nitrogen atmosphere, then slowly dropwise added with trifluoromethanesulfonic anhydride (8.94 g, 31.69 mmol, 5.32 mL), and continuously stirred for 20 minutes in ice bath. The reaction solution was added with ethyl acetate (100 mL) for dilution, washed with 2M dilute hydrochloric acid (50 mL×2), and then washed with saturated sodium chloride solution (50 mL). Organic phases were dried with anhydrous sodium sulfate, filtered, and concentrated under reduced pressure to obtain crude product of 2-isopropylethyl trifluoromethanesulfonate 118b (4.6 g), which was directly used for the next reaction.

Step 2

*Tert*-butyl 2-((diphenylmethylene)amino)-4-isopropoxybutanoate

*Tert*-butyl 2-((diphenylmethylene)amino)acetate 39a (8.63 g, 29.21 mmol) was dissolved in tetrahydrofuran (100 mL), cooled to -78°C under the protection of argon, slowly dropwise added with lithium bis(trimethylsilyl)amide (1M tetrahydrofuran solution, 38.95 mL), stirred for 15 minutes, slowly dropwise added with 2-isopropylethyl trifluoromethanesulfonate 118b (4.6 g, 19.47 mmol), continuously stirred for 15 minutes, slowly heated to room temperature, continuously stirred for 30 minutes, and then added with water (50 mL) for quenching the reaction. The reaction solution was extracted with ethyl acetate (100 mL×3). Organic phases were combined, washed with saturated sodium chloride solution (100 mL), dried with anhydrous sodium sulfate, filtered, and concentrated under reduced pressure to obtain crude product of *tert*-butyl 2-((diphenylmethylene)amino)-4-isopropoxybutanoate 118c (7.43 g), which was directly used for the next reaction.
MS m/z (ESI): 382.2 [M+H]

Step 3

*Tert*-butyl O-isopropylhomoserinate

The crude product of *tert*-butyl 2-((diphenylmethylene)amino)-4-isopropoxybutanoate 118c (7.43 g, 19.48 mmol) obtained in the last step was dissolved in tetrahydrofuran (25 mL), added with 2M dilute hydrochloric acid (20 mL), and reacted at room temperature overnight. The reaction solution was dropwise added with saturated sodium bicarbonate solution to adjust the pH to 8, extracted with ethyl acetate (100 mL×3), then organic phases were combined, washed with water (100 mL×3) and saturated sodium chloride solution (100 mL) in turn, dried with anhydrous sodium sulfate, filtered, and concentrated under reduced pressure. The obtained residue was further separated and purified by silicagel

column chromatography (eluent: system B) to obtain tert-butyl O-isopropyl homoserinate118d (1.6 g) with ayield of 38%. MS m/z (ESI): 218.2 [M+H]

Step 4

*Tert*-butyl *N*-(2-((5-chloro-2-(4-chloro-1*H*-1,2,3-triazol-1-yl)phenyl)amino)-2-oxoethyl)-O-isopropylhomoserinate

2-Chloro-*N*-(5-chloro-2-(4-chloro-1*H*-1,2,3-triazol-1-yl)phenyl)acetamide 24d (300 mg, 981.84 μmol) and *tert*-butyl O-isopropyl homoserinate 118d (320.04 mg, 1.47 mmol) were dissolved in N,N-dimethylformamide (10 mL), added with N,N-diisopropylethylamine (380.68 mg, 2.95 mmol, 486.80 μL), and reacted at 70°C overnight. The reaction solution was added with water (50 mL) for quenching the reaction, and extracted with ethyl acetate (60 mL×3). Organic phases were combined, and then washed with water (60 mL) and saturated sodium chloride solution (60 mL) in turn, dried with anhydrous sodium sulfate, filtered, and concentrated under reduced pressure. The obtained residue was further separated and purified by silicagel column chromatography (eluent: system A) to obtain *tert*-butyl *N*-(2-((5-chloro-2-(4-chloro-1*H*-1,2,3-triazol-1-yl)phenyl)amino)-2-oxoethyl)-O-isopropylhomoseriate 118e (380 mg) with a yield of 80%. MS m/z (ESI): 485.8 [M+H]

Step 5

*Tert*-butyl *N*-(2-((5-chloro-2-(4-chloro-1*H*-1,2,3-triazol-1-yl)phenyl)amino)-2-oxoethyl)-*N*-(2-chloroacetyl)-*O*-isopropyl homoseriate

*Tert-butyl* *N*-(2-((5-chloro-2-(4-chloro-1*H*-1,2,3-triazol-1-yl)phenyl)amino)-2-oxoethyl)-O-isopropylhomoserinate 118e (380 mg, 781.26 μmol) was dissolved in dichloromethane (10 mL), added with triethylamine (158.11 mg, 1.56 mmol), slowly dropwise added with chloroacetyl chloride (132.36 mg, 1.17 mmol, 93.21 μL) in ice bath, and reacted for 1 hour in ice bath. The reaction solution was added with water (50 mL) for quenching the reaction, and then extracted with ethyl acetate (60 mL×3). Organic phases were combined, washed with saturated sodium chloride solution (60 mL), dried with anhydrous sodium sulfate, filtered and concentrated under reduced pressure to obtain crude product of *tert*-butyl *N*-(2-((5-chloro-2-(4-chloro-1*H*-1,2,3-triazol-1-yl)phenyl)amino)-2-oxoethyl)-*N*-(2-chloroacetyl)-*O*-isopropylhomoserinate 118f (439 mg), which was directly used for the next reaction. MS m/z (ESI): 506.0 [M+H-56]

Step 6

*Tert*-butyl 2-(4-(5-chloro-2-(4-chloro-1*H*-1,2,3-triazol-1-yl)phenyl)-2,5-dioxopiperazin-1-yl)-4-isopropoxybutanoate

The crude product of *tert*-butyl *N*-(2-((5-chloro-2-(4-chloro-1*H*-1,2,3-triazol-1-yl)phenyl)amino)-2-oxoethyl)-*N*-(2-chloroacetyl)-*O*-isopropylhomoserinate 118f (439 mg, 779.93 μmol) obtained in the last step was dissolved in methanol (10 mL), slowly dropwise added with sodium methylate (5.4M methanol solution, 144.43 μL, 779.93 μmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-4-isopropoxybutanoate 118g (223 mg) with a yield of 54%. MS m/z (ESI): 469.8 [M+H-56]

Step 7

2-(4-(5-chloro-2-(4-chloro-1*H*-1,2,3-triazol-1-yl)phenyl)-2,5-dioxopiperazin-1-yl)-4-isopropoxybutanoic acid

Tert-butyl 2-(4-(5-chloro-2-(4-chloro-1*H*-1,2,3-triazol-1-yl)phenyl)-2,5-dioxopiperazin-1-yl)-4-isopropoxybutanoate 118g (223 mg, 423.62 μmol) was dissolved in dichloromethane (10 mL), slowly dropwise added with trifluoroacetic acid (1 mL), and reacted at room temperature overnight. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-4-isopropoxybutanoic acid 118h (150 mg), which was directly used for the next reaction. MS m/z (ESI): 470.1 [M+H]

Step 8

2-(4-(5-Chloro-2-(4-chloro-1*H*-1,2,3-triazol-1-yl)phenyl)-2,5-dioxopiperazin-1-yl)-4-isopropoxy-*N*-(2-methyl-2*H*-indazol-5-yl)butanamide

The crude product of 2-(4-(5-chloro-2-(4-chloro-1*H*-1,2,3-triazol-1-yl)phenyl)-2,5-dioxopiperazin-1-yl)-4-isopropoxybutanoic acid 118g (150 mg, 318.94 μmol) obtained in the last step and 2-methyl-2*H*-indazol-5-amine 19a (70.88 mg, 481.60 μmol) were dissolved in ethyl acetate (15 mL), added with propylphosphonic anhydride (50% ethyl acetate solution, 814.54 mg, 1.28 mmol) and N,N-diisopropylethylamine (206.10 mg, 1.59 mmol, 263.55 μL), and reacted at 60°C for 1 hour. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-4-isopropoxy-*N*-(2-methyl-2*H*-indazol-5-yl)butanamide 118 (67 mg) with a yield of 34%.

MS m/z (ESI): 598.8 [M+H]

$^1$H NMR (400 MHz, DMSO-$d_6$) δ 10.02 (s, 1H), 8.74 (s, 1H), 8.25 (s, 1H), 8.10 (s, 1H), 7.87 (s, 1H), 7.80-7.70 (m, 2H), 7.53 (d, J = 9.2 Hz, 1H), 7.28 (dd, J = 9.2, 1.9 Hz, 1H), 5.12 (brs, 1H), 4.66-3.76 (m, 4H), 4.13 (s, 3H), 3.53 (p, J = 5.9 Hz, 1H), 3.47-3.26 (m, 2H), 2.18-1.92 (m, 2H), 1.15-1.01 (m, 6H).

Example 119

2-(4-(5-Chloro-2-(4-chloro-1*H*-1,2,3-triazol-1-yl)phenyl)-2,5-dioxopiperazin-1-yl)-3-(2,4-difluorophenyl)-*N*-(2-methyl-2*H*-indazol-5-yl)propanamide

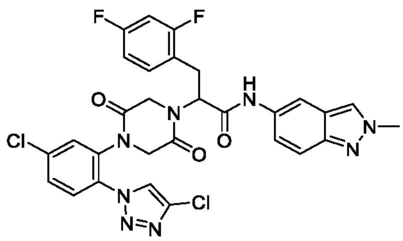

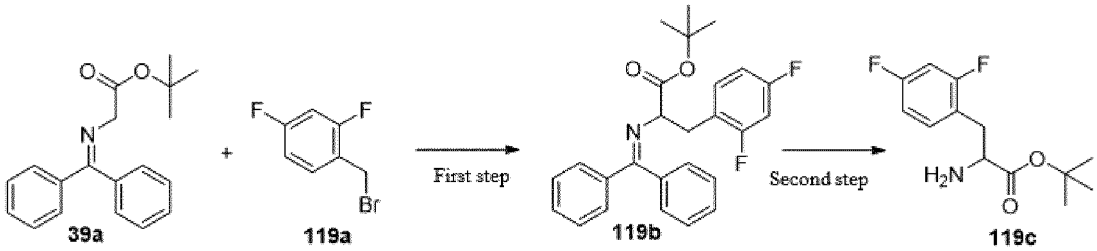


39a 119a 119b 119c

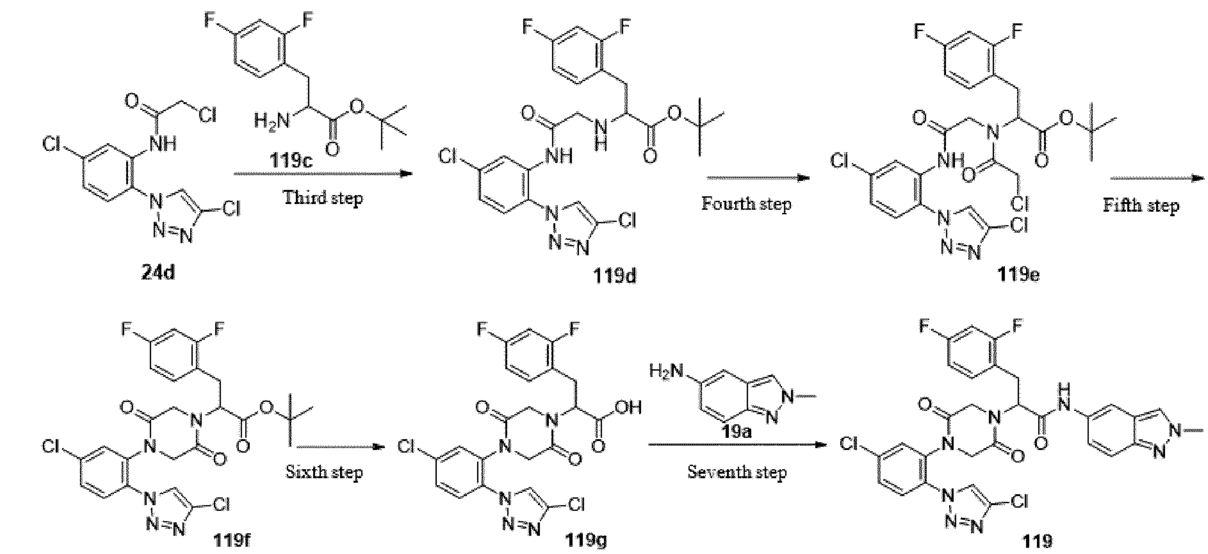


Step 1

*Tert*-butyl 3-(2,4-difluorophenyl)-2-((diphenylmethylene)amino)propanoate

*Tert*-butyl 2-((diphenylmethylene)amino)acetate 39a (4.28 g, 14.49 mmol) was dissolved in N,N-dimethylformamide (25 mL), added with potassium tert-butoxide (1.63 g, 14.49 mmol) in batches at 0°C, reacted at 0°C for 15 minutes, then added with 2,4-difluorobenzyl bromide 119a (2 g, 9.66 mmol, 1.24 mL), reacted to room temperature, and then reacted for 3 hours. The reaction solution was added with water (60 mL) for quenching the reaction, and then extracted with ethyl acetate (60 mL×3). Organic phases were combined, washed with water (60 mL×3) and saturated sodium chloride solution (60 mL) in turn, dried with anhydrous sodium sulfate, filtered and concentrated under reduced pressure to obtain crude product of tert-butyl 3-(2,4-difluorophenyl)-2-((diphenylmethylene)amino)propanoate 119b (4.07 g), which was directly used for the next reaction.
MS m/z (ESI): 422.0 [M+H]

Step 2

*Tert*-butyl 2-amino-3-(2,4-difluorophenyl)propanoate

The crude product of *tert*-butyl 3-(2,4-difluorophenyl)-2-((diphenylmethylene)amino) propanoate 119b (4.07 g, 9.66 mmol) obtained in the last step was dissolved in tetrahydrofuran (20 mL), added with 2M dilute hydrochloric acid (15 mL), and reacted at room temperature overnight. The reaction solution was dropwise added with saturated sodium bicarbonate solution to adjust the pH to 8, extracted with ethyl acetate (60 mL×3), then organic phases were combined, washed with water (60 mL×3) and saturated sodium chloride solution (60 mL) in turn, dried with anhydrous sodium sulfate, filtered, and concentrated under reduced pressure. The obtained residue was further separated and purified by silicagel column chromatography (eluent: system B) to obtain *tert*-butyl 2-amino-3-(2,4-difluorophenyl)propanoate 119c (1.9 g) with a yield of 76%.
MS m/z (ESI): 202.1 [M+H-56]

Step 3

*Tert*-butyl 2-((2-((5-chloro-2-(4-chloro-1*H*-1,2,3-triazol-1-yl)phenyl)amino)-2-oxoethyl)amino)-3-(2,4-difluorophenyl)propanoate

2-chloro-*N*-(5-chloro-2-(4-chloro-1*H*-1,2,3-triazol-1-yl)phenyl)acetamide 24d (300 mg, 981.84 μmol) and *tert*-butyl 2-amino-3-(2,4-difluorophenyl)propanoate 119c (241.83 mg, 981. 378.91 mg, 1.47 mmol) were dissolved in N,N-dimethylformamide (10 mL), added with N,N-diisopropylethylamine (380.68 mg, 2.95 mmol, 486.80 μL), and reacted at 70°C overnight. The reaction solution was added with water (60 mL) for quenching the reaction, and extracted with ethyl acetate (60 mL×3). Organic phases were combined, and then washed with water (60 mL) and saturated sodium chloride solution (60 mL) in turn, dried with anhydrous sodium sulfate, filtered, and concentrated under reduced pressure.

The obtained residue was further separated and purified by silicagel column chromatography (eluent: system A) to obtain *tert*-butyl 2-((2-((5-chloro-2-(4-chloro-1*H*-1,2,3-triazol-1-yl)phenyl)amino)-2-oxoethyl)amino)-3-(2,4-difluorophenyl)propanoate 119d (497 mg) with a yield of 96%.
MS m/z (ESI): 526.1 [M+H]

Step 4

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(2,4-difluorophenyl)propanoate

*Tert*-butyl 2-((2-((5-chloro-2-(4-chloro-1*H*-1,2,3-triazol-1-yl)phenyl)amino)-2-oxoethyl)amino)-3-(2,4-difluorophenyl)propanoate 119d (497 mg, 944.22 μmol) was dissolved in dichloromethane (10 mL), added with triethylamine (191.09 mg, 1.89 mmol, 262.49 μL), slowly dropwise added with chloroacetyl chloride (159.96 mg, 1.42 mmol, 113.45 μL) in ice bath, and reacted for 1 hour in ice bath. The reaction solution was added with water (50 mL) for quenching the reaction, and then extracted with dichloromethane (50 mL×3). Organic phases were combined, washed with saturated sodium chloride solution (50 mL), dried with anhydrous sodium sulfate, filtered and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(2,4-difluorophenyl)propanoate 119e (569 mg), which was directly used for the next reaction.
MS m/z (ESI): 546.0 [M+H-56]

Step 5

*Tert*-butyl 2-(4-(5-chloro-2-(4-chloro-1*H*-1,2,3-triazol-1-yl)phenyl)-2,5-dioxopiperazin-1-yl)-3-(2,4-difluorophenyl)propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(2,4-difluorophenyl)propanoate 119e (569 mg, 943.86 μmol) obtained in the last step was dissolved in methanol (10 mL), slowly dropwise added with sodium methylate (5.4M methanol solution, 174.79 μL, 943.86 μmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(2,4-difluorophenyl)propanoate (300 mg) with a yield of 56%.
MS m/z (ESI): 510.0 [M+H-56]

Step 6

2-(4-(5-chloro-2-(4-chloro-1*H*-1,2,3-triazol-1-yl)phenyl)-2,5-dioxopiperazin-1-yl)-3-(2,4-difluorophenyl)propanoic acid

Tert-butyl 2-(4-(5-chloro-2-(4-chloro-1*H*-1,2,3-triazol-1-yl)phenyl)-2,5-dioxopiperazine-1-yl)-3-(2,4-difluorophenyl)propanoate 119f (300 mg, 529.68 μmol) was dissolved in dichloromethane (10 mL), slowly dropwise added with trifluoroacetic acid (1 mL), and reacted at room temperature for 2 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(2,4-difluorophenyl)propanoic acid 119g (250 mg), which was directly used for the next reaction.
MS m/z (ESI): 509.8 [M+H]

Step 7

2-(4-(5-Chloro-2-(4-chloro-1*H*-1,2,3-triazol-1-yl)phenyl)-2,5-dioxopiperazin-1-yl)-3-(2,4-difluorophenyl)-*N*-(2-methyl-2*H*-indazol-5-yl)propanamide

The crude product of 2-(4-(5-chloro-2-(4-chloro-1*H*-1,2,3-triazol-1-yl)phenyl)-2,5-dioxopiperazin-1-yl)-3-(2,4-difluorophenyl)propanoic acid 119g (250 mg, 489.93 μmol) obtained in the last step and 2-methyl-2*H*-indazol-5-amine 19a (108.16 mg, 734.89 μmol) were dissolved in ethyl acetate (10 mL), added with propylphosphonic anhydride (50% ethyl acetate solution, 1.247 g, 1.96 mmol) and N,N-diisopropylethylamine (316.59 mg, 2.45 mmol, 404.85 μL), and reacted at 60°C for 2 hours. After the reaction was completed, the reaction solution was concentrated under reduced pressure. The obtained residue was further separated and purified by silicagel column chromatography (eluent: system

B) to obtain 2-(4-(5-chloro-2-(4-chloro-1*H*-1,2,3-triazol-1-yl)phenyl)-2,5-dioxopiperazin-1-yl)-3-(2,4-difluorophenyl)-*N*-(2-methyl-2*H*-indazol-5-yl)propanamide 119 (90 mg) with a yield of 29%.

MS m/z (ESI): 639.1 [M+H]

$^1H$ NMR (400 MHz, DMSO-$d_6$) δ 10.06 (s, 1H), 8.69 (s, 1H), 8.26 (s, 1H), 8.06 (s, 1H), 7.84-7.68 (m, 3H), 7.53 (d, J = 9.2 Hz, 1H), 7.42-7.33 (m, 1H), 7.28-7.14 (m, 2H), 7.09-6.98 (m, 1H), 5.39-5.25 (m, 1H), 4.40-3.95 (m, 4H), 4.13 (s, 3H), 3.25-3.18 (m, 1H), 3.17-3.06 (m, 1H).

Example 120

2-(4-(2-(4-chloro-1*H*-1,2,3-triazol-1-yl)-5-methylphenyl)-2,5-dioxopiperazin-1-yl)-*N*-(2-methyl-2*H*-indazol-5-yl)-3-phenylpropanamide

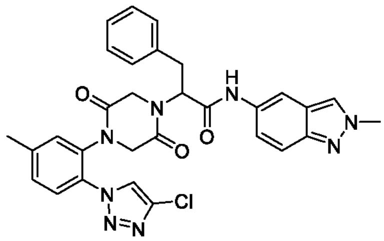

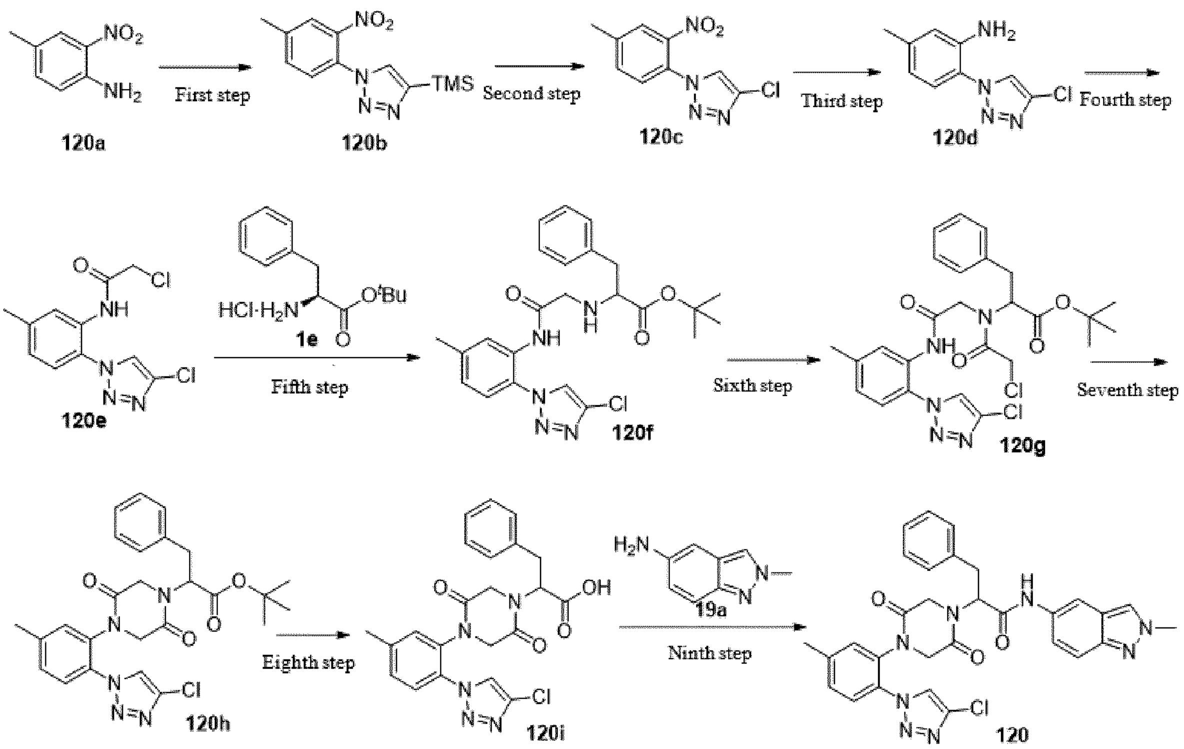


Step 1

1-(4-Methyl-2-nitrophenyl)-4-(trimethylsilyl)-1*H*-1,2,3-triazole

4-Methyl-2-nitroaniline 120a (10 g, 65.72 mmol) was dissolved in acetonitrile (200 mL), added with isoamyl nitrite (11.55 g, 98.59 mmol), cooled to 0°C, slowly added dropwise with azidotrimethylsilane (11.36 g, 98.59 mmol), continuously stirred for 1 hour, then ice bath was removed, and the reaction solution was heated to room temperature and reacted for 2 hours. TLC test showed that the raw materials were completely reacted. Cuprous oxide (9.40 g, 65.72 mmol) and trimethylsilylacetylene (19.37 g, 197.17 mmol) were added in turn, and the reaction solution was continuously

stirred at room temperature for reaction for 16 hours. The reaction solution was filtered with diatomite, added with ethyl acetate (200 mL) and water (200 mL), and then subjected to liquid separation. Organic phases were washed with saturated sodium chloride solution (200 mL), dried with anhydrous sodium sulfate, filtered, and concentrated under reduced pressure. The obtained residue was further separated and purified by silicagel column chromatography (eluent: system A) to obtain 1-(4-methyl-2-nitrophenyl)-4-(trimethylsilyl)-1*H*-1,2,3-triazole 120b (5.0 g) with a yield of 28%.
MS m/z (ESI): 277.1 [M+H]

Step 2

4-chloro-1-(4-methyl-2-nitrophenyl)-1H-1,2,3-triazole

1-(4-Methyl-2-nitrophenyl)-4-(trimethylsilyl)-1*H*-1,2,3-triazole 120b (5 g, 18.09 mmol), N-chlorosuccinimide (24.16 g, 180.92 mmol) and potassium fluoride (10.51 g, 180.92 mmol) were dissolved in acetonitrile (200 mL) in turn, heated to 90°C, and stirred for reaction for 36 hours. The reaction solution was added with ethyl acetate (200 mL) and water (200 mL), and then subjected to liquid separation. Organic phases were washed with saturated sodium chloride solution (200 mL), dried with anhydrous sodium sulfate, filtered, and concentrated under reduced pressure. The obtained residue was further separated and purified by silicagel column chromatography (eluent: system A) to obtain 4-chloro-1-(4-methyl-2-nitrophenyl)-1*H*-1,2,3-triazole 120c (3.0 g) with a yield of 70%.
MS m/z (ESI): 239.0 [M+H]

Step 3

2-(4-Chloro-1*H*-1,2,3-triazol-1-yl)-5-methylaniline

4-Chloro-1-(4-methyl-2-nitrophenyl)-1*H*-1,2,3-triazole 120c (3 g, 12.57 mmol), iron powder (3.51 g, 62.86 mmol) and ammonium chloride (342.58 mg, 6.29 mmol) were dissolved in mixed solvent of ethanol (30 mL) and water (6 mL), heated to 80°C and reacted for 1 hour. The reaction solution was filtered with diatomite, added with ethyl acetate (100 mL) and water (100 mL) for extraction, and then subjected to liquid separation. Organic phases were washed with saturated sodium chloride solution (100 mL), dried with anhydrous sodium sulfate, filtered, and concentrated under reduced pressure to obtain crude product of 2-(4-chloro-1*H*-1,2,3-triazol-1-yl)-5-methylaniline 120d (2.5 g), which was directly used for the next reaction.
MS m/z (ESI): 209.1 [M+H]

Step 4

2-Chloro-*N*-(2-(4-chloro-1*H*-1,2,3-triazol-1-yl)-5-methylphenyl)acetamide

2-(4-chloro-1*H*-1,2,3-triazol-1-yl)-5-methylaniline 120d (2.5 g, 11.98 mmol) and triethylamine (3.64 g, 35.95 mmol) were dissolved in dichloromethane (25 mL), added with chloroacetyl chloride (1.76 g, 15.58 mmol), cooled to 0°C, and reacted for 1 hour. The reaction solution was washed with water (25 mL) and saturated sodium chloride solution (25 mL) in turn, dried with anhydrous sodium sulfate, filtered, and concentrated under reduced pressure to obtain crude product of 2-chloro-*N*-(2-(4-chloro-1*H*-1,2,3-triazol-1-yl)-5-methylphenyl)acetamide 120e (3 g), which was directly used for the next reaction.
MS m/z (ESI): 285.0 [M+H]

Step 5

*Tert*-butyl (2-((2-(4-chloro-1*H*-1,2,3-triazol-1-yl)-5-methylphenyl)amino)-2-oxoethyl)phenylalanine

2-chloro-*N*-(2-(4-chloro-1*H*-1,2,3-triazol-1-yl)-5-methylphenyl)acetamide 120e (3 g, 10.52 mmol), *tert*-butyl 3-phenyl-L-alaninate hydrochloride 1e (4.07g, 15.78 mmol) and N,N-diisopropylethylamine (7.59 g, 52.61 mmol) were dissolved in N,N-dimethylformamide (20 mL), heated to 80°C, and reacted for 12 hours. The reaction solution was added with ethyl acetate (50 mL) and water (50 mL) for extraction, and then subjected to liquid separation. Organic phases were washed with saturated sodium chloride solution (50 mL), dried with anhydrous sodium sulfate, filtered, and concentrated under reduced pressure to obtain crude product of *tert*-butyl (2-((2-(4-chloro-1*H*-1,2,3-triazol-1-yl)-5-methylphenyl)amino)-2-oxoethyl)phenylalaninate 120f (4 g), which was directly used for the next reaction.
MS m/z (ESI): 470.2 [M+H]

Step 6

*tert*-butyl *N*-(2-((2-(4-chloro-1*H*-1,2,3-triazol-1-yl)-5-methylphenyl)amino)-2-oxoethyl)-*N*-(2-chloroacetyl)phenylalaninate

*Tert*-butyl (2-((2-(4-chloro-1*H*-1,2,3-triazol-1-yl)-5-methylphenyl)amino)-2-oxoethyl)phenylalaninate 120f (4 g, 8.51 mmol) and triethylamine (2.58 g, 25.53 mmol) were dissolved in dichloromethane (30 mL), added with chloroacetyl chloride (1.25 g, 11.06 mmol), cooled to 0°C, and reacted for 1 hour. The reaction solution was washed with water (25 mL) and saturated sodium chloride solution (25 mL) in turn, dried with anhydrous sodium sulfate, filtered, and concentrated under reduced pressure to obtain crude product of *tert*-butyl *N*-(2-((2-(4-chloro-1*H*-1,2,3-triazol-1-yl)-5-methylphenyl)amino)-2-oxoethyl)-*N*-(2-chloroacetyl)phenylalaninate120g (4 g), which was directly used for the next reaction.
MS m/z (ESI): 546.2 [M+H]

Step 7

*Tert*-butyl 2-(4-(2-(4-chloro-1*H*-1,2,3-triazol-1-yl)-5-methylphenyl)-2,5-dioxopiperazin-1-yl)-3-phenylpropanoate

*Tert*-butyl *N*-(2-((2-(4-chloro-1*H*-1,2,3-triazol-1-yl)-5-methylphenyl)amino)-2-oxoethyl)-*N*-(2-chloroacetyl)phenylalaninate 120g (4 g, 7.32 mmol) was dissolved in methanol (50 mL), added with sodium methylate (5.4M methanol solution, 4.067 mL, 21.96 mmol), then cooled to 0°C and reacted for 1 hour. The reaction solution was concentrated under reduced pressure, and the obtained residue was further separated and purified by silicagel column chromatography (eluent: system A) to obtain *tert*-butyl 2-(4-(2-(4-chloro-1*H*-1,2,3-triazol-1-yl)-5-methylphenyl)-2,5-dioxopiperazin-1-yl)-3-phenylpropanoate 120h (2.5 g) with a yield of 67%.
MS m/z (ESI): 454.1 [M+H-56]

Step 8

2-(4-(2-(4-chloro-1*H*-1,2,3-triazol-1-yl)-5-methylphenyl)-2,5-dioxopiperazin-1-yl)-3-phenylpropanoic acid

Tert-butyl 2-(4-(2-(4-chloro-1*H*-1,2,3-triazol-1-yl)-5-methylphenyl)-2,5-dioxopiperazin-1-yl)-3-phenylpropanoate 120h (2.5 g, 4.86 mmol) was dissolved in dichloromethane (10 mL), added with trifluoroacetic acid (1 mL), and reacted at room temperature for 1 hour. After the reaction was completed, the reaction solution was concentrated under reduced pressure to obtain crude product of 2-(4-(2-(4-chloro-1*H*-1,2,3-triazol-1-yl)-5-methylphenyl)-2,5-dioxopiperazin-1-yl)-3-phenylpropanoic acid 120i (2 g), which was directly used for the next reaction.
MS m/z (ESI): 454.1 [M+H]

Step 9

2-(4-(2-(4-chloro-1H-1,2,3-triazol-1-yl)-5-methylphenyl)-2,5-dioxopiperazin-1-yl)-*N*-(2-methyl-2*H*-indazol-5-yl)-3-phenylpropanamide

The crude product of 2-(4-(2-(4-chloro-1*H*-1,2,3-triazol-1-yl)-5-methylphenyl)-2,5-dioxopiperazin-1-yl)-3-phenylpropanoic acid 120i (200 mg, 440.65 μmol) obtained in the last step and 2-methyl-2*H*-indazol-5-amine 19a (84.31 mg, 572.8 μmol) were dissolved in ethyl acetate (20 mL), added with propylphosphonic anhydride (50% ethyl acetate solution, 560 mg, 0.88 mmol) and N,N-diisopropylethylamine (170.85 mg, 0.88mmol, 218.48 μL), and reacted at 60°C for 3 hours. After the reaction was completed, the reaction solution was concentrated under reduced pressure. The obtained residue was further separated and purified by silicagel column chromatography (eluent: system B) to obtain 2-(4-(2-(4-chloro-1*H*-1,2,3-triazol-1-yl)-5-methylphenyl)-2,5-dioxopiperazin-1-yl)-*N*-(2-methyl-2*H*-indazol-5-yl)-3-phenylpropanamide 120 (80 mg) with a yield of 30%.

MS m/z (ESI): 583.2 [M+H]
$^1$H NMR (400 MHz, DMSO-$d_6$) δ 10.11 (s, 1H), 8.60 (s, 1H), 8.26 (s, 1H), 8.10 (s, 1H), 7.59-7.49 (m, 2H), 7.45-7.38 (m, 1H), 7.35-7.17 (m, 7H), 5.42-5.31 (m, 1H), 4.49-3.89 (m, 4H), 4.14 (s, 3H), 3.30-3.23 (m, 1H), 3.17-3.03 (m, 1H), 2.41 (s, 3H).

Example 121

2-(4-(5-Chloro-2-(4-(trifluoromethyl)-1*H*-1,2,3-triazol-1-yl)phenyl)-2,5-dioxopiperazin-1-yl)-*N*-(2-methyl-2*H*-indazol-5-yl)-3-phenylpropanamide

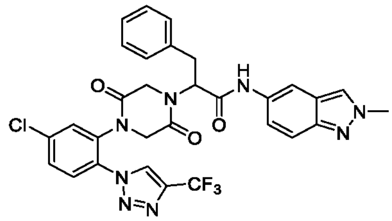

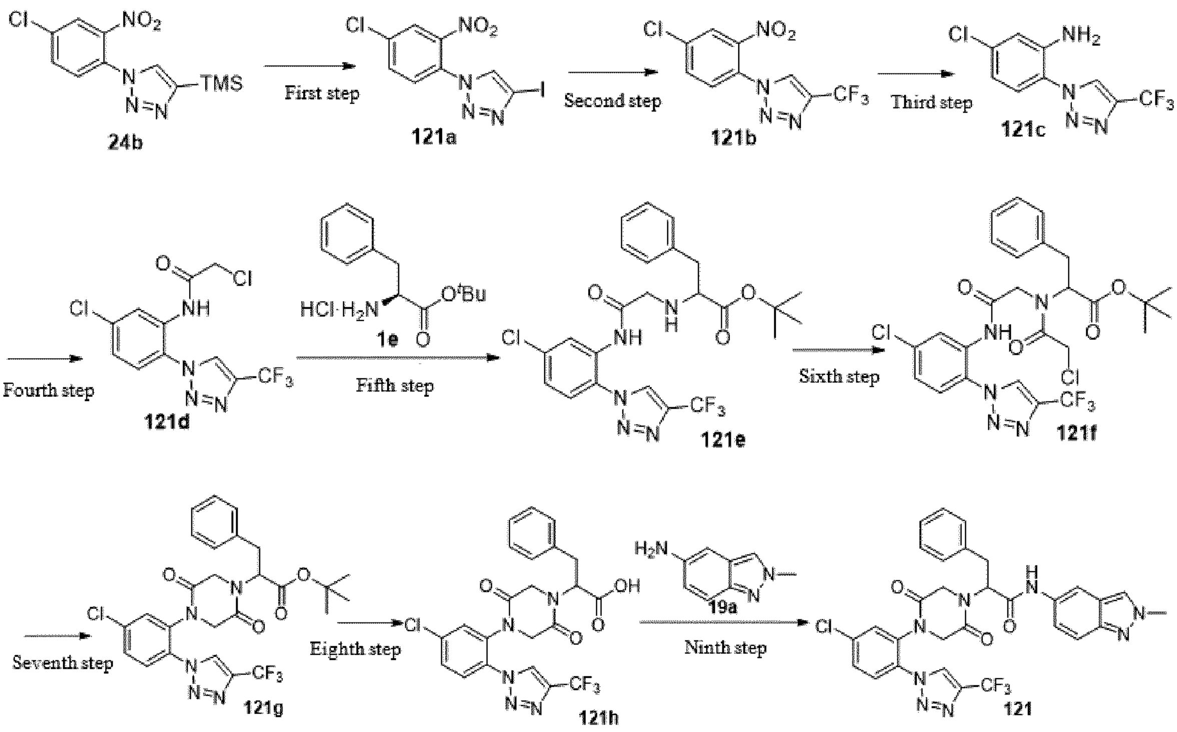


Step 1

1-(4-Chloro-2-nitrophenyl)-4-iodo-1*H*-1,2,3-triazole

1-(4-chloro-2-nitrophenyl)-4-(trimethylsilyl)-1*H*-1,2,3-triazole 24b (3.5 g, 11.79 mmol), N-iodosuccinimide (10.61 g, 47.17 mmol) and silica gel (17.69 g, 294.83 mmol) were added in acetonitrile (200 mL) in turn, heated to 90°C, and stirred for reaction for 4 hours. The reaction solution was filtered and concentrated under reduced pressure, then added with ethyl acetate (200 mL) and water (200 mL) for extraction, and then subjected to liquid separation. Organic phases were washed with saturated sodium chloride solution (200 mL), and dried with anhydrous sodium sulfate, filtered, and concentrated under reduced pressure. The obtained residue was further separated and purified by silicagel column chromatography (eluent: system A) to obtain 1-(4-chloro-2-nitrophenyl)-4-iodo-1*H*-1,2,3-triazole 121a (1.5 g) with a yield of 36%.
MS m/z (ESI): 350.9 [M+H]

Step 2

1-(4-Chloro-2-nitrophenyl)-4-(trifluoromethyl)-1*H*-1,2,3-triazole

1-(4-Chloro-2-nitrophenyl)-4-iodo-1*H*-1,2,3-triazole 121a (3.3 g, 9.42 mmol), methyldifluoro(fluorosulfonyl)acetate (9.04 g, 47.08 mmol), cuprous iodide (1.79 g, 9.42 mmol) and tetrabutylammonium iodide (1.39 g,3.77 mmol) were dissolved in N, N-dimethylformamide (50 mL), and reacted at 120°C for 8 hours. The reaction solution was filtered with diatomite, added with ethyl acetate (50 mL) and water (50 mL), and then subjected to liquid separation. Organic phases were washed with saturated sodium chloride solution (200 mL), dried with anhydrous sodium sulfate, filtered, and concentrated under reduced pressure. The obtained residue was further separated and purified by silicagel column chromatography (eluent: system A) to obtain 1-(4-chloro-2-nitrophenyl)-4-(trifluoromethyl)-1*H*-1,2,3-triazole 121b (1.0 g) with a yield of 36%.
MS m/z (ESI): 293.0 [M+H]

Step 3

5-Chloro-2-(4-(trifluoromethyl)-1*H*-1,2,3-triazol-1-yl aniline

1-(4-Chloro-2-nitrophenyl)-4-(trifluoromethyl)-1*H*-1,2,3-triazole 121b (1g, 3.42 mmol), iron powder (0.95 g, 17.09 mmol) and ammonium chloride (91.42 mg, 1.71 mmol) were dissolved in mixed solvent of ethanol (10 mL) and water (2 mL), heated to 80°C and reacted for 1 hour. The reaction solution was filtered with diatomite, added with ethyl acetate (100 mL) and water (100 mL) for extraction, and then subjected to liquid separation. Organic phases were washed with saturated salt solution (100 mL), dried with anhydrous sodium sulfate, and filtered. The filtrate was concentrated under reduced pressure to obtain crude product of 5-chloro-2-(4-(trifluoromethyl)-1*H*-1,2,3-triazol-1-yl aniline 121c (0.8 g), which was directly used for the next reaction.
MS m/z (ESI): 263.0 [M+H]

Step 4

2-Chloro-*N*-(5-chloro-2-(4-(trifluoromethyl)-1*H*-1,2,3-triazol-1-yl)phenyl)acetamide

5-chloro-2-(4-(trifluoromethyl)-1*H*-1,2,3-triazol-1-yl aniline 121c (0.8 g, 3.05 mmol) and triethylamine (0.93 g, 9.16 mmol) were dissolved in dichloromethane (25 mL), added with chloroacetyl chloride ((0.44 g, 3.97 mmol), cooled to 0°C, and reacted for 1 hour. The reaction solution was washed with water (25 mL) and saturated sodium chloride solution (25 mL) in turn, dried with anhydrous sodium sulfate, filtered, and concentrated under reduced pressure to obtain crude product of 2-chloro-*N*-(5-chloro-2-(4-(trifluoromethyl)-1*H*-1,2,3-triazol-1-yl)phenyl)acetamide 121d (0.7 g), which was directly used for the next reaction.
MS m/z (ESI): 339.0 [M+H]

Step 5

*Tert*-butyl (2-((5-chloro-2-(4-(trifluoromethyl)-1*H*-1,2,3-triazol-1-yl)phenyl)amino)-2-oxoethyl)phenylalaninate

2-chloro-*N*-(5-chloro-2-(4-(trifluoromethyl)-1*H*-1,2,3-triazol-1-yl)phenyl)acetamide 121d (0.7 g, 2.06 mmol), tert-butyl 3-phenyl-L-alaninate hydrochloride 1e (0.79 g, 3.10 mmol) and N,N-diisopropylethylamine (1.49 g, 10.32 mmol) were dissolved in N,N-dimethylformamide (20 mL), heated to 80°C, and reacted for 12 hours. The reaction solution was added with ethyl acetate (50 mL) and water (50 mL) for extraction, and then subjected to liquid separation. Organic phases were washed with saturated sodium chloride solution (50 mL), dried with anhydrous sodium sulfate, filtered, and concentrated under reduced pressure to obtain crude product of *tert*-butyl (2-((5-chloro-2-(4-(trifluoromethyl)-1*H*-1,2,3-triazol-1-yl)phenyl)amino)-2-oxoethyl)phenylalaninate 121e (0.5 g), which was directly used for the next reaction.
MS m/z (ESI): 524.2[M+H]

Step 6

*Tert*-butyl *N*-(2-((5-chloro-2-(4-(trifluoromethyl)-1*H*-1,2,3-triazol-1-yl)phenyl)amino)-2-oxoethyl)-*N*-(2-chloroacetyl)phenylalaninate

*Tert*-butyl (2-((5-chloro-2-(4-(trifluoromethyl)-1*H*-1,2,3-triazol-1-yl)phenyl)amino)-2-oxoethyl)phenylalaninate

121e (0.5 g, 0.95 mmol) and triethylamine (0.29 g, 2.86 mmol) were dissolved in dichloromethane (30 mL), added with chloroacetyl chloride (0.13 g, 1.15 mmol), cooled to 0°C, and reacted for 1 hour. The reaction solution was washed with water (25 mL) and saturated sodium chloride solution (25 mL) in turn, dried with anhydrous sodium sulfate, filtered, and concentrated under reduced pressure to obtain crude product of *tert*-butyl *N*-(2-((5-chloro-2-(4-(trifluoromethyl)-1*H*-1,2,3-triazol-1-yl(phenyl)amino)-2-oxoethyl)-*N*-(2-chloroacetyl)phenylalaninate 121f (0.4 g), which was directly used for the next reaction.
MS m/z (ESI): 544.1[M+H-56]

Step 7

*Tert*-butyl 2-(4-(5-chloro-2-(4-(trifluoromethyl)-1*H*-1,2,3-triazol-1-yl)phenyl)-2,5-dioxopiperazin-1-yl)-3-phenylpropanoate

*Tert*-butyl *N*-(2-((5-chloro-2-(4-(trifluoromethyl)-1*H*-1,2,3-triazol-1-yl)phenyl)amino)-2-oxoethyl)-*N*-(2-chloroacetyl)phenylalaninate 121f (0.4 g, 0.67 mmol) was dissolved in methanol (50 mL), added with sodium methylate (5.4M methanol solution, 0.37 mL, 2.00 mmol), then cooled to 0°C and reacted for 1 hour. The reaction solution was concentrated under reduced pressure, and the obtained residue was further separated and purified by silicagel column chromatography (eluent: system A) to obtain *tert*-butyl 2-(4-(5-chloro-2-(4-(trifluoromethyl)-1*H*-1,2,3-triazol-1-yl)phenyl)-2,5-dioxopiperazin-1-yl)-3-phenylpropanoate 121g (0.15 g) with ayield of 32%.
MS m/z (ESI): 508.1 [M+H-56]

Step 8

2-(4-(5-Chloro-2-(4-(trifluoromethyl)-1*H*-1,2,3-triazol-1-yl)phenyl)-2,5-dioxopiperazin-1-yl)-3-phenylpropanoic acid

*Tert*-butyl 2-(4-(5-Chloro-2-(4-(trifluoromethyl)-1*H*-1,2,3-triazol-1-yl)phenyl)-2,5-dioxopiperazin-1-yl)-3-phenylpropanoate 121g (0.15 g, 0.2 mmol) was dissolved in dichloromethane (10 mL), added with trifluoroacetic acid (1 mL), and reacted at room temperature for 1 hour. After the reaction was completed, the reaction solution was concentrated under reduced pressure to obtain crude product of 2-(4-(5-chloro-2-(4-(trifluoromethyl)-1*H*-1,2,3-triazol-1-yl)phenyl)-2,5-dioxopiperazin-1-yl)-3-phenylpropanoic acid 121h (0.1 g), which was directly used for the next reaction.
MS m/z (ESI): 508.1 [M+H]

Step 9

2-(4-(5-chloro-2-(4-(trifluoromethyl)-1*H*-1,2,3-triazol-1-yl)phenyl)-2,5-dioxopiperazin-1-yl)-*N*-(2-methyl-2*H*-indazol-5-yl)-3-phenylpropanamide

The crude product of 2-(4-(5-chloro-2-(4-(trifluoromethyl)-1*H*-1,2,3-triazol-1-yl)phenyl)-2,5-dioxopiperazin-1-yl)-3-phenylpropanoic acid 121h (100 mg, 0.19 mmol) obtained in the last step and 2-methyl-2*H*-indazol-5-amine 19a (37.67 mg,0.26 mmol) were dissolved in ethyl acetate (20 mL), added with propylphosphonic anhydride (50% ethyl acetate solution, 375.45 mg, 0.59 mmol) and N,N-diisopropylethylamine (76.35 mg, 0.59 mol, 97.63 μL), and reacted at 60°C for 3 hours. After the reaction was completed, the reaction solution was concentrated under reduced pressure. The obtained residue was further separated and purified by silicagel column chromatography (eluent: system B) to obtain 2-(4-(5-chloro-2-(4-(trifluoromethyl)-1*H*-1,2,3-triazol-1-yl)phenyl)-2,5-dioxopiperazin-1-yl)-*N*-(2-methyl-2*H*-indazol-5-yl)-3-phenylpropanamide 121 (57 mg) with a yield of 45%.

MS m/z (ESI): 637.2 [M+H]
$^1$H NMR (400 MHz, DMSO-$d_6$) δ 10.11 (s, 1H), 9.23 (s, 1H), 8.24 (s, 1H), 8.09 (s, 1H), 7.85-7.68 (m, 3H), 7.53 (d, J = 9.2 Hz, 1H), 7.34-7.17 (m, 6H), 5.44-5.34 (m, 1H), 4.27-3.91 (m, 4H), 4.14 (s, 3H), 3.30-3.21 (m, 1H), 3.13-3.01 (m, 1H).

Example 122

4-(*tert*-butoxy)-2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)butanamide

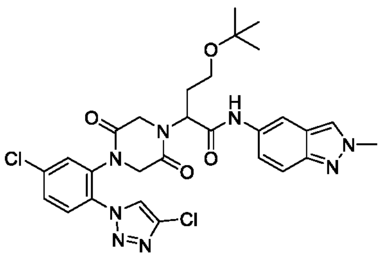

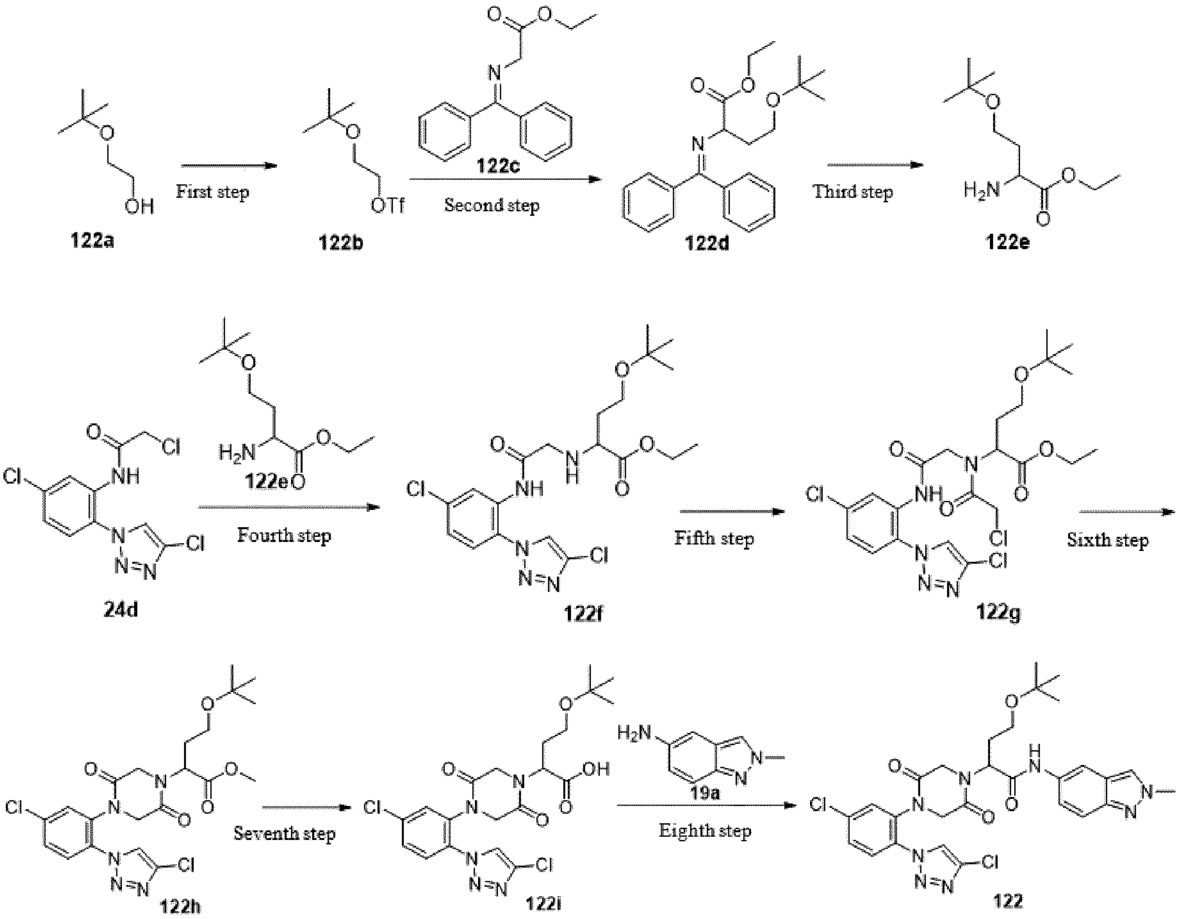


122a 122b 122d 122e 24d 122e 122f 122g 122h 122i 122

Step 1

2-(*Tert*-butoxy)ethyl trifluoromethanesulfonate

2-*tert*-butoxyethanol 122a (2 g, 16.92 mmol) was dissolved in dichloromethane (20 mL), added with pyridine (1.61 g, 20.31 mmol), cooled to 0°C in nitrogen atmosphere, then slowly dropwise added with trifluoromethanesulfonic anhydride (5.25 g, 18.62 mmol), and continuously stirred for 20 minutes in ice bath. The reaction solution was added with dichloromethane (100 mL) for dilution, washed with 2M dilute hydrochloric acid (50 mL×2) and saturated sodium chloride solution (50 mL) in turn. Organic phases were dried with anhydrous sodium sulfate, filtered, and concentrated under reduced pressure to obtain crude product of 2-(*tert*-butoxy)ethyl trifluoromethanesulfonate 122b (4.24 g), which was directly used for the next reaction.

Step 2

Ethyl 4-(*tert*-butoxy)-2-((diphenylmethylene)amino)butanoate

Ethyl 2-((diphenylmethylene)amino)acetate 122c (6.79 g, 25.42 mmol) was dissolved in tetrahydrofuran (66

mL), cooled to -78°C under the protection of argon, slowly dropwise added with lithium bis(trimethylsilyl)amide (1M tetrahydrofuran solution, 33.89 mL), stirred for 15 minutes, slowly dropwise added with 2-(tert-butoxy)ethyl trifluoromethanesulfonate 122b (4.24 g, 16.94 mmol), continuously stirred for 15 minutes, slowly heated to room temperature, continuously stirred for 30 minutes under room temperature, and then added with water (50 mL) for quenching the reaction. The reaction solution was extracted with ethyl acetate (100 mL×3). Organic phases were combined, washed with saturated sodium chloride solution (100 mL), dried with anhydrous sodium sulfate, filtered, and concentrated under reduced pressure to obtain crude product of ethyl 4-(*tert*-butoxy)-2-((diphenylmethylene)amino)butanoate 122d (6.23 g), which was directly used for the next reaction. MS m/z (ESI): 368.1 [M+H]

Step 3

Ethyl *O*-(*tert*-butyl)homoserinate

The crude product of ethyl 4-(*tert*-butoxy)-2-((diphenylmethylene)amino)butanoate 122d (6.23 g, 16.95 mmol) obtained in the last step was dissolved in tetrahydrofuran (25 mL), added with 2M dilute hydrochloric acid (25 mL), and reacted at room temperature overnight. The reaction solution was dropwise added with saturated sodium bicarbonate solution to adjust the pH to 8, extracted with ethyl acetate (100 mL×3), then organic phases were combined, washed with water (100 mL×3) and saturated sodium chloride solution (100 mL) in turn, dried with anhydrous sodium sulfate, filtered, and concentrated under reduced pressure. The obtained residue was further separated and purified by silicagel column chromatography (eluent: system B) to obtain ethyl *O*-(*tert*-butyl)homoserinate 122e (1.6 g) with a yield of 45%. MS m/z (ESI): 204.1 [M+H]

Step 4

Ethyl *O*-(*tert*-butyl)-*N*-(2-((5-chloro-2-(4-chloro-1*H*-1,2,3-triazol-1-yl)phenyl)amino)-2-oxoethyl)homoserinate

2-chloro-*N*-(5-chloro-2-(4-chloro-1*H*-1,2,3-triazol-1-yl)phenyl)acetamide 24d (300 mg, 981.84 μmol) and ethyl O-(*tert*-butyl)homoserinate 122e (300 mg, 981.84 μmol) were dissolved in N,N-dimethylformamide (20 mL), added with N,N-diisopropylethylamine (253.79 mg, 1.96 mmol), and reacted at 70°C overnight. The reaction solution was added with water (50 mL) for quenching the reaction, and then extracted with ethyl acetate (60 mL×3). Organic phases were combined and then washed with water (60 mL) and saturated sodium chloride solution (60 mL) in turn, dried with anhydrous sodium sulfate, filtered, and concentrated under reduced pressure. The obtained residue was further separated and purified by silicagel column chromatography (eluent: system A) to obtain ethyl *O*-(*tert*-butyl)-*N*-(2-((5-chloro-2-(4-chloro-1*H*-1,2,3-triazol-1-yl)phenyl)amino)-2-oxoethyl)homoserinate 122f (230 mg) with a yield of 50%.
MS m/z (ESI): 471.9 [M+H]

Step 5

Ethyl *O*-(*tert*-butyl)-*N*-(2-((5-chloro-2-(4-chloro-1*H*-1,2,3-triazol-1-yl)phenyl)amino)-2-oxoethyl)-*N*-(2-chloroacetyl)homoserinate

Ethyl O-(*tert*-butyl)-*N*-(2-((5-chloro-2-(4-chloro-1*H*-1,2,3-triazol-1-yl)phenyl)amino)-2-oxoethyl)homoserinate 122f (420 mg, 486.91 μmol) was dissolved in dichloromethane (10 mL), added with triethylamine (98.54 mg, 973.82 μmol), slowly dropwise added with chloroacetyl chloride (82.49 mg, 730.37 μmol) in ice bath, and reacted for 2 hours in ice bath. The reaction solution was added with water (50 mL) for quenching the reaction, and then extracted with ethyl acetate (60 mL×3). Organic phases were combined, washed with saturated sodium chloride solution (60 mL), dried with anhydrous sodium sulfate, filtered and concentrated under reduced pressure to obtain crude product of ethyl *O*-(*tert*-butyl)-*N*-(2-((5-chloro-2-(4-chloro-1*H*-1,2,3-triazol-1-yl)phenyl)amino)-2-oxoethyl)-*N*-(2-chloroacetyl)homoserinate 122g (267 mg), which was directly used for the next reaction.
MS m/z (ESI): 492.0 [M+H-56]

Step 6

Methyl 4-(*tert*-butoxy)-2-(4-(5-chloro-2-(4-chloro-1*H*-1,2,3-triazol-1-yl)phenyl)-2,5-dioxopiperazin-1-yl)butanoate

The crude product of ethyl *O*-(*tert*-butyl)-*N*-(2-((5-chloro-2-(4-chloro-1*H*-1,2,3-triazol-1-yl)phenyl)amino)-2-oxoethyl)-*N*-(2-chloroacetyl)homoserinate 122g (267 mg, 486.48 μmol) obtained in the last step was dissolved in methanol (10 mL), slowly dropwise added with sodium methylate (5.4M methanol solution, 90.09 μL,486.48 μmol) at 0°C, and

continuously reacted at 0°C for 2 hours. The reaction solution was dropwise added with 2M dilute hydrochloric acid to adjust the pH to 7, and concentrated under reduced pressure. The obtained residue was further separated and purified by silicagel column chromatography (eluent: system A) to obtain methyl 4-(*tert*-butoxy)-2-(4-(5-chloro-2-(4-chloro-1*H*-1,2,3-triazol-1-yl)phenyl)-2,5-dioxopiperazin-1-yl)butanoate 122h (200 mg) with a yield of 82%.
MS m/z (ESI): 498.1 [M+H]

Step 7

4-(*tert*-butoxy)-2-(4-(5-chloro-2-(4-chloro-1*H*-1,2,3-triazol-1-yl)phenyl)-2,5-dioxopiperazin-1-yl)butanoic acid

Methyl 4-(tert-butoxy)-2-(4-(5-chloro-2-(4-chloro-1*H*-1,2,3-triazol-1-yl)phenyl)-2,5-dioxopiperazin-1-yl) butanoate 122h (200 mg, 390.33 μmol) was dissolved in mixed solvent of tetrahydrofuran (6 mL), methanol (2 mL) and water (2 mL), added with lithium hydroxide monohydrate (16.38 mg, 390.33 μmol), and reacted at 0°C for reaction for 2 hours. After the reaction was completed, the reaction solution was added with 2M dilute hydrochloric acid for neutralization, and concentrated under reduced pressure. The obtained residue was further separated and purified by silicagel column chromatography (eluent: system B) to obtain 4-(*tert*-butoxy)-2-(4-(5-chloro-2-(4-chloro-1*H*-1,2,3-triazol-1-yl)phenyl)-2,5-dioxopiperazin-1-yl)butanoic acid 122i (152 mg) with a yield of 80%.
MS m/z (ESI): 484.1 [M+H]

Step 8

4-(*Tert*-butoxy)-2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)butanamide

4-(*tert*-butoxy)-2-(4-(5-chloro-2-(4-chloro-1*H*-1,2,3-triazol-1-yl)phenyl)-2,5-dioxopiperazin-1-yl)butanoic acid 122i (152 mg, 313.83 μmol) and 2-methyl-2*H*-indazol-5-amine 19a (69.28 mg, 470.75 μmol) were dissolved in ethyl acetate (15 mL), added with propylphosphonic anhydride (50% ethyl acetate solution, 801.81 mg, 1.26 mmol) and N,N-diisopropylethylamine (202.80 mg, 1.57 mmol, 259.34 μL), and reacted at 60°C for 15 hours. After the reaction was completed, the reaction solution was concentrated under reduced pressure. The obtained residue was further separated and purified by silicagel column chromatography (eluent: system B) to obtain 4-(*tert*-butoxy)-2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)butanamide 122 (150 mg) with a yield of 76%.

MS m/z (ESI): 613.2 [M+H]
$^1$H NMR (400 MHz, DMSO-$d_6$) δ 10.00 (s, 1H), 8.72 (s, 1H), 8.24 (s, 1H), 8.10 (s, 1H), 7.86 (s, 1H), 7.80-7.69 (m, 2H), 7.53 (d, J = 9.2 Hz, 1H), 7.28 (dd, J = 9.2, 1.9 Hz, 1H), 5.18-5.02 (m, 1H), 4.51-3.89 (m, 4H), 4.13 (s, 3H), 3.39-3.27 (m, 2H), 2.17-2.04 (m, 1H), 2.04-1.89 (m, 1H), 1.12 (s, 9H).

Example 123

2-(4-(5-chloro-2-(4-chloro-1*H*-1,2,3-triazol-1-yl)phenyl)-2,5-dioxopiperazin-1-yl)-3-(1-cyclopropyl-1*H*-pyrazol-3-yl)-*N*-(2-methyl-2*H*-indazol-5-yl)propanamide

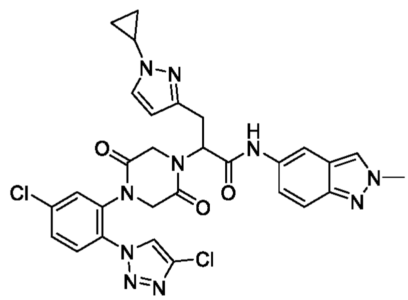

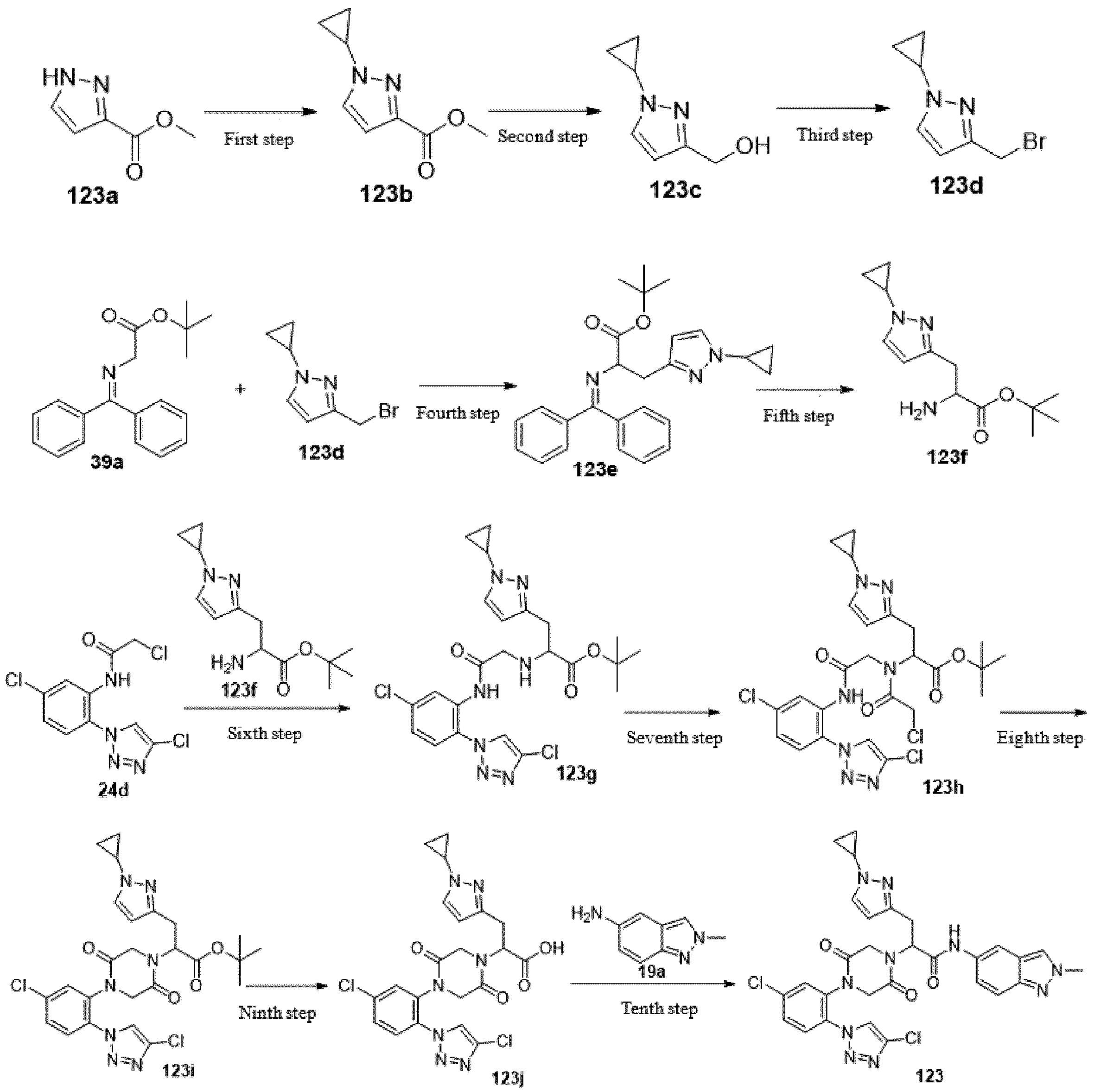


Step 1

Methyl 1-cyclopropyl-1*H*-pyrazole-3-carboxylate

Methyl 1*H*-pyrazole-3-carboxylate 123a (5 g, 39.65 mmol), cyclopropylboronic acid (6.81 g, 79.29 mmol) and sodium carbonate (8.40 g, 79.29 mmol) were dissolved in 1,2-dichloroethane (100 mL), added with cupric acetate anhydrous (7.20 g, 39.65 mmol) and 2,2-bipyridyl (6.19 g, 39.65 mmol) at 70°C, and reacted for 8 hours under oxygen atmosphere. The reaction solution was filtered and concentrated under reduced pressure, and the obtained residue was further separated and purified by silicagel column chromatography (eluent: system A) to obtain methyl 1-cyclopropyl-1*H*-pyrazole-3-carboxylate 123b (2.6 g) with a yield of 40%.
MS m/z (ESI): 167.1 [M+H]

Step 2

(1-cyclopropyl-1*H*-pyrazole-3-yl)methanol

Methyl 1-cyclopropyl-1*H*-pyrazole-3- carboxylate 123b (500 mg, 3.01 mmol) was dissolved in tetrahydrofuran (20 mL), slowly added with lithium aluminium hydride (1153.09 mg, 4.51 mmol) in batches in ice bath, and reacted for 30 minutes in ice bath. The reaction solution was added with water (0.15 mL) for quenching the reaction, and then added

with 15% sodium hydroxide solution (0.15 mL) and water (0.45 mL), stirred for 10 minutes, dried with anhydrous sodium sulfate, filtered and concentrated under reduced pressure to obtain crude product of (1-cyclopropyl-1*H*-pyrazole-3-yl)methanol 123c (415 mg), which was directly used for the next reaction.
MS m/z (ESI): 139.1[M+H]

Step 3

3-(bromomethyl)- 1-cyclopropyl-1*H*-pyrazole

(1-cyclopropyl-1*H*-pyrazole-3-yl)methanol 123c (415 mg, 3.00 mmol) was dissolved in dichloromethane (20 mL), slowly dropwise added with phosphorus tribromide (1.22 g, 4.51 mmol, 423.46 μL), reacted for 10 minutes in ice bath, heated to room temperature and reacted for 1 hour. The reaction solution was added with water (5 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of 3-(bromomethyl)- 1-cyclopropyl-1*H*-pyrazole 123d (540 mg) with a yield of 89%.
MS m/z (ESI): 201.0 [M+H]

Step 4

*Tert*-butyl 3-(1-cyclopropyl-1*H*-pyrazol-3-yl)-2-((diphenylmethylene)amino)propanoate

*Tert*-butyl 2-((diphenylmethylene)amino)acetate 39a (610.23 mg, 2.07 mmol) was dissolved in N,N-dimethylformamide (10 mL), added with potassium tert-butoxide (255.00 mg, 2.27 mmol) in batches at 0°C, reacted at 0°C for 15 minutes, then added with 3-(bromomethyl- 1 -cyclopropyl-1*H*-pyrazole 123d (540 mg, 2.69 mmol), heated to room temperature, and then reacted for 3 hours. The reaction solution was added with water (60 mL) for quenching the reaction, and then extracted with ethyl acetate (60 mL×3). Organic phases were combined, washed with water (60 mL×3) and saturated sodium chloride solution (60 mL) in turn, dried with anhydrous sodium sulfate, filtered and concentrated under reduced pressure to obtain crude product of *tert*-butyl 3-(1-cyclopropyl-1*H*-pyrazol-3-yl)-2-((diphenylmethylene)amino)propanoate 123e (858 mg), which was directly used for the next reaction.
MS m/z (ESI): 416.0 [M+H]

Step 5

Tert-butyl 2-amino-3-(1-cyclopropyl-1*H*-pyrazol-3-yl)propanoate

The crude product of *tert*-butyl 3-(1-cyclopropyl-1*H*-pyrazol-3-yl)-2-((diphenylmethylene)amino)propanoate 123e (858 mg, 2.06 mmol) obtained in the last step was dissolved in tetrahydrofuran (10 mL), added with 2M dilute hydrochloric acid (10 mL), and reacted at room temperature overnight. The reaction solution was dropwise added with saturated sodium bicarbonate solution to adjust the pH to 8, extracted with ethyl acetate (60 mL×3), then organic phases were combined, washed with water (60 mL×3) and saturated sodium chloride solution (60 mL) in turn, dried with anhydrous sodium sulfate, filtered, and concentrated under reduced pressure. The obtained residue was further separated and purified by silicagel column chromatography (eluent: system B) to obtain tert-butyl 2-amino-3-(1-cyclopropyl-1*H*-pyrazol-3-yl)propanoate 123f (371 g) with a yield of 80%.
MS m/z (ESI): 252 [M+H]

Step 6

*Tert*-butyl 2-((2-((5-chloro-2-(4-chloro-1*H*-1,2,3-triazol-1-yl)phenyl)amino)-2-oxoethyl)amino)-3-(1-cyclopropyl-1*H*-pyrazol-3-yl)propanoate

2-chloro-*N*-(5-chloro-2-(4-chloro-1*H*-1,2,3-triazol-1-yl)phenyl)acetamide 24d (375.87 mg, 1.23 mmol) and tert-butyl 2-amino-3-(1-cyclopropyl-1*H*-pyrazol-3-yl)propanoate 123f (371 mg, 1.48 mmol) were dissolved in N,N-dimethylformamide (15 mL), added with N,N-diisopropylethylamine (794.92 mg, 6.15 mmol, 1.02 mL), and reacted at 70°C overnight. The reaction solution was added with water (75 mL) for quenching the reaction, and then extracted with ethyl acetate (50 mL×3). Organic phases were combined and then washed with water (50 mL) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain tert-butyl 2-((2-((5-chloro-2-(4-chloro-1*H*-1,2,3-triazol-1-yl)phenyl)amino)-2-oxoethyl)amino)-3-(1-cyclopropyl-1*H*-pyrazol-3-yl)propanoate 123g (600 mg) with a yield of 94%.
MS m/z (ESI): 520.2 [M+H]

Step 7

*Tert*-butyl 2-(2-chloro-*N*-(2-((5-chloro-2-(4-chloro-1*H*-1,2,3-triazol-1-yl)phenyl)amino)-2-oxoethyl)acetamido)-3-(1-cyclopropyl-1*H*-pyrazol-3-yl)propanoate

*Tert*-butyl 2-((2-((5-chloro-2-(4-chloro-1*H*-1,2,3-triazol-1-yl)phenyl)amino)-2-oxoethyl)amino)-3-(1-cyclopropyl-1*H*-pyrazol-3-yl)propanoate 123g (600 mg, 1.15 mmol) was dissolved in dichloromethane (10 mL), added with triethylamine (583.33 mg, 5.76 mmol, 801.27 μL), slowly dropwise added with chloroacetyl chloride (156.26 mg, 1.38 mmol, 110.04 μL) in ice bath, and reacted for 1 hour in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(1-cyclopropyl-1*H*-pyrazol-3-yl)propanoate 123h (688 mg), which was directly used for the next reaction.
MS m/z (ESI): 596.1 [M+H]

Step 8

Tert-butyl 2-(4-(5-chloro-2-(4-chloro-1*H*-1,2,3-triazol-1-yl)phenyl)-2,5-dioxopiperazin-1-yl)-3-(1-cyclopropyl-1*H*-pyrazol-3-yl)propanoate

The crude product of *tert*-butyl 2-(2-chloro-*N*-(2-((5-chloro-2-(4-chloro-1*H*-1,2,3-triazol-1-yl)phenyl)amino)-2-oxoethyl)acetamido)-3-(1-cyclopropyl-1*H*-pyrazol-3-yl)propanoate 123h (688 mg, 1.15 mmol) obtained in the last step was dissolved in methanol (10 mL), slowly dropwise added with sodium methylate (5.4M methanol solution, 234.80 μL, 1.27 mmol) at 0°C, and continuously reacted at 0°C for 1 hour. The reaction solution was dropwise added with 2M dilute hydrochloric acid to adjust the pH to 7, and concentrated under reduced pressure. The obtained residue was further separated and purified by silicagel column chromatography (eluent: system A) to obtain *tert*-butyl 2-(4-(5-chloro-2-(4-chloro-1*H*-1,2,3-triazol-1-yl)phenyl)-2,5-dioxopiperazin-1-yl)-3-(1-cyclopropyl-1*H*-pyrazole-3-yl)propanoate 123i (362 mg) with a yield of 56%.
MS m/z (ESI): 560.2 [M+H]

Step 9

2-(4-(5-chloro-2-(4-chloro-1*H*-1,2,3-triazol-1-yl)phenyl)-2,5-dioxopiperazin-1-yl)-3-(1-cyclopropyl-1*H*-pyrazole-3-yl)propanoic acid

*Tert*-butyl 2-(4-(5-chloro-2-(4-chloro-1*H*-1,2,3-triazol-1-yl)phenyl)-2,5-dioxopiperazin-1-yl)-3-(1-cyclopropyl-1*H*-pyrazol-3-yl)propanoate 123i (362 mg, 645.93 μmol) was dissolved in dichloromethane (10 mL), slowly dropwise added with trifluoroacetic acid (1 mL), and reacted at room temperature for 2 hours. After the reaction was completed, the reaction solution was concentrated under reduced pressure to obtain crude product of 2-(4-(5-chloro-2-(4-chloro-1*H*-1,2,3-triazol-1-yl)phenyl)-2,5-dioxopiperazin-1-yl)-3-(1-cyclopropyl-1*H*-pyrazole-3-yl)propanoic acid 123j (352 mg), which was directly used for the next reaction.
MS m/z (ESI): 504.1 [M+H]

Step 10

2-(4-(5-chloro-2-(4-chloro-1*H*-1,2,3-triazol-1-yl)phenyl)-2,5-dioxopiperazin-1-yl)-3-(1-cyclopropyl-1*H*-pyrazol-3-yl)-*N*-(2-methyl-2*H*-indazol-5-yl)propanamide

The crude product of 2-(4-(5-chloro-2-(4-chloro-1*H*-1,2,3-triazol-1-yl)phenyl)-2,5-dioxopiperazin-1-yl)-3-(1-cyclopropyl-1H-pyrazol-3-yl)propanoic acid 123j (325 mg, 644.43 μmol) and 2-methyl-2*H*-indazol-5-amine 19a (142.27 mg, 966.64 μmol) were dissolved in ethyl acetate (10 mL), added with propylphosphonic anhydride (50% ethyl acetate solution, 820.9 mg, 1.29 mmol) and N,N-diisopropylethylamine ((333.14 mg, 2.58 mmol, 426.01 μL), and reacted at 60°C for 1 hour. After the reaction was completed, the reaction solution was concentrated under reduced pressure. The obtained residue was further separated and purified by silicagel column chromatography (eluent: system B) to obtain

2-(4-(5-chloro-2-(4-chloro-1*H*-1,2,3-triazol-1-yl)phenyl)-2,5-dioxopiperazin-1-yl)-3-(1-cyclopropyl-1*H*-pyrazol-3-yl)-*N*-(2-methyl-2*H*-indazol-5-yl)propanamide 123 (178 mg) with a yield of 43%.

MS m/z (ESI): 633.2 [M+H]
$^1H$ NMR (400 MHz, DMSO-$d_6$) δ 10.14 (s, 1H), 8.69 (s, 1H), 8.25 (s, 1H), 8.12 (s, 1H), 7.91-7.59 (m, 4H), 7.54 (d, J = 9.1 Hz, 1H), 7.27 (d, J = 9.1 Hz, 1H), 6.05 (s, 1H), 5.30 (dd, J = 10.3, 5.6 Hz, 1H), 4.46-3.97 (m, 5H), 4.13 (s, 3H), 3.22-3.10 (m, 1H), 3.09-2.98 (m, 1H), 1.04-0.81 (m, 4H).

Examples 124-126 were synthesized according to the methods for synthesizing of Examples 1-47 of the present invention. The spectrum parameters of Examples 124-126 were shown in the following table:

| No. and structure of example | MS m/z (ESI): | $^1H$ NMR (400 MHz, DMSO-$d_6$) |
|---|---|---|
| 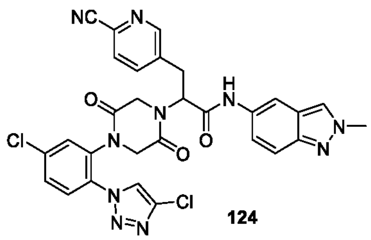 124 | 629.1 [M+H] | δ 10.12 (s, 1H), 8.71 (s, 1H), 8.66 (s, 1H), 8.26 (s, 1H), 8.07 (s, 1H), 8.02-7.91 (m, 2H), 7.83-7.65 (m, 3H), 7.55 (d, J = 9.2 Hz, 1H), 7.22 (dd, J = 9.2, 2.0 Hz, 1H), 5.43 (dd, J = 10.0, 5.8 Hz, 1H), 4.51-3.99 (m, 4H), 4.13 (s, 3H), 3.36-3.18 (m, 2H). |
| 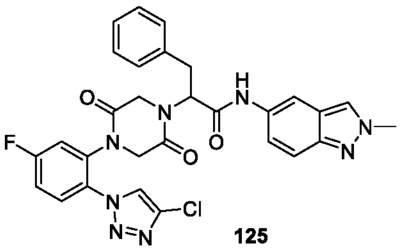 125 | 587.2 [M+H] | δ 10.12 (s, 1H), 8.63 (s, 1H), 8.26 (s, 1H), 8.11 (s, 1H), 7.77 (dd, J = 9.0, 5.5 Hz, 1H), 7.61-7.45 (m, 3H), 7.36-7.19 (m, 6H), 5.39 (dd, J = 9.9, 6.0 Hz, 1H), 4.40-3.92 (m, 4H), 4.13 (s, 3H), 3.29-3.23 (m, 1H), 3.16-3.03 (m, 1H). |
| 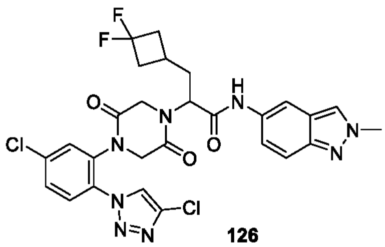 126 | 616.8 [M+H] | δ 10.11 (s, 1H), 8.74 (s, 1H), 8.25 (s, 1H), 8.08 (s, 1H), 7.86 (d, J = 2.0 Hz, 1H), 7.81-7.70 (m, 2H), 7.55 (d, J = 9.2 Hz, 1H), 7.26 (dd, J = 9.2, 1.9 Hz, 1H), 5.10-4.98 (m, 1H), 4.73-3.90 (m, 4H), 4.13 (s, 3H), 2.81-2.55 (m, 2H), 2.46-2.21 (m, 2H), 2.18-1.87 (m, 3H). |

Example 131

2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)-*N*-(2-methyl-2*H*-indazol-5-yl)propanamide

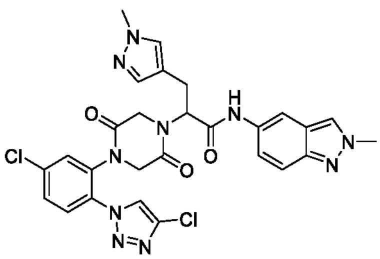

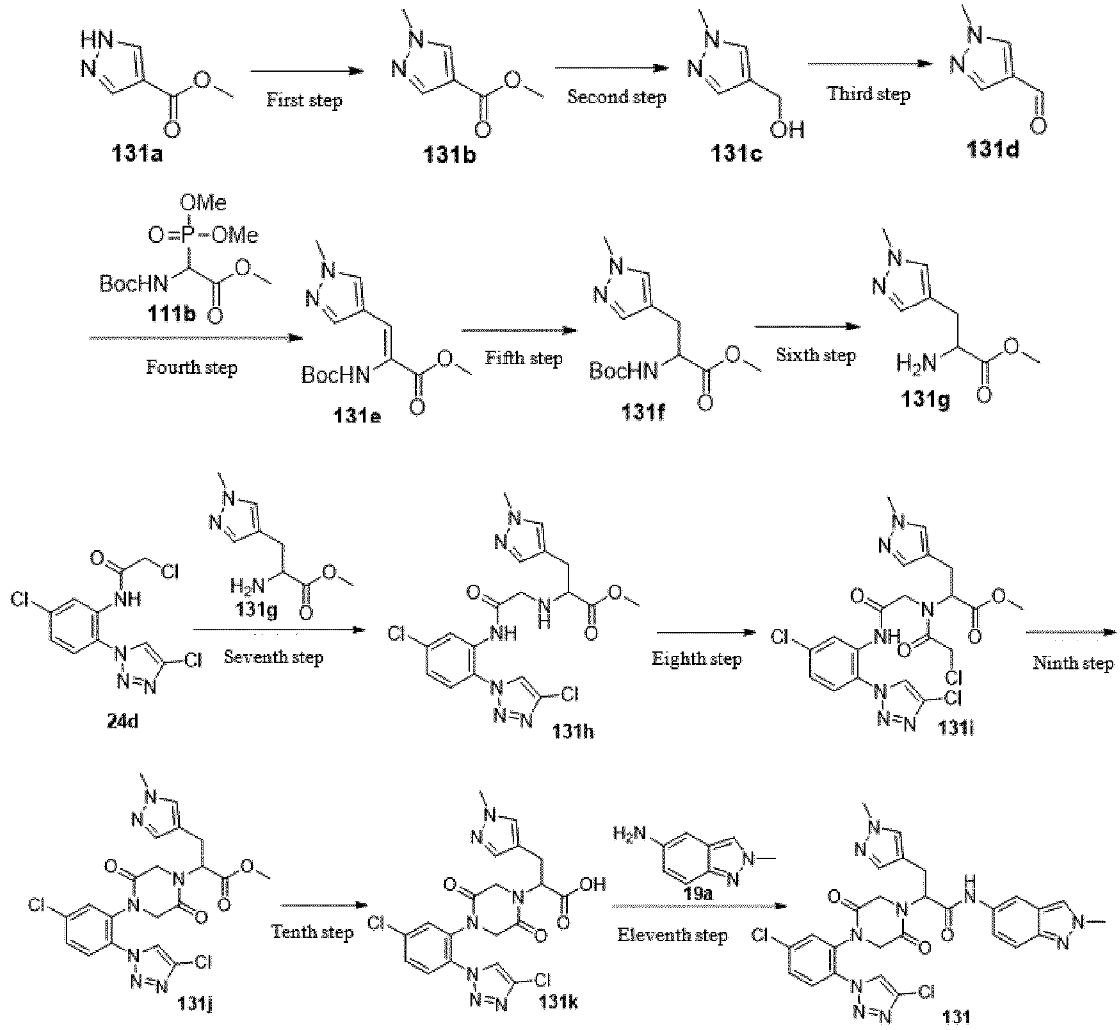


Step 1

Methyl 1-methyl-1*H*-pyrazole-4-carboxylate

Methyl pyrazole-4-carboxylate 131a (5 g, 39.65 mmol) was dissolved in N,N-dimethylformamide (50 mL), added with potassium carbonate (10.96 g, 79.29 mmol) and methyl iodide (6.19 g, 43.61 mmol) in turn, and stirred at room temperature for reaction for 1 hour. The reaction solution was added with water (50 mL), and extracted with ethyl acetate (50 mL×3). Organic phases were combined, washed with water (50 mL) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain methyl 1-methyl-1*H*-pyrazole-4-carboxylate 131b (5.04 g) with a yield of 90%.
MS m/z (ESI): 141.1 [M+H]

Step 2

(1-methyl-1*H*-pyrazol-4-yl)methanol

Methyl 1-methyl-1*H*-pyrazol-4-carboxylate 131b (5.04 g, 35.93 mmol) was dissolved in tetrahydrofuran (50 mL), added with lithium aluminium hydride (1.50 g, 39.53 mmol) at 0°C, and stirred at 0°C for reaction for 1 hour. The reaction solution was slowly poured into ether (100 mL) dissolved with sodium sulfafe decahydrate (30 g), added with a small amount of water (2 mL), and continuously stirred for half an hour until the upper solution was clear. The reaction solution was filtered and the filtrate was concentrated under reduced pressure to obtain crude product of (1-methyl-1*H*-pyrazol-4-yl)methanol 131c (3 g), which was directly used for the next reaction with a yield of 75%.
MS m/z (ESI): 113.1 [M+H]

Step 3

1-methyl-1*H*-pyrazole-4-carbaldehyde

(1-methyl-1*H*-pyrazol-4-yl)methanol 131c (3 g, 26.75 mmol) was dissolved in dichloromethane (5 mL), stirred for 10 minutes in ice bath, added with Dess-Martin periodinane (11.24 g, 26.50 mmol), then the ice bath was removed, and the reaction solution was stirred at room temperature and reacted for 2 hours. The reaction solution was filtered and concentrated under reduced pressure, and the obtained residue was further separated and purified by silicagel column chromatography (eluent: system A) to obtain 1-methyl-1*H*-pyrazole-4-carbaldehyde 131d (2.6 g) with a yield of 33%.
MS m/z (ESI): 111.0 [M+H]

Step 4

Methyl (*Z*)-2-((*tert*-butoxycarbonyl)amino)-3-(1-methyl-1H-pyrazol-4-yl)acrylate

(+/-)-Boc-alpha-phosphonoglycine trimethyles 111b (7.72 g, 25.97 mmol) was dissolved in dichloromethane (50 mL), stirred for 10 minutes, added with 1-methyl-1*H*-pyrazole-4-carbaldehyde131d (2.6 g, 23.61 mmol) and caesium carbonate (15.39 g, 47.22 mmol), and stirred at room temperature for reaction for 1 hour. After the reaction was completed, the reaction solution was added with water (50 mL) for quenching the reaction, and then extracted with dichloromethane (100 mL×2). Organic phases were combined and then washed with water (50 mL×2) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain methyl (*Z*)-2-((*tert*-butoxycarbonyl)amino)-3-(1-methyl-1*H*-pyrazol-4-yl) acrylate 131e (1.5 g) with a yield of 23%.
MS m/z (ESI): 282.0 [M+H]

Step 5

Methyl 2-((*tert*-butoxycarbonyl)amino)-3-(1-methyl-1*H*-pyrazol-4-yl)propanoate

Methyl (*Z*)-2-((*tert*-butoxycarbonyl)amino)-3-(1-methyl-1*H*-pyrazol-4-yl) acrylate 131e (1.5 g, 5.33 mmol) was dissolved in methanol (20 mL), added with 10% palladium carbon (567.46 mg, 5.33 mmol, 10%), and stirred at 50°C for reaction for 25 hours in hydrogen atmosphere. After the reaction was completed, the reaction solution was concentrated under reduced pressure to obtain crude product of methyl 2-((*tert*-butoxycarbonyl)amino)-3-(1-methyl-1*H*-pyrazol-4-yl)propanoate 131f (1.2 g), which was directly used for the next reaction, with a yield of 79%.
MS m/z (ESI): 284.0 [M+H]

Step 6

Methyl 2-amino-3-(1-methyl-1*H*-pyrazol-4-yl)propanoate

Methyl 2-((*tert*-butoxycarbonyl)amino)-3-(1-methyl-1*H*-pyrazol-4-yl)propanoate 131f (1.5 g, 5.29 mmol) was dissolved in dichloromethane (20 mL), slowly dropwise added with trifluoroacetic acid (2 mL), and stirred at room temperature for reaction for 1 hour. After the reaction was completed, the reaction solution was concentrated under reduced pressure to obtain crude product of methyl 2-amino-3-(1-methyl-1*H*-pyrazol-4-yl)propanoate 131g (0.7 g) with a yield of 72%.
MS m/z (ESI): 184.1 [M+H]

Step 7

Methyl 2-((2-((5-chloro-2-(4-chloro-1*H*-1,2,3-triazol-1-yl)phenyl)amino)-2-oxoethyl)amino)-3-(1-methyl-1*H*-pyrazol-4-yl)propanoate

2-Chloro-*N*-(5-chloro-2-(4-chloro-1*H*-1,2,3-triazol-1-yl)phenyl)acetamide 24d (300 mg, 981.84 μmol) and methyl 2-amino-3-(1-methyl-1*H*-pyrazol-4-yl)propanoate 131g (269.82 mg, 1.47 mmol) were dissolved in N,N-dimethylformamide (20 mL), added with N,N-diisopropylethylamine (708.20 mg, 4.91 mmol, 905.63 μL), and reacted at 80°C for 12 hours. After the reaction was completed, the reaction solution was added with water (40 mL) for quenching the reaction, and then extracted with ethyl acetate (40 mL×3). Organic phases were combined and then washed with water

(40 mL) and saturated sodium chloride solution (40 mL) in turn, dried with anhydrous sodium sulfate, filtered, and concentrated under reduced pressure. The obtained residue was further separated and purified by silicagel column chromatography (eluent: system A) to obtain methyl 2-((2-((5-chloro-2-(4-chloro-1*H*-1,2,3-triazol-1-yl)phenyl)amino)-2-oxoethyl)amino)-3-(1-methyl-1*H*-pyrazol-4-yl)propanoate 131h (150 mg) with a yield of 34%.
MS m/z (ESI): 452.1 [M+H]

Step 8

Methyl 2-(2-chloro-*N*-(2-((5-chloro-2-(4-chloro-1*H*-1,2,3-triazol-1-yl)phenyl)amino)-2-oxoethyl)acetamido)-3-(1-methyl-1*H*-pyrazol-4-yl)propanoate

Methyl 2-((2-((5-chloro-2-(4-chloro-1*H*-1,2,3-triazol-1-yl)phenyl)amino)-2-oxoethyl)amino)-3-(1-methyl-1*H*-pyrazol-4-yl)propanoate 131h (150 mg, 331.64 μmol) was dissolved in dichloromethane (10 mL), added with triethylamine (100.68 mg, 994.93 μmol, 138.30 μL), slowly dropwise added with chloroacetyl chloride (44.95 mg, 397.97 μmol, 31.68 μL) in ice bath, and reacted for 1 hour in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of methyl 2-(2-chloro-*N*-(2-((5-chloro-2-(4-chloro-1*H*-1,2,3-triazol-1-yl)phenyl)amino)-2-oxoethyl)acetamido)-3-(1-methyl-1*H*-pyrazol-4-yl)propanoate 131i (150 mg), which was directly used for the next reaction.
MS m/z (ESI): 528.1 [M+H]

Step 9

Methyl 2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)propanoate

The crude product of methyl 2-(2-chloro-*N*-(2-((5-chloro-2-(4-chloro-1*H*-1,2,3-triazol-1-yl)phenyl)amino)-2-oxoethyl)acetamido)-3-(1-methyl-1*H*-pyrazol-4-yl)propanoate 131i (150 mg, 283.67 μmol) obtained in last step was dissolved in methanol (50 mL), slowly dropwise added with sodium methylate (5.4M methanol solution, 157.60 μL, 851.02 μmol) at 0°C, and continuously reacted for 2 hours at 0°C. The reaction solution was dropwise added with 2M dilute hydrochloric acid to adjust the pH to 7, and concentrated under reduced pressure. The obtained residue was further separated and purified by silicagel column chromatography (eluent: system A) to obtain methyl 2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)propanoate 131j (130 mg) with a yield of 93%.
MS m/z (ESI): 492.1 [M+H]

Step 10

2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)propanoic acid

Methyl 2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)propanoate 131j (130 mg, 264.06 μmol) was dissolved in mixed solvent of tetrahydrofuran (10 mL), methanol (3 mL) and water (3 mL), added with mixed solvent of methanol (3 mL) and water (3 mL), added with lithium hydroxide monohydrate (33.24 mg, 792.18 μmol), and reacted at 0°C for 2 hours. After the reaction was completed, the reaction solution was dropwise added with 2M dilute hydrochloric acid to neutralize the reaction solution, and then the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)propanoic acid 131k (100 mg) with a yield of 79%.
MS m/z (ESI): 478.1 [M+H]

Step 11

2-(4-(5-chloro-2-(4-chloro-1*H*-1,2,3-triazol-1-yl)phenyl)-2,5-dioxopiperazin-1-yl)-3-(1-methyl-1*H* pyrazol-4-yl)-*N*-(2-methyl-2*H*-indazol-5-yl)propanamide

2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)propanoic acid 131k (100 mg, 209.08 μmol) and 2-methyl-2*H*-indazol-5-amine 19a (46.16 mg, 313.62 μmol) were

dissolved in ethyl acetate (20 mL), added with propylphosphonic anhydride (50% ethyl acetate solution, 266.10 mg, 418.16 μmol) and N,N-diisopropylethylamine (135.11 mg, 1.045 mmol, 172.77 μL), and reacted at 25°C for 3 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(1-methyl-1*H*-pyrazol-4-yl)-*N*-(2-methyl-2*H*-indazol-5-yl)propanamide 131 (1.5 mg) with a yield of 1%.
MS m/z (ESI): 607.1 [M+H]

Example 132

2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol -3-yl)-*N*-(2-methyl-2*H*-indazol-5-yl)propanamide

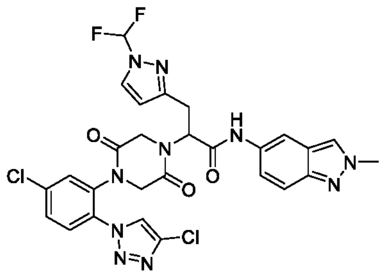

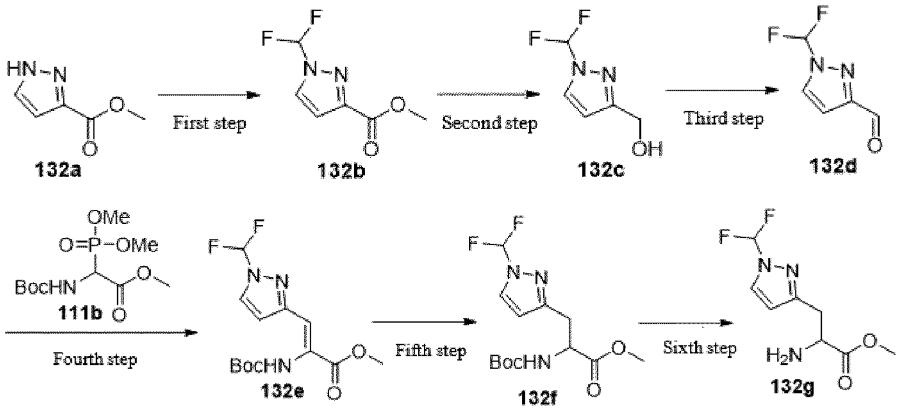

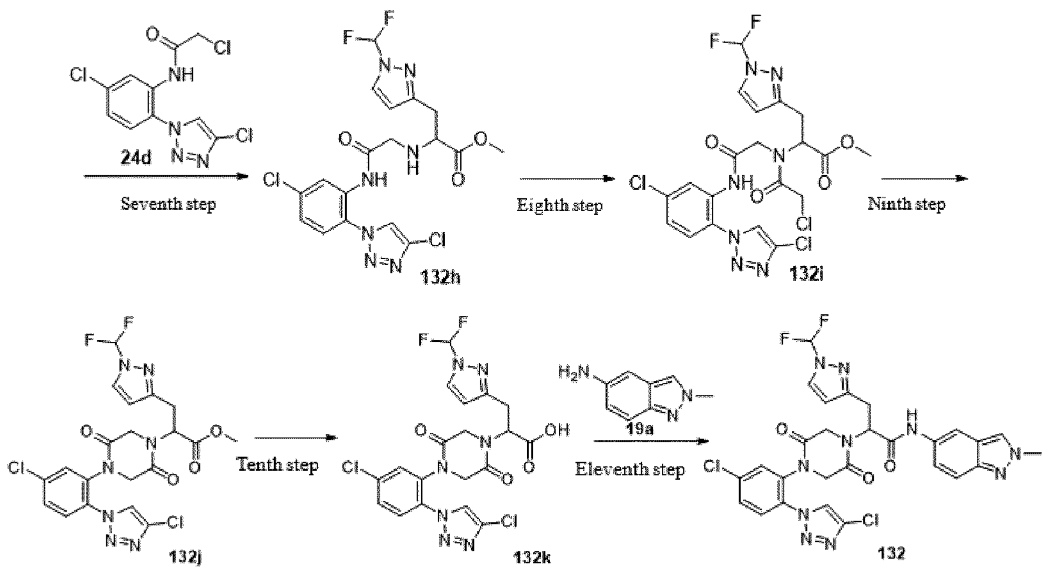


Step 1

Methyl 1-(difluoromethyl)-1*H*-pyrazol-3-carboxylate

Methyl 1*H*-pyrazole-3-carboxylate 132a (10 g, 79.29 mmol) was dissolved in N,N-dimethylacetamide (10 mL), added with caesium carbonate (51.67 g, 158.58 mmol) and sodium difluorochloroacetate (15.72 g, 103.08 mmol) in turn, and reacted at 100°C for 5 hours. The reaction solution was added with water (50 mL), and extracted with ethyl acetate (50 mL×3). Organic phases were combined, washed with water (50 mL) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain methyl 1-(difluoromethyl)-1*H*-pyrazole-3-carboxylate 132b (6.5 g) with a yield of 47%.
MS m/z (ESI): 177.0 [M+H]

Step 2

(1-(difluoromethyl)-1*H*-pyrazol-3-yl)methanol

Methyl 1-(difluoromethyl)-1*H*-pyrazole-3-carboxylate 132b (8 g, 45.42 mmol) was dissolved in tetrahydrofuran (50 mL), added with lithium aluminium hydride (3.44 g, 90.85 mmol) at 0°C, and stirred at 0°C for reaction for 1 hour. The reaction solution was slowly poured into ether (150 mL) dissolved with sodium sulfafe decahydrate (50 g), added with a small amount of water (5 mL), and continuously stirred for half an hour until the upper solution was clear. The reaction solution was filtered and concentrated under reduced pressure to obtain crude product of (1-(difluoromethyl)-1*H*-pyrazol-3-yl)methanol 132c (6 g), which was directly used for the next reaction, with a yield of 89%. MS m/z (ESI): 149.0 [M+H]

Step 3

1-(difluoromethyl)-1*H*-pyrazole-3-carbaldehyde

(1-(difluoromethyl)-1*H*-pyrazole-3-yl)methanol 132c (2 g, 13.50 mmol) was dissolved in dichloromethane (30 mL), stirred for 10 minutes in ice bath, added with Dess-Martin periodinane (8.59 g, 20.26 mmol), then the ice bath was removed, and the reaction solution was stirred at room temperature and reacted for 2 hours. The reaction solution was filtered and concentrated under reduced pressure, and the obtained residue was further separated and purified by silicagel column chromatography (eluent: system A) to obtain 1-(difluoromethyl)-1*H*-pyrazole-3-carbaldehyde 132d (1.6 g) with a yield of 81%.
MS m/z (ESI): 147.0 [M+H]

Step 4

Methyl (*Z*)-2-((*tert*-butoxycarbonyl)amino)-3-(1-(difluoromethyl)-1*H*-pyrazol-3-yl) acrylate

(+/-)-Boc-alpha-phosphonoglycine trimethyles 111b (4.15 g, 13.96 mmol) was dissolved in N,N-dimethylformamide (30 mL), stirred for 10 minutes, added with 1-(difluoromethyl)-1*H*-pyrazole-3-carbaldehyde 132d (1.7 g, 11.64 mmol) and caesium carbonate (7.58 g, 23.27 mmol), and stirred at room temperature for reaction for 1 hour. After the reaction was completed, the reaction solution was added with water (50 mL) for quenching the reaction, and then extracted with ethyl acetate (100 mL×2). Organic phases were combined and then washed with water (50 mL×2) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain methyl (*Z*)-2-((*tert*-butoxycarbonyl)amino)-3-(1-(difluoromethyl)-1*H*-pyrazol-3-yl) acrylate 132e (2.5 g) with a yield of 68%.
MS m/z (ESI): 218.2 [M+H-100]

Step 5

Methyl 2-((*tert*-butoxycarbonyl)amino)-3-(1-(difluoromethyl)-1*H*-pyrazol-3-yl) propanoate

Methyl (*Z*)-2-((*tert*-butoxycarbonyl)amino)-3-(1-(difluoromethyl)-1*H*-pyrazol-3-yl) acrylate 132e (2.3 g, 7.25 mmol) was dissolved in methanol (15 mL), added with 10% palladium carbon ((146.43 mg, 72.49 mmol, 10%), and stirred at room temperature for reaction for 1 hour in hydrogen atmosphere. After the reaction was completed, the reaction solution was concentrated under reduced pressure to obtain crude product of methyl 2-((*tert*-butoxycarbonyl)amino)-3-(1-(difluoromethyl)-1*H*-pyrazol-3-yl)propanoate 132f (2.1 g), which was directly used for the next reaction, with a yield of 91%.
MS m/z (ESI): 220.1 [M+H-100]

Step 6

Methyl 2-amino-3-(1-(difluoromethyl)-1*H*-pyrazol-3-yl)propanoate

Methyl 2-((*tert*-butoxycarbonyl)amino)-3-(1-(difluoromethyl)-1*H*-pyrazol-3-yl)propanoate 132f (2.1 g, 6.58 mmol) was dissolved in dichloromethane (20 mL), added with trifluoroacetic acid (2 mL), stirred at room temperature, and reacted for 2 hours. After the reaction was completed, the reaction solution was concentrated under reduced pressure to obtain crude product of methyl 2-amino-3-(1-(difluoromethyl)-1*H*-pyrazol-3-yl) propanoate 132g (1.4 g) with a yield of 97%.
MS m/z (ESI): 220.1 [M+H]

Step 7

Methyl 2-((2-((5-chloro-2-(4-chloro-1*H*-1,2,3-triazol-1-yl)phenyl)amino)-2-oxoethyl)amino)-3-(1-(difluoromethyl)-1*H*-pyrazol-3-yl)propanaoate

2-chloro-*N*-(5-chloro-2-(4-chloro-1*H*-1,2,3-triazol-1-yl)phenyl)acetamide 24d (500 mg, 1.64 mmol) and methyl 2-amino-3-(1-(difluoromethyl)-1*H*-pyrazol-3-yl)propanoate 132g (430.42 mg, 1.96 mmol) were dissolved in N,N-dimethylformamide (20 mL), added with N,N-diisopropylethylamine (708.20 mg, 4.91 mmol, 905.63 μL), and reacted at 80°C for 12 hours. The reaction solution was added with water (40 mL) for quenching the reaction, extracted with ethyl acetate (40 mL×3), and then washed with water (40 mL) and saturated sodium chloride solution (40 mL), dried with anhydrous sodium sulfate, filtered, and concentrated under reduced pressure. The obtained residue was further separated and purified by silicagel column chromatography (eluent: system A) to obtain methyl 2-((2-((5-chloro-2-(4-chloro-1*H*-1,2,3-triazol-1-yl)phenyl)amino)-2-oxoethyl)amino)-3-(1-(difluoromethyl)-1*H*-pyrazol-3-yl)propanoate 132h (300 mg) with a yield of 37%.
MS m/z (ESI): 488.1 [M+H]

Step 8

Methyl 2-(2-chloro-*N*-(2-((5-chloro-2-(4-chloro-1*H*-1,2,3-triazol-1-yl)phenyl)amino)-2-oxoethyl)acetamido)-3-(1-(difluoromethyl)-1*H*-pyrazol-3-yl)propanoate

Methyl 2-((2-((5-chloro-2-(4-chloro-1*H*-1,2,3-triazol-1-yl)phenyl)amino)-2-oxoethyl)amino)-3-(1-(difluoromethyl)-1*H*-pyrazol-3-yl)propanoate 132h (300 mg, 614.41 μmol) was dissolved in dichloromethane (30 mL), added with triethylamine (238.22 mg, 1.84 mmol, 304.63 μL), slowly dropwise added with chloroacetyl chloride (104.09 mg, 921.61 μmol, 73.35 μL) in ice bath, and reacted for 2 hours in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of methyl 2-(2-chloro-*N*-(2-((5-chloro-2-(4-chloro-1*H*-1,2,3-triazol-1-yl)phenyl)amino)-2-oxoethyl)acetamido)-3-(1-(difluoromethyl)-1*H*-pyrazol-3-yl)propanoate 132i (200 mg), which was directly used for the next reaction.
MS m/z (ESI): 564.0 [M+H]

Step 9

Methyl 2-(4-(5-chloro-2-(4-chloro-1*H*-1,2,3-triazol-1-yl)phenyl)-2,5-dioxopiperazin-1-yl)-3-(1-(difluoromethyl-1*H*-pyrazol-3-yl)propanoate

The crude product of methyl 2-(2-chloro-*N*-(2-((5-chloro-2-(4-chloro-1*H*-1,2,3-triazol-1-yl)phenyl)amino)-2-oxoethyl)acetamido)-3-(1-(difluoromethyl)-1*H*-pyrazol-3-yl)propanoate 132i (200 mg, 354.13 μmol) obtained in the last step was dissolved in methanol (30 mL), slowly dropwise added with sodium methylate (5.4M methanol solution, 196.30 μL, 1.06 mmol) at 0°C, and continuously reacted for 3 hours at 0°C. The reaction solution was dropwise added with 2M dilute hydrochloric acid to adjust the pH to 7, and concentrated under reduced pressure. The obtained residue was further separated and purified by silicagel column chromatography (eluent: system A) to obtain methyl 2-(4-(5-chloro-2-(4-chloro-1*H*-1,2,3-triazol-1-yl)phenyl)-2,5-dioxopiperazin-1-yl)-3-(1-(difluoromethyl-1*H*-pyrazol-3-yl)propanoate 132j (100 mg) with a yield of 53%.
MS m/z (ESI): 528.1 [M+H]

Step 10

2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl)propanoic acid

Methyl 2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl)propanoate 132j (100 mg, 189.29 μmol) was dissolved in mixed solvent of tetrahydrofuran (10 mL), methanol (3 mL), and water (3 mL), added with lithium hydroxide monohydrate (39.72 mg, 946.44 μmol), and reacted at 0°C for 2 hours. After the reaction was completed, the reaction solution was added with 2M dilute hydrochloric acid for neutralization, and then concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl)propanoic acid 132k (90 mg), with a yield of 92%.
MS m/z (ESI): 514.1 [M+H]

Step 11

2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl)-*N*-(2-methyl-2*H*-indazol-5-yl)propanamide

2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl)propanoic acid 132k (100 mg, 194.45 μmol) and 2-methyl-2H-indazol-5-amine 19a (37.20 mg, 252.79 μmol) were dissolved in ethyl acetate (20 mL), added with propylphosphonic anhydride (50% ethyl acetate solution, 247.48 mg, 388.90 μmol) and N,N-diisopropylethylamine (75.39 mg, 583.35 μmol, 96.41 μL), and reacted at 60°C for 3 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(1-(difluoromethyl)-1*H*-pyrazol-3-yl)-*N*-(2-methyl-2*H*-indazol-5-yl)propanamide 132 (56 mg) with a yield of 45%.

MS m/z (ESI): 643.2 [M+H]

$^1$H NMR (400 MHz, DMSO-$d_6$) $\delta$ 10.16 (s, 1H), 8.70 (s, 1H), 8.26 (s, 1H), 8.14 (d, J = 2.6 Hz, 1H), 8.12 (s, 1H), 7.82 (s, 1H), 7.78-7.70 (m, 2H), 7.74 (d, J = 59.3 Hz, 1H), 7.55 (d, J = 9.1 Hz, 1H), 7.26 (dd, J = 9.2, 2.0 Hz, 1H), 6.38 (s, 1H), 5.36 (dd, J= 10.1, 5.7 Hz, 1H), 4.49-3.99 (m, 4H), 4.14 (s, 3H), 3.32-3.22 (m, 1H), 3.21-3.11 (m, 1H).

Example 133

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(1-(methyl-$d_3$)-1*H*-pyrazol-3-yl)propanamide

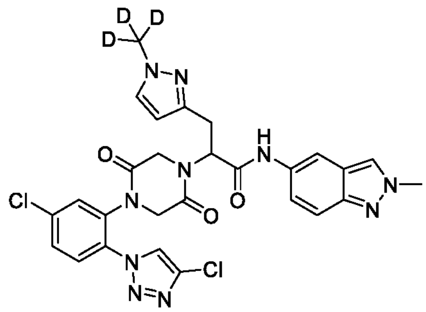

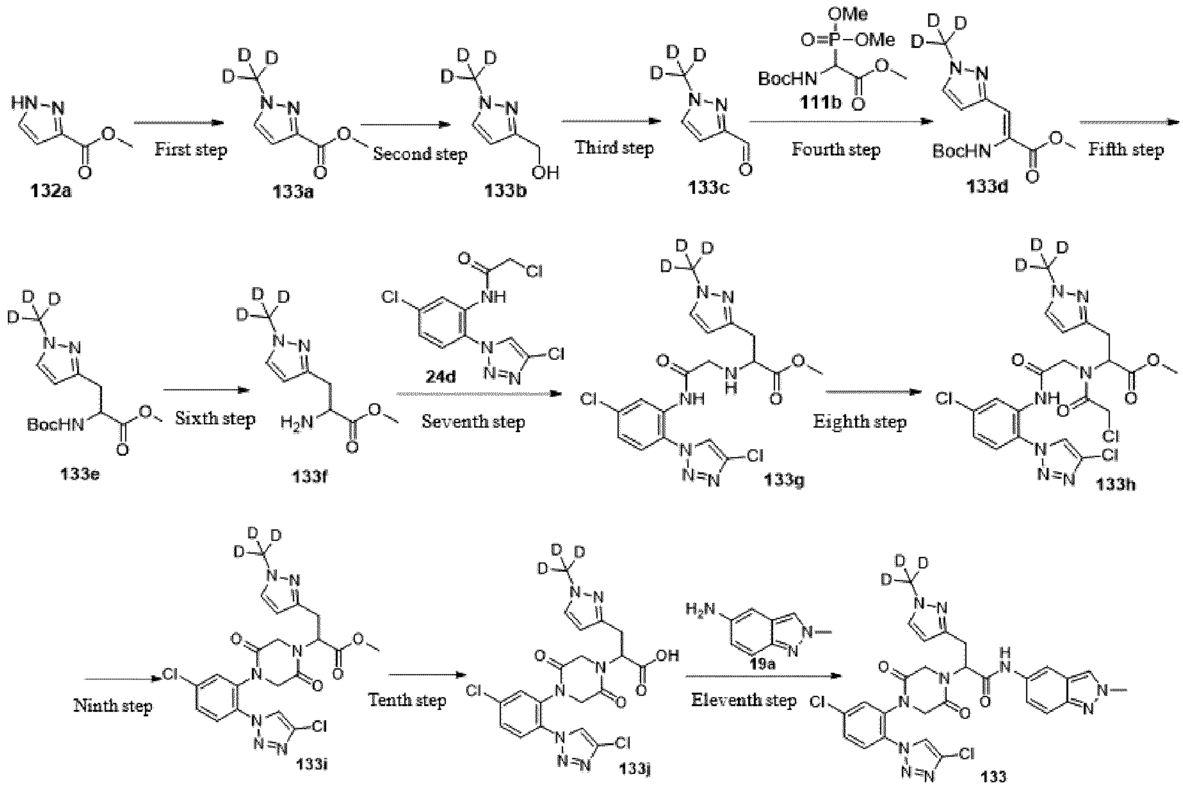


Step 1

Methyl 1-(methyl-$d_3$)-1*H*-pyrazole-3-carboxylate

Methyl 1*H*-pyrazole-3-carboxylate 132a (4. 0 g, 31.72 mmol) was dissolved in N,N-dimethylformamide (200 mL), added with caesium carbonate (20.67 g, 63.44 mmol) and iodomethane-$d_3$ 15.06 g, 34.89 mmol) in turn, and sitrred

at room temperature for reaction for 1 hour. The reaction solution was added with water (100 mL), and then extracted with ethyl acetate (100 mL×3). Organic phases were combined and then washed with water (100 mL) and saturated sodium chloride solution (100 mL) in turn, dried with anhydrous sodium sulfate, filtered, and concentrated under reduced pressure. The obtained residue was further separated and purified by silicagel column chromatography (eluent: system A) to obtain methyl 1-(methyl-*d*3)-1*H*-pyrazole-3-carboxylate 133a (1 g) with a yield of 22%.
MS m/z (ESI): 144.1 [M+H]

Step 2

(1-(methyl-$d_3$)-1*H*-pyrazole-3-yl)methanol

Raw materials methyl 1-(methyl-*d*3)-1*H*-pyrazole-3-carboxylate 133a (1 g, 6.99 mmol) was dissolved in tetrahydrofuran (50 mL), added with lithium aluminium hydride (291.22 mg, 7.68 mmol) at 0°C, and stirred at 0°C for reaction for 1 hour. The reaction solution was slowly poured into ether (100 mL) dissolved with sodium sulfafe decahydrate (30 g), added with a small amount of water (2 mL), and continuously stirred for half an hour until the upper solution was clear. The reaction solution was filtered and concentrated under reduced pressure to obtain crude product of (1 -(methyl-$d_3$)-1*H*-pyrazol-3-yl)methanol 133b (0.8 g), which was directly used for the next reaction, with ayield of 99%. MS m/z (ESI): 116.1 [M+H]

Step 3

1-(methyl-$d_3$)-1*H*-pyrazole-3-carbaldehyde

(1-(methyl-$d_3$)-1*H*-pyrazol-3-yl)methanol 133b (0.8 g, 6.95 mmol) was dissolved in dichloromethane (10 mL), stirred for 10 minutes in ice bath, added with Dess-Martin periodinane (2.95 g, 6.95 mmol), then the ice bath was removed, and the reaction solution was stirred at room temperature and reacted for 1 hour. The reaction solution was filtered and concentrated under reduced pressure, and the obtained residue was further separated and purified by silicagel column chromatography (eluent: system A) to obtain 1-(methyl-$d_3$)-1*H*-pyrazole-3-carbaldehyde 133c (0.6 g), with a yield of 76%.
MS m/z (ESI): 114.0 [M+H]

Step 4

Methyl (*Z*)-2-((*tert*-butoxycarbonyl)amino)-3-(1-(methyl-$d_3$)-1*H*-pyrazol-3-yl) acrylate

(+/-)-Boc-alpha-phosphonoglycine trimethyles 111b (1.58 g, 5.30 mmol) was dissolved in dichloromethane (30 mL), stirred for 10 minutes, added with 1-(methyl-$d_3$)-1*H*-pyrazole-3-carbaldehyde 133c (600 mg, 5.30 mmol) and caesium carbonate (3.46 g, 10.61 mmol), and stirred at room temperature for reaction for 1 hour. After the reaction was completed, the reaction solution was added with water (50 mL) for quenching the reaction, and then extracted with dichloromethane (50 mL×2). Organic phases were combined and then washed with water (50 mL×2) and saturated sodium chloride solution (50 mL) in turn, dried with anhydrous sodium sulfate, filtered, and concentrated under reduced pressure. The obtained residue was further separated and purified by silicagel column chromatography (eluent: system A) to obtain methyl (*Z*)-2-((*tert*-butoxycarbonyl)amino)-3-(1-(methyl-$d_3$)-1*H*-pyrazol-3-yl) acrylate 133d (1 g) with a yield of 66%.
MS m/z (ESI): 285.2 [M+H]

Step 5

Methyl 2-((*tert*-butoxycarbonyl)amino)-3-(1-(methyl-$d_3$)-1*H*-pyrazol-3-yl)propanoate

Methyl (*Z*)-2-((*tert*-butoxycarbonyl)amino)-3-(1-(methyl-$d_3$)-1*H*-pyrazol-3-yl) acrylate 133d (1 g, 3.52 mmol) was dissolved in methanol (20 mL), added with 10% palladium carbon (374.29 mg, 3.52 mmol, 10%), and stirred at 50°C for reaction for 5 hours in hydrogen atmosphere. After the reaction was completed, the reaction solution was concentrated under reduced pressure to obtain crude product of methyl 2-((*tert*-butoxycarbonyl)amino)-3-(1-(methyl-$d_3$)-1*H*-pyrazol-3-yl)propanoate 133e (0.8 g), which was directly used for the next reaction, with a yield of 80%.
MS m/z (ESI): 287.2 [M+H]

Step 6

Methyl 2-amino-3-(1-(methyl-$d_3$)-1*H*-pyrazol-3-yl)propanoate

Methyl 2-((*tert*-butoxycarbonyl)amino)-3-(1-(methyl-$d_3$)-1*H*-pyrazol-3-yl)propanoate 133e (800 mg, 2.79 mmol) was dissolved in dichloromethane (10 mL), slowly dropwise added with trifluoroacetic acid (1 mL), and stirred at room temperature for reaction for 1 hour. After the reaction was completed, the reaction solution was concentrated under reduced pressure to obtain crude product of methyl 2-amino-3-(1-(methyl-$d_3$)-1*H*-pyrazol-3-yl) propanoate 133f (400 mg) with a yield of 77%. MS m/z (ESI): 187.1 [M+H]

Step 7

Methyl 2-((2-((5-chloro-2-(4-chloro-1*H*-1,2,3-triazol-1-yl)phenyl)amino)-2-oxoethyl)amino)-3-(1-(methyl-$d_3$)-1*H*-pyrazol-3-yl)propanoate

2-Chloro-*N*-(5-chloro-2-(4-chloro-1*H*-1,2,3-triazol-1-yl)phenyl)acetamide 24d (400 mg, 1.31 mmol) and methyl 2-amino-3-(1-(methyl-$d_3$)-1*H*-pyrazol-3-yl)propanoate 133f (365.94 mg, 1.965 mmol) were dissolved in N,N-dimethylformamide (20 mL), added with N,N-diisopropylethylamine (944.27 mg, 6.55 mmol, 1.208 mL), and reacted at 80°C for 12 hours. The reaction solution was added with water (40 mL) for quenching the reaction, extracted with ethyl acetate (40 mL×3), and then washed with water (40 mL) and saturated sodium chloride solution (40 mL), dried with anhydrous sodium sulfate, filtered, and concentrated under reduced pressure. The obtained residue was further separated and purified by silicagel column chromatography (eluent: system A) to obtain methyl 2-((2-((5-chloro-2-(4-chloro-1*H*-1,2,3-triazol-1-yl)phenyl)amino)-2-oxoethyl)amino)-3-(1-(methyl-$d_3$)-1*H*-pyrazol-3-yl)propanoate 133g (140 mg) with a yield of 23%.
MS m/z (ESI): 455.1 [M+H]

Step 8

Methyl 2-(2-chloro-*N*-(2-((5-chloro-2-(4-chloro-1*H*-1,2,3-triazol-1-yl)phenyl)amino)-2-oxoethyl)acetamido)-3-(1-(methyl-$d_3$)-1*H*-pyrazol-3-yl)propanoate

Methyl 2-((2-((5-chloro-2-(4-chloro-1*H*-1,2,3-triazol-1-yl)phenyl)amino)-2-oxoethyl)amino)-3-(1-(methyl-$d_3$)-1*H*-pyrazol-3-yl)propanoate 133g (140 mg, 307.48 μmol) was dissolved in acetonitrile (10 mL), added with potassium carbonate (127.49 mg, 922.44 μmol), slowly dropwise added with chloroacetyl chloride (52.09 mg, 461.22 μmol, 36.73 μL) in ice bath, and reacted for 1 hour in ice bath. The reaction solution was added with water (30 mL) for quenching the reaction, and then extracted with dichloromethane (30 mL×3). Organic phases were combined, washed with saturated sodium chloride solution (50 mL), dried with anhydrous sodium sulfate, filtered and concentrated under reduced pressure to obtain crude product of methyl 2-(2-chloro-*N*-(2-((5-chloro-2-(4-chloro-1*H*-1,2,3-triazol-1-yl)phenyl)amino)-2-oxoethyl)acetamido)-3-(1-(methyl-$d_3$)-1*H*-pyrazol-3-yl)propanoate 133h (140 mg), which was directly used for the next reaction.
MS m/z (ESI): 531.1 [M+H]

Step 9

Methyl 2-(4-(5-chloro-2-(4-chloro-1*H*-1,2,3-triazol-1-yl)phenyl)-2,5-dioxopiperazin-1-yl)-3-(1-(methyl-$d_3$)-1*H* pyrazol-3-yl)propanoate

The crude product of methyl 2-(2-chloro-*N*-(2-((5-chloro-2-(4-chloro-1*H*-1,2,3-triazol-1-yl)phenyl)amino)-2-oxoethyl)acetamido)-3-(1-(methyl-$d_3$)-1*H*-pyrazol-3-yl)propanoate 133h (140 mg, 263.26 μmol) obtained in last step was dissolved in methanol (00 mL), slowly dropwise added with sodium methylate (5.4M methanol solution, 146.26 μL, 789.78 μmol) at 0°C, and continuously reacted for 2 hours at 0°C. The reaction solution was dropwise added with 2M dilute hydrochloric acid to adjust the pH to 7, and concentrated under reduced pressure. The obtained residue was further separated and purified by silicagel column chromatography (eluent: system A) to obtain methyl 2-(4-(5-chloro-2-(4-chloro-1*H*-1,2,3-triazol-1-yl)phenyl)-2,5-dioxopiperazin-1-yl)-3-(l-(methyl-$d_3$)-1*H* pyrazol-3-yl)propanoate 133i (120 mg) with a yield of 92%.
MS m/z (ESI): 495.1 [M+H]

Step 10

2-(4-(5-chloro-2-(4-chloro-1*H*-1,2,3-triazol-1-yl)phenyl)-2,5-dioxopiperazin-1-yl)-3-(1-(methyl-$d_3$)-1*H*-pyrazol-3-yl)propanoic acid

Methyl 2-(4-(5-chloro-2-(4-chloro-1*H*-1,2,3-triazol-1-yl)phenyl)-2,5-dioxopiperazin-1-yl)-3-(1-(methyl-$d_3$)-1*H*-pyrazol-3-yl)propanoate 133i (120 mg, 242.26 μmol) was dissolved in mixed solvent of tetrahydrofuran (10 mL), methanol (3 mL) and water (3 mL), added with lithium hydroxide monohydrate (30.50 mg, 726.78 μmol), and reacted at 0°C for 2 hours. After the reaction was completed, the reaction solution was added with 2M dilute hydrochloric acid for neutralization, and then concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-3-(1-(methyl-$d_3$)-1*H*-pyrazol-3-yl)propanoic acid 133j (100 mg), with a yield of 86%. MS m/z (ESI): 481.1 [M+H]

Step 11

2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(1-(methyl-$d_3$)-1*H*-pyrazol-3-yl)propanamide

2-(4-(5-Chloro-2-(4-chloro-1*H*-1,2,3-triazol-1-yl)phenyl)-2,5-dioxopiperazin-1-yl)-3-(1-(methyl-$d_3$)-1H-pyrazol-3-yl)propanoic acid 133j (100.63 mg, 209.08 μmol) and 2-methyl-2*H*-indazol-5-amine 19a (46.16 mg, 313.62 μmol) were dissolved in ethyl acetate (20 mL), added with propylphosphonic anhydride (50% ethyl acetate solution, 266.10 mg, 418.16 μmol) and N,N-diisopropylethylamine (135.11 mg, 1.045 mmol, 172.77 μL), and reacted at 25°C for 3 hours. After the reaction was completed, the reaction solution was concentrated under reduced pressure. The obtained residue was further separated and purified by silicagel column chromatography (eluent: system B) to obtain 2-(4-(5-chloro-2-(4-chloro-1*H*-1,2,3-triazol-1-yl)phenyl)-2,5-dioxopiperazin-1-yl)-*N*-(2-methyl-2*H*-indazol-5-yl)-3-(1-(methyl-$d_3$)-1*H*-pyrazol-3-yl)propanamide 133 (7 mg) with a yield of 5%.

MS m/z (ESI): 610.1 [M+H]

$^1$H NMR (400 MHz, DMSO-$d_6$) δ 10.18 (s, 1H), 8.71 (s, 1H), 8.27 (s, 1H), 8.24 (s, 1H), 8.11 (s, 1H), 7.86-7.70 (m, 2H), 7.58-7.51 (m, 1H), 7.32-7.18 (m, 2H), 6.04 (s, 1H), 5.39-2.31 (m, 1H), 4.46-3.94 (m, 4H), 4.13 (s, 3H), 3.31-3.14 (m, 2H).

Example 134

4-(2-(4-(5-chloro-2-(4-chloro-1*H*-1,2,3-triazol-1-yl)phenyl)-2,5-dioxopiperazin-1-yl)-3-(4-fluorophenyl)propanamido)-2-fluoro-*N*-methylbenzamide

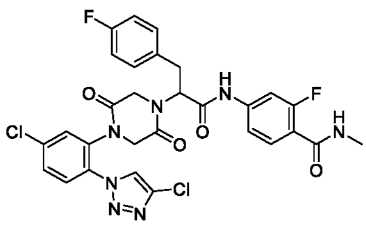

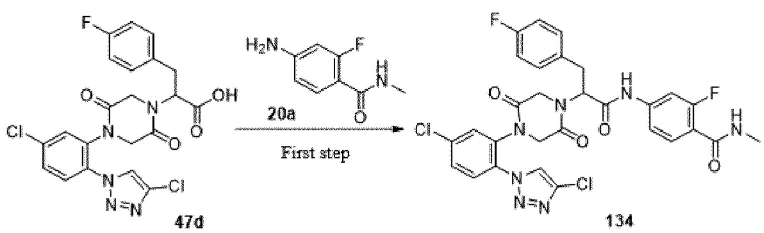


47d

134

2-(4-(5-chloro-2-(4-chloro-1*H*-1,2,3-triazol-1-yl)phenyl)-2,5-dioxopiperazin-1-yl)-3-(4-fluorophenyl)propanoic acid 47d (50 mg, 101.57 μmol) and 4-amino-2-fluoro-N-methylbenzamide 20a (25.62 mg, 152.35 μmol) were dissolved in ethyl acetate (10 mL), added with propylphosphonic anhydride (50% ethyl acetate solution, 258.53 mg, 406.27 μmol) and N,N-diisopropylethylamine (65.63 mg, 507.83 μmol, 83.93 μL), and reacted at 60°C for 4 hours. After the reaction was completed, the reaction solution was concentrated under reduced pressure. The obtained residue was further separated and purified by silicagel column chromatography (eluent: system B) to obtain 4-(2-(4-(5-chloro-2-(4-chloro-1*H*-1,2,3-triazol-1-yl)phenyl)-2,5-dioxopiperazin-1-yl)-3-(4-fluorophenyl)propanamido)-2-fluoro-*N*-methylbenzamide 134 (23 mg) with a yield of 35%.

MS m/z (ESI): 641.8 [M+H]

$^1$H NMR (400 MHz, DMSO-$d_6$) δ 10.54 (s, 1H), 8.64 (s, 1H), 8.08 (s, 1H), 7.84-7.56 (m, 5H), 7.40-7.25 (m, 3H), 7.20-7.07 (m, 2H), 5.32 (s, 1H), 4.60-3.89 (m, 4H), 3.28-3.05 (m, 2H), 2.77 (d, J = 4.5 Hz, 3H).

Example 135

4-(2-(4-(5-chloro-2-(4-chloro-1*H*-1,2,3-triazol-1-yl)phenyl)-2,5-dioxopiperazin-1-yl)-3-(4-fluorophenyl)propanamido)-2-fluorobenzamide

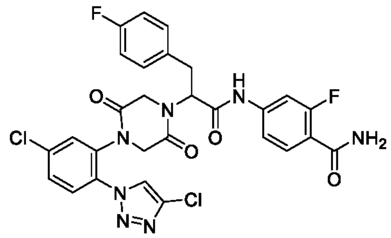

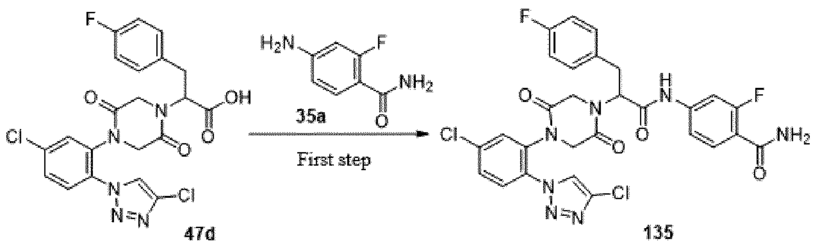


47d 135

2-(4-(5-chloro-2-(4-chloro-1*H*-1,2,3-triazol-1-yl)phenyl)-2,5-dioxopiperazin-1-yl)-3-(4-fluorophenyl)propanoic acid 47d (50 mg, 101.57 μmol) and 4-amino-2-fluorobenzamide 35a (23.48 mg, 152.35 μmol) were dissolved in ethyl acetate (10 mL), added with propylphosphonic anhydride (50% ethyl acetate solution, 258.53 mg, 406.27 μmol) and N,N-diisopropylethylamine (65.63 mg, 507.83 μmol, 83.93 μL), and reacted at room temperature for 4 hours. After the reaction was completed, the reaction solution was concentrated under reduced pressure. The obtained residue was further separated and purified by silicagel column chromatography (eluent: system B) to obtain 4-(2-(4-(5-chloro-2-(4-chloro-1*H*-1,2,3-triazol-1-yl)phenyl)-2,5-dioxopiperazin-1-yl)-3-(4-fluorophenyl)propanamido)-2-fluorobenzamide 135 (20 mg) with a yield of 31%.

MS m/z (ESI): 627.7 [M+H]

$^1$H NMR (400 MHz, DMSO-$d_6$) δ 10.55 (s, 1H), 8.65 (s, 1H), 7.85-7.60 (m, 5H), 7.60-7.48 (m, 2H), 7.41-7.25 (m, 3H), 7.20-7.07 (m, 2H), 5.32 (s, 1H), 4.55-3.89 (m, 4H), 3.29-3.05 (m, 2H).

Examples 136 and 137

(*R*)-4-(2-(4-(5-chloro-2-(4-chloro-1*H*-1,2,3-triazol-1-yl)phenyl)-2,5-dioxopiperazin-1-yl)-3-phenylpropanamido)-2-fluorobenzamide 136

(*S*)-4-(2-(4-(5-chloro-2-(4-chloro-1*H*-1,2,3-triazol-1-yl)phenyl)-2,5-dioxopiperazin-1-yl)-3-phenylpropanamido)-2-fluorobenzamide 137

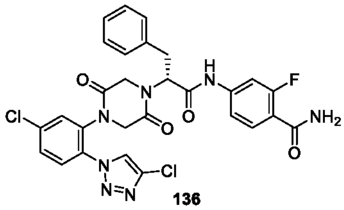
136

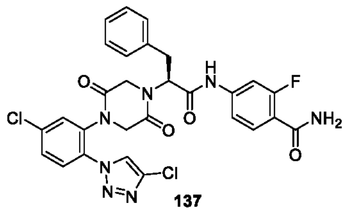
137

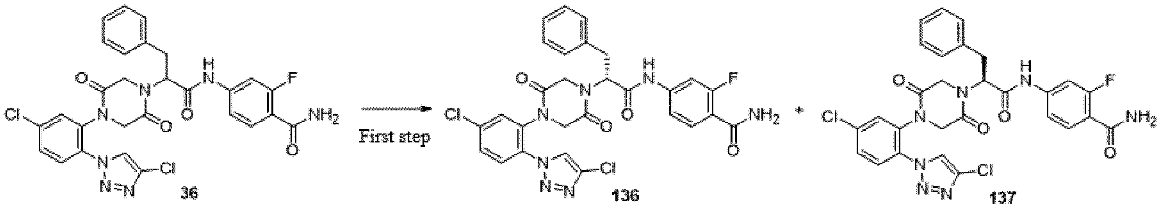

36 136 137

4-(2-(4-(5-chloro-2-(4-chloro-1*H*-1,2,3-triazol-1-yl)phenyl)-2,5-dioxopiperazin-1-yl)-3-phenylpropanamido)-2-fluorobenzamide 36 (80 mg, 0.131 mmol) was subjected to SFC chiral resolution (column model: ChiralPak AS, 250×30 mm I.D., 10 μm; mobile phase: A for $CO_2$ and B for ethanol (0.1%$NH_3 \cdot H_2O$); column pressure: 100 bar; flow rate: 60 mL/min; detection wavelength: 220 nm; column temperature: 38°C) to obtain (*R*)-4-(2-(4-(5-chloro-2-(4-chloro-1*H*-1,2,3-triazol-1-yl)phenyl)-2,5-dioxopiperazin-1-yl)-3-phenylpropanamido)-2-fluorobenzamide 136 (retention time ($T_R$): 1.750 min; 26 mg) and (*S*)-4-(2-(4-(5-chloro-2-(4-chloro-1*H*-1,2,3-triazol-1-yl)phenyl)-2,5-dioxopiperazin-1-yl)-3-phenylpropanamido)-2-fluorobenzamide 137 (retention time ($T_R$): 2.114 min; 30 mg).

**136**

MS m/z (ESI): 609.8 [M+H]
$^1$H NMR (400 MHz, DMSO-$d_6$) δ 10.55 (s, 1H), 8.64 (s, 1H), 7.80-7.61 (m, 5H), 7.61-7.46 (m, 2H), 7.42-7.18 (m, 6H), 5.41-5.28 (m, 1H), 4.47-3.92 (m, 4H), 3.30-3.22 (m, 1H), 3.18-3.05 (m, 1H).

**137**

MS m/z (ESI): 609.8 [M+H]
$^1$H NMR (400 MHz, DMSO-$d_6$) δ 10.55 (s, 1H), 8.63 (s, 1H), 7.86-7.61 (m, 5H), 7.61-7.45 (m, 2H), 7.39-7.16 (m, 6H), 5.41-5.26 (m, 1H), 4.46-3.89 (m, 4H), 3.30-3.21 (m, 1H), 3.19-3.04 (m, 1H).

Example138 was synthesized according to the methods for synthesizing of Examples 1-47 of the present invention. The spectrum parameters of Example 138 were shown in the following table:

| | | |
|---|---|---|
| 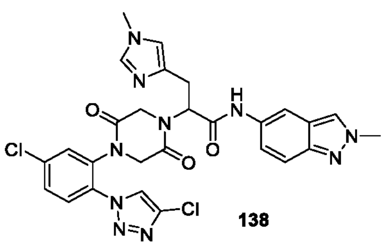 138 | 607.2 [M+H] | δ 10.24 (s, 1H), 8.70 (s, 1H), 8.25 (s, 1H), 8.13 (s, 1H), 7.86 (s, 1H), 7.79-7.70 (m, 2H), 7.59-7.49 (m, 2H), 7.28 (dd, J = 9.1, 2.0 Hz, 1H), 6.91 (s, 1H), 5.25 (dd, J = 9.9, 5.6 Hz, 1H), 4.55-4.00 (m, 4H), 4.13 (s, 3H), 3.63 (s, 3H), 3.16-3.06 (m, 1H), 3.03-2.92 (m, 1H). |

## Biological evaluation

### Test Example 1. Determination of the compound of the present invention on activity of Factor XIa protease

The following method was used to determine an inhibition degree of the compound of the present invention on an activity of Native human Factor XIa protease in vitro. In the method, a Factor XIa chromogenic substrate was used to test the inhibition degree on the activity of the Factor XIa protease. The Native human Factor XIa protease was purchased from Abcam (article number ab62411), and S-2366 was purchased from Chromogenix (article number 82109039).

An experimental process was briefly described as follows: a test compound was dissolved in DMSO to prepare a 10 mM storage solution first, then the compound was diluted with a Factor XIa reaction buffer (100 mM Tris-HCl, 200 mM NaCl, 0.02% Tween 20, pH 7.4) by a quadruple gradient, and a final concentration of the test compound in a reaction system ranged from 10,000 nM to 0.61 nM. The reaction was carried out in a 384-well microplate. 2 μL of the test compounds at different concentrations diluted with the reaction buffer were added to wells first, and 2 μL of 1% DMSO (a final concentration of DMSO in the reaction system was 0.1%) was used to replace blank and control wells. Subsequently, 8 μL of enzyme working solution (a final concentration of Factor XIa was 1.25 μg/uL) was added to each well, and 8 μL of Factor XIa reaction buffer was used to replace the blank well, centrifuged for 30 seconds, and incubated on ice for 5 minutes. Finally, 10 μL of substrate working solution (a final concentration of S-2366 was 1 mM) was added to each well, and centrifuged for 30 seconds, and the reaction was started. The solution was incubated at 37°C for 10 minutes to determine a light absorption value at 405 nm. By comparing with a light absorption value of the control group (0.1% DMSO), a percentage inhibition rate of the compound at each concentration was calculated, and nonlinear regression analysis was carried out on value-inhibition rate with the concentration of the compound by GraphPad Prism 5 software, so as to obtain an $IC_{50}$ value of the compound, as shown in Table 1.

Table 1 $IC_{50}$ data of the inhibition of the compounds of the present invention on activity of Factor XIa protease

| Number of compound | $IC_{50}$ (nM) |
|---|---|
| | Factor XIa |
| 2 | 44.9 |
| 3 | 36.7 |
| 4 | 49.8 |
| 5 | 29.9 |
| 6 | 34.6 |
| 7 | 15.5 |
| 8 | 17.7 |
| 9 | 7.8 |
| 10 | 43.9 |
| 11 | 24.9 |
| 12 | 22.8 |
| 13 | 14.9 |
| 14 | 44.0 |
| 15 | 25.1 |
| 17 | 34.8 |
| 18 | 20.5 |
| 19 | 8.5 |
| 20 | 30.5 |
| 23 | 43.2 |
| 24 | 23.2 |

(continued)

| Number of compound | $IC_{50}$ (nM) |
|---|---|
| | Factor XIa |
| 25 | 14.8 |
| 26 | 16.9 |
| 31 | 38.9 |
| 33 | 26.1 |
| 34 | 10.4 |
| 35 | 32.0 |
| 36 | 27.1 |
| 37 | 17.6 |
| 38 | 61.4 |
| 40 | 44.7 |
| 41 | 38.5 |
| 45 | 19.1 |
| 47 | 45.2 |
| 52 | 17.6 |
| 96 | 42.8 |
| 97 | 21.2 |
| 99 | 24.4 |
| 108 | 35.8 |
| 109 | 12.7 |
| 111 | 45.3 |
| 112 | 25.6 |
| 114 | 48.6 |
| 115 | 46.7 |
| 121 | 32.7 |
| 137 | 27.3 |

It could be seen from Table 1 that the compounds of the present invention all had a good inhibitory effect on the activity of the FXIa protease.

**Test Example 2. Determination of in-vitro anticoagulation of the compound of the present invention on human plasma**

The following method was used to determine the in-vitro anticoagulation of the compound of the present invention in the human plasma. In the method, an APTT kit of the MediRox company was used to test. The APTT kit was purchased from MediRox (article number MRX930), and the human plasma was prepared internally.

The preparation of the human plasma was briefly described as follows: human plasma was collected in a blood collection tube containing sodium citrate, and centrifuged at 3,000 rpm for 10 minutes at room temperature, and plasma was collected, and packaged separately and stored at -80°C.

An APTT test process was briefly described as follows: a test compound was dissolved in DMSO to prepare a 10 mM storage solution first, then the test compound was diluted with DMSO by a triple gradient, and a final concentration of the test compound in a reaction system ranged from 217 μM to 0.03 μM. 4 μL of test compounds at different concentrations diluted with DMSO were added to a centrifuge tube containing 180 μL of plasma, and 4 μL of DMSO was

added to a blank group, shaken and mixed evenly, and incubated at 37°C for 5 minutes. Subsequently, an APTT reagent was placed in a reagent holder according to instrument requirements, the incubated plasma sample was placed in a detection position of a coagulation analyzer, and a coagulation time was determined and recorded (s). With a final concentration of the compound as an abscissa and Ratio (Ratio=Ti/T0, wherein T0 was a coagulation time of a blank control, and Ti was a coagulation time of a compound to be tested) as an ordinate, a coagulation dose-effect curve was drawn by Graph Pad Prism5, and a concentration of the compound when the coagulation time was prolonged twice was calculated, which was namely a CT2 value, as shown in Table 2.

Table 2 CT2 data of in-vitro anticoagulation of the compounds of the present invention on human plasma

| No. of compound | CT2 (μM) |
|---|---|
| 2 | 12.6 |
| 3 | 6.6 |
| 5 | 17.8 |
| 6 | 4.9 |
| 7 | 9.8 |
| 9 | 2.9 |
| 11 | 8.1 |
| 12 | 4.4 |
| 13 | 4.0 |
| 14 | 14.3 |
| 15 | 9.9 |
| 16 | 15.6 |
| 17 | 11.2 |
| 18 | 7.9 |
| 19 | 3.0 |
| 20 | 8.6 |
| 22 | 16.0 |
| 25 | 6.6 |
| 26 | 7.0 |
| 29 | 6.7 |
| 31 | 13.7 |
| 32 | 6.3 |
| 33 | 18.5 |
| 34 | 5.9 |
| 35 | 5.8 |
| 36 | 15.9 |
| 37 | 7.5 |
| 38 | 19.0 |
| 39 | 12.7 |
| 41 | 8.5 |
| 42 | 16.3 |
| 43 | 19.6 |

(continued)

| No. of compound | CT2 (μM) |
|---|---|
| 45 | 11.3 |
| 46 | 19.1 |
| 47 | 9.3 |
| 52 | 2.0 |
| 96 | 5.3 |
| 97 | 12.5 |
| 99 | 6.9 |
| 105 | 10.1 |
| 108 | 10.6 |
| 109 | 12.6 |
| 111 | 19.6 |
| 112 | 5.6 |
| 114 | 14.9 |
| 115 | 11.7 |
| 116 | 14.0 |
| 117 | 6.3 |
| 121 | 16.1 |
| 122 | 18.7 |
| 123 | 12.8 |
| 131 | 11.5 |
| 132 | 12.7 |
| 133 | 14.8 |
| 134 | 19.4 |
| 135 | 19.2 |

It could be seen from Table 2 that the compounds of the present invention all had good in-vitro anti coagulation in the human plasma.

**Test Example 3 Study on metabolic stability of the compound of the present invention in dog and mouse liver microsomes**

**1. Experimental purpose**

The experiment was intended to study the metabolic stability of the compounds of the present invention in dog and mouse liver microsomes.

**2. Experimental scheme**

A test compound and the compound of Comparative Example 1 were incubated with dog and mouse liver microsomes (the Corning Company, USA) respectively, and a coenzyme NADPH was added to start the reaction. 135 μL of acetonitrile containing an internal standard (IS) was added to wells of a corresponding plate after 0, 5, 15, 30 and 45 minutes respectively to terminate the reaction. After protein precipitation, the solution was centrifuged at 5,594 rpm for 15 minutes, and a supernatant was taken. The supernatant was diluted with water by 1: 1 (volume ratio) and then analyzed by a LC-MS/MS method. Ketanserin was used as an internal reference compound, and two copies of Ketanserin

were incubated in parallel. Incubation conditions were summarized in Table 3.

Table 3 Incubation conditions

| | |
|---|---|
| Liver microsome | 0.5 mg·mL$^{-1}$ (test compound); 0.5 mg·mL$^{-1}$ (Ketanserin) |
| Incubation buffer | Phosphate buffer (100 mM, 1.0 mM EDTA, pH 7.4) |
| Initial concentration of test compound in incubation | 1 μM |
| Final volume of incubation system | 0.18 mL |
| Incubation time | 0, 5, 15, 30, 45 minutes (the compound of the present invention)<br>0, 5, 15, 30, 45 minutes (Ketanserin) |
| DMSO | 10 mM |
| NADPH | 6 mM |
| Parallel reaction | Two copies in parallel |

### 3. Data analysis

A peak area ratio of analyte/internal standard ($A_{analyte}/A_{IS}$) was obtained by an instrument, and a remaining percentage (%Control) was calculated by ratios of $A_{analyte}/A_{IS}$ in a sample at a non-zero time point and a sample at a zero time point. Ln (%Control) was plotted and fitted linearly with the incubation time. A scavenging constant (k, min$^{-1}$) and a scavenging half-life ($T_{1/2}$, min) of the tested compound were calculated by the following equation.

$$k = - \text{slope}$$

$$T_{1/2} = 0.693/k$$

### 4. Experimental results

Stability data of the compound in the example of the present invention were shown in Table 4.

Table 4 Stability data of the compound of the present invention in dog and mouse liver microsomes

| Number of compound | Half-life period ($T_{1/2}$, min) | |
|---|---|---|
| | Dog | Mouse |
| Comparative Example 1 | 30.54 | 23.76 |
| 96 | 919.22 | 332 |

Conclusion: compared with Comparative Example 1, the compound in Example 96 of the present invention had a longer half-life period and a higher stability in dog and mouse liver microsomes.

Remark: Comparative Example 1 was the compound in Example 99 of WO2017005725A1, and prepared according to Example 99 of WO2017005725A1, with a structure as follows.

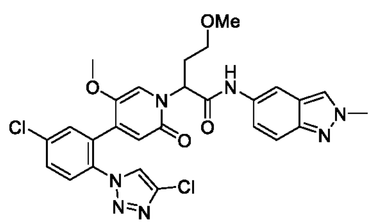

**Test Example 4. Study on pharmacokinetics of the compound of the present invention in SD rat**

1. Experimental purpose

Taking a SD rat as a test animal, the SD rat was administrated with the compound of the present invention by intravenous injection and/or intragastric administration, and drug concentrations in plasma at different times were determined by a LC/MS/MS method to study pharmacokinetic characteristics of the compound of the present invention in the SD rat.

2. Experimental scheme

2.1 Experimental drugs and animals

Compounds with numbers 25, 36, 38, 45, 47, 96, 99, 111, 113 and 117.

SD rats, male, 195 g to 235 g, 6 weeks old to 8 weeks old, purchased from Shanghai JieSiJie Laboratory Animal Co., Ltd.

2.2 Preparation of drug

Preparation of drug administrated by intravenous injection: a proper amount of compound to be tested was added to a proper amount of DMAC: (30% Soltol HS 15): Saline = 10%: 10%: 80% (v/v/v), and shaken in a vortex manner, so as to prepare a final solution with a concentration of 0.5 mg/mL.

Preparation of drug administrated by intragastric administration: a proper amount of compound to be tested was added to a proper amount of DMAC: (30% Soltol HS 15): Saline = 10%: 10%: 80% (v/v/v), and shaken in a vortex manner, so as to prepare a final solution with a concentration of 1 mg/mL.

2.3 Administration

SD rats were divided into an intravenous injection group of the compound to be tested (3 rats/group) and an intragastric administration group (3 rats/group) of the compound to be tested.

Intravenous injection group: the drug was administrated by dorsalis pedis vein injection (according to a dosage of 1 mg/kg and a volume of 2 mL/kg) without fasting.

Intragastric administration group: the drug was administrated by intragastric administration (according to a dosage of 10 mg/kg and a volume of 10 mL/kg) after fasting overnight, and food was taken 4 hours after administration.

3. Operation

Intravenous injection group: about 150 μL of blood was collected into an EDTA-K2 anticoagulant tube through a jugular vein 0.083 hour, 0.25 hour, 0.5 hour, 1 hour, 2 hours, 4 hours, 8 hours and 24 hours after administration.

Intragastric administration group: about 150 μL of blood was collected into an EDTA-K2 anticoagulant tube through a jugular vein 0.25 hour, 0.5 hour, 1 hour, 2 hours, 4 hours, 8 hours and 24 hours after administration.

The blood sample was stored in wet ice first, and plasma was centrifugally separated within 15 minutes after sampling (centrifugation conditions: 2,000 g, 4°C, 5 minutes). The collected upper plasma was stored at -70°C before analysis.

Contents of the compound to be tested in rat plasma after intravenous injection and intragastric administration were determined by LC-MS/MS.

4. Results of pharmacokinetic parameters

The results of the pharmacokinetic parameters in the test example were shown in Table 5.

Table 5 Results of pharmacokinetic parameters in rats

| Number of compound | Pharmacokinetic parameter | | | | |
|---|---|---|---|---|---|
| | Administration mode Administration dosage | Blood concentration $C_{max}$ (ng/mL) | Curve area $AUC_{0-t}$ (ng·h/mL) | Half-life period $T_{1/2}$(h) | Bioavailability F(%) |
| 25 | Intragastric administration 10 mg/kg | 1393±521 | 3464±1227 | 0.895±0.0751 | 80.4±28.5 |
| | Injection administration 1 mg/kg | N/A | 431±117 | 0.818±0.370 | |
| 36 | Intragastric administration 10 mg/kg | 798±868 | 2194±2747 | 1.15±0.0495 | N/A |
| 38 | Intragastric administration 10 mg/kg | 1362±564 | 3082±281 | 2.63±0.172 | N/A |
| 45 | Intragastric administration 10 mg/kg | 1168±576 | 4712±2117 | 2.04±1.08 | N/A |
| 47 | Intragastric administration 10 mg/kg | 2855±268 | 12243±3192 | 2.13±0.18 | N/A |
| 96 | Intragastric administration 10 mg/kg | 1790±510 | 3856±728 | 1.54±0.784 | 72.5±13.7 |
| | Injection administration 1mg/kg | N/A | 532±87.8 | 0.942±0.078 | |
| 99 | Intragastric administration 10 mg/kg | 1096±498 | 4850±1569 | 0.994±NA | 148.8±48.1 |
| | Injection administration 1mg/kg | N/A | 326±61.2 | 0.595±0.222 | |
| 111 | Intragastric administration 10 mg/kg | 1418±703 | 2562±409 | 2.37±1.21 | N/A |
| 113 | Intragastric administration 10 mg/kg | 3423±2354 | 3022±2541 | 1.38±0.297 | 54.7±46 |
| | Injection administration 1mg/kg | N/A | 552±108 | 0.5±0.0254 | |
| 117 | Intragastric administration 10 mg/kg | 2217±552 | 3938±984 | 0.735±0.0242 | N/A |

Conclusion: the compound of the present invention had good drug metabolism and absorption in rats, good blood concentration, area under curve and half-life period, and good pharmacokinetic properties.

**Test Example 5. Study on pharmacokinetics of the compound of the present invention in ICR mouse**

1. Experimental purpose

Taking an ICR mouse as a tested animal, the ICR mouse was administrated with the compound in Example 96 of the present invention by intravenous injection and intragastric administration, and drug concentrations in plasma at different times were determined by a LC/MS/MS method to study pharmacokinetic characteristics of the compound of the present invention in the mouse.

2. Experimental scheme

2.1 Experimental drugs and animals

Compound in Example 96.
ICR mice, male, 29.2 g to 34.9 g, purchased from Beijing Vital River Laboratory Animal Technology Co., Ltd.

2.2 Preparation of drug

Preparation of drug administrated by intravenous injection: a proper amount of compound to be tested was added to a proper amount of DMAC: (30% Soltol HS 15): Saline = 10%: 10%: 80% (v/v/v), and shaken in a vortex manner, so as to prepare a final solution with a concentration of 0.2 mg/mL.
Preparation of drug administrated by intragastric administration: a proper amount of compound to be tested was added to a proper amount of DMA: (30% Soltol HS 15): Saline = 10%: 10%: 80% (v/v/v), and shaken in a vortex manner, so as to prepare a final solution with a concentration of 0.5 mg/mL.

2.3 Administration

ICR mice were divided into an intravenous injection group and an intragastric administration group (9 rats in single group).
Intravenous injection group: the drug was administrated by intravenous injection (according to a dosage of 1 mg/kg and a volume of 5 mL/kg) without fasting.
Intragastric administration group: the drug was administrated by intragastric administration (according to a dosage of 5 mg/kg and a volume of 10 mL/kg) after fasting overnight, and food was taken 4 hours after administration.

3. Operation

Intravenous injection group: about 80 μL of blood was collected into an EDTA-K2 anticoagulant tube through an orbit 0.083 hour, 0.25 hour, 0.5 hour, 1 hour, 2 hours, 4 hours, 8 hours, 12 hours and 24 hours after administration.
Intragastric administration group: about 80 μL of blood was collected through an orbit 0.25 hour, 0.5 hour, 1 hour, 2 hours, 4 hours, 8 hours, 12 hours and 24 hours before and after administration.
The whole blood sample was placed in an EDTA-K2 anticoagulant tube. Plasma was centrifugally separated (centrifugation conditions: 1,500 g, 10 minutes). The collected upper plasma was stored at - 40°C to -20°C before analysis.
Contents of the compound to be tested in mouse plasma after intravenous and intragastric administration were determined by LC-MS/MS.

4. Results of pharmacokinetic parameters

The results of the pharmacokinetic parameters in the test example were shown in Table 6.

Table 6 Results of pharmacokinetic parameters in mice

| Number of compound | Pharmacokinetic parameter | | | | |
|---|---|---|---|---|---|
| | Administration mode Administration dosage | Blood concentration $C_{max}$ (ng/mL) | Curve area $AUC_{0-t}$ (ng·h/mL) | Half-life period $T_{1/2}$ (h) | Bioavailability F(%) |
| 96 | Intragastric administration 5 mg/kg | 2040 | 4330 | 1.84 | 69.8% |
| | Injection administration 1 mg/kg | N/A | 1240 | 0.664 | |

Conclusion: the compound of the present invention had good drug metabolism and absorption in ICR mice, good blood concentration, area under curve and half-life period, and good pharmacokinetic properties.

**Test Example 6. Study on pharmacokinetics of the compound of the present invention in cynomolgus macaque**

1. Experimental purpose

Taking a cynomolgus macaque as a tested animal, the cynomolgus macaque was administrated with the compound in Example 96 of the present invention by intragastric administration, and drug concentrations in plasma at different times were determined by a LC/MS/MS method to study pharmacokinetic characteristics of the compound of the present invention in the cynomolgus macaque.

2. Experimental scheme

2.1 Experimental drugs and animals

Compound in Example 96.
Cynomolgus macaque, male, 4.6 kg to 5.7 kg, adult, purchased from Guangxi Fangcheng Gang Spring Biological Technology Development Corporation Ltd.

2.2 Preparation of drug

Preparation of drug administrated by intragastric administration: a proper amount of compound to be tested was added to a proper amount of DMAC: (30% Kolliphor HS 15): Saline = 5%: 10%: 85% (v/v/v), and shaken in a vortex manner, so as to prepare a final solution with a concentration of 3 mg/mL.

2.3 Administration

Cynomolgus macaques were organized into one group, which was an intragastric administration group (3 cynomolgus macaques in single group).
Intragastric administration group: the drug was administrated by intragastric administration (according to a dosage of 15 mg/kg and a volume of 5 mL/kg) after fasting overnight, and food was taken 4 hours after administration.

3. Operation

Intragastric administration group: about 500 μL of blood was collected into an EDTA-K2 anticoagulant tube through a cephalic vein and a saphenous vein 0.25 hour, 0.5 hour, 1 hour, 2 hours, 4 hours, 8 hours and 24 hours after administration.
The blood sample was placed on wet ice first, and plasma was centrifugally separated within 15 minutes after sampling (centrifugation conditions: 2,000 g, 4°C, 5 minutes). The collected upper plasma was stored at -70°C before analysis.
Contents of the compound to be tested in cynomolgus macaque plasma after intragastric administration were determined by LC-MS/MS.

4. Results of pharmacokinetic parameters

The results of the pharmacokinetic parameters in the test example were shown in Table 7.

Table 7 Results of pharmacokinetic parameters in cynomolgus macaques

| Number of compound | Pharmacokinetic experiment | | | |
|---|---|---|---|---|
| | Administration mode<br>Administration dosage | Blood concentration $C_{max}$ (ng/mL) | Curve area $AUC_{0-t}$ (ng·h/mL) | Half-life period $T_{1/2}$ (h) |
| 96 | Intragastric administration 15 mg/kg | 834±482 | 4211±1245 | 3.71±0.42 |

Conclusion: the compound of the present invention had good drug metabolism and absorption in cynomolgus macaques, and good pharmacokinetic properties.
Unless otherwise specified, the terms used in the present invention have the meanings commonly understood

by those skilled in the art.

The described embodiments of the present invention are only for illustrative purposes, and are not intended to limit the scope of protection of the present invention. Those skilled in the art can make various other substitutions, changes and improvements within the scope of the present invention. Therefore, the present invention is not limited to the above-mentioned embodiments, but only limited by the claims.

**Claims**

1. A compound represented by general formula (I) or a stereoisomer, a tautomer, a pharmaceutically acceptable salt or a prodrug thereof:

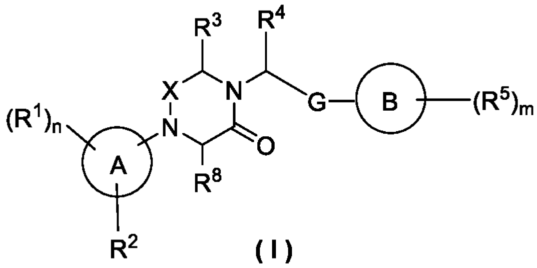
(I)

wherein:

X is selected from C=O or $CR^7$; and preferably, X is selected from C=O;
G is selected from bond or -C(O)-NH-; and preferably, G is selected from -C(O)-NH-;
ring A is selected from aryl or heteroaryl;
ring B is selected from cycloalkyl, heterocyclyl, aryl, heteroaryl or fused ring; wherein, the heteroaryl is preferably a 5- to 10- membered heteroaryl;
$R^1$ are the same or different, and are each independently selected from hydrogen atom, deuterium atom, halogen, alkyl, haloalkyl, nitro, cyano, hydroxyalkyl, cycloalkyl, heterocyclyl, aryl, heteroaryl, $-OR^9$ or $-NR^{10}R^{11}$;
$R^2$ is selected from halogen, cyano, alkoxy, heteroaryl or $-C(O)R^9$; wherein, the alkoxy or heteroaryl is optionally further substituted by one or more substituents selected from alkyl, haloalkyl, nitro, cyano, halogen, cycloalkyl, heterocyclyl, aryl, heteroaryl, $-OR^9$, $-C(O)R^9$, $-C(O)OR^9$, - $NHC(O)R^9$, $-NHC(O)OR^9$, $-NR^{10}R^{11}$ or $-C(O)NR^{10}R^{11}$,
$R^3$ is selected from hydrogen atom, halogen or alkyl; wherein, the alkyl is optionally further substituted by one or more substituents selected from halogen, hydroxyl or alkoxy; and $R^3$ is preferably hydrogen atom;
$R^4$ is selected from hydrogen atom, deuterium atom, alkyl, cycloalkyl, heterocyclyl, aryl, heteroaryl or $-OR^9$, wherein the alkyl, cycloalkyl, heterocyclyl, aryl or heteroaryl is optionally further substituted by one or more substituents selected from halogen, hydroxyl or $R^6$;
$R^5$ are the same or different and are each independently selected from hydrogen atom, alkyl, halogen, nitro, cyano, cycloalkyl, heterocyclyl, aryl, heteroaryl, $-OR^9$, $-C(O)R^9$, $-C(O)OR^9$, - $NHC(O)R^9$, $-NHC(O)OR^9$, $-NR^{10}R^{11}$, $-C(O)NR^{10}R^{11}$, $-CH_2NHC(O)OR^9$, $-CH_2NR^{10}R^{11}$, $-S(O)_rR^9$ or - $S(O)_rNR^{10}R^{11}$, wherein the alkyl, cycloalkyl, heterocyclyl, aryl or heteroaryl is optionally further substituted by one or more substituents selected from deuterium atom, halogen, nitro, cyano, hydroxyl, =O, $-OR^9$, $-C(O)R^9$, $-C(O)OR^9$, $-NHC(O)R^9$, $-NHC(O)OR^9$, $-NR^{10}R^{11}$, $-C(O)NR^{10}R^{11}$, - $CH_2NHC(O)OR^9$, $-CH_2NR^{10}R^{11}$ or $-S(O)_rR^9$;
alternatively, two $R^5$ together with the same carbon atom bound therewith form C=O;
$R^6$ is selected from deuterium atom, halogen, alkyl, alkoxy, amino, nitro, cyano, hydroxyl, cycloalkyl, heterocyclyl, aryl or heteroaryl, wherein the alkyl, alkoxy, cycloalkyl, heterocyclyl, aryl or heteroaryl is optionally further substituted by one or more $R^A$ substituents;
$R^A$ is selected from deuterium atom, halogen, alkyl, hydroxyl, alkoxy, amino, nitro, cyano, cycloalkyl, heterocyclyl, aryl, heteroaryl or $-NR^{13}C(O)R^{14}$, wherein the alkyl, alkoxy, cycloalkyl, heterocyclyl, aryl or heteroaryl is optionally further substituted by one or more substituents selected from deuterium atom, halogen, hydroxyl, alkyl, haloalkyl, alkoxy, haloalkoxy or cyano;
$R^7$ is selected from hydrogen atom, halogen or $-OR^B$, and is preferably hydrogen atom;
$R^B$ is selected from alkyl, cycloalkyl, heterocyclyl, aryl or heteroaryl, wherein the alkyl, cycloalkyl, heterocyclyl, aryl or heteroaryl is optionally further substituted by one or more substituents selected from deuterium atom, halogen, alkyl, haloalkyl, hydroxyl, alkoxy or haloalkoxy;
$R^8$ is selected from hydrogen atom, halogen or alkyl; wherein the alkyl is optionally further substituted by one

or more substituents selected from halogen, hydroxyl or alkoxy; and $R^8$ is preferably hydrogen atom;
$R^9$ is selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxyl, halogen, nitro, cyano, alkyl, alkoxy, haloalkyl, haloalkoxy, cycloalkyl, heterocyclyl, aryl, heteroaryl, =O, -C(O)R12, -C(O)OR12, -OC(O)$R^{12}$, -OC(O)O$R^{12}$, - N$R^{13}R^{14}$, -C(O)N$R^{13}R^{14}$, -$SO_2$N$R^{13}R^{14}$ or -N$R^{13}$C(O)$R^{14}$;
$R^{10}$ and $R^{11}$ are each independently selected from hydrogen atom, hydroxyl, halogen, alkyl, alkoxy, cycloalkyl, heterocyclyl, aryl or heteroaryl, wherein the alkyl, alkoxy, cycloalkyl, heterocyclyl, aryl or heteroaryl is optionally further substituted by one or more substituents selected from hydroxyl, halogen, nitro, cyano, alkyl, alkoxy, cycloalkyl, heterocyclyl, aryl, heteroaryl, =O, -C(O)$R^{12}$, - C(O)O$R^{12}$, -OC(O)$R^{12}$, -N$R^{13}R^{14}$, -C(O)N$R^{13}R^{14}$, -$SO_2$N$R^{13}R^{14}$ or -N$R^{13}$C(O)$R^{14}$;
alternatively, $R^{10}$ and $R^{11}$ together with the connected N atom form one 4- to 8- membered heterocyclyl, wherein the 4- to 8- membered heterocycle internally contains one or more N, O or S(O)$_n$, and the 4- to 8- membered heterocycle is optionally further substituted by one or more substituents selected from hydroxyl, halogen, nitro, cyano, alkyl, alkoxy, cycloalkyl, heterocyclyl, aryl, heteroaryl, =O, -C(O)$R^{12}$, -C(O)O$R^{12}$, -OC(O)$R^{12}$, -N$R^{13}R^{14}$, -C(O)N$R^{13}R^{14}$, -$SO_2$N$R^{13}R^{14}$ or -N$R^{13}$C(O)$R^{14}$;
$R^{12}$, $R^{13}$ and $R^{14}$ are each independently selected from hydrogen atom, alkyl, amino, cycloalkyl, heterocyclyl, aryl or heteroaryl, wherein the alkyl, cycloalkyl, heterocyclyl, aryl or heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or carboxylate;
m is 0, 1, 2, 3, or 4;
n is 0, 1, 2, 3, or 4; and
r is 0, 1 or 2.

2. The compound or the stereoisomer, the tautomer, the pharmaceutically acceptable salt or the prodrug thereof according to claim 1, wherein the compound or the stereoisomer, the tautomer, the pharmaceutically acceptable salt or the prodrug thereof is a compound represented by general formula (II) or a stereoisomer, a tautomer, a pharmaceutically acceptable salt or a prodrug thereof,

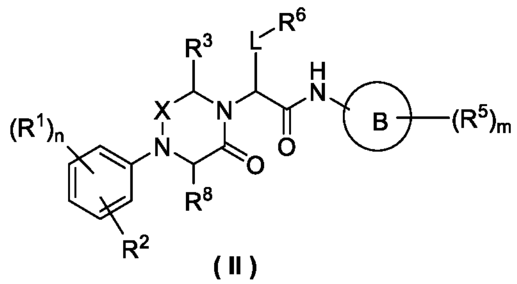

(II)

wherein:

L is selected from bond or alkylene, wherein the alkylene is optionally further substituted by one or more substituents selected from deuterium atom, halogen or hydroxyl; and
ring B, X, $R^1$ to $R^3$, $R^5$, $R^6$, $R^8$, m and n are as defined in claim 1.

3. The compound or the stereoisomer, the tautomer, the pharmaceutically acceptable salt or the prodrug thereof according to claim 2, wherein the compound or the stereoisomer, the tautomer, the pharmaceutically acceptable salt or the prodrug thereof is a compound represented by general formula (III) or a stereoisomer, a tautomer, a pharmaceutically acceptable salt or a prodrug thereof,

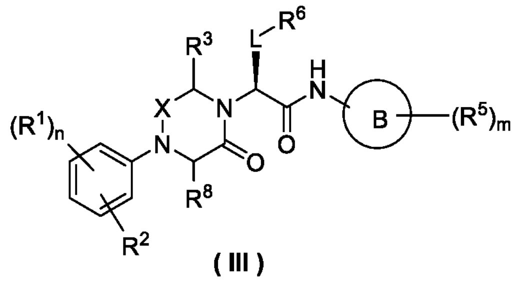

(III)

wherein: ring B, X, L, $R^1$ to $R^3$, $R^5$, $R^6$, $R^8$, m and n are as defined in claim 2.

**4.** The compound or the stereoisomer, the tautomer, the pharmaceutically acceptable salt or the prodrug thereof according to claim 2, wherein the compound or the stereoisomer, the tautomer, the pharmaceutically acceptable salt or the prodrug thereof is a compound represented by general formula (IV) or a stereoisomer, a tautomer, a pharmaceutically acceptable salt or a prodrug thereof,

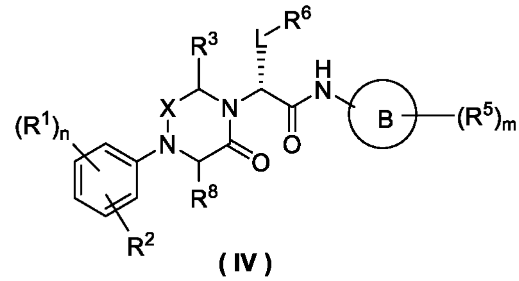

( IV )

wherein: ring B, X, L, $R^1$ to $R^3$, $R^5$, $R^6$, $R^8$, m and n are as defined in claim 2.

**5.** The compound or the stereoisomer, the tautomer, the pharmaceutically acceptable salt or the prodrug thereof according to any one of claims 1 to 4, wherein ring B is selected from 3- to 6-membered cycloalkyl, 4- to 8- membered heterocyclyl, phenyl, 5- to 10- membered heteroaryl or 8- to 10- membered fused ring; wherein, the 3- to 6- membered cycloalkyl is preferably cyclohexyl; and the 4- to 8- membered heterocyclyl is preferably tetrahydropyranyl or piperidinyl.

**6.** The compound or the stereoisomer, the tautomer, the pharmaceutically acceptable salt or the prodrug thereof according to any one of claims 1 to 4, wherein

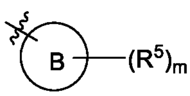

is selected from:

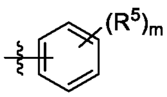 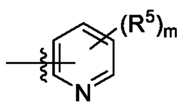 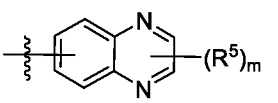 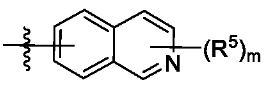 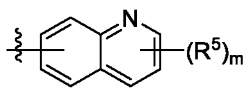

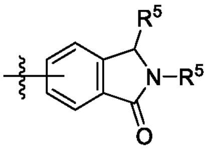 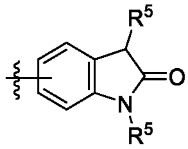 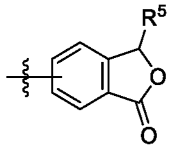 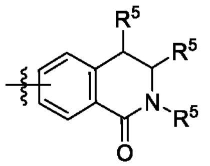 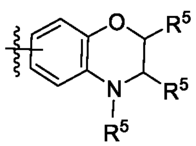

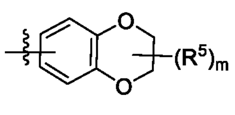 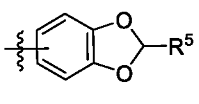 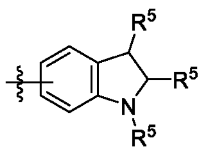 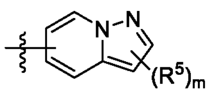 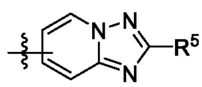

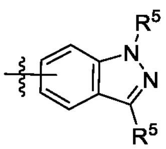 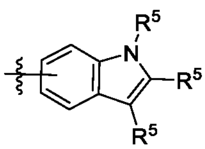 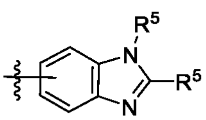 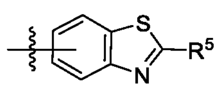 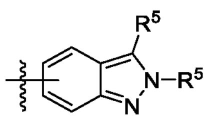

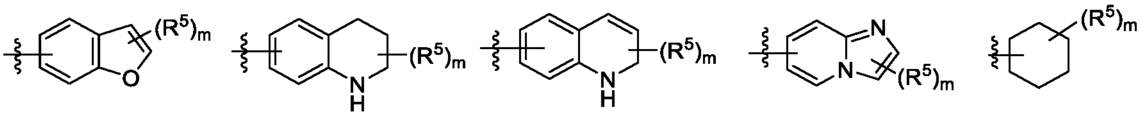

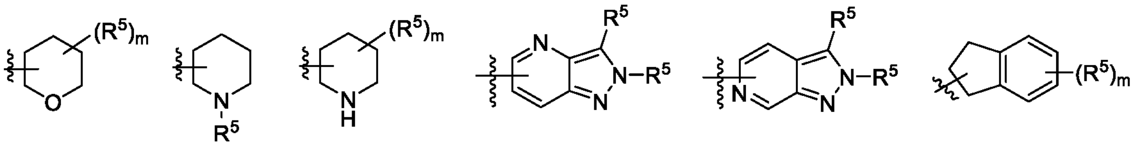

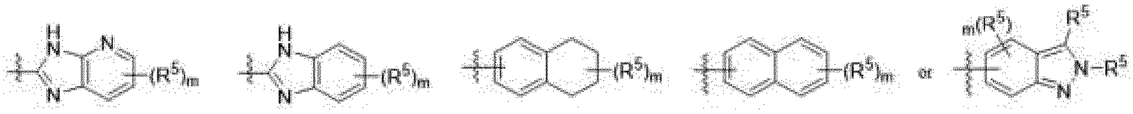

wherein: $R^5$ and m are as defined in claim 1.

**7.** The compound or the stereoisomer, the tautomer, the pharmaceutically acceptable salt or the prodrug thereof according to any one of claims 1 to 4, wherein

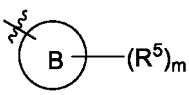

is selected from:

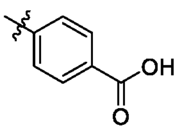 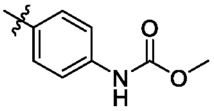 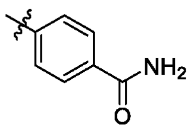 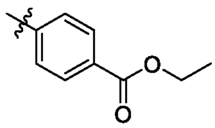 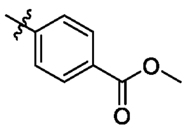

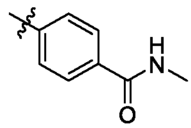 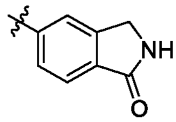 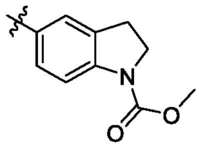 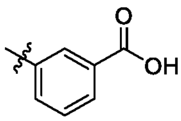 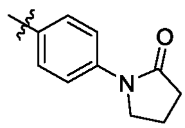

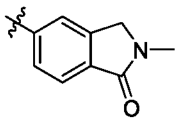 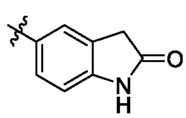 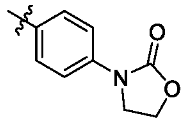 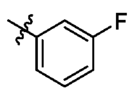 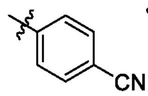 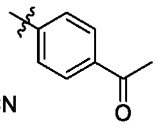

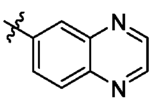 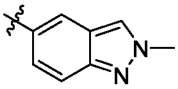 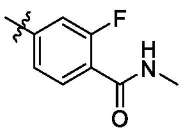 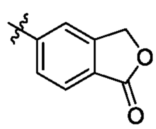 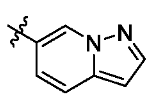 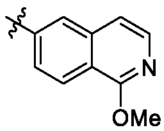

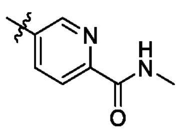 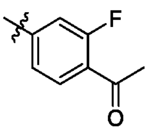 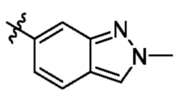 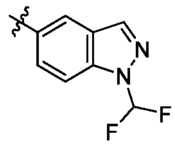 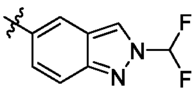 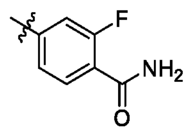

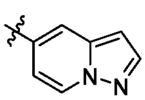 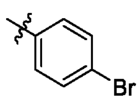 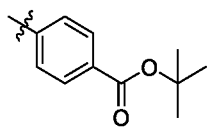 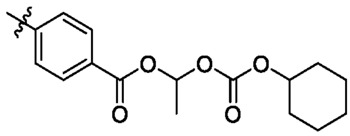 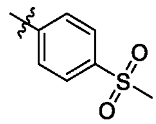

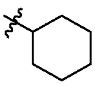 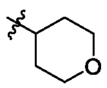 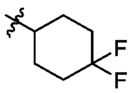 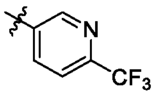 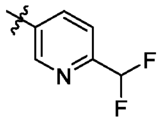 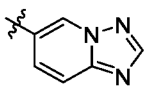 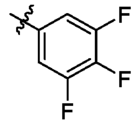

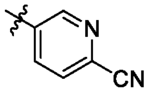 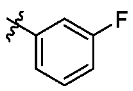 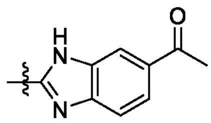 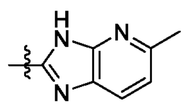 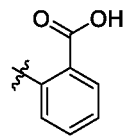 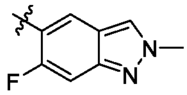

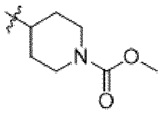 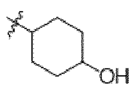 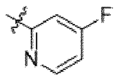 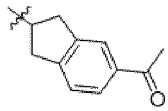 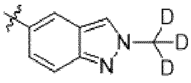 or 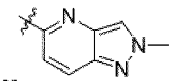 .

8. The compound or the stereoisomer, the tautomer, the pharmaceutically acceptable salt or the prodrug thereof according to claim 2, wherein the compound or the stereoisomer, the tautomer, the pharmaceutically acceptable salt or the prodrug thereof is a compound represented by general formula (V) or a stereoisomer, a tautomer, a pharmaceutically acceptable salt or a prodrug thereof,

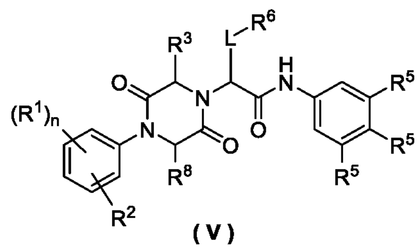

**( V )**

wherein: L, $R^1$ to $R^3$, $R^5$, $R^6$, $R^8$ and n are as defined in claim 2.

9. The compound or the stereoisomer, the tautomer, the pharmaceutically acceptable salt or the prodrug thereof according to claim 8, wherein the compound or the stereoisomer, the tautomer, the pharmaceutically acceptable salt or the prodrug thereof is a compound represented by general formula (V-A) or (V-B) or a stereoisomer, a tautomer, a pharmaceutically acceptable salt or a prodrug thereof,

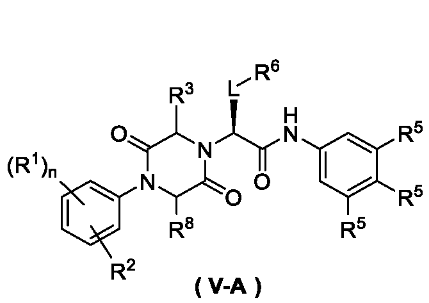

**( V-A )**

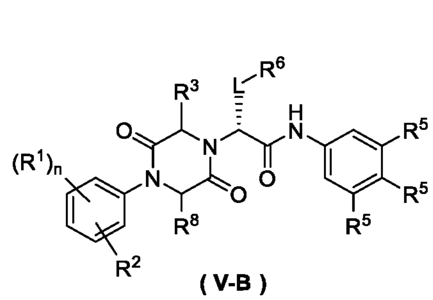

**( V-B )**

wherein: L, $R^1$ to $R^3$, $R^5$, $R^6$, $R^8$ and n are as defined in claim 8.

10. The compound or the stereoisomer, the tautomer, the pharmaceutically acceptable salt or the prodrug thereof according to claim 2, wherein the compound or the stereoisomer, the tautomer, the pharmaceutically acceptable salt or the prodrug thereof is a compound of general formula (VI) or a stereoisomer, a tautomer, a pharmaceutically

acceptable salt or a prodrug thereof,

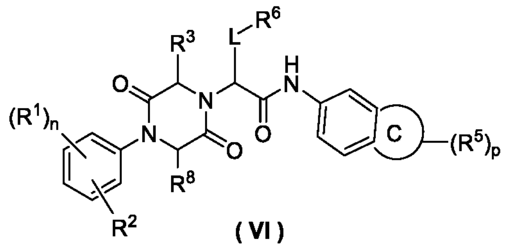

( VI )

wherein:

ring C is selected from 5- to 6- membered heteroaryl, 5- to 6- membered aryl, 4- to 8- membered heterocyclyl or 4- to 8- membered cycloalkyl;
p is 0, 1 or 2; and
L, $R^1$ to $R^3$, $R^5$, $R^6$, $R^8$ and n are as defined in claim 2.

**11.** The compound or the stereoisomer, the tautomer, the pharmaceutically acceptable salt or the prodrug thereof according to claim 10, wherein the compound or the stereoisomer, the tautomer, the pharmaceutically acceptable salt or the prodrug thereof is a compound of general formula (VI-A) or (VI-B) or a stereoisomer, a tautomer, a pharmaceutically acceptable salt or a prodrug thereof,

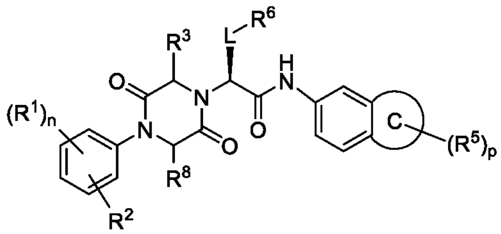

( VI-A )

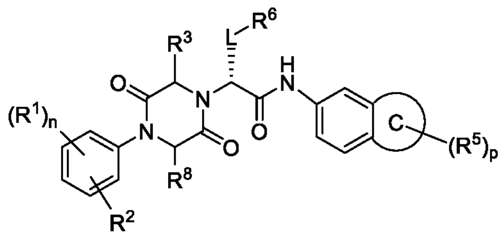

( VI-B )

wherein: ring C, L, $R^1$ to $R^3$, $R^5$, $R^6$, $R^8$, p and n are as defined in claim 10.

**12.** The compound or the stereoisomer, the tautomer, the pharmaceutically acceptable salt or the prodrug thereof according to any one of claims 2 to 11, wherein: $R^2$ is selected from 5- membered heteroaryl; wherein the 5-membered heteroaryl is optionally further substituted by one or more substituents selected from alkyl, haloalkyl, cyano or halogen; preferably, $R^2$ is selected from triazolyl or tetrazolyl; wherein the triazolyl is optionally further substituted by halogen; wherein the halogen is preferably Cl.

**13.** The compound or the stereoisomer, the tautomer, the pharmaceutically acceptable salt or the prodrug thereof according to any one of claims 2 to 11, wherein:

L is $-(CR^aR^b)s-$, wherein s is 1, 2, 3 or 4;
$R^a$ and $R^b$ are each independently selected from hydrogen atom, deuterium atom or alkyl;
$R^6$ is selected from alkyl, alkoxy, 3- to 8- membered cycloalkyl, 4- to 8- membered heterocyclyl, phenyl or 5- to 10- membered heteroaryl, wherein the alkyl, alkoxy, 3-8 membered cycloalkyl, 4- to 8- membered heterocyclyl, phenyl or 5- to 10- membered heteroaryl is optionally further substituted by one or more $R^A$ substituents; and
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano.

**14.** The compound or the stereoisomer, the tautomer, the pharmaceutically acceptable salt or the prodrug thereof according to claim 13, wherein:

L is selected from $-CH_2-$, $-CD_2-$ or $-CH_2CH_2-$;

$R^6$ is selected from alkyl, alkoxy, 3- to 8- membered cycloalkyl, 4- to 8- membered heterocyclyl, phenyl or 5- to 10- membered heteroaryl, wherein the alkyl, alkoxy, 3- to 8- membered cycloalkyl, 4-to 8- membered heterocyclyl, phenyl or 5- to 10- membered heteroaryl is optionally further substituted by one or more $R^A$ substituents; and
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano.

**15.** The compound or the stereoisomer, the tautomer, the pharmaceutically acceptable salt or the prodrug thereof according to claim 13 or 14, wherein:

$R^6$ is selected from alkyl, alkoxy, phenyl, pyridinyl, pyrazolyl, imidazolyl, 3- to 6- membered cycloalkyl or 4- to 6- membered heterocyclyl, wherein the alkyl, alkoxy, phenyl, pyridinyl, pyrazolyl, imidazolyl, 3- to 6- membered cycloalkyl or 4- to 6- membered heterocyclyl is optionally further substituted by one or more $R^A$ substituents; and
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano.

**16.** The compound or the stereoisomer, the tautomer, the pharmaceutically acceptable salt or the prodrug thereof according to any one of claims 1 to 15, wherein:

$R^5$ are the same or different, and are each independently selected from hydrogen atom, alkyl, haloalkyl, halogen, cyano, heterocyclyl, $-OR^9$, $-C(O)R^9$, $-C(O)OR^9$, $-NHC(O)R^9$, $-NHC(O)OR^9$, - $NR^{10}R^{11}$, $-C(O)NR^{10}R^{11}$, $-CH_2NHC(O)OR^9$, $-CH_2NR^{10}R^{11}$, $-S(O)_rR^9$ or $-S(O)_rNR^{10}R^{11}$;
alternatively, two $R^5$ together with the same carbon atom bound therewith form C=O;
$R^9$ is selected from hydrogen atom or $C_1$-$C_4$ alkyl, wherein the $C_1$-$C_4$ alkyl is optionally further substituted by one or more substituents selected from hydroxyl, halogen, nitro, cyano or $-OC(O)OR^{12}$; and
$R^{10}$ and $R^{11}$ are each independently selected from hydrogen atom, $C_1$-$C_4$ alkyl or 5- to 6- membered heterocyclyl, wherein the $C_1$-$C_4$ alkyl or 5- to 6- membered heterocyclyl is optionally further substituted by one or more substituents selected from halogen, hydroxyl, $C_1$-$C_4$ alkoxy, nitro, cyano or =O.

**17.** The compound or the stereoisomer, the tautomer, the pharmaceutically acceptable salt or the prodrug thereof according to claim 1, wherein the compound or the stereoisomer, the tautomer, the pharmaceutically acceptable salt or the prodrug thereof is a compound represented by general formula (VII) or a stereoisomer, a tautomer, a pharmaceutically acceptable salt or a prodrug thereof,

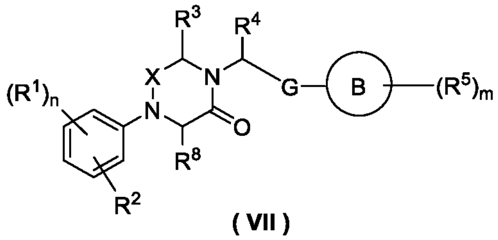

( VII )

wherein:

X is selected from C=O or $CR^7$;
G is selected from bond or -C(O)-NH-;
ring B is selected from 3- to 6- membered cycloalkyl, 4- to 8- membered heterocyclyl, phenyl, 5-to 10- membered heteroaryl or 8- to 10- membered fused ring;
$R^1$ are the same or different, and are each independently selected from hydrogen atom, deuterium atom, halogen, alkyl or haloalkyl;
$R^2$ is selected from halogen, cyano, alkoxy, heteroaryl or $-C(O)R^9$; wherein the alkoxy or heteroaryl is optionally further substituted by one or more substituents selected from haloalkyl or halogen;
$R^3$ is selected from hydrogen atom;
$R^4$ is selected from hydrogen atom, deuterium atom or alkyl, wherein the alkyl is optionally further substituted

by one or more substituents selected from halogen, hydroxyl or $R^6$;
$R^5$ are the same or different, and are each independently selected from hydrogen atom, alkyl, halogen, nitro, cyano, cycloalkyl, heterocyclyl, haloalkyl, aryl, heteroaryl, $-OR^9$, $-C(O)R^9$, $-C(O)OR^9$, $-NHC(O)R^9$, $-NHC(O)OR^9$, $-NR^{10}R^{11}$, $-C(O)NR^{10}R^{11}$, $-CH_2NHC(O)OR^9$, $-CH_2NR^{10}R^{11}$, $-S(O)_2R^9$ or $-S(O)_2NR^{10}R^{11}$, wherein the alkyl, cycloalkyl, heterocyclyl, aryl or heteroaryl is optionally further substituted by one or more substituents selected from deuterium atom, halogen or =O;
alternatively, two $R^5$ together with the same carbon atom bound therewith form C=O;
$R^6$ is selected from alkyl, alkoxy, phenyl, pyridinyl, pyrazolyl, imidazolyl, 3- to 6- membered cycloalkyl or 4- to 6- membered heterocyclyl, wherein the alkyl, alkoxy, phenyl, pyridinyl, pyrazolyl, imidazolyl, 3- to 6- membered cycloalkyl or 4- to 6- membered heterocyclyl is optionally further substituted by one or more $R^A$ substituents;
$R^A$ is selected from deuterium atom, halogen, alkyl, alkoxy, cyano, amino, phenyl or 5- to 10-membered heteroaryl, wherein the alkyl, alkoxy, phenyl or 5- to 10- membered heteroaryl is optionally further substituted by one or more substituents selected from deuterium atom, halogen, hydroxyl, alkyl, haloalkyl, alkoxy, haloalkoxy or cyano;
$R^7$ is selected from hydrogen atom;
$R^8$ is selected from hydrogen atom or $C_1$-$C_4$ alkyl;
$R^9$ is selected from hydrogen atom or $C_1$-$C_4$ alkyl, wherein the $C_1$-$C_4$ alkyl is optionally further substituted by one or more substituents selected from hydroxyl, halogen, nitro, cyano or $-OC(O)OR^{12}$;
$R^{10}$ and $R^{11}$ are each independently selected from hydrogen atom, $C_1$-$C_4$ alkyl or 5- to 6- membered heterocyclyl, wherein the $C_1$-$C_4$ alkyl or 5- to 6- membered heterocyclyl is optionally further substituted by one or more substituents selected from halogen, hydroxyl, $C_1$-$C_4$ alkoxy, nitro, cyano or =O;
$R^{12}$, $R^{13}$ and $R^{14}$ are each independently selected from hydrogen atom, alkyl or cycloalkyl;
m is 0, 1 or 2; and
n is 0, 1 or 2.

**18.** The compound represented by general formula (I) or the stereoisomer, the tautomer, the pharmaceutically acceptable salt or the prodrug thereof according to claim 1, wherein the compound is:

| No. of compound | Structure | No. of compound | Structure |
|---|---|---|---|
| 1 | 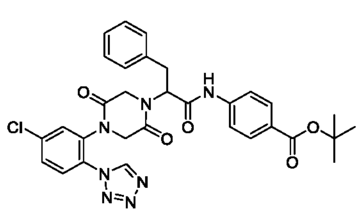 | 2 | 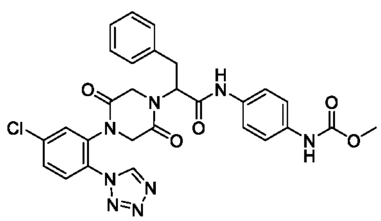 |
| 3 | 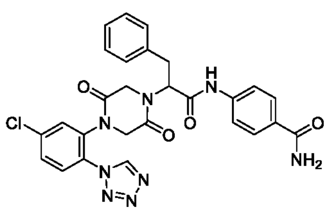 | 4 | 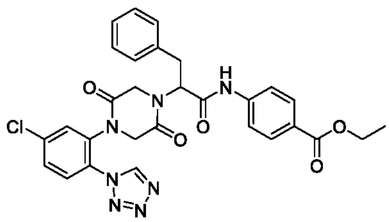 |
| 5 | 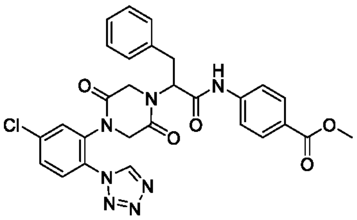 | 6 | 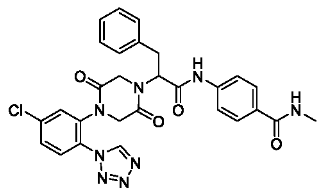 |

(continued)

| No. of compound | Structure | No. of compound | Structure |
|---|---|---|---|
| 7 | 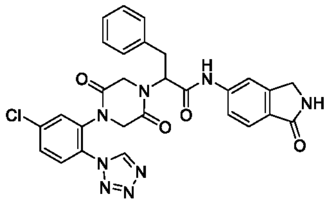 | 8 | 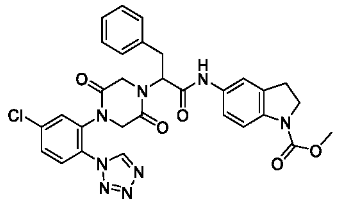 |
| 9 | 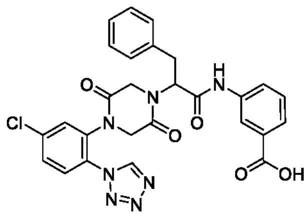 | 10 | 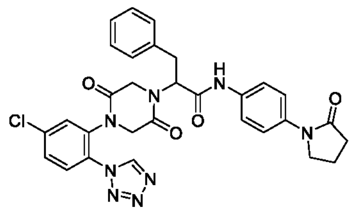 |
| 11 | 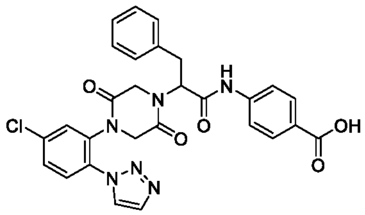 | 12 | 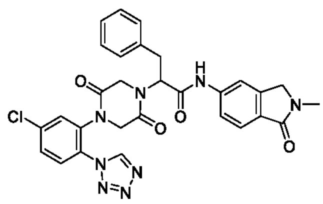 |
| 13 | 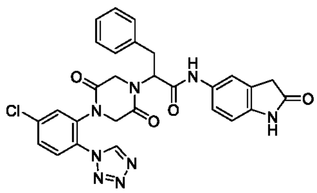 | 14 | 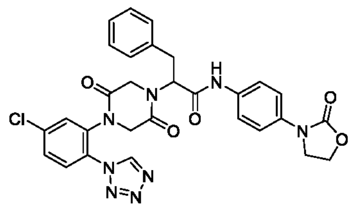 |
| 15 | 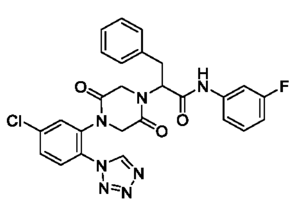 | 16 | 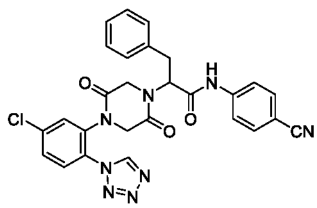 |
| 17 | 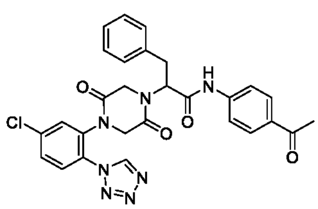 | 18 | 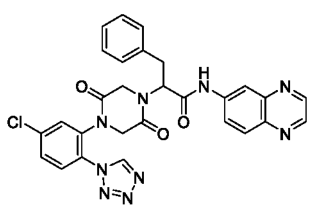 |
| 19 | 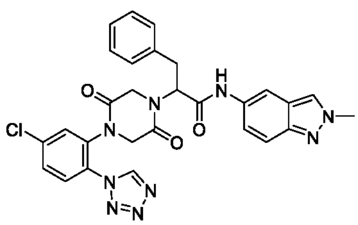 | 20 | 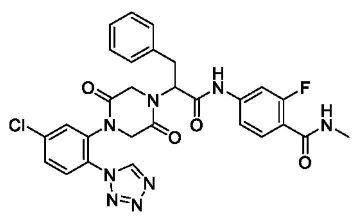 |

(continued)

| No. of compound | Structure | No. of compound | Structure |
|---|---|---|---|
| 21 | 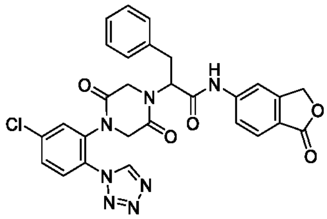 | 22 | 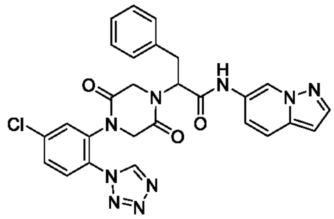 |
| 23 | 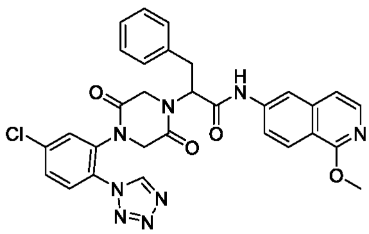 | 24 | 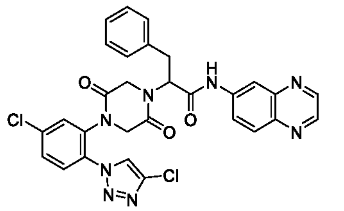 |
| 25 | 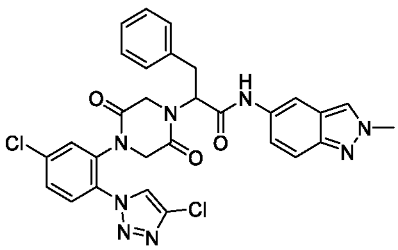 | 26 | 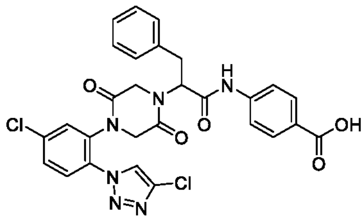 |
| 27 | 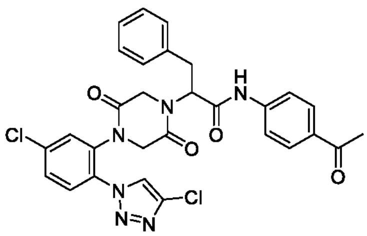 | 28 | 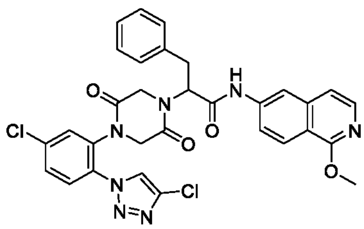 |
| 29 | 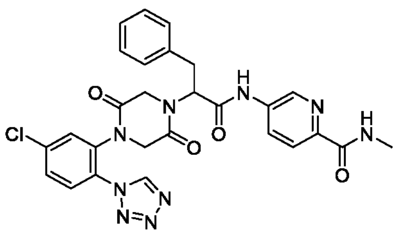 | 30 | 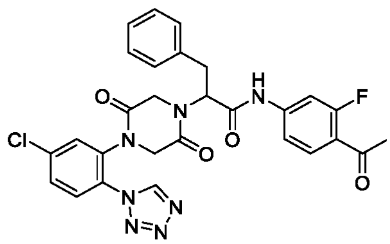 |
| 31 | 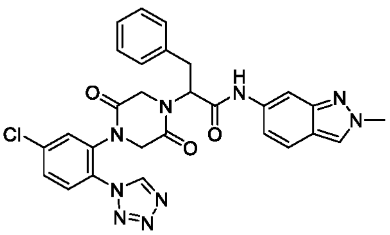 | 32 | 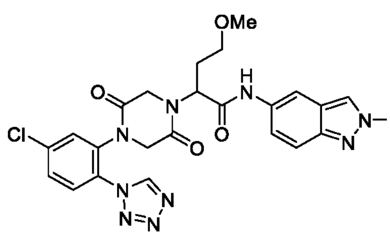 |

(continued)

| No. of compound | Structure | No. of compound | Structure |
|---|---|---|---|
| 33 | 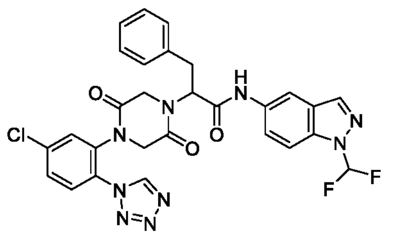 | 34 | 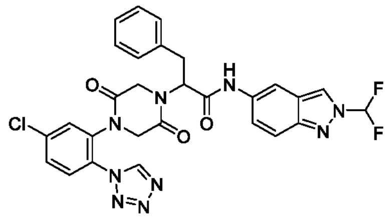 |
| 35 | 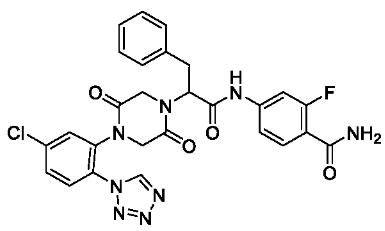 | 36 | 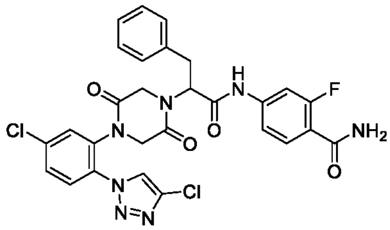 |
| 37 | 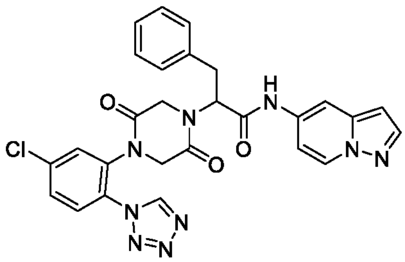 | 38 | 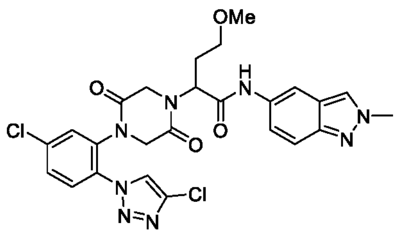 |
| 39 | 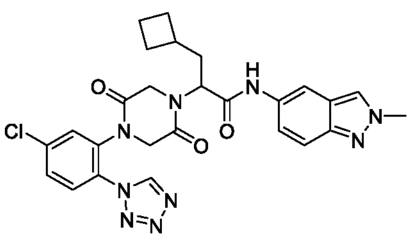 | 40 | 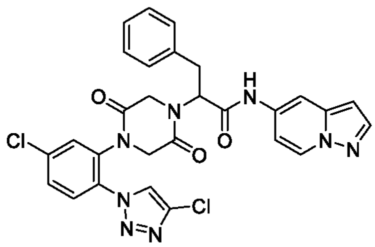 |
| 41 | 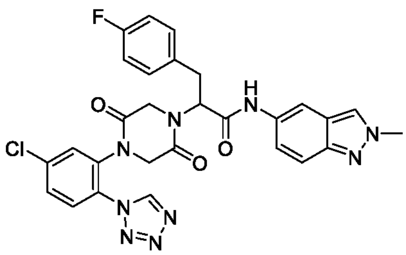 | 42 | 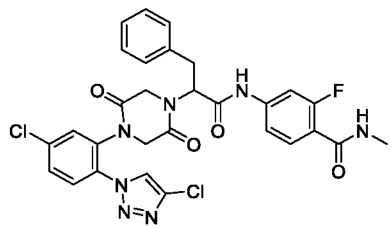 |
| 43 | 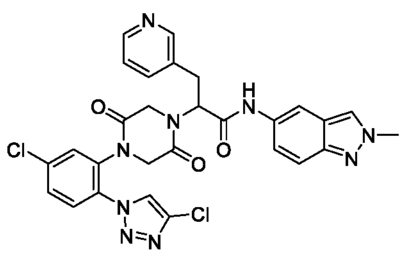 | 44 | 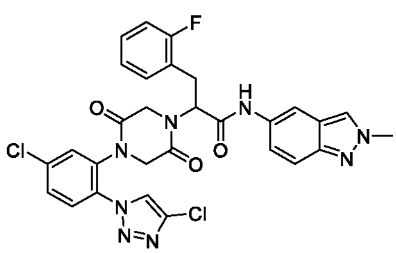 |

(continued)

| No. of compound | Structure | No. of compound | Structure |
|---|---|---|---|
| 45 | 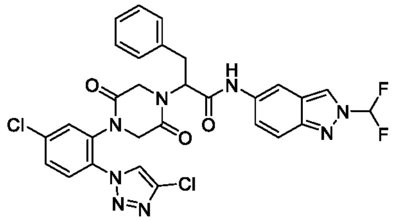 | 46 | 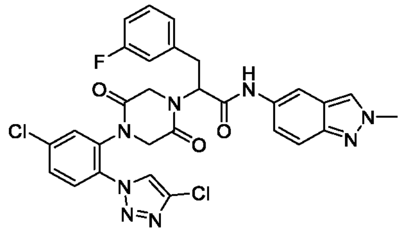 |
| 47 | 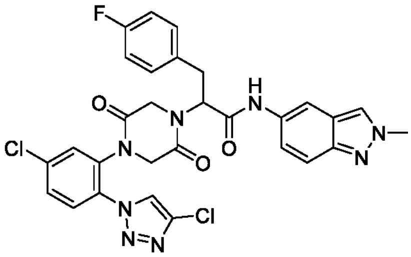 | 48 | 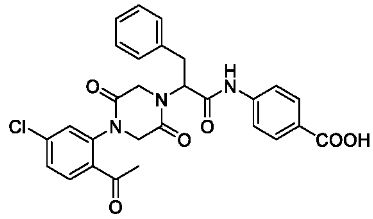 |
| 49 | 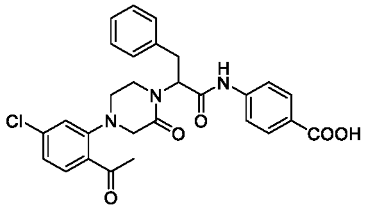 | 50 | 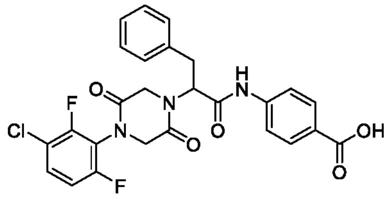 |
| 51 | 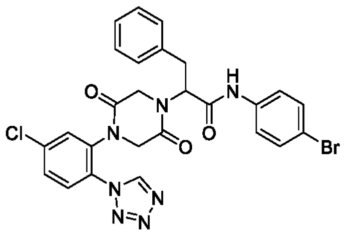 | 52 | 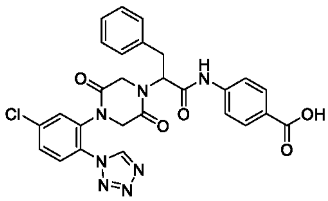 |
| 53 | 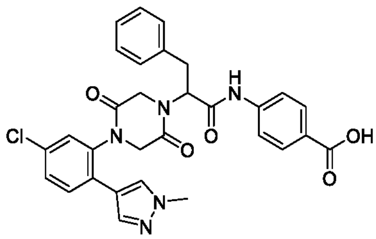 | 54 | 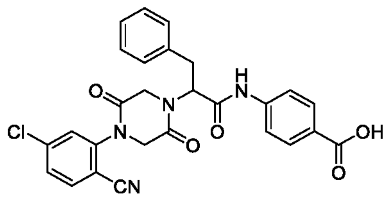 |
| 55 | 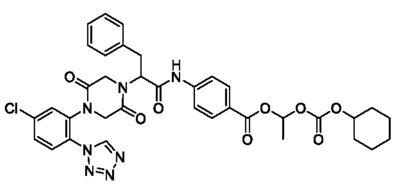 | 56 | 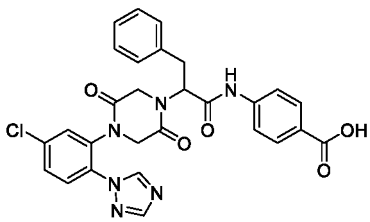 |

(continued)

| No. of compound | Structure | No. of compound | Structure |
|---|---|---|---|
| 57 | 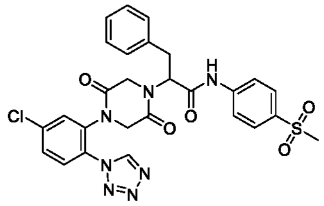 | 58 | 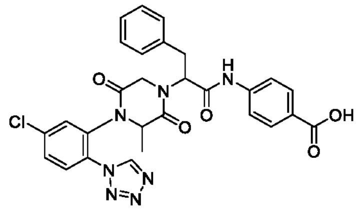 |
| 59 | 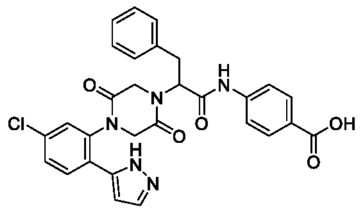 | 60 | 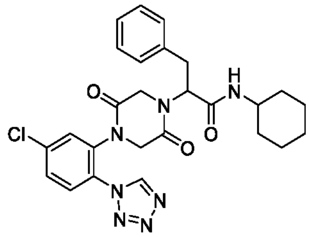 |
| 61 | 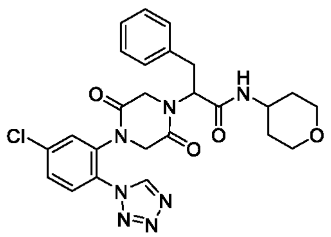 | 62 | 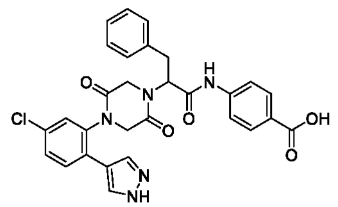 |
| 63 | 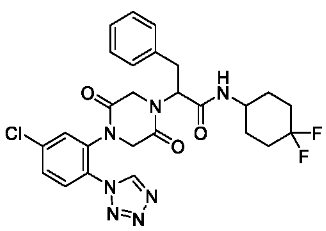 | 64 | 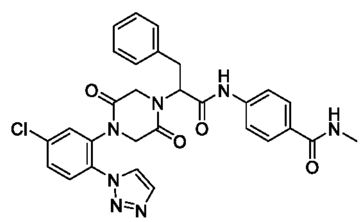 |
| 65 | 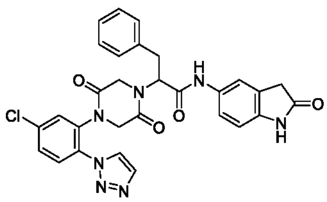 | 66 | 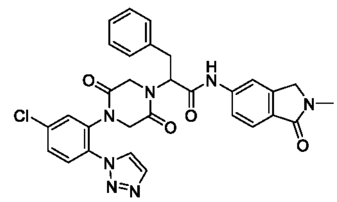 |
| 67 | 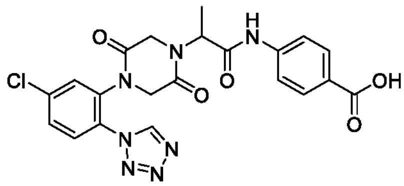 | 68 | 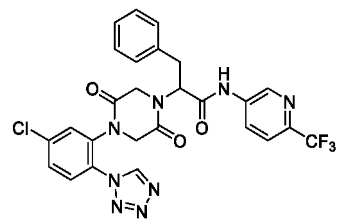 |

(continued)

| No. of compound | Structure | No. of compound | Structure |
|---|---|---|---|
| 69 | 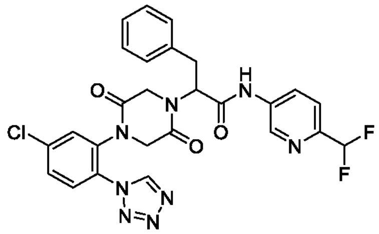 | 70 | 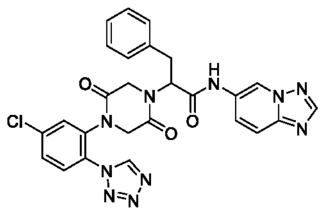 |
| 71 | 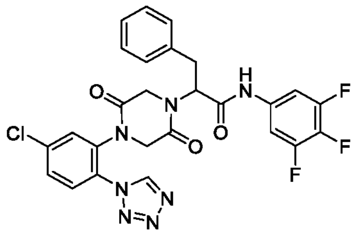 | 72 | 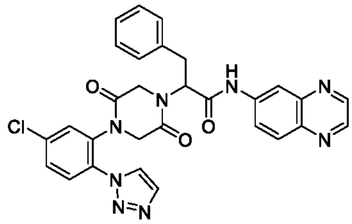 |
| 73 | 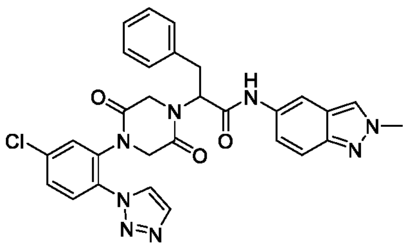 | 74 | 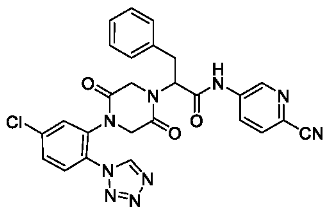 |
| 75 | 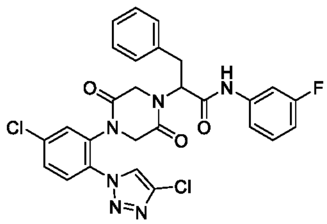 | 76 | 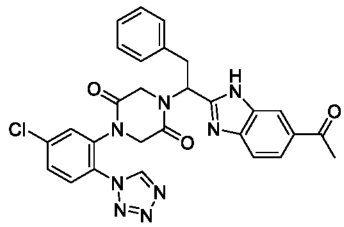 |
| 77 | 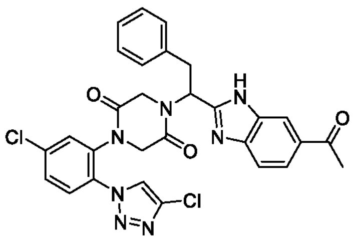 | 78 | 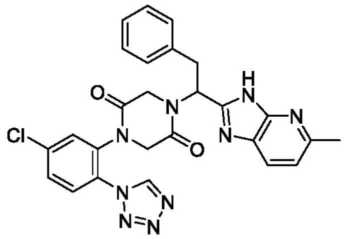 |
| 79 | 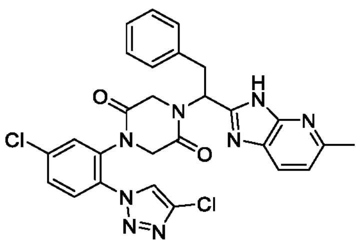 | 80 | 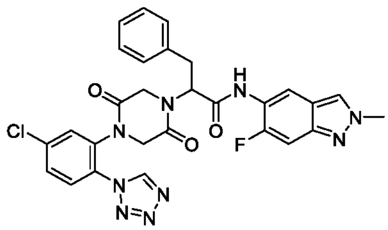 |

(continued)

| No. of compound | Structure | No. of compound | Structure |
|---|---|---|---|
| 81 | 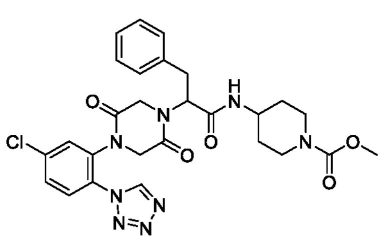 | 82 | 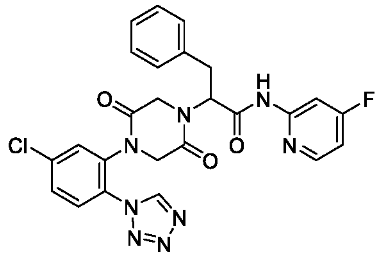 |
| 83 | 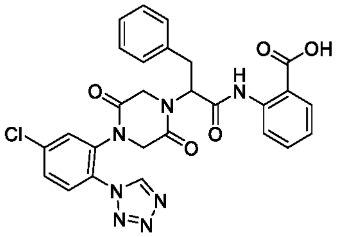 | 84 | 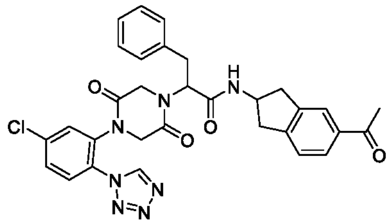 |
| 85 | 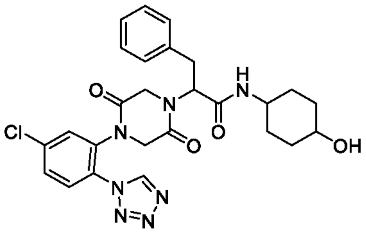 | 86 | 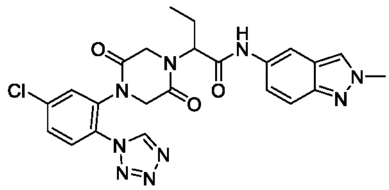 |
| 87 | 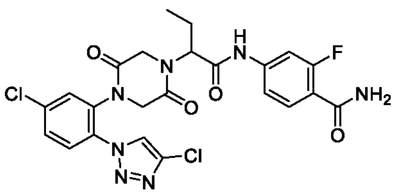 | 88 | 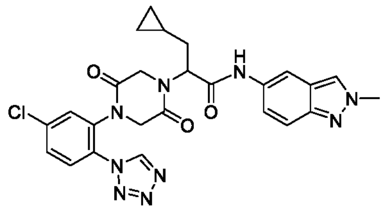 |
| 89 | 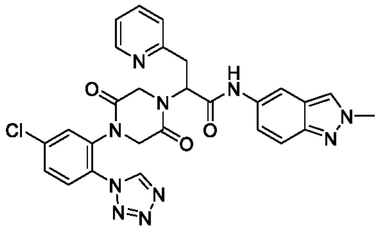 | 90 | 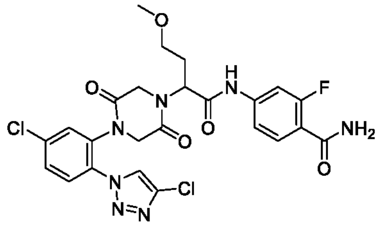 |
| 91 | 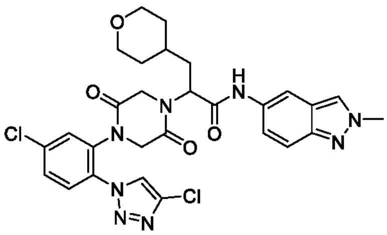 | 92 | 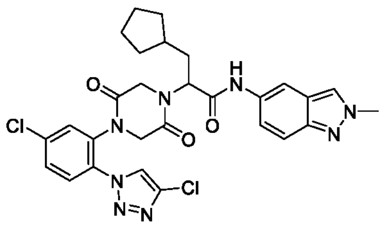 |

(continued)

| No. of compound | Structure | No. of compound | Structure |
|---|---|---|---|
| 93 | 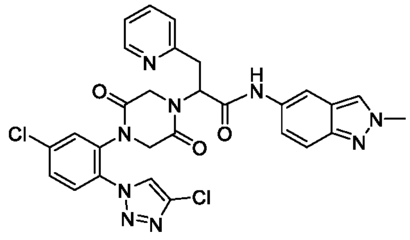 | 94 | 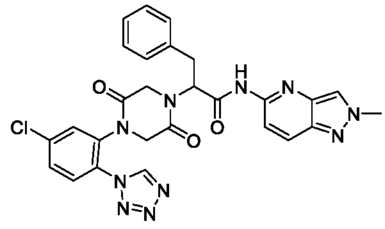 |
| 95 | 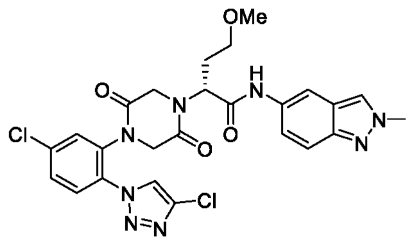 | 96 | 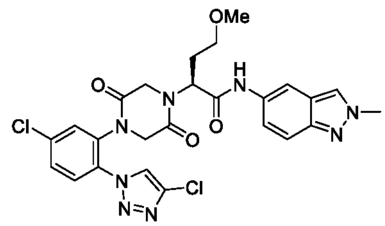 |
| 97 | 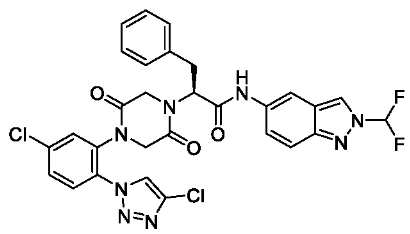 | 98 | 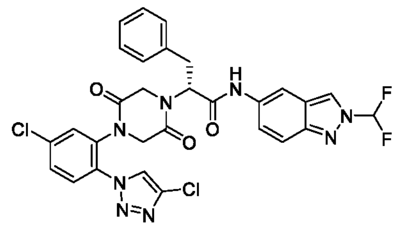 |
| 99 | 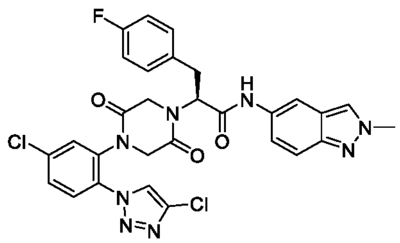 | 100 | 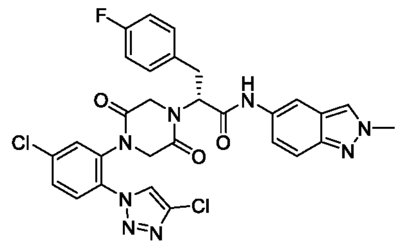 |
| 101 | 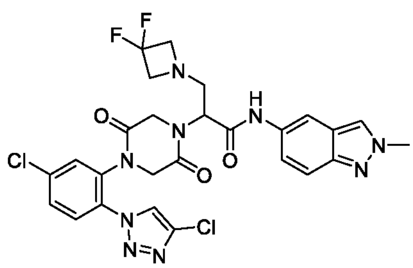 | 102 | 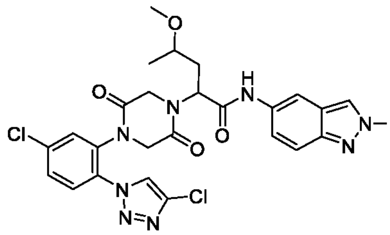 |
| 103 | 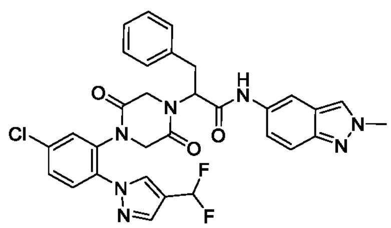 | 104 | 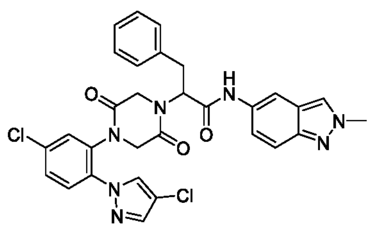 |

(continued)

| No. of compound | Structure | No. of compound | Structure |
|---|---|---|---|
| 105 | 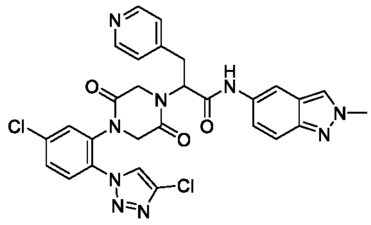 | 106 | 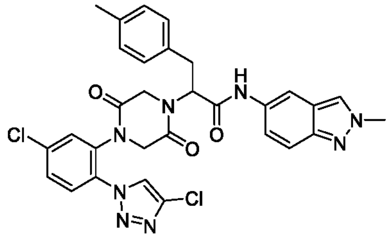 |
| 107 | 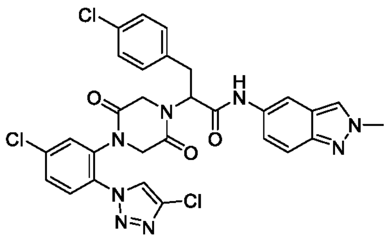 | 108 | 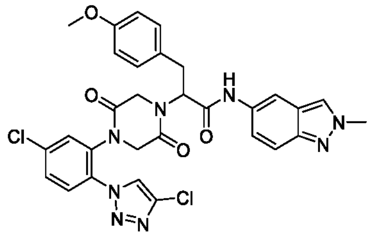 |
| 109 | 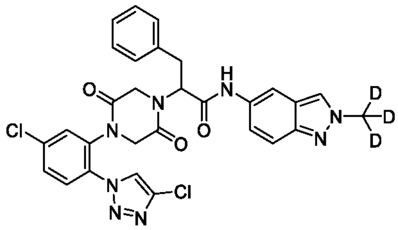 | 110 | 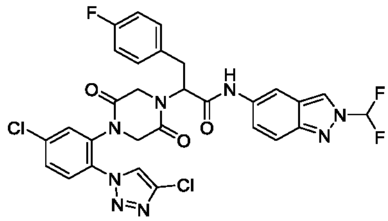 |
| 111 | 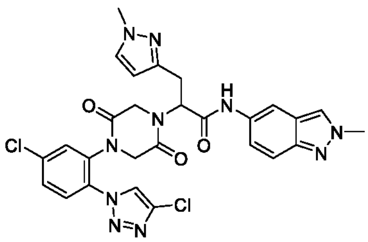 | 112 | 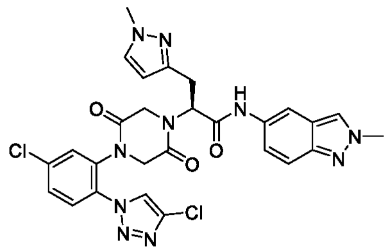 |
| 113 | 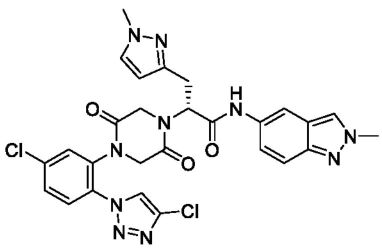 | 114 | 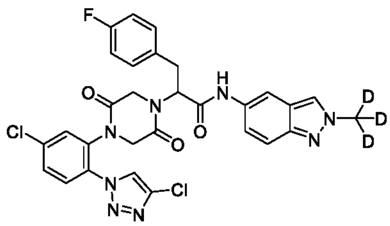 |
| 115 | 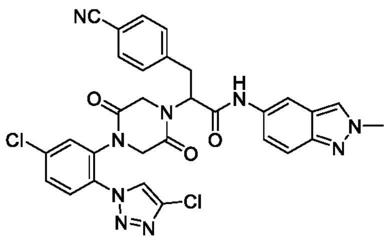 | 116 | 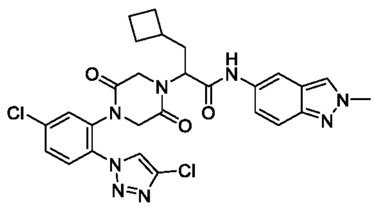 |

(continued)

| No. of compound | Structure | No. of compound | Structure |
|---|---|---|---|
| 117 | 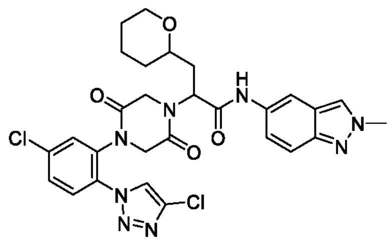 | 118 | 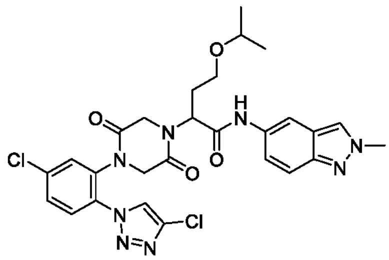 |
| 119 | 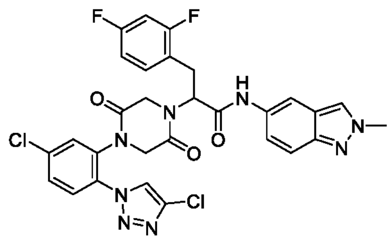 | 120 | 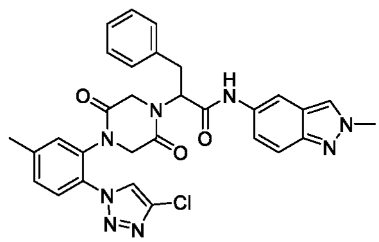 |
| 121 | 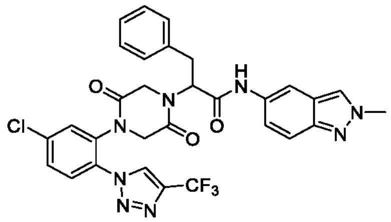 | 122 | 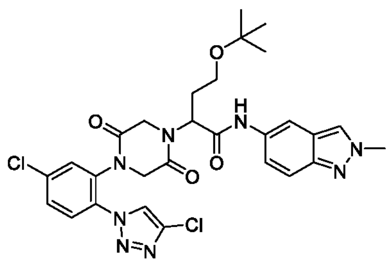 |
| 123 | 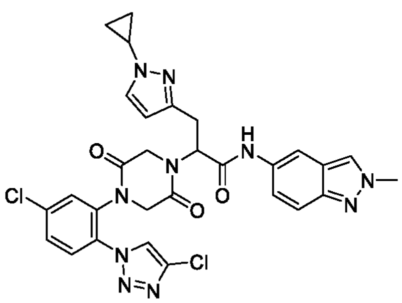 | 124 | 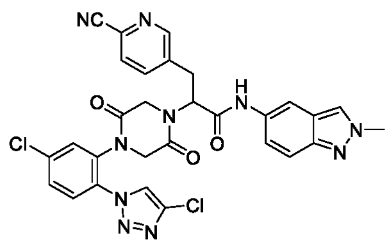 |
| 125 | 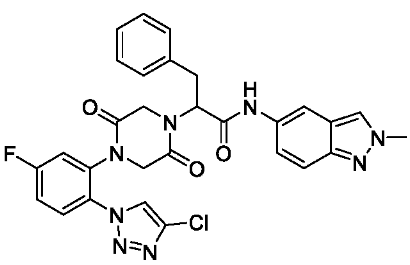 | 126 | 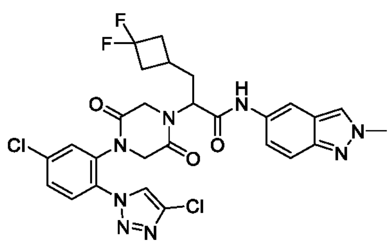 |

(continued)

| No. of compound | Structure | No. of compound | Structure |
|---|---|---|---|
| 131 | 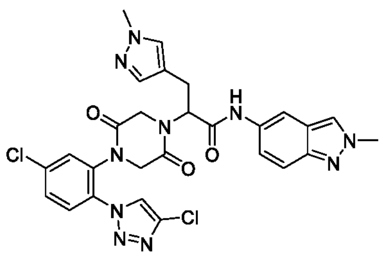 | 132 | 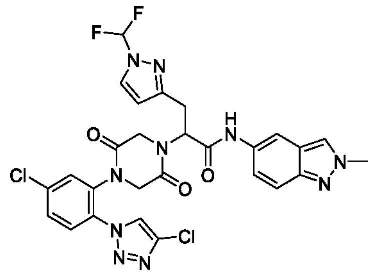 |
| 133 | 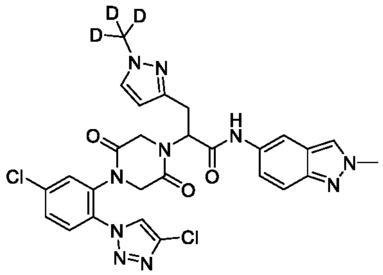 | 134 | 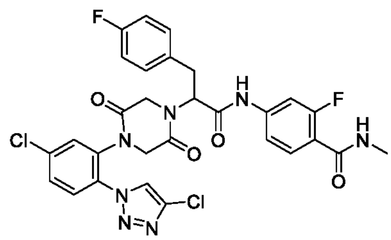 |
| 135 | 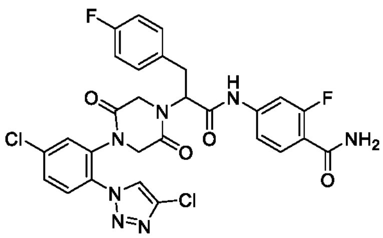 | 136 | 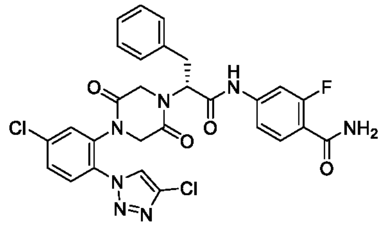 |
| 137 | 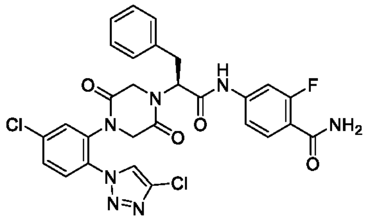 | 138 | 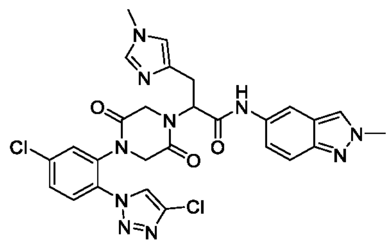 |

**19.** A preparation method for the compound represented by general formula (II) or the stereoisomer, the tautomer, the pharmaceutically acceptable salt or the prodrug thereof according to claim 2, comprising the following steps of:

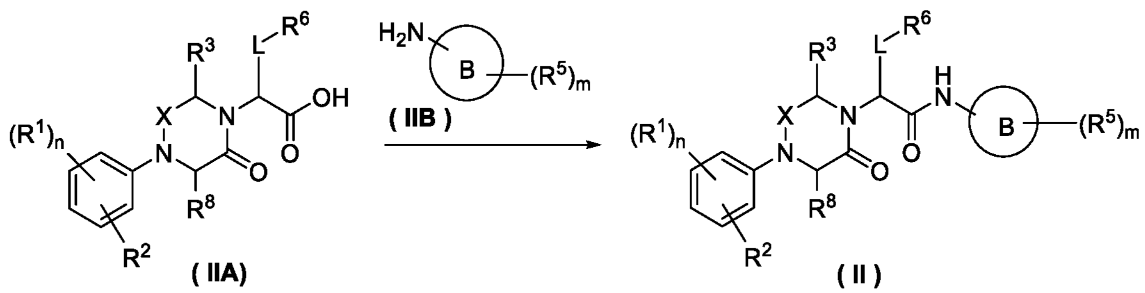

(IIA) (IIB) (II)

subjecting the compound represented by general formula (ΠA) and the compound represented by general formula (IIB) to a condensation reaction, and optionally further hydrolyzing the mixture under acidic conditions to obtain the

compound represented by general formula (II);
wherein: ring B, X, L, $R^1$ to $R^3$, $R^5$, $R^6$, $R^8$, m and n are as defined in claim 2.

**20.** A compound represented by general formula (IIA) or a stereoisomer, a tautomer, a pharmaceutically acceptable salt or a prodrug thereof, wherein:

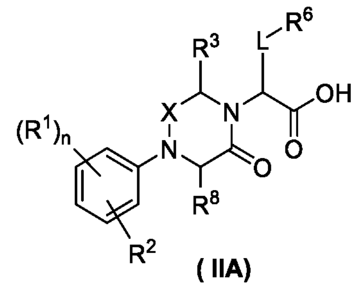

( IIA)

wherein: X, L, $R^1$ to $R^3$, $R^6$, $R^8$ and n are as defined in claim 2.

**21.** The compound represented by general formula (IIA) or the stereoisomer, the tautomer, the pharmaceutically acceptable salt or the prodrug thereof according to claim 20, wherein the compound is:

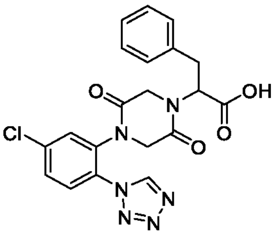 , 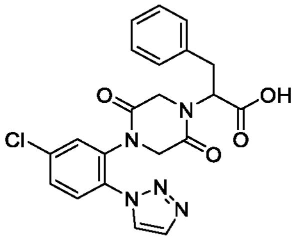 , 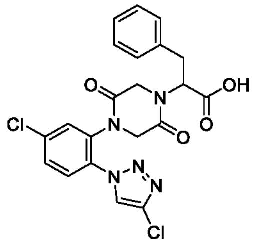 , 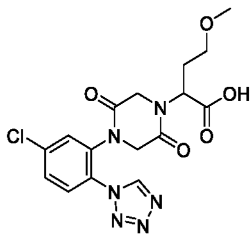 ,

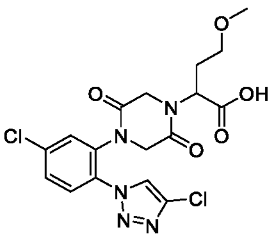 , 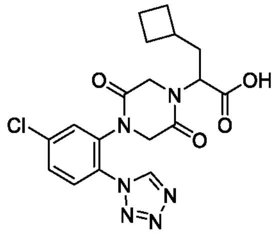 , 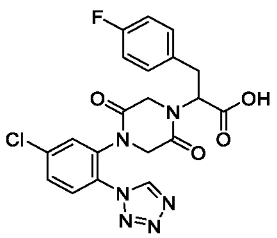 , 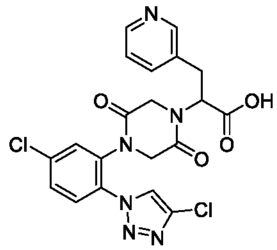 ,

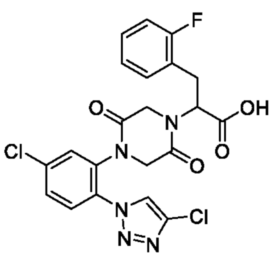 , 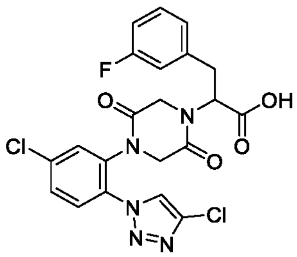 , 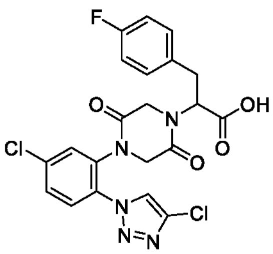 , 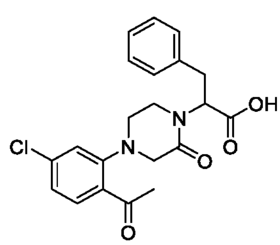 ,

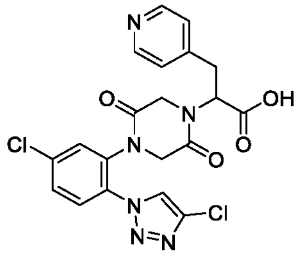 , 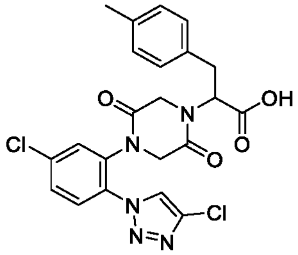 , 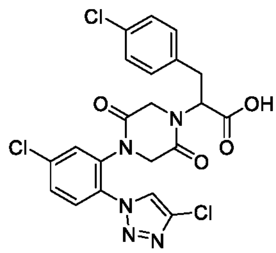 , 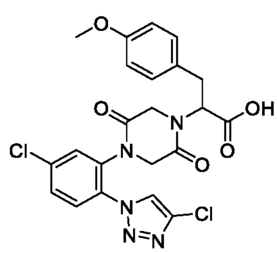 ,

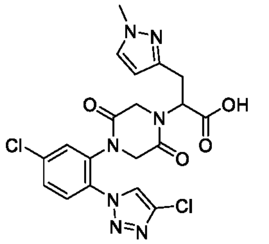 , 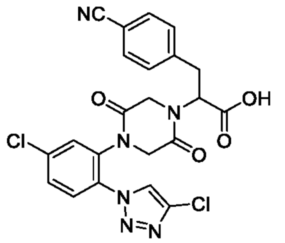 , 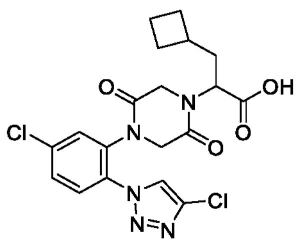 , 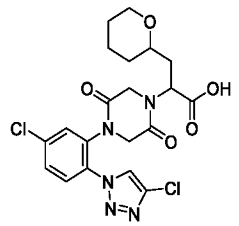 ,

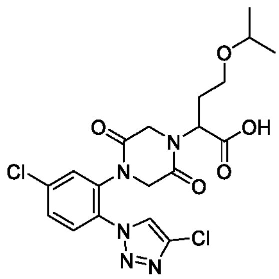 , 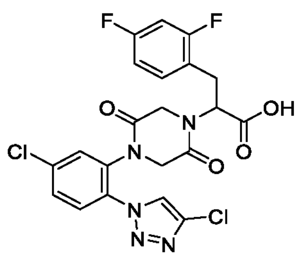 , 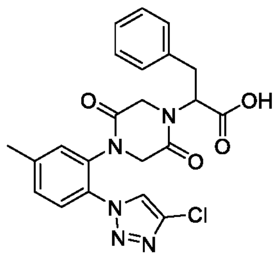 , 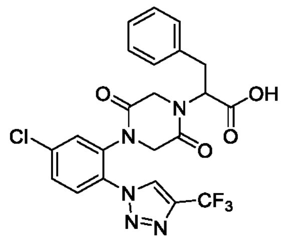 ,

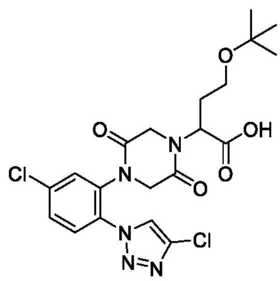 , 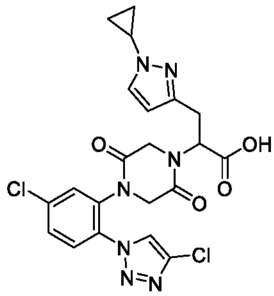 , 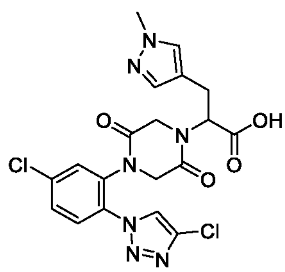 , 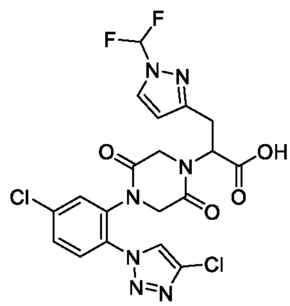

or

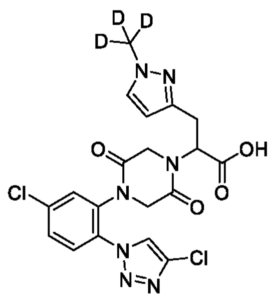 .

**22.** A pharmaceutical composition, wherein the pharmaceutical composition comprises an effective dose of the compound or the stereoisomer, the tautomer, the pharmaceutically acceptable salt or the prodrug according to any one of claims 1 to 18, and a pharmaceutically acceptable carrier, an excipient or a combination thereof.

**23.** Use of the compound or the stereoisomer, the tautomer, the pharmaceutically acceptable salt or the prodrug according to any one of claims 1 to 18, or the pharmaceutical composition according to claim 22 in the preparation of an inhibitor of coagulation factor XIa or a dual inhibitor of coagulation factor XIa and plasma kallikrein.

**24.** Use of the compound or the stereoisomer, the tautomer, the pharmaceutically acceptable salt or the prodrug according to any one of claims 1 to 18, or the pharmaceutical composition according to claim 22 in the preparation of a medicament for treating and/or preventing a disease mediated by a coagulation factor Xia;
wherein:

the disease mediated by the coagulation factor XIa is preferably a cardiovascular and cerebrovascular disease;
the cardiovascular and cerebrovascular disease is preferably selected from a coagulation disease or a thromboembolic disease; and
the thromboembolic disease is preferably selected from arterial cardiovascular thromboembolism, venous cardiovascular thromboembolism, arterial cerebrovascular thromboembolism, venous cerebrovascular thromboembolism, and ventricular or peripheral circulation thromboembolism; wherein, the thromboembolic disease is more preferably selected from unstable angina pectoris, acute coronary syndrome, atrial fibrillation, myocardial infarction, sudden ischemic death, transient ischemic attack, stroke, atherosclerosis, peripheral occlusive arterial diseases, venous thrombosis, thrombophlebitis, arterial embolism, coronary artery thrombosis, cerebral arterial thrombosis, cerebral embolism, renal embolism, pulmonary embolism, and thrombosis caused by a medical implant, device or surgery, wherein blood is contacted with an artificial surface capable of promoting thrombosis; and the venous thrombosis is preferably deep venous thrombosis.

**25.** Use of the compound or the stereoisomer, the tautomer, the pharmaceutically acceptable salt or the prodrug according to any one of claims 1 to 18, or the pharmaceutical composition according to claim 22 in the preparation of an anti-coagulation drug.

**26.** Use of the compound or the stereoisomer, the tautomer, the pharmaceutically acceptable salt or the prodrug according to any one of claims 1 to 18, or the pharmaceutical composition according to claim 22 in the preparation of a medicament for treating or preventing a thromboembolic disease, wherein:

the thromboembolic disease is selected from arterial cardiovascular thromboembolism, venous cardiovascular thromboembolism, arterial cerebrovascular thromboembolism, venous cerebrovascular thromboembolism, and ventricular or peripheral circulation thromboembolism; and
the thromboembolic disease is more preferably selected from unstable angina pectoris, acute coronary syndrome, atrial fibrillation, myocardial infarction, sudden ischemic death, transient ischemic attack, stroke, atherosclerosis, peripheral occlusive arterial diseases, venous thrombosis, thrombophlebitis, arterial embolism, coronary artery thrombosis, cerebral arterial thrombosis, cerebral embolism, renal embolism, pulmonary embolism, and thrombosis caused by a medical implant, device or operation, wherein blood is contacted with an artificial surface capable of promoting thrombosis; and the venous thrombosis is preferably deep venous thrombosis.

## INTERNATIONAL SEARCH REPORT

International application No.
**PCT/CN2021/118671**

**A. CLASSIFICATION OF SUBJECT MATTER**

C07D 403/10(2006.01)i； A61K 31/4425(2006.01)i； A61P 7/02(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS; VEN; WOTXT; USTXT; EPTXT; CJFD; CNKI; 万方; ISI Web of Science; STN-registry; STN-CAPLUS: 浙江海正药业股份有限公司, 上海昂睿医药技术有限公司, 邱海波, 马玉涛, 陈阿欢, 朱亚波, 张斌浩, 卢勇平, 叶成, 胡泰山, 钱文建, 陈磊, 哌嗪, FXI, XIa, 凝血因子, Piperazinedione, +phenyl+, +piperazinyl+

**C. DOCUMENTS CONSIDERED TO BE RELEVANT**

| Category* | Citation of document, with indication, where appropriate, of the relevant passages | Relevant to claim No. |
|---|---|---|
| X | CN 109563092 A (KANCERA AB) 02 April 2019 (2019-04-02)<br>description paragraph [1253] | 1, 6, 16, 17 |
| X | WO 2020047447 A1 (CYTOKINETICS INC) 05 March 2020 (2020-03-05)<br>description pages 110, 111, 139, 140, 148, 149, 152-154, 157, 158, 160, 183, 184, 186 | 1, 6, 16, 17 |
| X | WO 2018089433 A1 (NAVITOR PHARM INC) 17 May 2018 (2018-05-17)<br>description pages 171, 172, 187, 313, 428-431, 435 | 1, 6, 16, 17 |
| X | WO 2011016559 A1 (TAKEDA PHARMACEUTICAL COMPANY LIMITED) 10 February 2011 (2011-02-10)<br>description paragraphs [0395], [0398], [0401], [0413], [0416], [0419], [0440], [0443], [0458], [0461], [0467], [0476], [04679], [0482], [0485], [0488], [0491], [0494], [0497], [0509], [0512], [0515], [0518] | 1, 6, 16, 17 |
| X | WO 2011002067 A1 (TAKEDA PHARMACEUTICAL COMPANY LIMITED) 06 January 2011 (2011-01-06)<br>description paragraphs [0496], [0499], [0502], [0671], [0674], [0677], [0680], [0683], embodiments 847-853, 939-941 | 1, 6, 16, 17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 November 2021** | **06 December 2021** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No. **PCT/CN2021/118671**

**C. DOCUMENTS CONSIDERED TO BE RELEVANT**

| Category* | Citation of document, with indication, where appropriate, of the relevant passages | Relevant to claim No. |
|---|---|---|
| X | "RN 2481594-13-0" *REGISTRY enters STN,* 16 September 2020 (2020-09-16), | 1, 6, 16, 17 |
| X | "RN 2367629-98-7" *REGISTRY enters STN,* 21 August 2019 (2019-08-21), | 1, 6, 16, 17 |
| X | "RN 2367634-00-0" *REGISTRY enters STN,* 21 August 2019 (2019-08-21), | 1, 6, 16, 17 |
| X | "RN 2244906-77-0" *REGISTRY enters STN,* 11 October 2018 (2018-10-11), | 1, 6, 16, 17 |
| X | "RN 2244906-78-1" *REGISTRY enters STN,* 11 October 2018 (2018-10-11), | 1, 6, 16, 17 |
| X | "RN 2231428-70-7" *REGISTRY enters STN,* 24 July 2018 (2018-07-24), | 1, 6, 16, 17 |
| X | "RN 2231428-72-9" *REGISTRY enters STN,* 24 July 2018 (2018-07-24), | 1, 6, 16, 17 |
| X | "RN 2104068-30-4" *REGISTRY enters STN,* 27 July 2017 (2017-07-27), | 1, 6, 16, 17 |
| X | "RN 2104068-32-6" *REGISTRY enters STN,* 27 July 2017 (2017-07-27), | 1, 6, 16, 17 |
| X | "RN 2089631-28-5" *REGISTRY enters STN,* 13 April 2017 (2017-04-13), | 1, 6, 16, 17 |
| X | "RN 2093456-10-9" *REGISTRY enters STN,* 28 April 2017 (2017-04-28), | 1, 6, 16, 17 |
| X | "RN 1962177-29-2" *REGISTRY enters STN,* 28 July 2016 (2016-07-28), | 1, 6, 16, 17 |
| X | "RN 1962177-30-5" *REGISTRY enters STN,* 28 July 2016 (2016-07-28), | 1, 6, 16, 17 |
| X | "RN 1400758-67-9" *REGISTRY enters STN,* 11 October 2012 (2012-10-11), | 1, 6, 16, 17 |
| X | "RN 1400758-92-0" *REGISTRY enters STN,* 11 October 2012 (2012-10-11), | 1, 6, 16, 17 |
| X | "RN 1372928-21-6" *REGISTRY enters STN,* 07 May 2012 (2012-05-07), | 1, 6, 16, 17 |
| X | "RN 1372928-22-7" *REGISTRY enters STN,* 07 May 2012 (2012-05-07), | 1, 6, 16, 17 |
| X | "RN 1352448-45-3" *REGISTRY enters STN,* 05 January 2012 (2012-01-05), | 1, 6, 16, 17 |
| X | "RN 1352448-61-3" *REGISTRY enters STN,* 05 January 2012 (2012-01-05), | 1, 6, 16, 17 |
| X | "RN 1261277-97-7" *REGISTRY enters STN,* 01 February 2011 (2011-02-01), | 1, 6, 16, 17 |
| X | "RN 1261278-50-5" *REGISTRY enters STN,* 01 February 2011 (2011-02-01), | 1, 6, 16, 17 |
| X | "RN 281205-93-4" *REGISTRY enters STN,* 28 July 2000 (2000-07-28), | 1, 6, 16, 17 |
| X | "RN 890021-38-2" *REGISTRY enters STN,* 29 June 2006 (2006-06-29), | 1, 6, 16, 17 |

**INTERNATIONAL SEARCH REPORT**

International application No.
**PCT/CN2021/118671**

**C. DOCUMENTS CONSIDERED TO BE RELEVANT**

| Category* | Citation of document, with indication, where appropriate, of the relevant passages | Relevant to claim No. |
|---|---|---|
| X | "RN 262295-75-0"<br>*REGISTRY enters STN,* 18 April 2000 (2000-04-18), | 1, 6, 16, 17 |
| X | "RN 501127-93-1"<br>*REGISTRY enters STN,* 01 April 2003 (2003-04-01), | 1, 6, 16, 17 |
| X | "RN 477220-60-3"<br>*REGISTRY enters STN,* 19 December 2002 (2002-12-19), | 1, 6, 16, 17 |
| X | "RN 262295-70-5"<br>*REGISTRY enters STN,* 18 April 2000 (2000-04-18), | 1, 6, 16, 17 |
| X | "RN 262295-76-1"<br>*REGISTRY enters STN,* 18 April 2000 (2000-04-18), | 1, 6, 16, 17 |
| X | "RN 262295-77-2"<br>*REGISTRY enters STN,* 18 April 2000 (2000-04-18), | 1, 6, 16, 17 |
| X | "RN 301541-42-4"<br>*REGISTRY enters STN,* 07 November 2000 (2000-11-07), | 1, 6, 16, 17 |
| X | "RN 301541-43-5"<br>*REGISTRY enters STN,* 07 November 2000 (2000-11-07), | 1, 6, 16, 17 |
| A | WO 2018039094 A1 (MERCK SHARP & DOHME LIMITED) 01 March 2018 (2018-03-01)<br>entire document | 1-26 |

Form PCT/ISA/210 (second sheet) (January 2015)

**INTERNATIONAL SEARCH REPORT**
**Information on patent family members**

International application No.
**PCT/CN2021/118671**

| Patent document cited in search report | Publication date (day/month/year) | Patent family member(s) | Publication date (day/month/year) |
|---|---|---|---|
| CN 109563092 A | 02 April 2019 | KR 20190026006 A | 12 March 2019 |
| | | WO 2018011138 A1 | 18 January 2018 |
| | | JP 6891262 B2 | 18 June 2021 |
| | | JP 2019520412 A | 18 July 2019 |
| | | EP 3481824 A1 | 15 May 2019 |
| | | US 11008318 B2 | 18 May 2021 |
| | | US 2020024272 A1 | 23 January 2020 |
| | | IN 201947004675 A | 22 March 2019 |
| | | CN 109563092 B | 14 September 2021 |
| | | US 2021267994 A1 | 02 September 2021 |
| WO 2020047447 A1 | 05 March 2020 | BR 112021003496 A2 | 18 May 2021 |
| | | AR 116283 A1 | 21 April 2021 |
| | | CA 3110237 A1 | 05 March 2020 |
| | | TW 202023648 A | 01 July 2020 |
| | | AU 2019328556 A1 | 11 March 2021 |
| | | CN 112867539 A | 28 May 2021 |
| | | EP 3843842 A1 | 07 July 2021 |
| | | KR 20210068422 A | 09 June 2021 |
| | | US 2020109148 A1 | 09 April 2020 |
| | | IN 202117014500 A | 07 May 2021 |
| | | SG 11202101769 A1 | 30 March 2021 |
| WO 2018089433 A1 | 17 May 2018 | None | |
| WO 2011016559 A1 | 10 February 2011 | None | |
| WO 2011002067 A1 | 06 January 2011 | None | |
| WO 2018039094 A1 | 01 March 2018 | US 10143681 B2 | 04 December 2018 |
| | | TW 201808908 A | 16 March 2018 |
| | | EP 3500556 A1 | 26 June 2019 |
| | | US 2018050022 A1 | 22 February 2018 |
| | | AR 109387 A1 | 28 November 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017151746 A1
- WO 2017151018 A1
- WO 2018039094 A1
- CN 108863962
- CN 105384739
- WO 2019099311 A
- US 20170275282 A1
- WO 2017005725 A1


### Non-patent literature cited in the description


- *Synthesis,* 2018, vol. 50 (3), 555-564, 557